# EUROPEAN PATENT SPECIFICATION

(11) **EP 1 438 337 B1**
(45) Date of publication and mention of the grant of the patent: **20.09.2006**
(21) Application number: 02770119.2
(22) Date of filing: 25.10.2002
(51) Int. Cl.: C07K 14/80, C12N 9/02, C12N 15/09

(54) **CRYSTALS OF CYTOCHROME P450 2C9, STRUCTURES THEREOF AND THEIR USE**
CYTOCHROME P450 2C9 KRISTALLE, STRUKTUREN UND DESSEN VERWENDUNG
CRISTAUX DE CYTOCHROME P450 2C9, LEURS STRUCTURES ET LEUR UTILISATION

(30) Priority: 25.10.2001 US 330585 P; 14.12.2001 US 339421 P; 20.12.2001 US 341267 P; 18.07.2002 US 396588 P
(43) Date of publication of application: 21.07.2004
(73) Proprietor: Astex Therapeutics Limited, Milton Road, Cambridge CB4 0QA (GB)
(72) Inventor: WILLIAMS, Pamela Ann, c/o Astex Therapeutics Ltd, Cambridge CB4 0QA (GB); COSME, Jose, Marie, c/o Astex Therapeutics Ltd, Cambridge CB4 0QA (GB); WARD, Alison, c/o Astex Therapeutics Ltd, Cambridge,CB4 0QA (GB); BREWERTON, Suzanne Clare, Astex Therapeutics Ltd, Cambridge,CB4 0QA (GB); HAMILTON, Bruce John, c/o Astex Therapeutics Ltd, Cambridge,CB4 0QA (GB); JHOTI, Harren, c/o Astex Therapeutics Ltd, Cambridge,CB4 0QA (GB); JONES, Michelle Ann, c/o Astex Therapeutics Ltd, Cambridge CB4 0QA (GB); VUILLARD, Laurent M. M., c/o Astex Therapeutic Ltd, Cambridge CB4 0QA (GB); WILLIAMS, Mark Gareth, c/o Astex TherapeuticsLtd, Cambridge, CB4 0QA (GB)
(74) Representative: Brasnett, Adrian Hugh
(86) International application number: PCT/GB2002/004872
(87) International publication number: WO 2003/035693

(56) References cited:
- WO-A-01/11035
- WO-A-01/14565
- HASEMANN C A ET AL: "CRYSTAL STRUCTURE AND REFINEMENT OF CYTOCHROME P450TERP AT 2.3 A RESOLUTION" JOURNAL OF MOLECULAR BIOLOGY, LONDON, GB, vol. 236, no. 4, 1994, pages 1169-1185, XP001120740 ISSN: 0022-2836
- PARK SAM-YONG ET AL: "Crystallization and preliminary X-ray diffraction analysis of a cytochrome P450 (CYP119) from Sulfolobus solfataricus." ACTA CRYSTALLOGRAPHICA SECTION D BIOLOGICAL CRYSTALLOGRAPHY, vol. 56, no. 9, September 2000 (2000-09), pages 1173-1175, XP009010347 ISSN: 0907-4449
- RIDDERSTROM MARIANNE ET AL: "Arginines 97 and 108 in CYP2C9 are important determinants of the catalytic function." BIOCHEMICAL AND BIOPHYSICAL RESEARCH COMMUNICATIONS, vol. 270, no. 3, 21 April 2000 (2000-04-21), pages 983-987, XP002241443 ISSN: 0006-291X
- PAYNE VILIA ANN ET AL: "Homology modeling and substrate binding study of human CYP2C9 enzyme." PROTEINS, vol. 37, no. 2, 1 November 1999 (1999-11-01), pages 176-190, XP009011018 ISSN: 0887-3585
- EKINS SEAN ET AL: "Pharmacophore and three-dimensional quantitative structure activity relationship methods for modeling cytochrome P450 active sites." DRUG METABOLISM AND DISPOSITION, vol. 29, no. 7, July 2001 (2001-07), pages 936-944, XP001122105 ISSN: 0090-9556
- LEWIS D F V: "HOMOLOGY MODELLING OF HUMAN CYTOCHROME P450 INVOLVED IN XENOBIOTIC METABOLISM AND RATIONALIZATION OF SUBSTRATE SELECTIVITY" EXPERIMENTAL AND TOXICOLOGIC PATHOLOGY, JENA, DE, vol. 51, no. 4/5, July 1999 (1999-07), pages 369-374, XP009003755 ISSN: 0940-2993
- STUBBINS M J ET AL: "GENETIC ANALYSIS OF THE HUMAN CYTOCHROME P450 CYP2C9 LOCUS" PHARMACOGENETICS, CHAPMAN & HALL, LONDON, GB, vol. 6, 1996, pages 429-439, XP000918307 ISSN: 0960-314X

## Description

### Field of the Invention.

The present invention relates to the human cytochrome P450 protein 2C9, methods for its crystallization, its X-ray crystal structure and the use thereof.

### Background to the Invention.

Cytochrome P450s (CYP450) form a very large and complex gene superfamily of hemeproteins that metabolise physiologically important compounds in many species of microorganisms, plants and animals. Cytochrome P450s are important in the oxidative, peroxidative and reductive metabolism of numerous and diverse endogenous compounds such as steroids, bile, fatty acids, prostaglandins, leukotrienes, retinoids and lipids. Many of these enzymes also metabolise a wide range of xenobiotics including drugs, environmental compounds and pollutants. Their involvement in drug metabolism is extensive, it is estimated that 50% of all known drugs are affected in some way by the action of CYP450 enzymes. Significant resource is employed by the pharmaceutical industry to optimise drug candidates in order to avoid their detrimental interactions with the CYP450 enzymes. Another level of complication results from the fact that these enzymes exhibit different tissue distributions and polymorphisms between individuals and ethnic populations

Most mammalian P450s are located in the liver, but other organs and tissues have high concentrations of certain cytochrome P450s, including the intestinal wall, lung, kidney, adrenal cortex and nasal epithelium. Mammals have about 50 unique CYP450 genes and each family member is 45-55 KDa in size and contains a heme moiety that catalyses a two-electron activation of oxygen. The source of electrons may be used to classify CYP450s. Those that receive electrons in a three protein chain in which electrons flow from a flavin adenine dinucleotide (FAD) containing reductase, to an iron-sulphur protein, and then to P450 belong to the group of class I P450s, and include most of the bacterial enzymes. Class II P450s receive electrons from a reductase containing both FAD and flavin mononucleotide (FMN), and comprise the microsomal P450s that are the main culprits of drug metabolism. The mammalian microsomal cytochrome P450s are integral membrane proteins anchored by an N-terminal transmembrane spanning α-helix. They are inserted in the membrane of the endoplasmic reticulum by a short, highly hydrophobic N-terminal segment that acts as a non-cleavable signal sequence for insertion into the membrane. The remainder of the mammalian cytochrome P450 protein is a globular structure that protrudes into the cytoplasmic space. Hence, the bulk of the enzyme faces the cytoplasmic surface of the lipid bilayer. P450s require other membranous enzymatic components for activity including the flavoprotein NADPH-cytochrome P450 oxidoreductase and, in some cases, cytochrome b5. A single cytochrome P450 oxidoreductase supports the activity of all the mammalian microsomal enzymes by interacting directly with the P450s and transferring the required two electrons from NADPH. Cytochrome P450s are able to incorporate one of the two oxygen atoms of an O₂ molecule into a broad variety of substrates with concomitant reduction of the other oxygen atom by two electrons to H₂O. Cytochrome P450 are known to catalyse hydroxylations, epoxidation, N-, S-, and O-dealkylations, N-oxidations, sulfoxidations, dehalogenations, and other reactions.

The genes of the P450 superfamily have been categorized by Nelson *et al* (Pharmacogenetics, 6; 1-42, 1996) who proposed a systematic nomenclature for the family members. This nomenclature is used widely in the art, and is adopted herein. Nelson *et al* provide cross-references to sequence database entries for P450 sequences.

*Homo sapiens* has 17 cytochrome P450 gene families and 42 subfamilies that total more than 50 sequenced isoforms. Cytochrome P450s from families 1, 2 and 3 constitute the major pathways for drug metabolism. Many drugs rely on hepatic metabolism by cytochrome P450s for clearance from the circulation and for pharmacological inactivation. Conversely, some drugs have to be converted in the body to their pharmacologically active metabolites by P450s. Many promising lead compounds are terminated in the development phase due to their interaction with one or more P450s. One of the greatest problems in drug discovery is the prediction of the role of cytochrome P450s on the metabolism or modification of drug leads. Early detection of metabolic problems associated with a chemical lead series is of paramount importance for the pharmaceutical industry. Obtaining crystal structures of the main human drug metabolising cytochrome P450s would be highly valuable for drug design, as this would provide detailed information on how P450 enzymes recognize drug molecules and the mode of drug binding. This in turn would allow drug companies to develop strategies to modify metabolic clearance and decrease the attrition rates of compounds in development.

The major human CYP450 isoforms involved in drug metabolism are CYP1A2, CYP2C9, CYP2C19, CYP2D6 and CYP3A4. The level of sequence identity between these family members ranges from about 20-80%, with much of the variability within the residues involved in substrate recognition. CYP450 enzymes are also present in bacteria and much of the understanding of substrate recognition is derived from crystal structures obtained of bacterial CYP450 enzymes.

It is well-known in the art of protein chemistry, that crystallising a protein is a chancy and difficult process without any clear expectation of success. It is now evident that protein crystallization is the main hurdle in protein structure determination. For this reason, protein crystallization has become a research subject in and of itself, and is not simply an extension of the protein crystallographer's laboratory. There are many references which describe the difficulties associated with growing protein crystals. For example, Kierzek, A.M. and Zielenkiewicz, P., (2001), Biophysical Chemistry, 91, 1-20, *Models ofprotein crystal* growth, and Wiencek, J.M. (1999) Annu. Rev. Biomed. Eng., 1, 505-534, *New Strategies for crystal growth.*

It is commonly held that crystallization of protein molecules from solution is the major obstacle in the process of determining protein structures. The reasons for this are many; proteins are complex molecules, and the delicate balance involving specific and non-specific interactions with other protein molecules and small molecules in solution, is difficult to predict.

Each protein crystallizes under a unique set of conditions, which cannot be predicted in advance. Simply supersaturating the protein to bring it out of solution may not work, the result would, in most cases, be an amorphous precipitate. Many precipitating agents are used, common ones are different salts, and polyethylene glycols, but others are known. In addition, additives such as metals and detergents can be added to modulate the behaviour of the protein in solution. Many kits are available (e.g. from Hampton Research), which attempt to cover as many parameters in crystallization space as possible, but in many cases these are just a starting point to optimise crystalline precipitates and crystals which are unsuitable for diffraction analysis. Successful crystallization is aided by a knowledge of the proteins behaviour in terms of solubility, dependence on metal ions for correct folding or activity, interactions with other molecules and any other information that is available. Even so, crystallization of proteins is often regarded as a time-consuming process, whereby subsequent experiments build on observations of past trials.

In cases where protein crystals are obtained, these are not necessarily always suitable for diffraction analysis; they may be limited in resolution, and it may subsequently be difficult to improve them to the point at which they will diffract to the resolution required for analysis. Limited resolution in a crystal can be due to several things. It may be due to intrinsic mobility of the protein within the crystal, which can be difficult to overcome, even with other crystal forms. It may be due to high solvent content within the crystal, which consequently results in weak scattering. Alternatively, it could be due to defects within the crystal lattice which mean that the diffracted x-rays will not be completely in phase from unit to unit within the lattice. Any one of these or a combination of these could mean that the crystals are not suitable for structure determination.

Some proteins never crystallize, and after a reasonable attempt it is necessary to examine the protein itself and consider whether it is possible to make individual domains, different N or C-terminal truncations, or point mutations. It is often hard to predict how a protein could be re-engineered in such a manner as to improve crystallisability. Our understanding of crystallisation mechanisms are still incomplete and the factors of protein structure which are involved in crystallisation are poorly understood.

As of 2000, eight cytochrome P450 structures have been solved by X-ray crystallography and are available in the public domain. All of the cytochrome P450s, whose structures have been solved, were expressed in *E. coli.* Six structures correspond to bacterial cytochrome P450s: P450cam (CYP101 Poulos *et al.,* 1985, *J. Biol. Chem.,* 260, 16122), the hemeprotein domain of P450BM3 (CYP102, Ravichandran *et al.,* 1993, *Science,* 261, 731), P450terp (CYP108, Hasemann *et al.*, 1994, *J. Mol. Biol.* 236, 1169), P450eryF (CYP107A1, Cupp-Vickery and Poulos, 1995, *Nature* Struct. Biol. 2, 144), P450 14α-sterol demethylase (CYP51, Podust *et al.,* 2001, *Proc. Natl. Acad. Sci. USA,* 98, 3068) and the crystal structure of a thermophilic cytochrome P450 (CYP119) from Archaeon sulfolobus solfataricus was solved (Yano *et al.,* 2000, *J. Biol. Chem.* 275, 31086). The structure of cytochrome P450nor was obtained from the denitrifying fungus Fusarium oxysporum (Shimizu *et al.* 2000, *J. Inorg. Biochem.* 81, 191). The eighth structure is that of the rabbit 2C5 isoform, the first and only structure of a mammalian cytochrome P450 (Williams *et al.* 2000, *Mol. Cell.* 5, 121).

WO011456 relates to the use of genetic material encoding a cytochrome P450 protein in the generation of a genetically modified cell, which cell has the capacity to produce a pigment in the presence of indole or a precursor, analogue or derivative thereof upon expression of said genetic material. Hasemann *et al* (J. Mol. Biol. (1994) Mar 4;236(4):1169-85) describes the crystal structure and refinement of cytochrome P450terp at 2.3 A resolution. Park *et al,* (*Acta Crystallographica,* D56, (2000), 1173-1175) describes the crystallization and preliminary X-ray diffraction analysis of cytochrome P450 CYP119 from *Sulfolobus solfataricus.* Ridderstrom et al (Biochem. Biophys. Res. Commun. (2000) Apr 21;270(3):983-7) describe that arginines 97 and 108 in CYP2C9 are important determinants of the catalytic function as determined by modelling and site-directed mutagenesis. Payne VA *et al* (Proteins. (1999) Nov 1;37(2):176-90) describes homology modeling and substrate binding study of human CYP2C9 enzyme. Ekins *et al* (Drug Metab. Dispos. (2001) Jul;29(7):936-44) describes pharmacophore and three-dimensional quantitative structure activity relationship methods for modeling cytochrome p450 active sites. Lewis (Exp. Toxicol. Pathol. (1999) Jul;51 (4-5):369-74) describes homology modelling of human cytochromes P450 involved in xenobiotic metabolism and rationalization of substrate selectivity.

The reason why the mammalian cytochrome P450s have been particularly difficult to crystallize, compared to their bacterial counterparts, resides in the nature of these proteins. The bacterial cytochrome P450s are soluble whereas the mammalian P450s are membrane-associated proteins. Thus, structural studies on mammalian cytochrome P450s may use the combination of heterologous expression systems that allow expression of single cytochrome P450s at high concentration with modification of their sequences to improve the solubility and the behaviour of these proteins in solution.

Due to significant sequence differences from both the bacterial proteins and rabbit proteins, to fully understand the role of the human CYP450 enzymes in drug metabolism, the crystal structures of human isoforms are still required.

Ibeanu et al., (1996), J Biol Chem, Vol. 271, 12496-12501 describe the production of modified 2C9 proteins in yeast in which certain residues, including Ser 220 and Pro 221, were altered. These altered proteins were found to exhibit 2C 19-like activity for omeprazole. The proteins retained wild-type N-terminal sequence.

### Disclosure of the Invention.

A nomenclature has been adopted to describe the secondary structure observed in cytochromes P450. The authors of the first structure of a P450, P450cam denoted the 12 helical segments A through L from the N-terminal to C-terminal direction and this naming has been continued as more structures have been determined. In addition, some P450 structures have shown more helices, for example the description of P450 BM3 details 15 helices (A, B, B', C, D, E, F, G, H, I, J, J', K, K', L), where the additional helices are indicated by the symbol.

Each helix is typically 6 amino acids (Helix H in 2C5) to 32 amino acids (Helix I in BM3) in length. The helices are linked by β-strands, short linkers and long flexible loops of up to 30 amino acids in length.

Among these flexible structures, one of the most pronounced is the loop between the F and G helices ("the F-G loop"). This loop is probably involved in the substrate access channel, and could move to accommodate the substrate in the active site. This loop has also been described as participating, with the N-terminus domain, to the anchorage of the cytochrome P450s to the membrane.

In the 2C5 structure (PDB ID 1DT6) helix F ends at residue 206 and helix G starts at residue 228, therefore the loop between is residues 207 to 227 (definitions from the secondary structure assignment program DSSP (Kabsch and Sander, Biopolymers 22 (1983) 2577-2637)). Of these 21 residues, 12 cannot be seen in the 2C5 structure. This is an indication of its flexible nature. We predict it will be similar in the rest of the 2C family and in other human P450s. The region is thought to be involved in membrane association of the enzyme in vivo, and orientation of the substrate access channels.

Using the models we have developed, we predicted the F-G loop in 2C9 was from Leu208 to Pro227. This region contains 20 residues, 11 of which can be classified as hydrophobic, further supporting the hypothesis that this region may be embedded in membranes or involved in aggregation as has been suggested. From the 2C9 structure of the invention, (definitions from DSSP), the loop between helixes F and G actually starts at 209 and ends at 227.

In one aspect, the invention provides a computer-based method for the analysis of the interaction of a molecular structure with a P450 structure, which comprises:
(a) providing a 2C9 P450 structure of any one of Tables 1, 2, 3 and 8 or coordinates having an rmsd of less than 2.0 Å from the backbone atoms of said Tables; or at least 100 selected coordinates thereof;
(b) providing a molecular structure to be fitted to said P450 structure or selected coordinates thereof; and
(c) fitting the molecular structure to said P450 structure.

In another aspect, the invention provides a method of predicting three dimensional structures of P450 homologues (e.g. 2C8, 2C18 or 2C19) or analogues of unknown structure, the method comprises the steps of:
aligning a representation of an amino acid sequence of a target P450 protein of unknown three-dimensional structure with the amino acid sequence of the P450 of Table 1, 2, 3 or 8 to match homologous regions of the amino acid sequences;
modelling the structure of the matched homologous regions of said target P450 of unknown structure on the corresponding regions of a 2C9 P450 structure having an rmsd of less than 2.0Å from the backbone atoms of any one of Tables 1, 2, 3 and 8;
determining a conformation for said target P450 of unknown structure which substantially preserves the structure of said matched homologous regions.

The invention also provides a method for determining the structure of a protein, which method comprises;
providing the co-ordinates of a 2C9 P450 structure having an rmsd of less than 2.0Å from the backbone atoms of any one of Tables 1, 2, 3 and 8, or at least 100 selected coordinates thereof, and
either (a) positioning said co-ordinates in the crystal unit cell of said protein so as to provide a structure for said protein, or (b) assigning NMR spectra peaks of said protein by manipulating said co-ordinates.

The invention further provides a method for determining the structure of a compound bound to P450 protein, said method comprising:
providing a crystal of P450 protein;
soaking the crystal with the compound to form a complex; and
determining the structure of the complex by employing the data of a 2C9 P450 structure having an rmsd of less than 2.0Å from the backbone atoms of any one of Tables 1, 2, 3 and 8, or at least 100 selected coordinates thereof.

The invention also provides a method for determining the structure of a compound bound to P450 protein, said method comprising:
mixing P450 protein with the compound;
crystallizing a P450 protein-compound complex; and
determining the structure of the complex by employing the data of a 2C9 P450 structure having an rmsd of less than 2.0Å from the backbone atoms of any one of Tables 1, 2, 3 and 8, or at least 100 selected coordinates thereof.

Preferred aspects of the invention are described further herein and in the accompanying claims.

The above aspects of the invention, both singly and in combination, all contribute to features of the invention which are advantageous.

### Description of the Drawings

Figure 1 sets out Table 1, providing the coordinates of a 2C9 structure,
Figure 2 sets out Table 2, providing the coordinates of a 2C9-FGloop K206E structure,
Figure 3 sets out Table 3, providing the coordinates of a 2C9-FGloop structure, and
Figure 4 sets out Table 7, providing modelled coordinates of residues 215, 216, 220, 221, 222, and 223 of a 2C9 wild type protein.
Figure 5 sets out Table 8, providing a refined structure of 2C9-FGloop K206E.
Figure 6 sets out Table 11, showing conditions in which crystals of proteins of the invention were obtained.
Figure 7 sets out Table 18, showing a homology model of 2C19.
Figure 8 sets out Table 19, showing 2C18 replacement coordinates.
Figure 9 sets out Table 20, showing 2C8 replacement coordinates.
Figure 10 shows the sequence alignment of the N-terminal truncated 2C9 variants and 2C9trunc with the published 2C9 wild type sequence (Meehan et al. 1988, *Am. J. Hum. Genet.* 42, 26).
Figure 11 shows data from 4-diclofenac hydroxylase assays.

### Description of Tables.

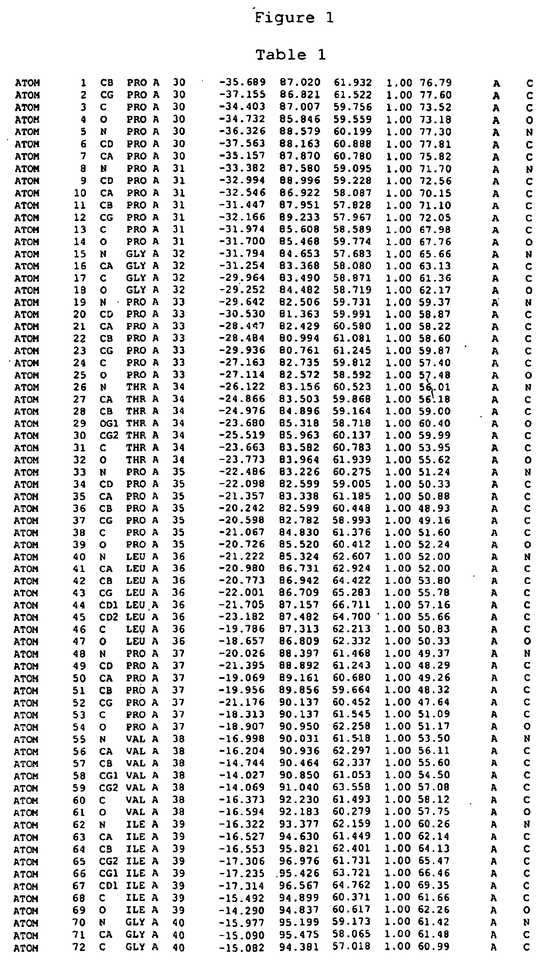

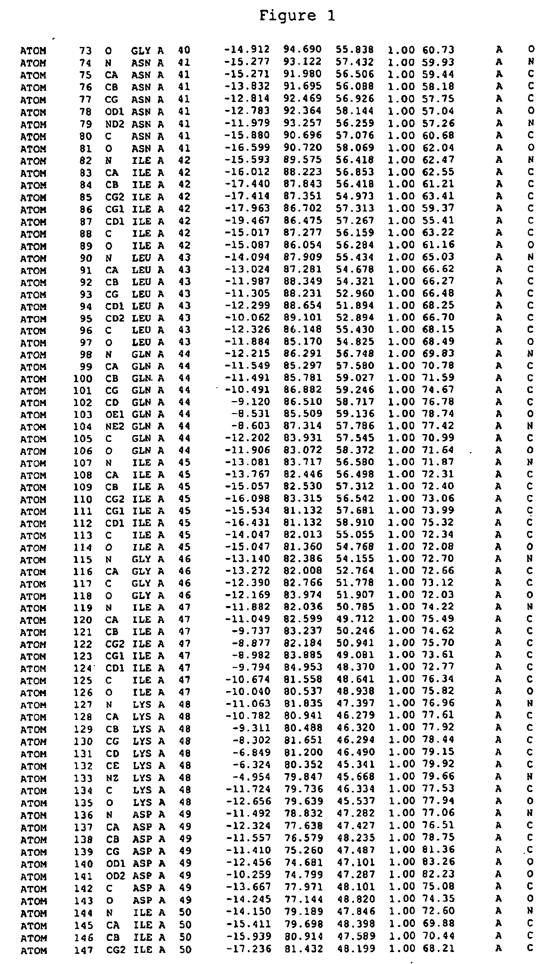

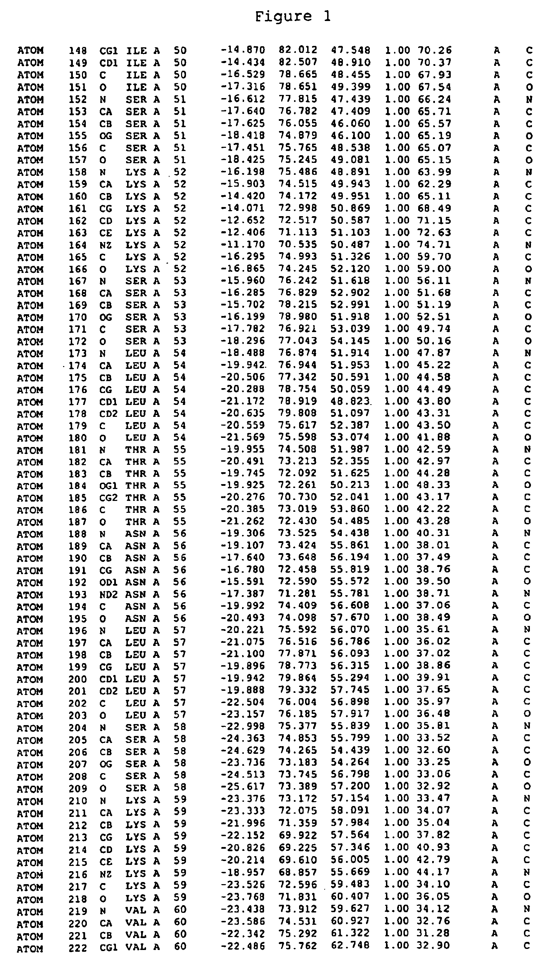

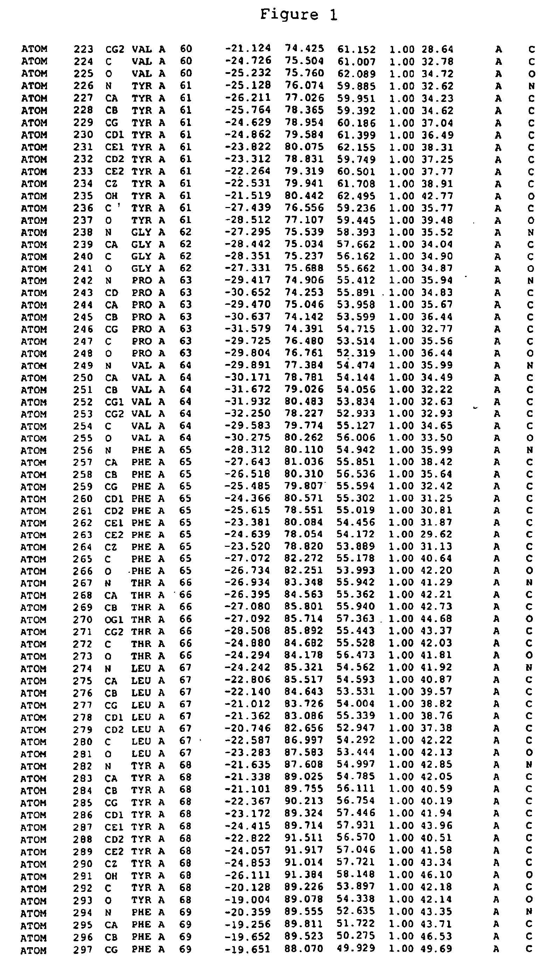

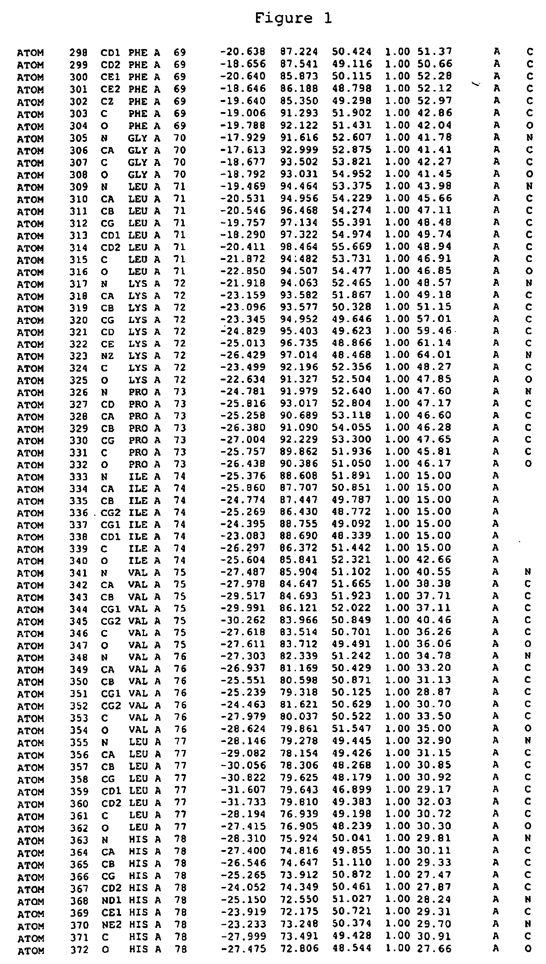

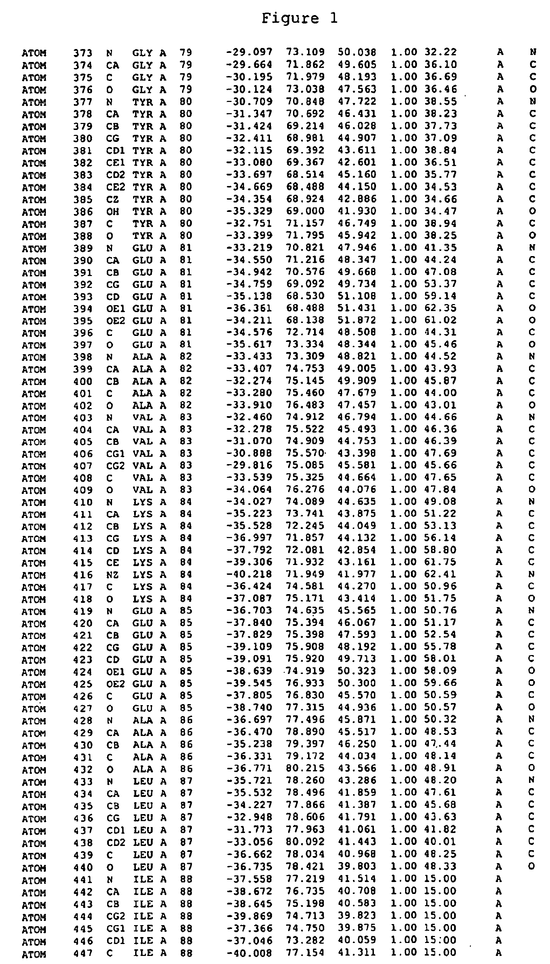

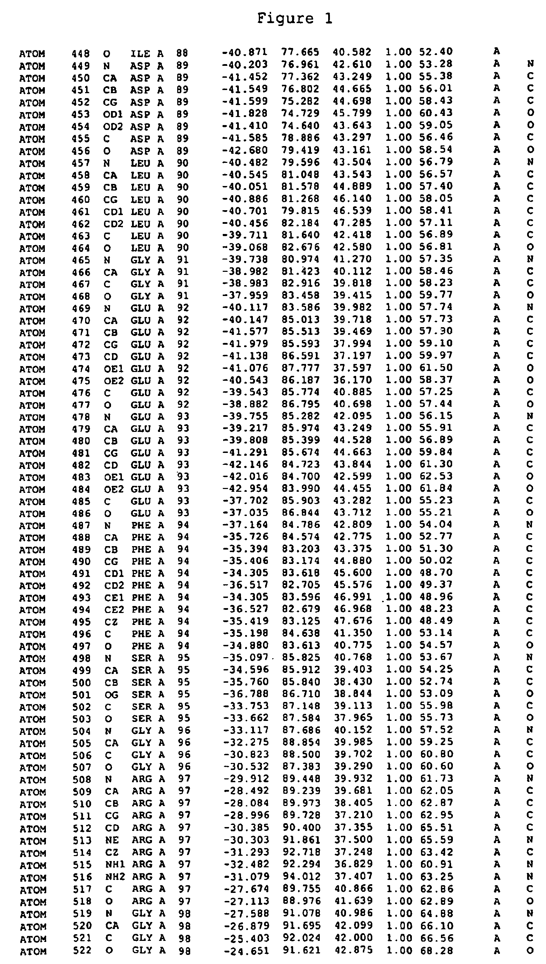

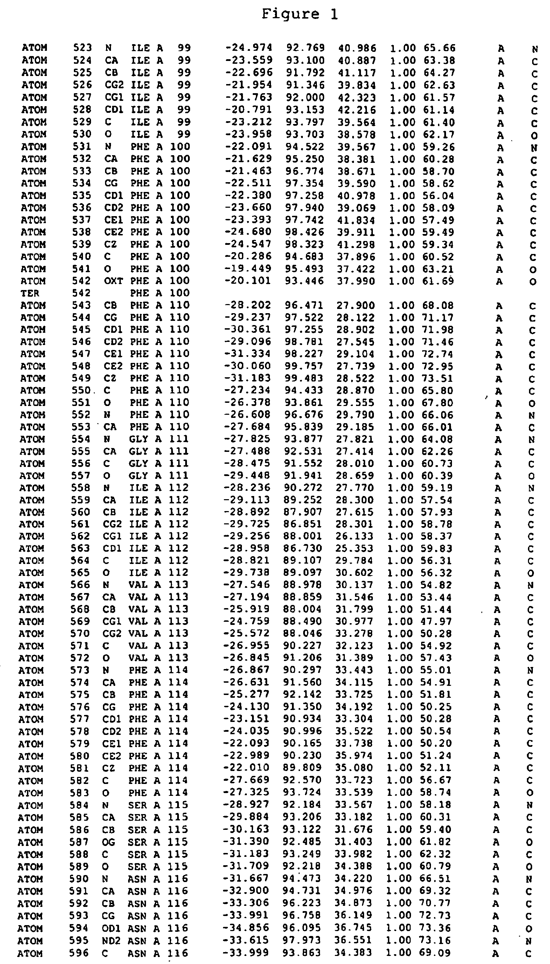

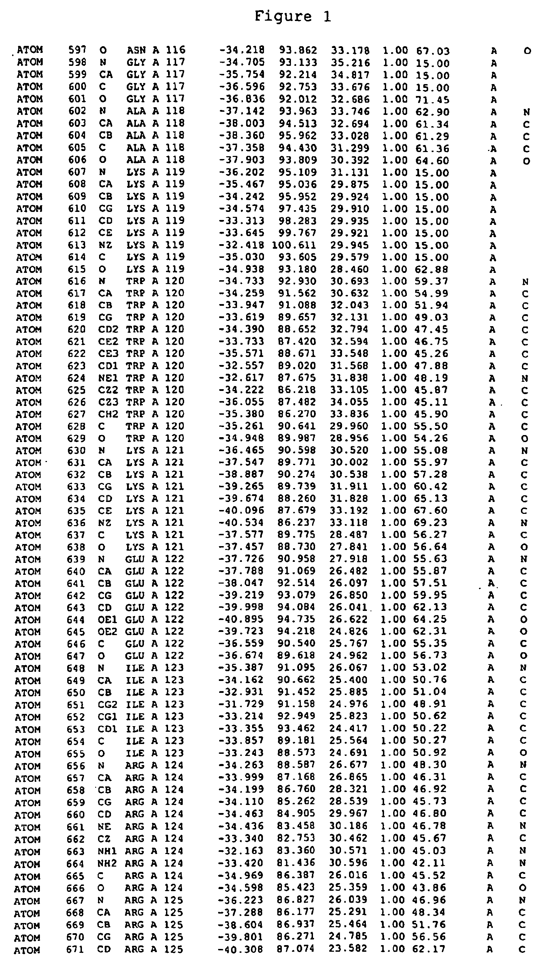

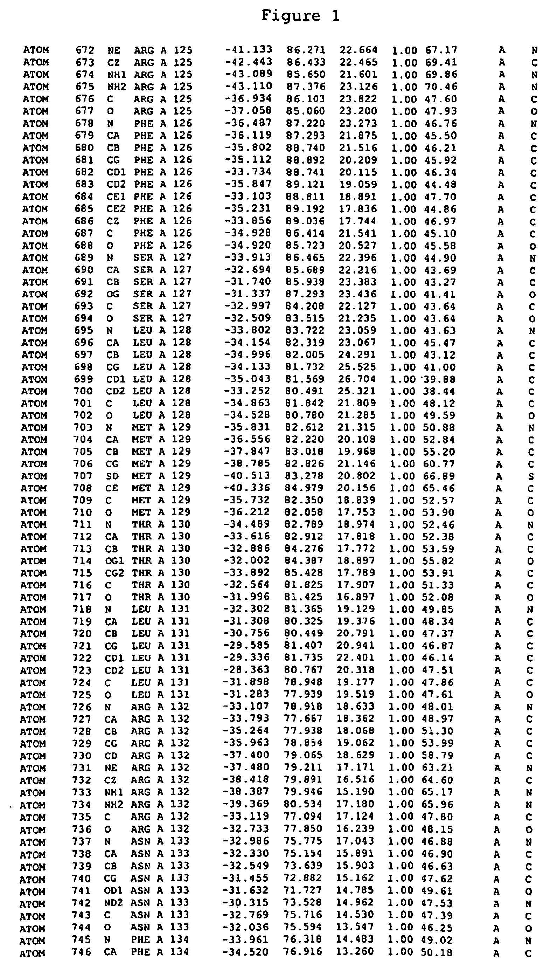

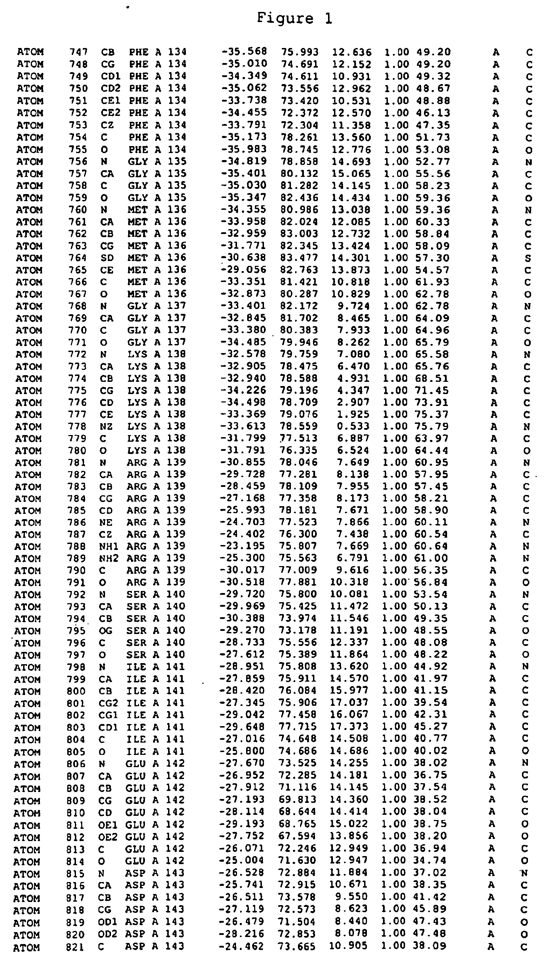

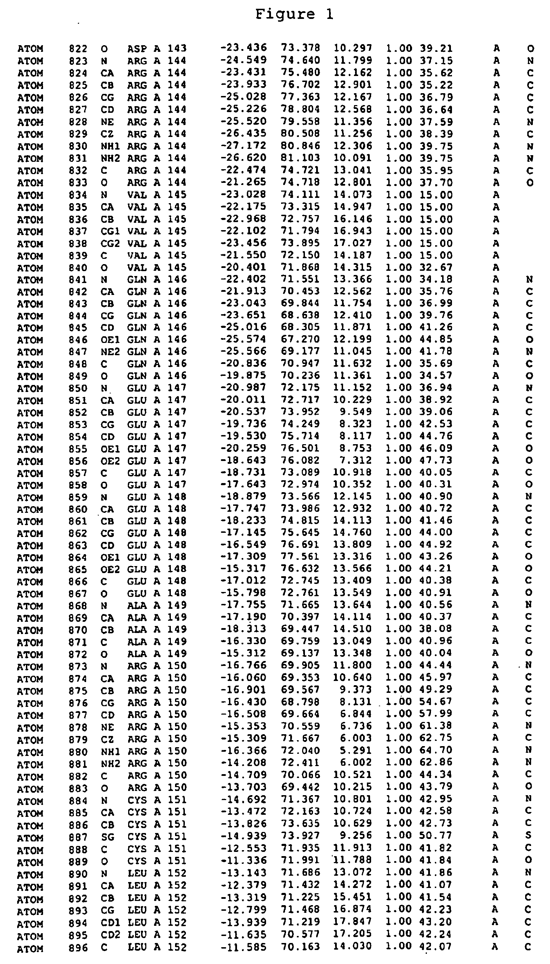

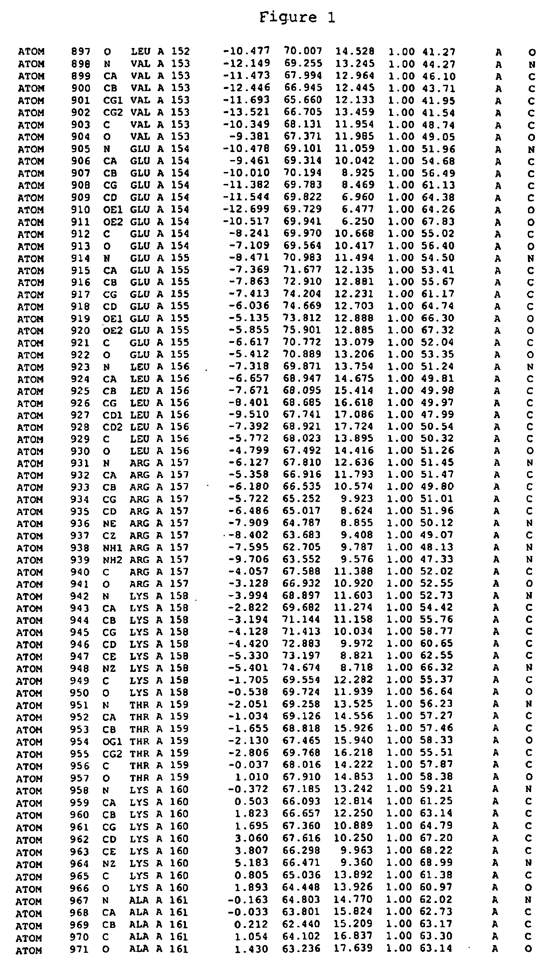

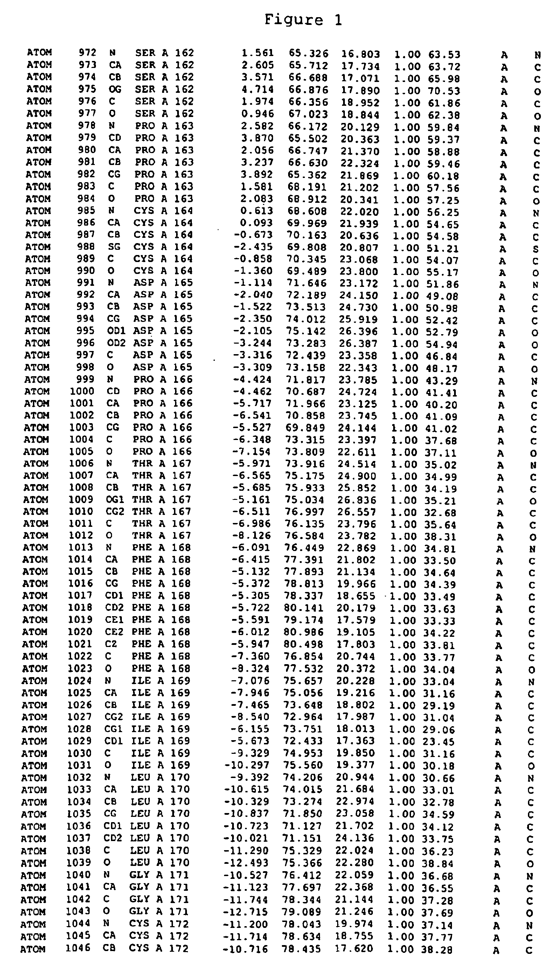

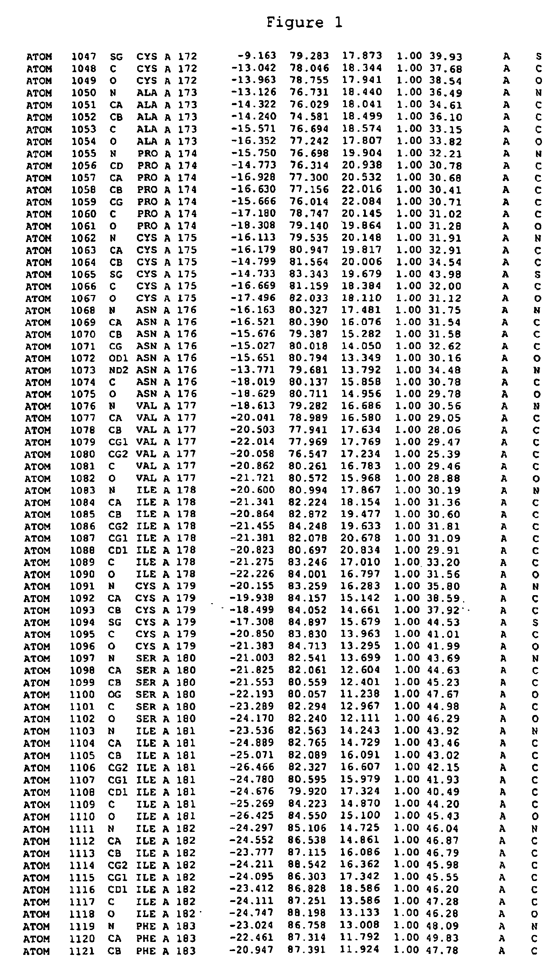

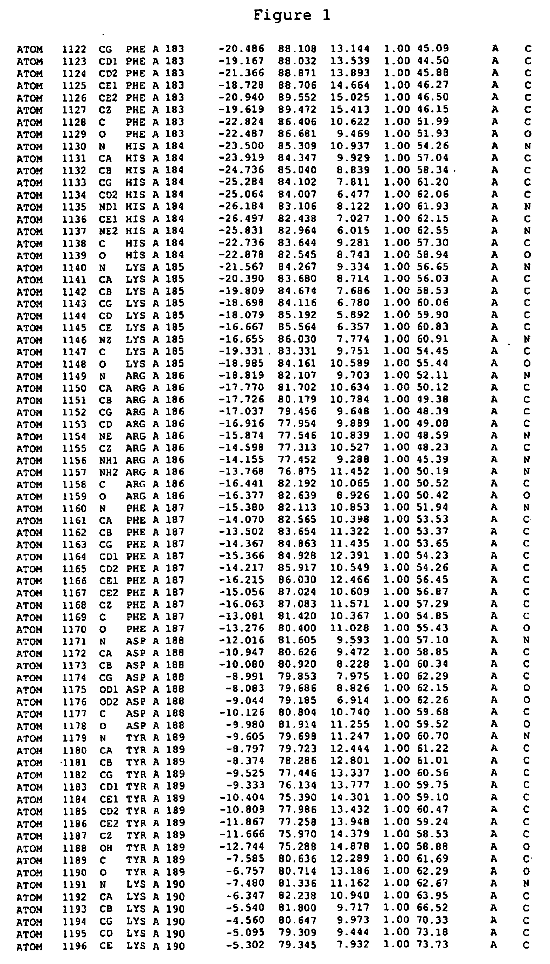

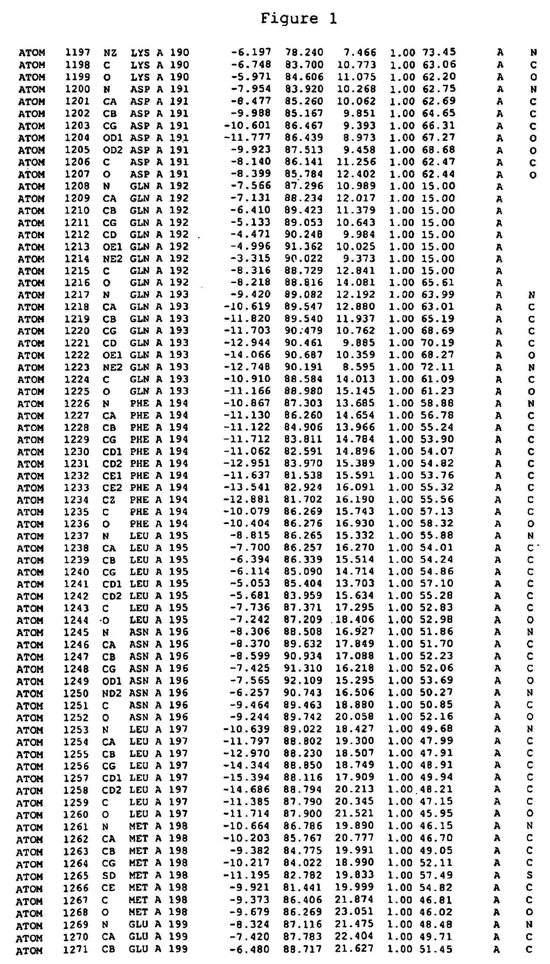

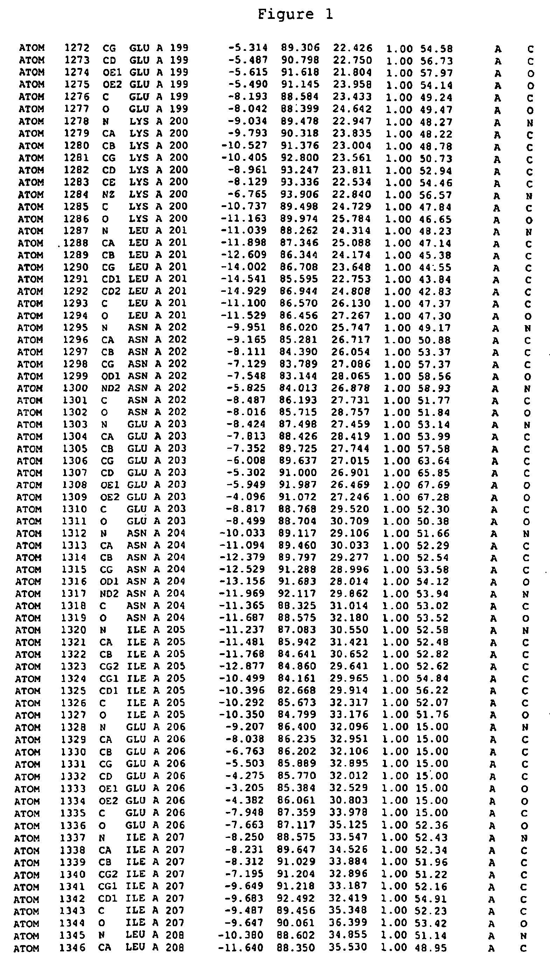

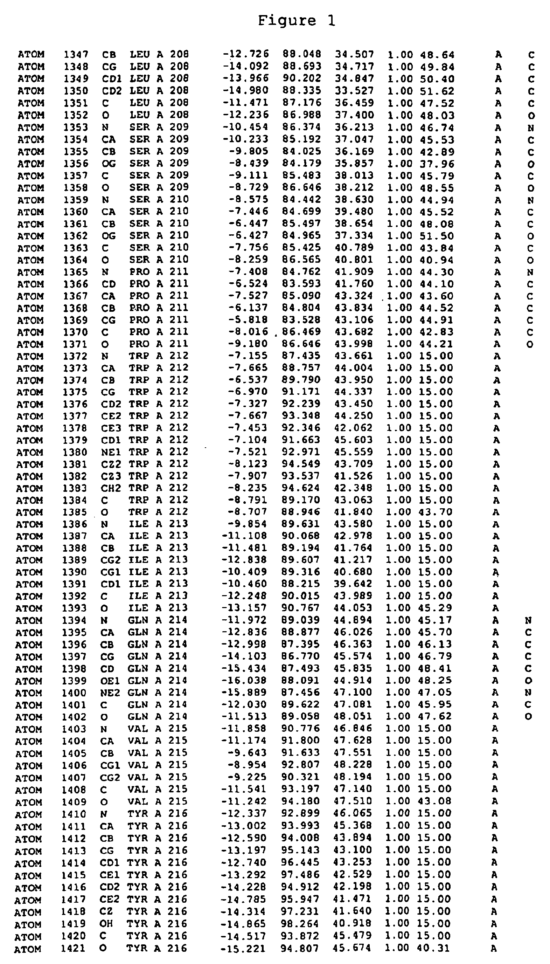

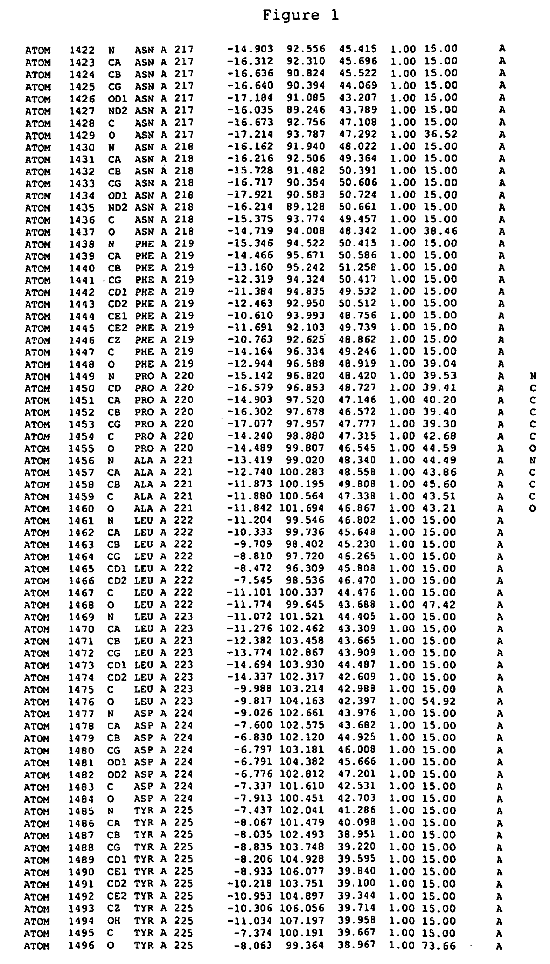

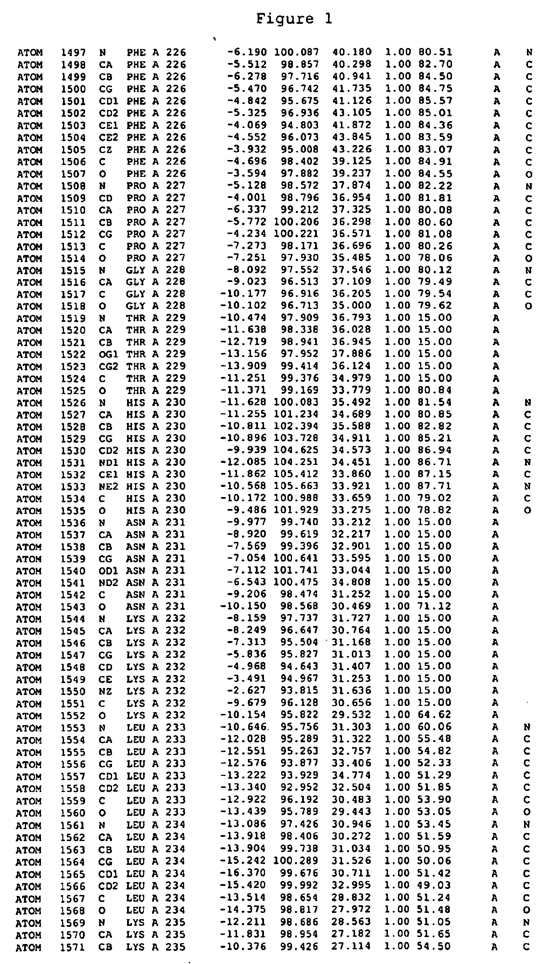

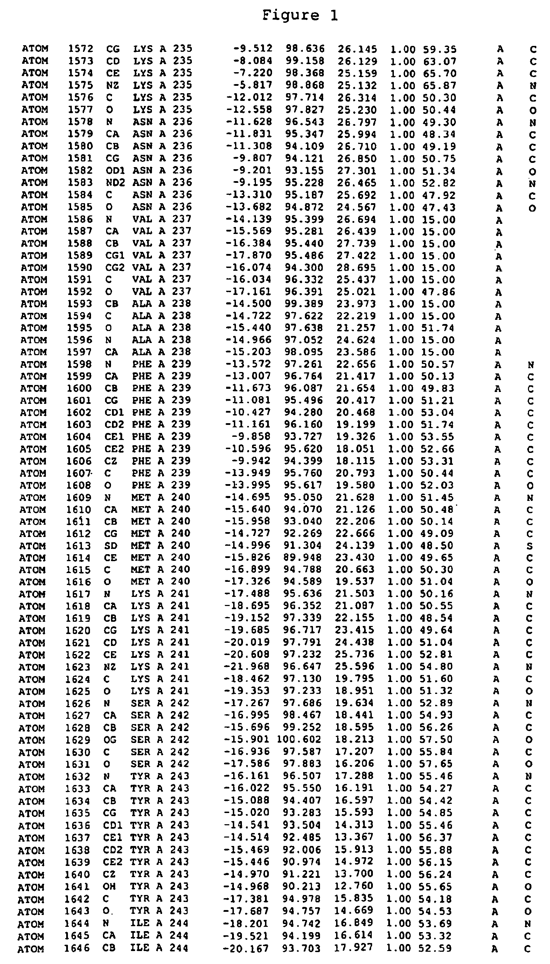

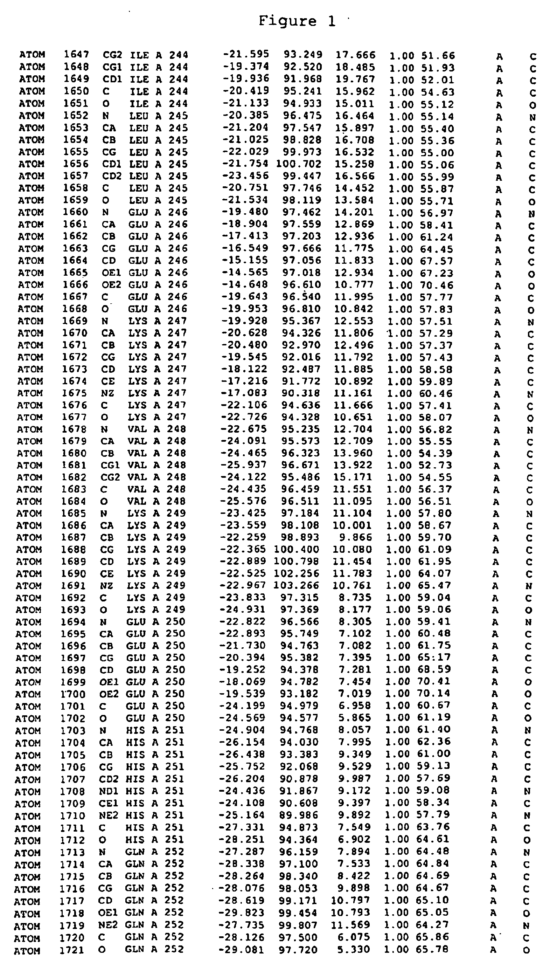

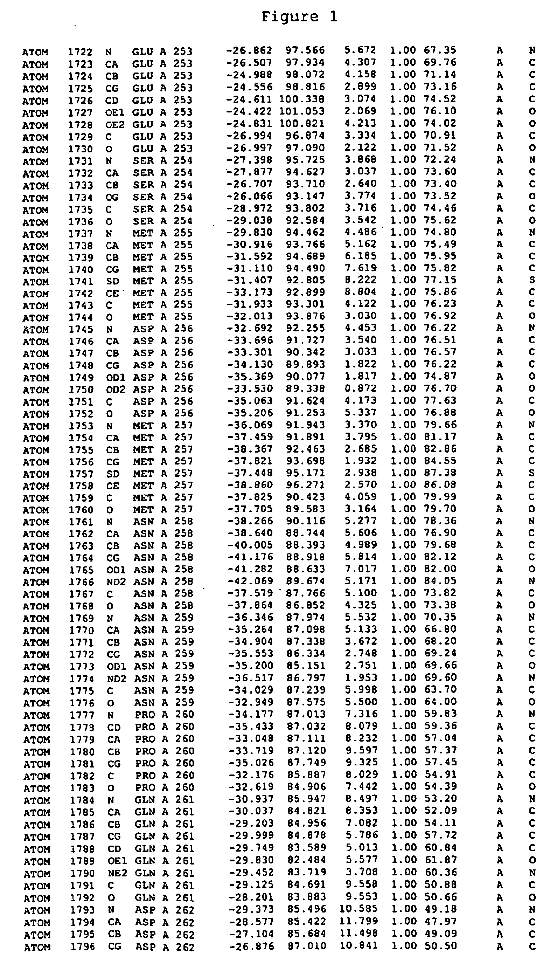

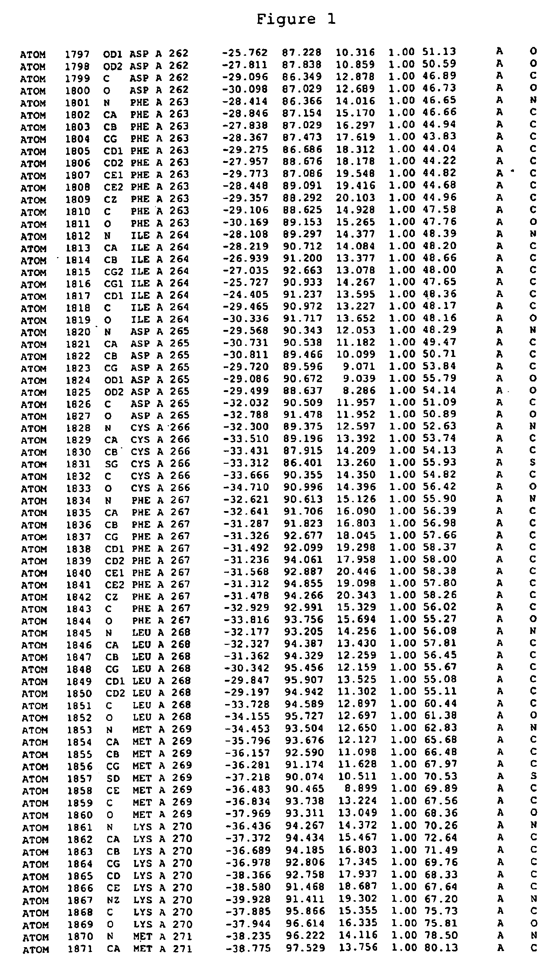

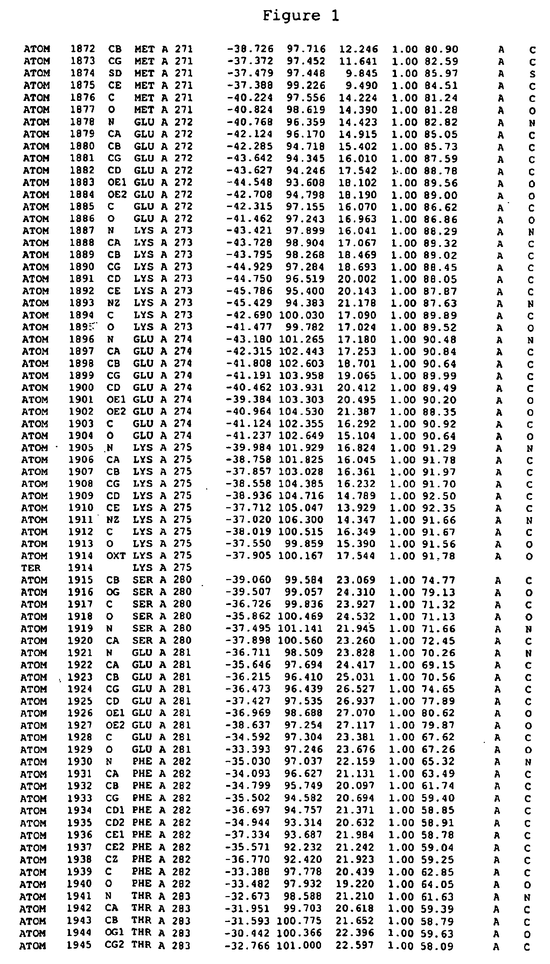

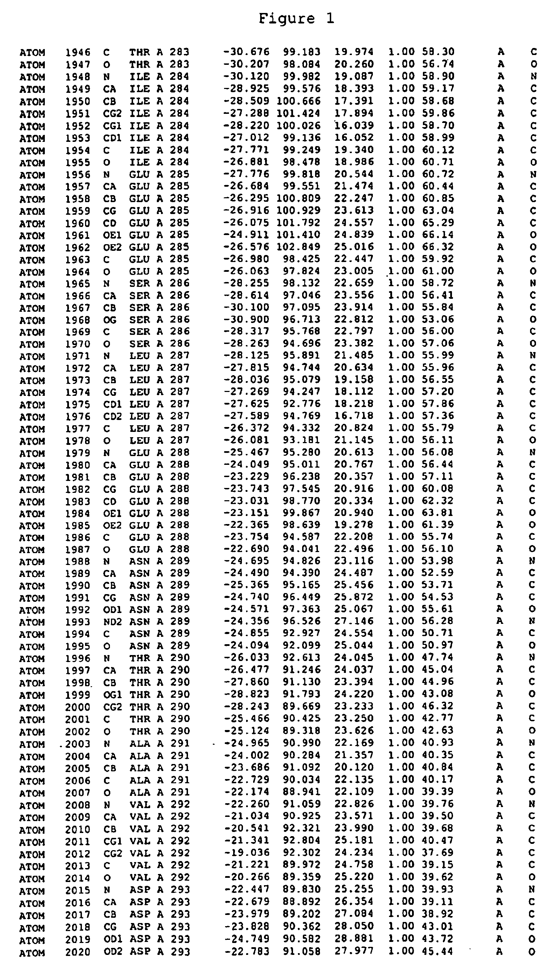

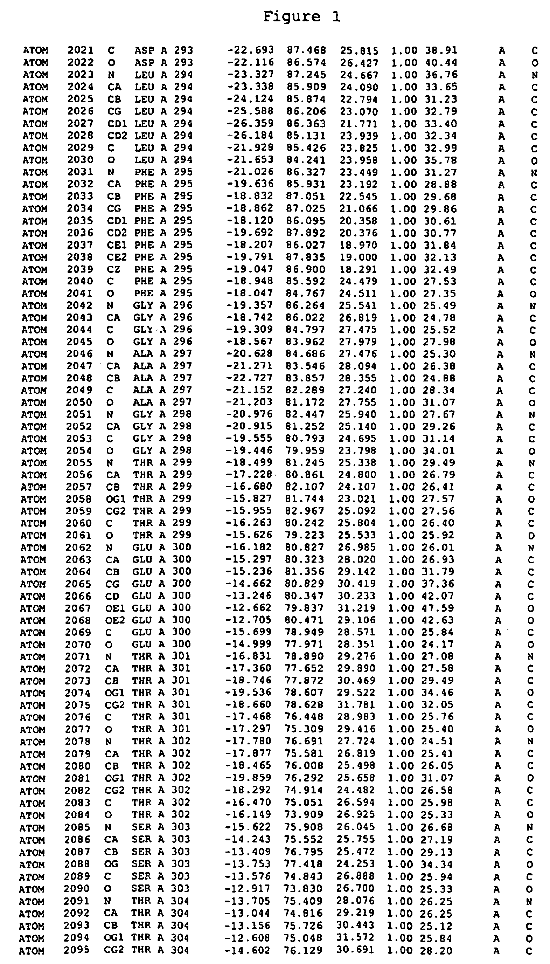

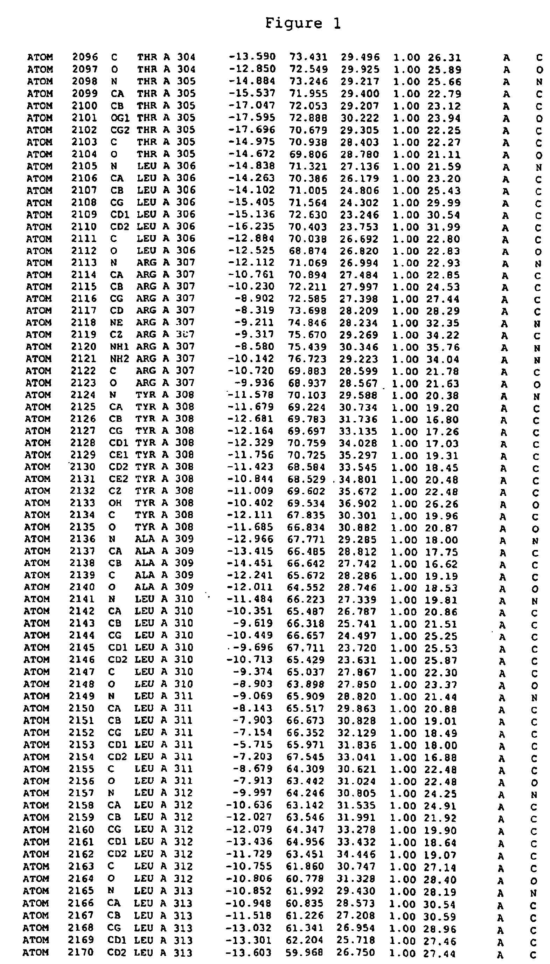

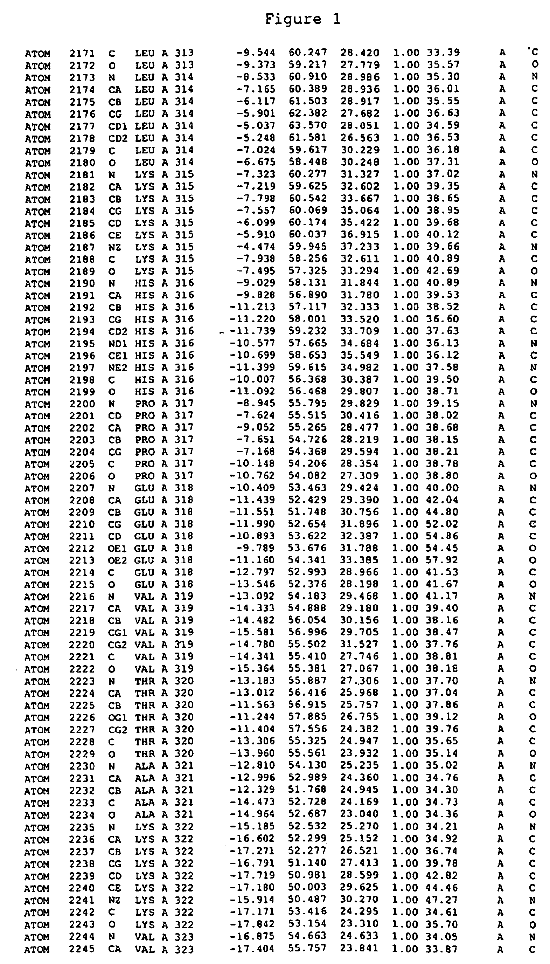

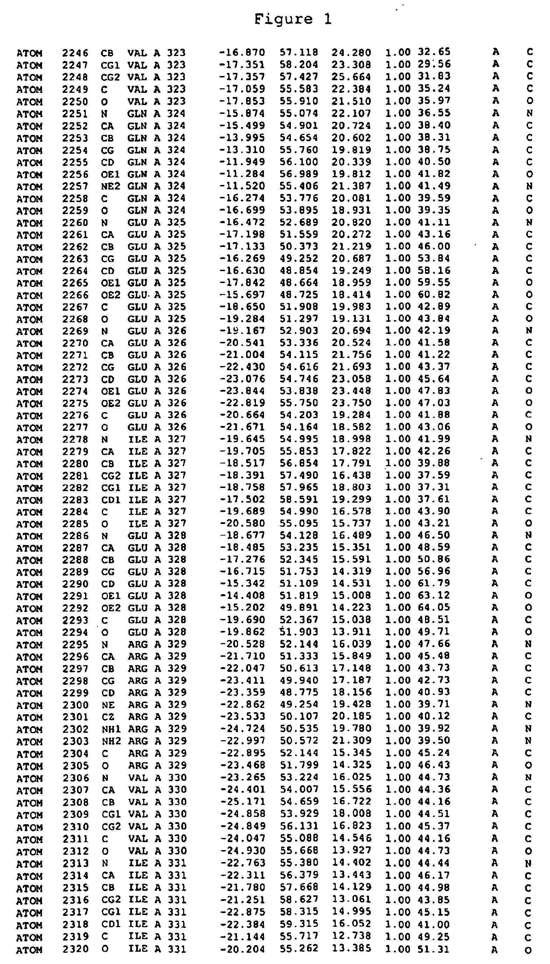

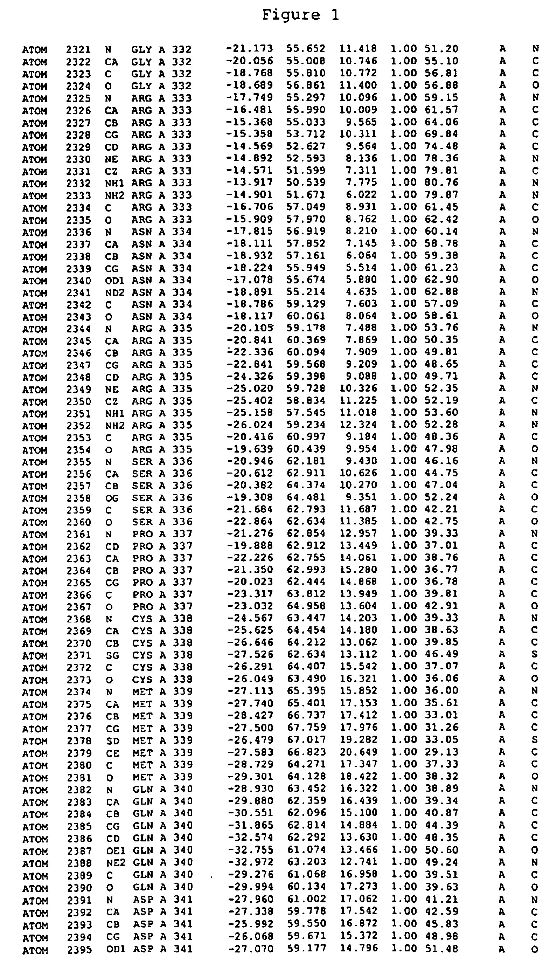

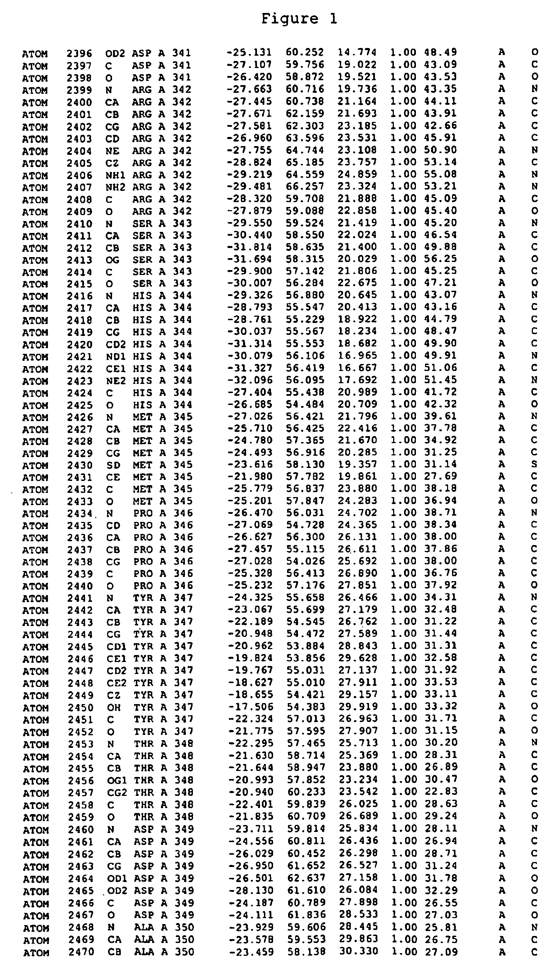

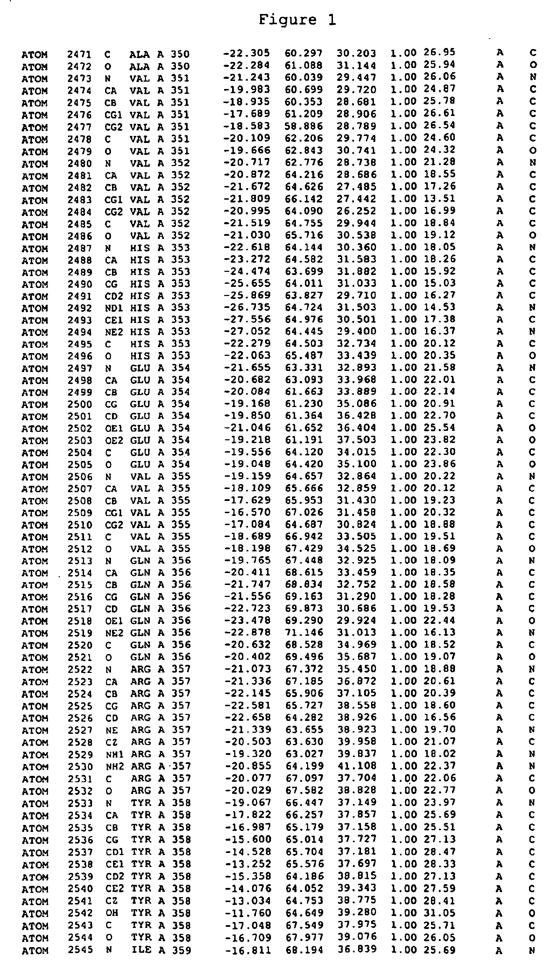

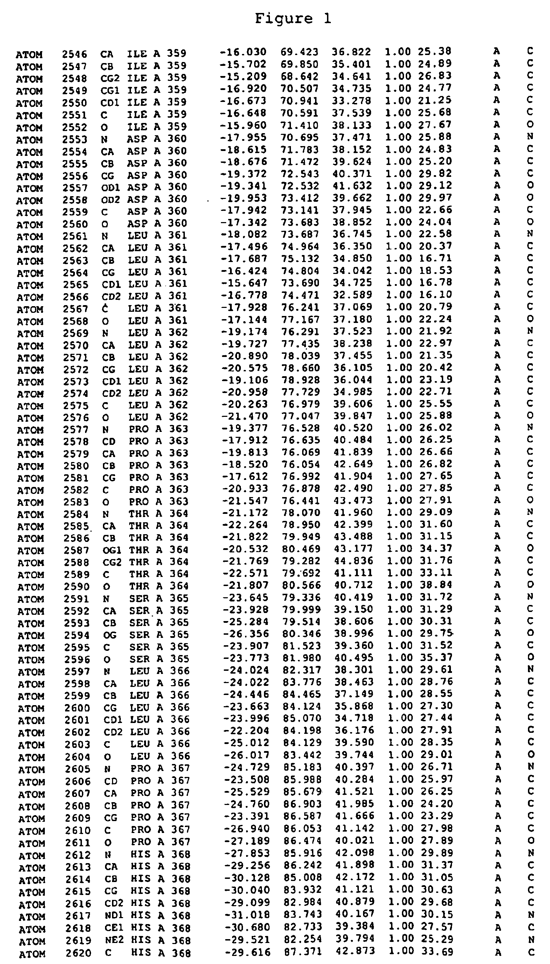

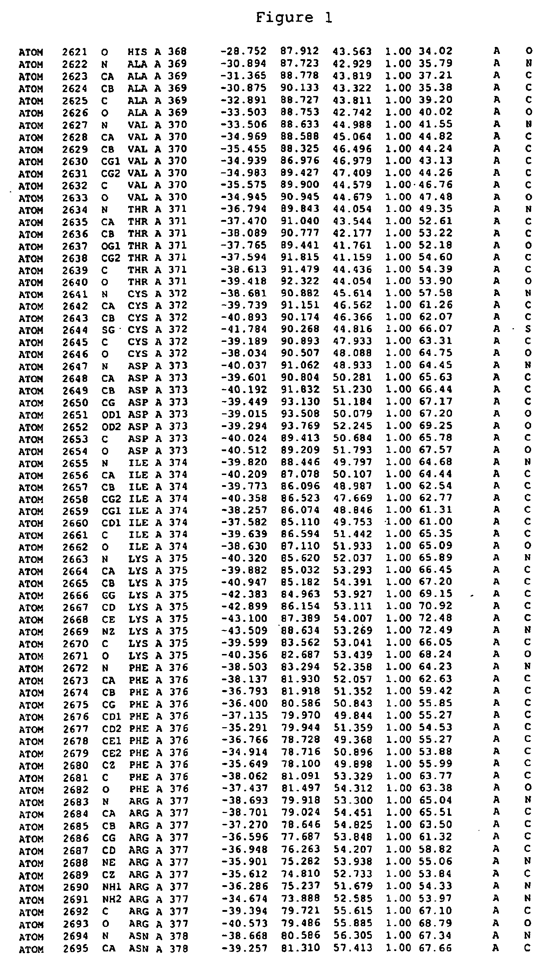

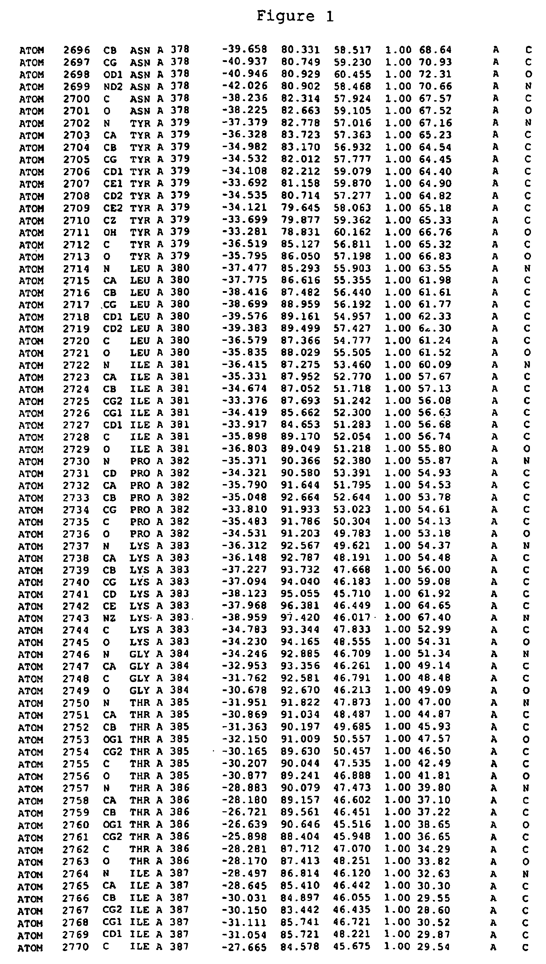

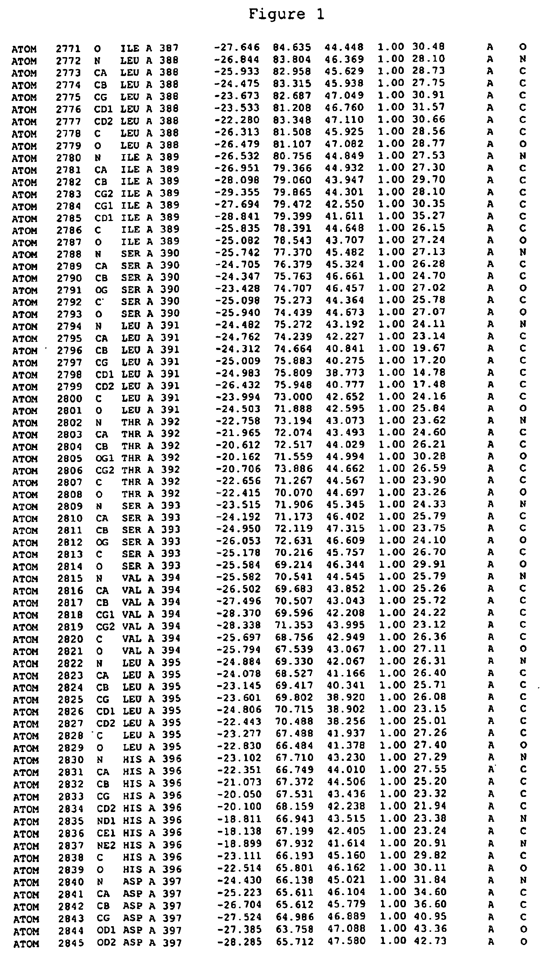

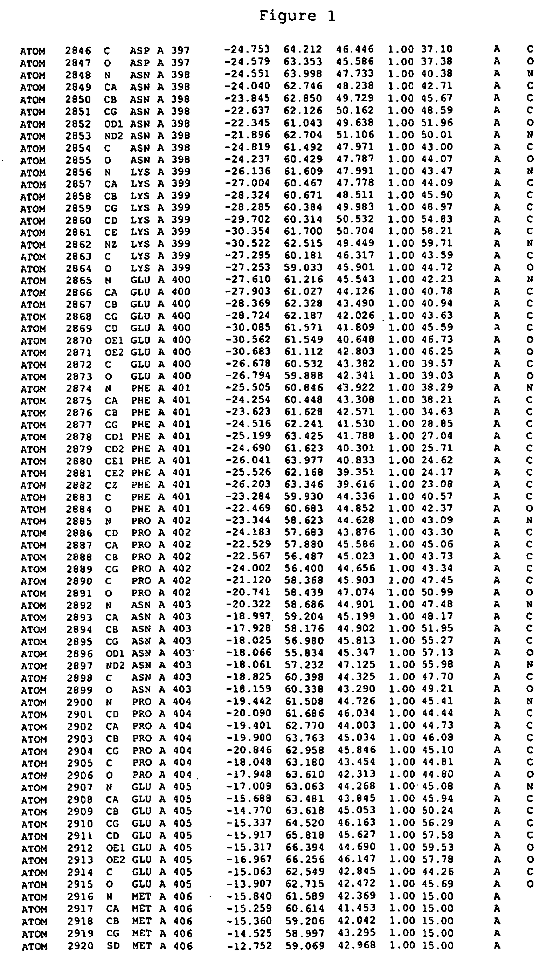

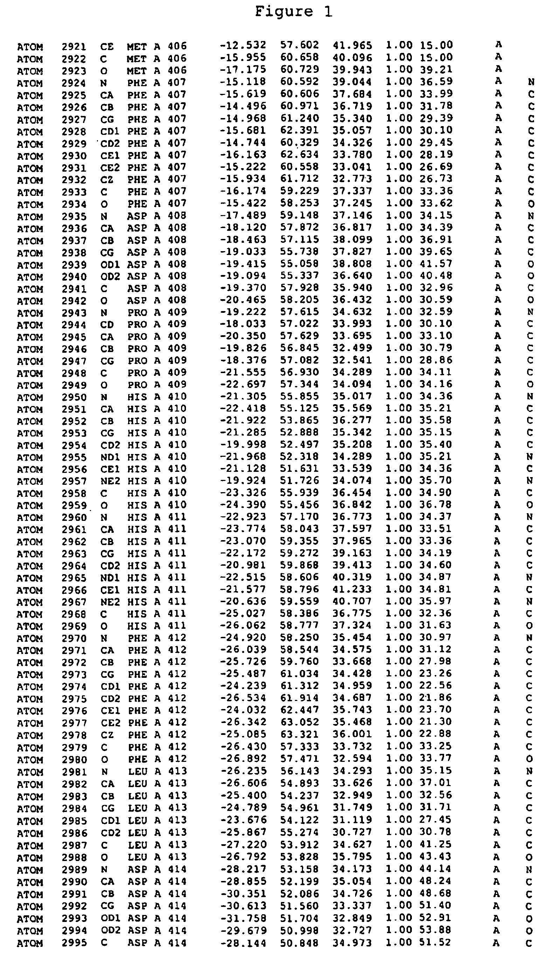

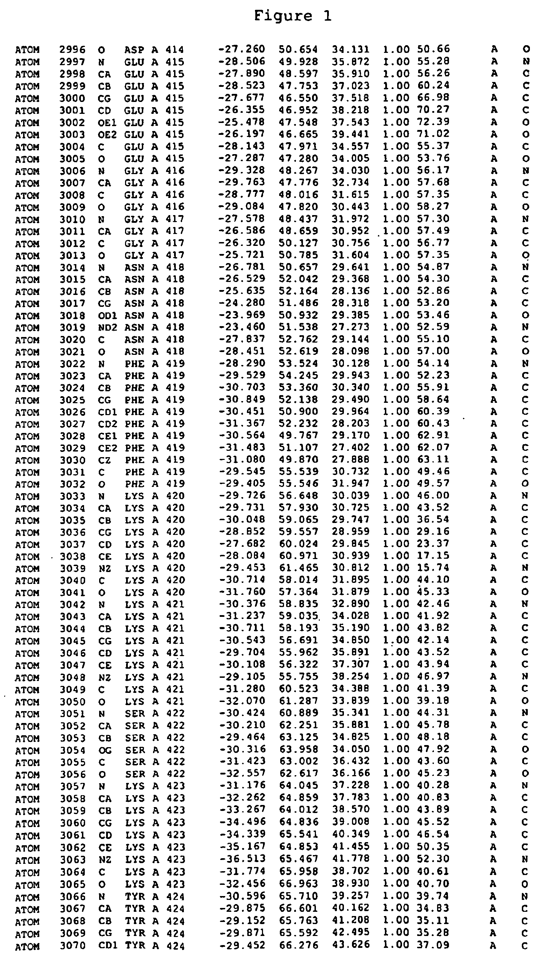

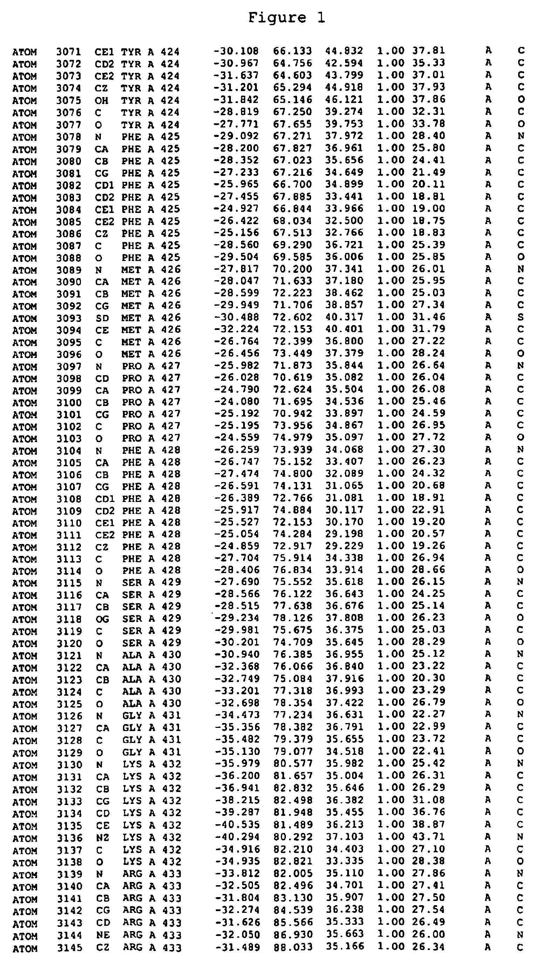

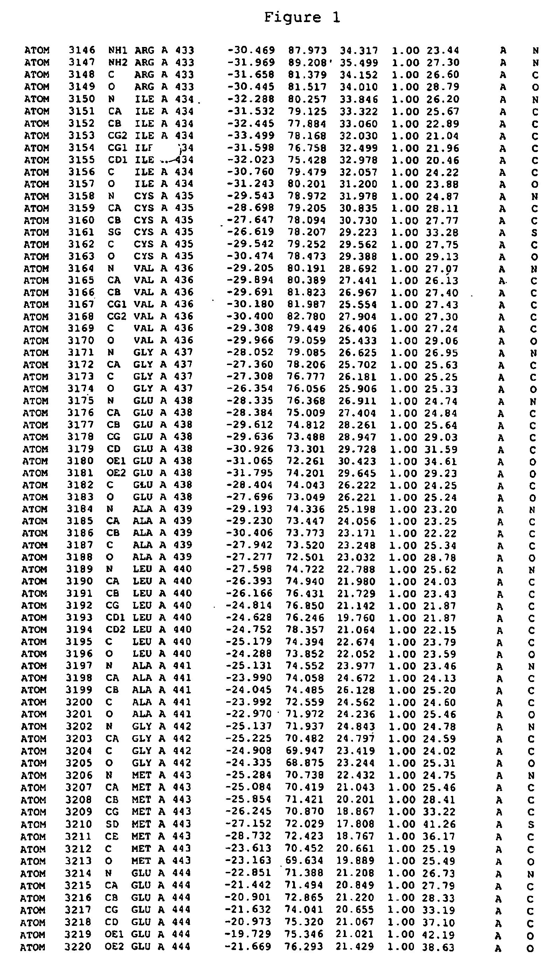

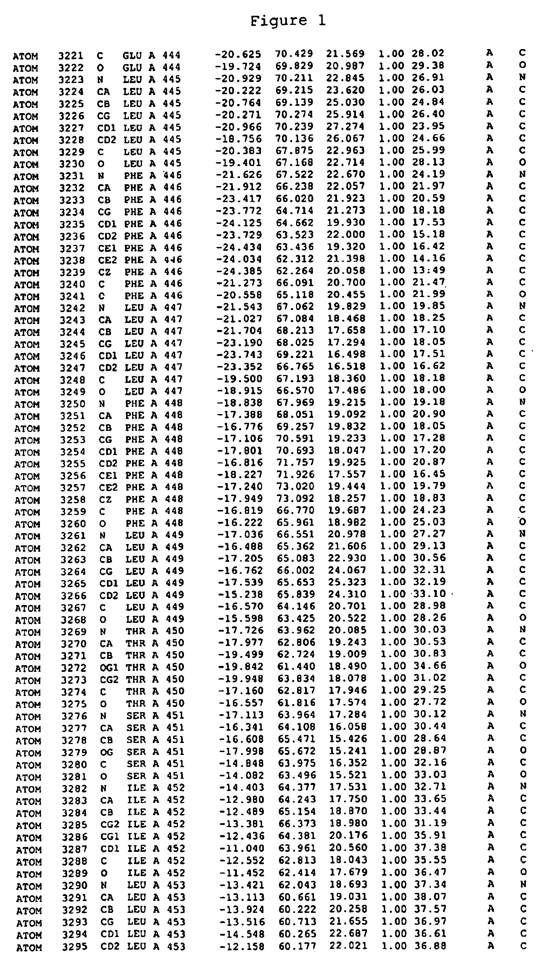

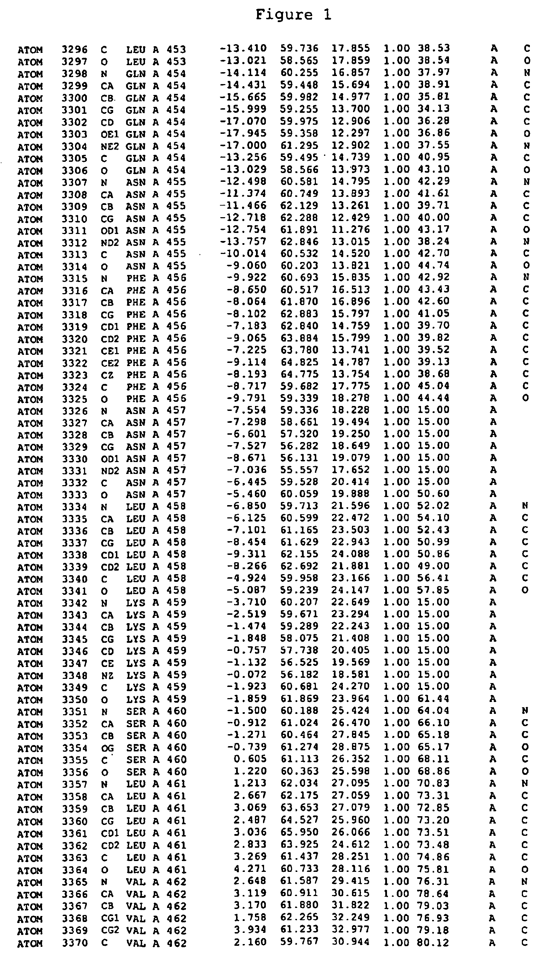

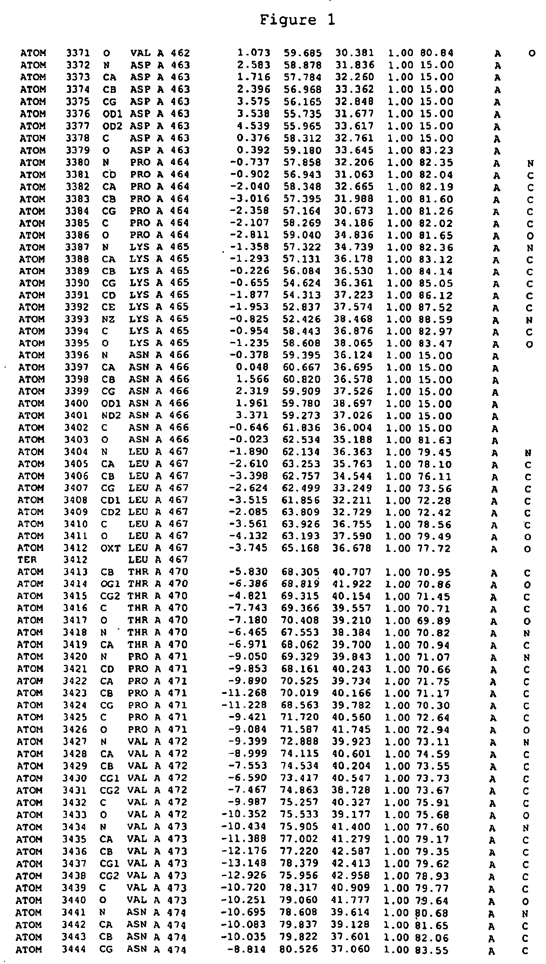

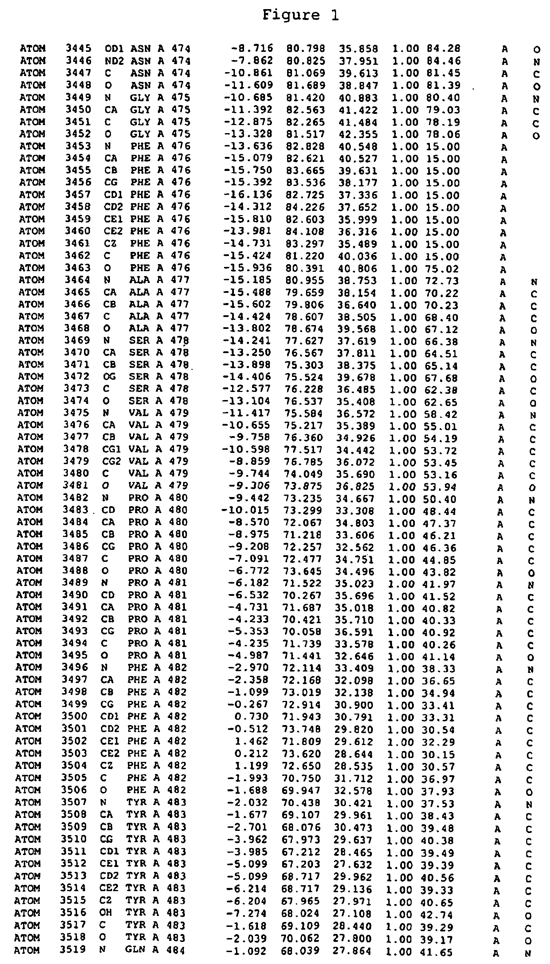

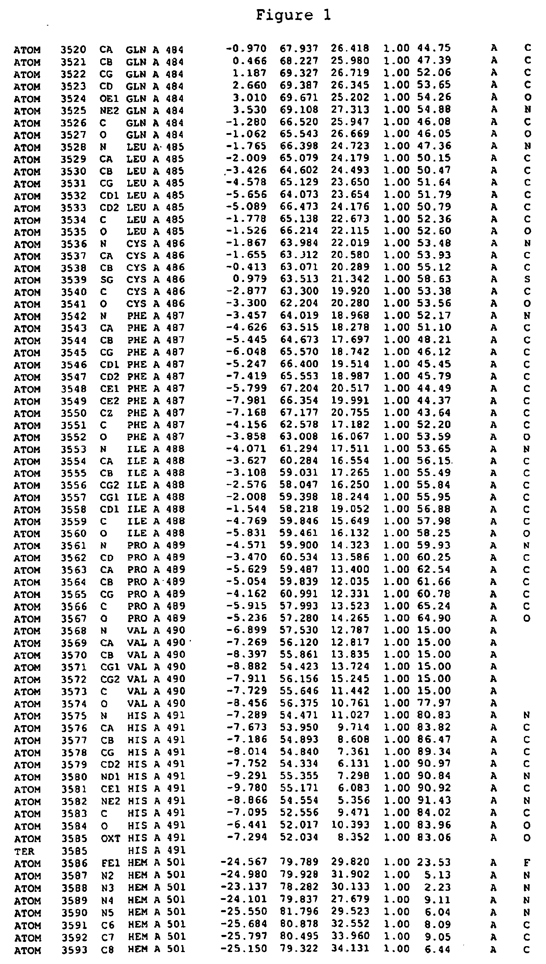

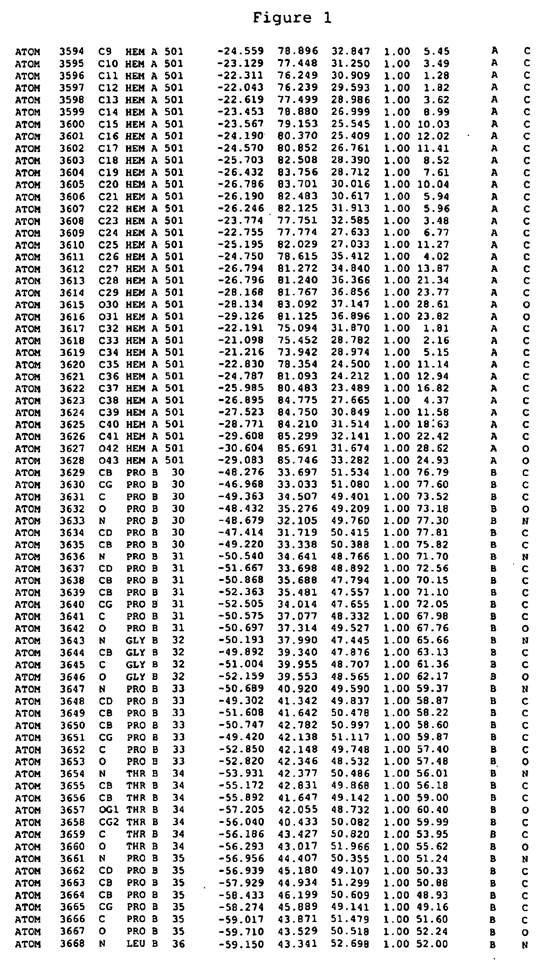

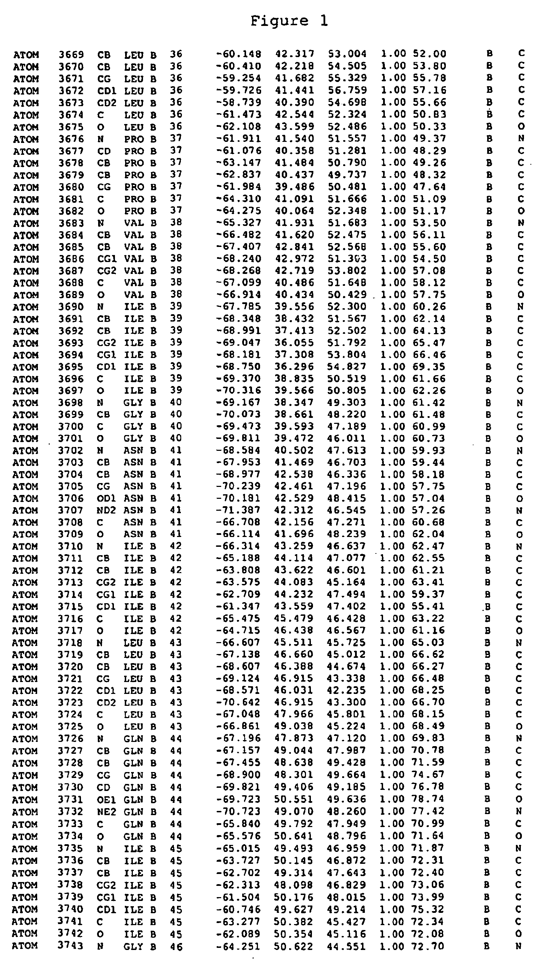

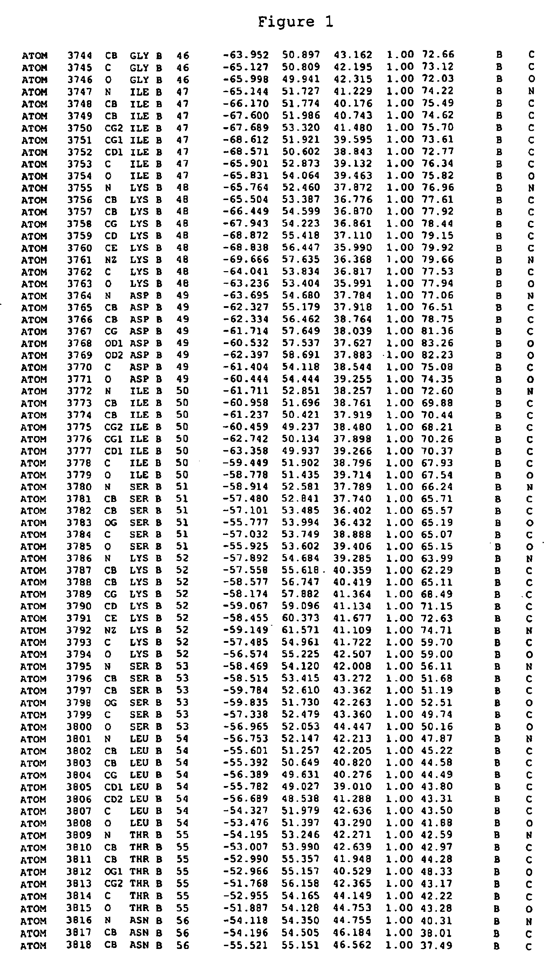

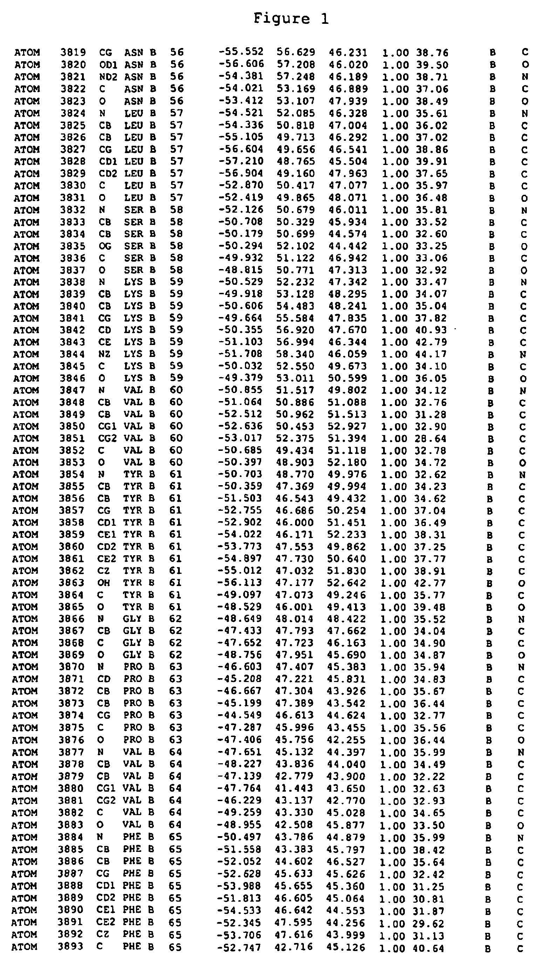

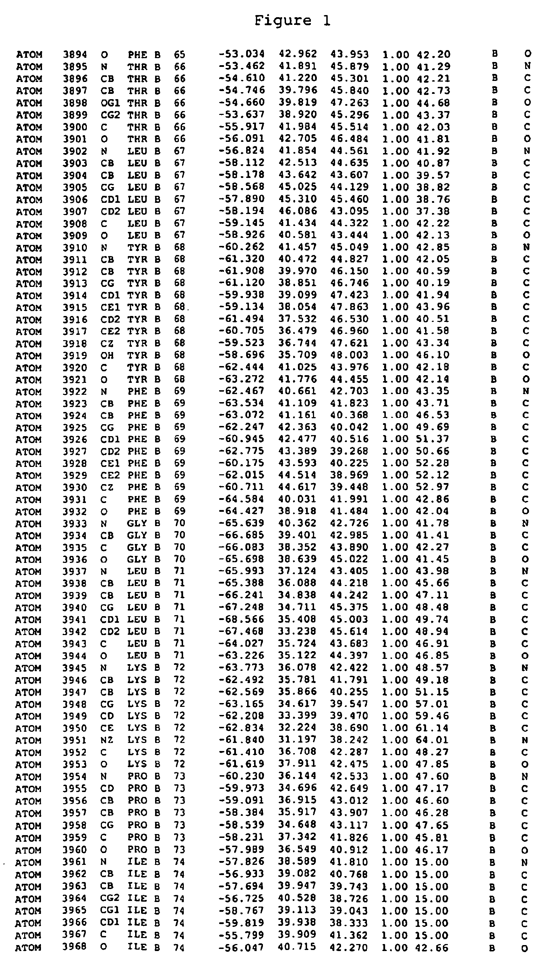

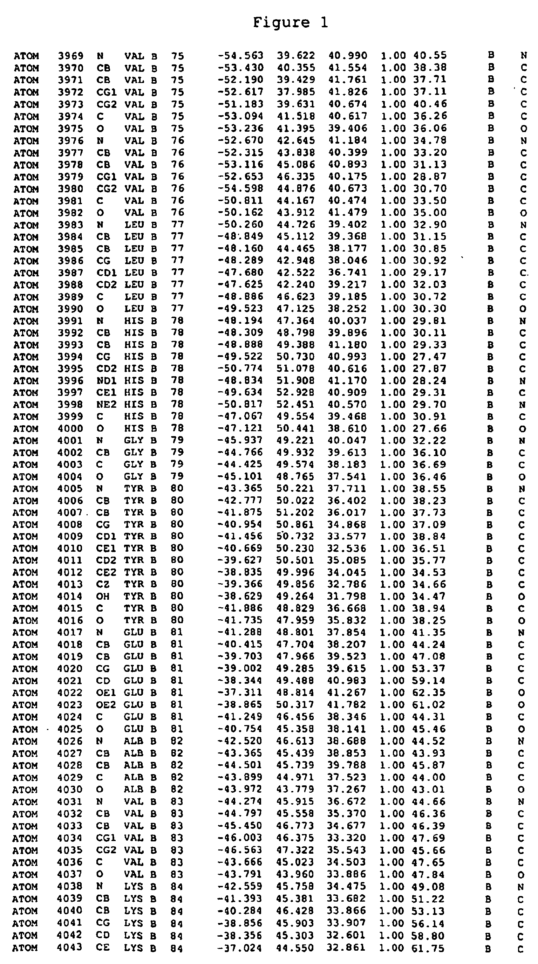

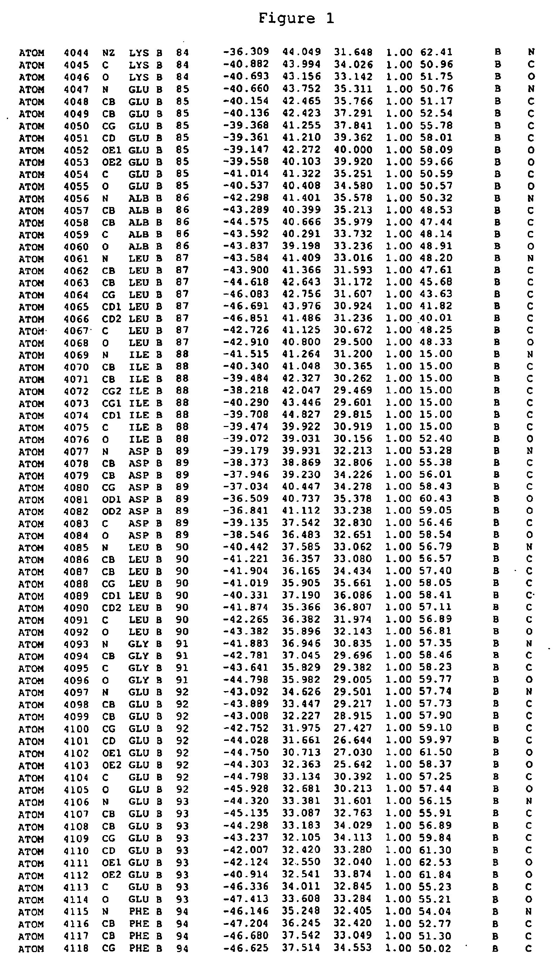

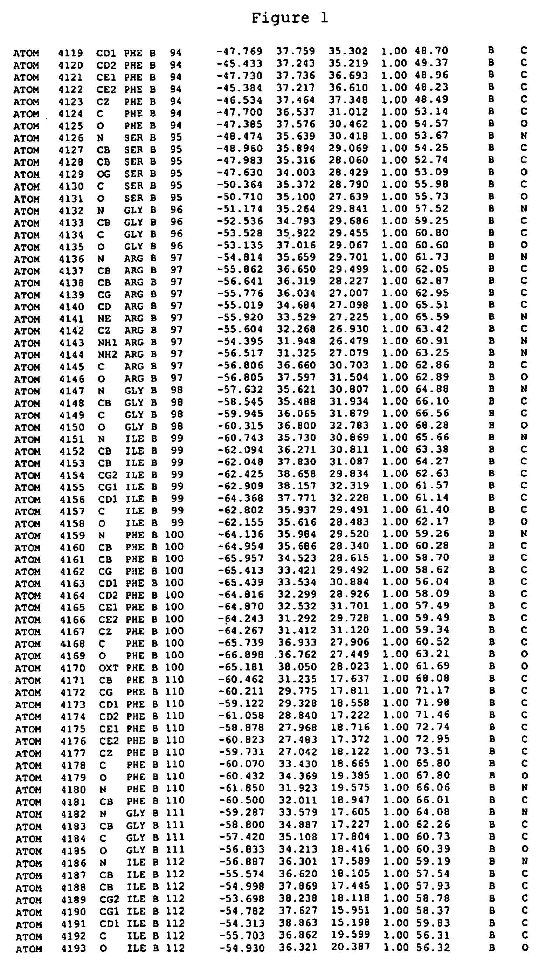

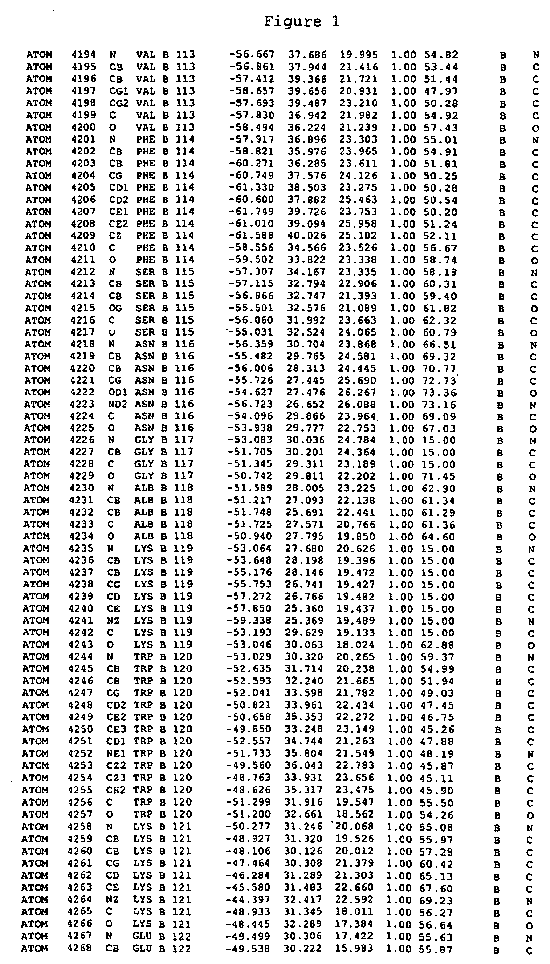

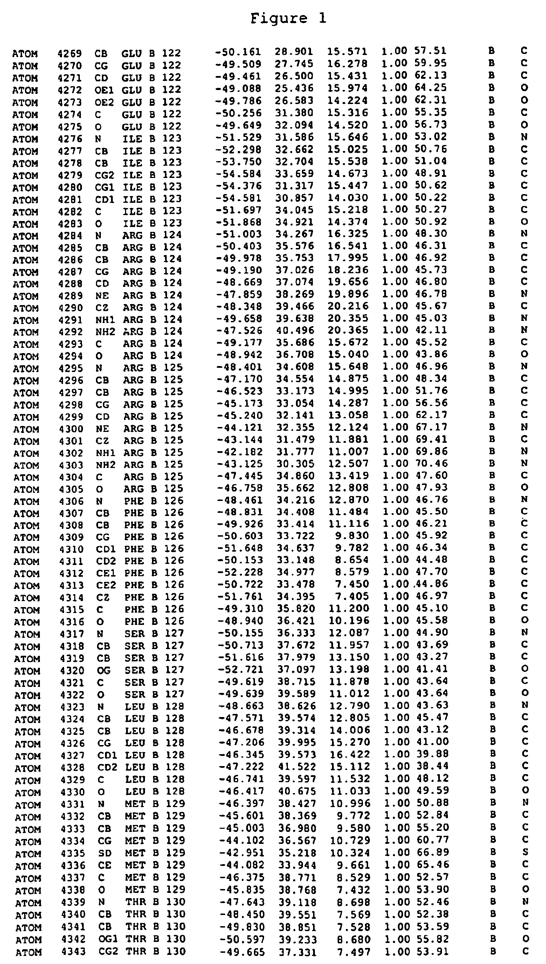

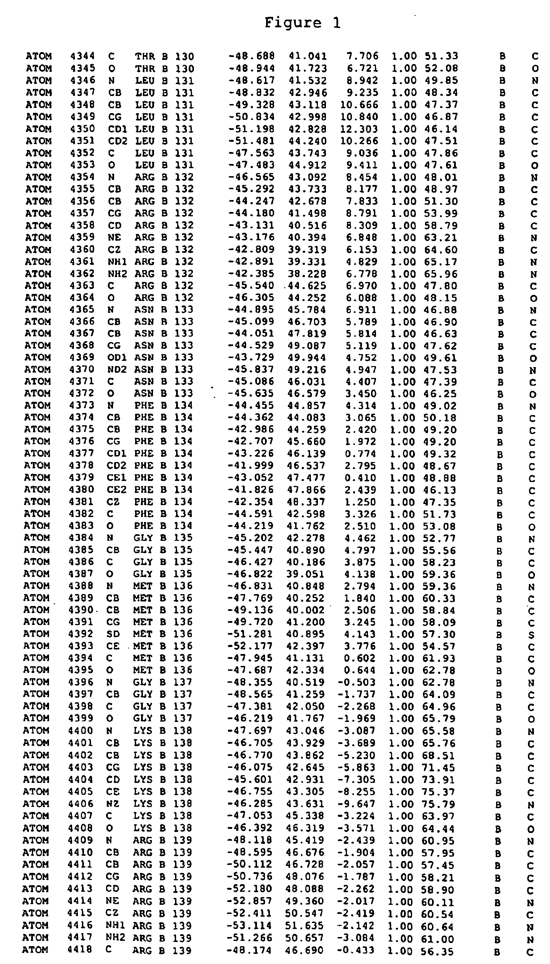

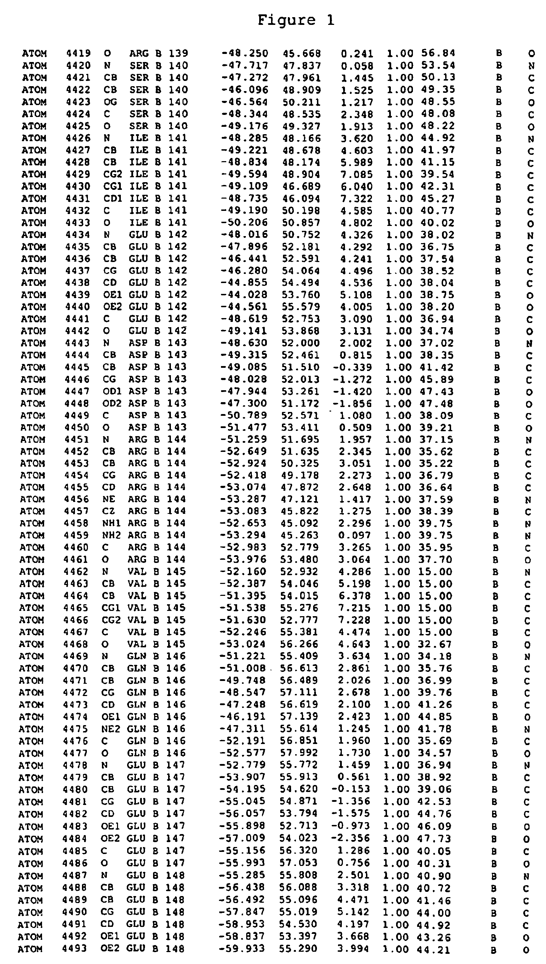

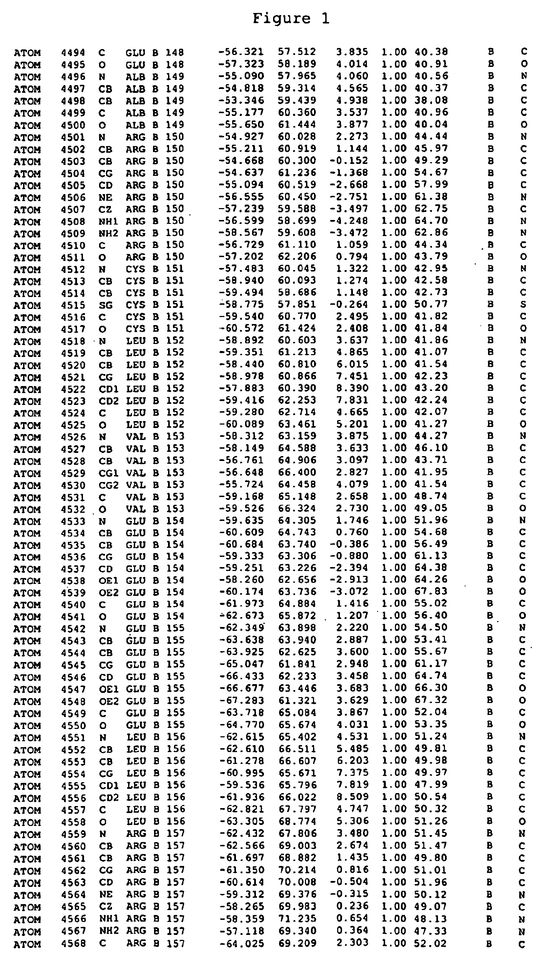

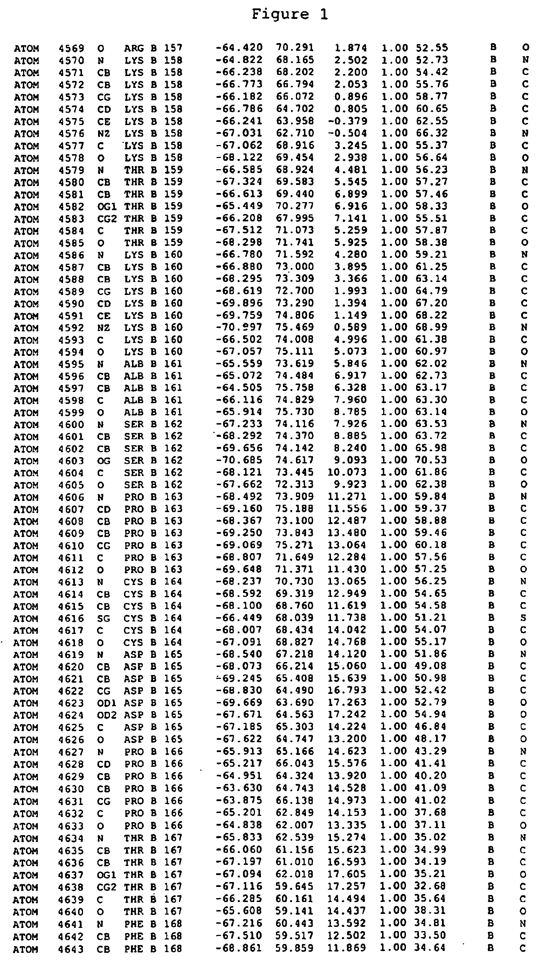

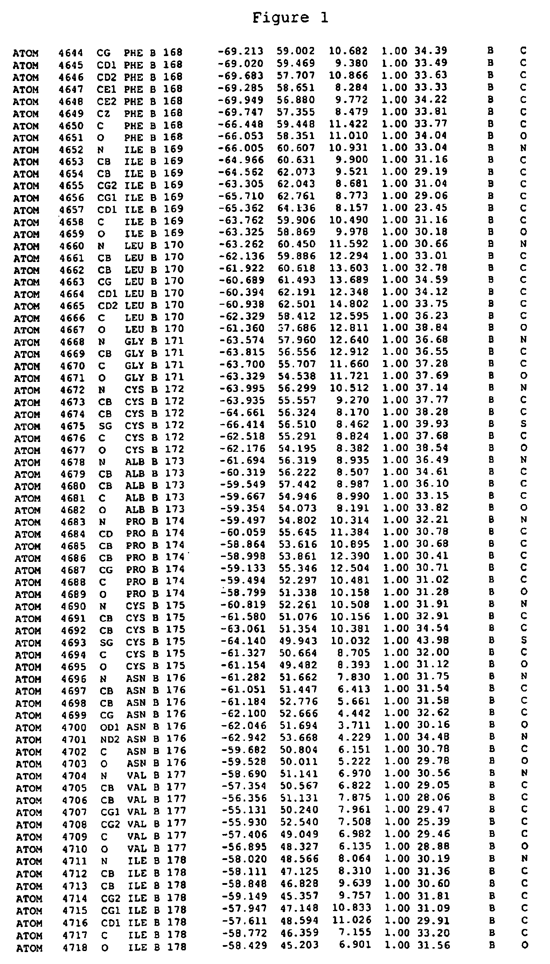

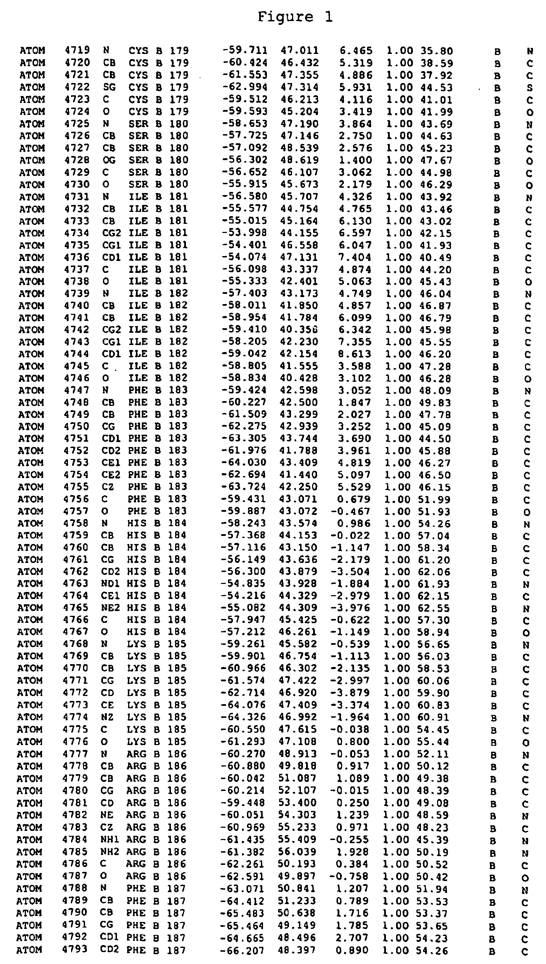

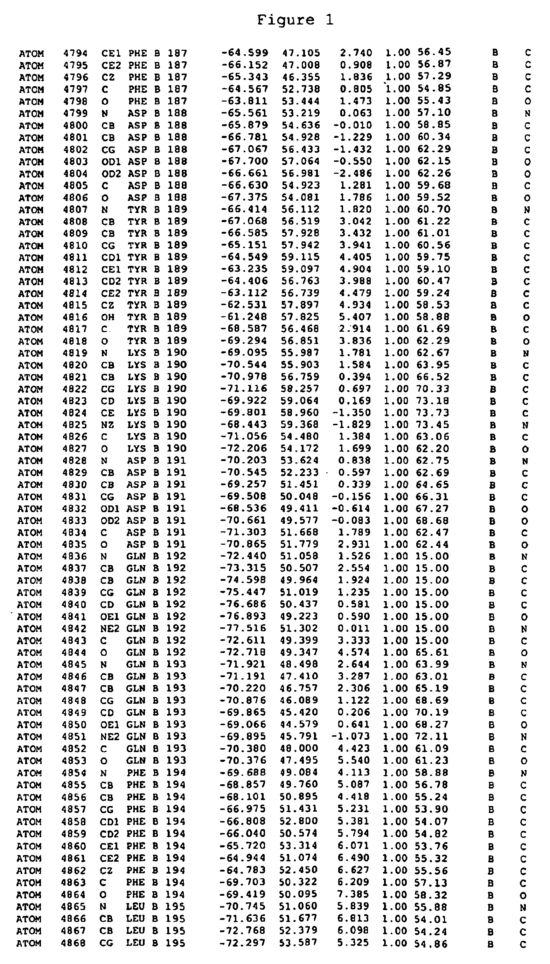

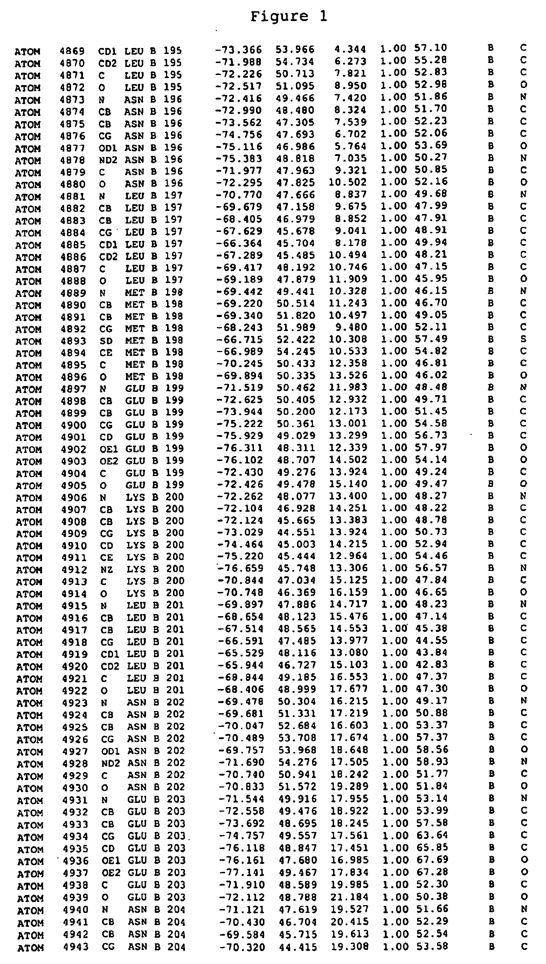

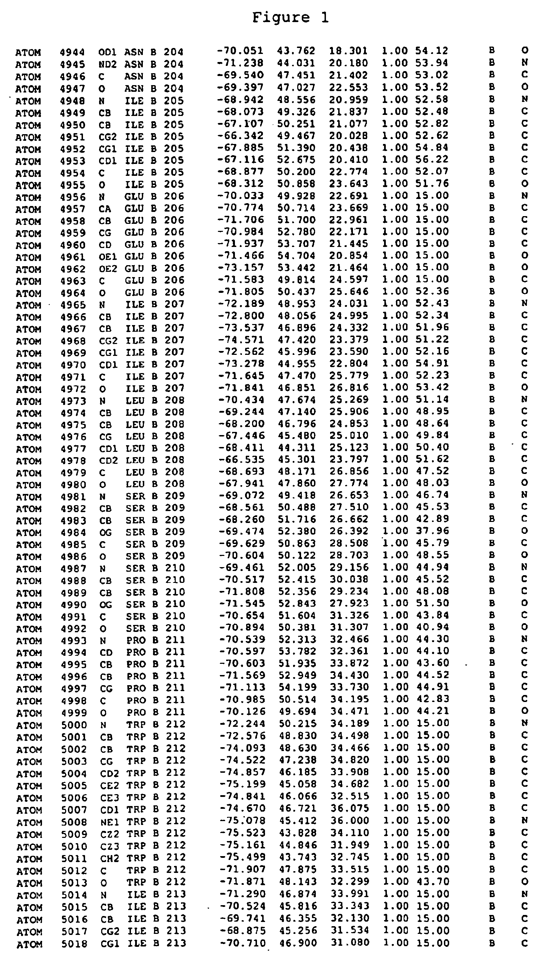

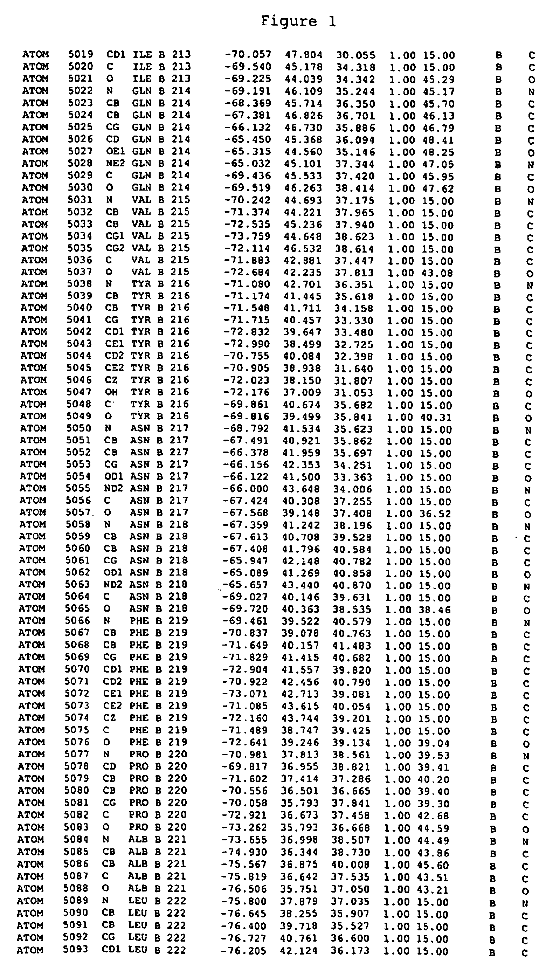

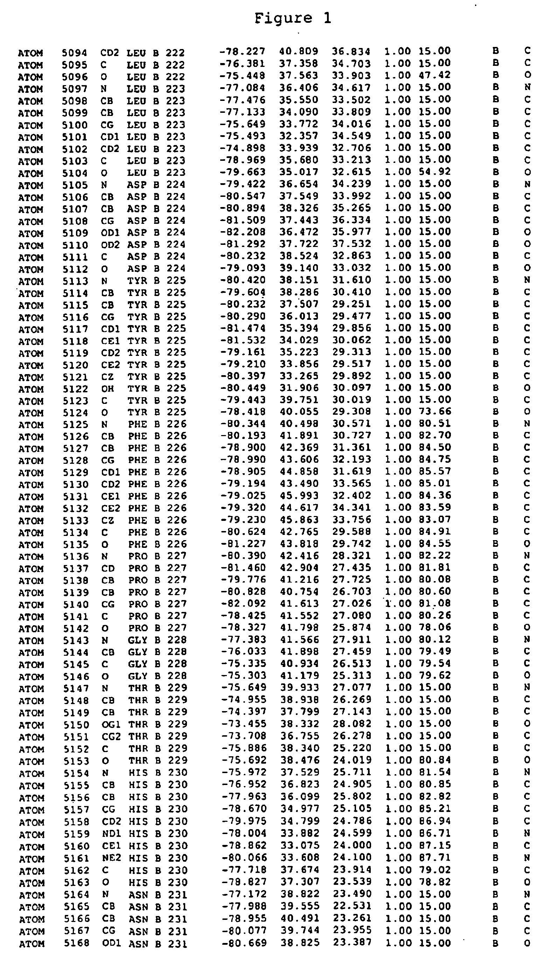

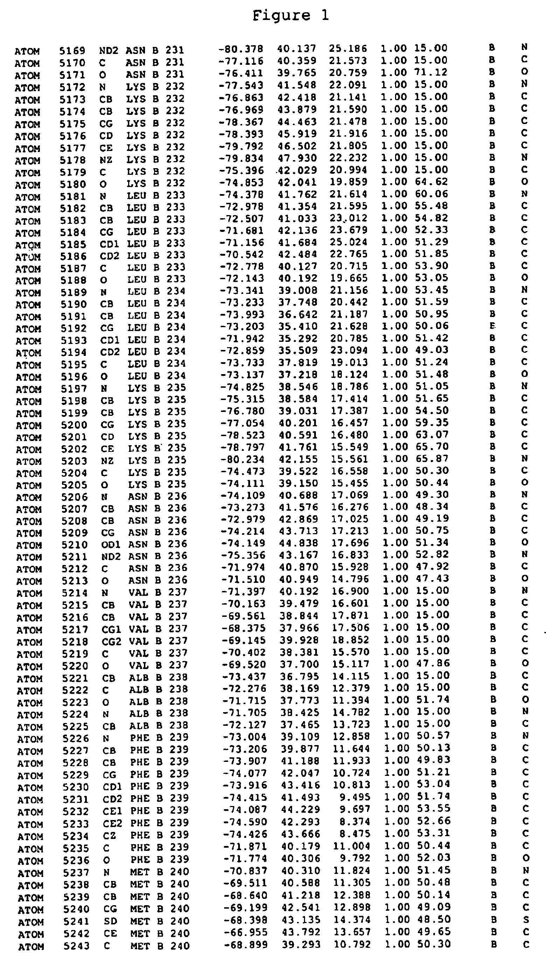

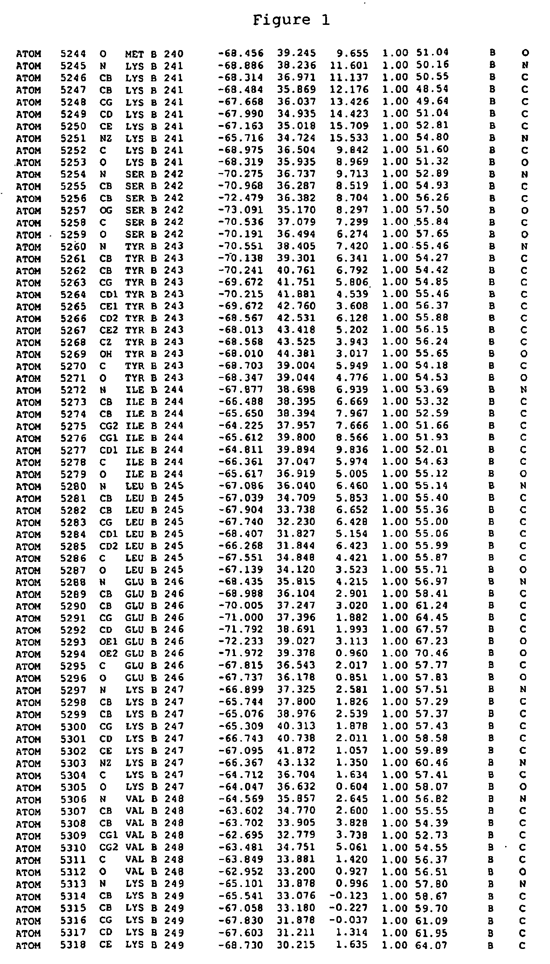

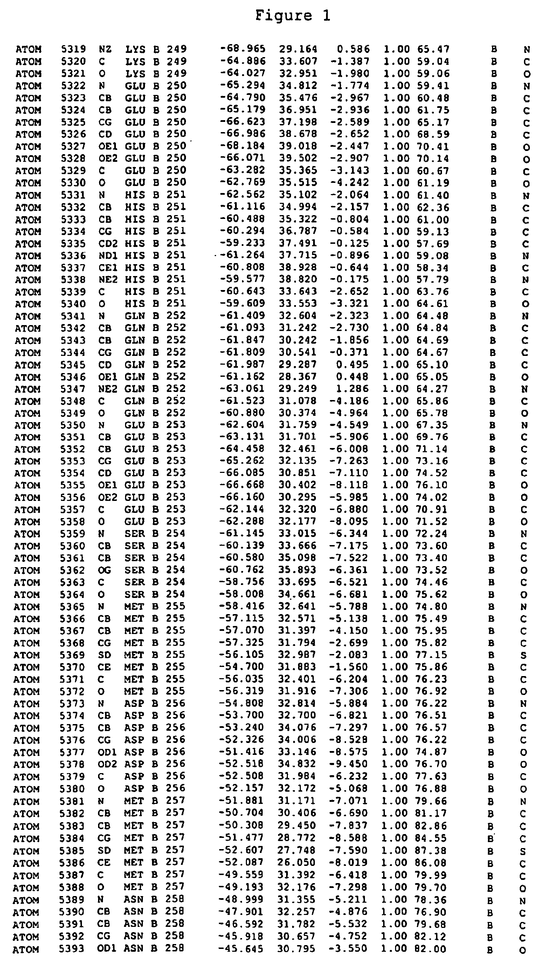

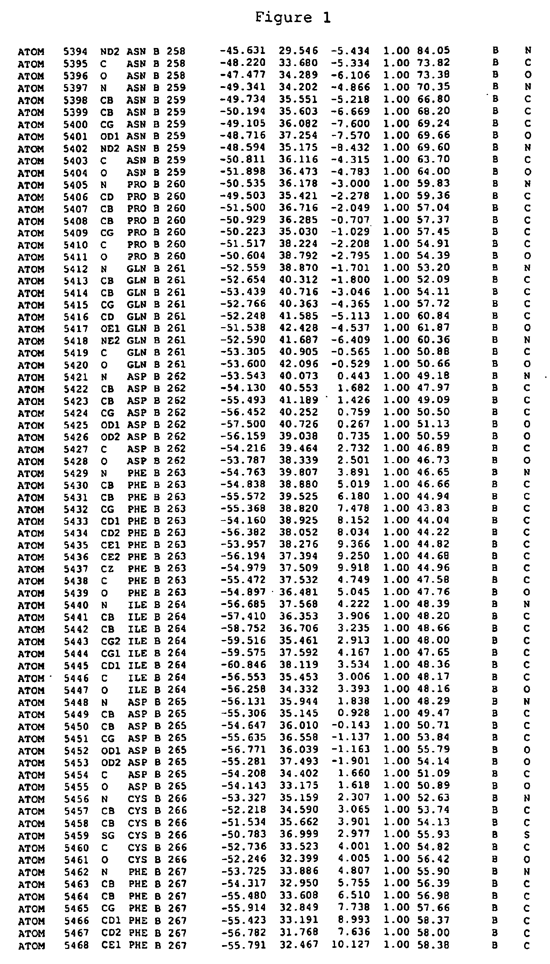

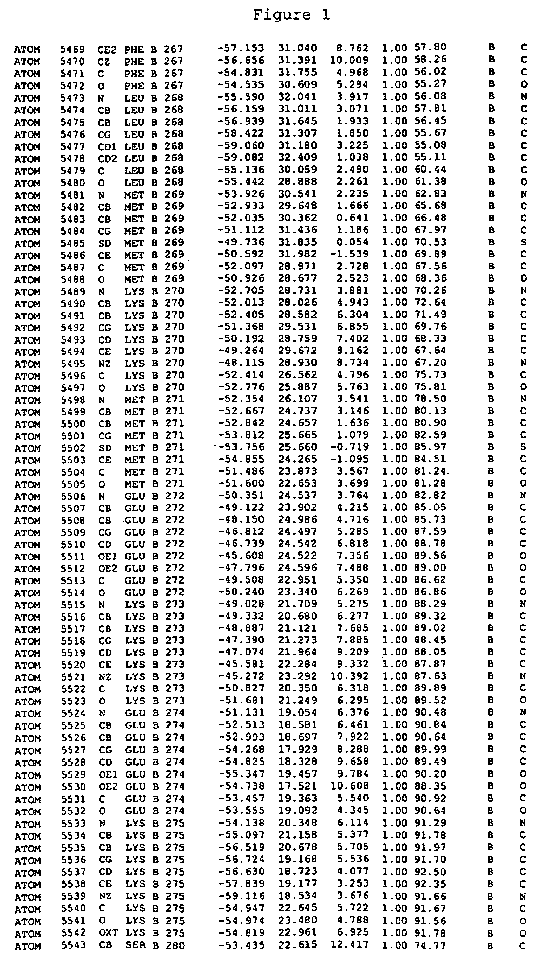

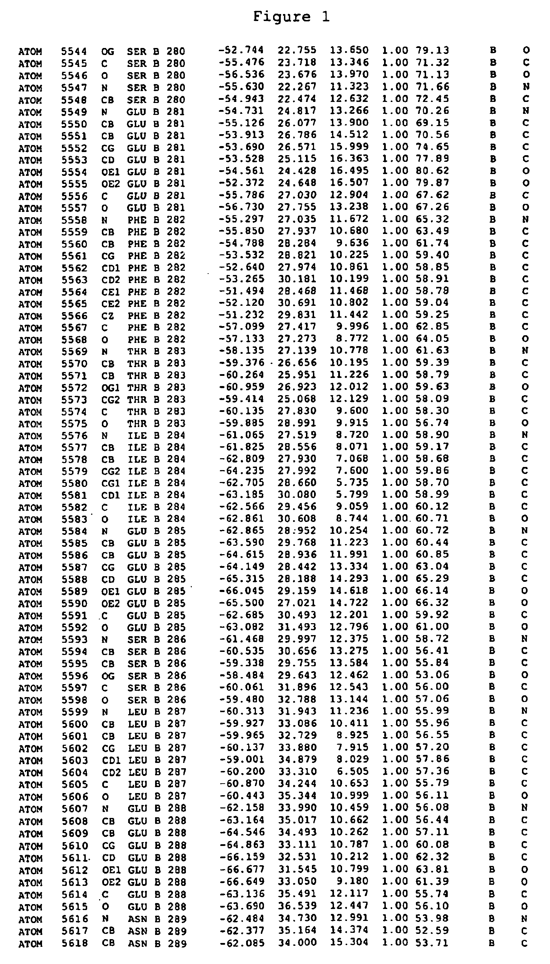

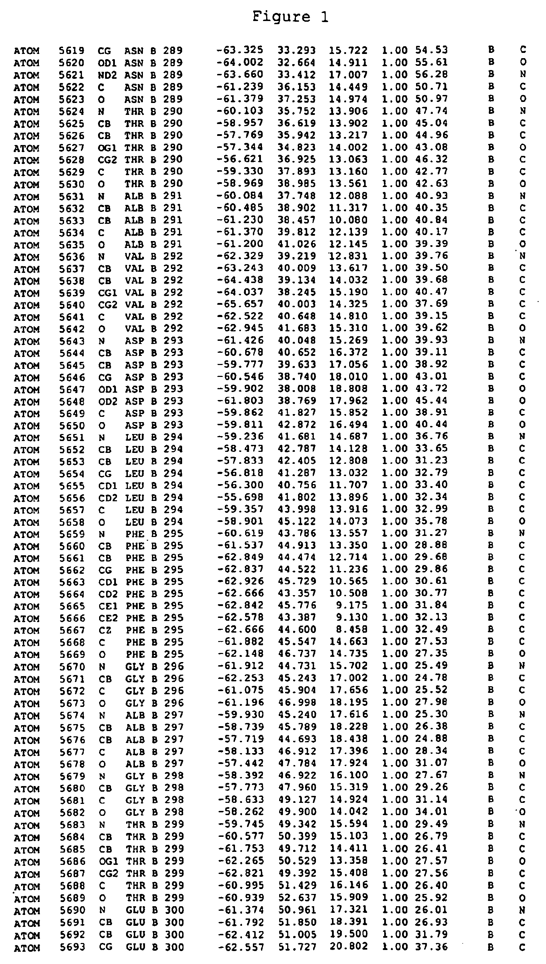

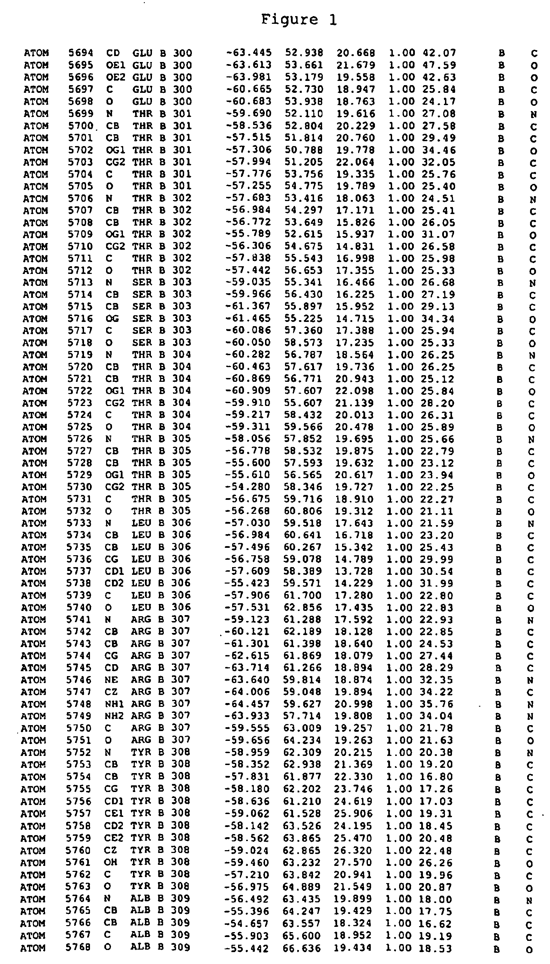

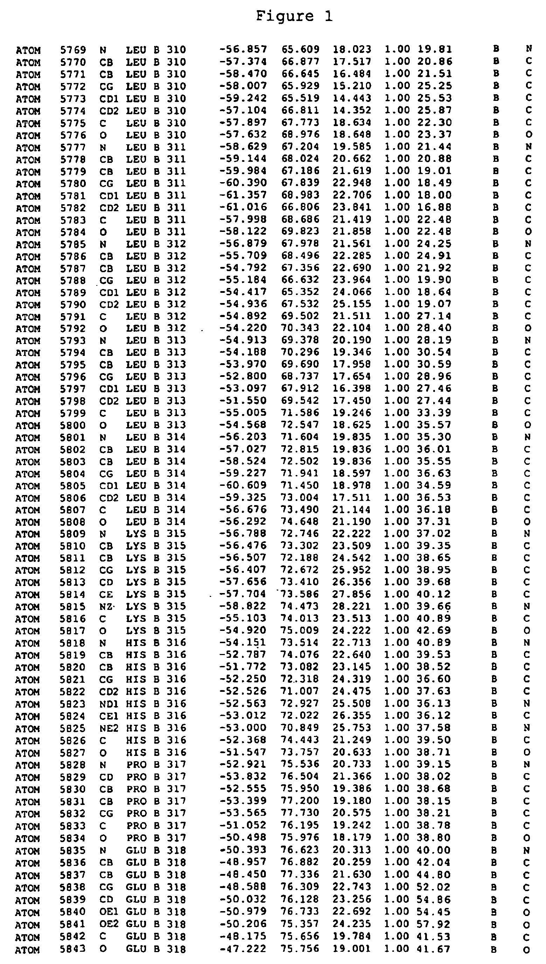

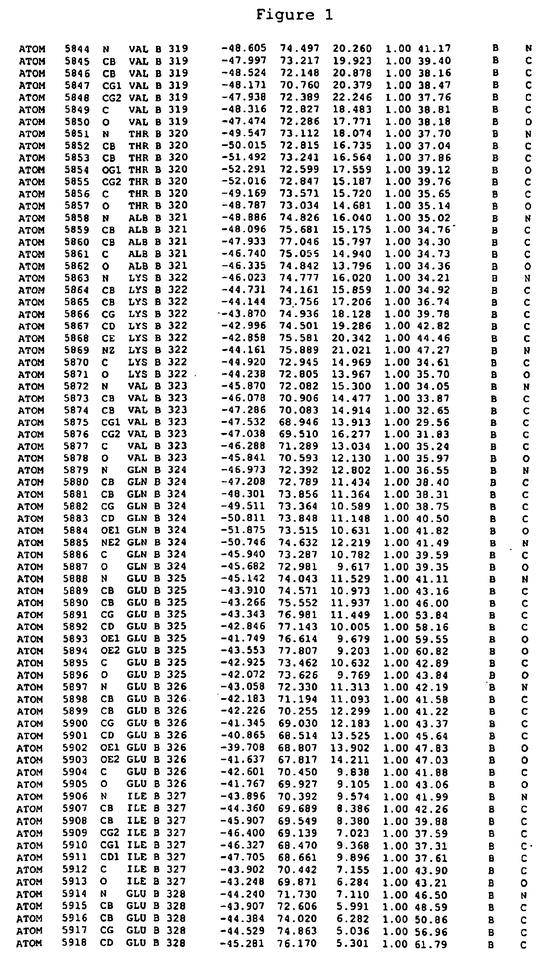

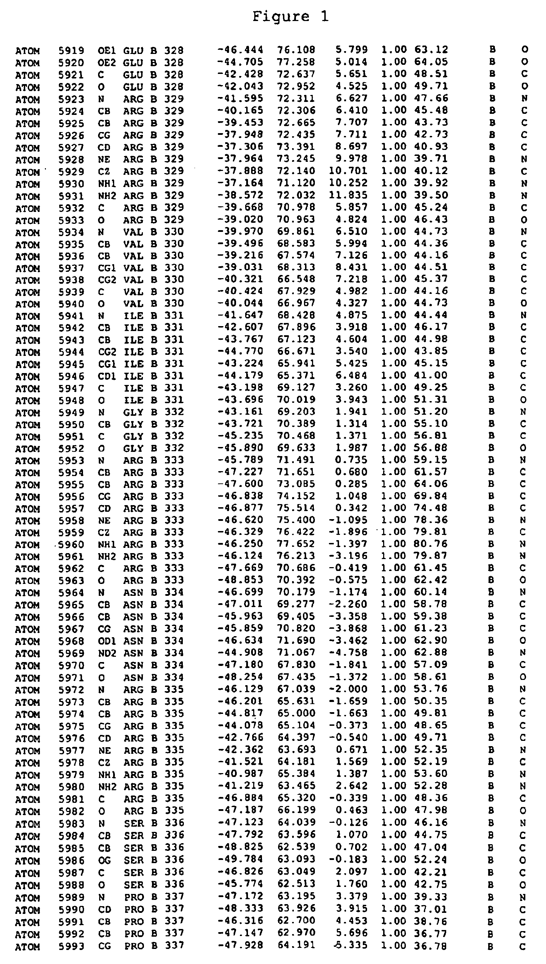

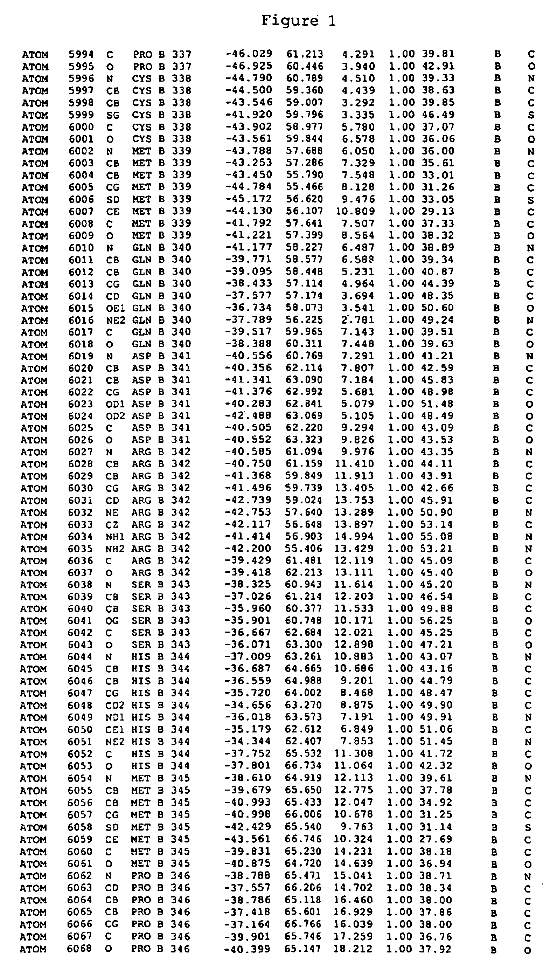

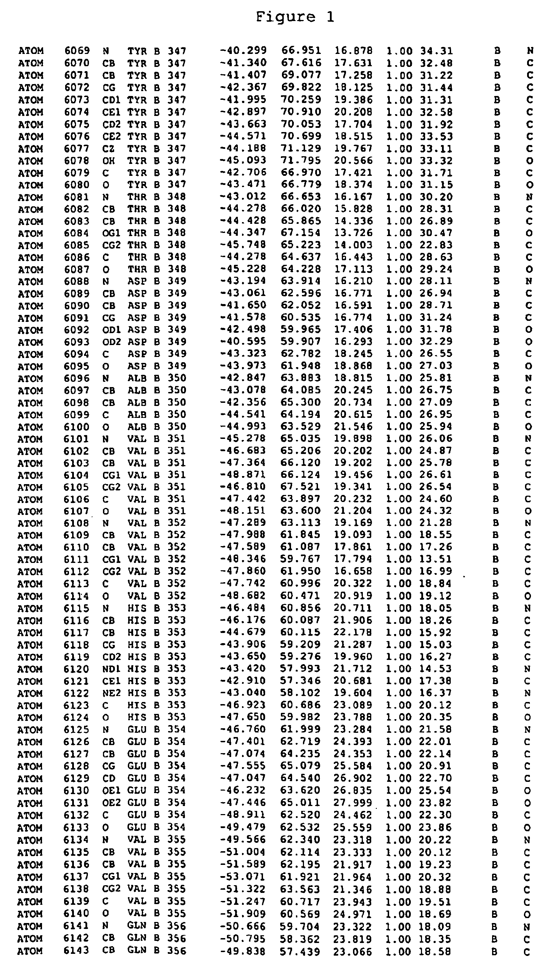

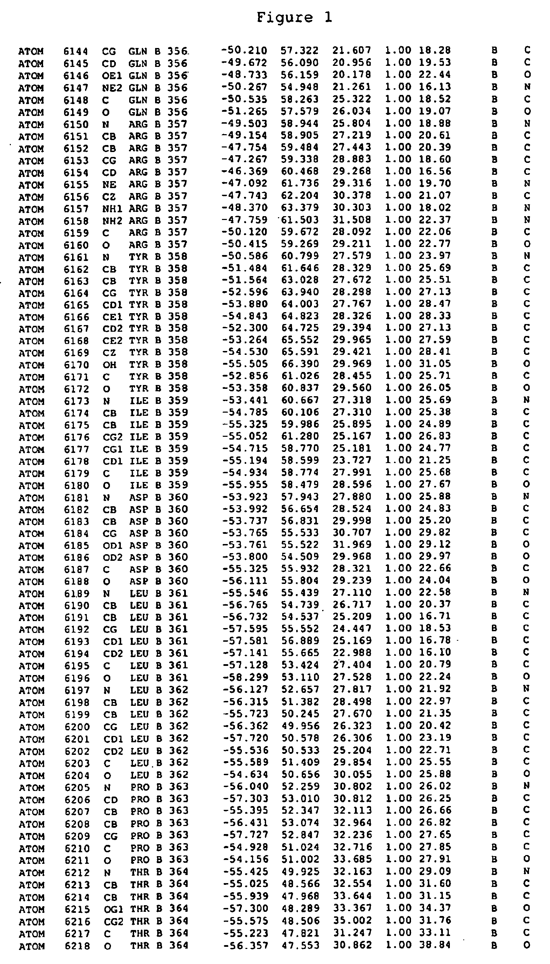

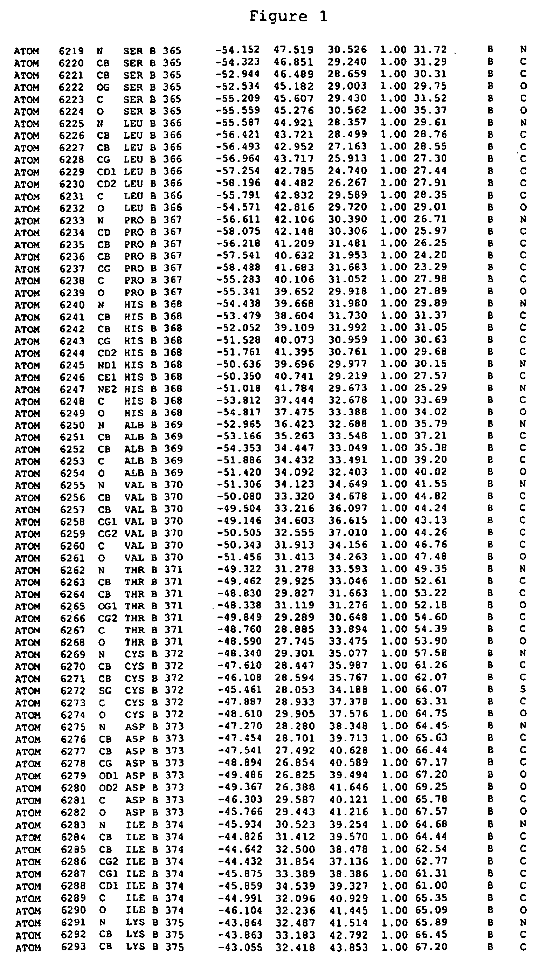

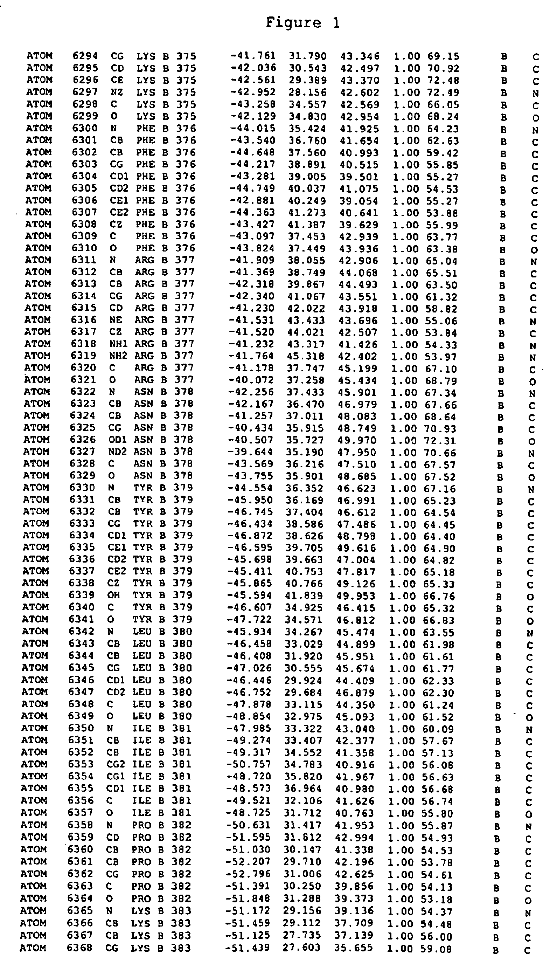

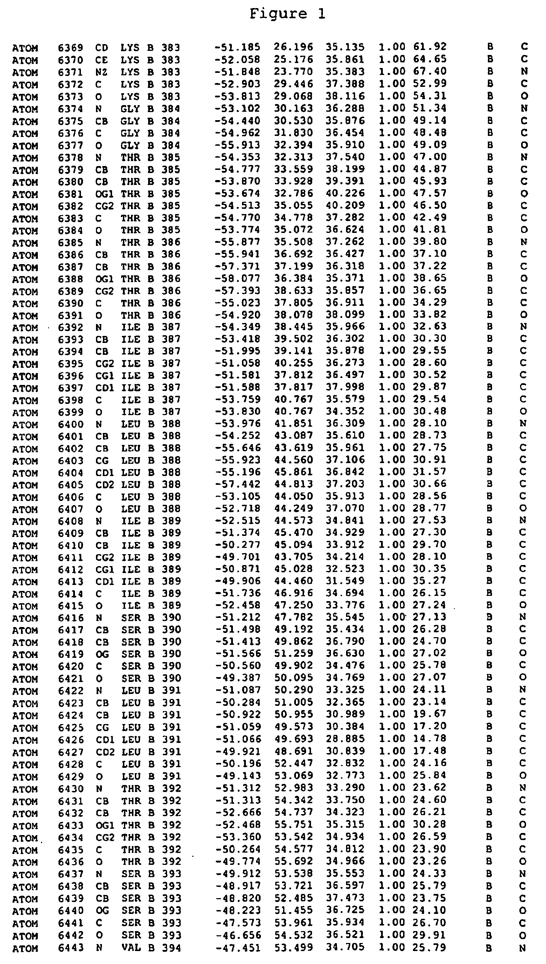

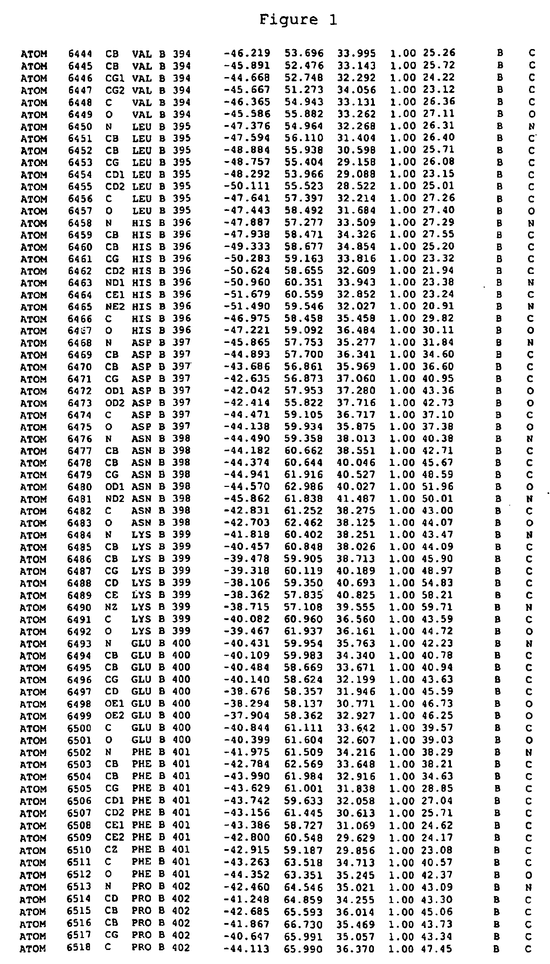

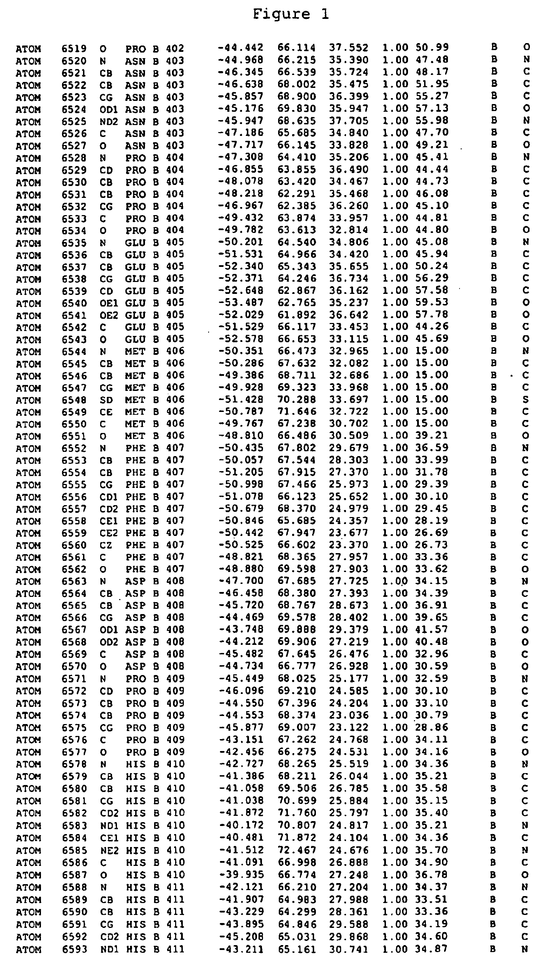

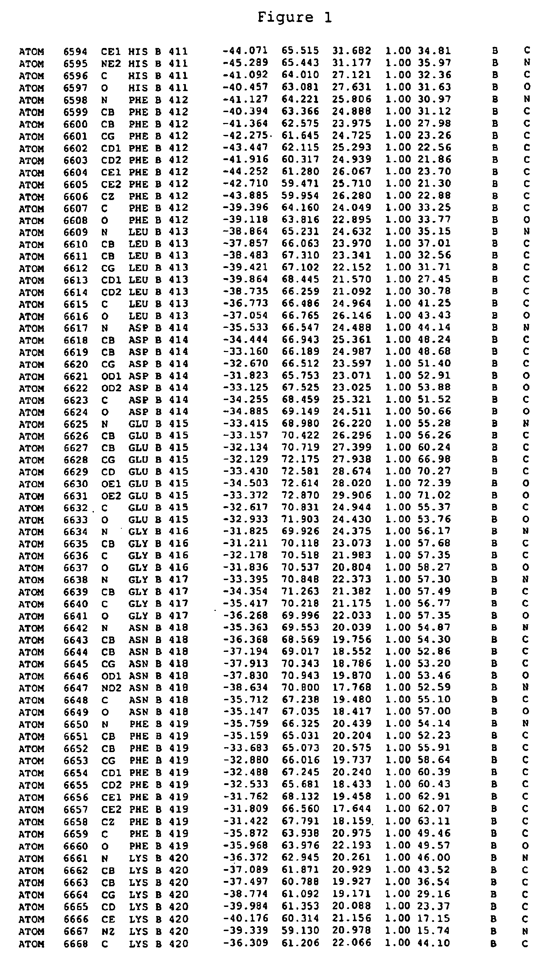

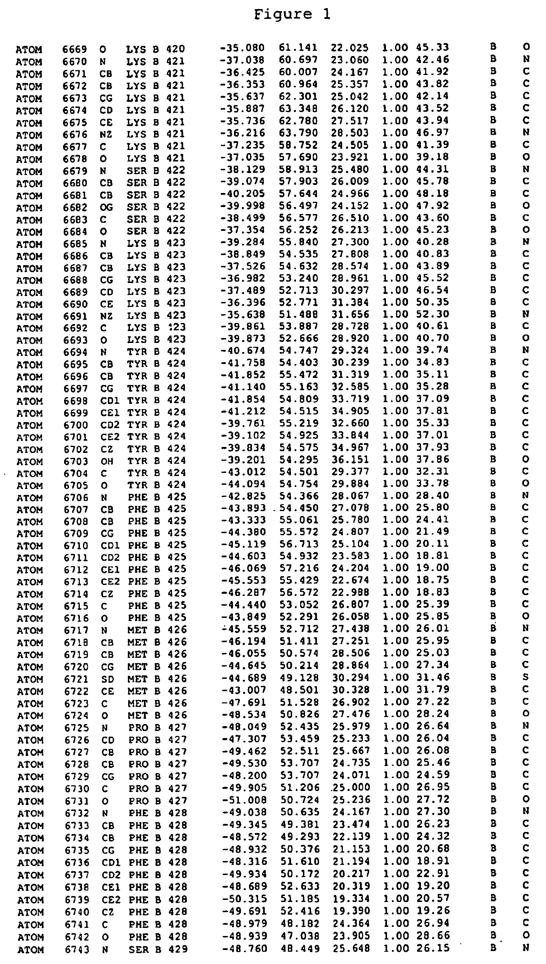

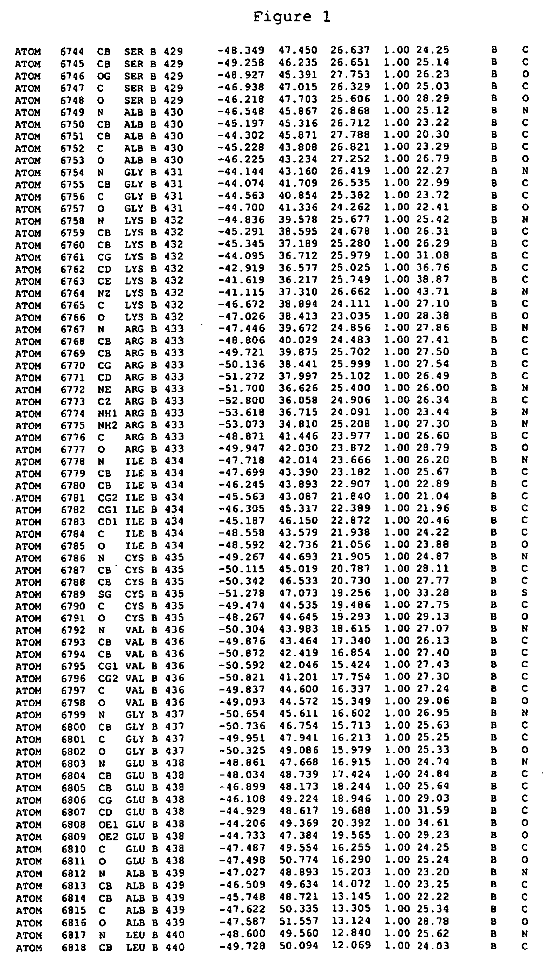

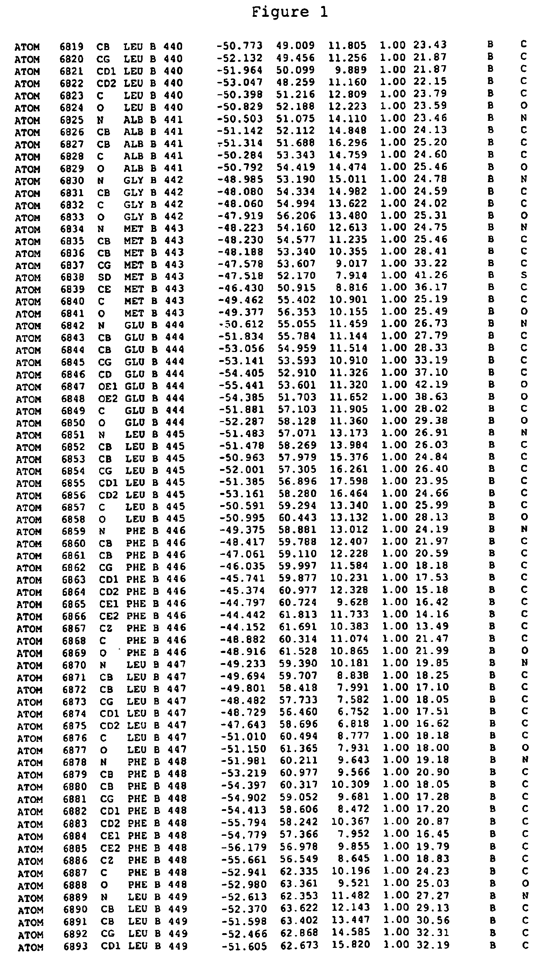

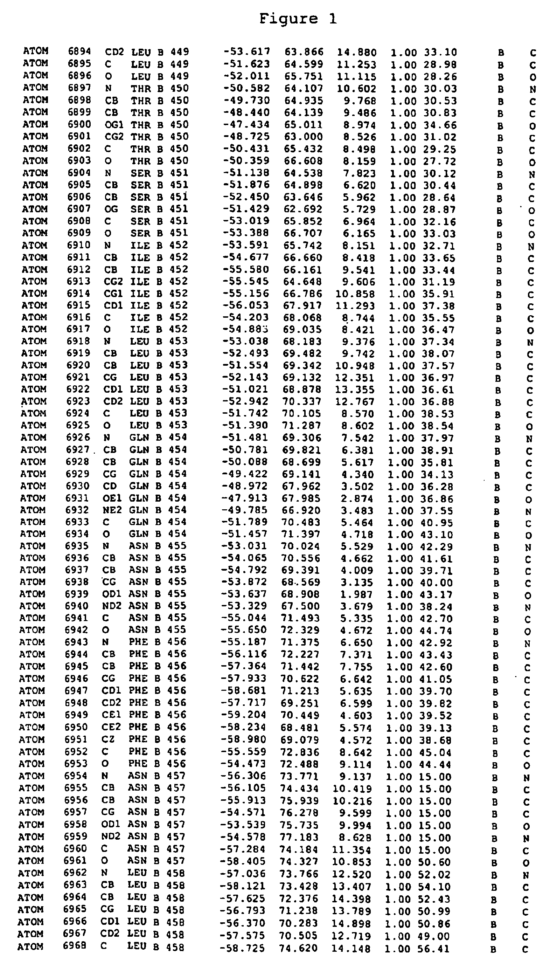

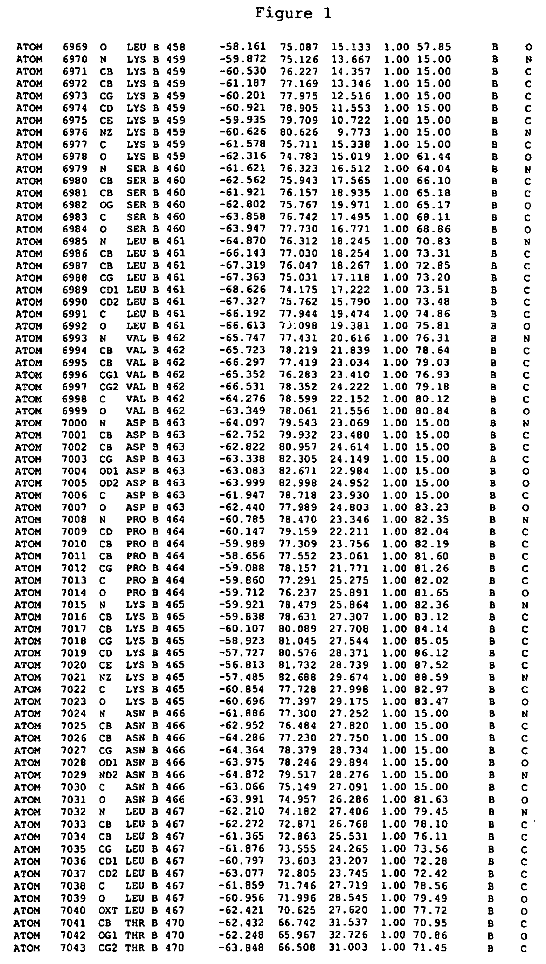

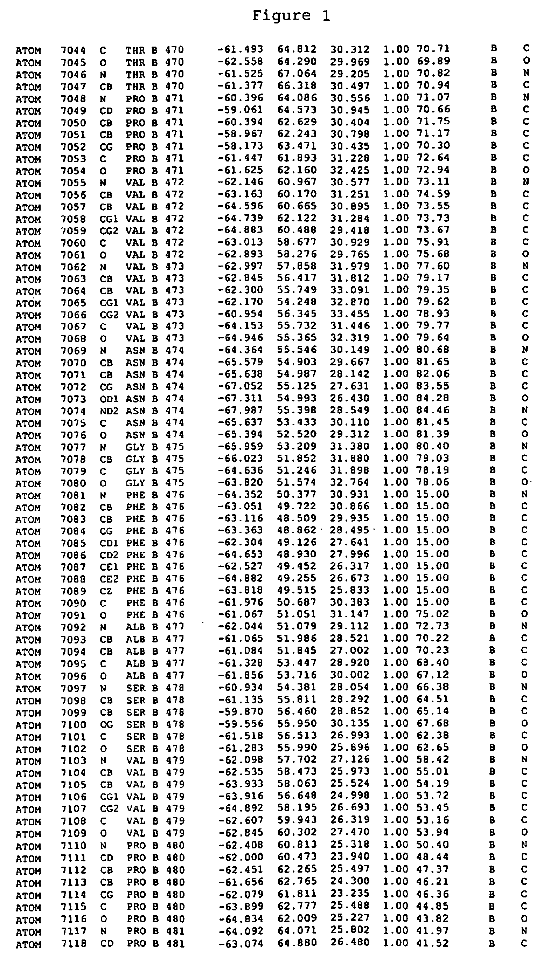

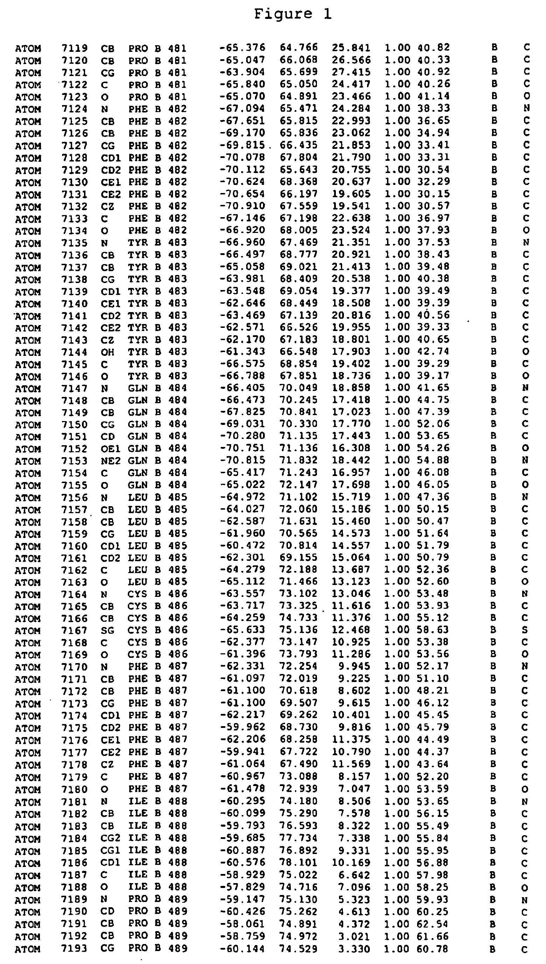

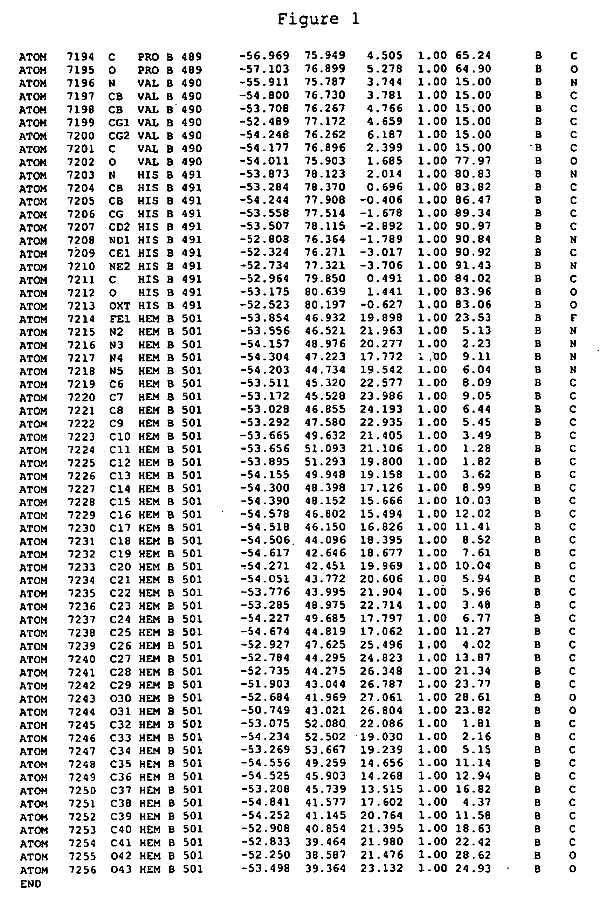

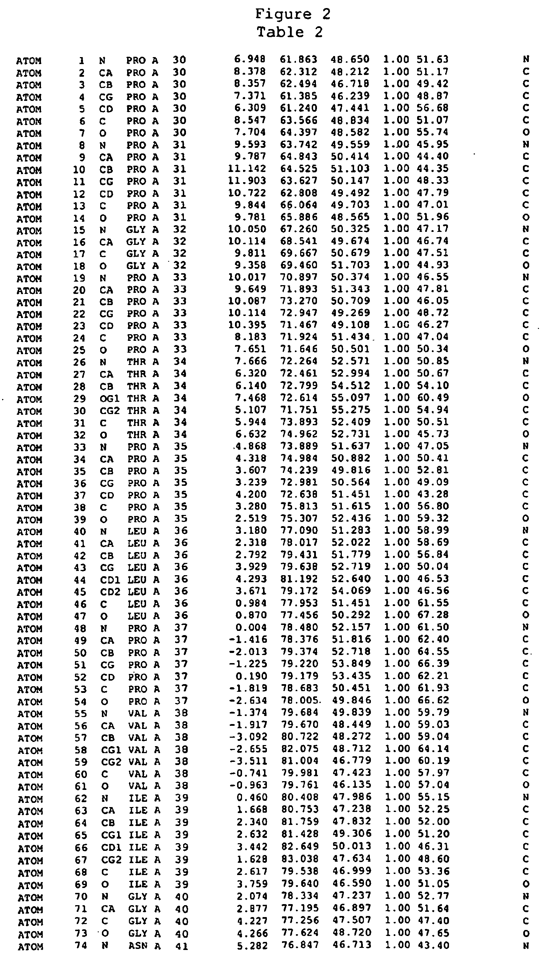

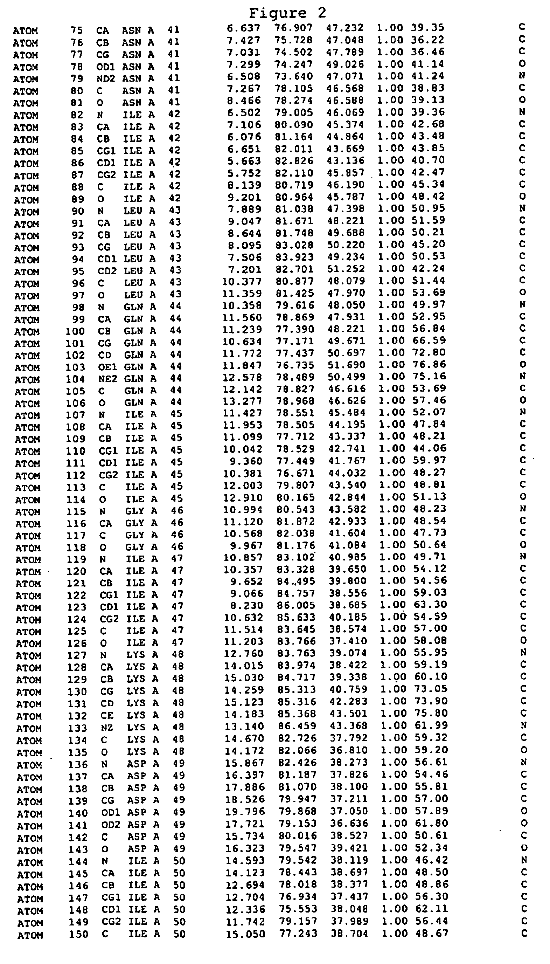

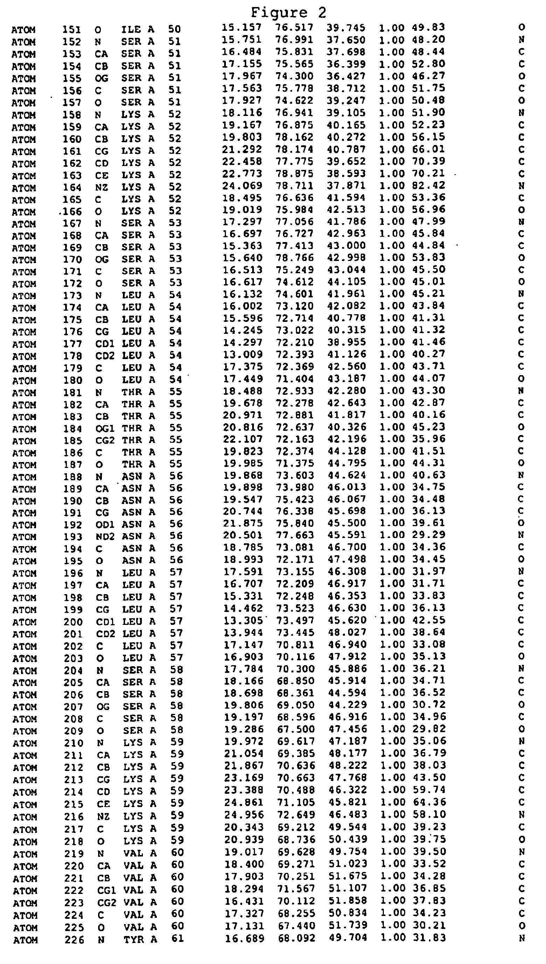

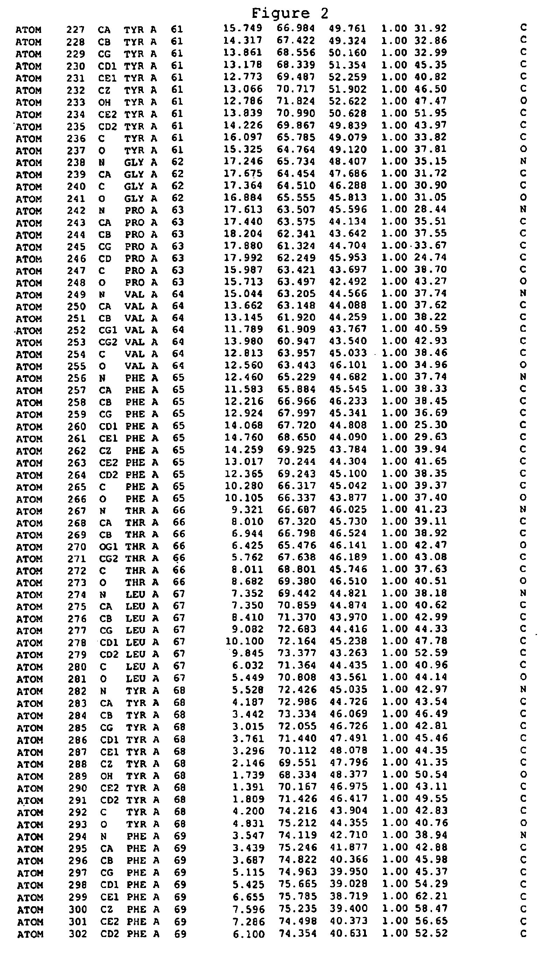

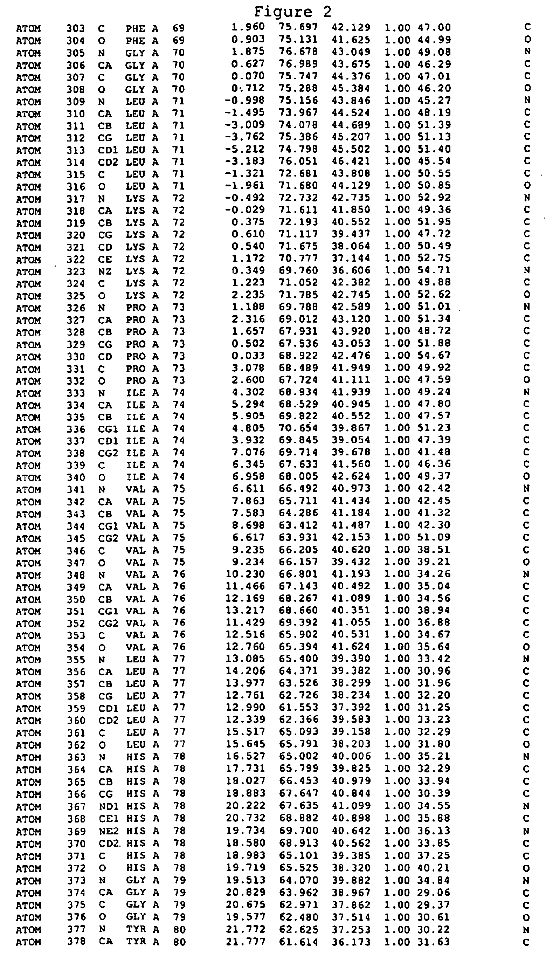

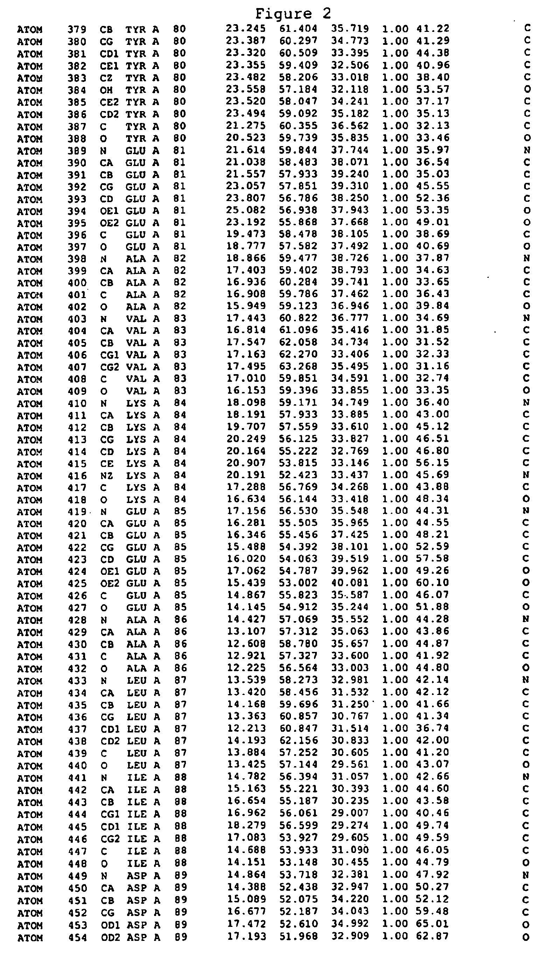

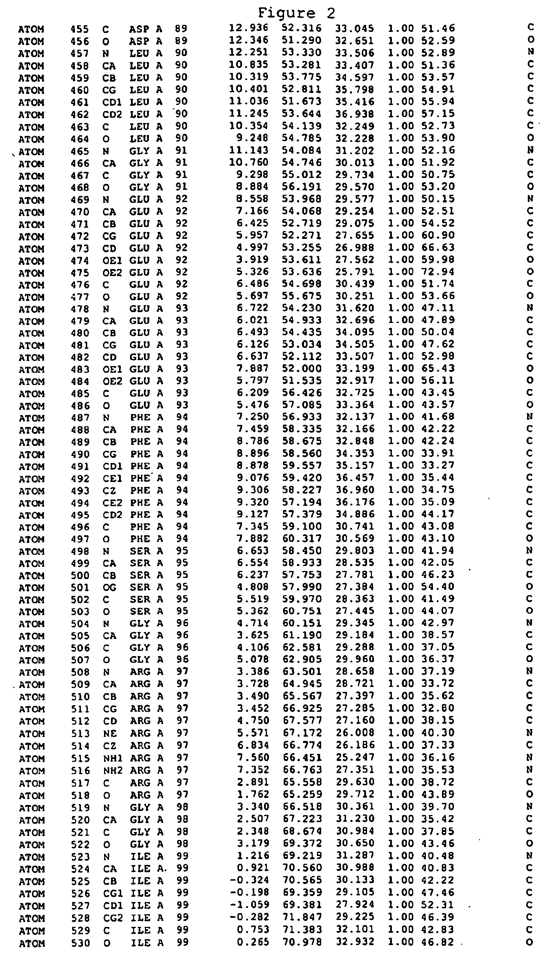

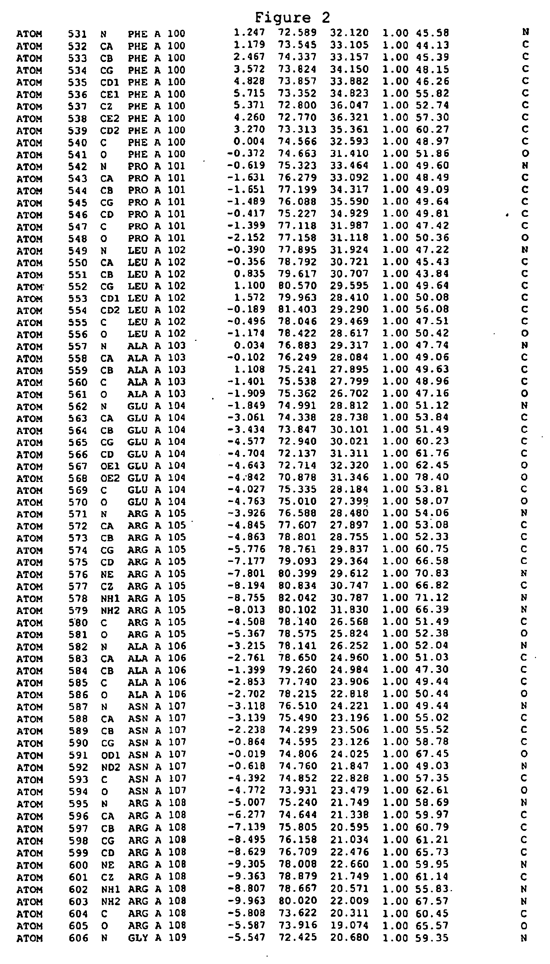

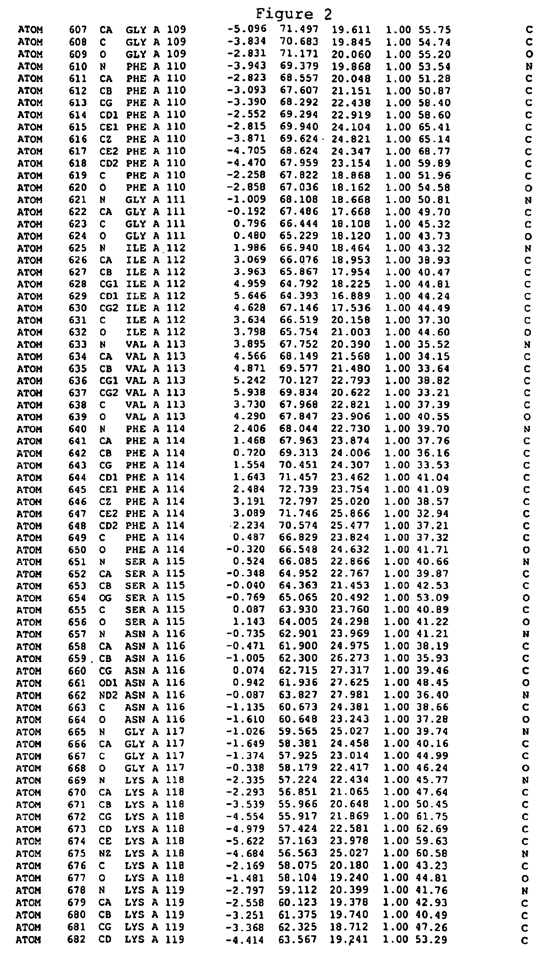

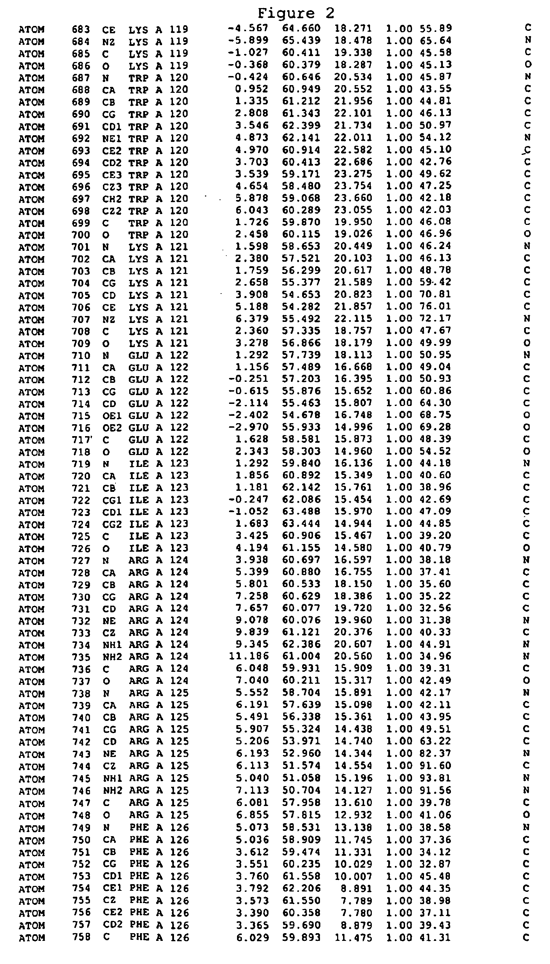

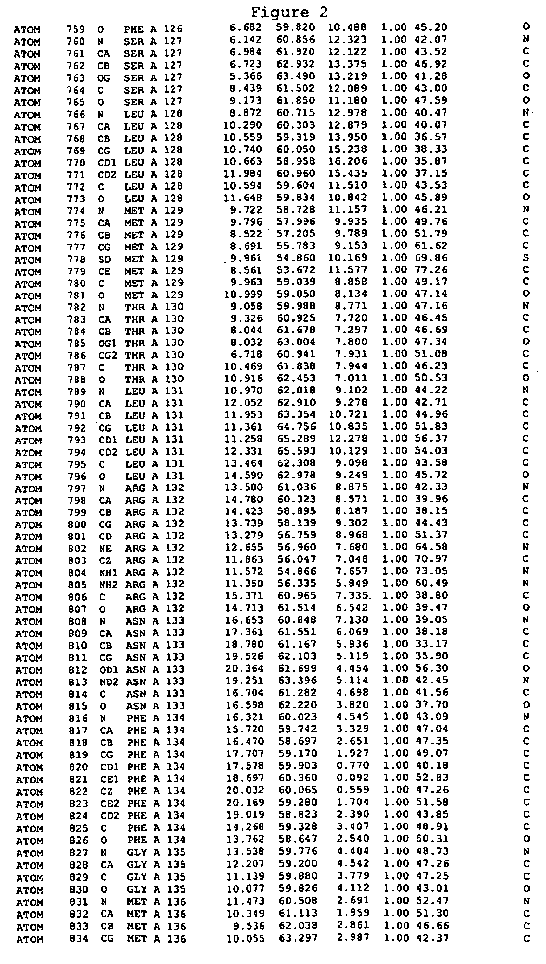

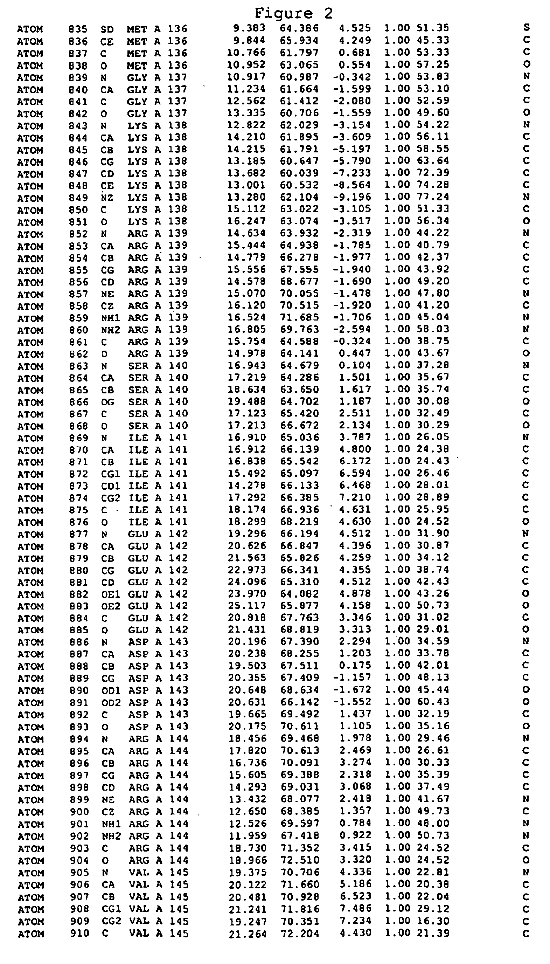

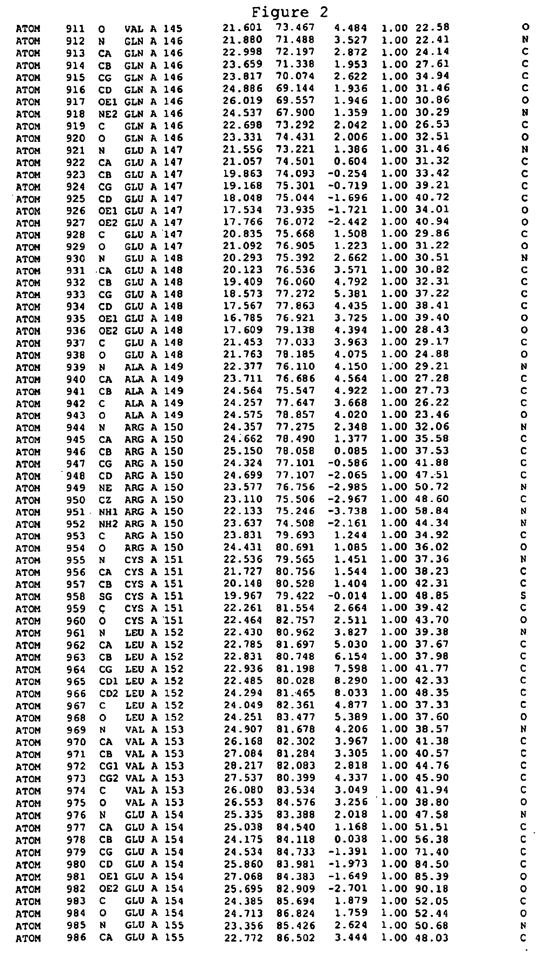

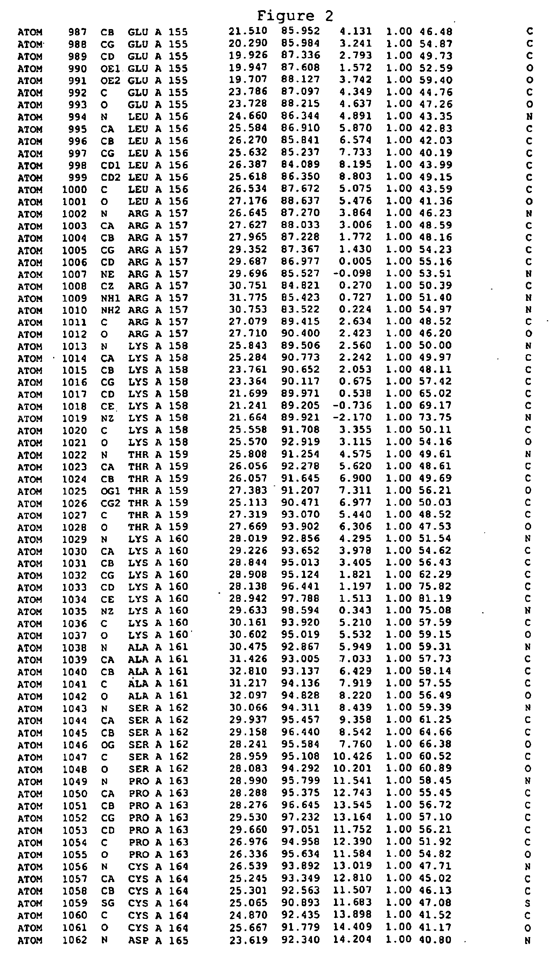

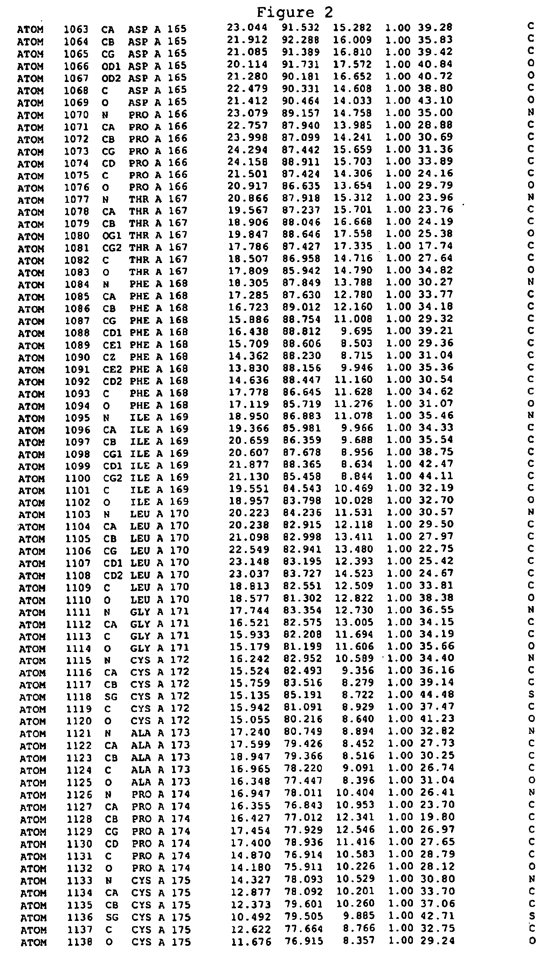

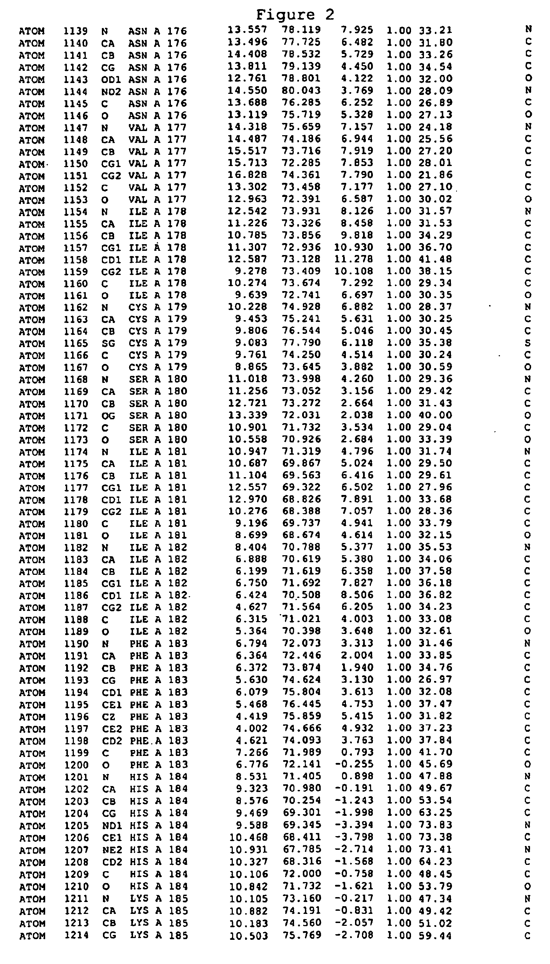

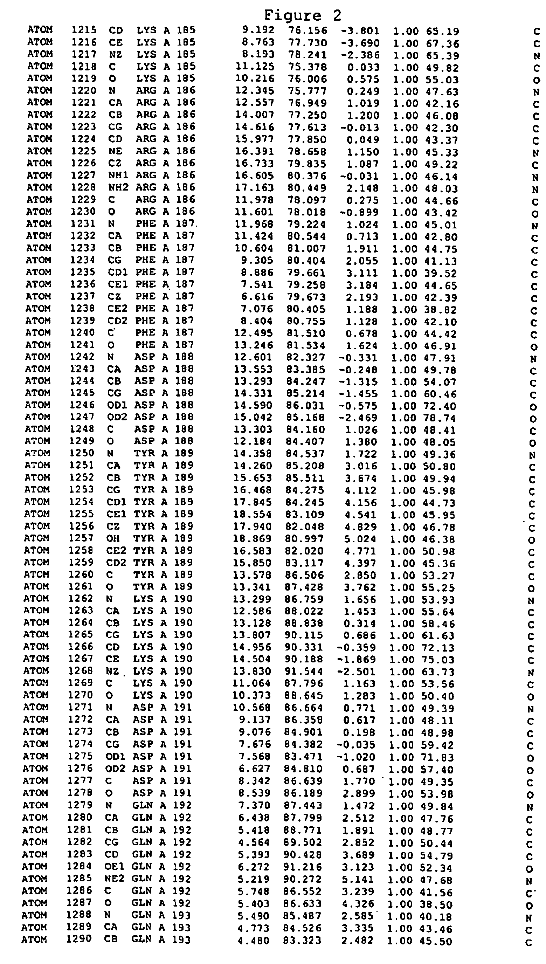

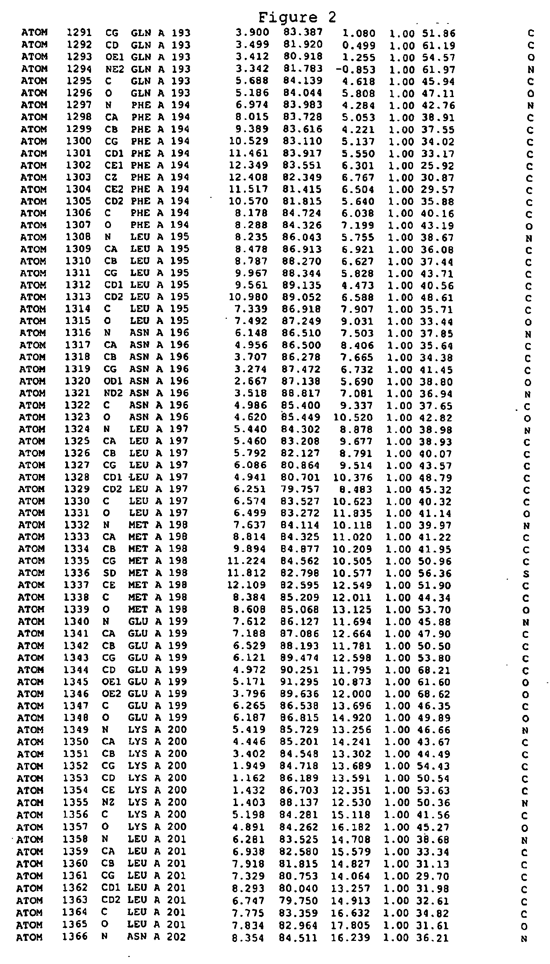

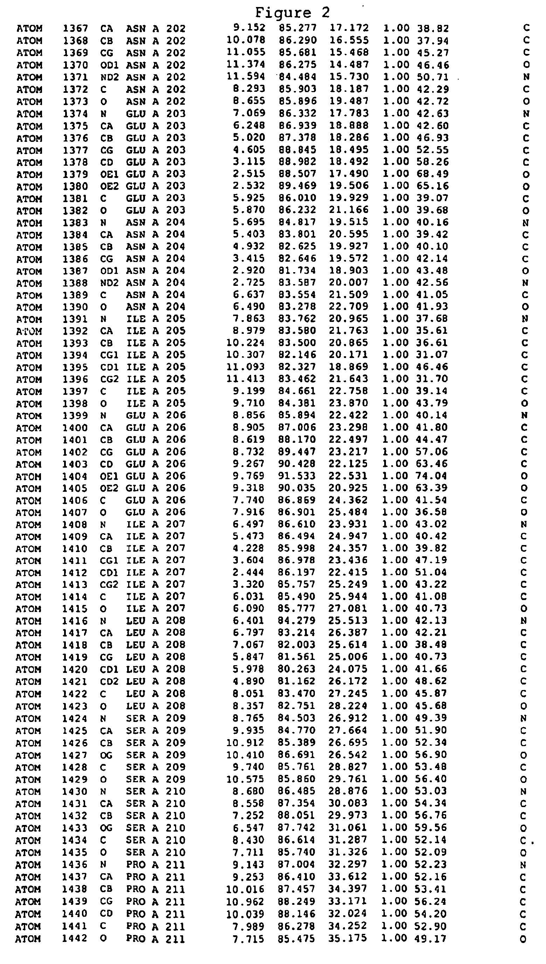

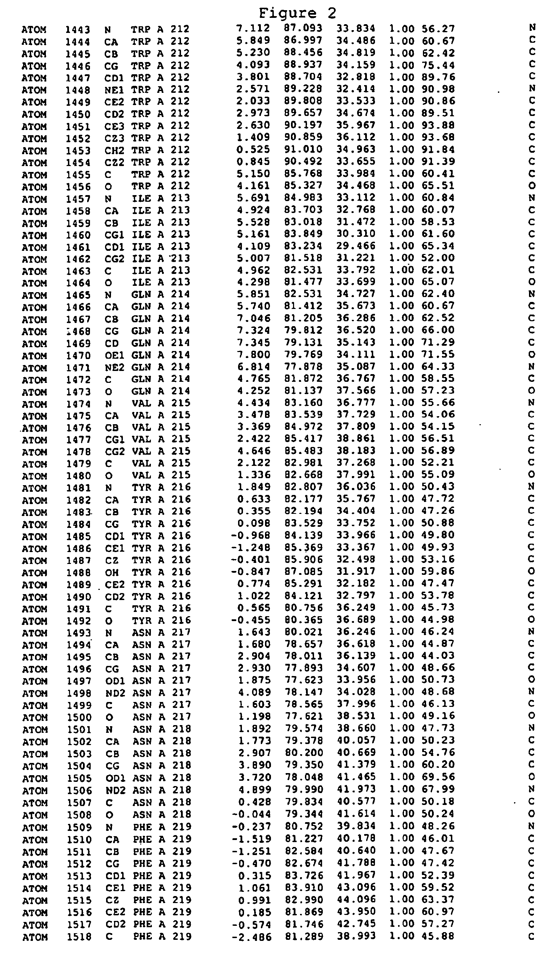

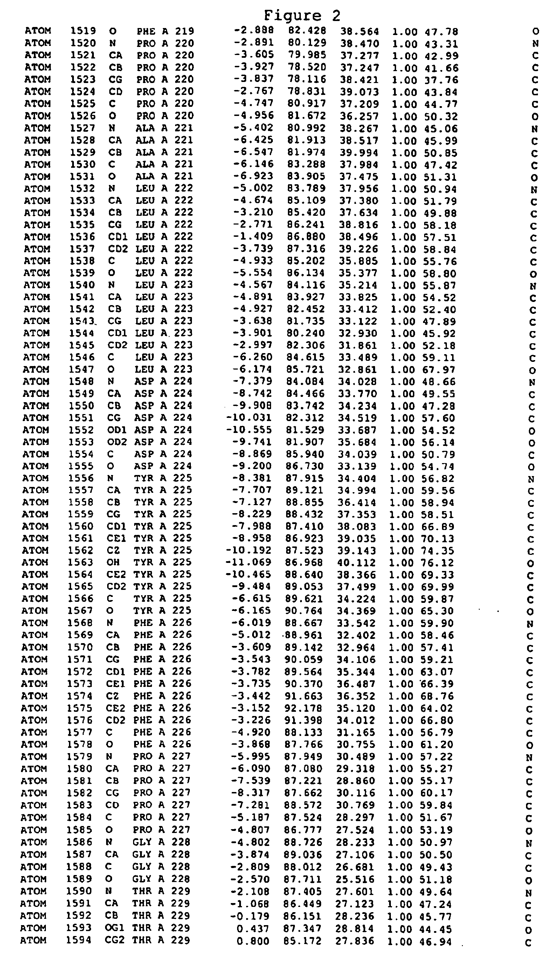

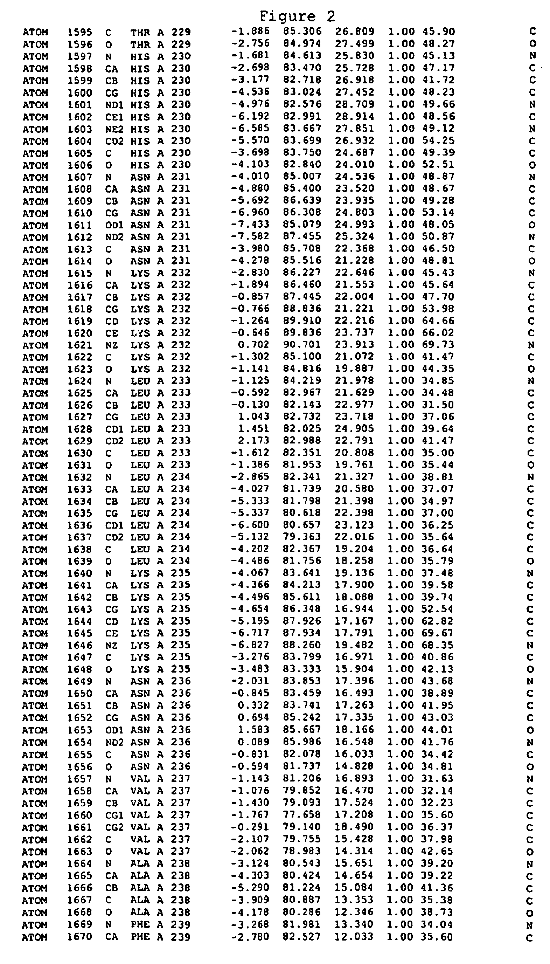

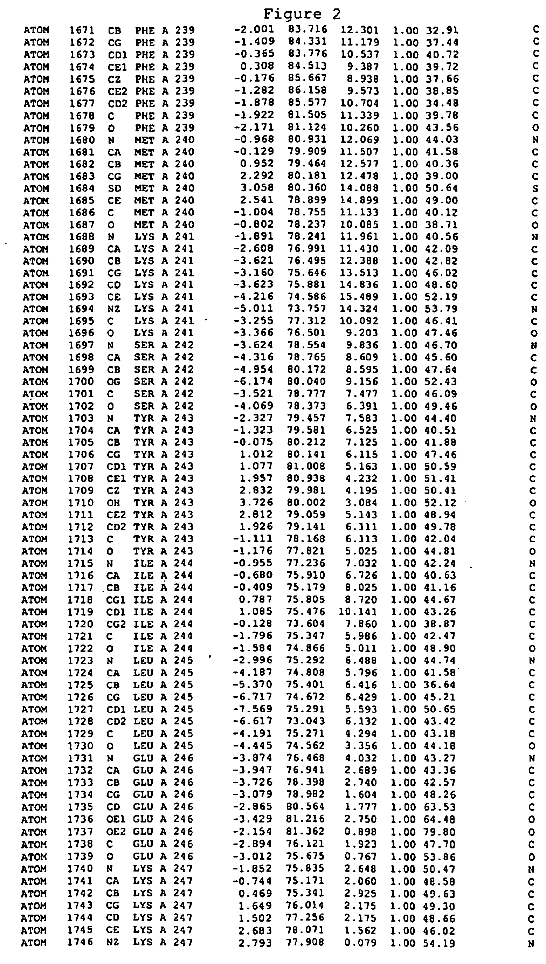

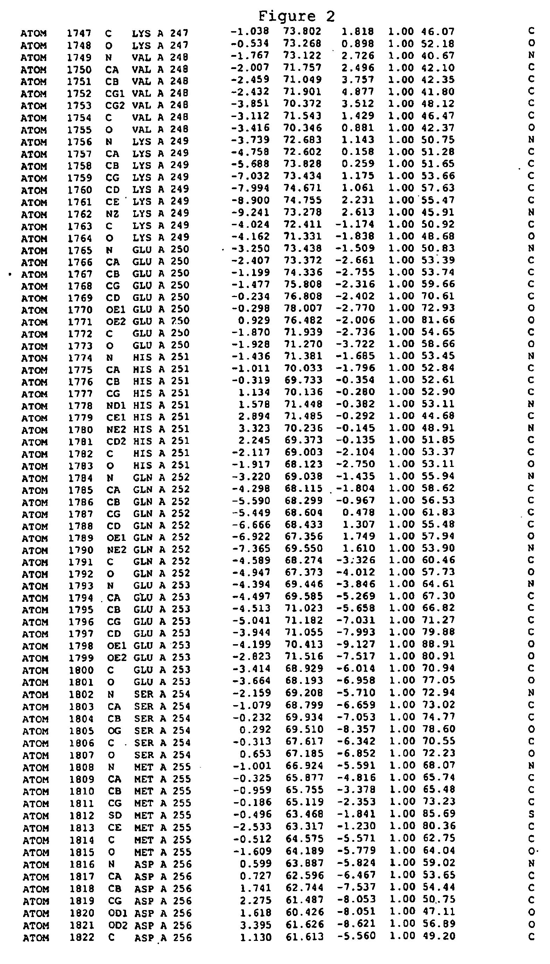

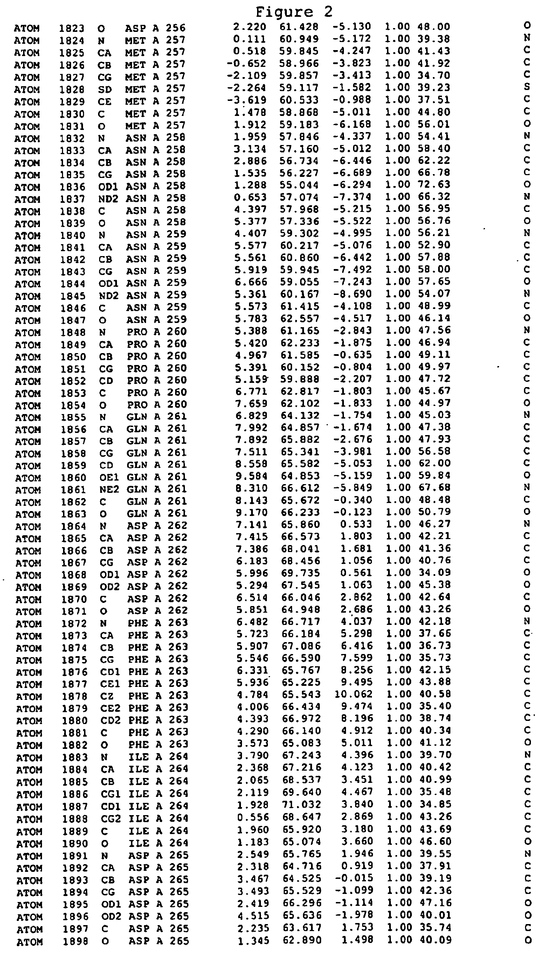

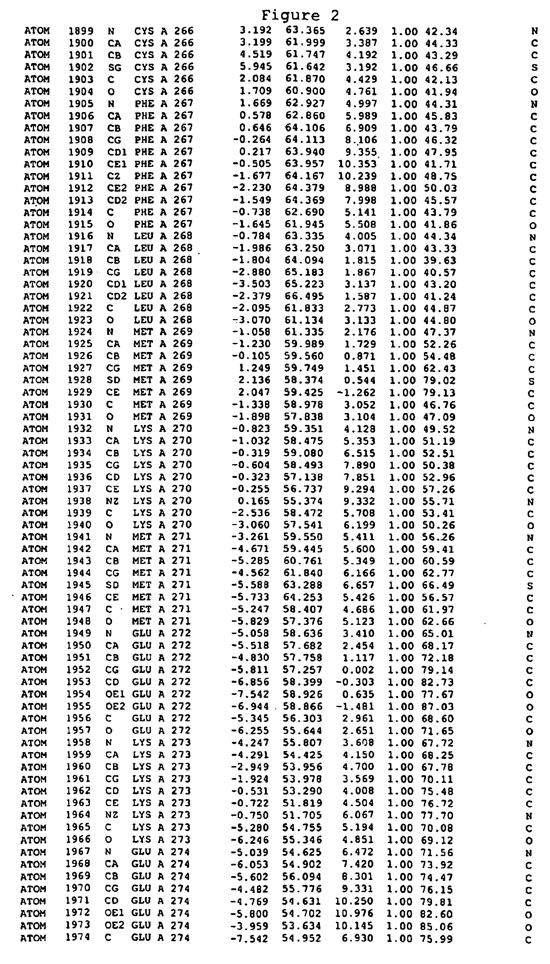

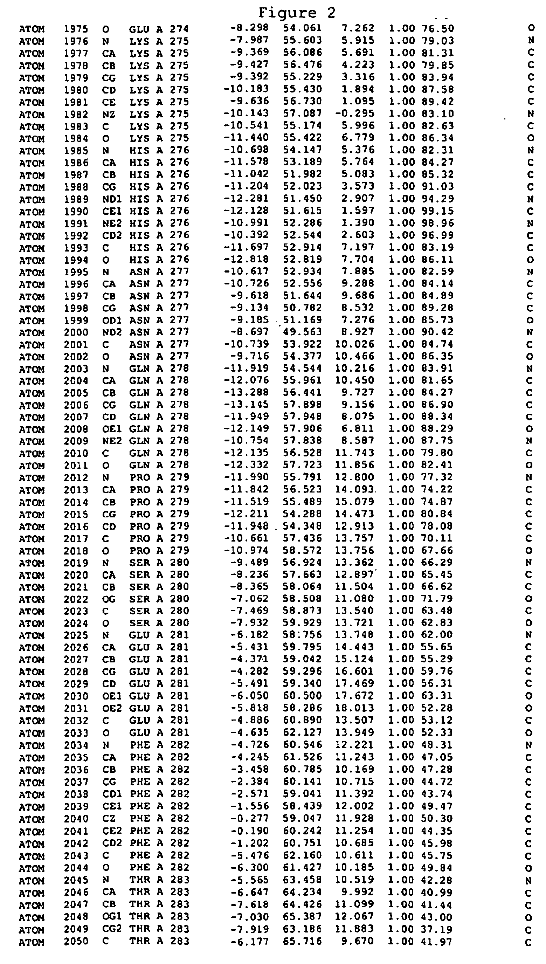

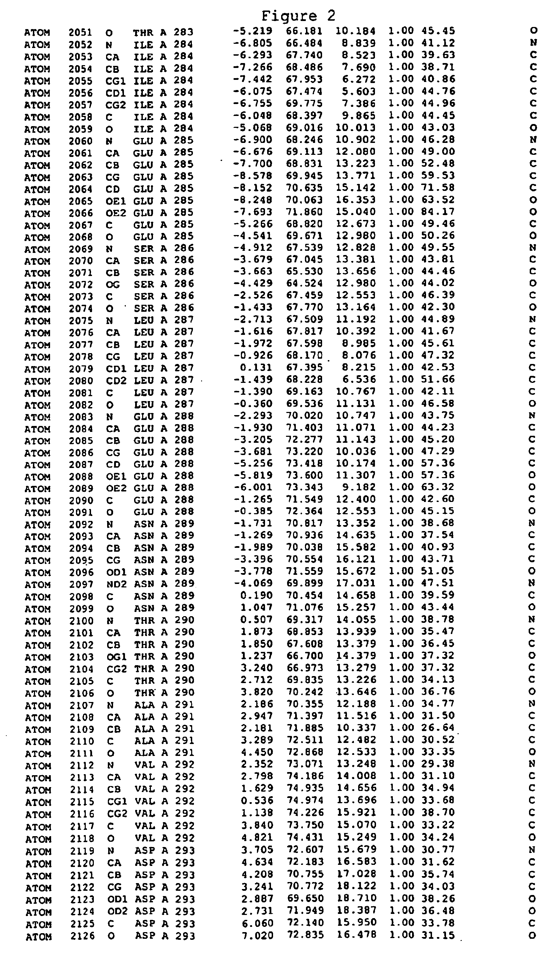

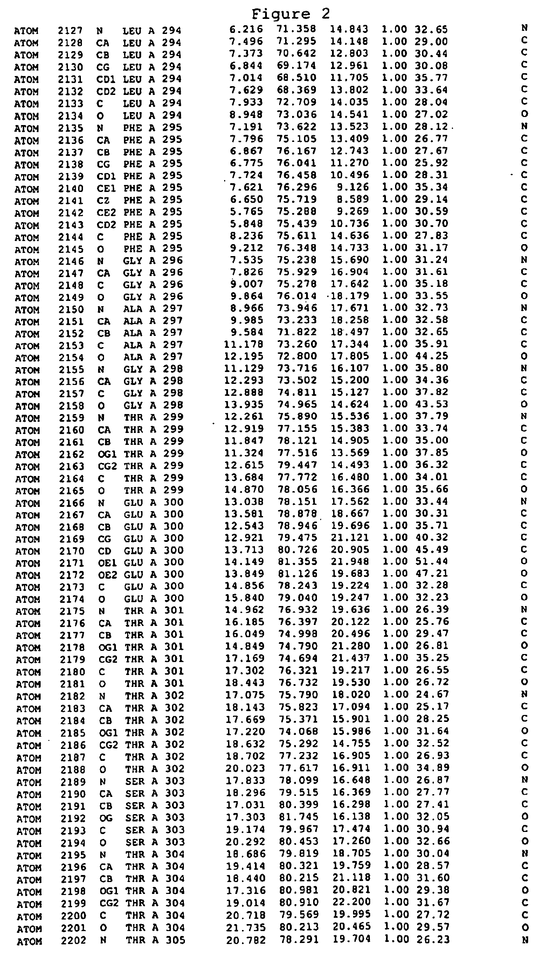

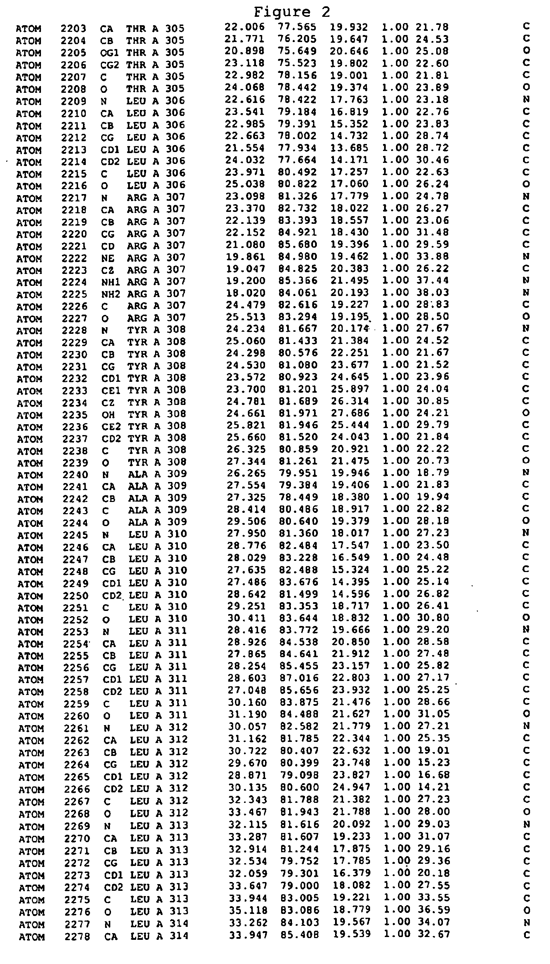

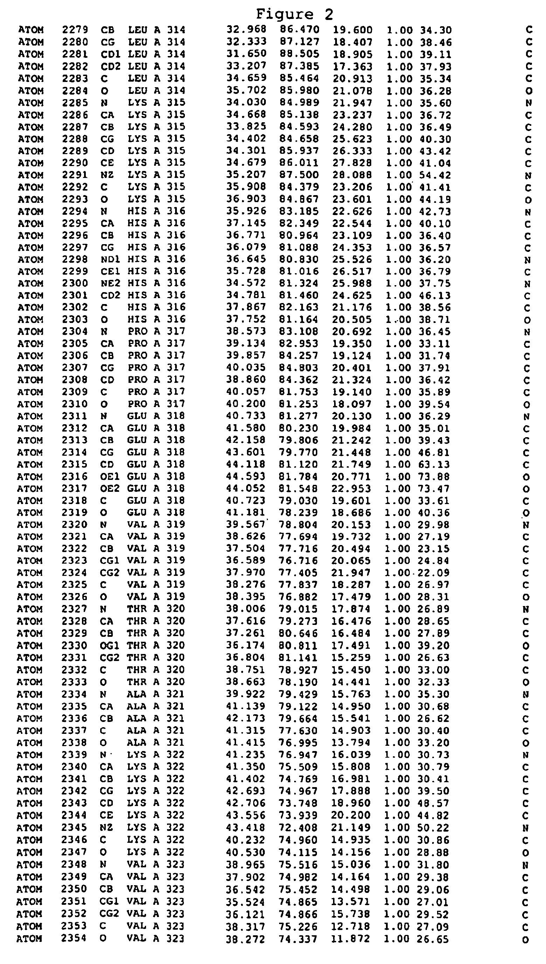

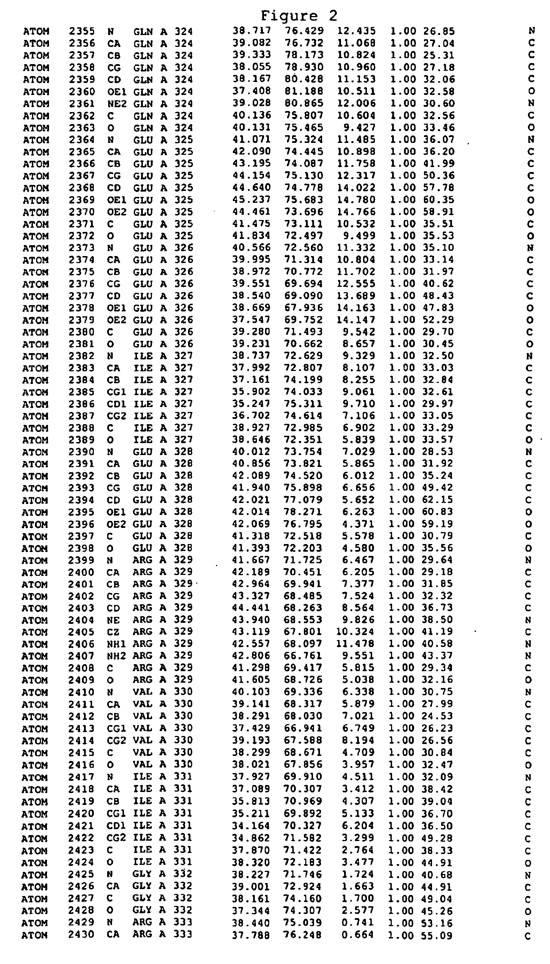

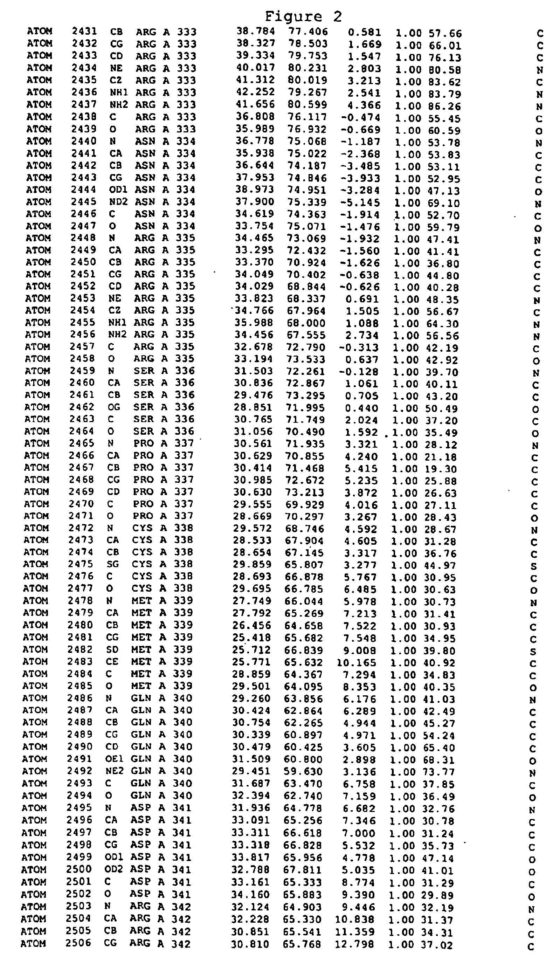

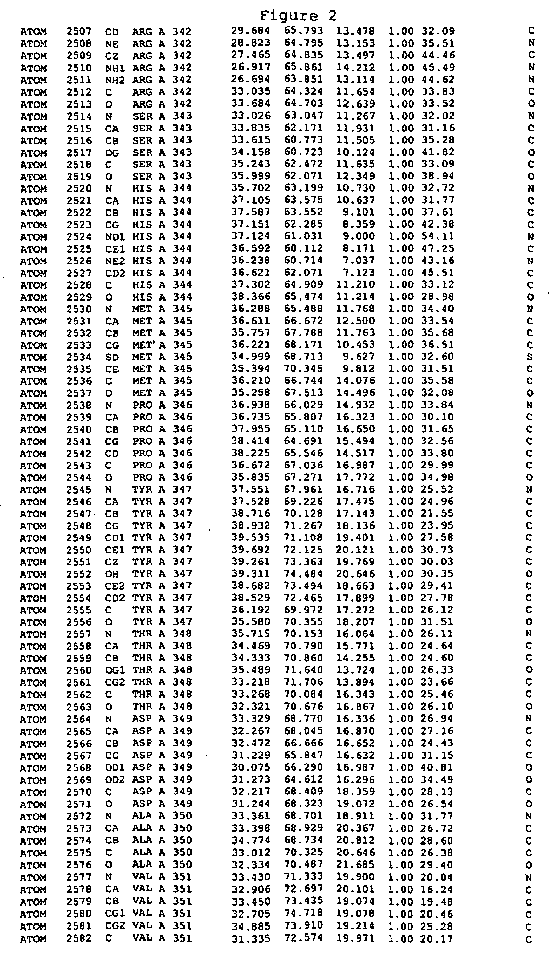

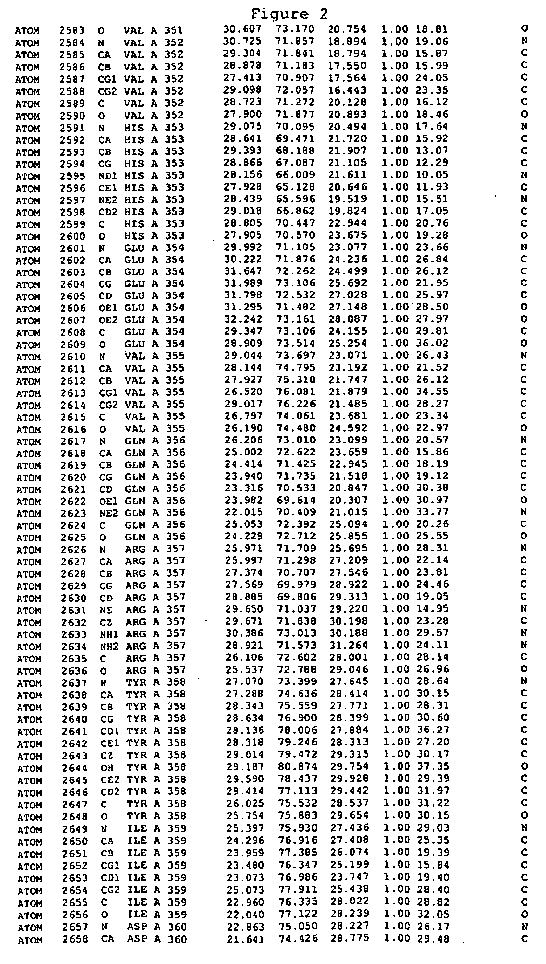

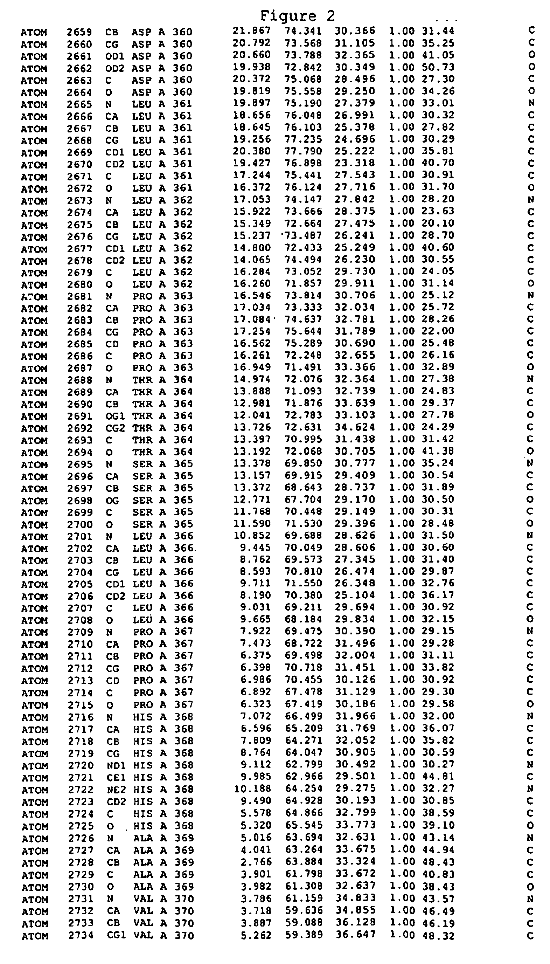

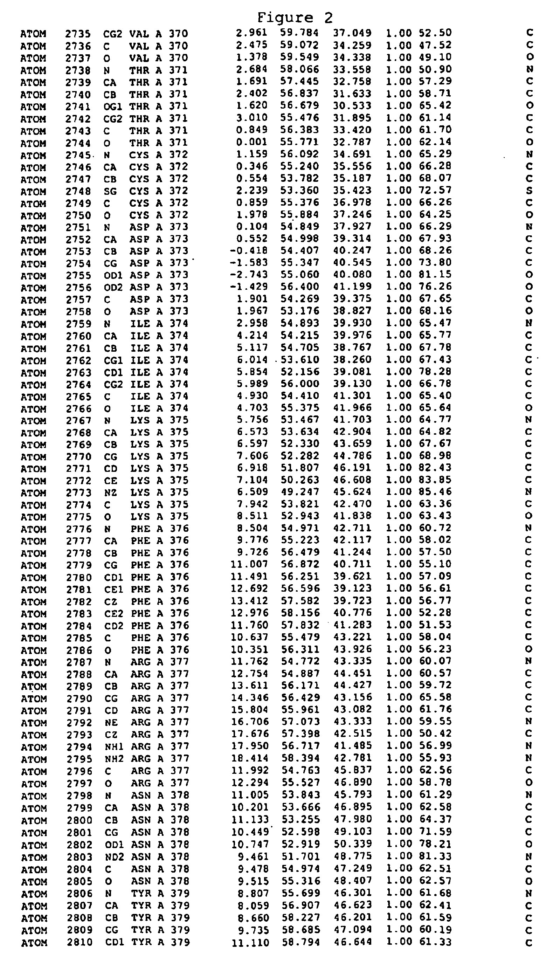

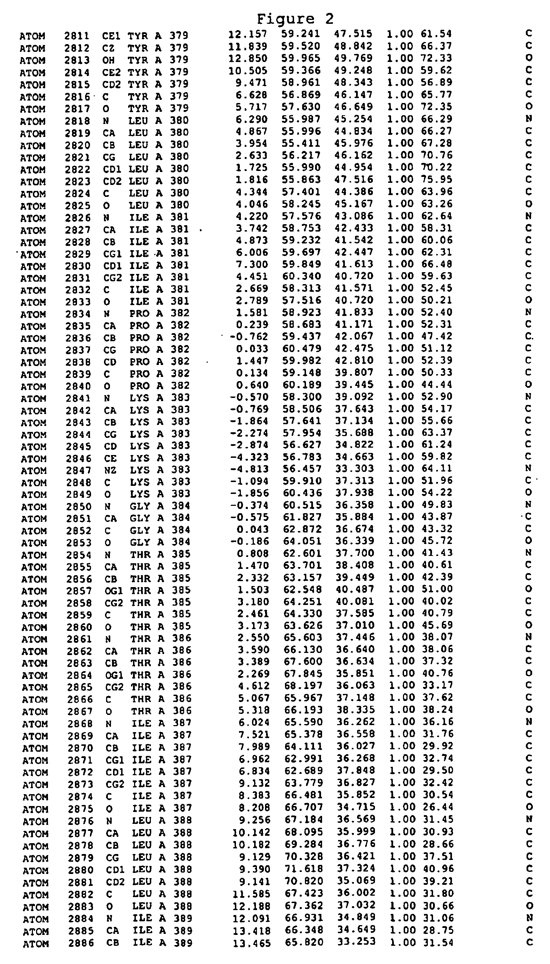

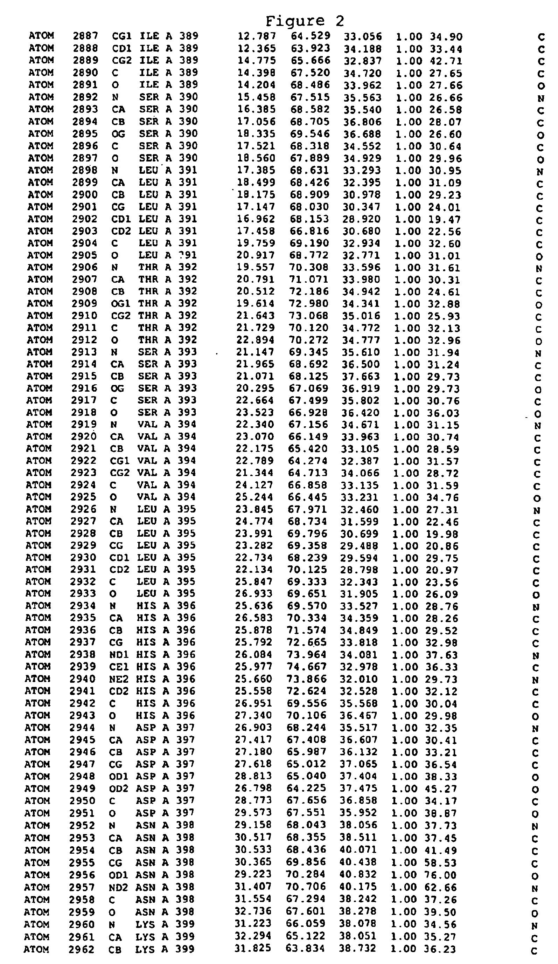

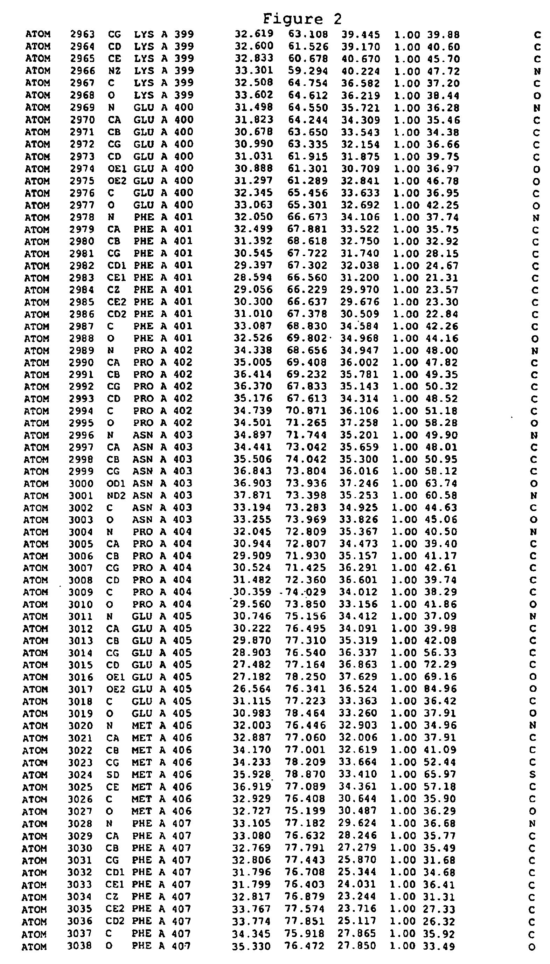

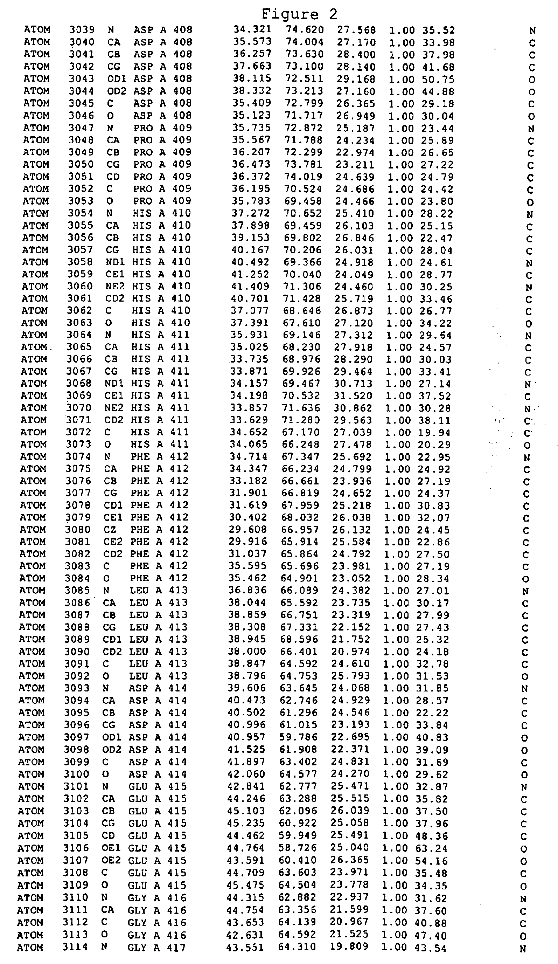

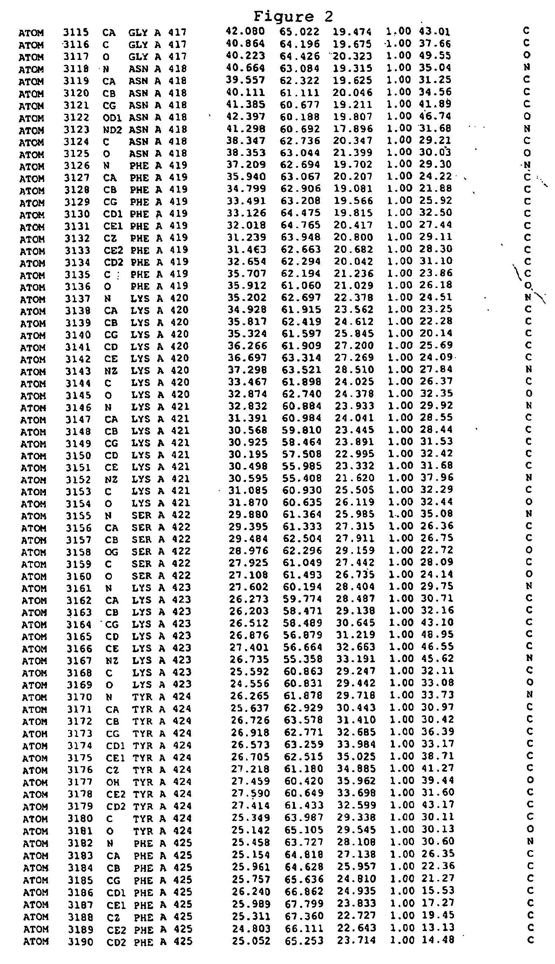

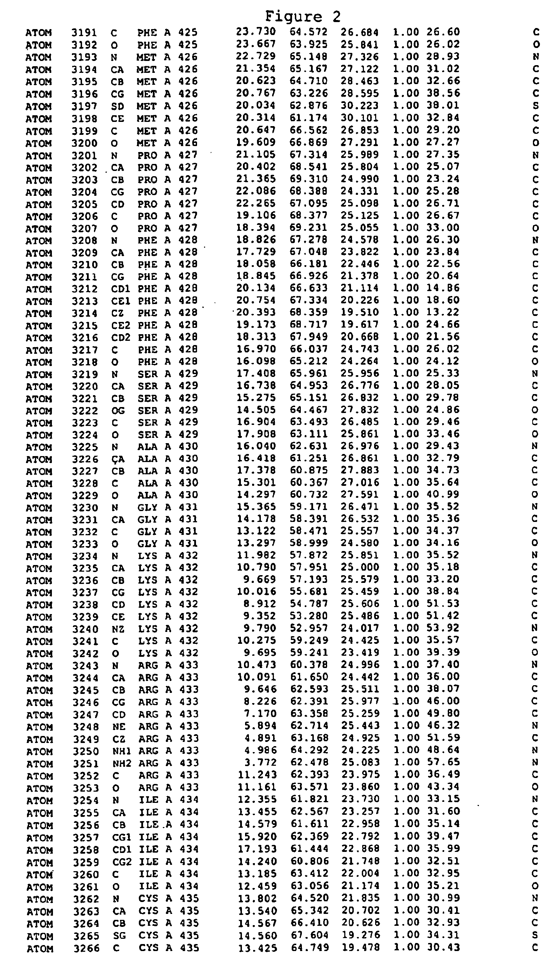

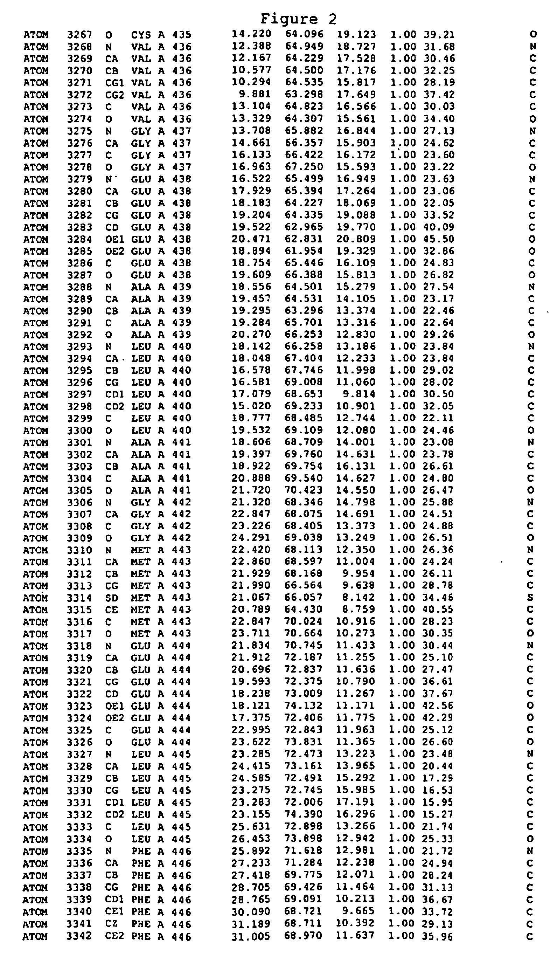

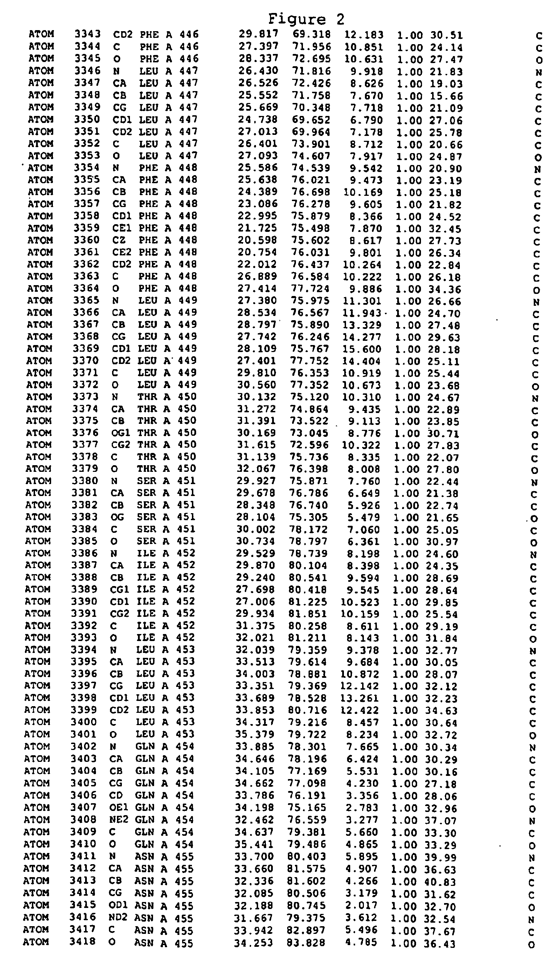

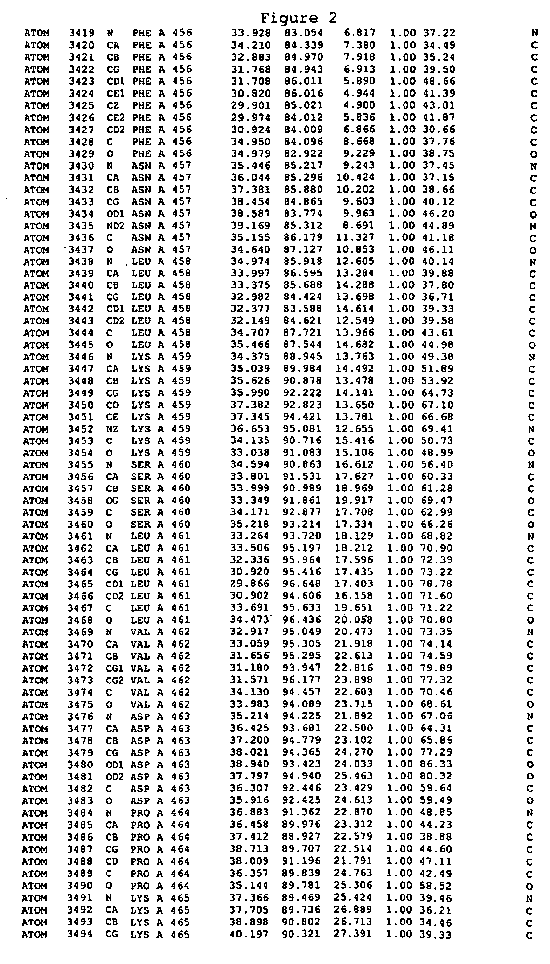

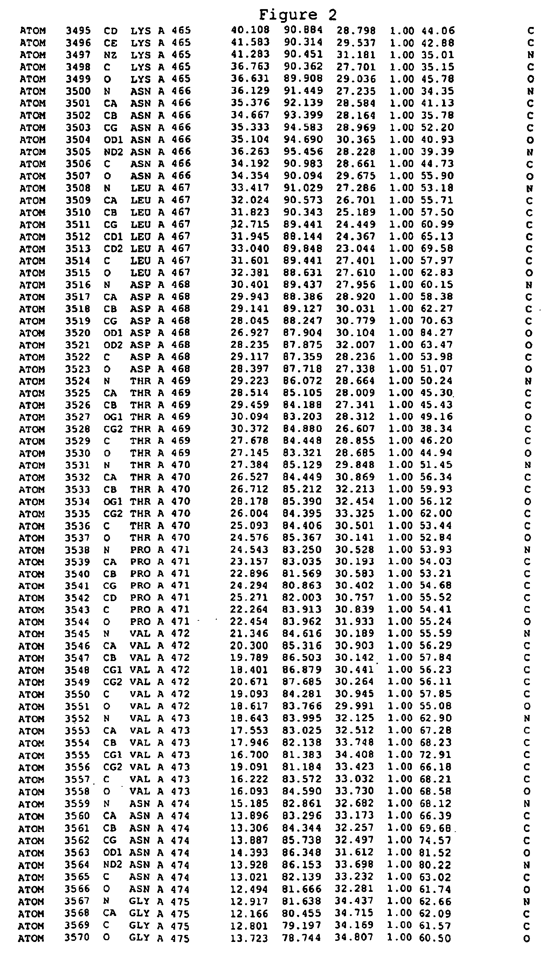

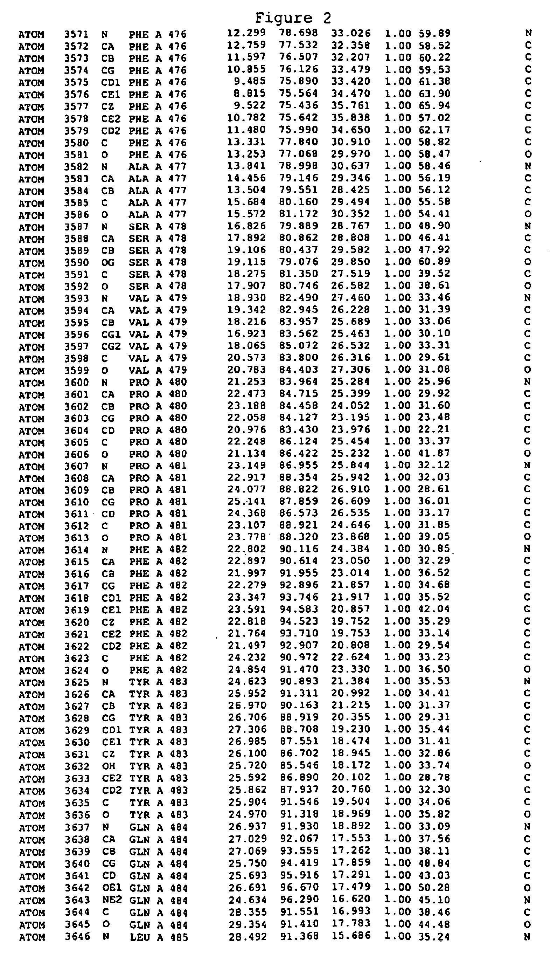

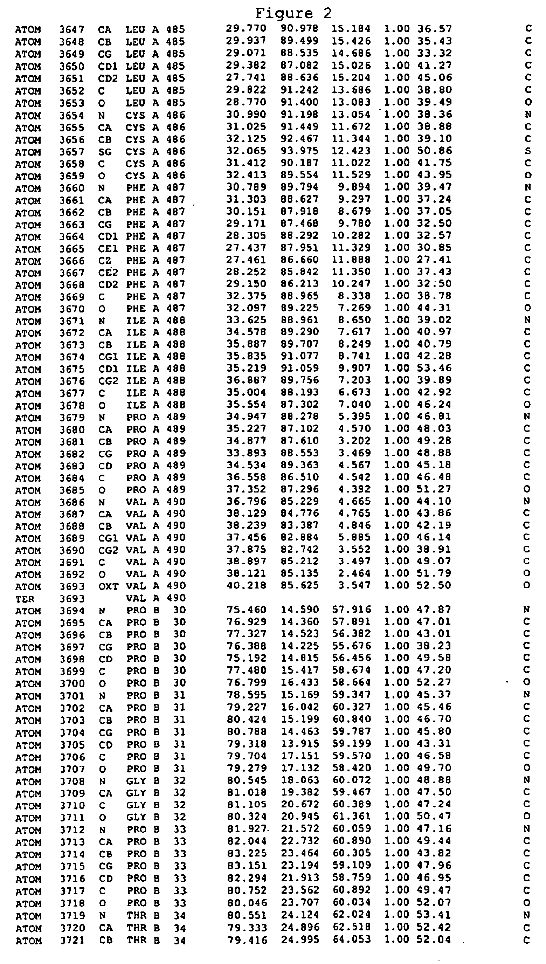

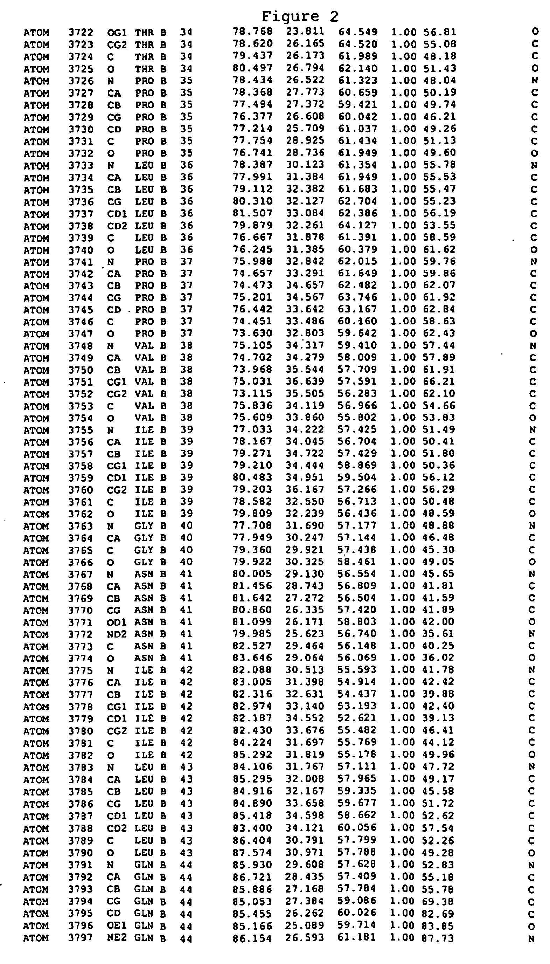

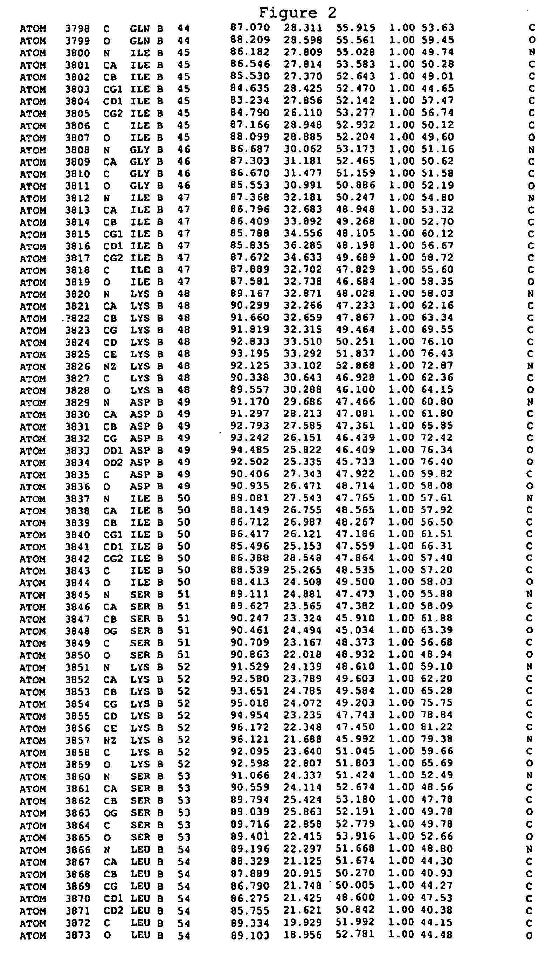

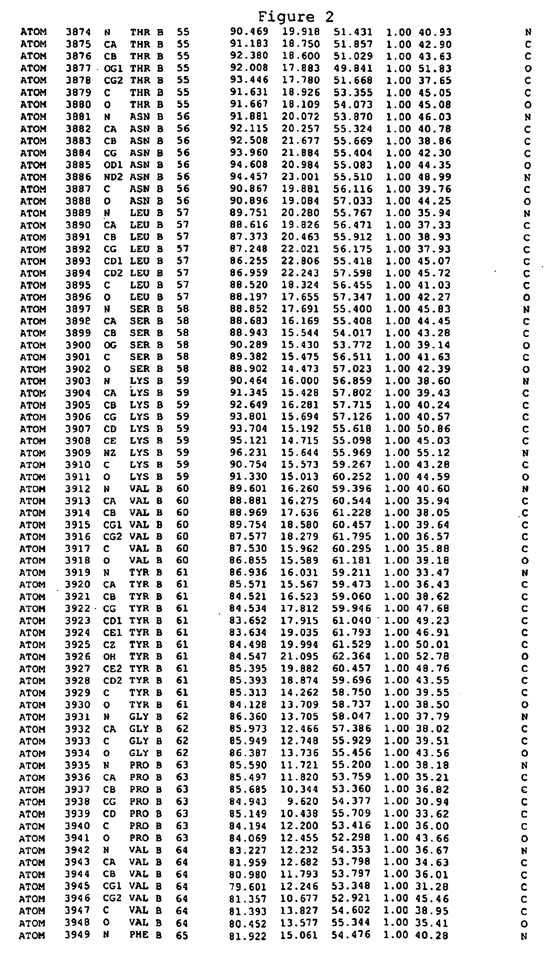

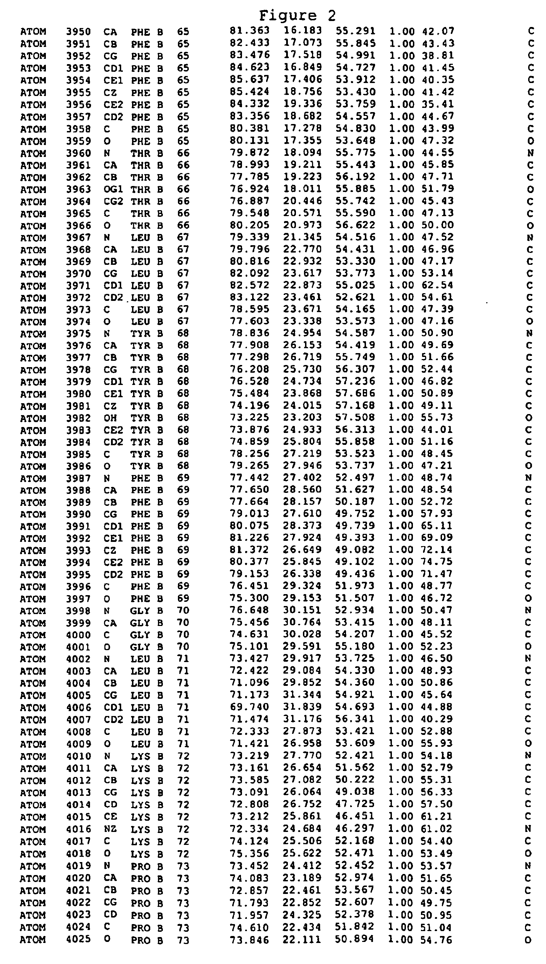

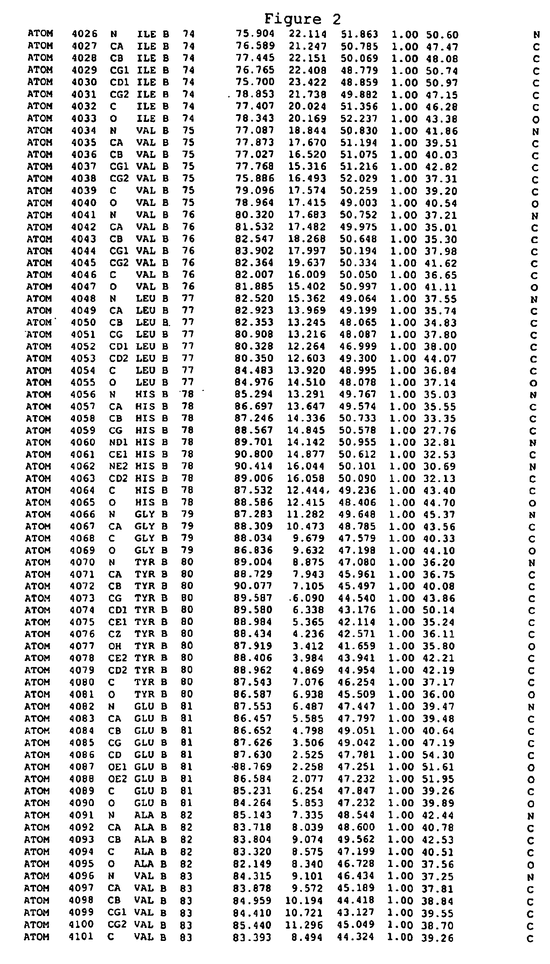

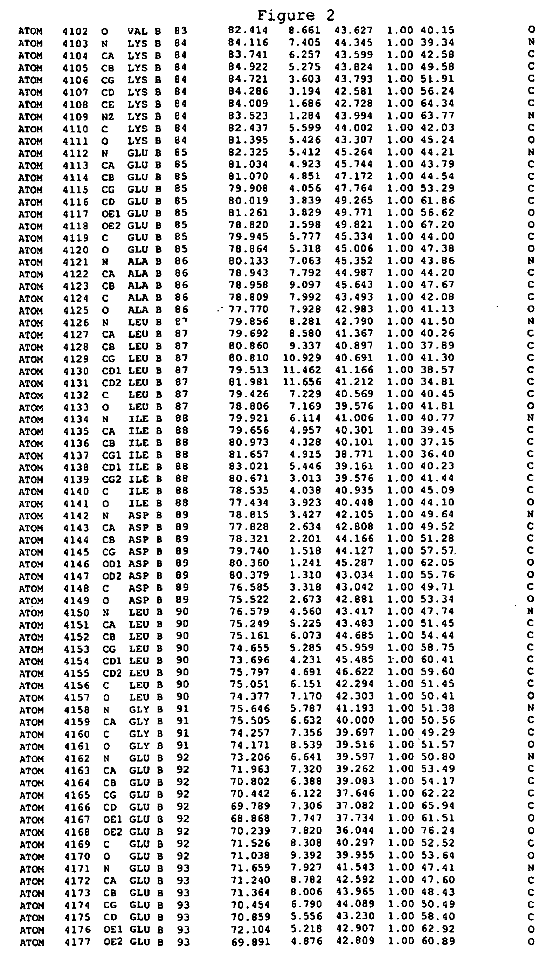

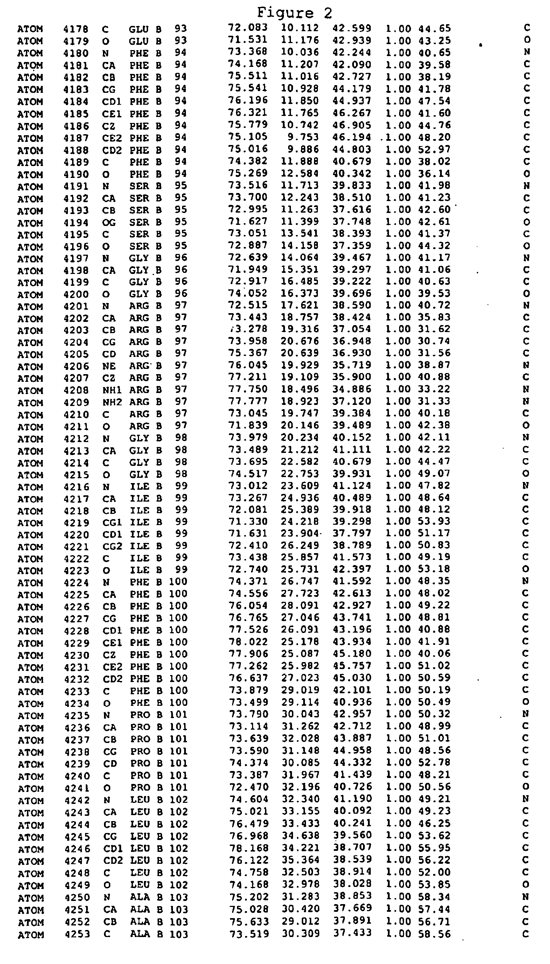

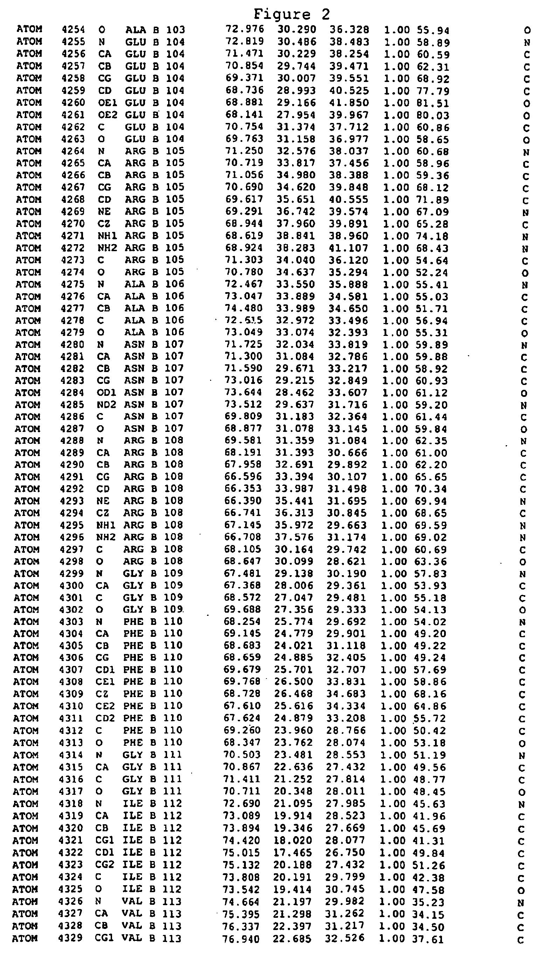

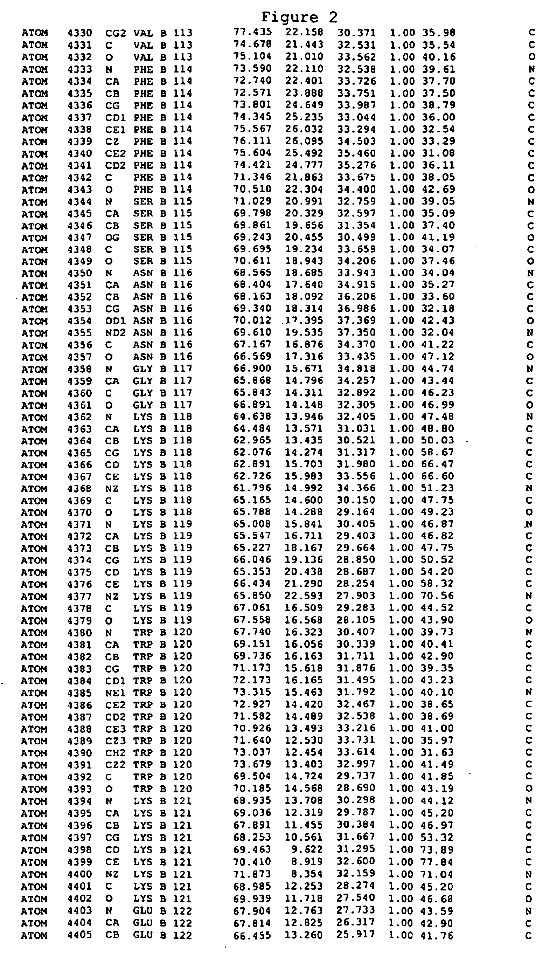

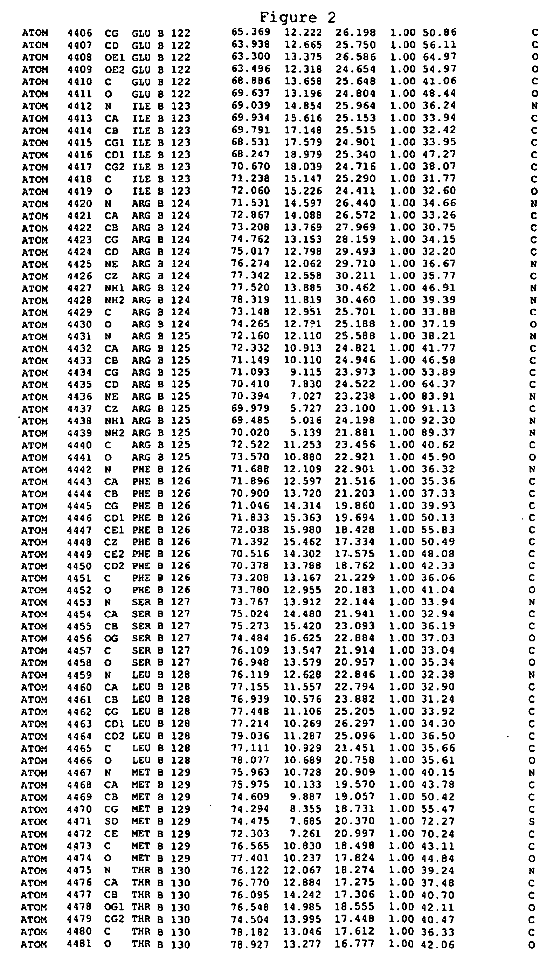

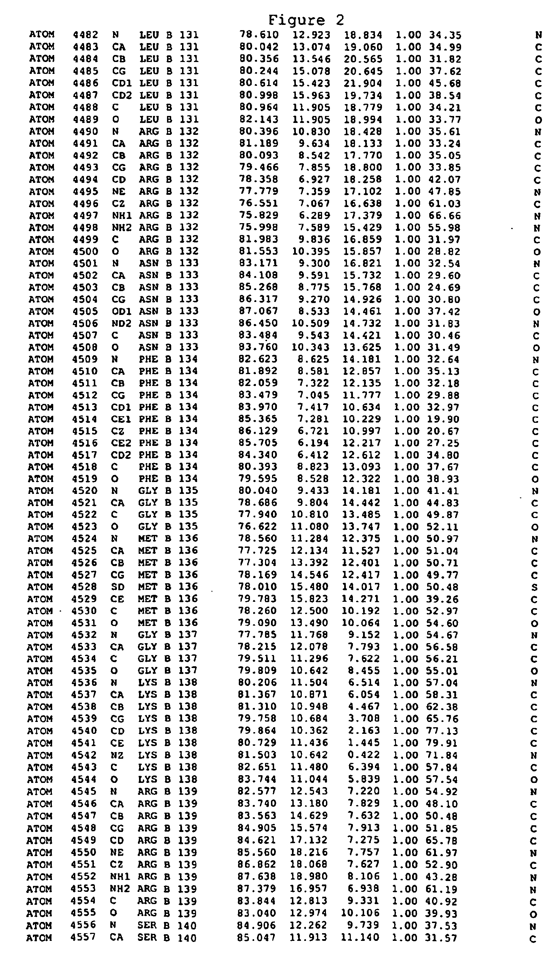

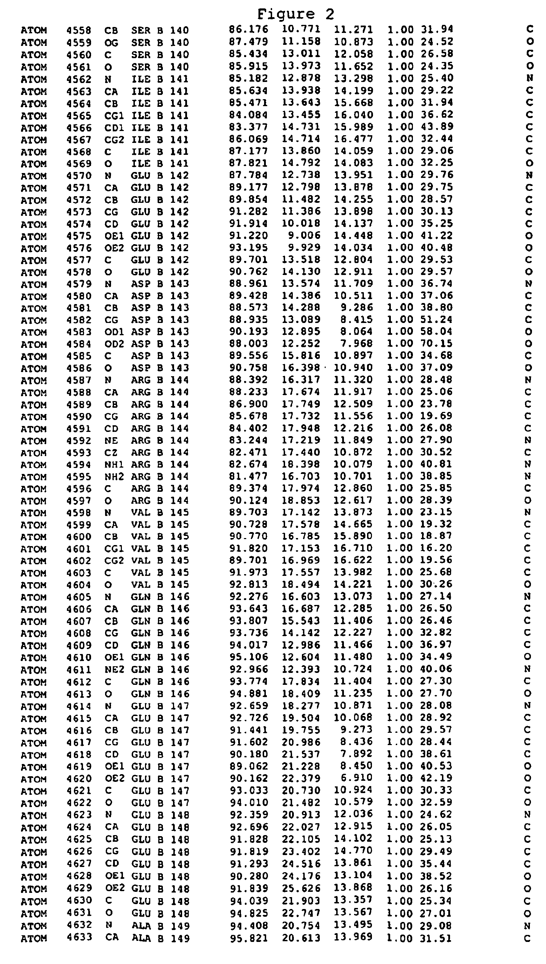

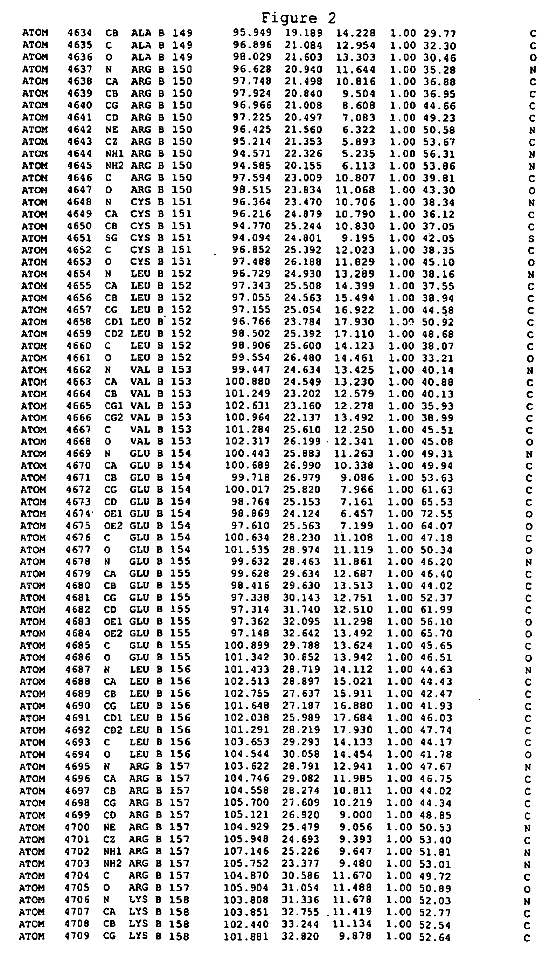

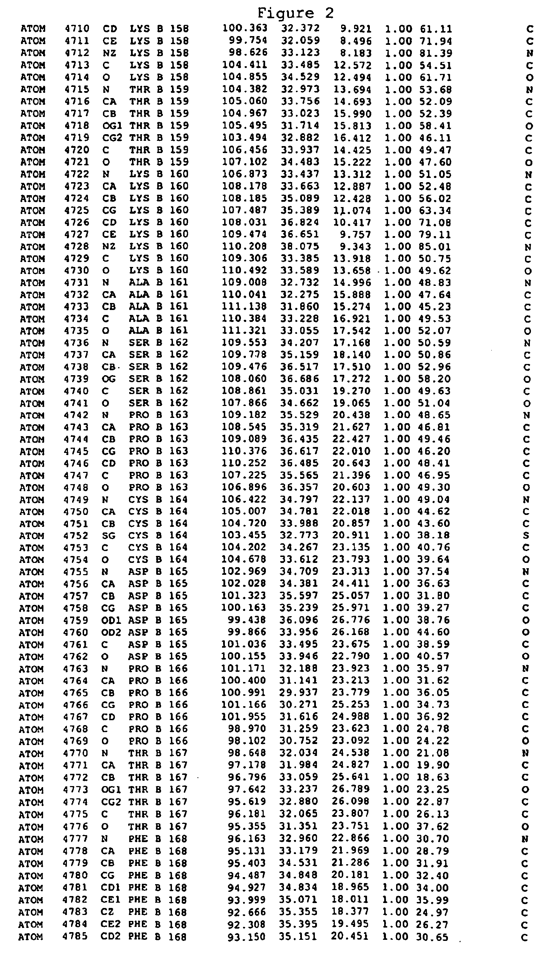

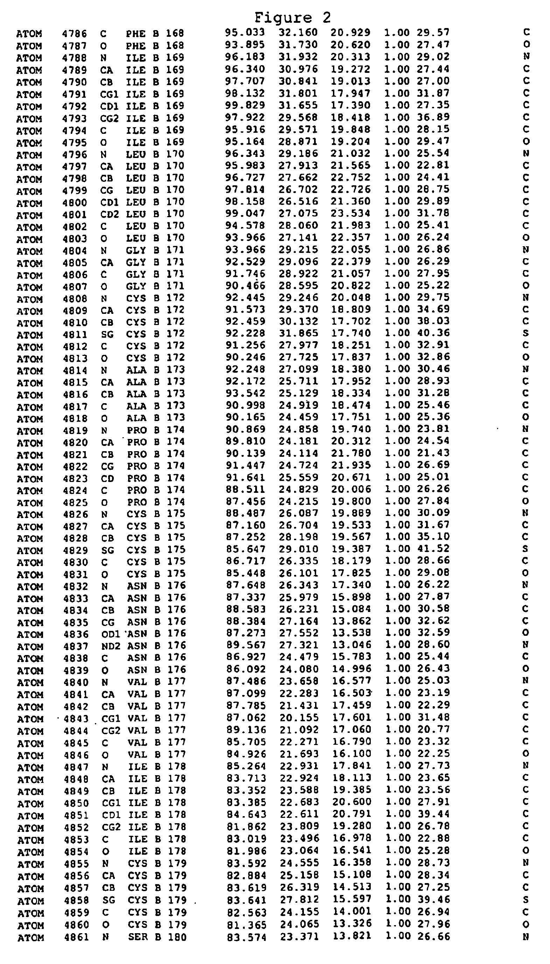

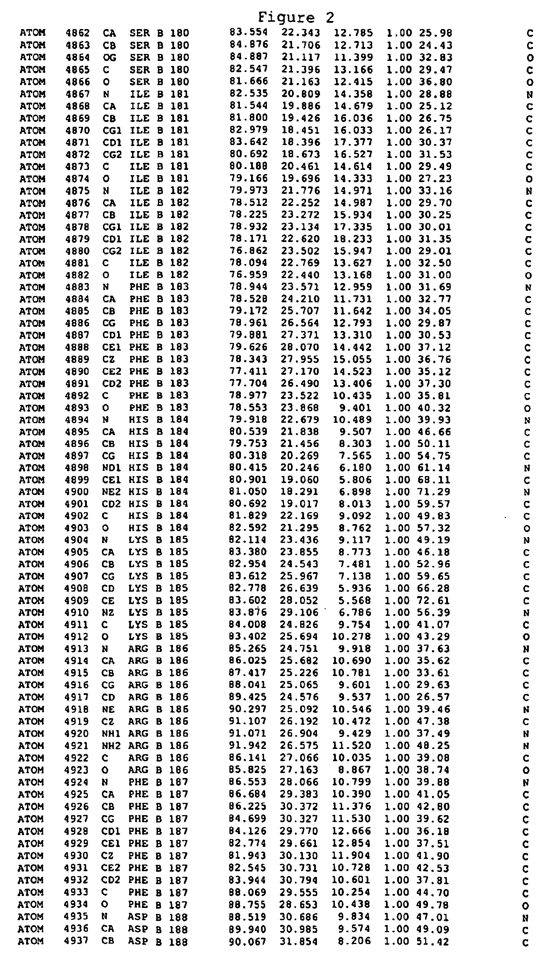

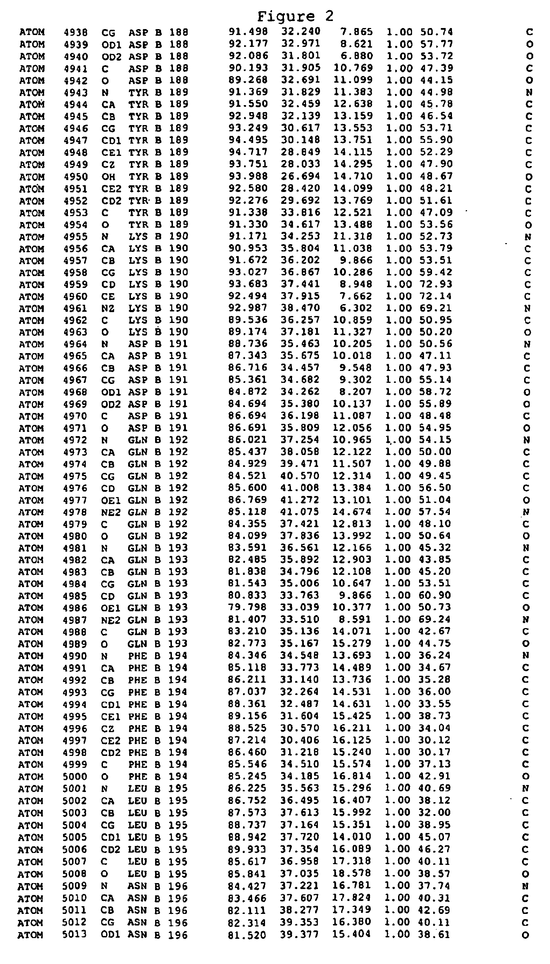

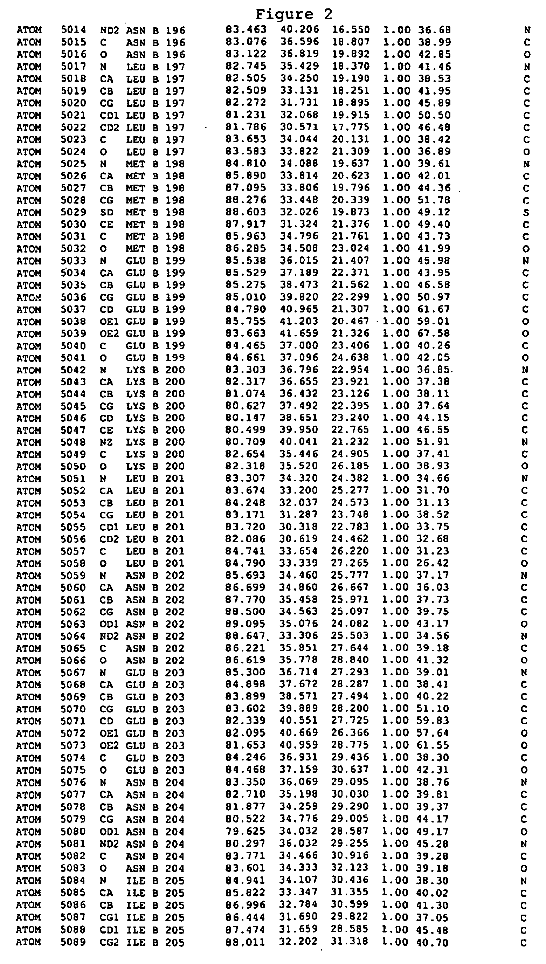

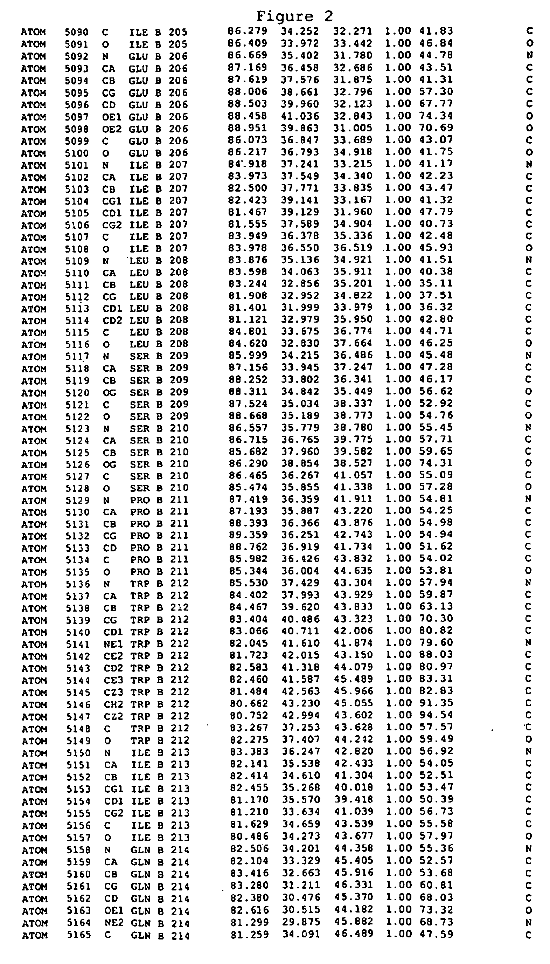

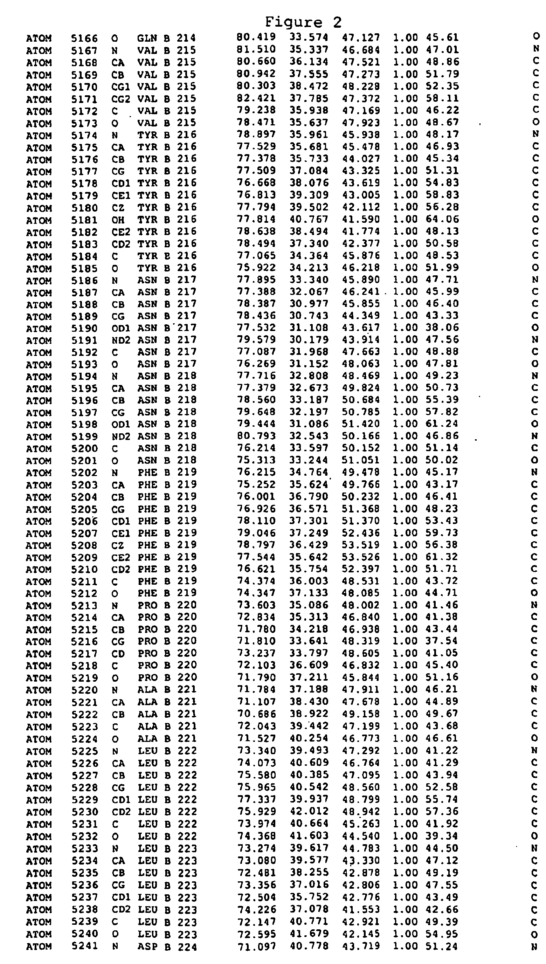

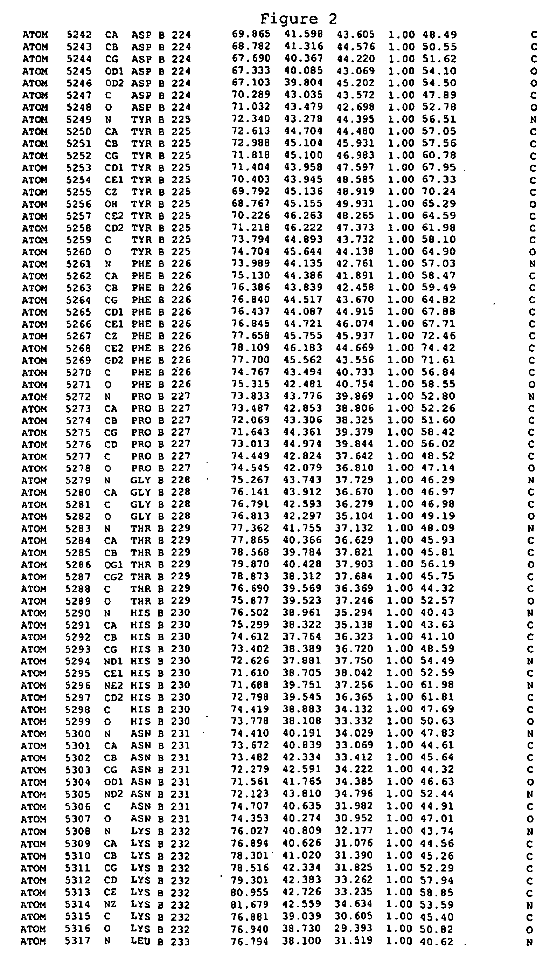

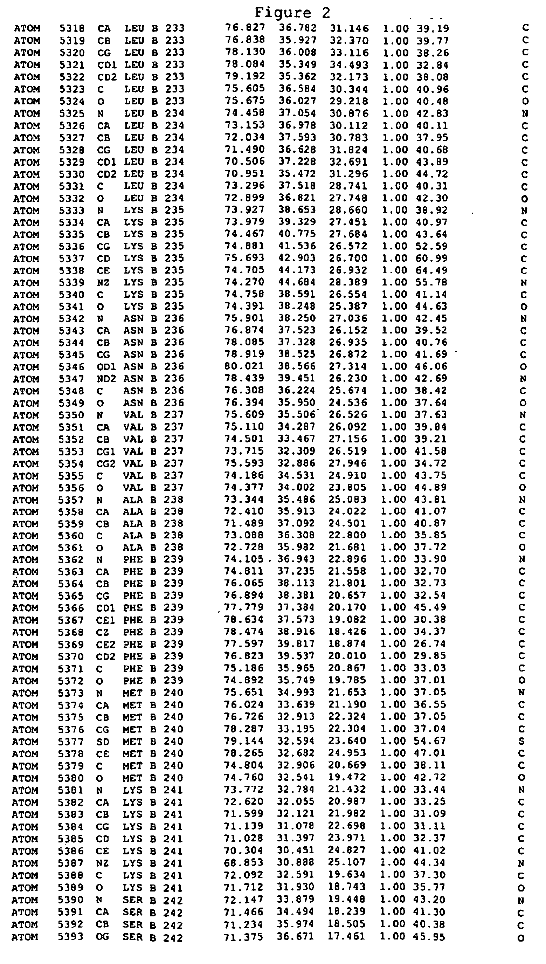

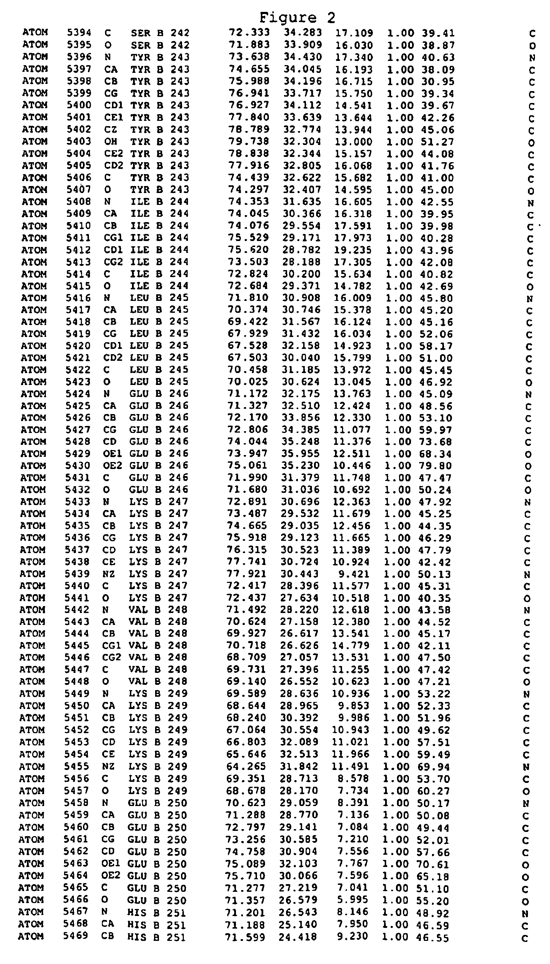

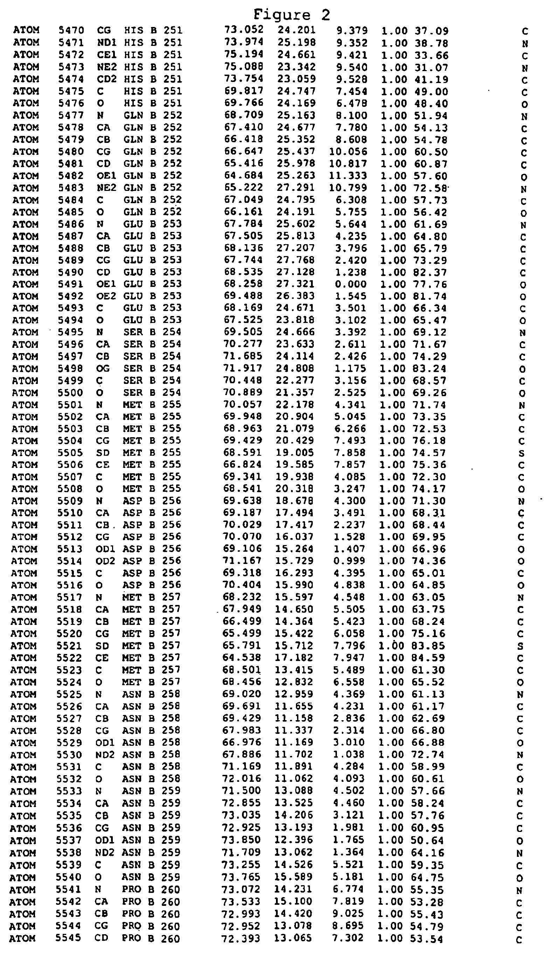

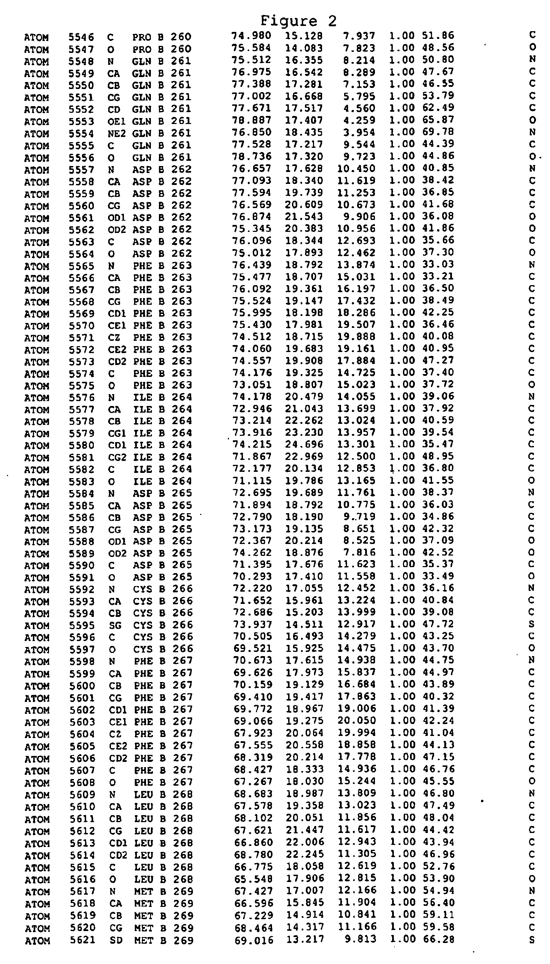

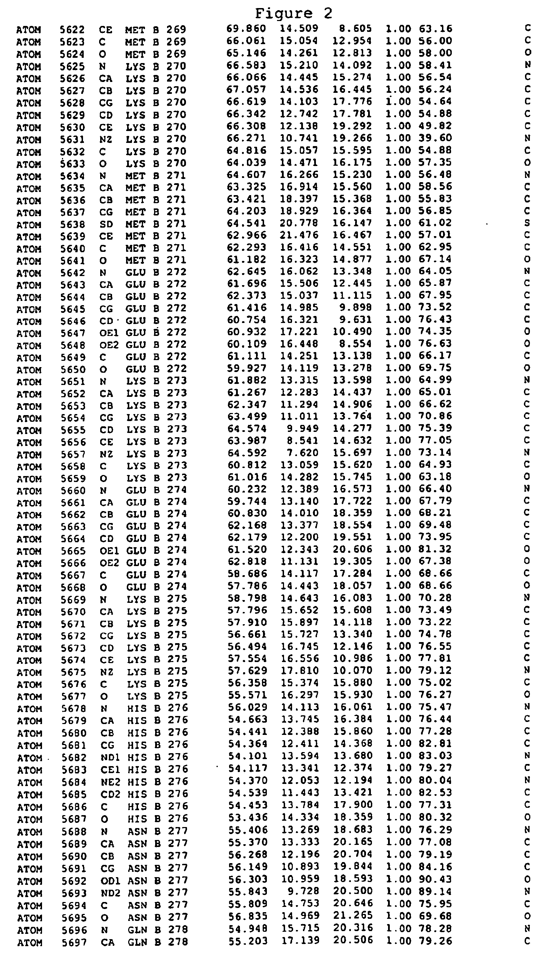

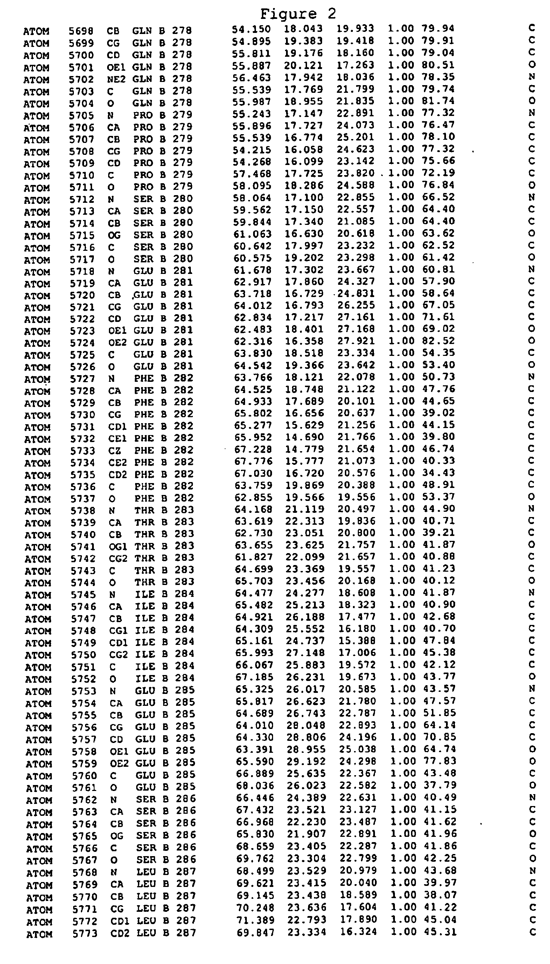

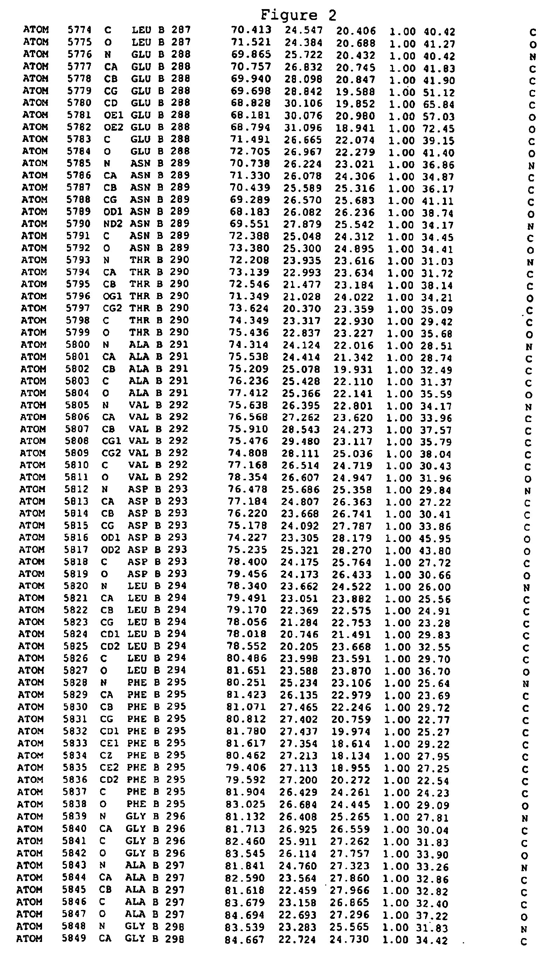

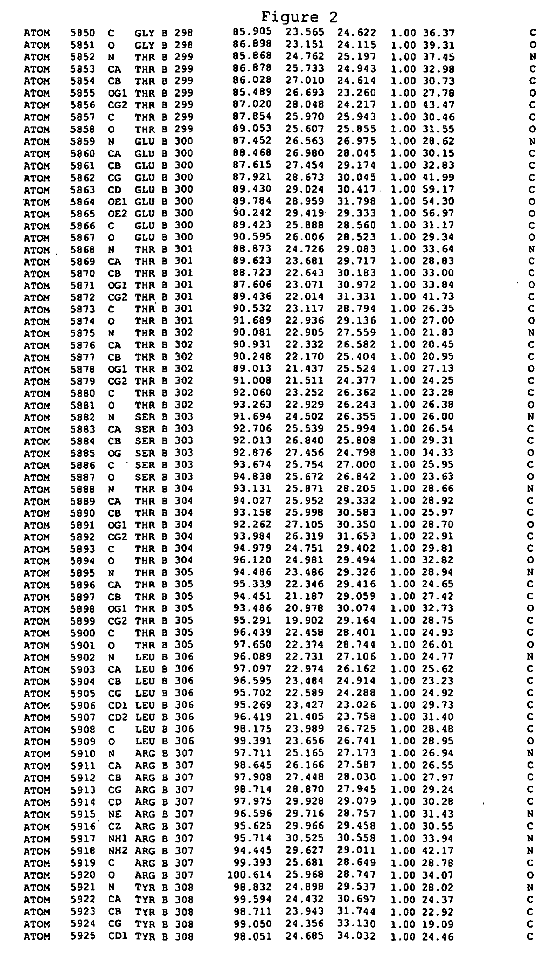

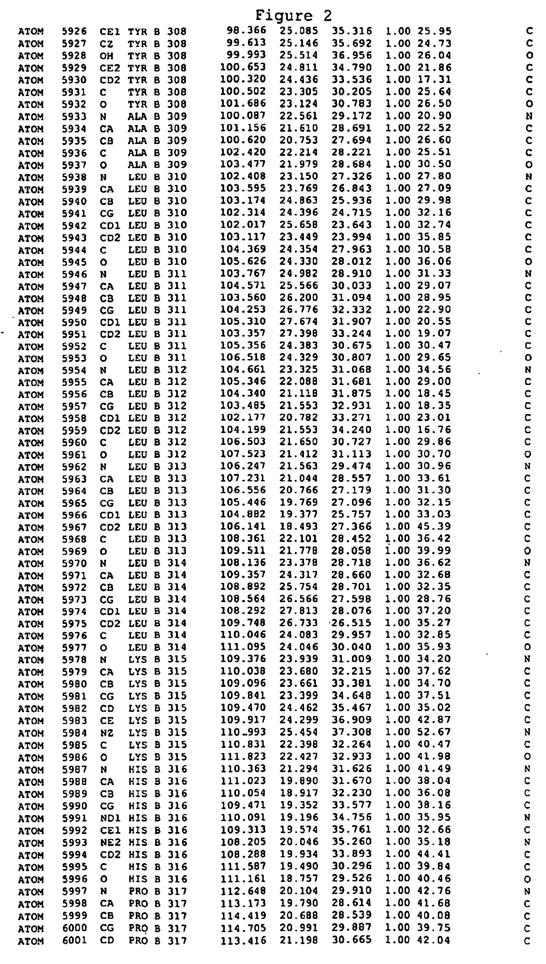

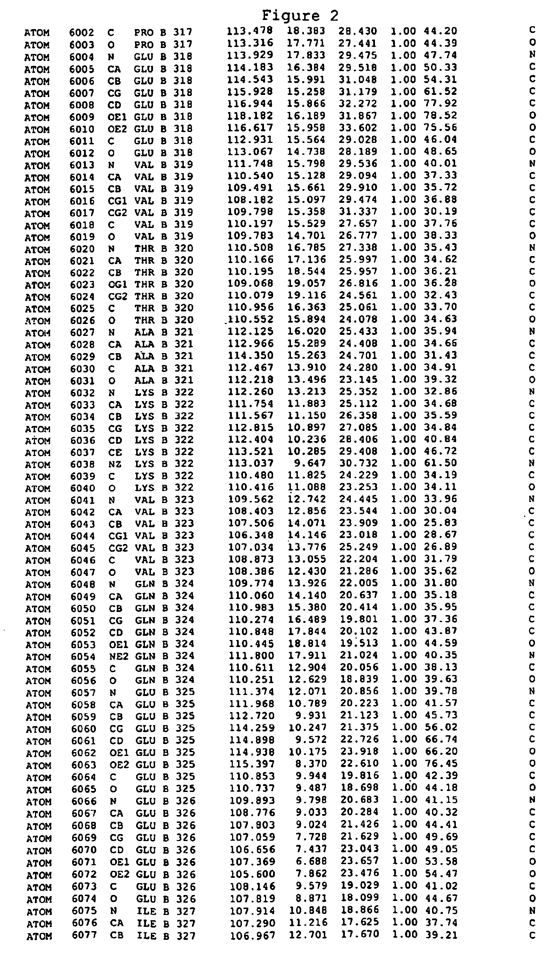

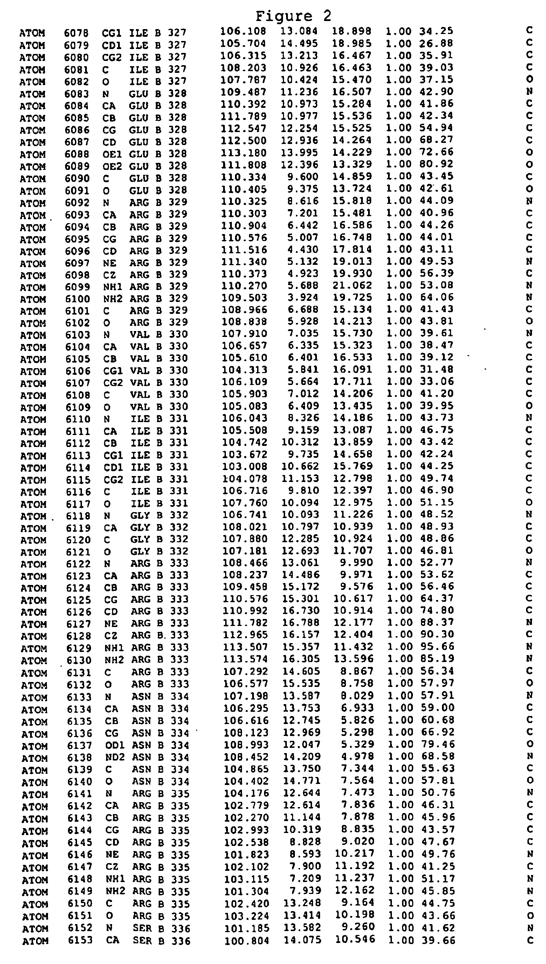

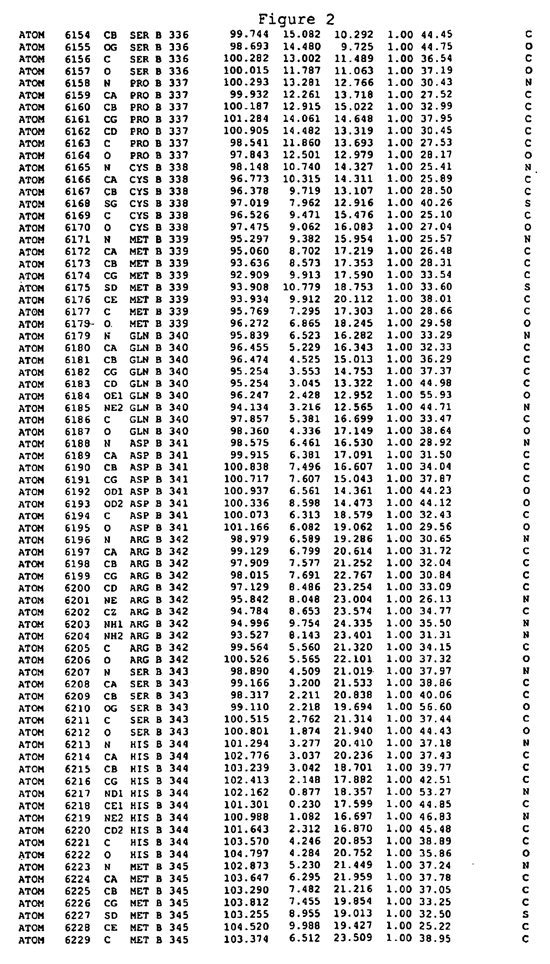

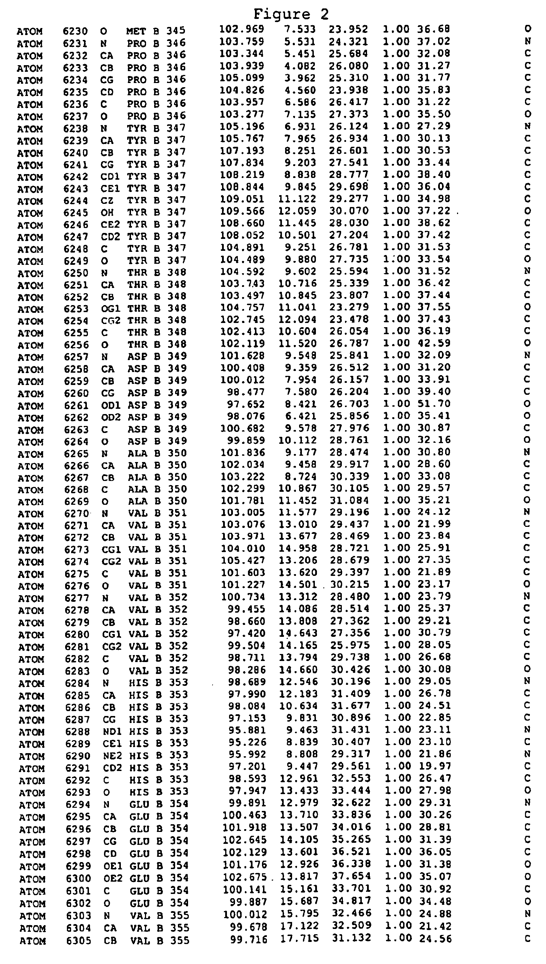

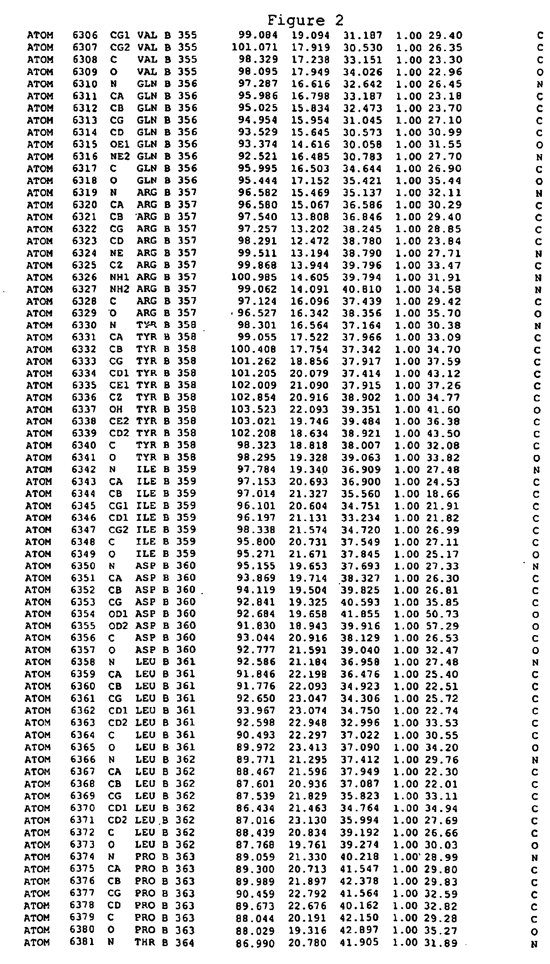

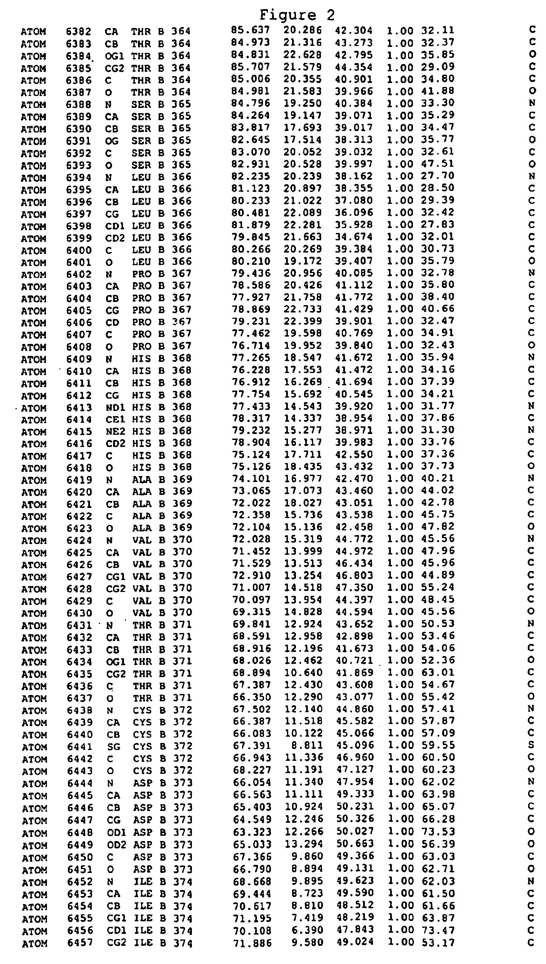

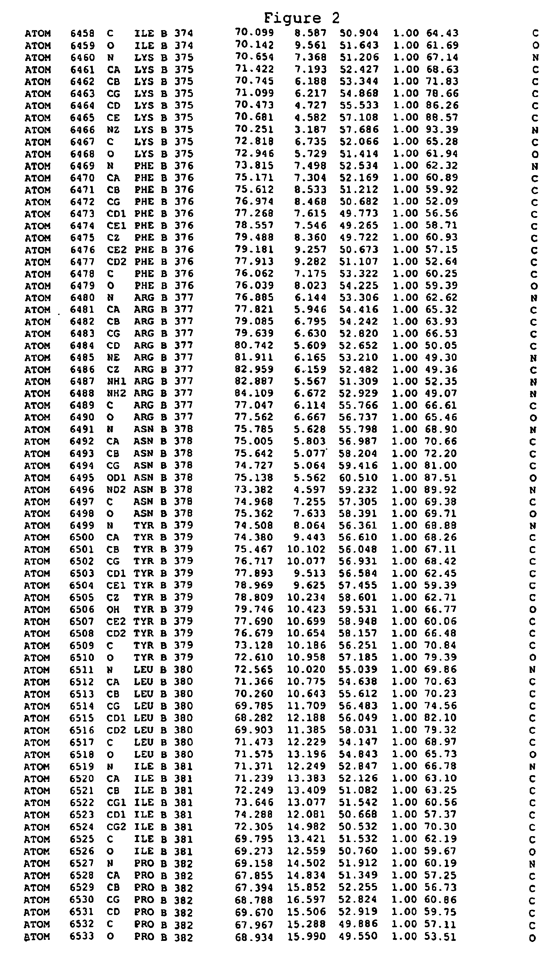

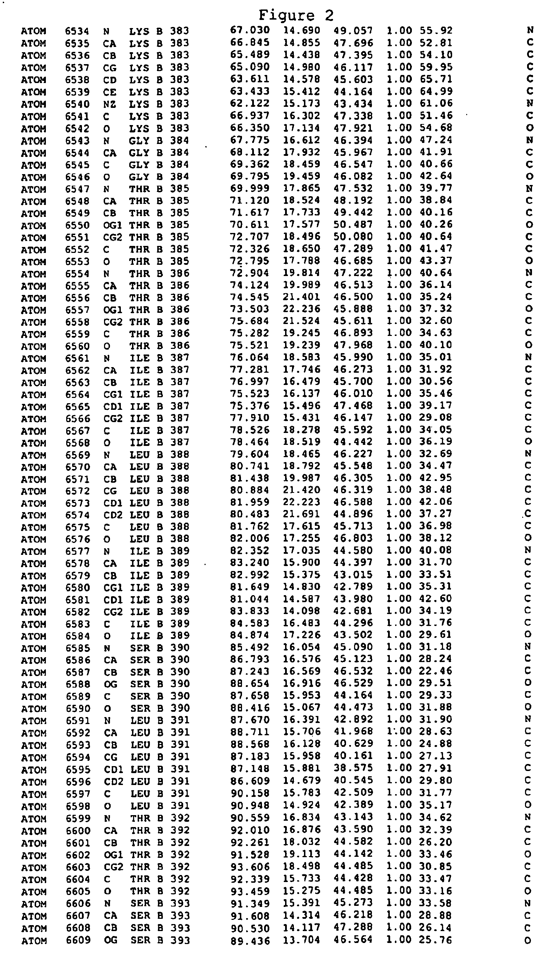

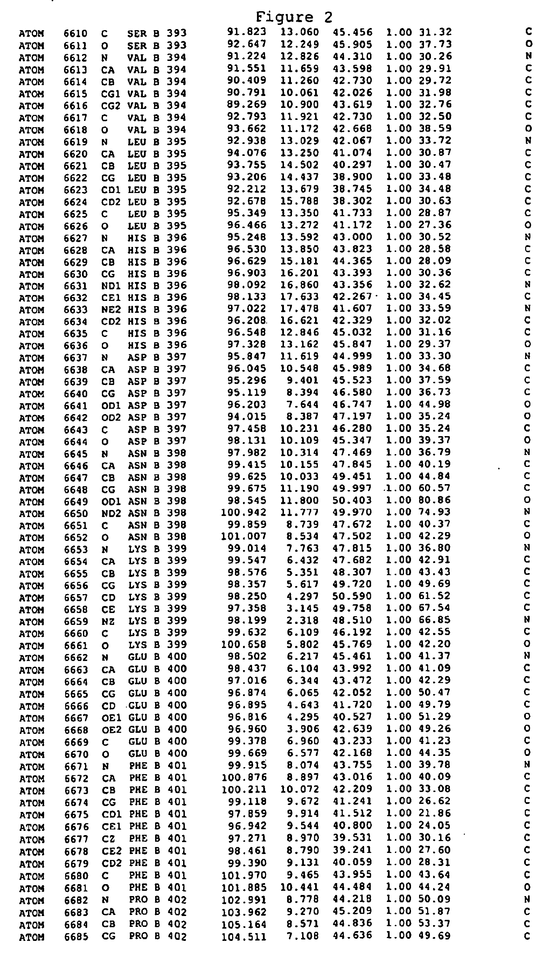

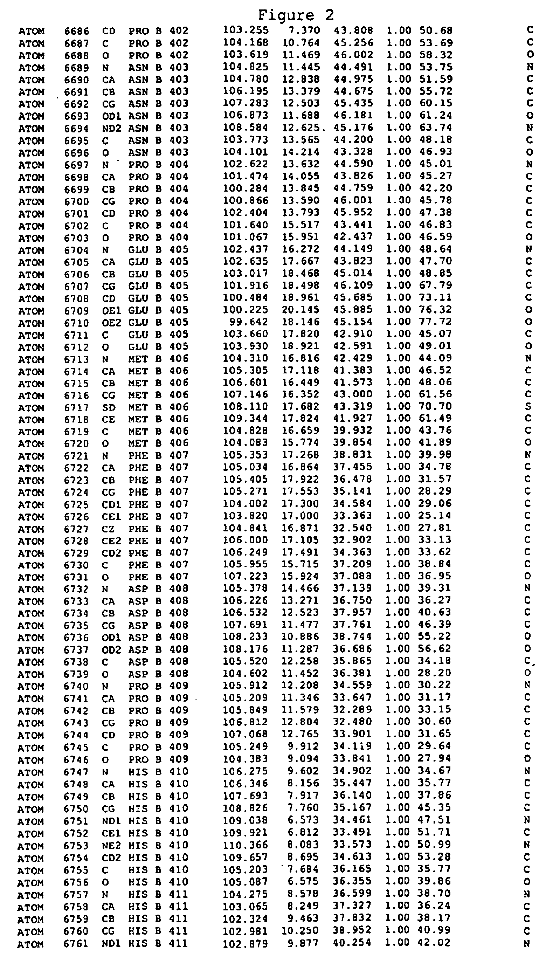

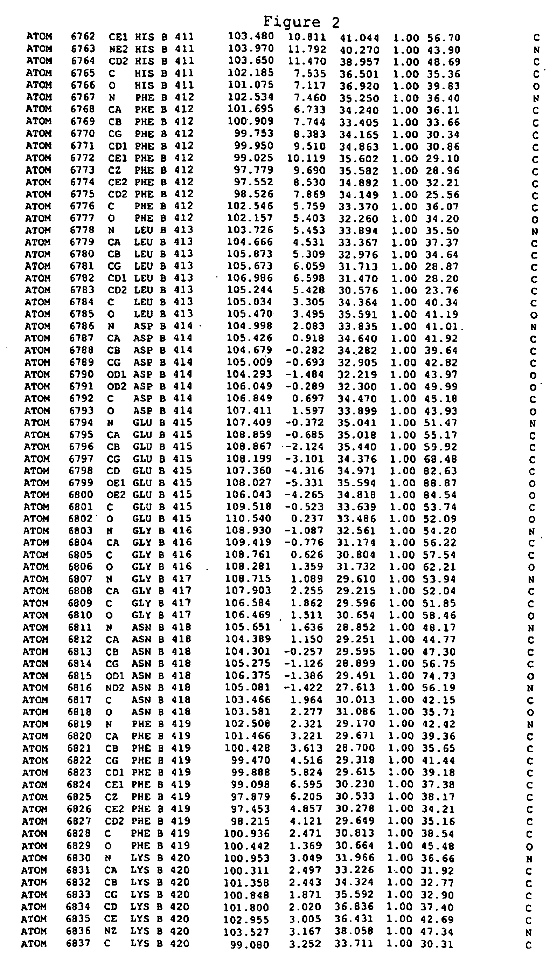

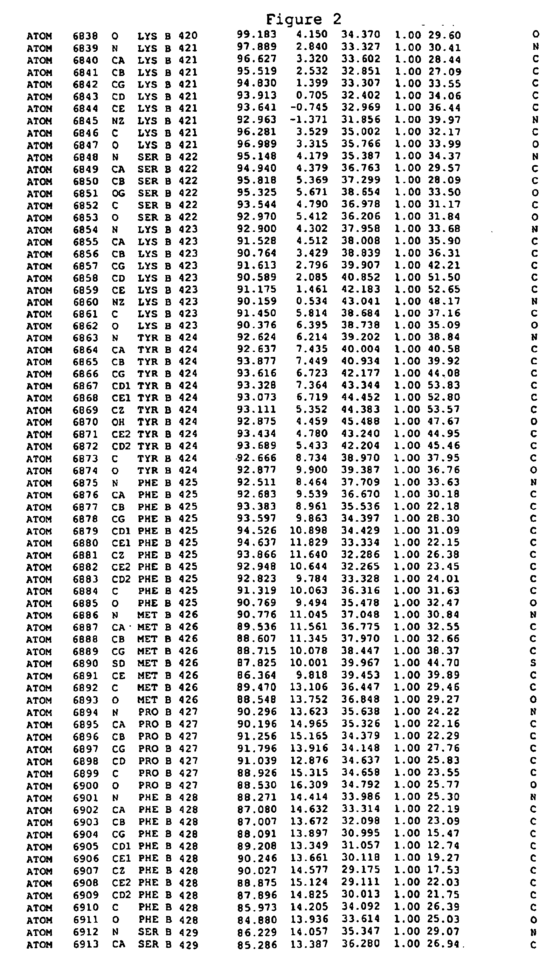

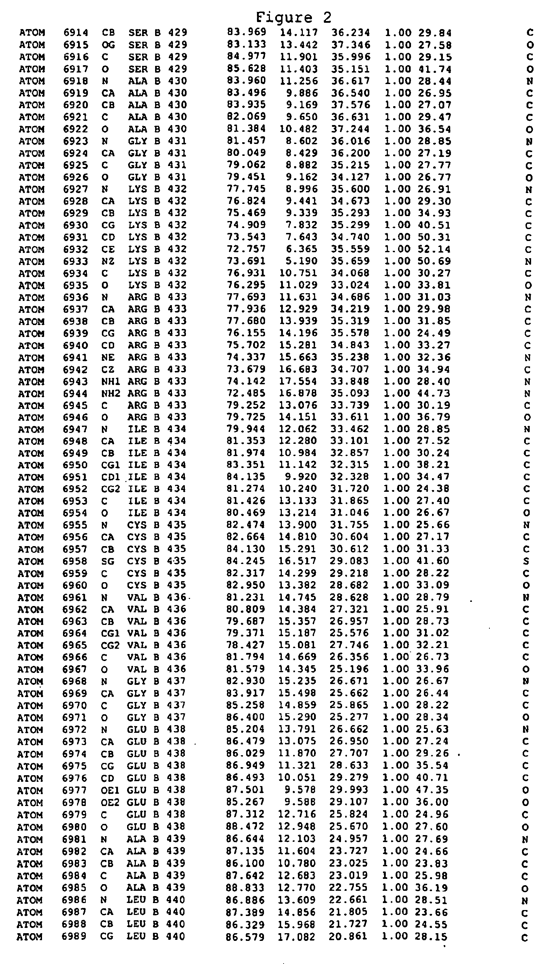

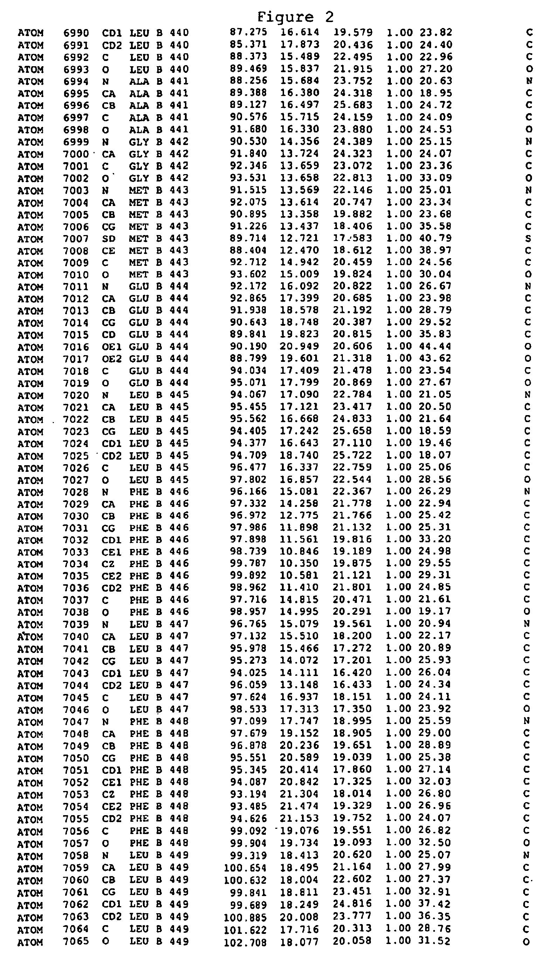

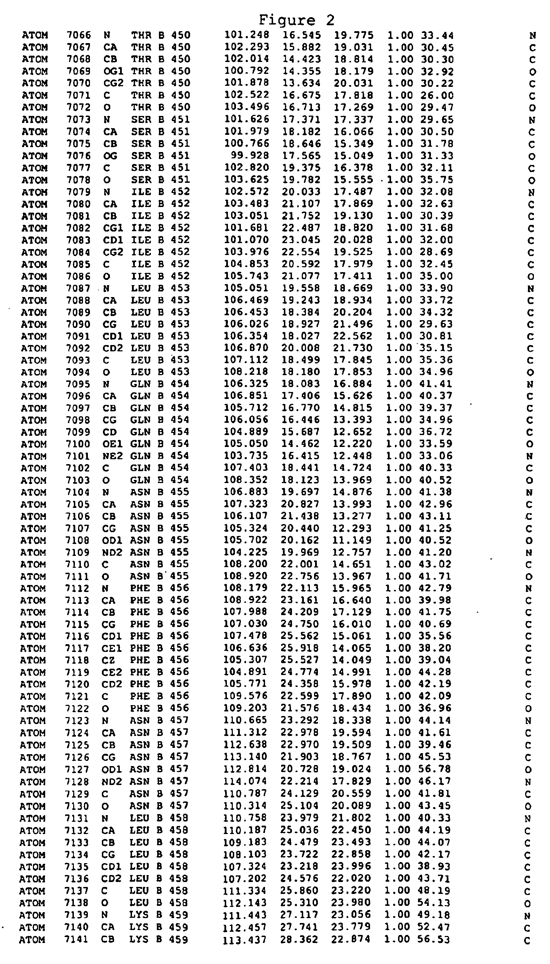

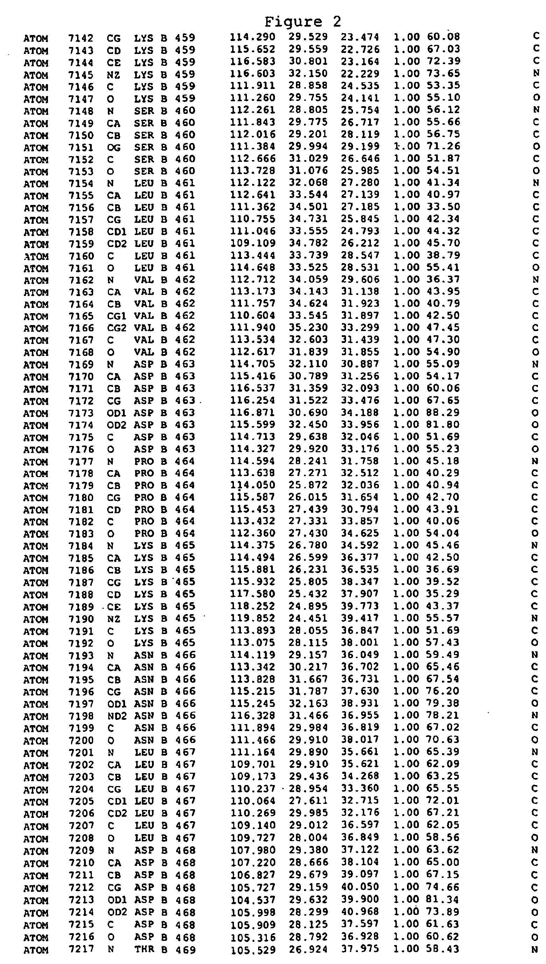

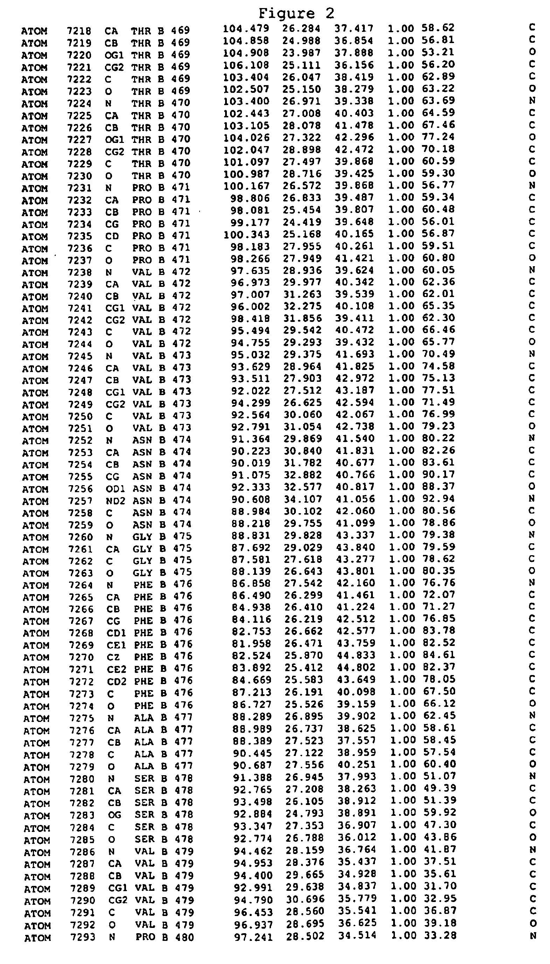

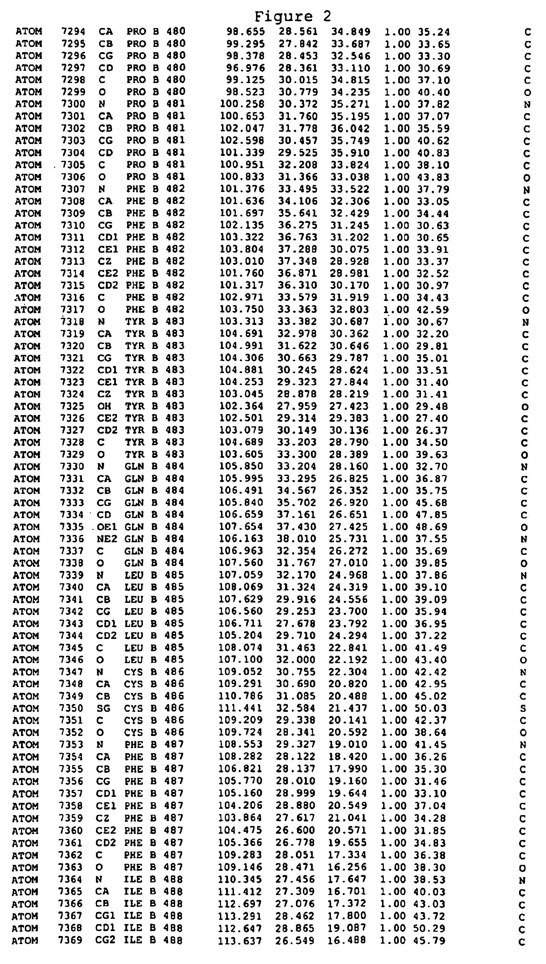

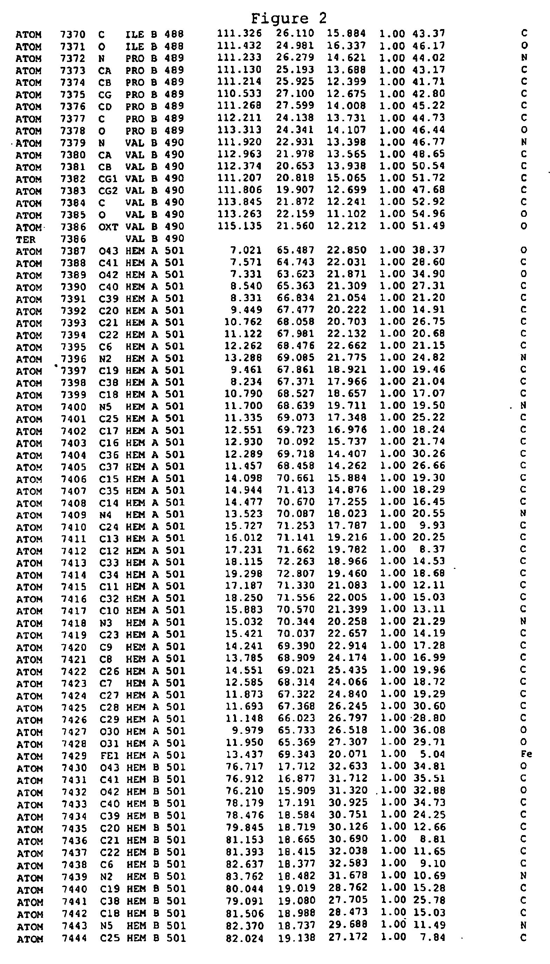

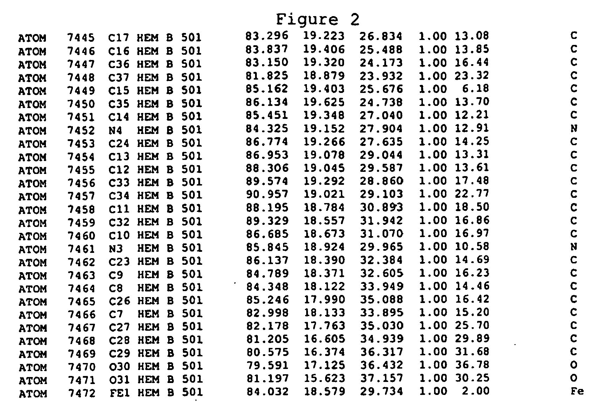

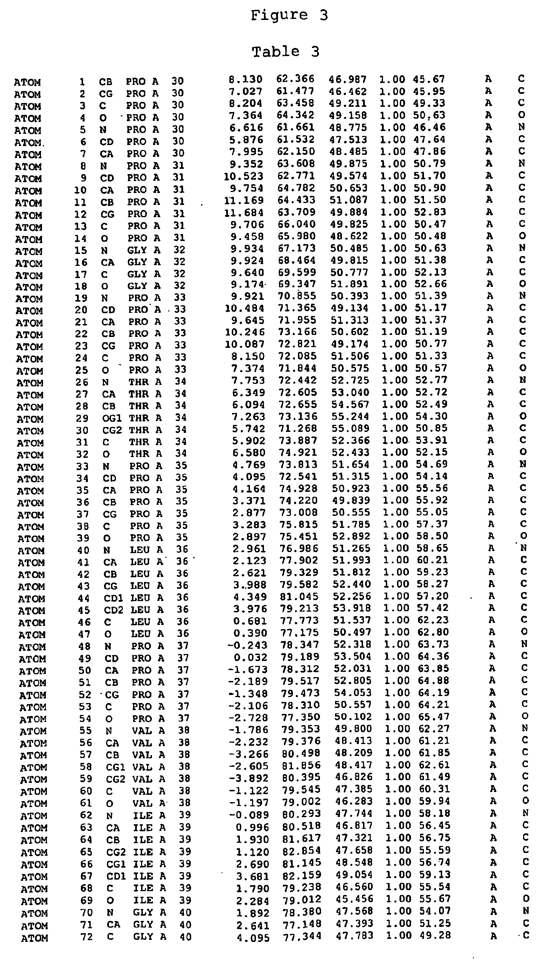

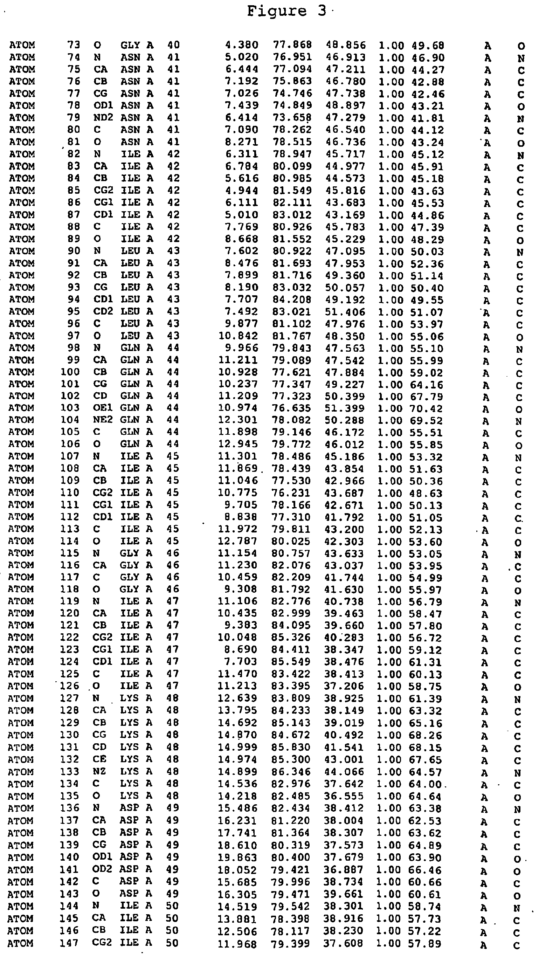

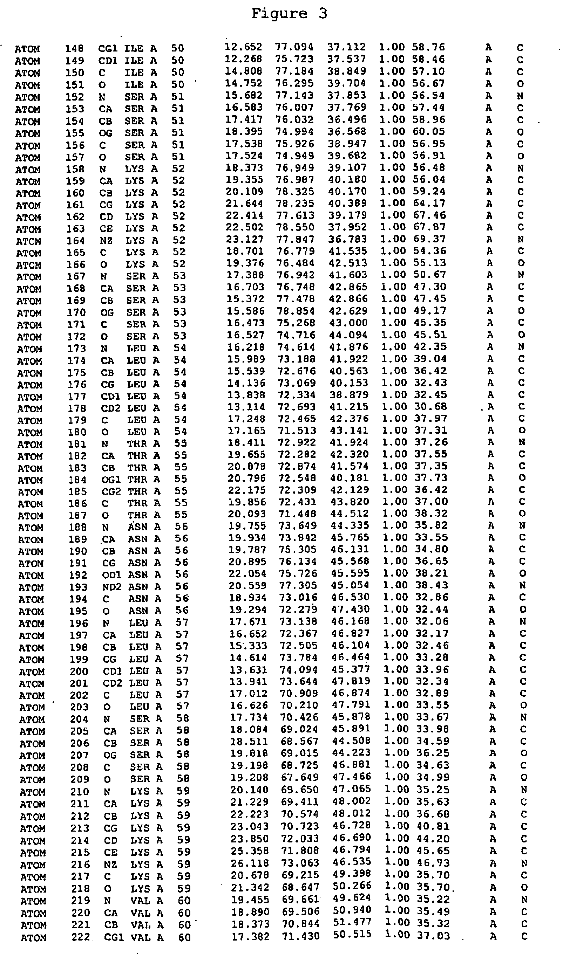

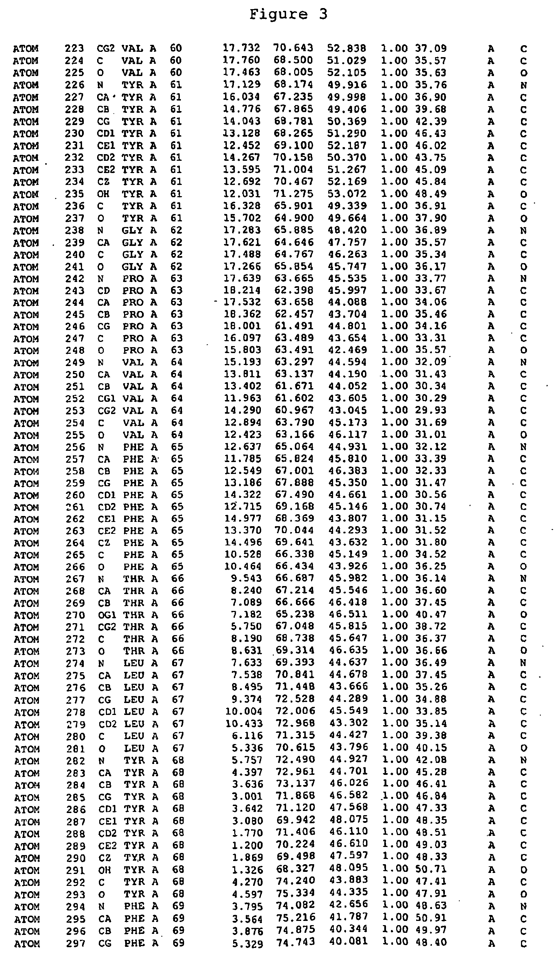

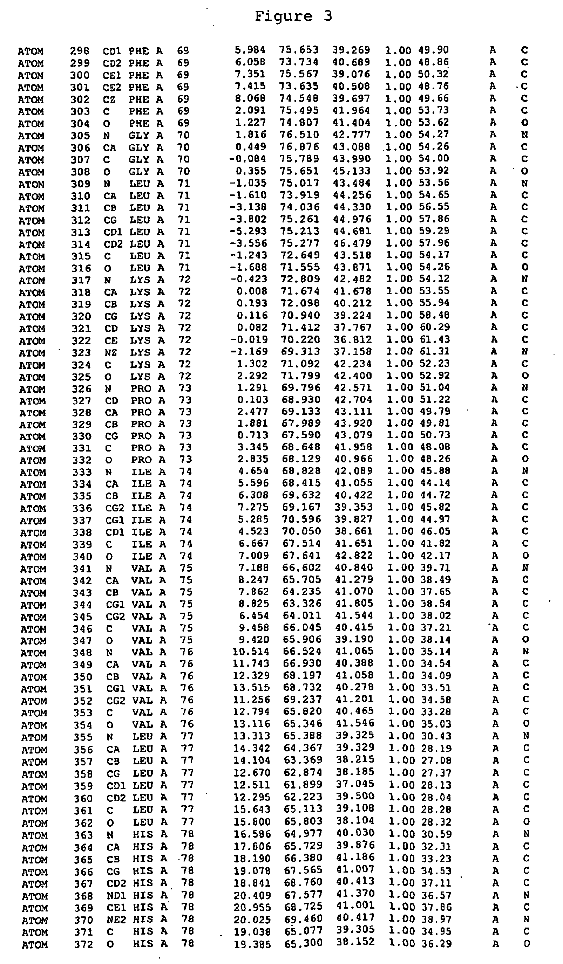

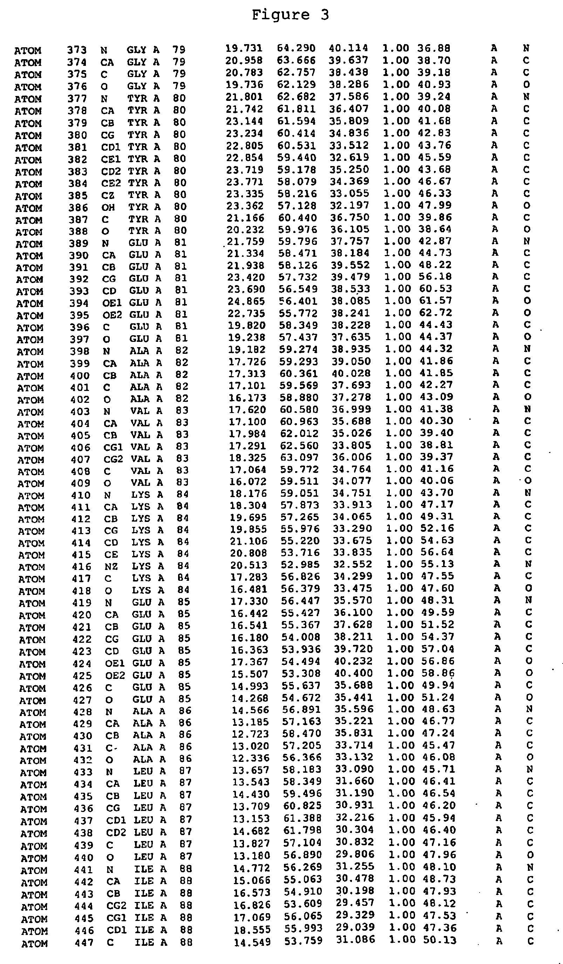

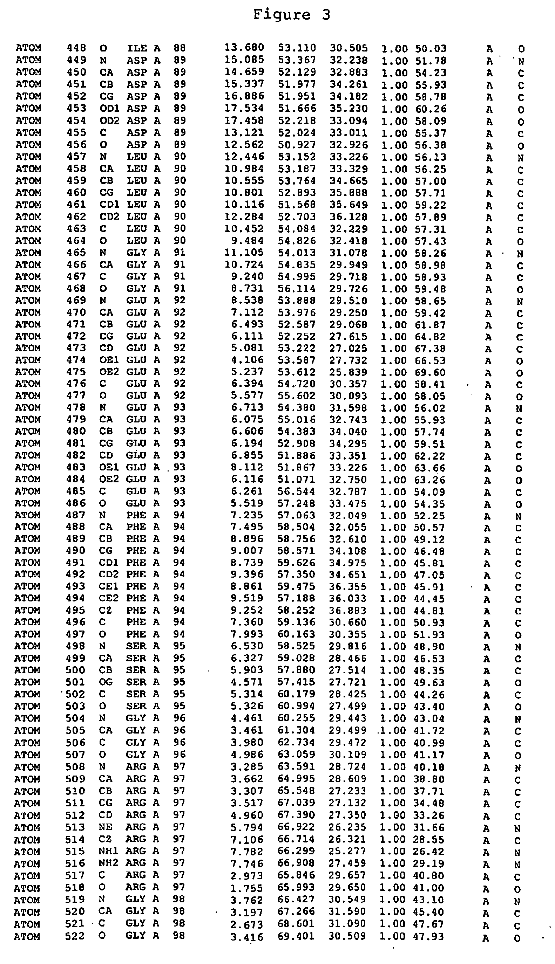

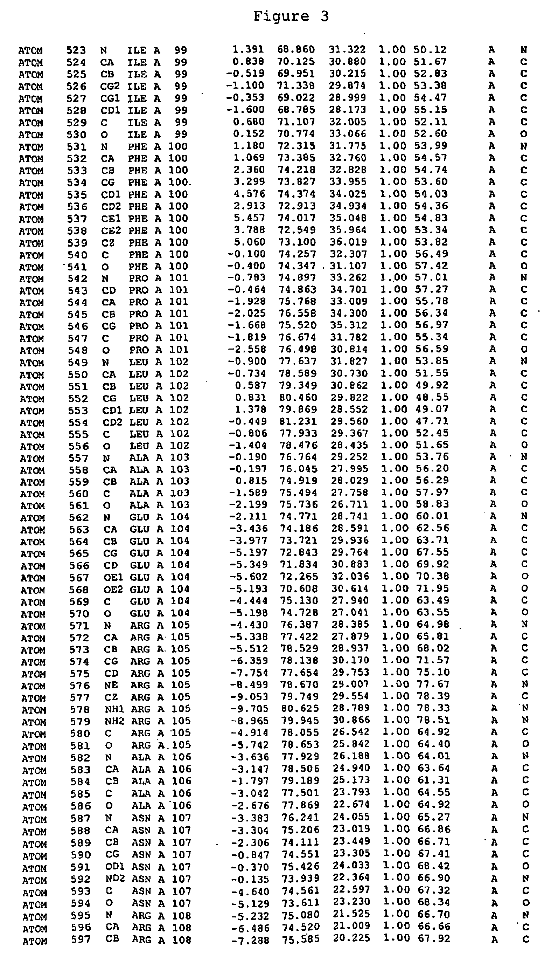

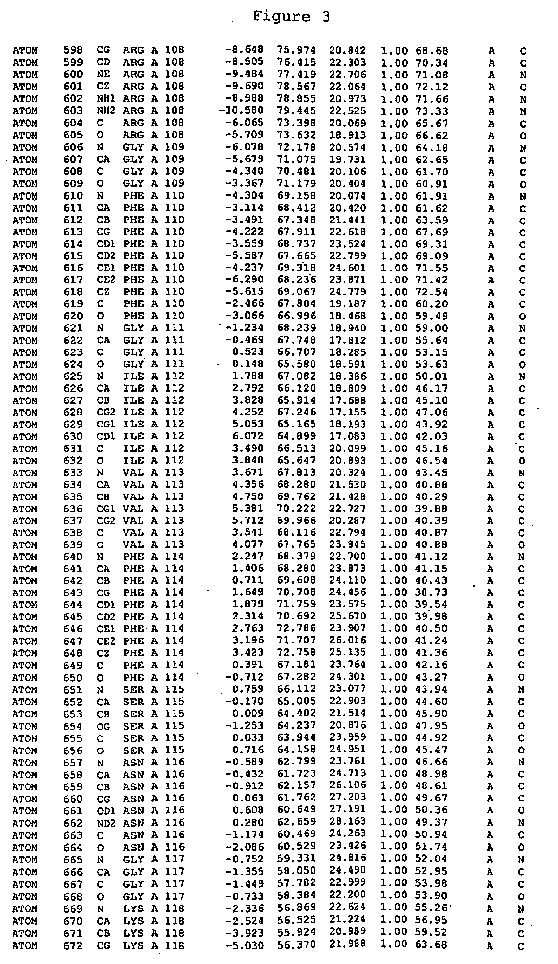

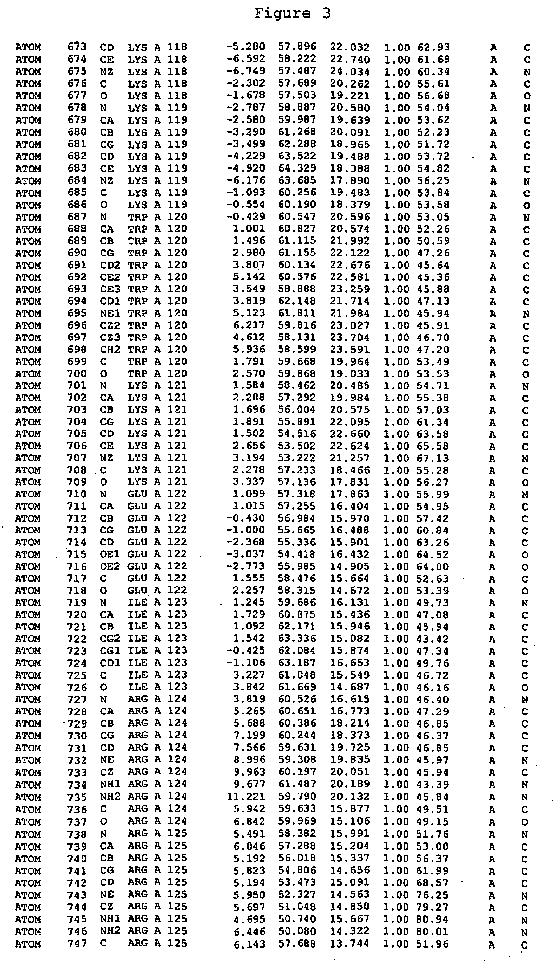

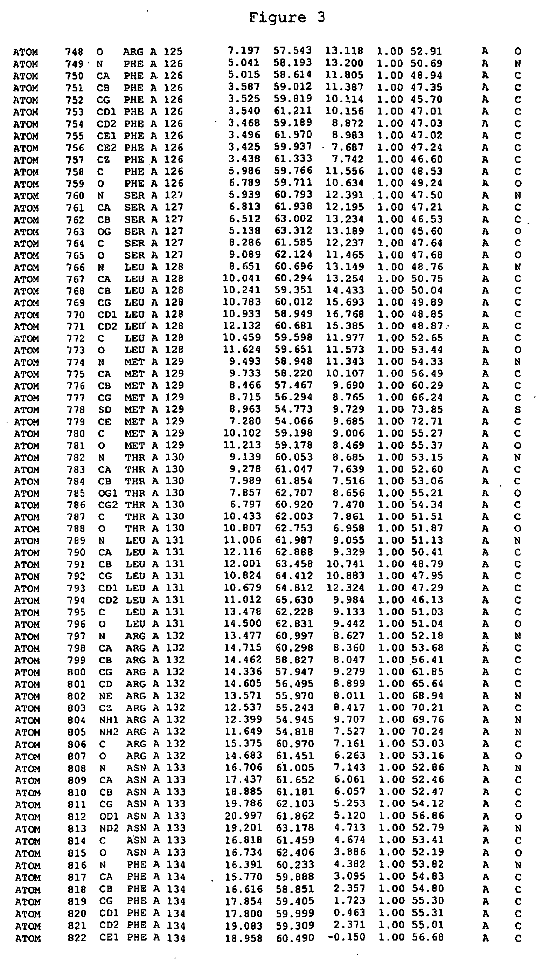

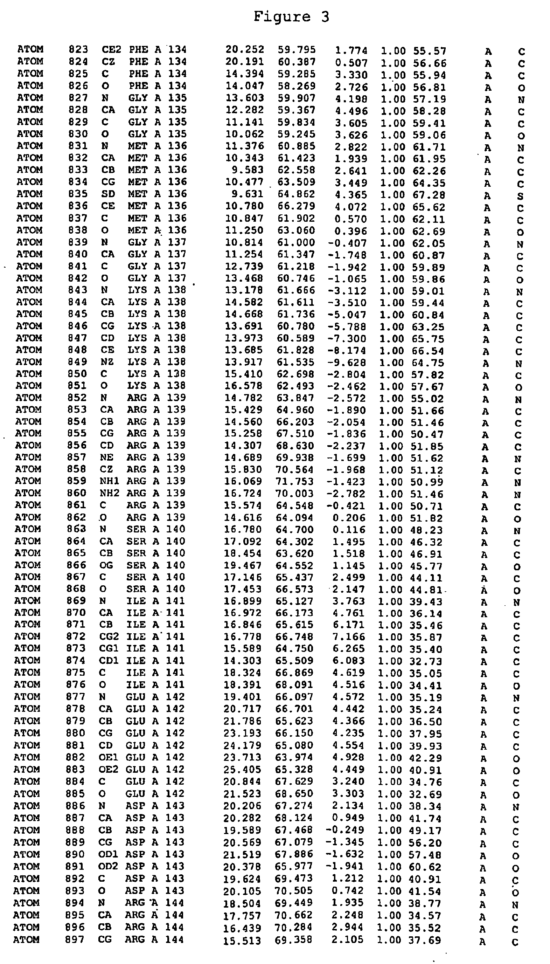

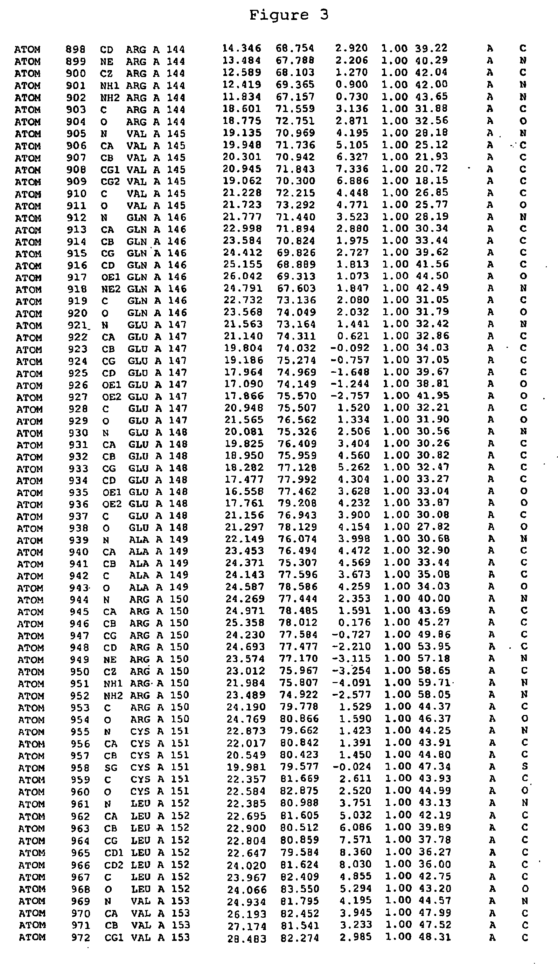

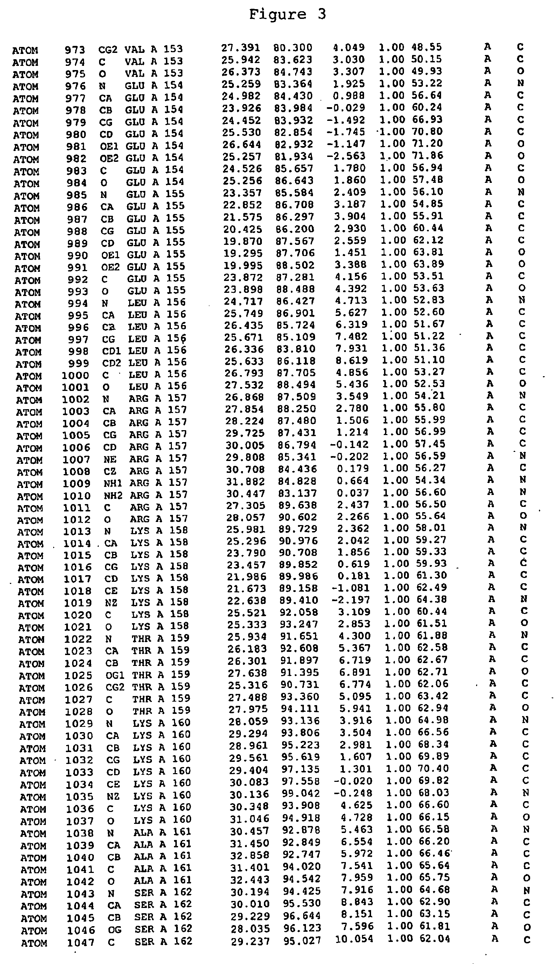

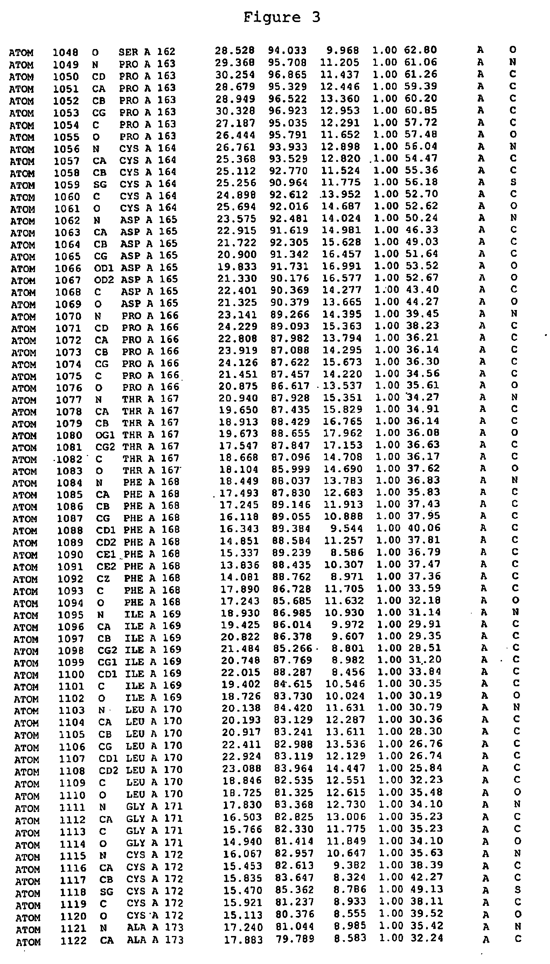

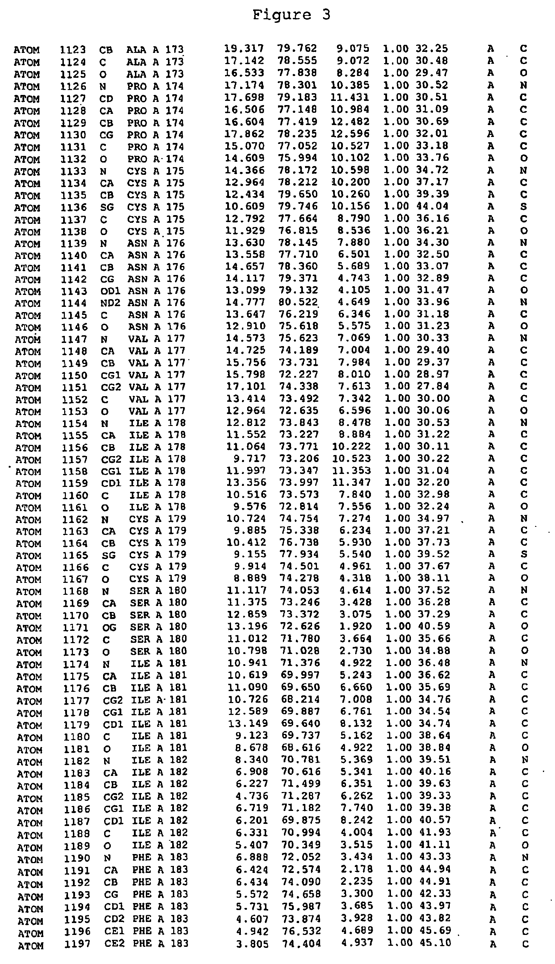

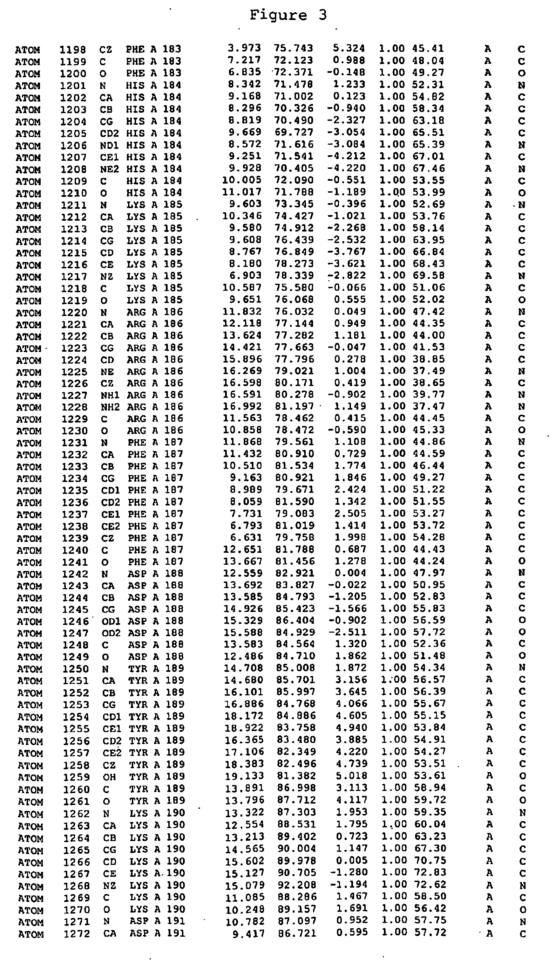

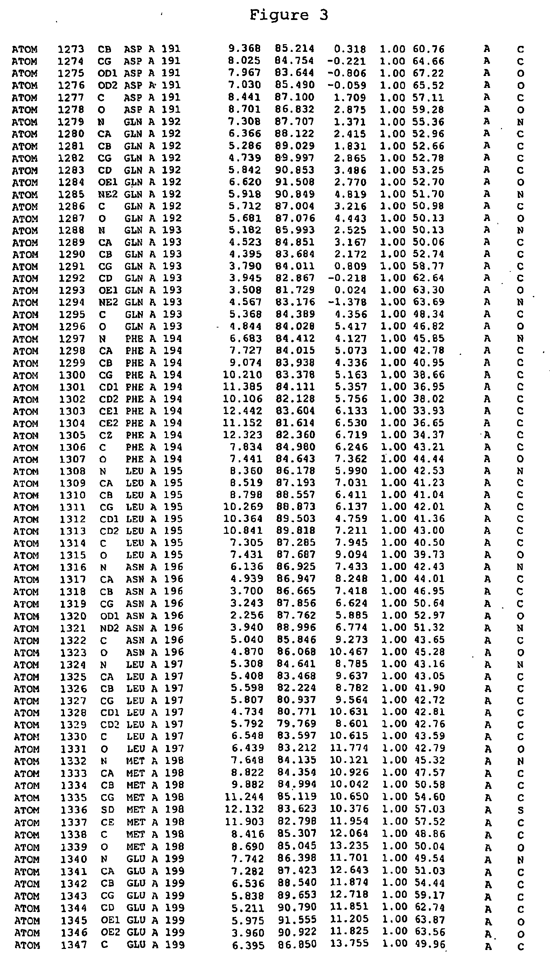

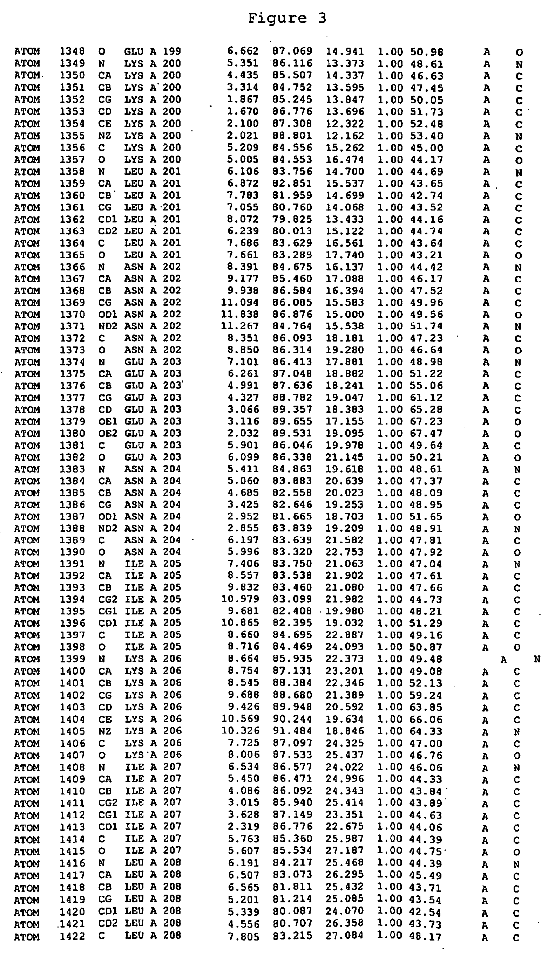

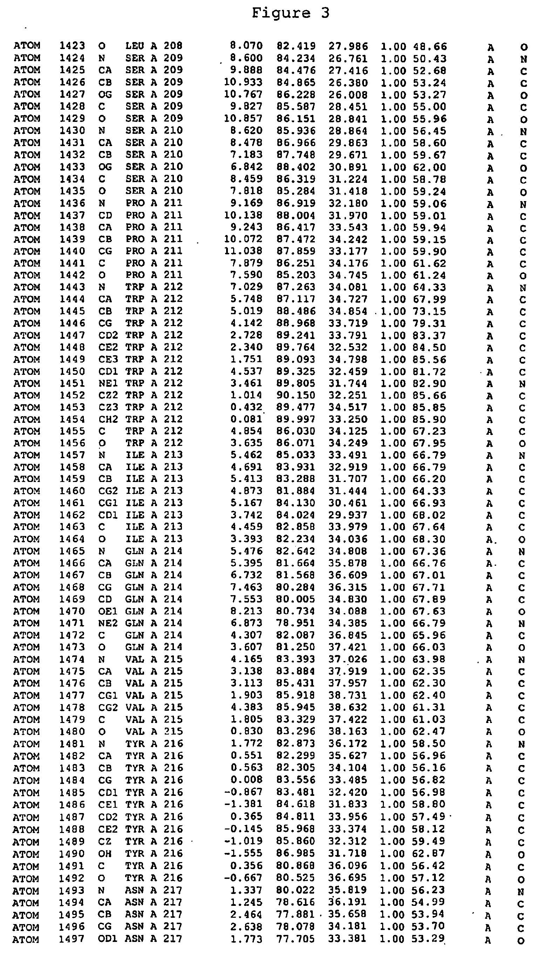

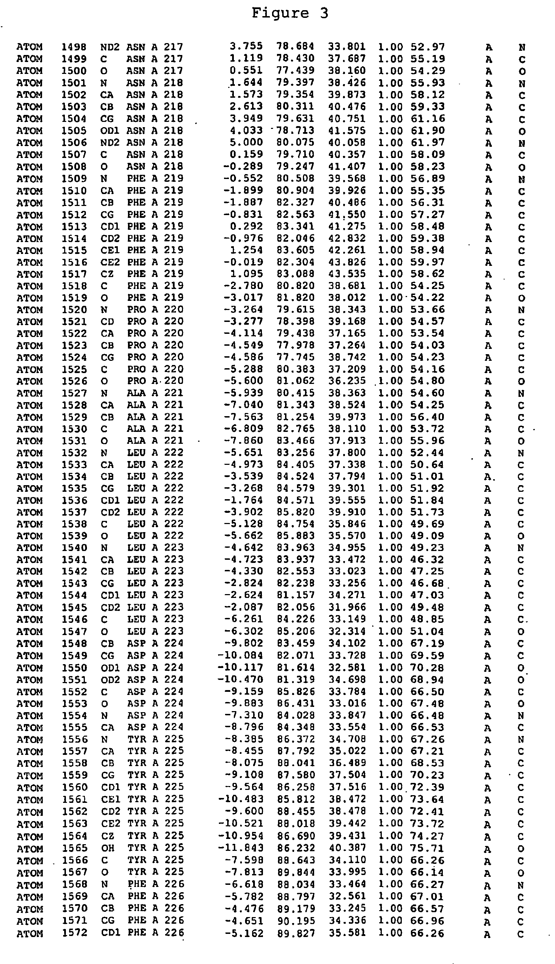

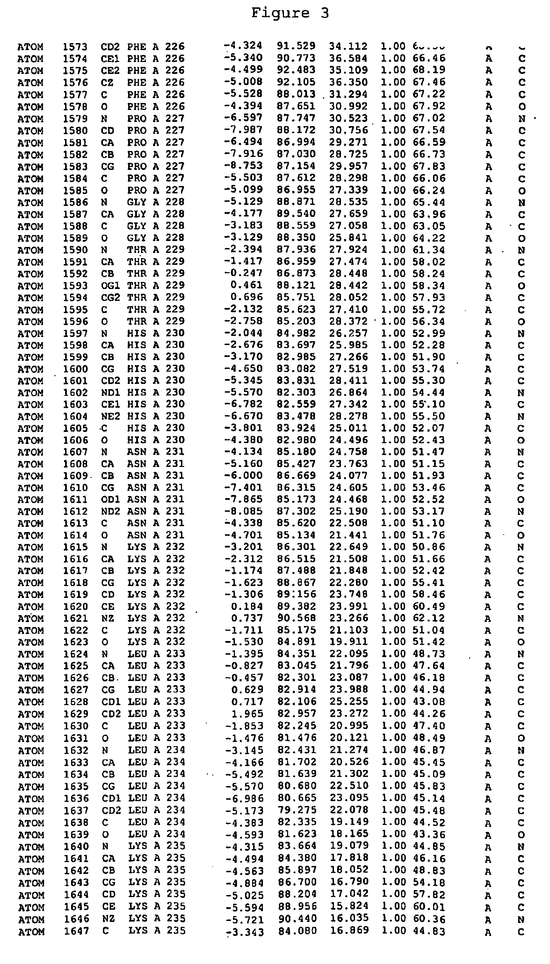

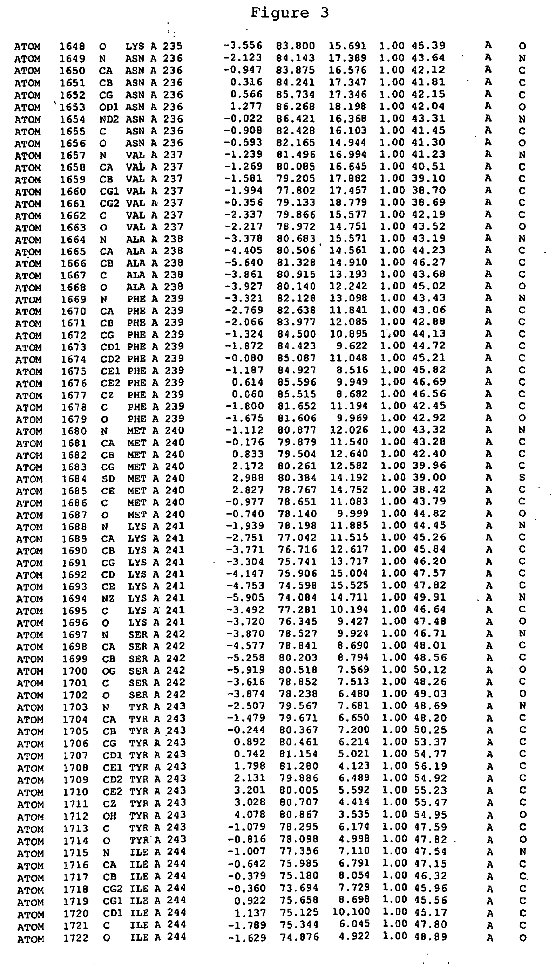

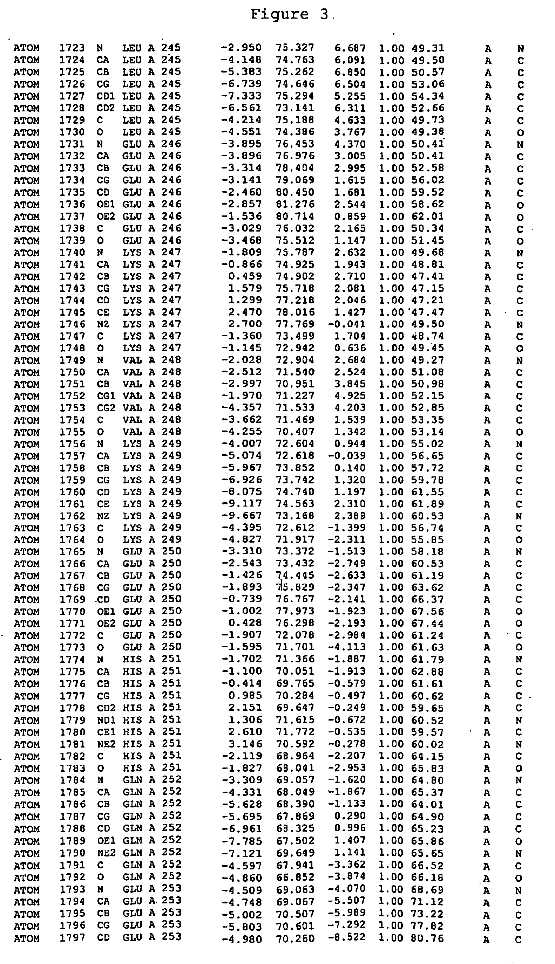

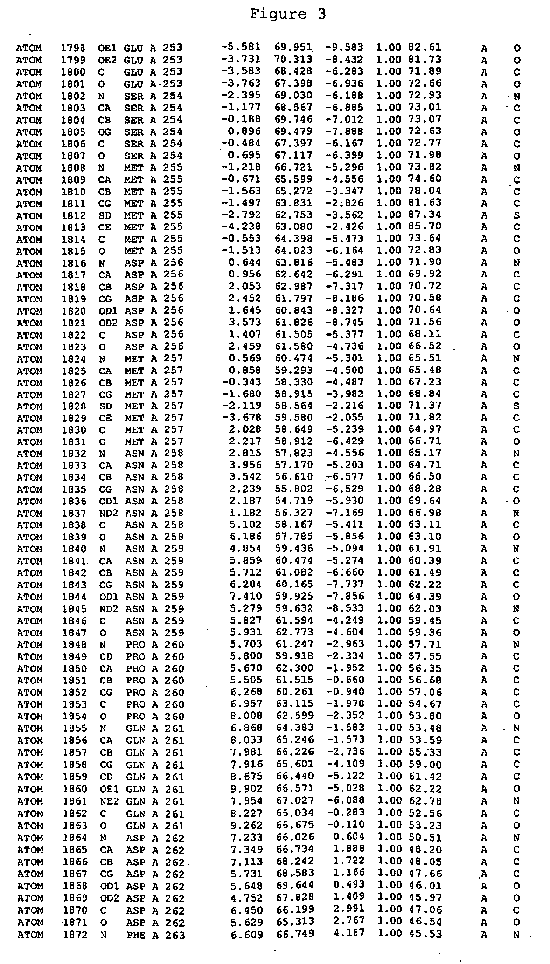

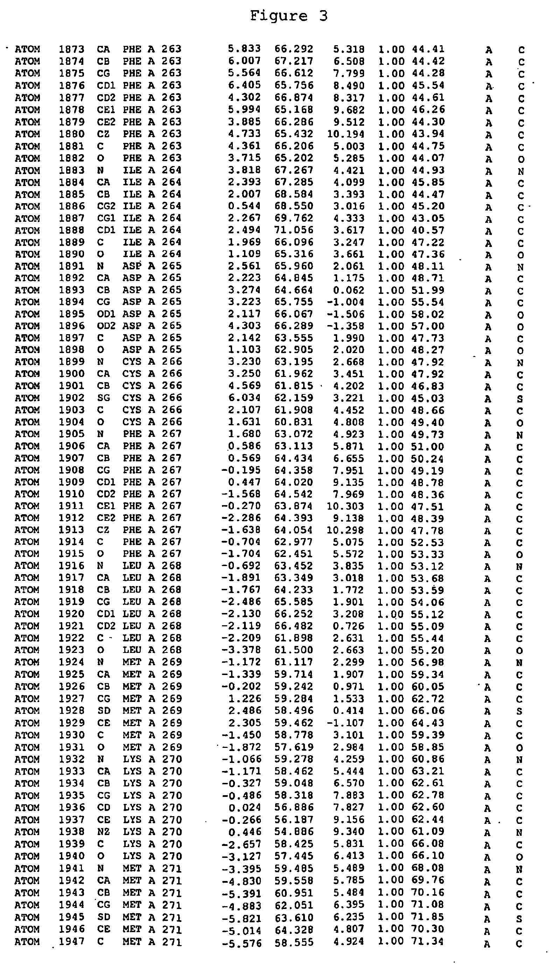

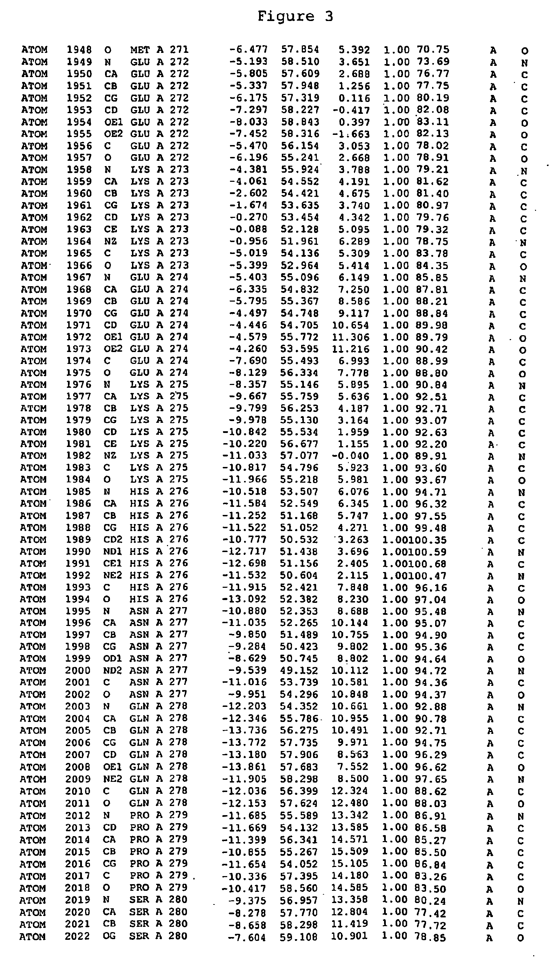

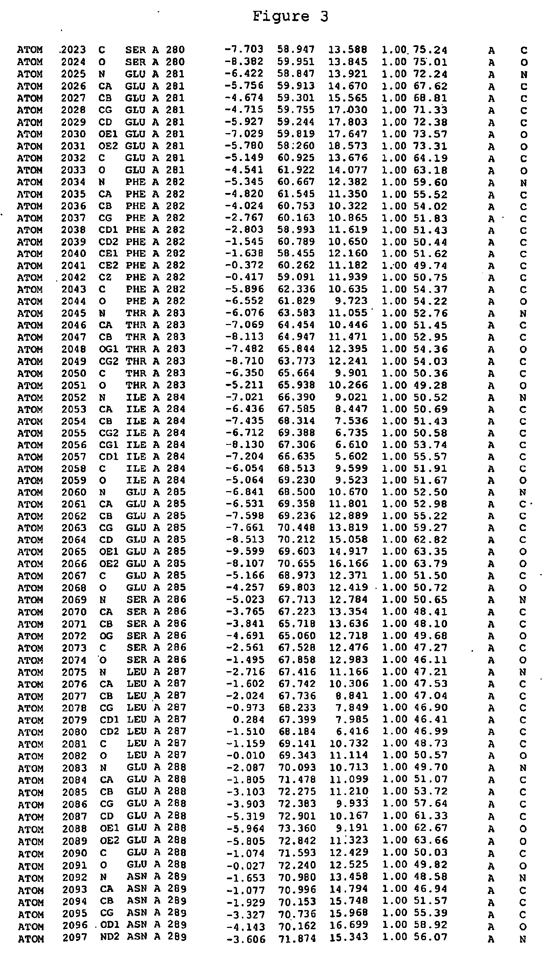

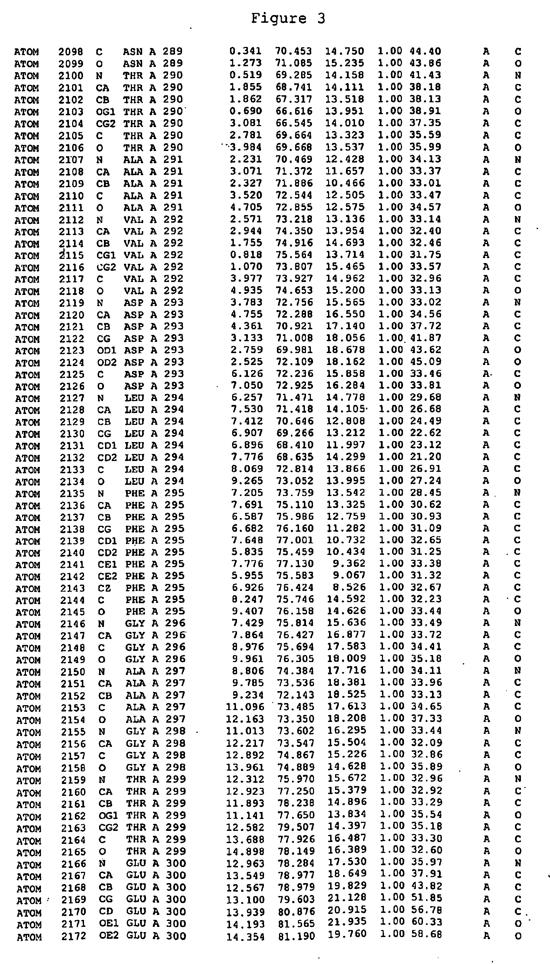

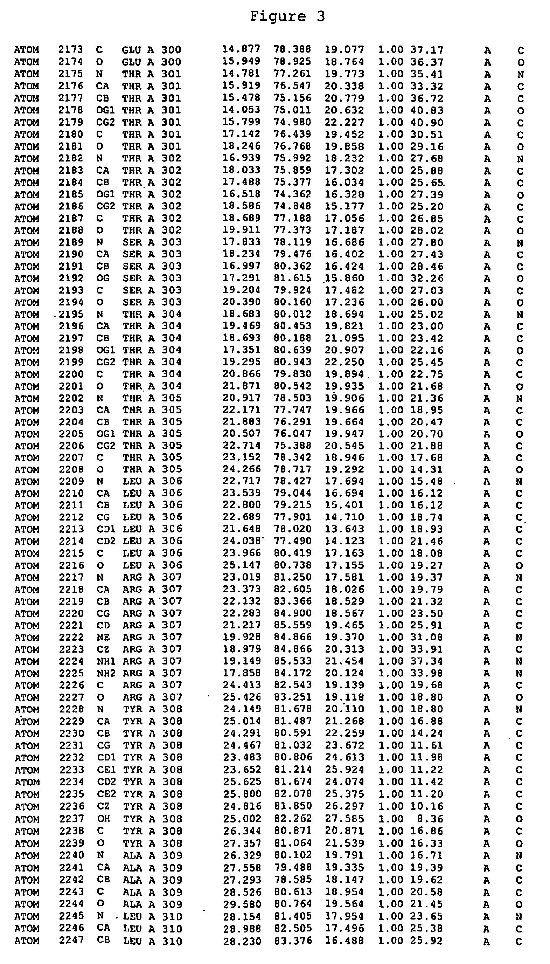

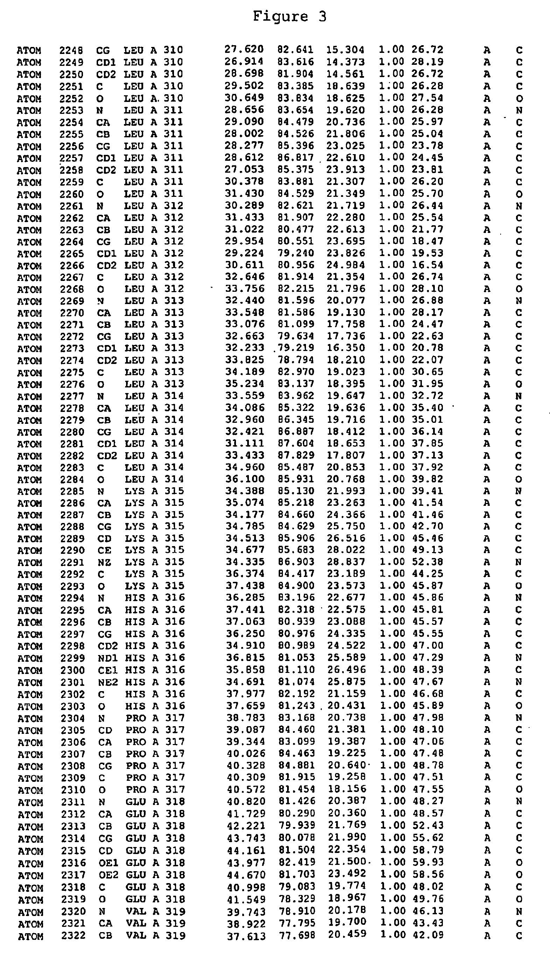

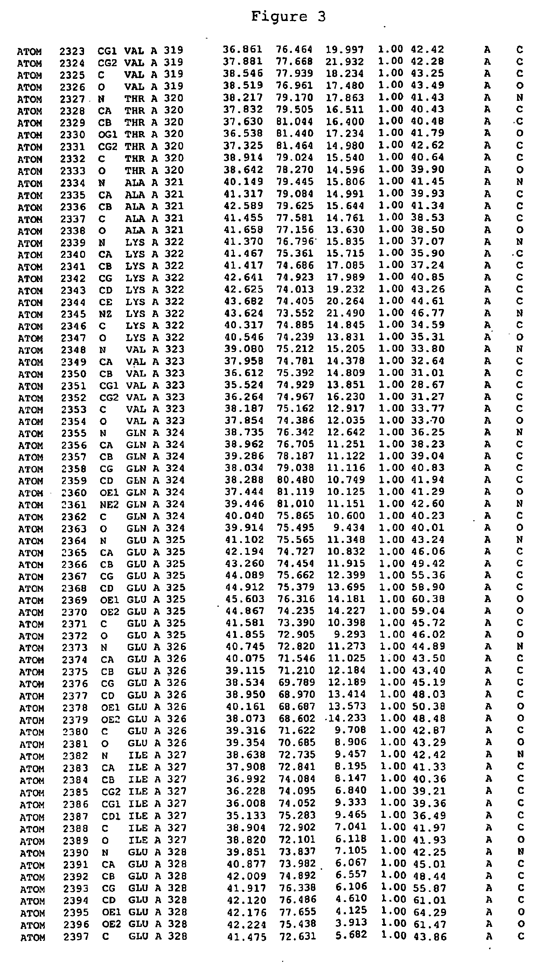

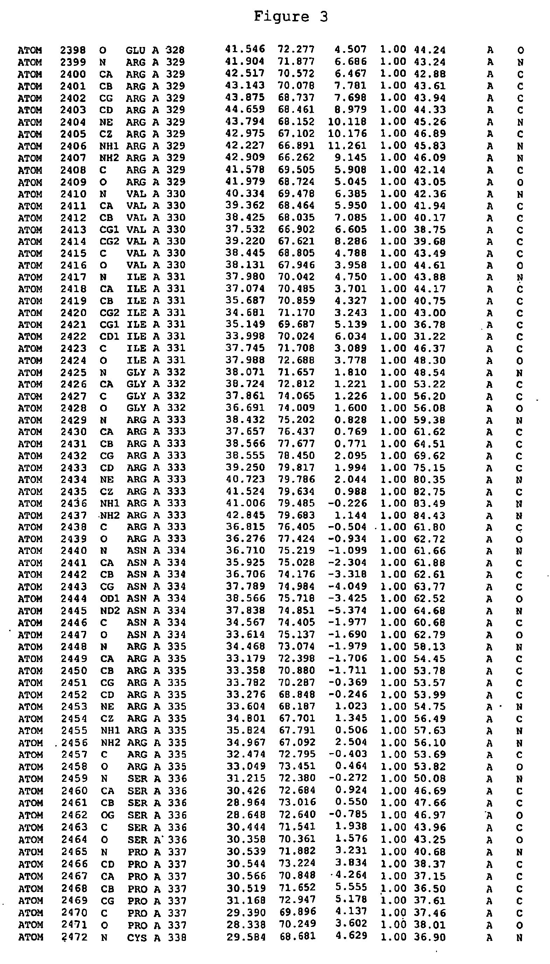

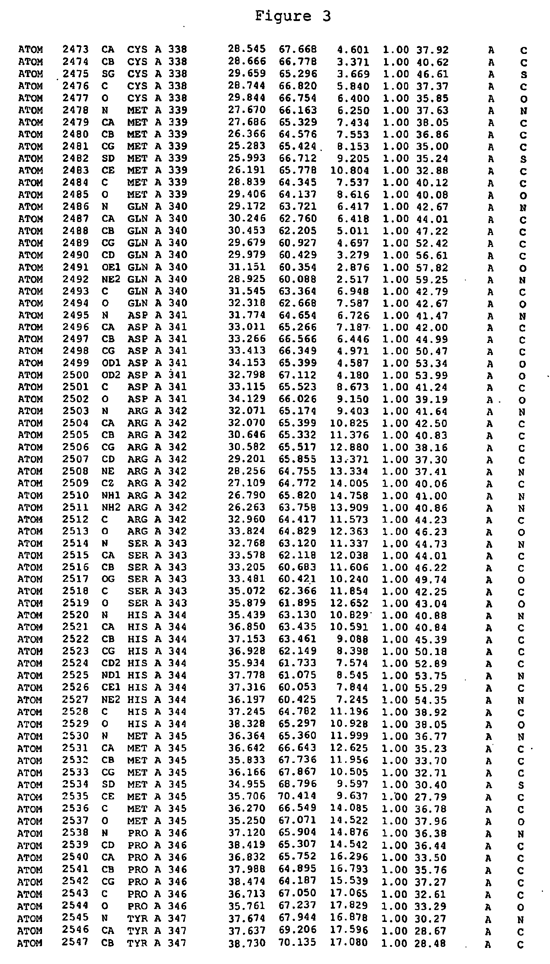

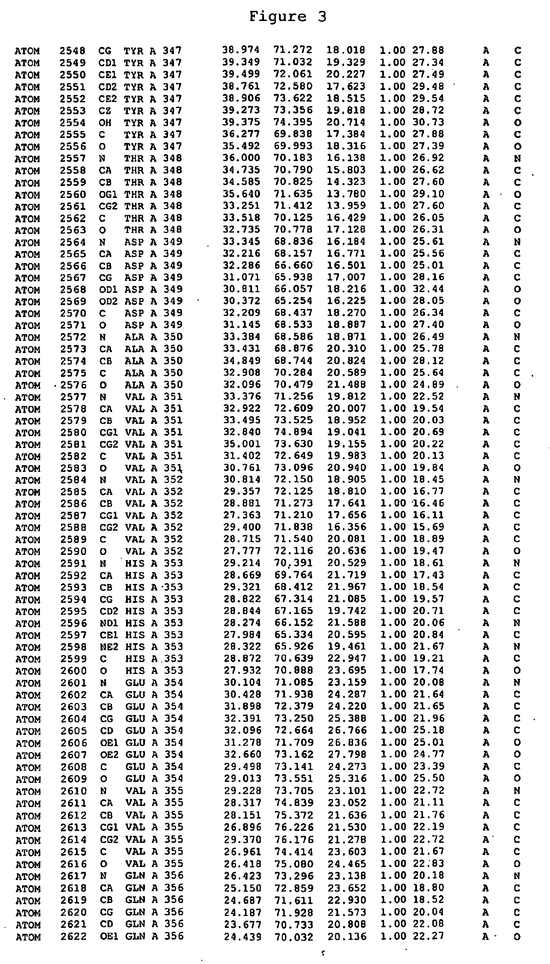

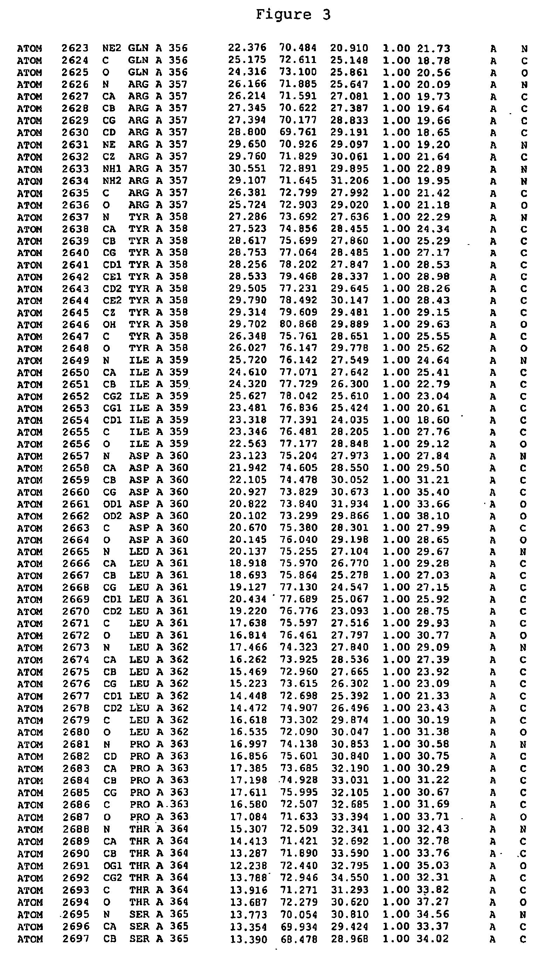

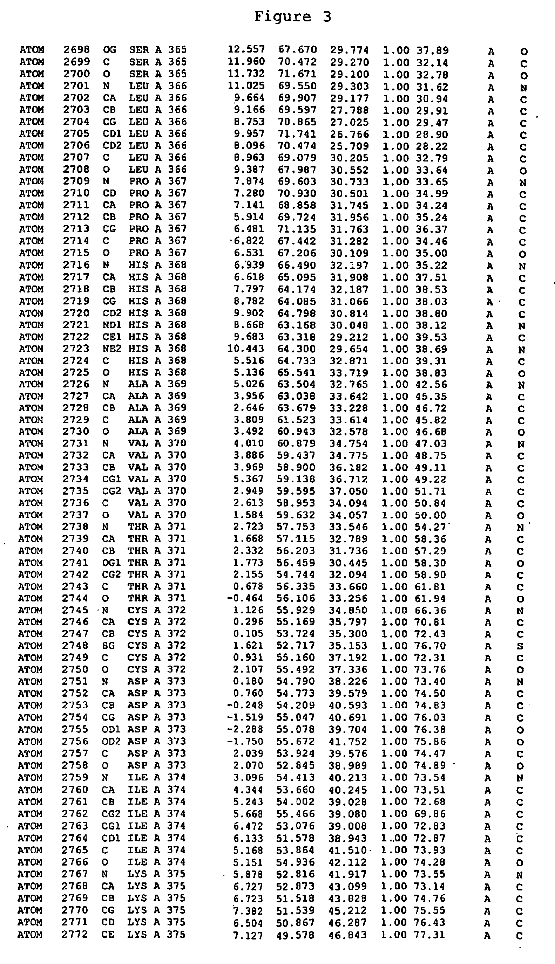

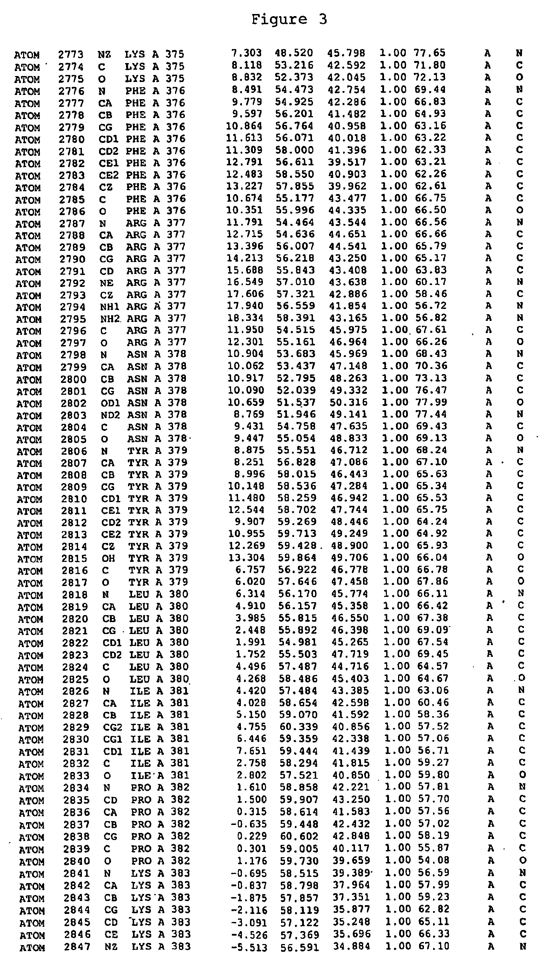

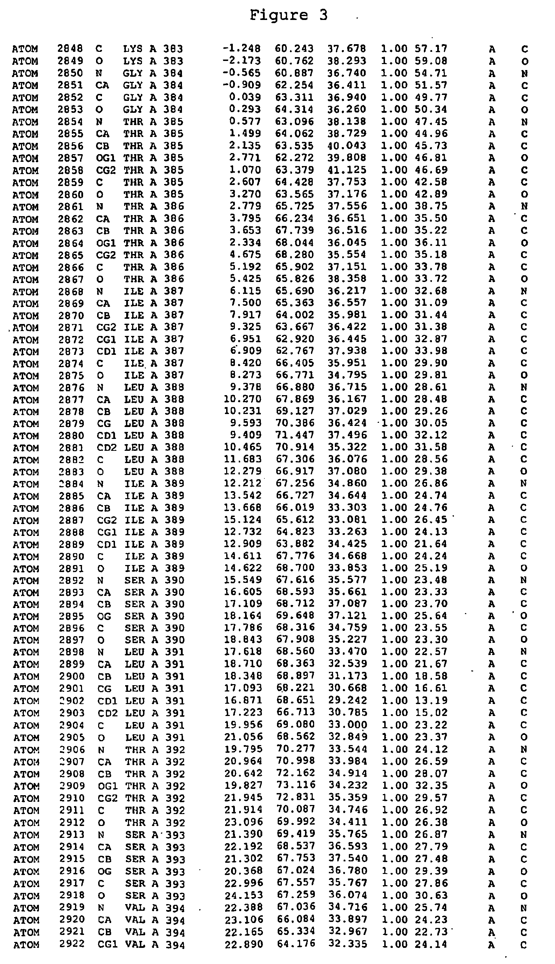

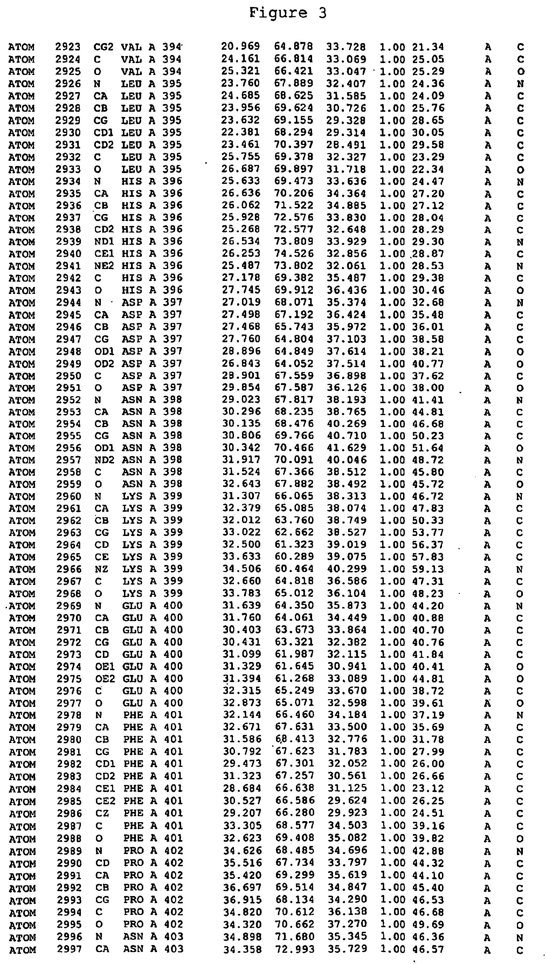

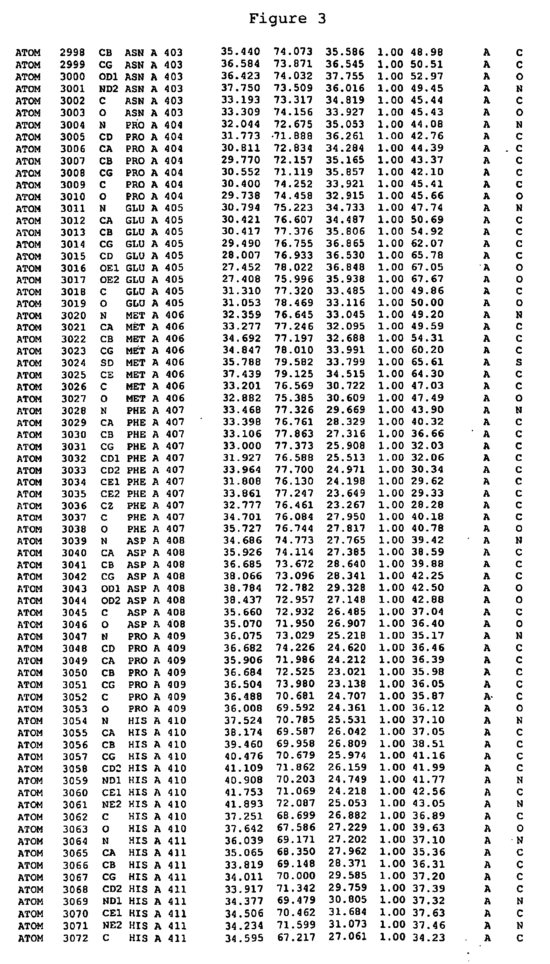

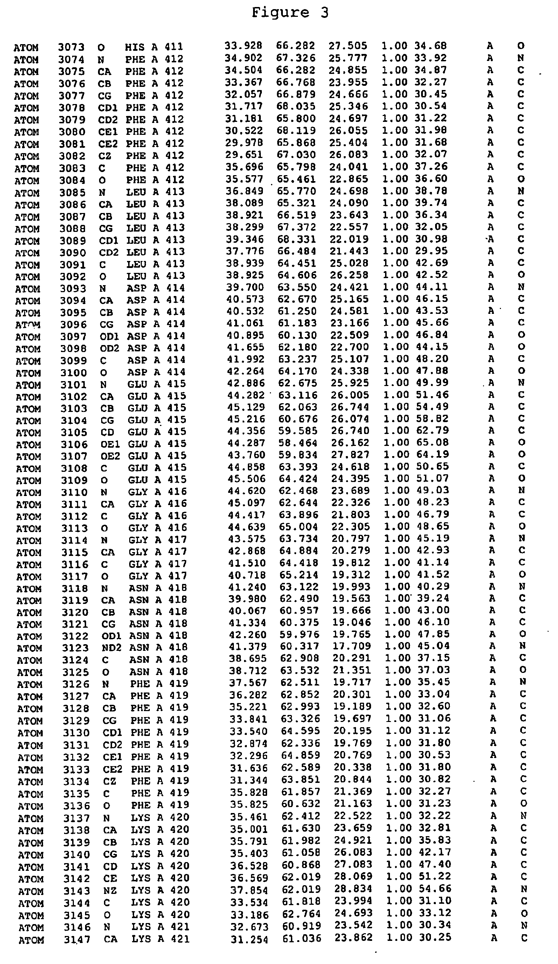

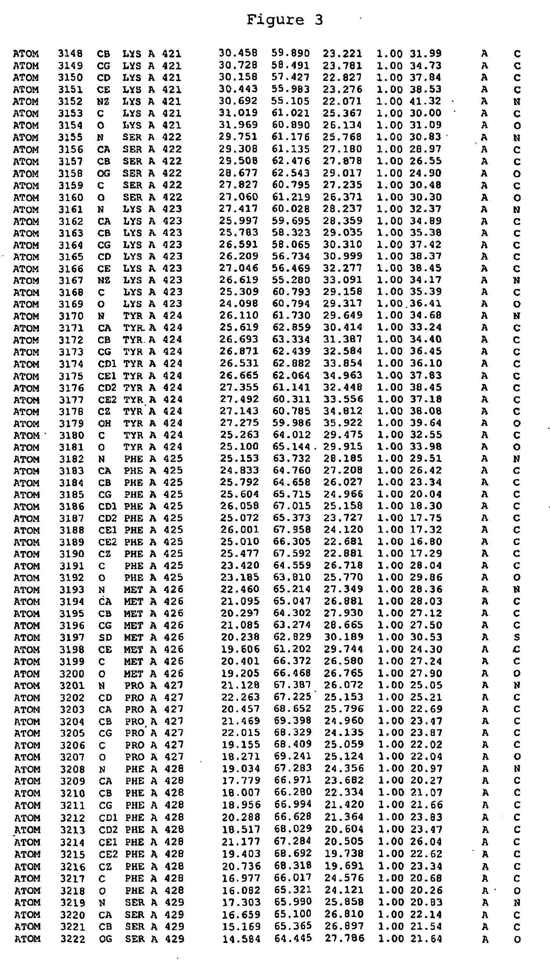

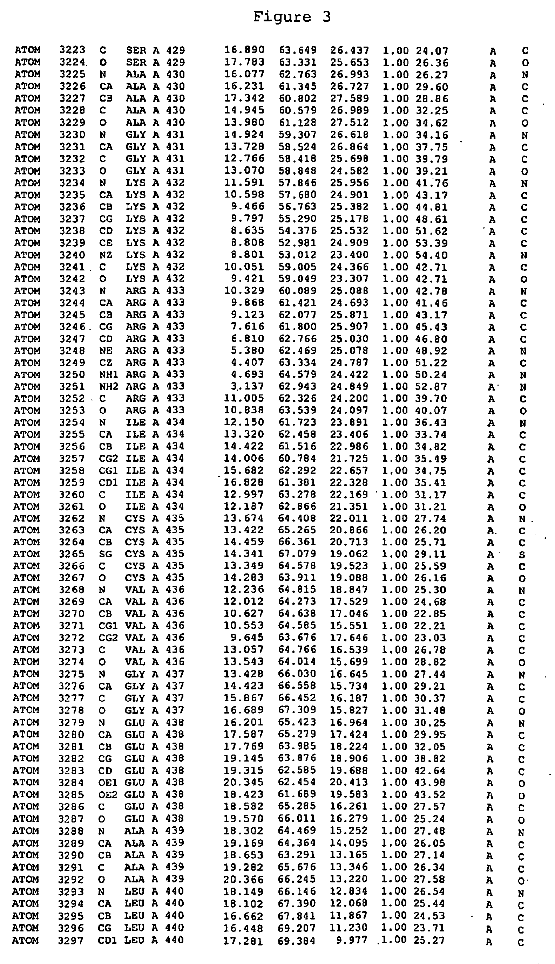

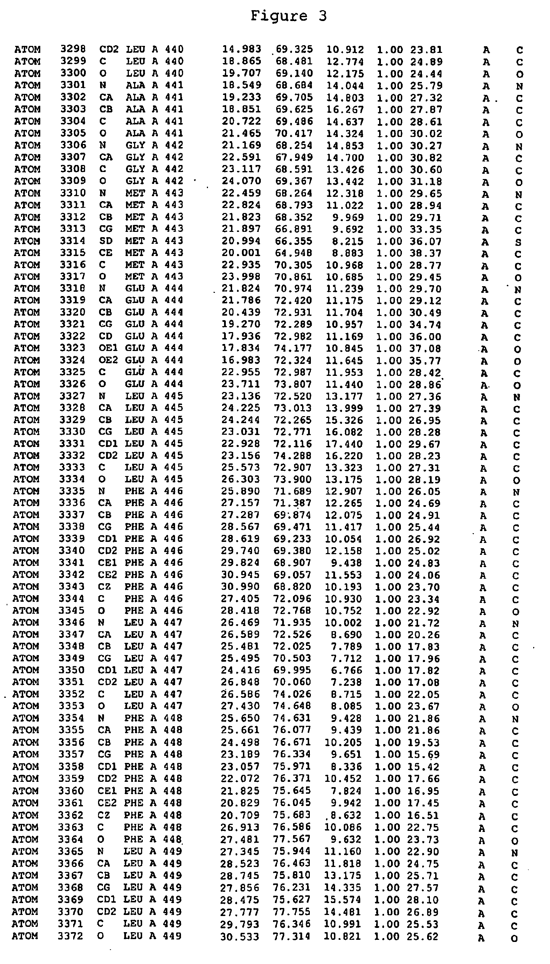

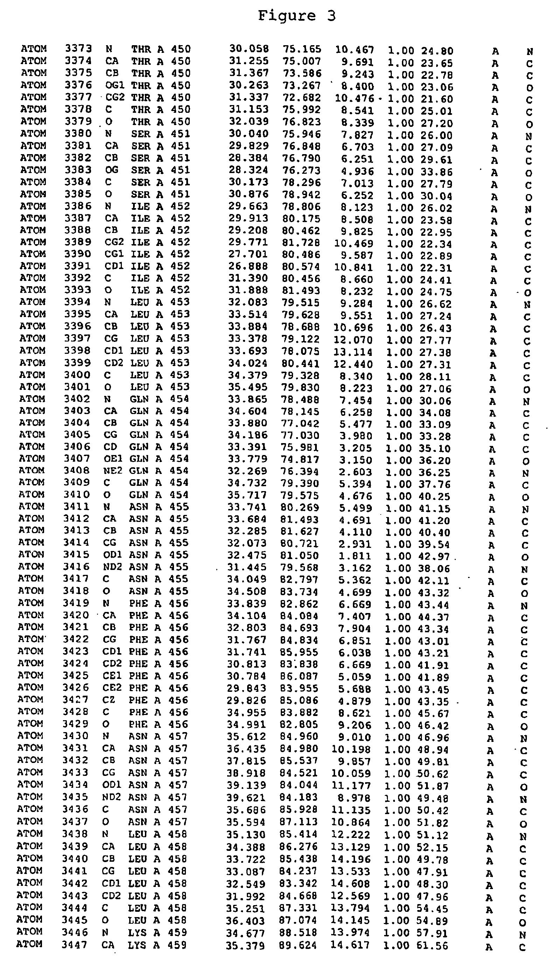

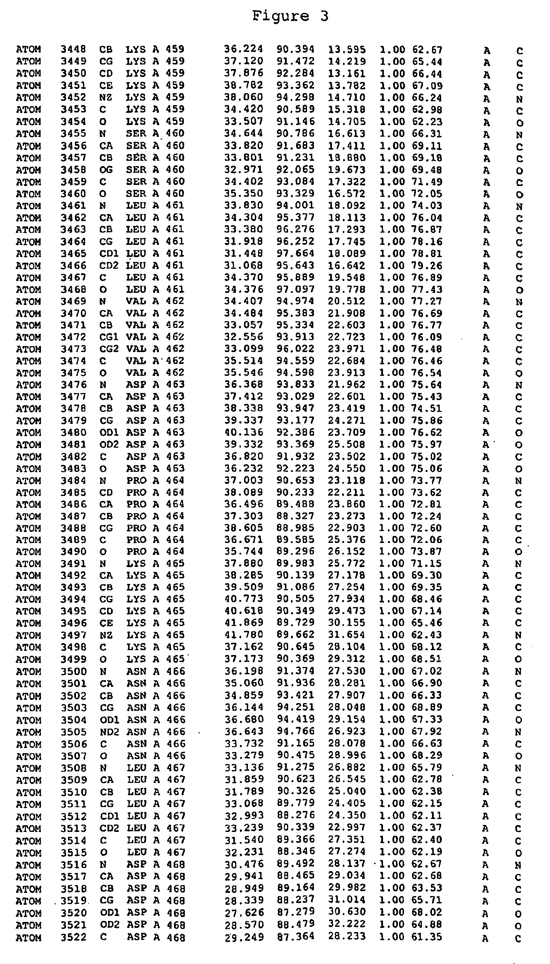

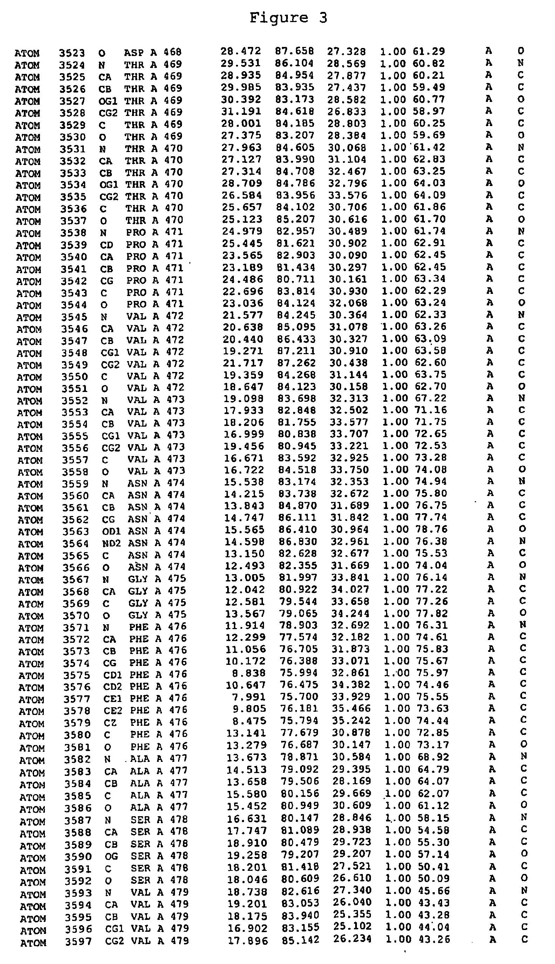

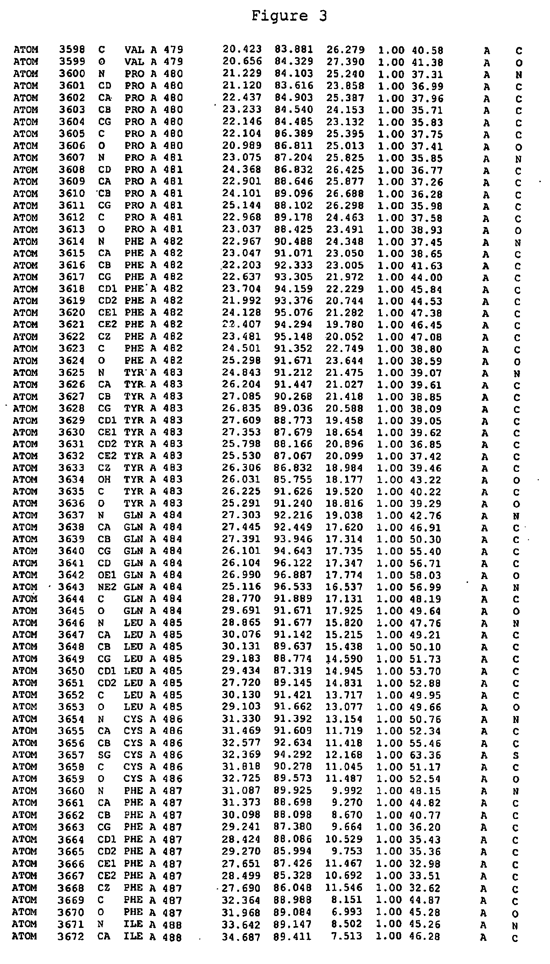

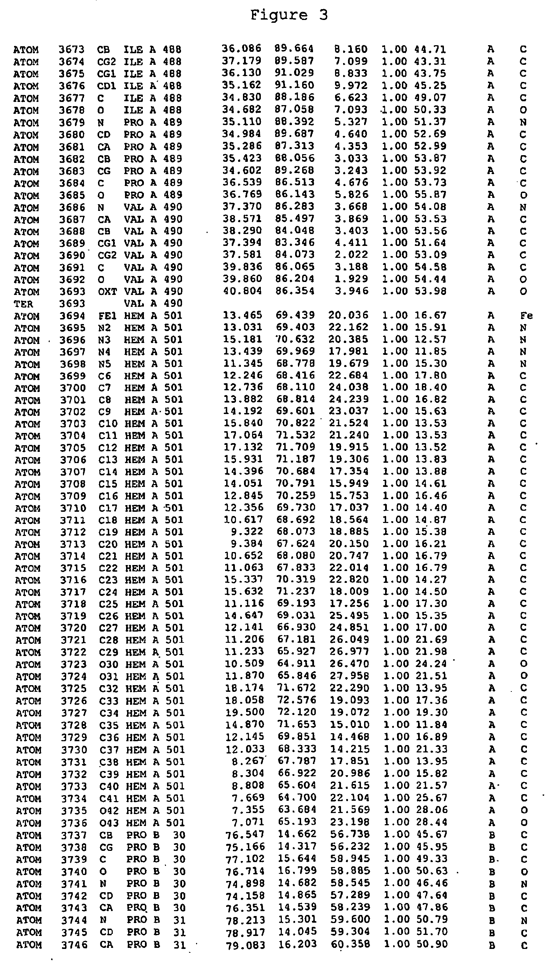

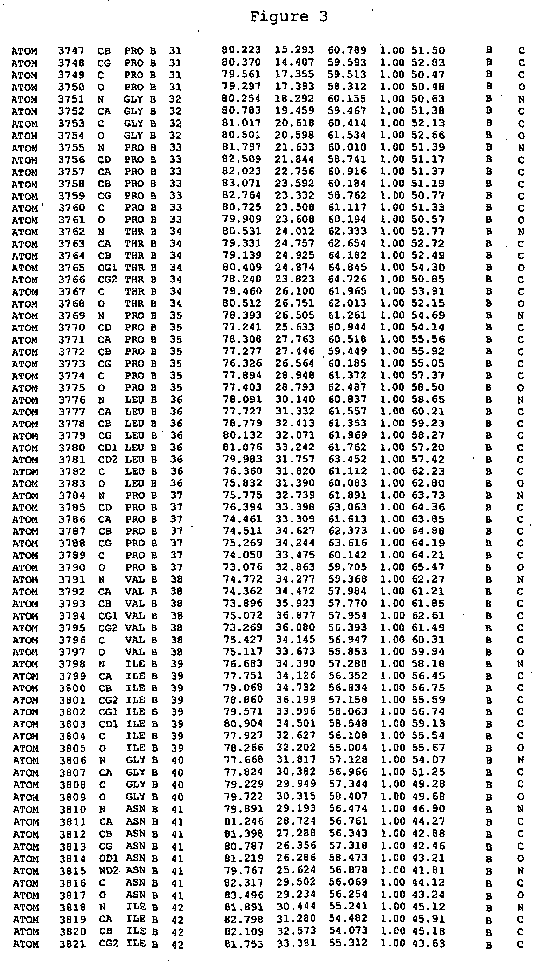

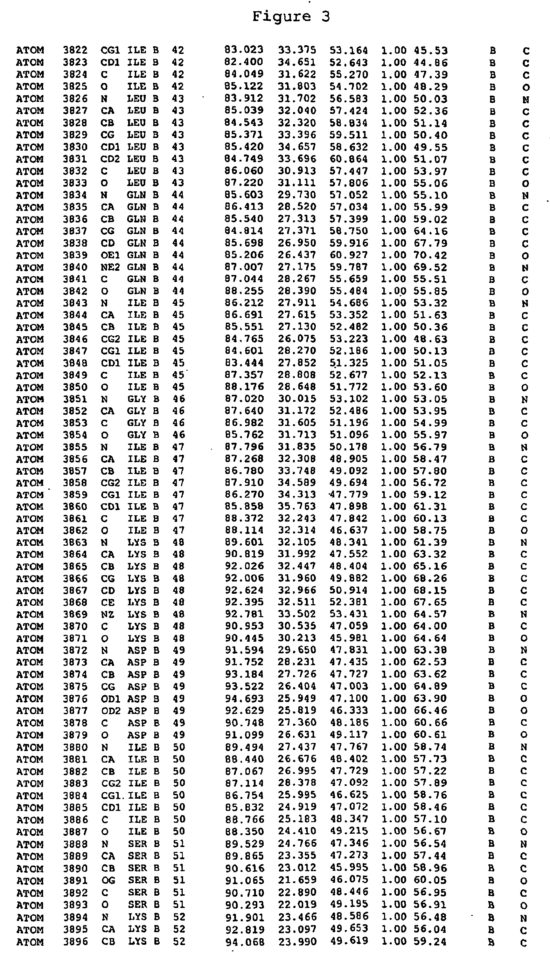

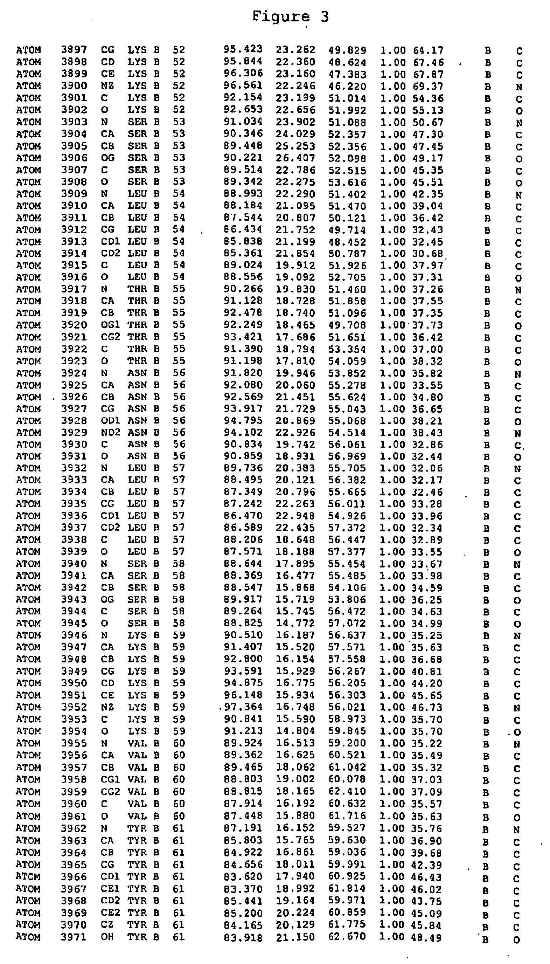

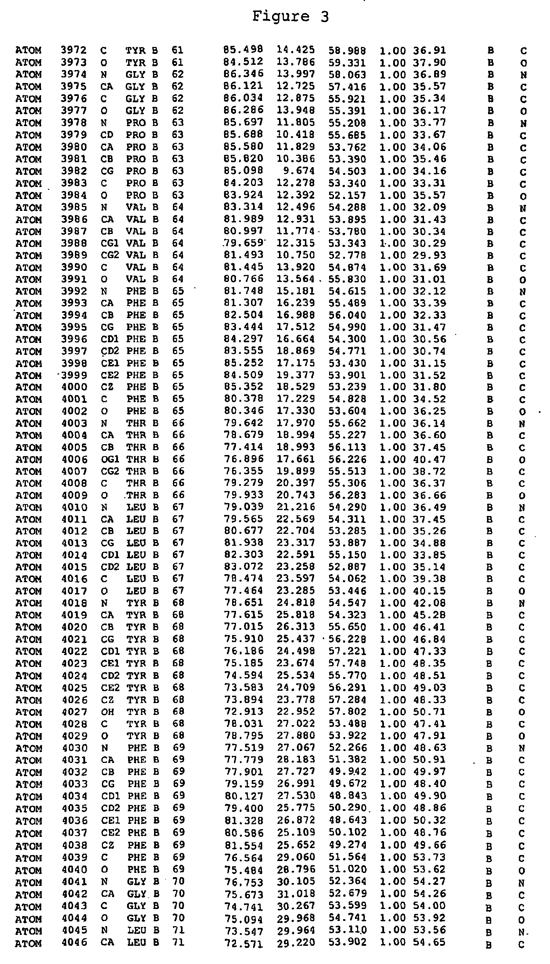

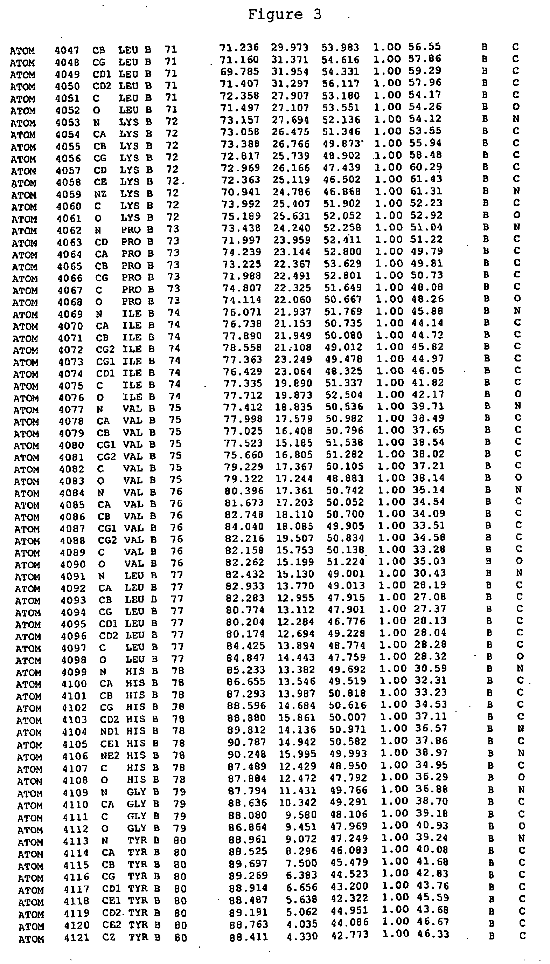

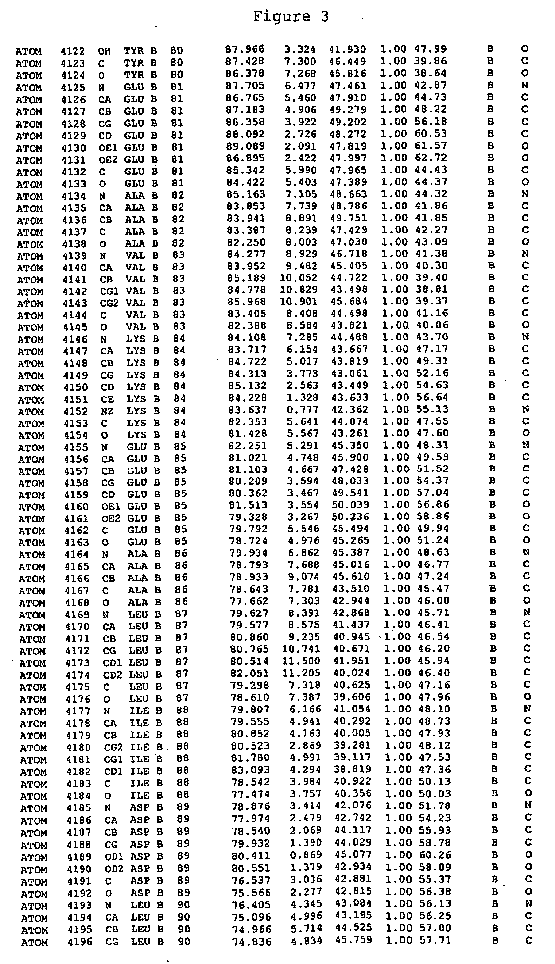

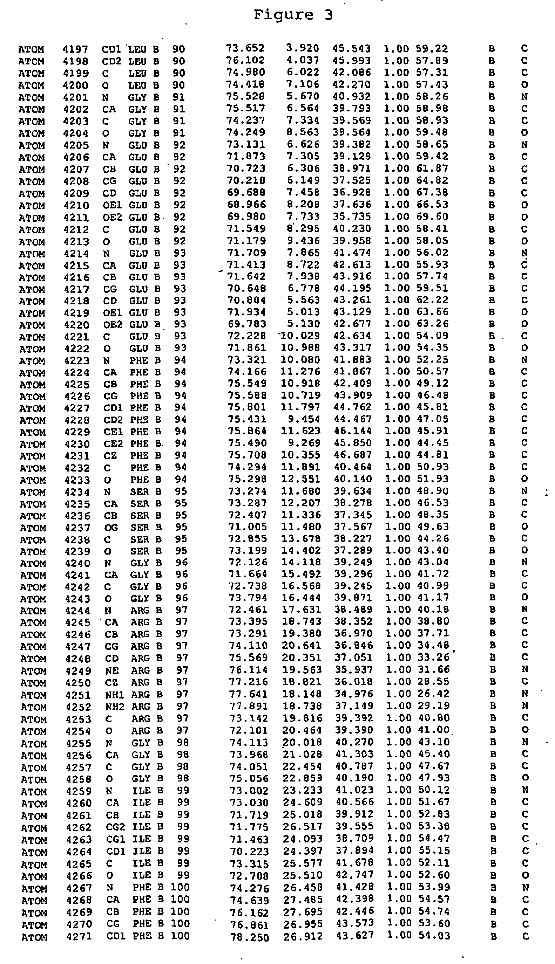

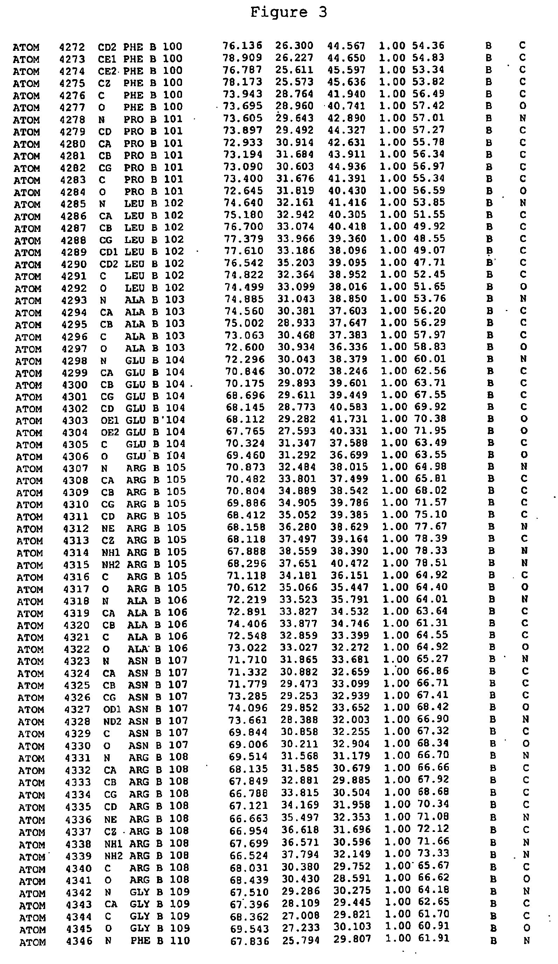

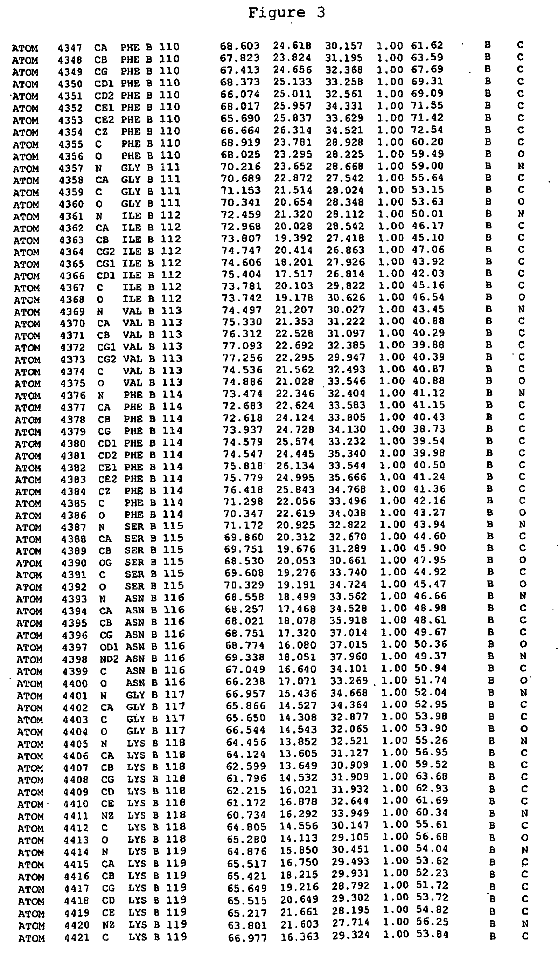

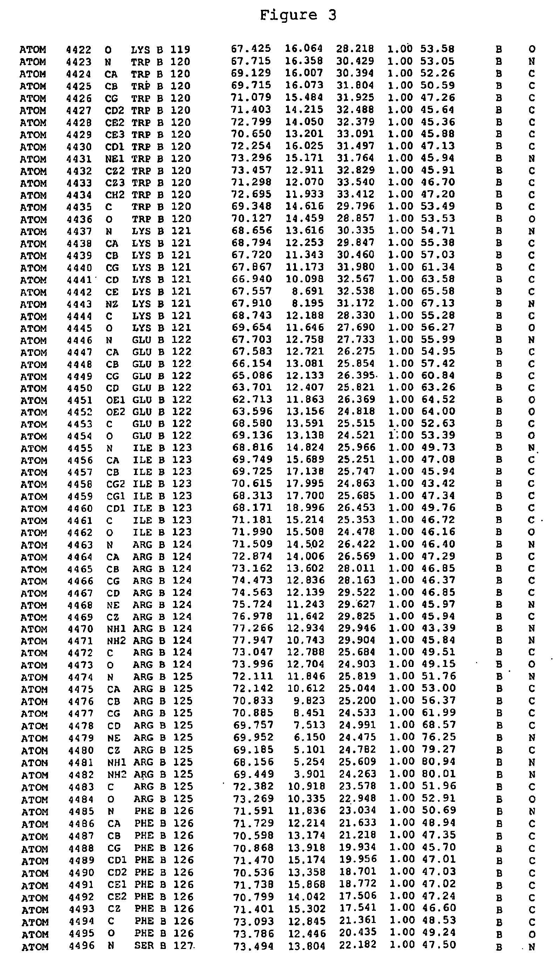

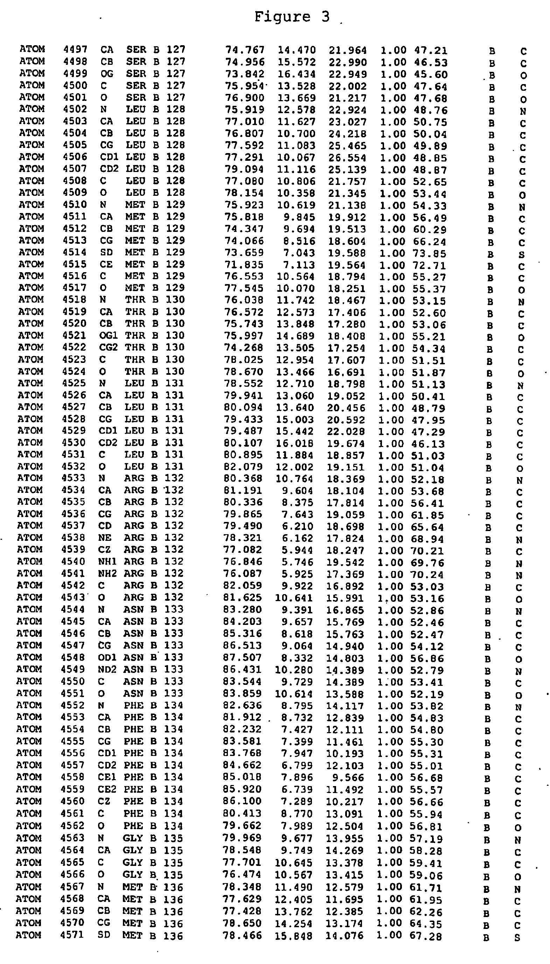

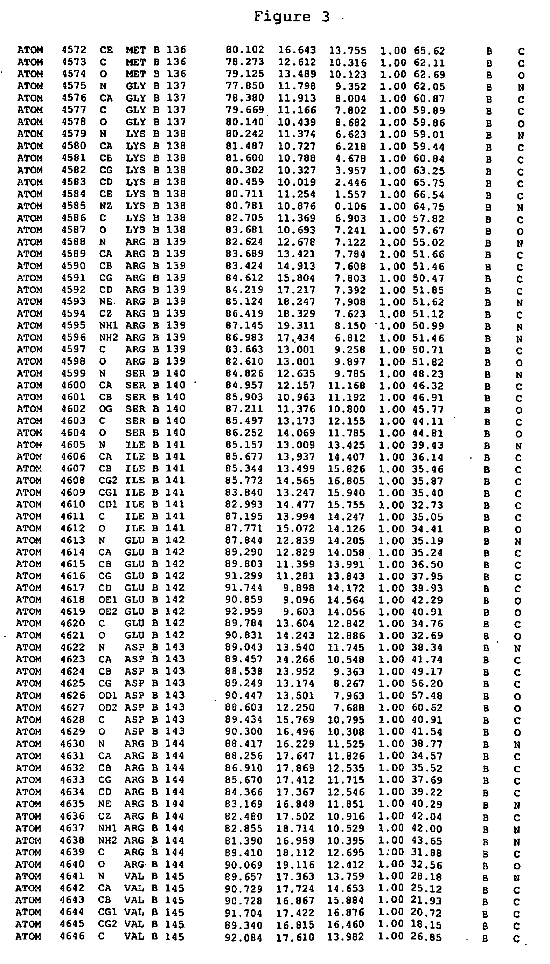

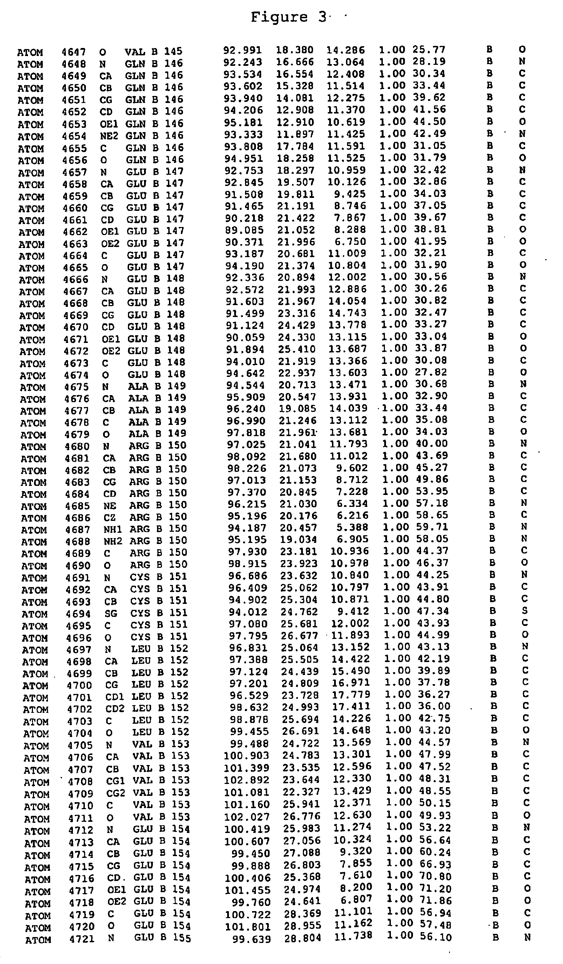

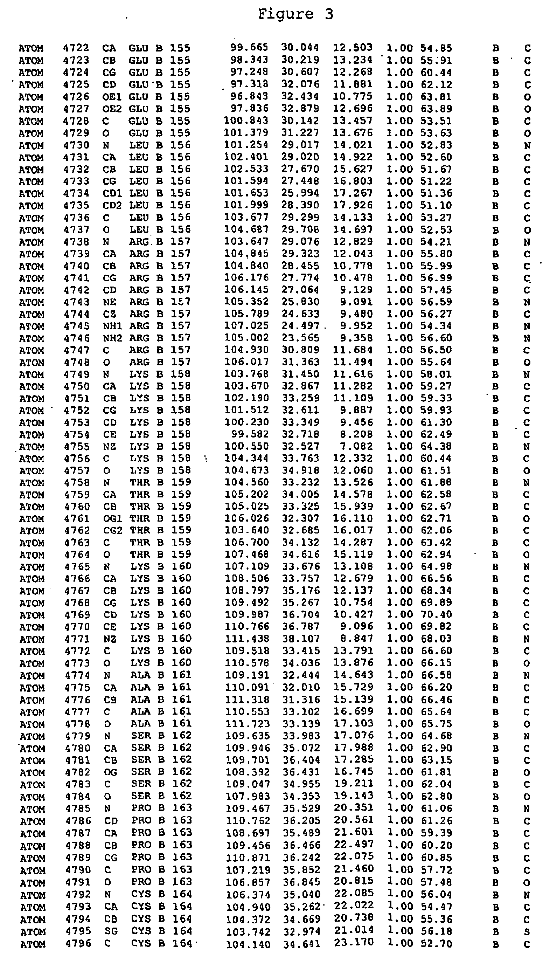

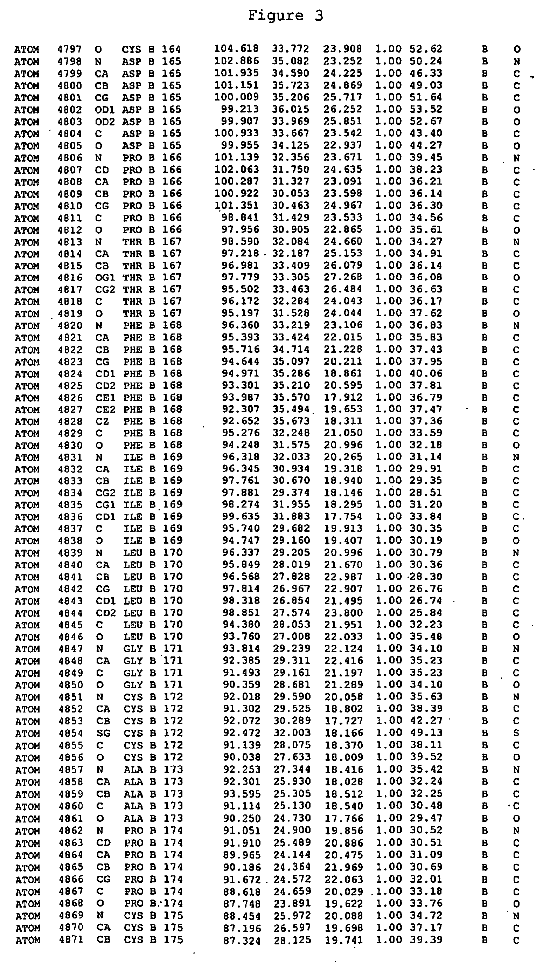

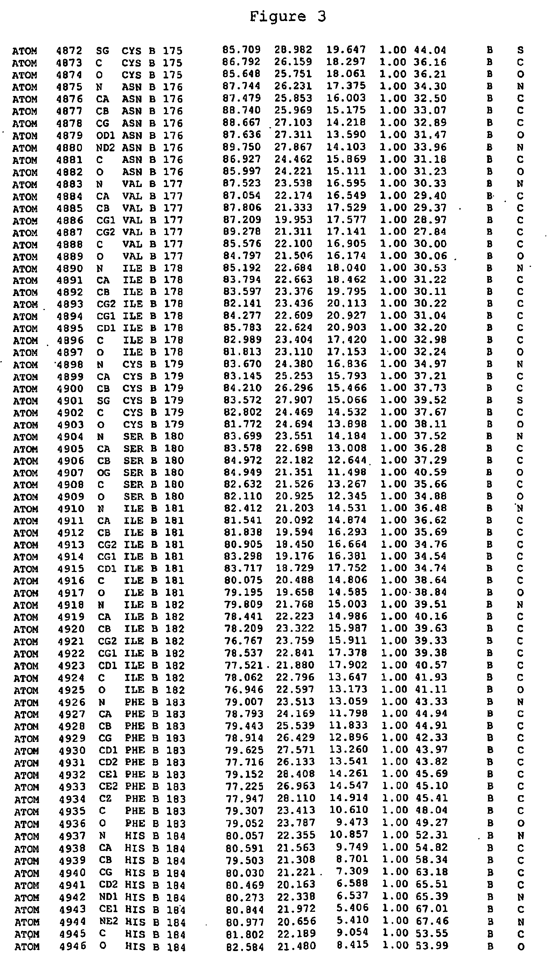

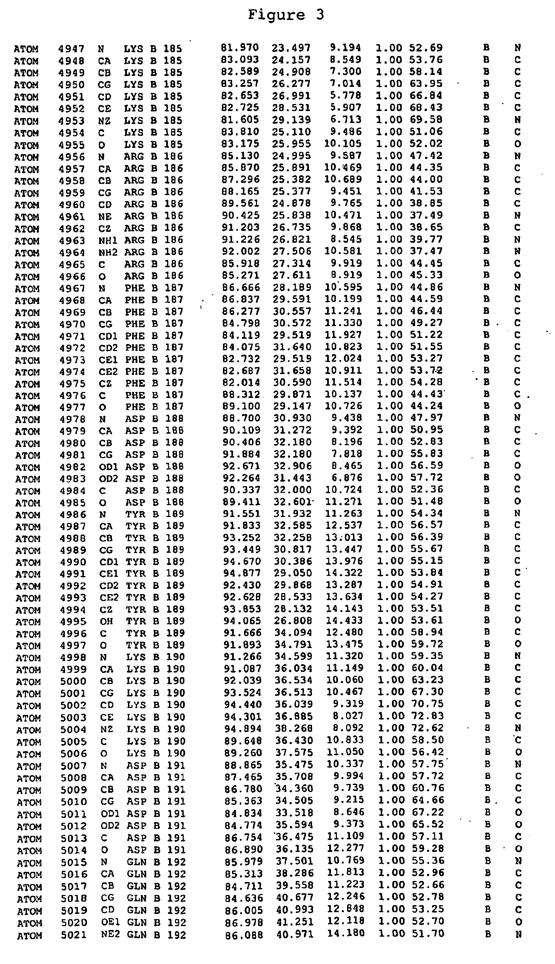

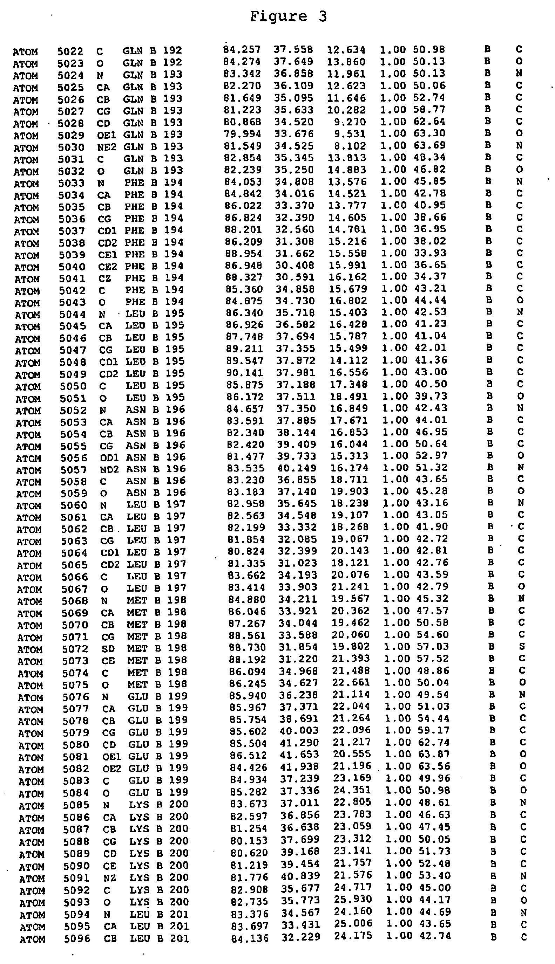

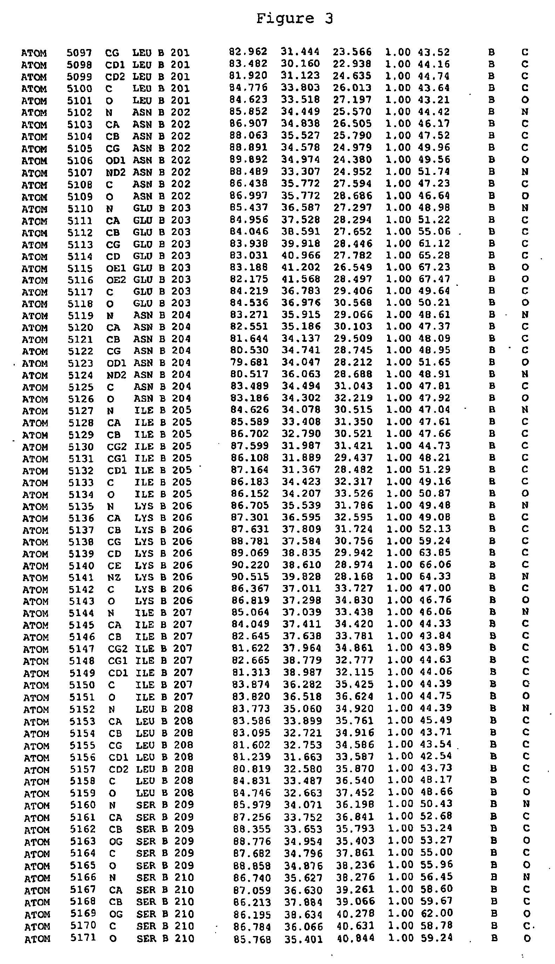

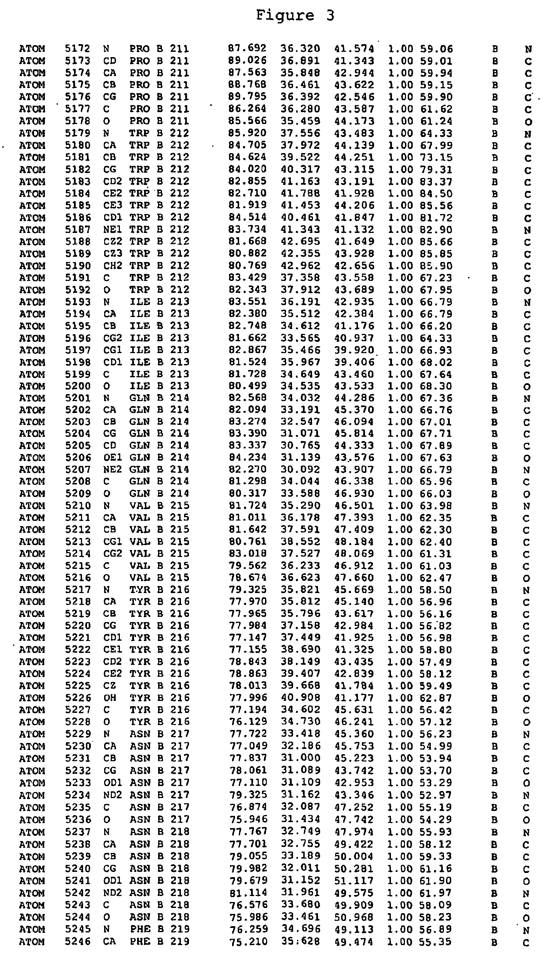

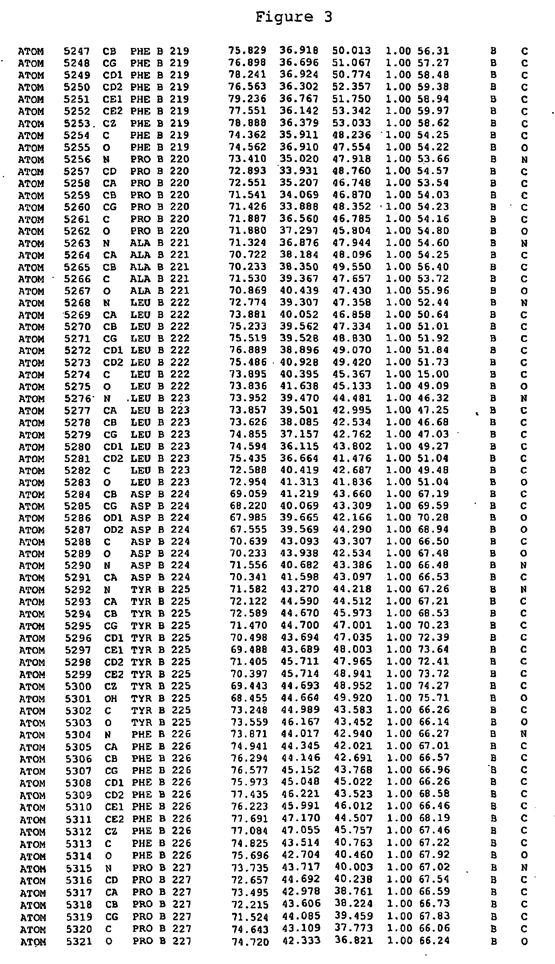

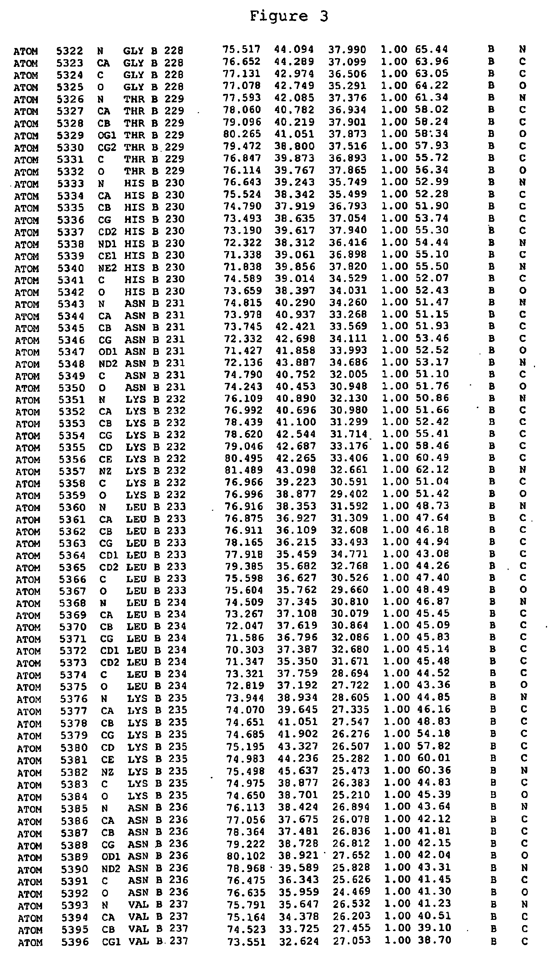

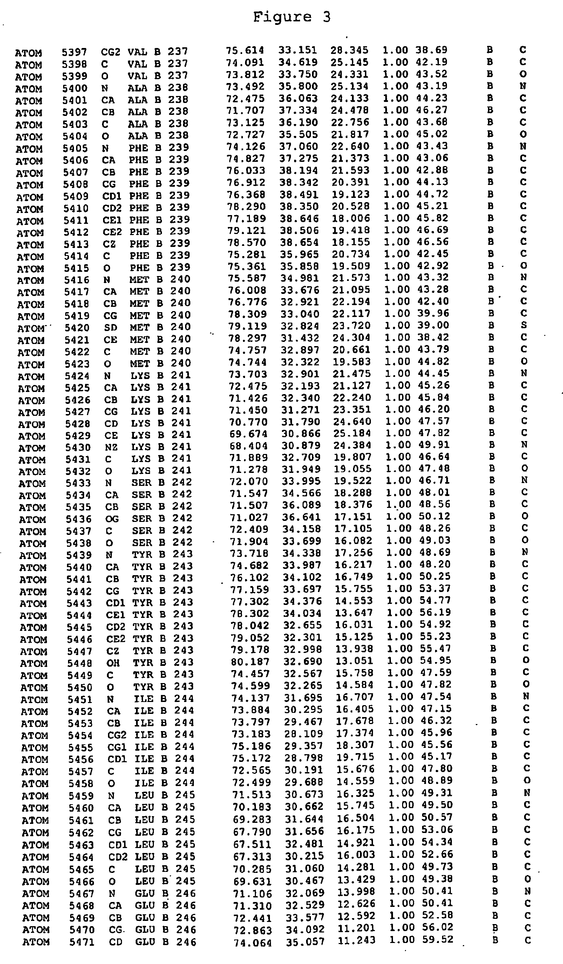

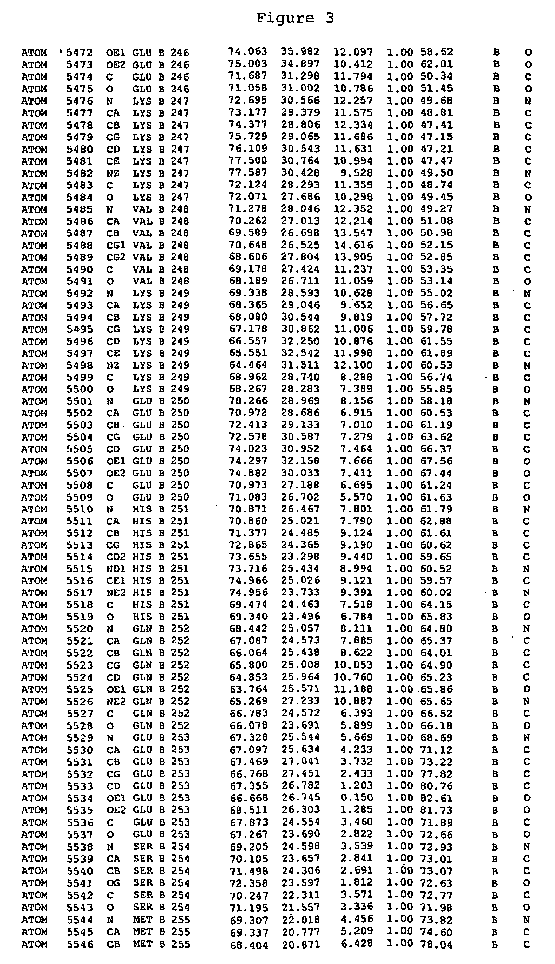

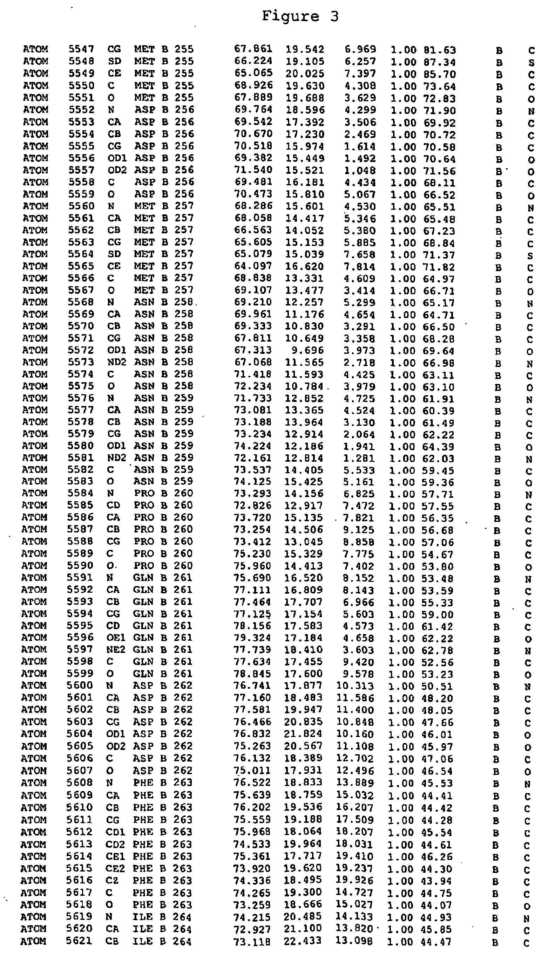

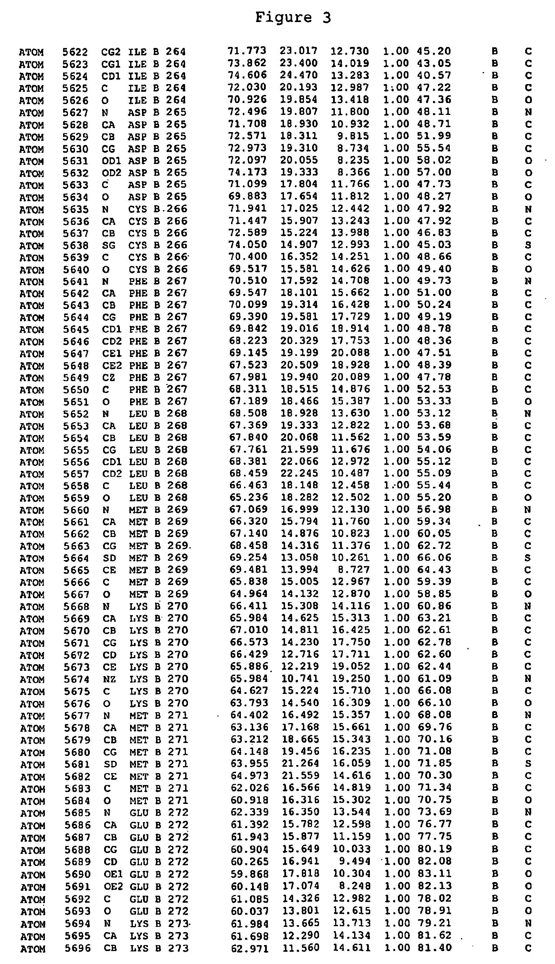

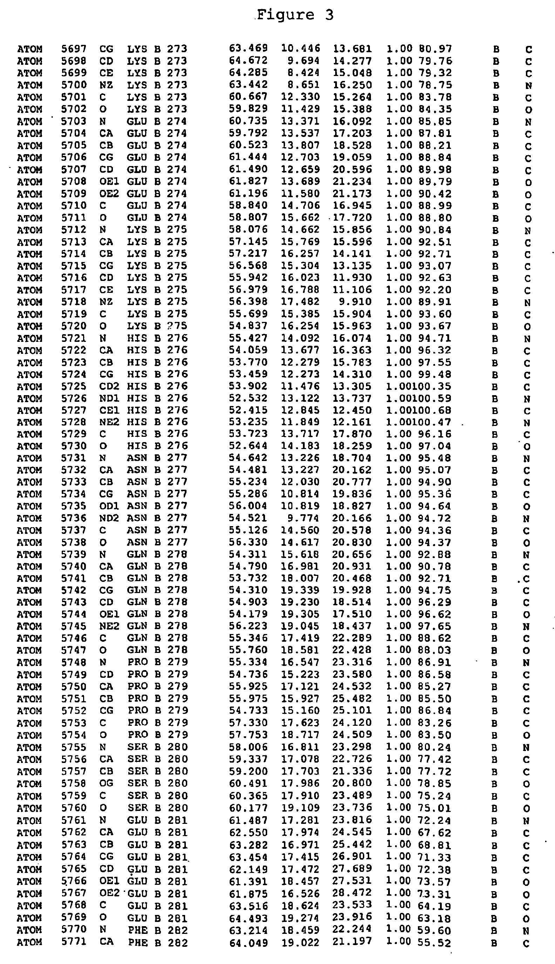

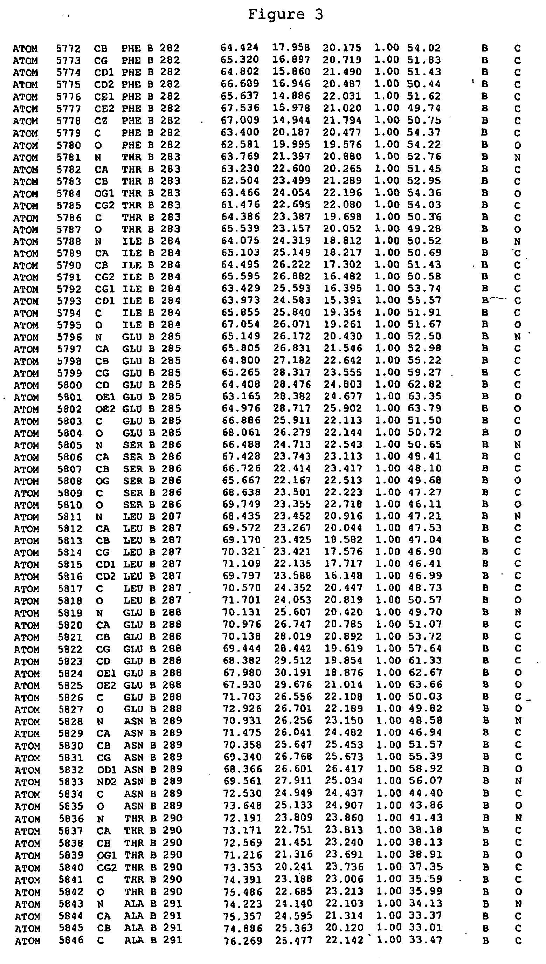

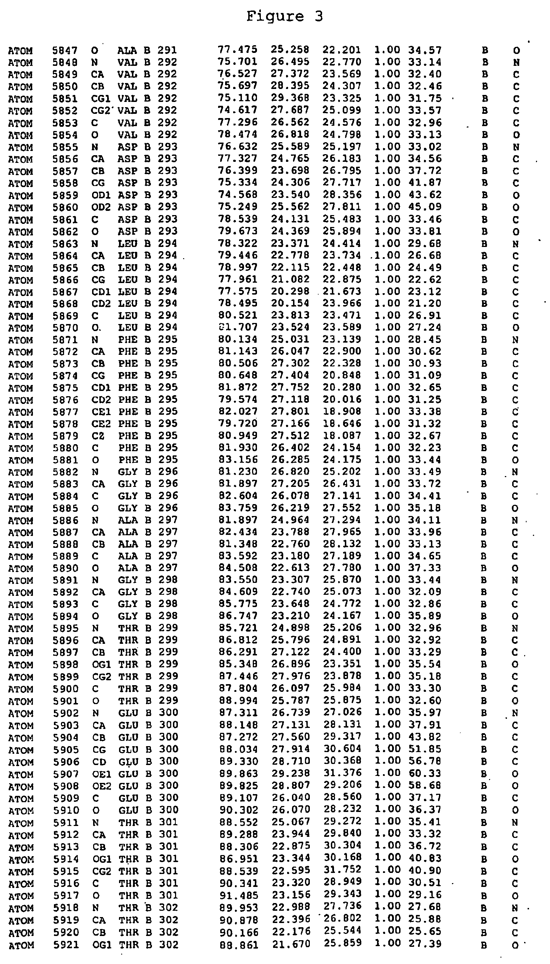

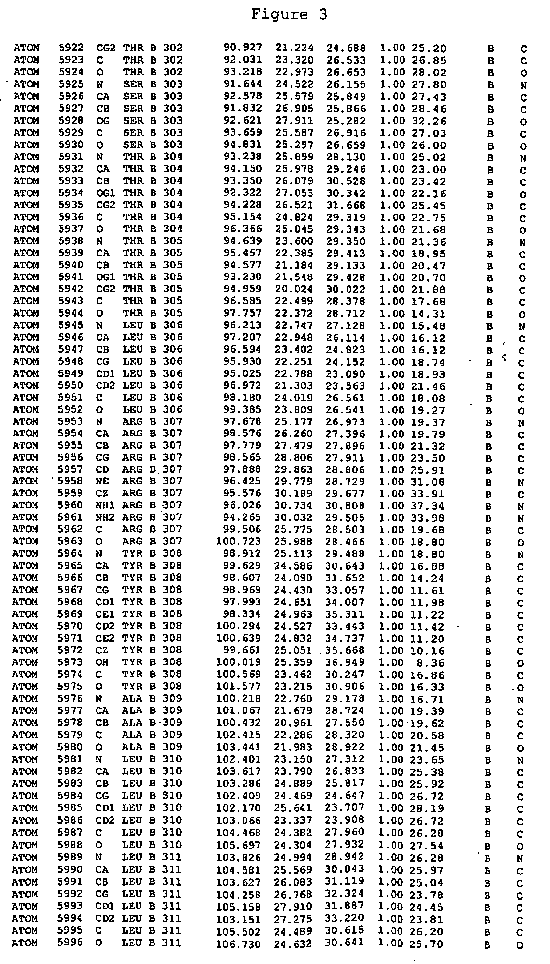

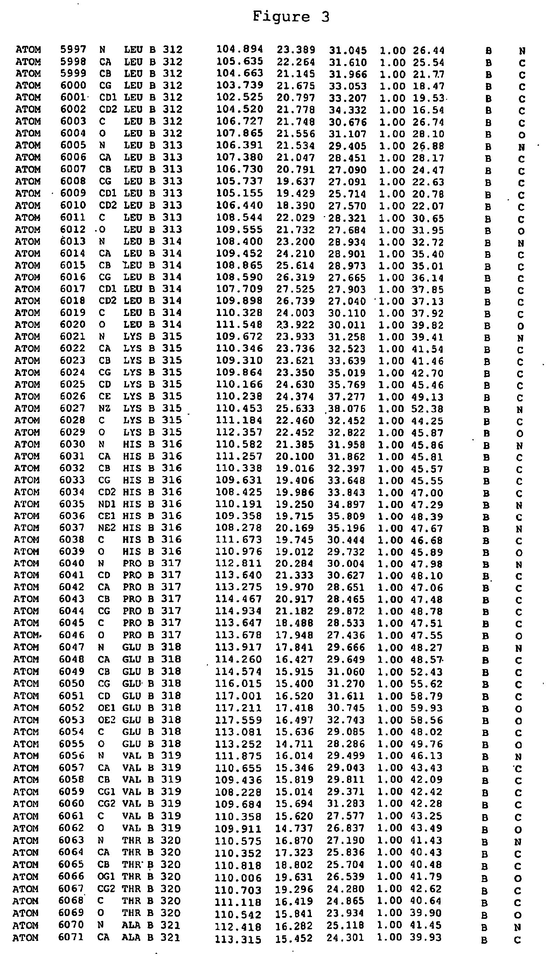

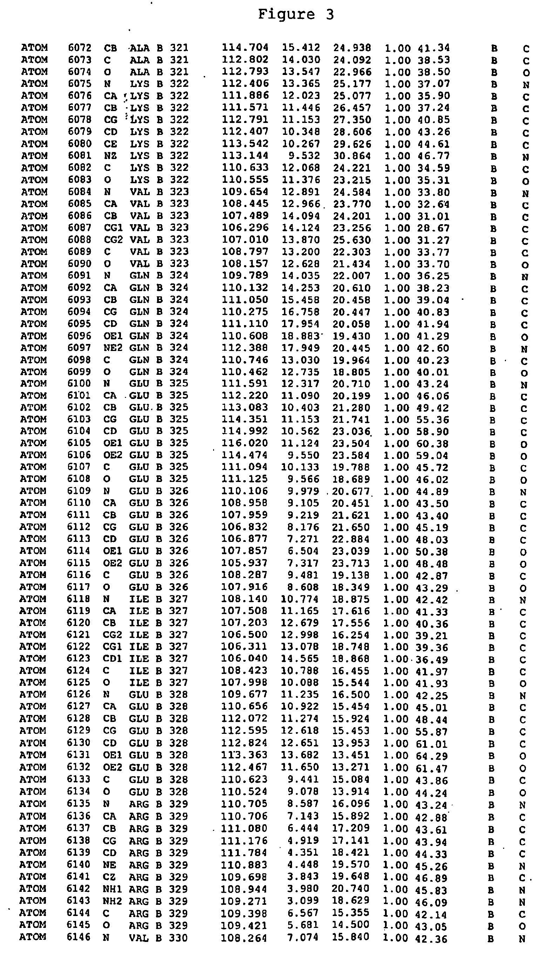

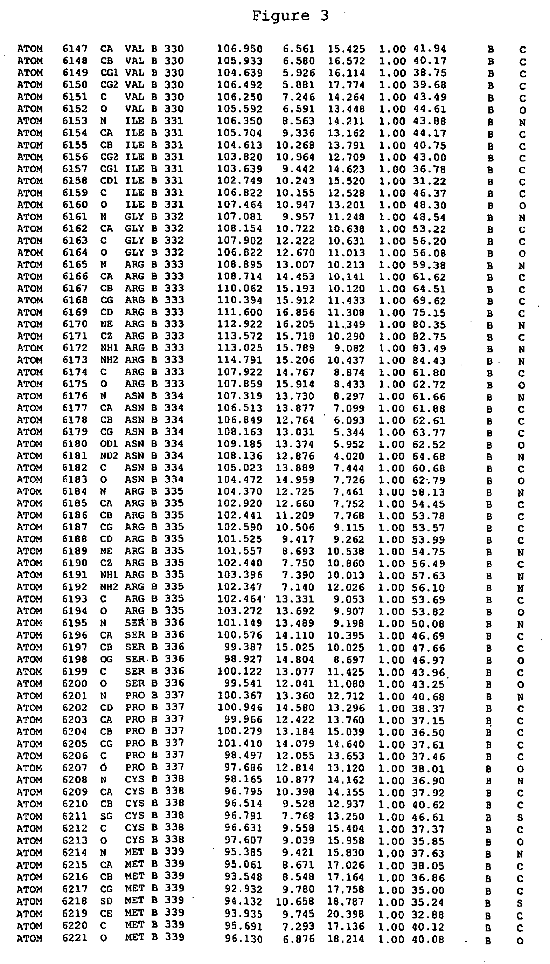

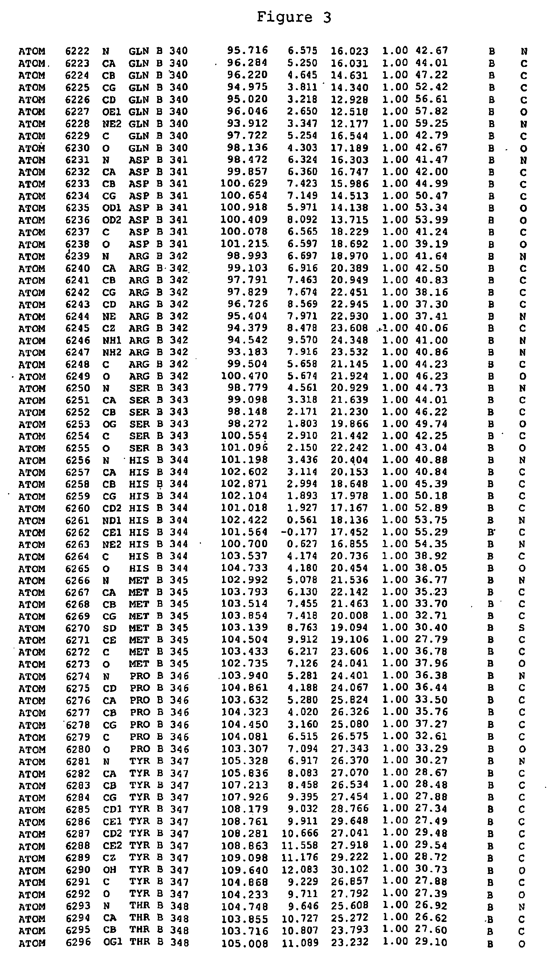

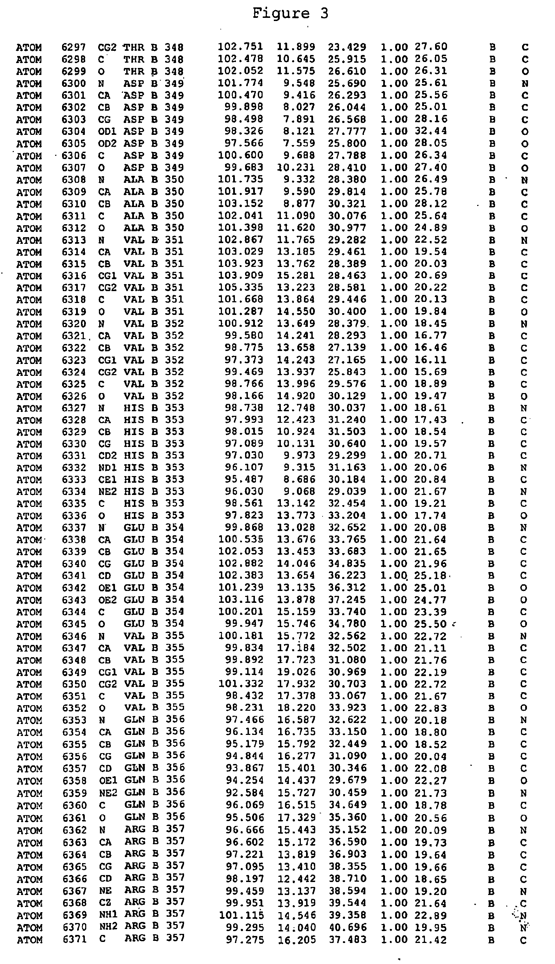

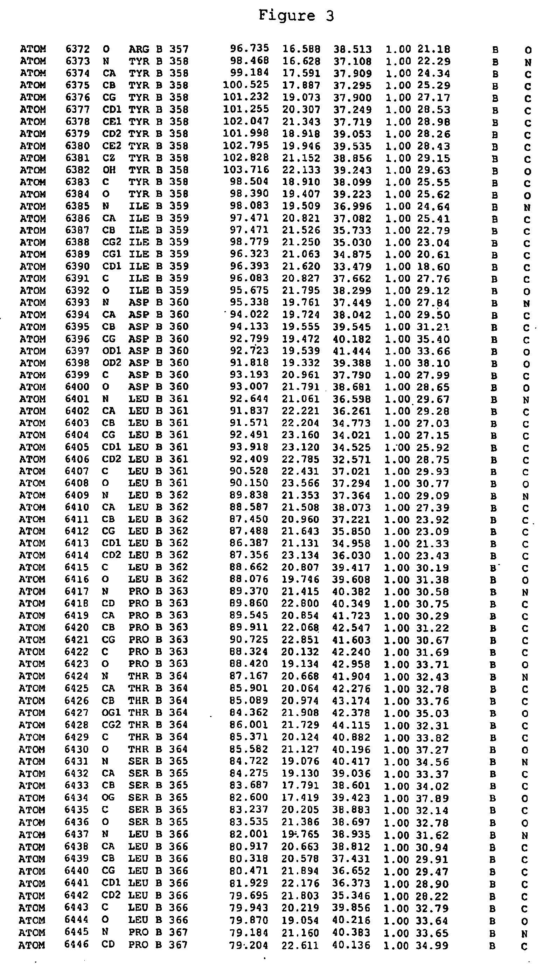

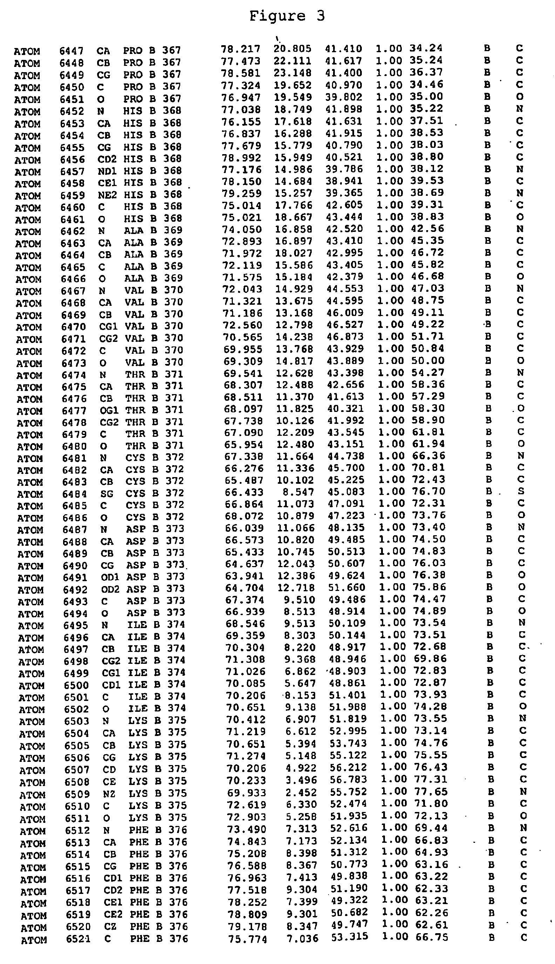

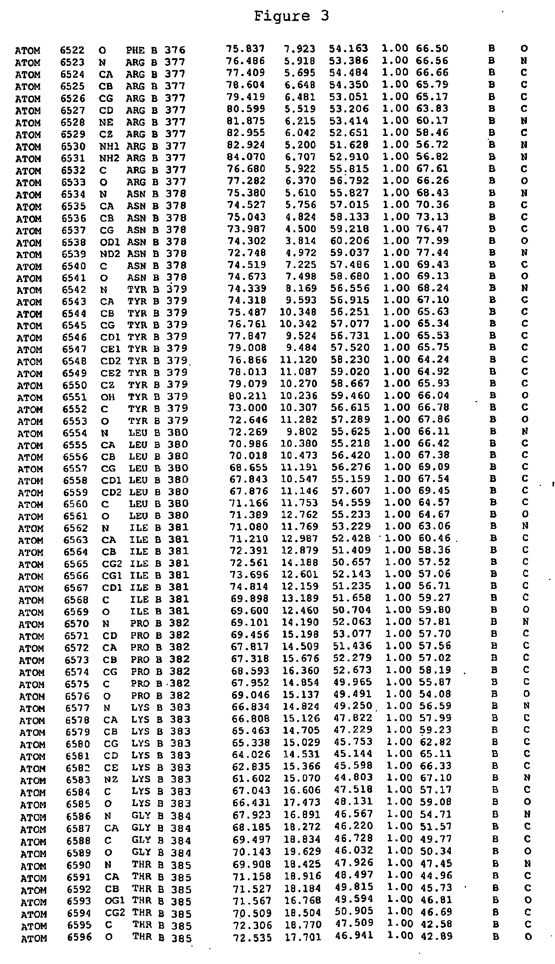

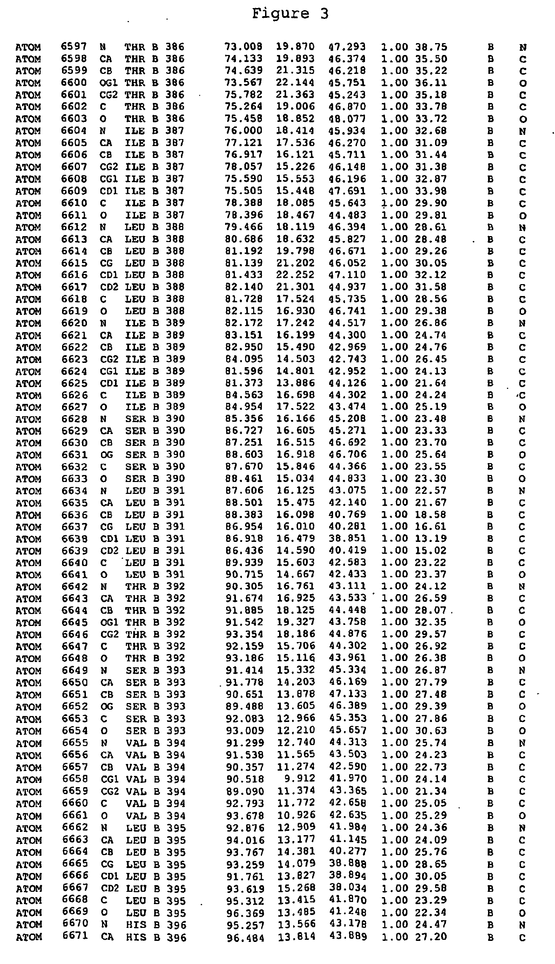

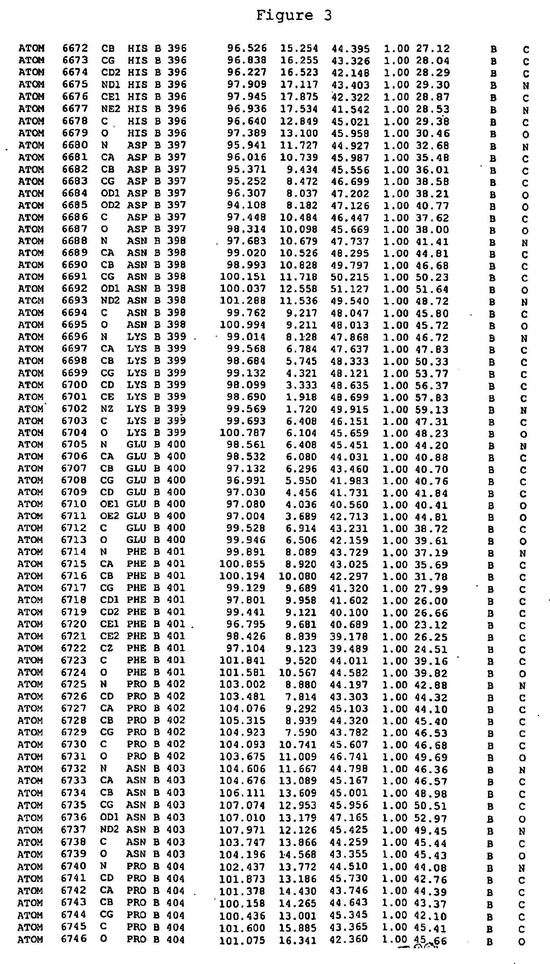

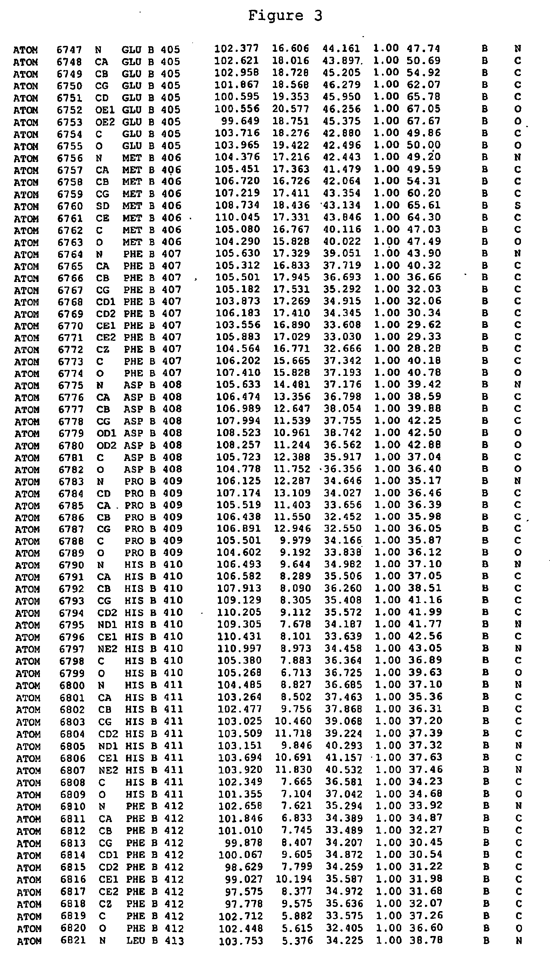

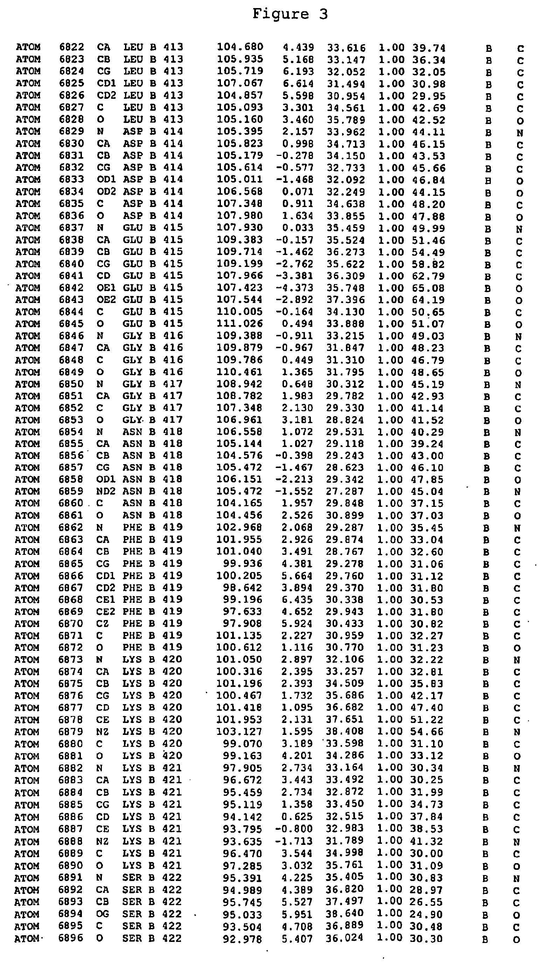

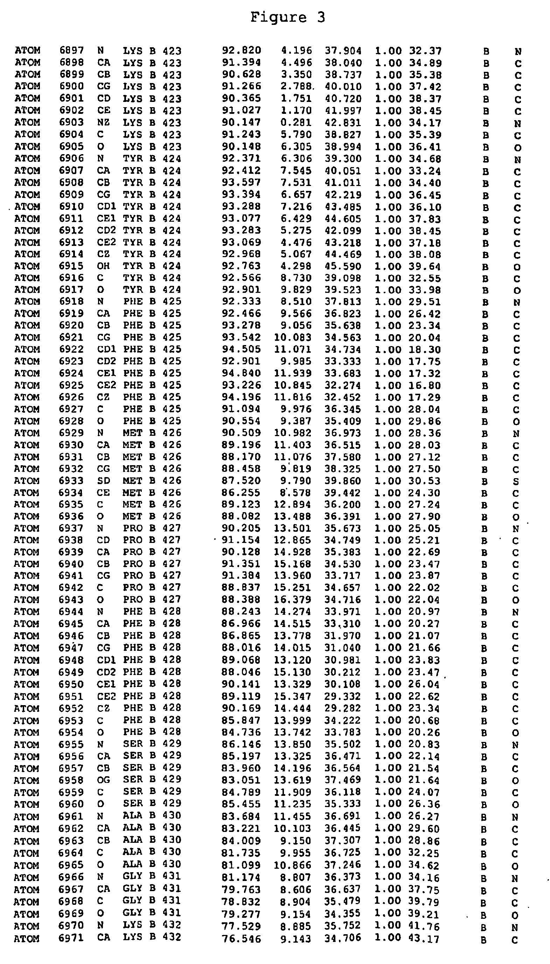

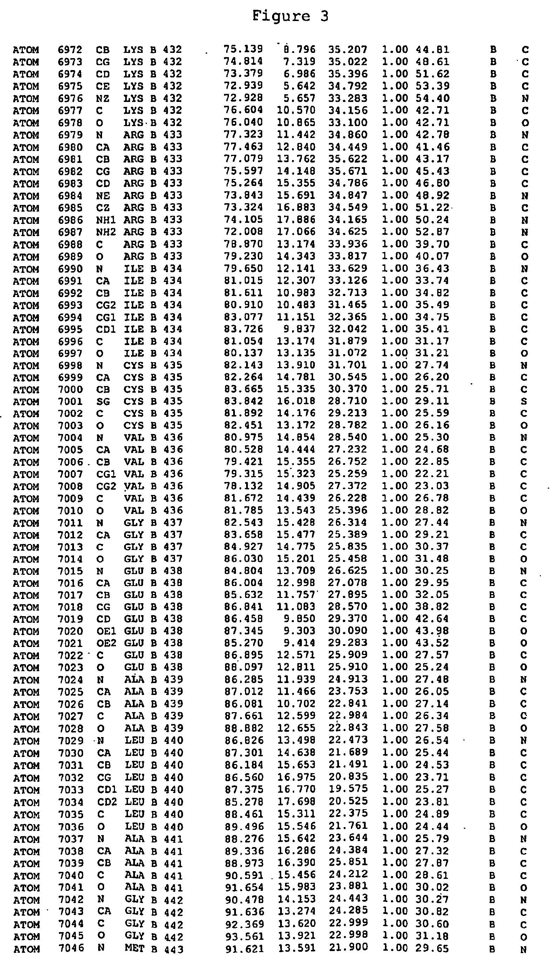

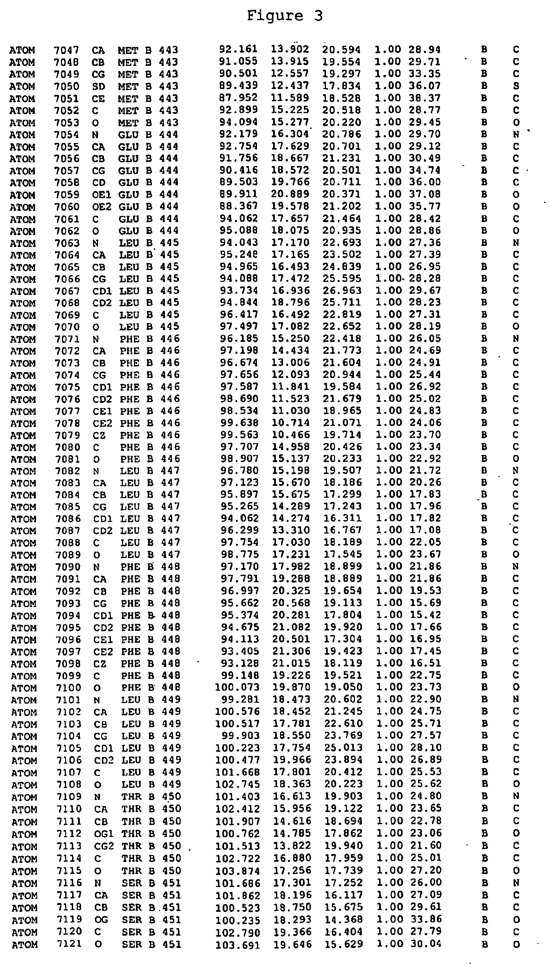

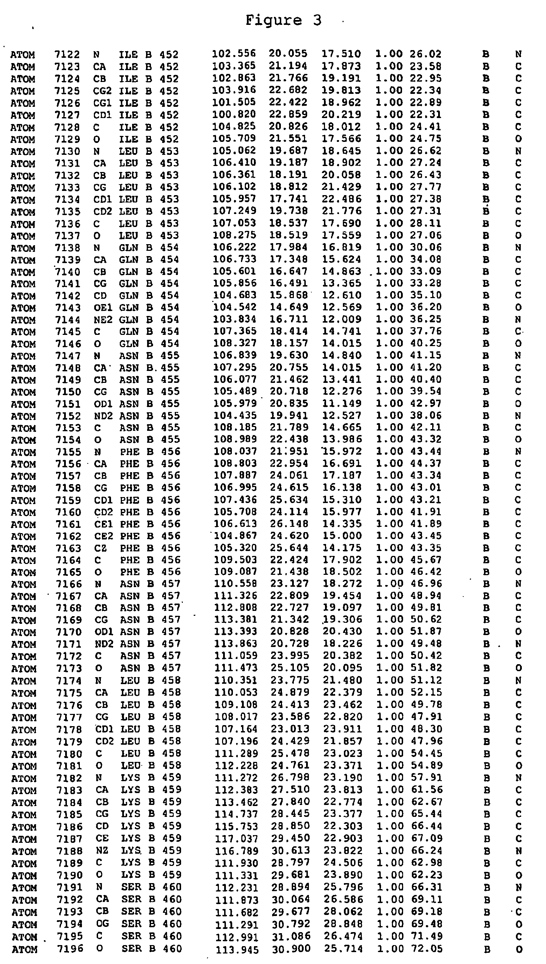

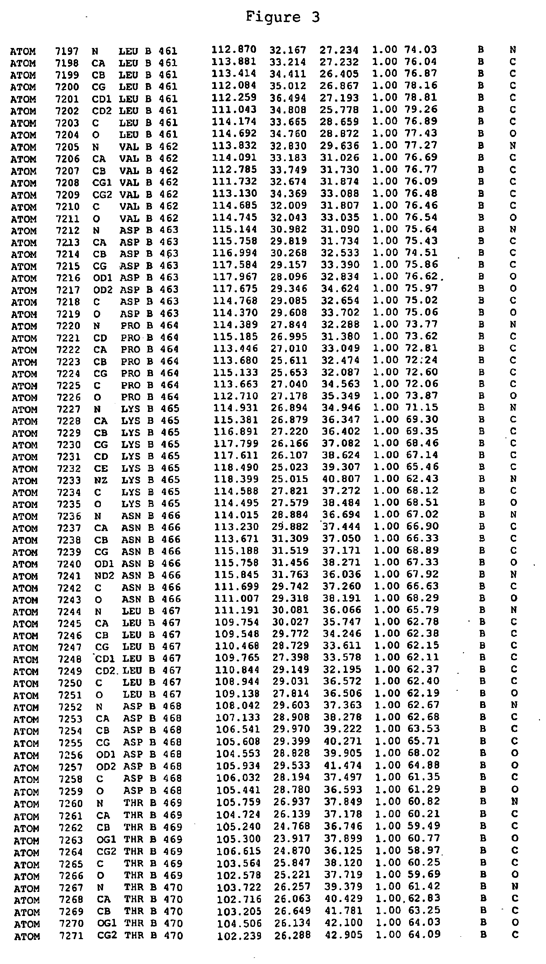

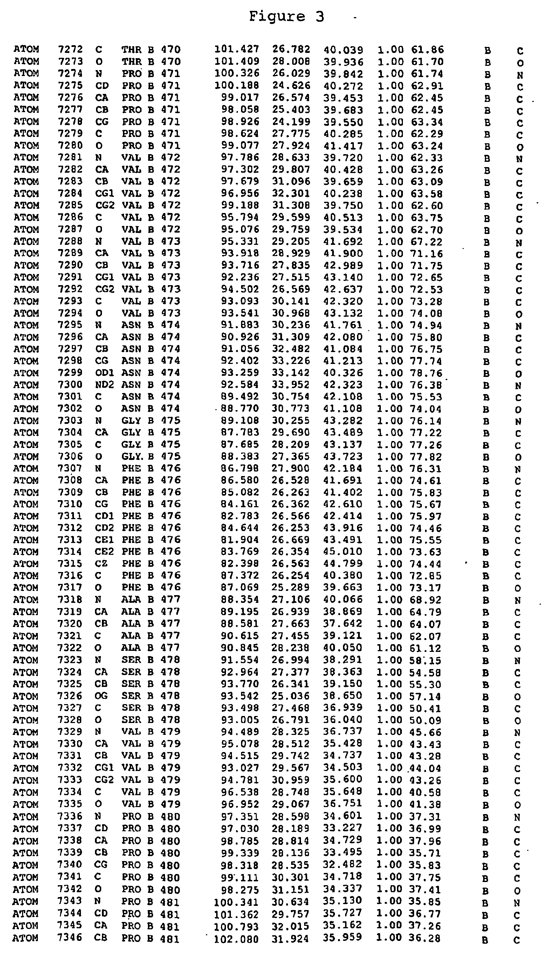

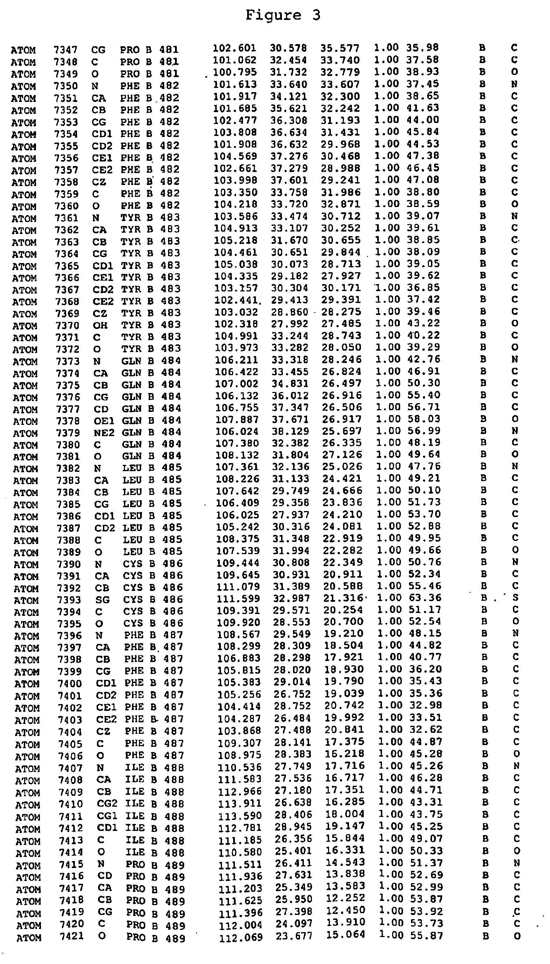

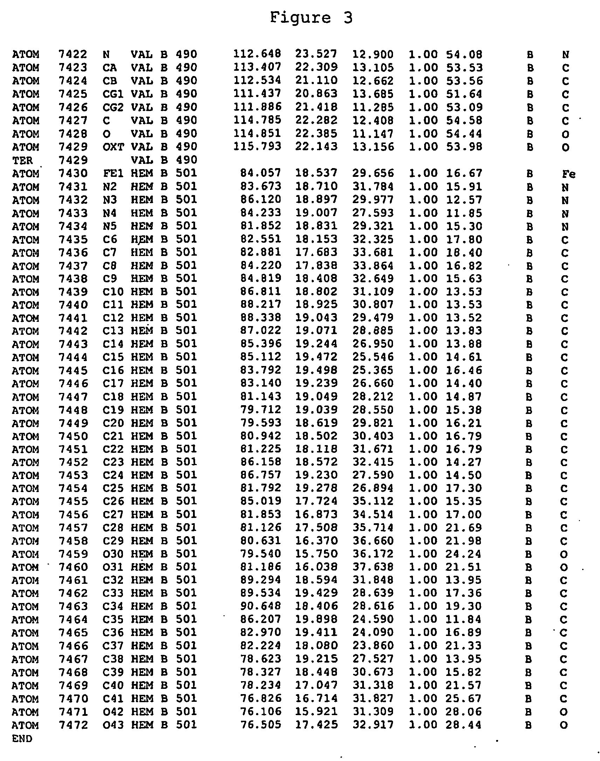

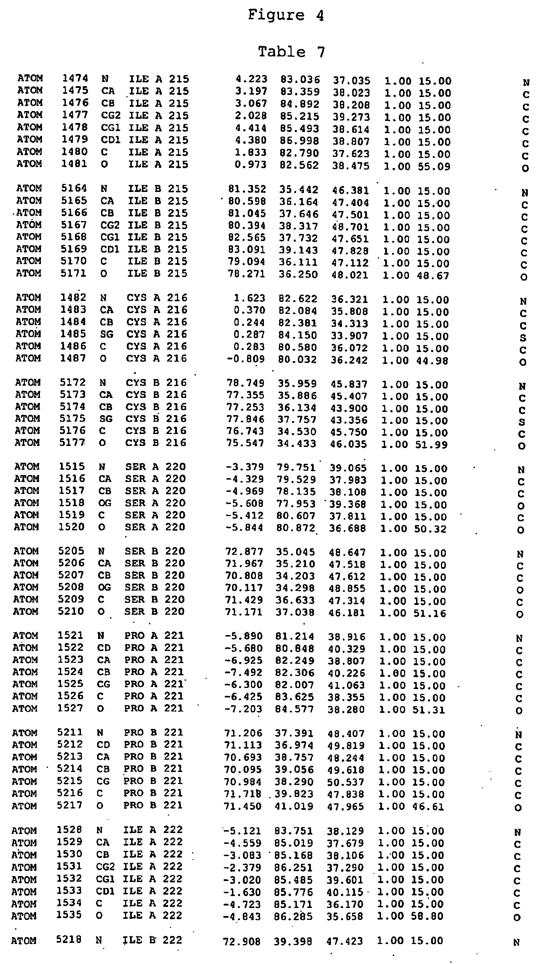

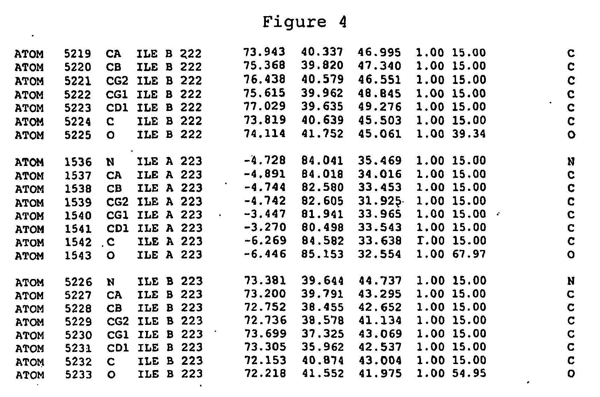

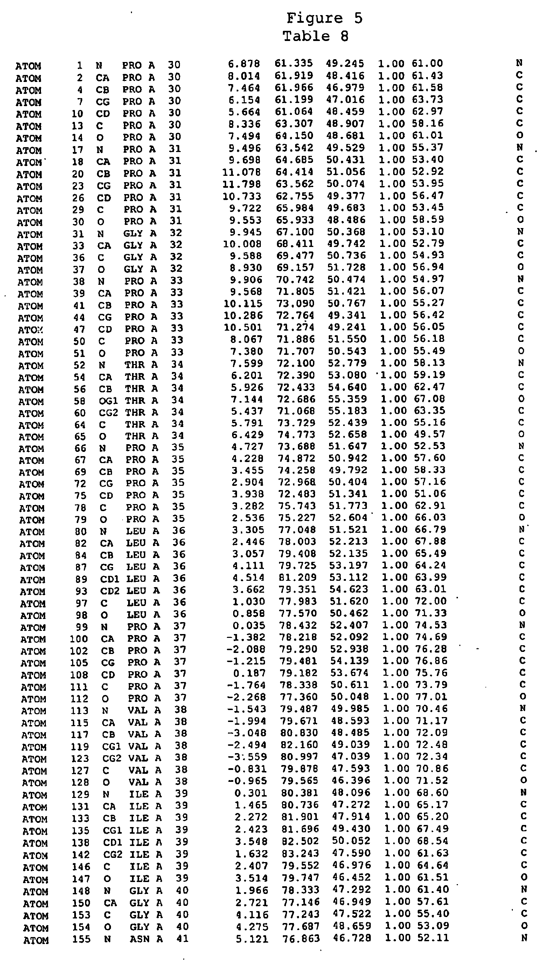

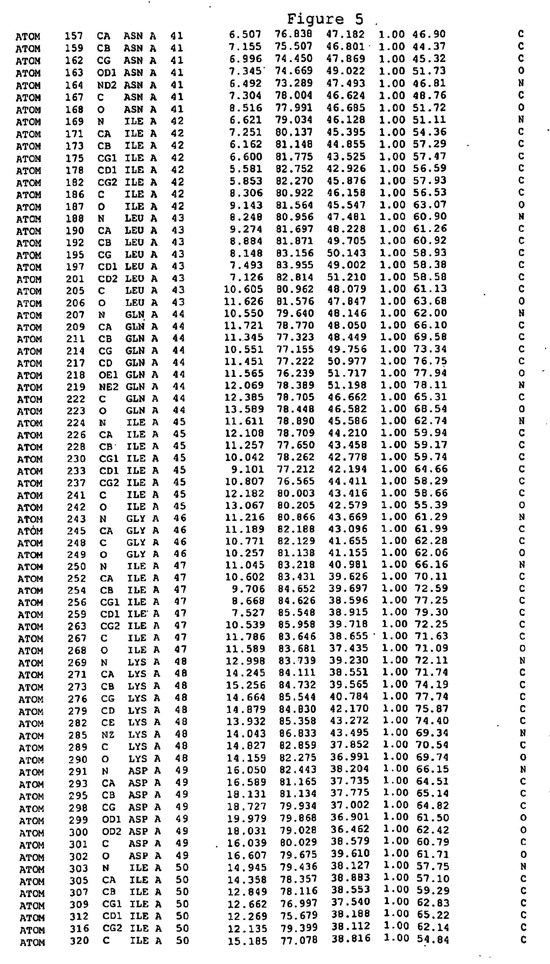

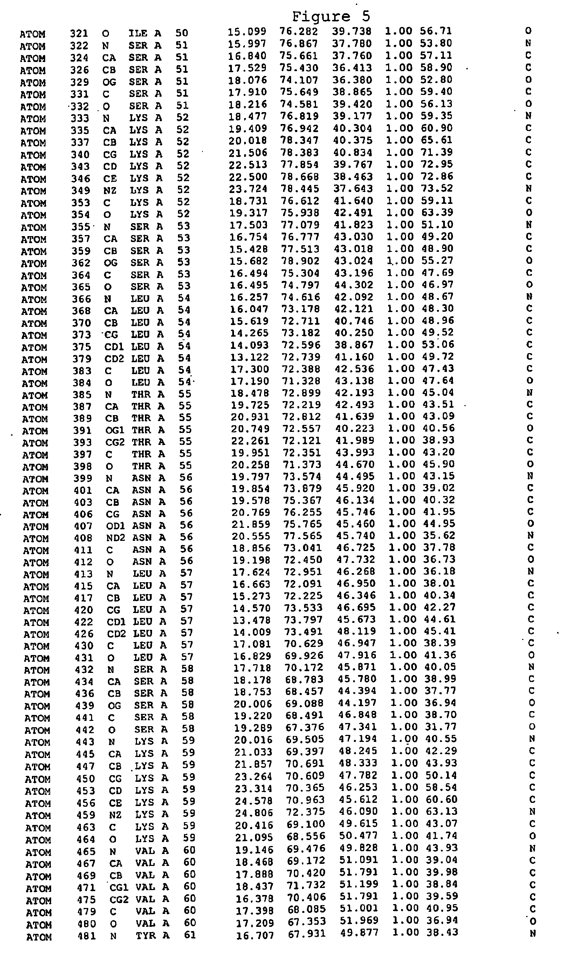

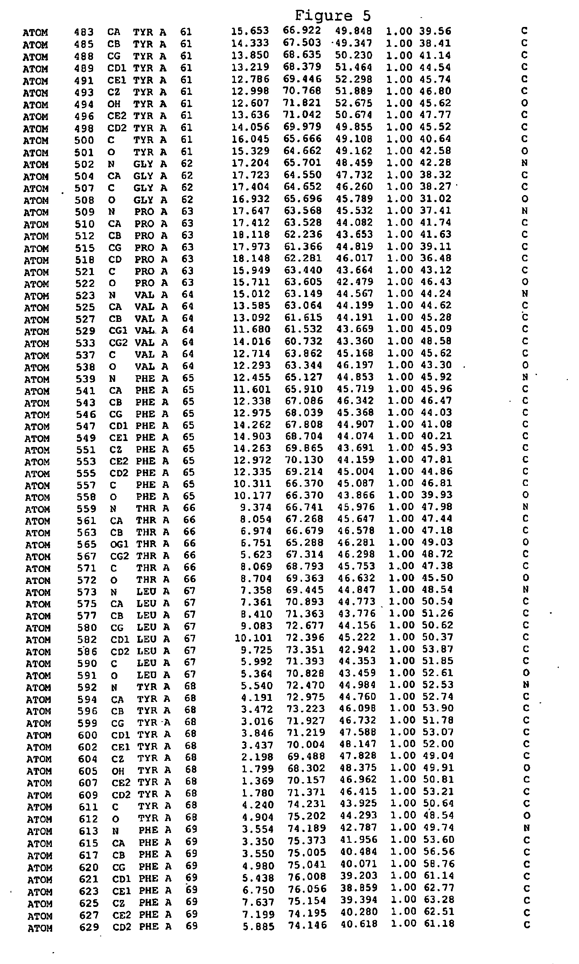

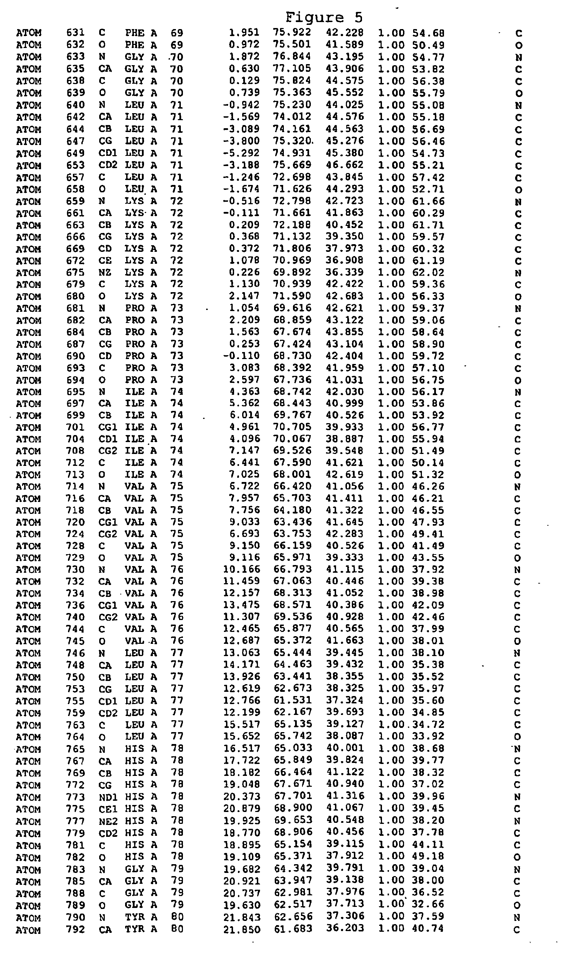

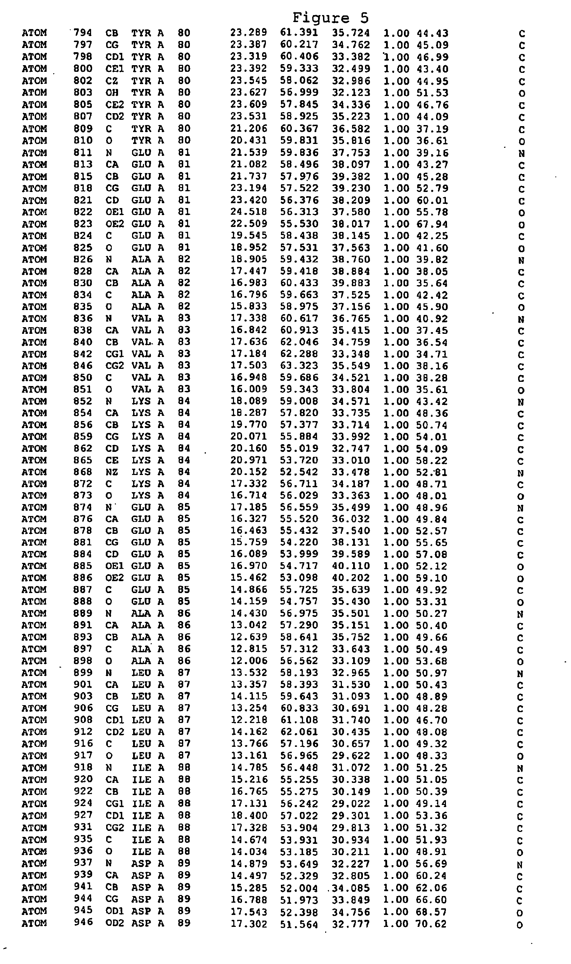

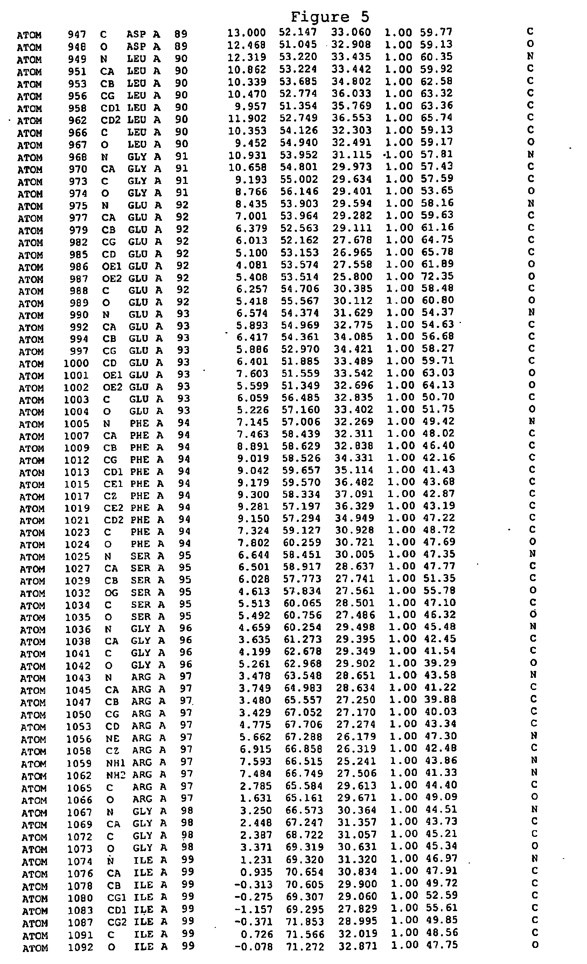

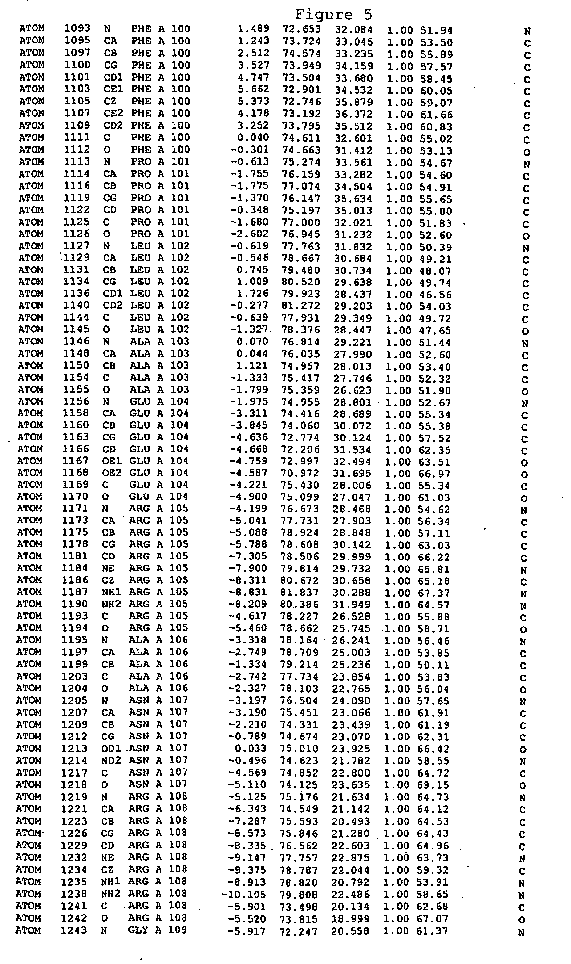

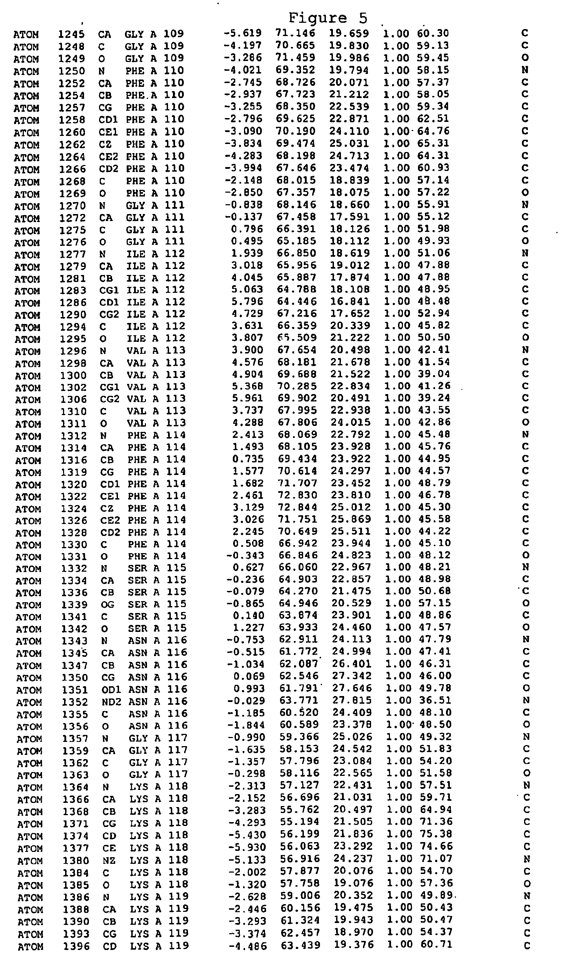

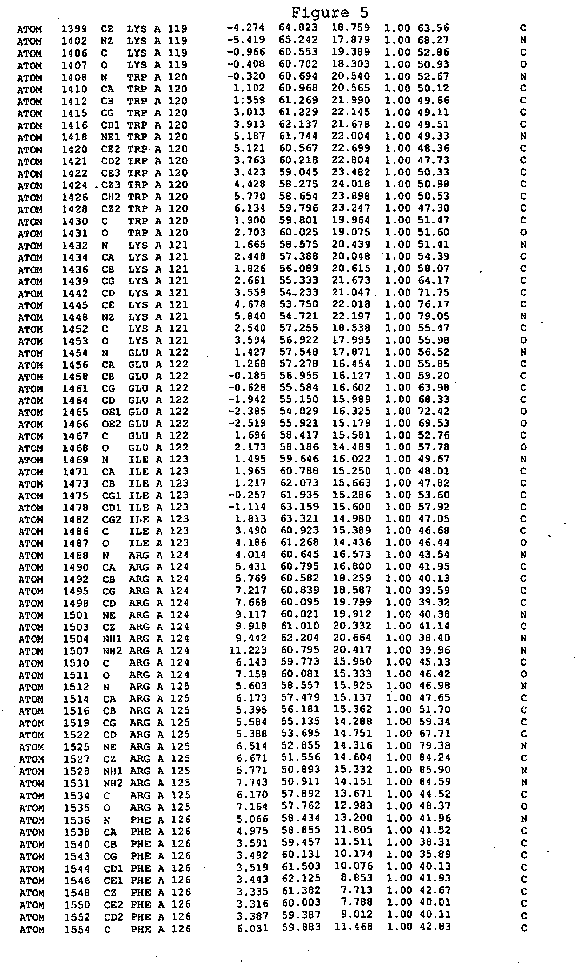

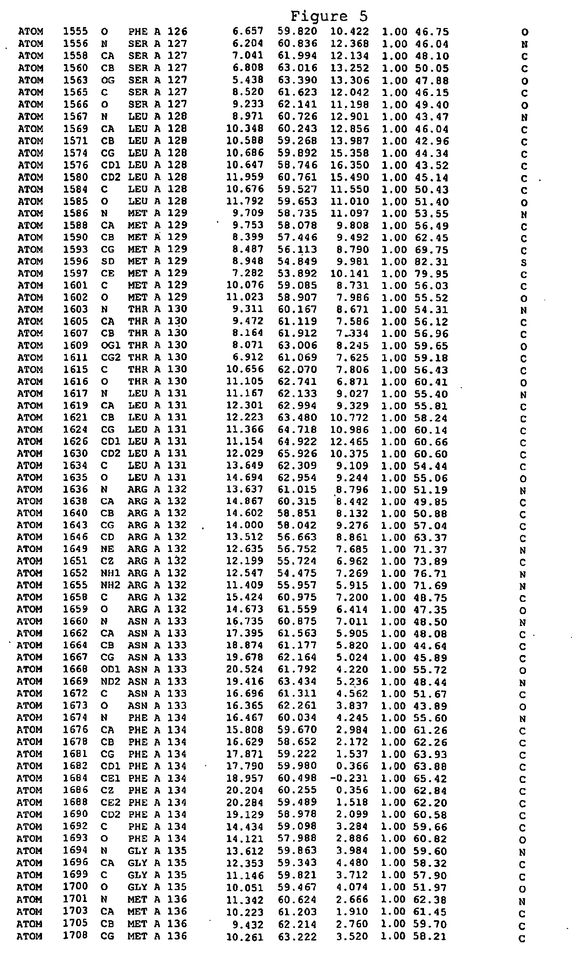

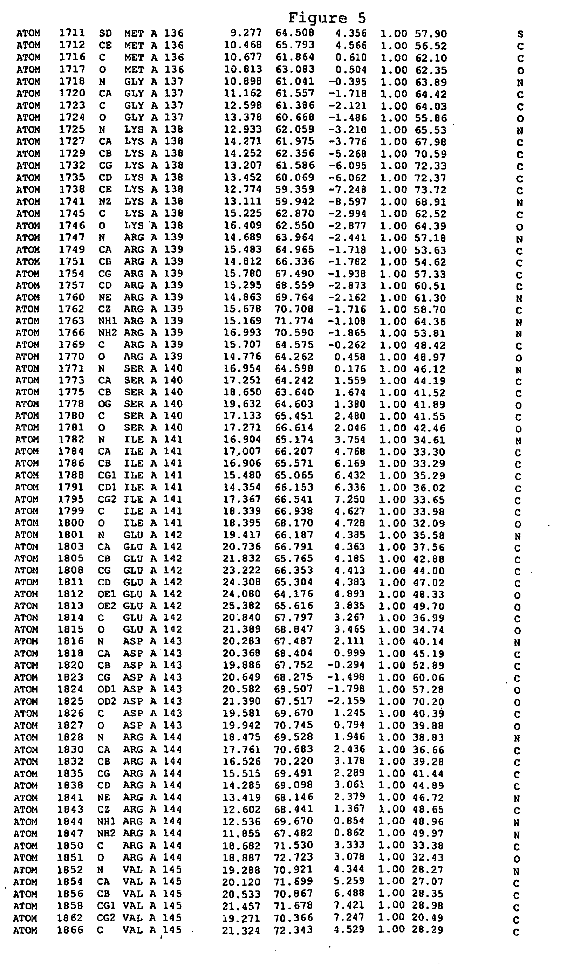

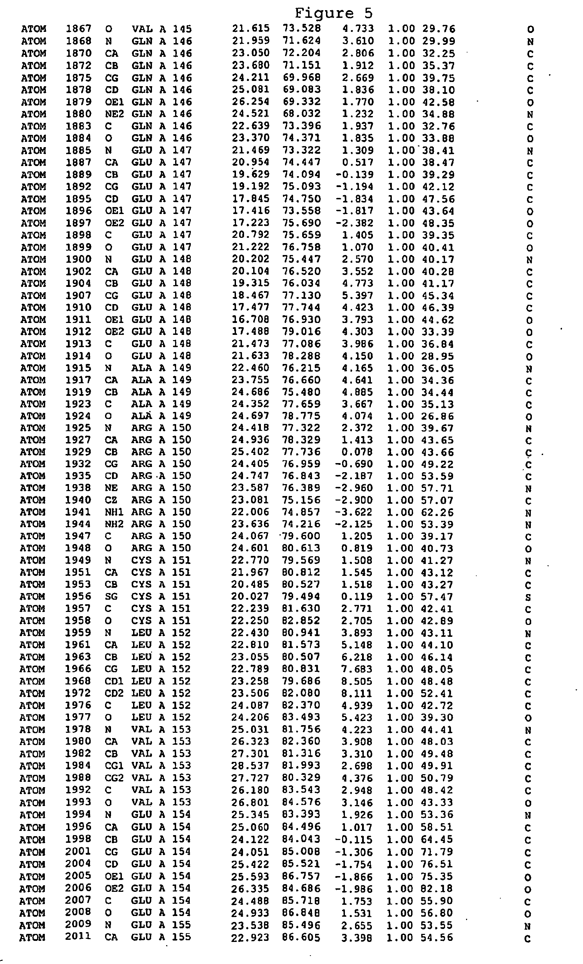

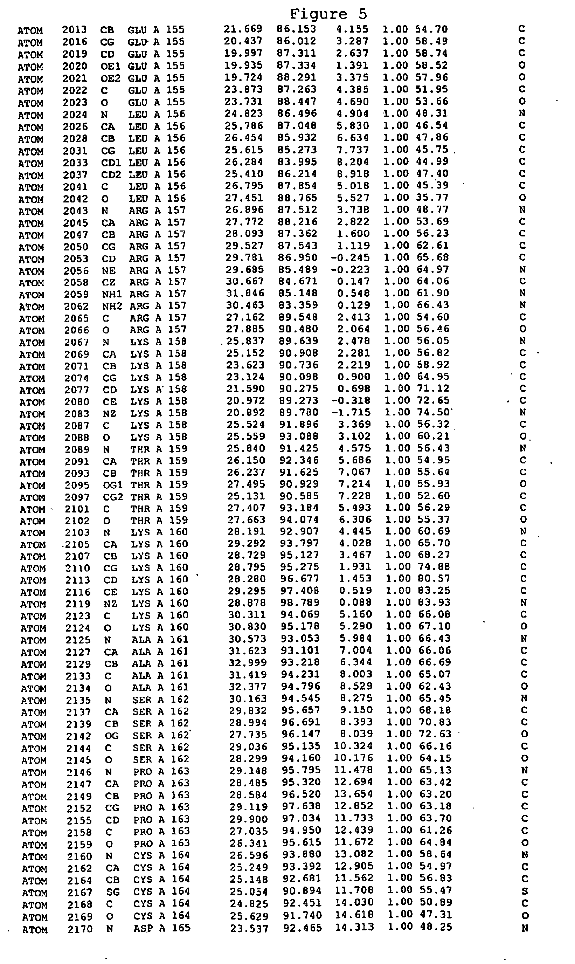

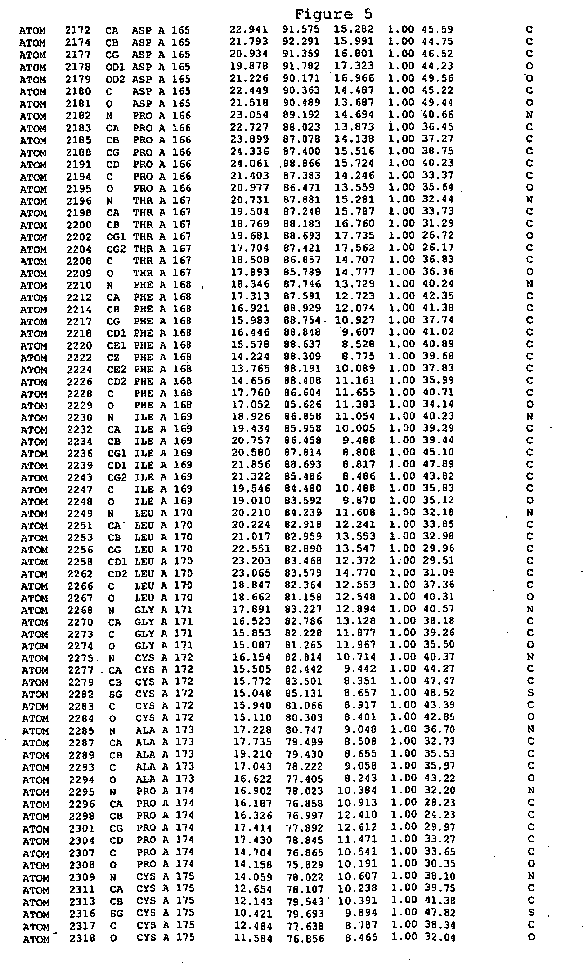

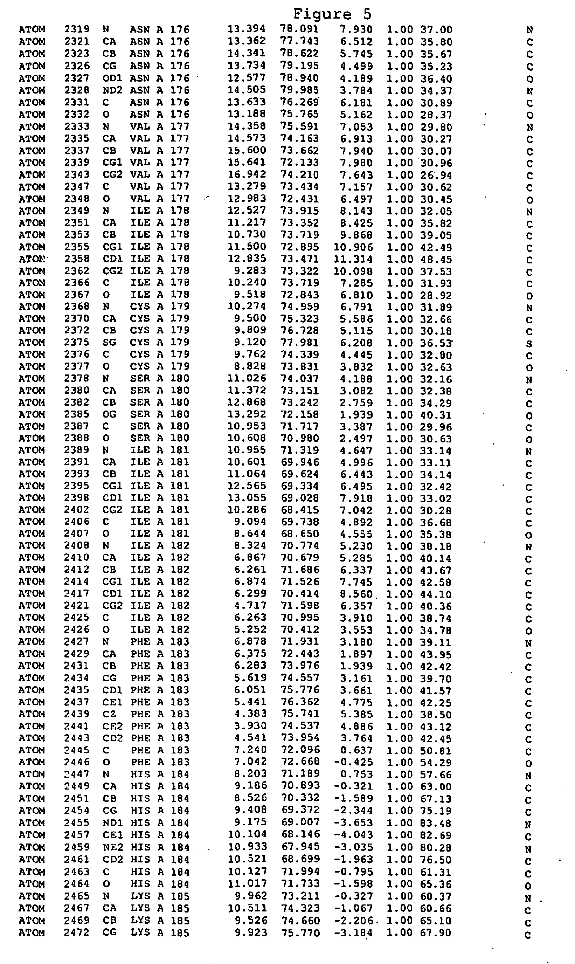

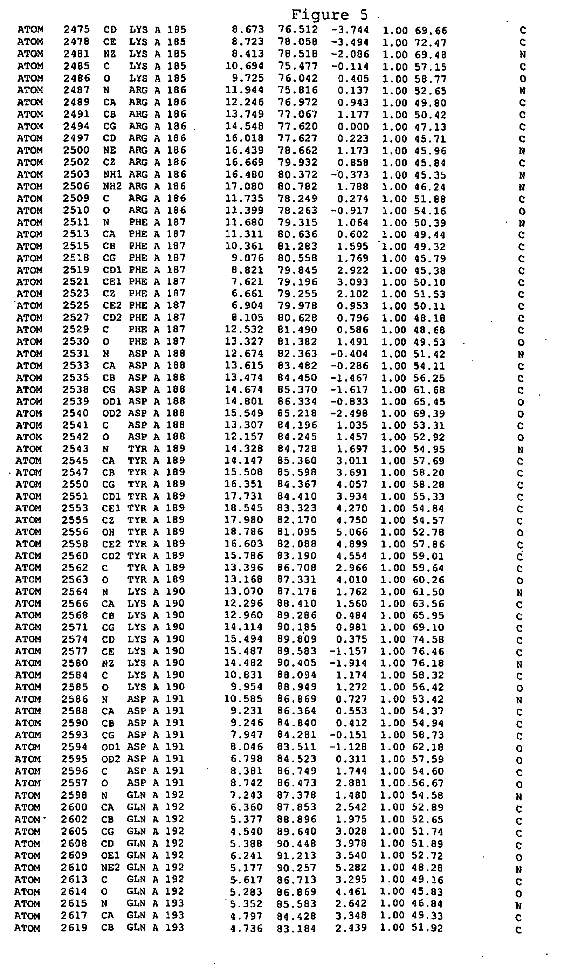

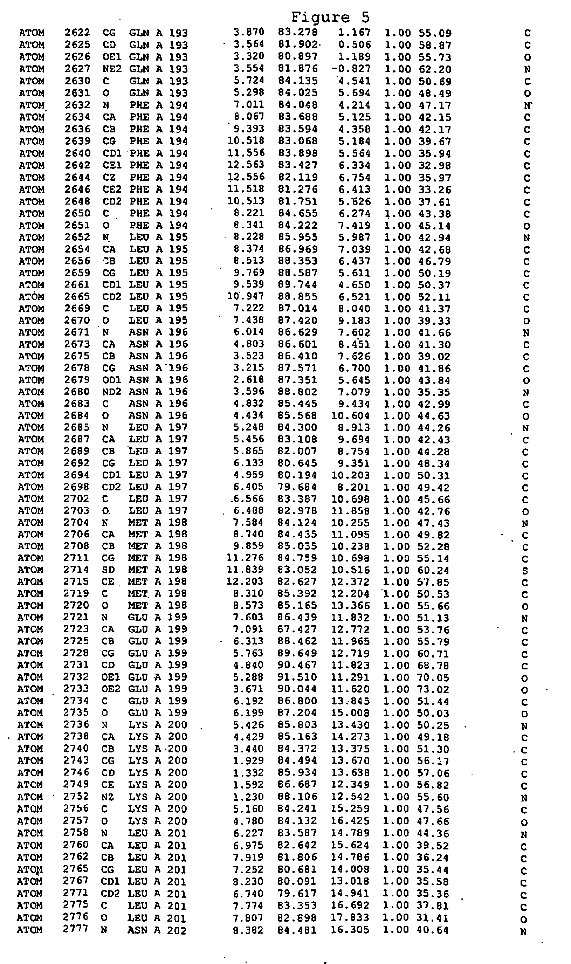

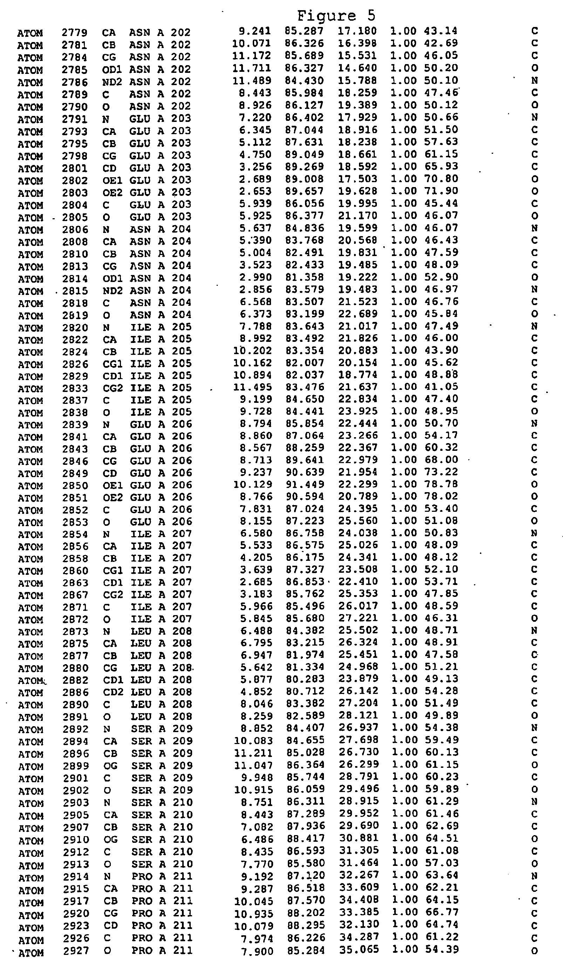

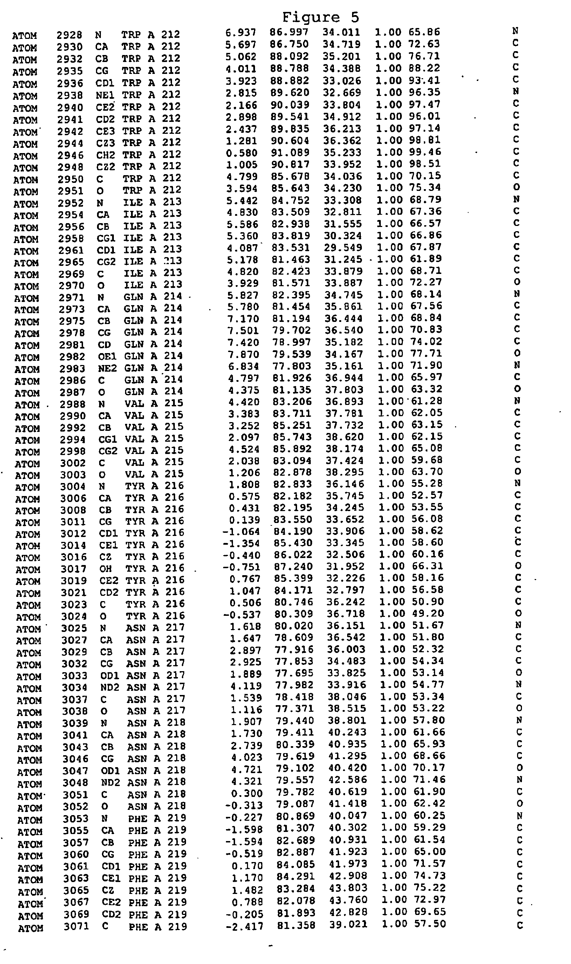

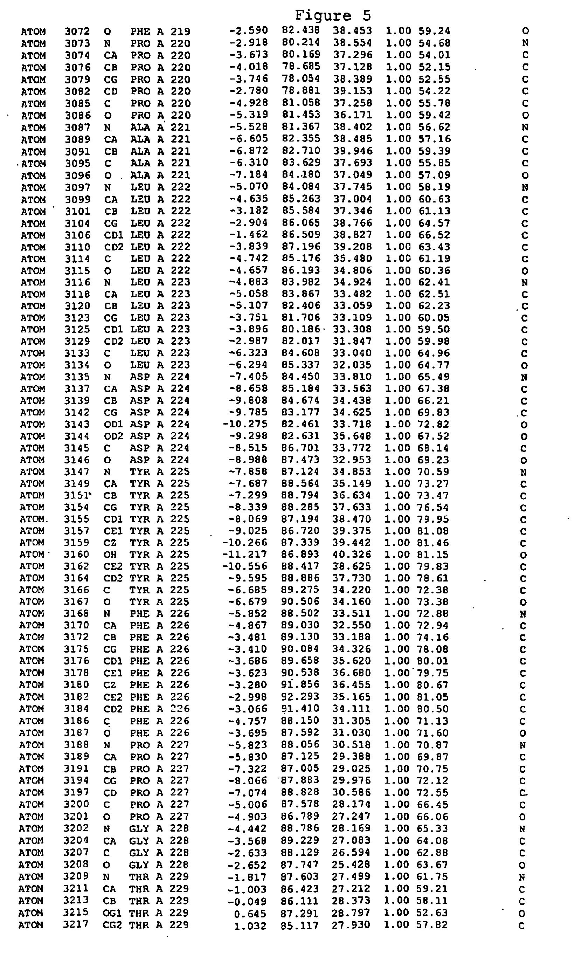

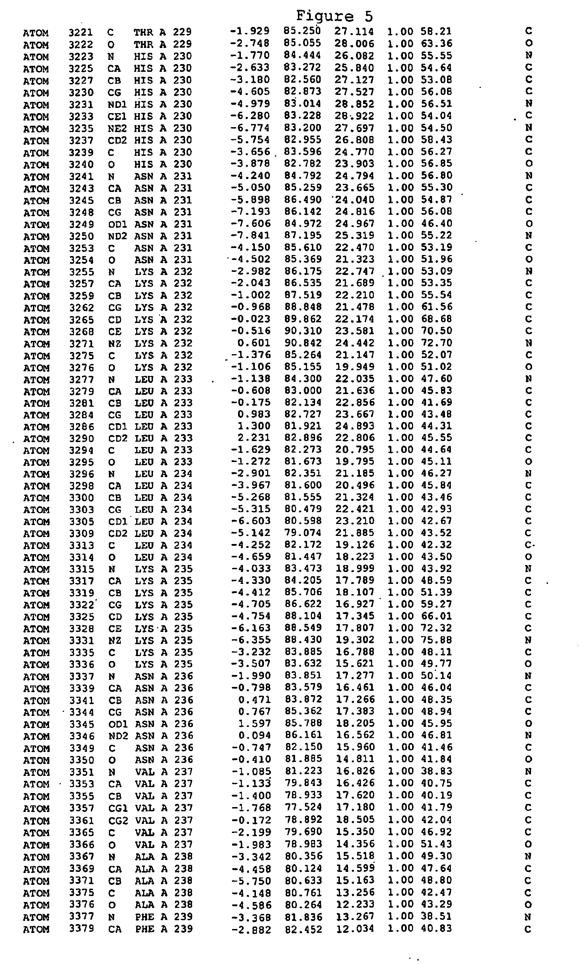

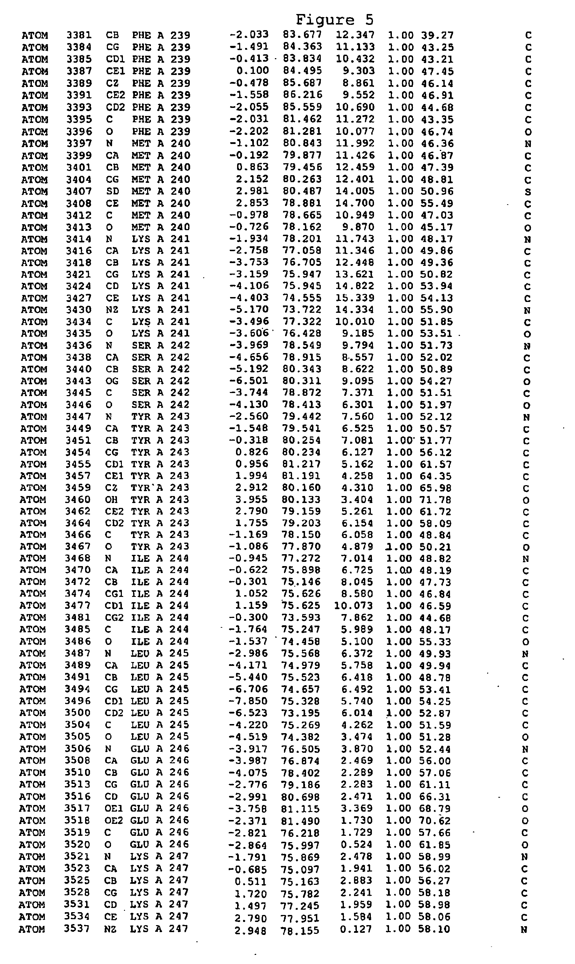

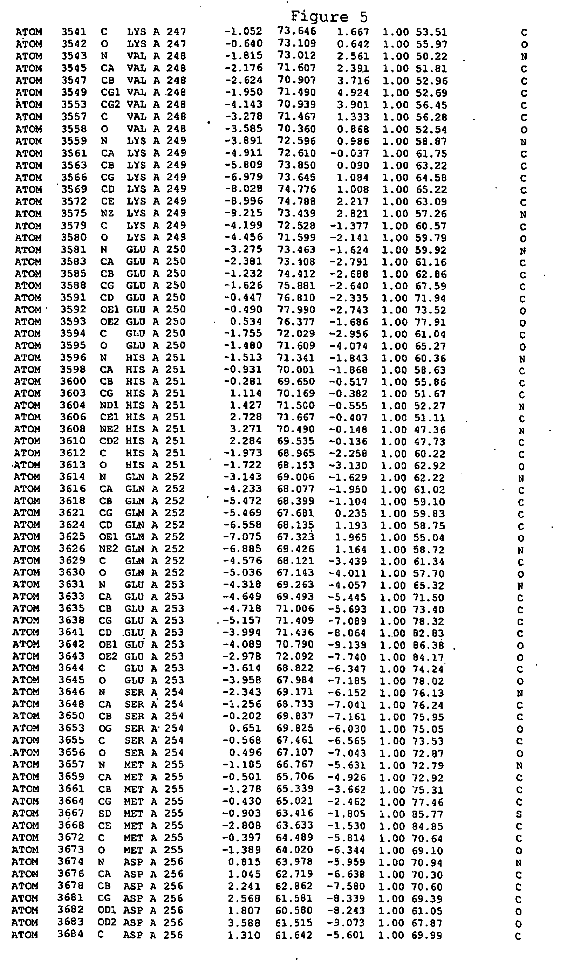

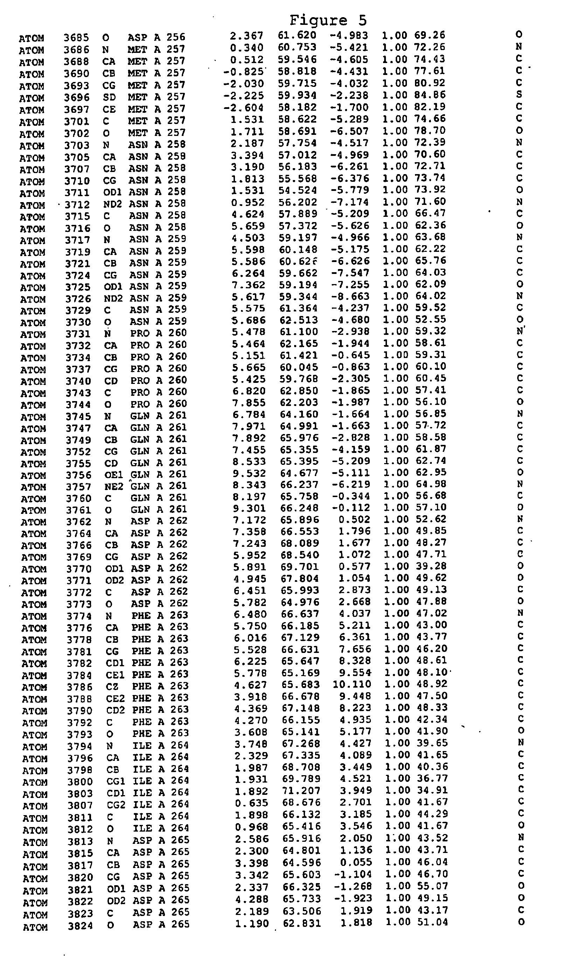

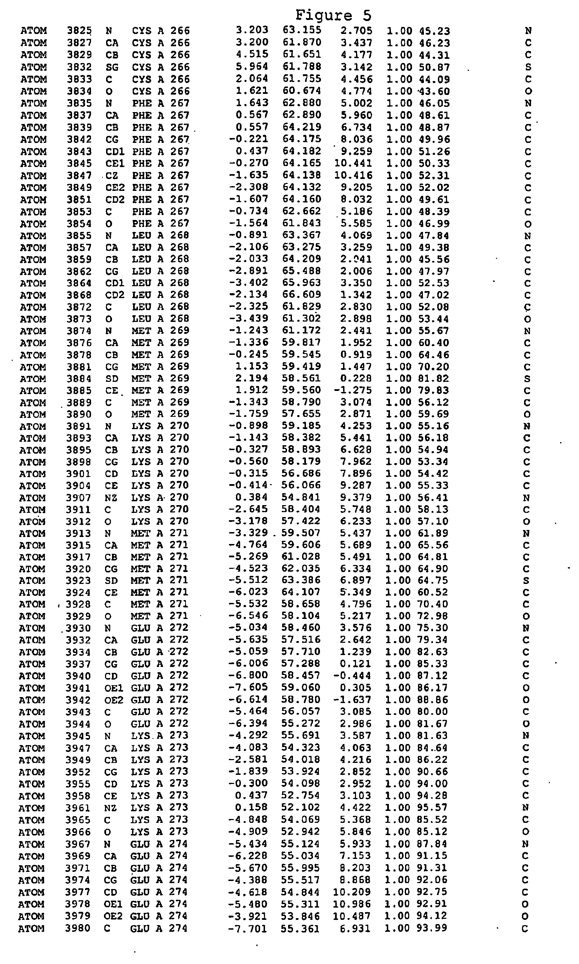

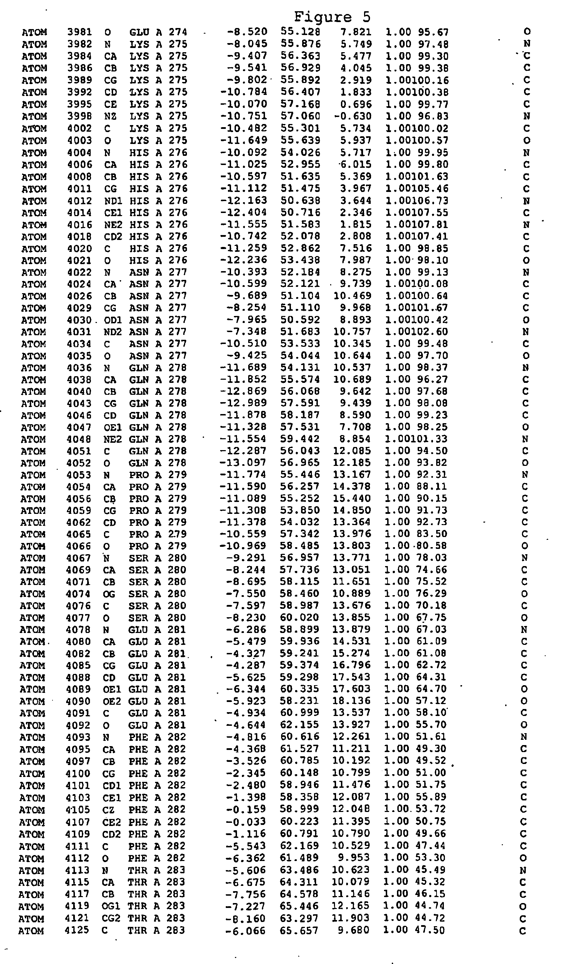

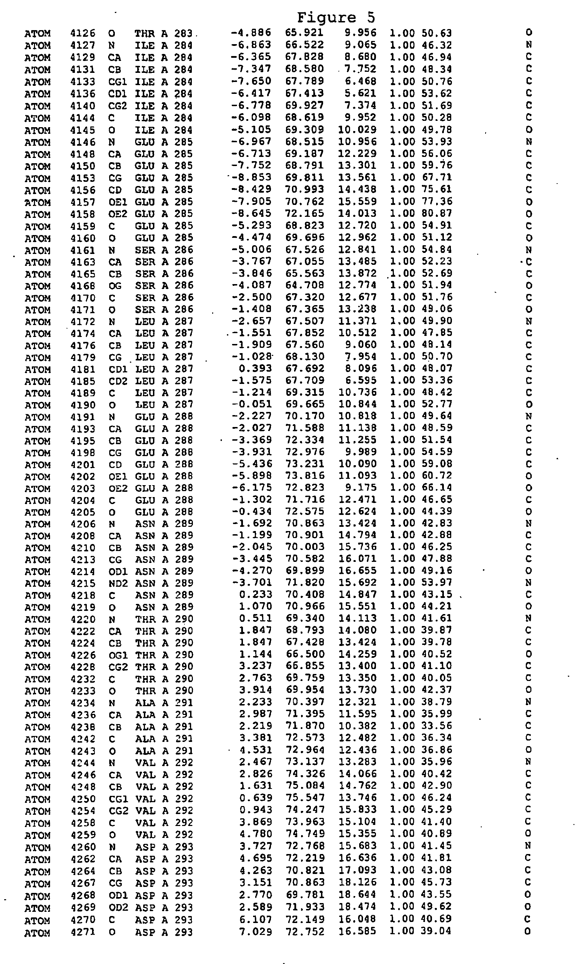

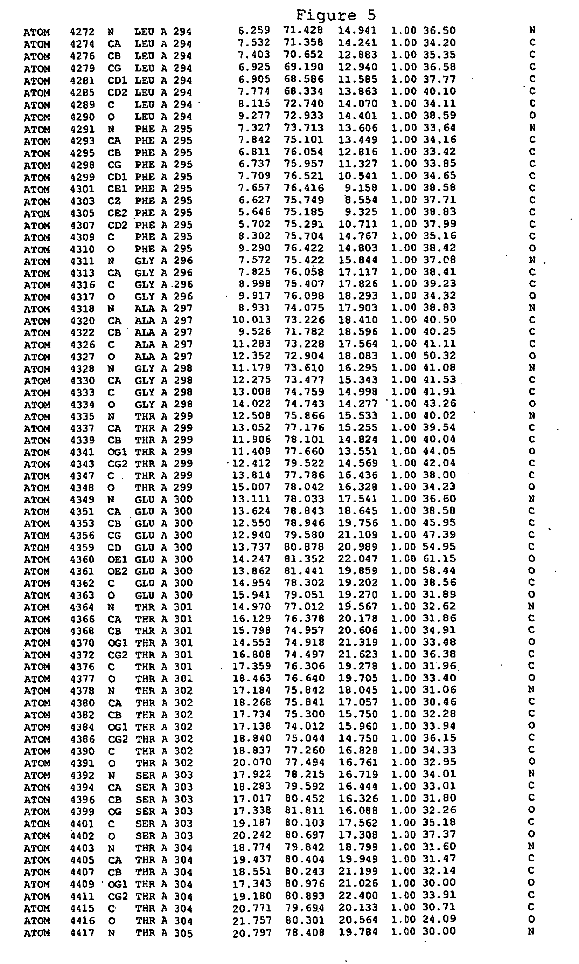

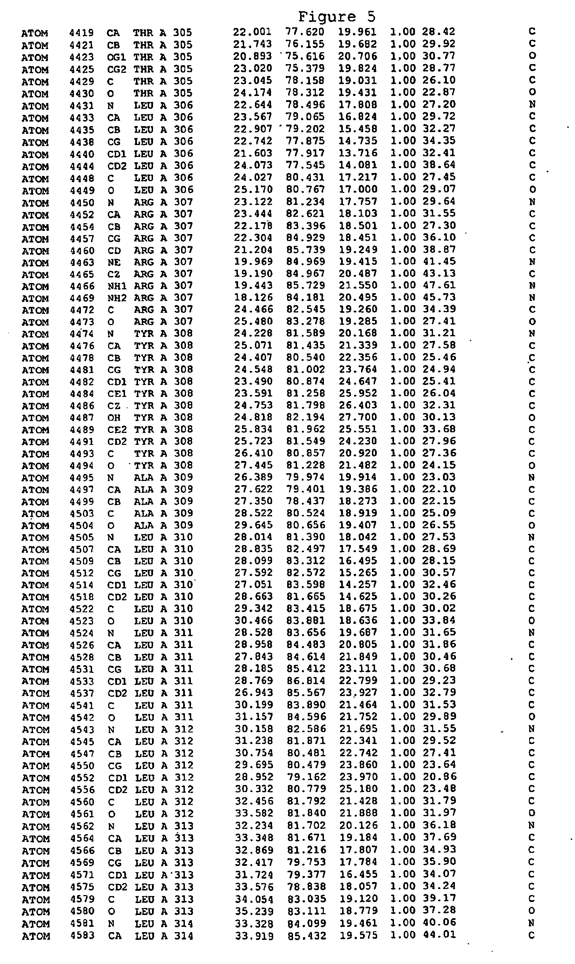

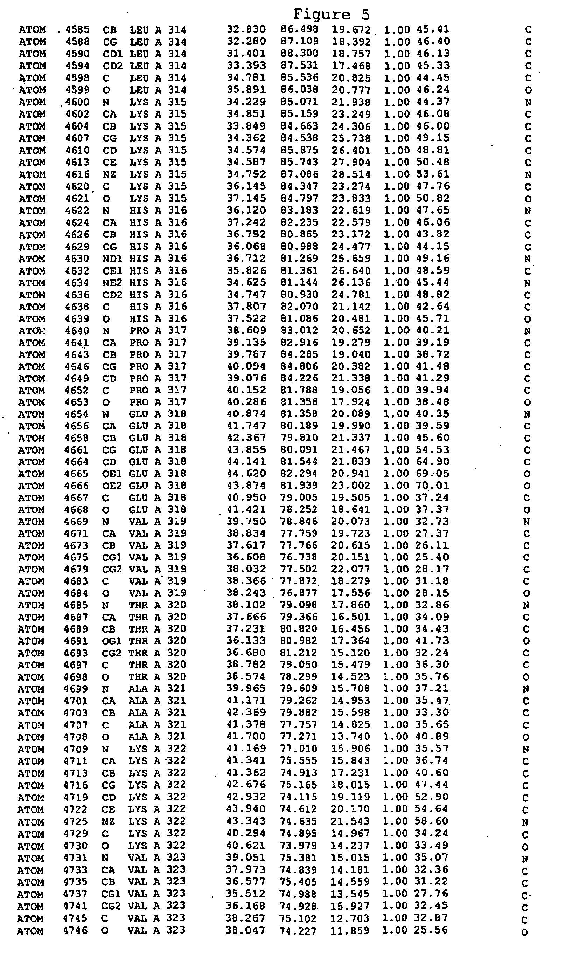

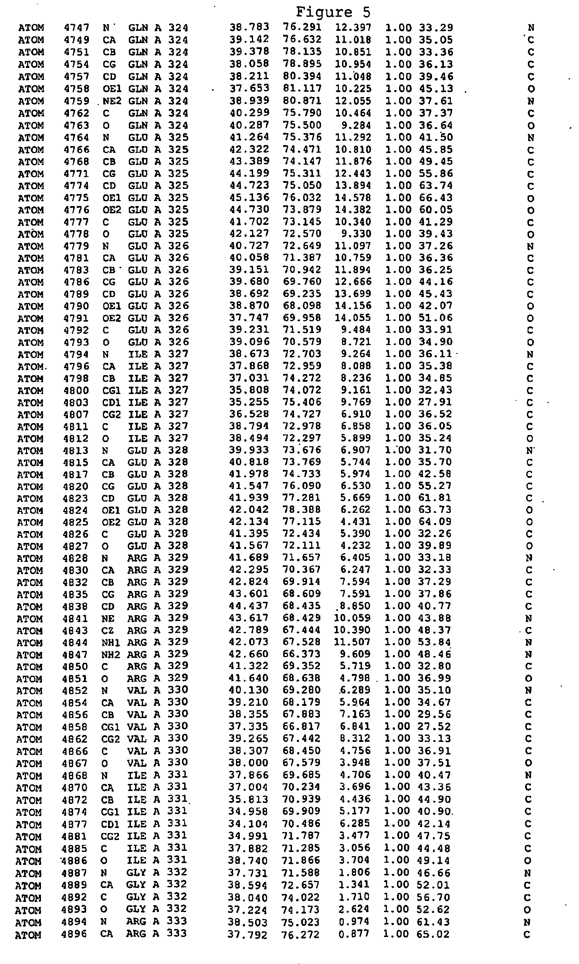

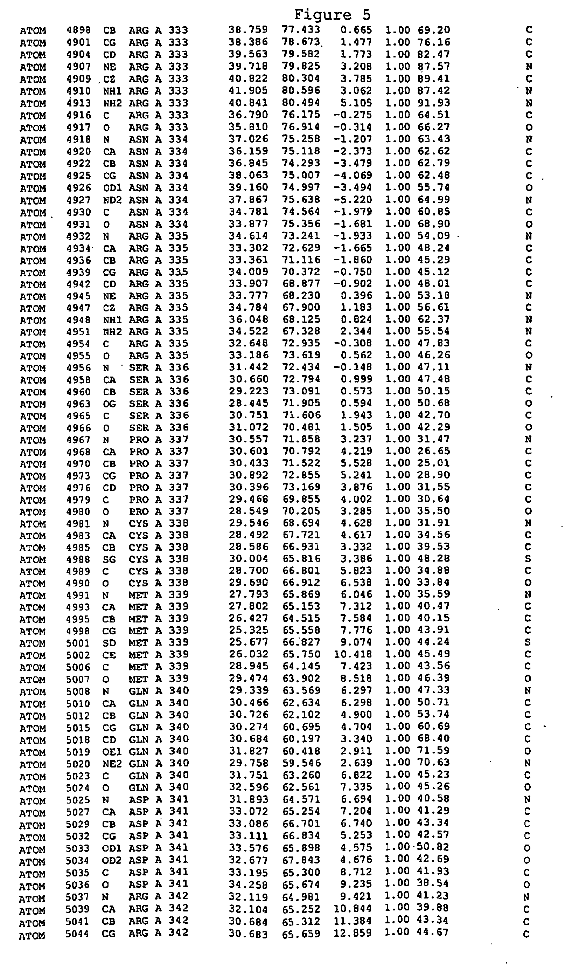

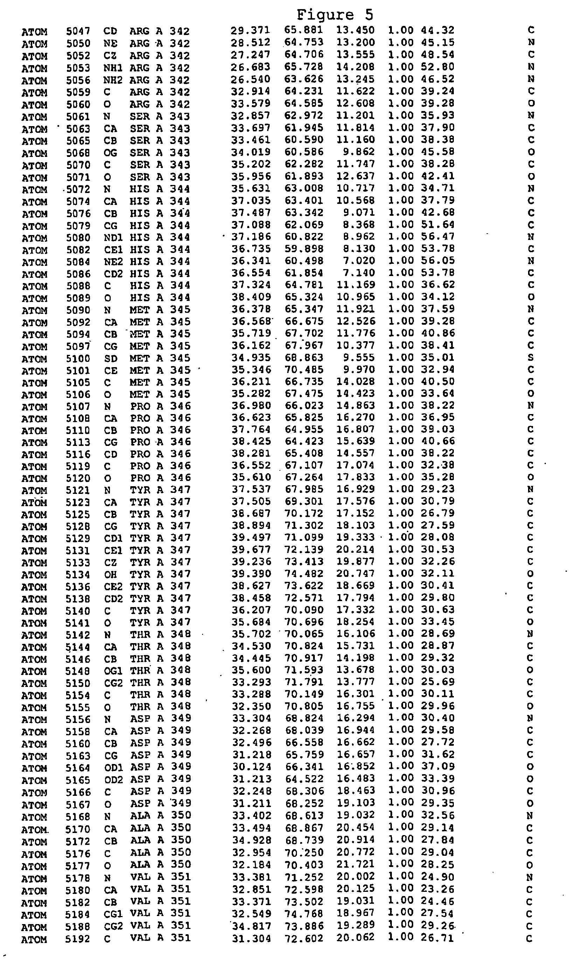

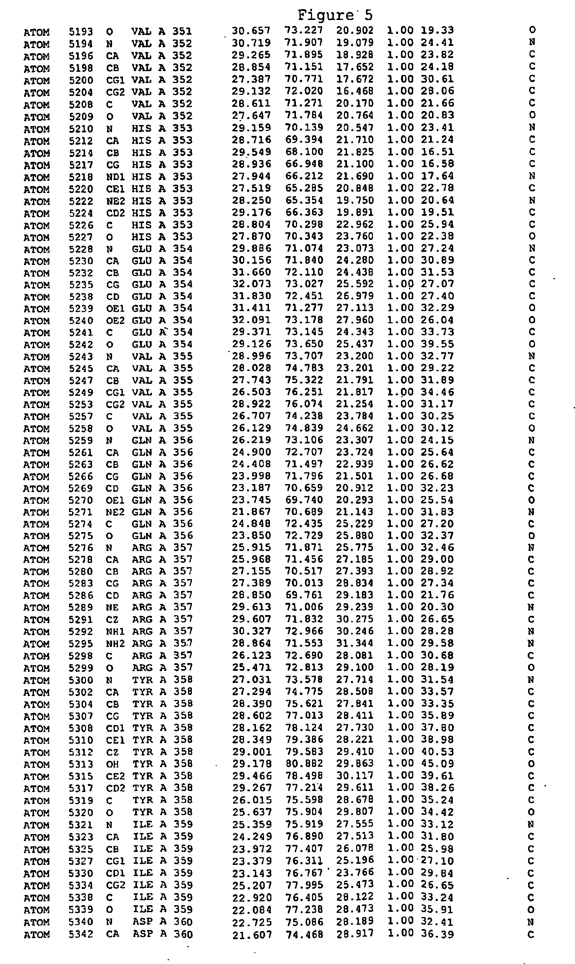

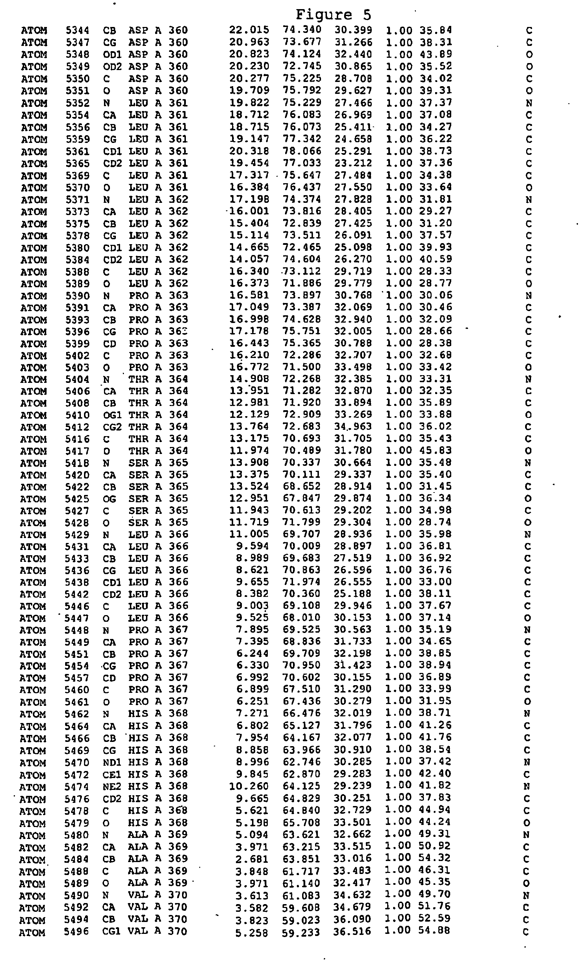

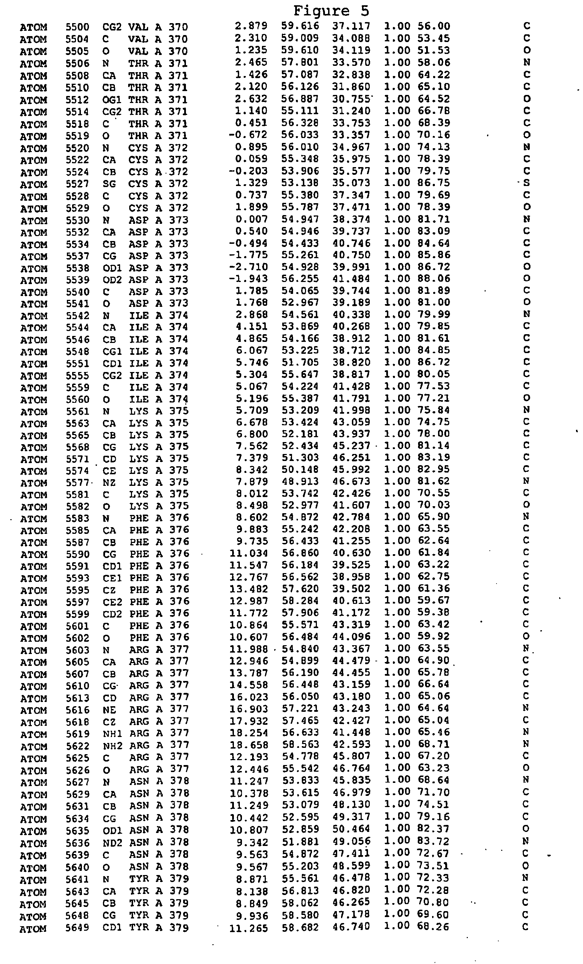

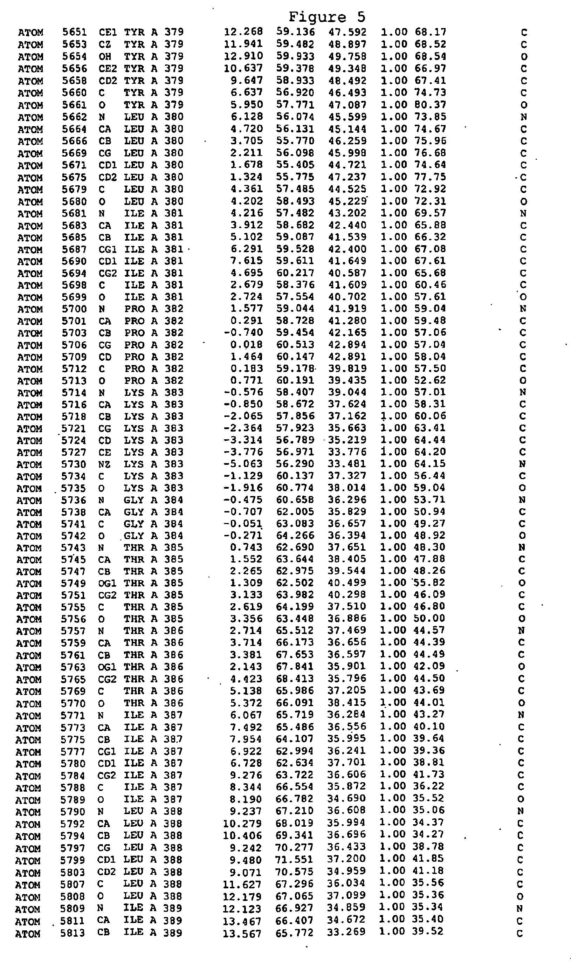

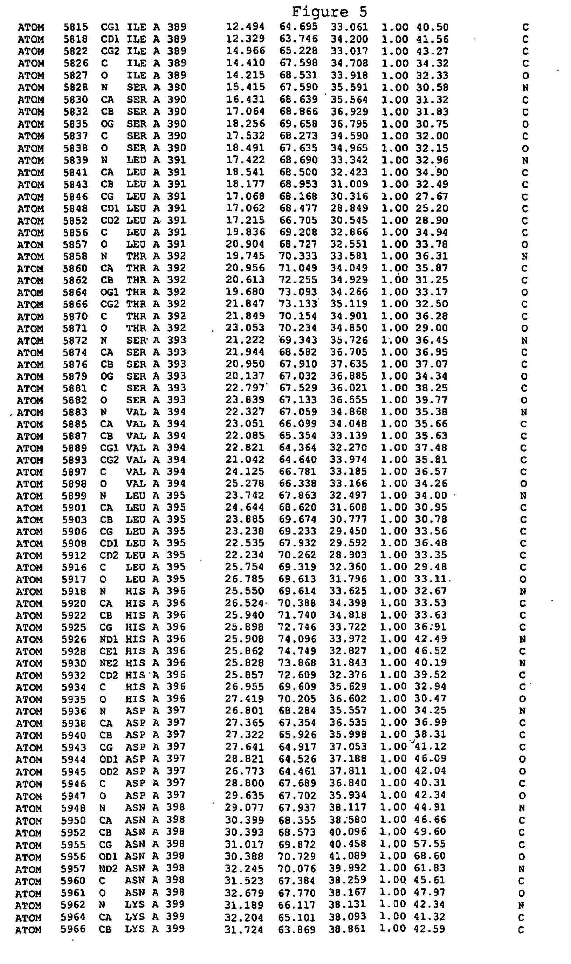

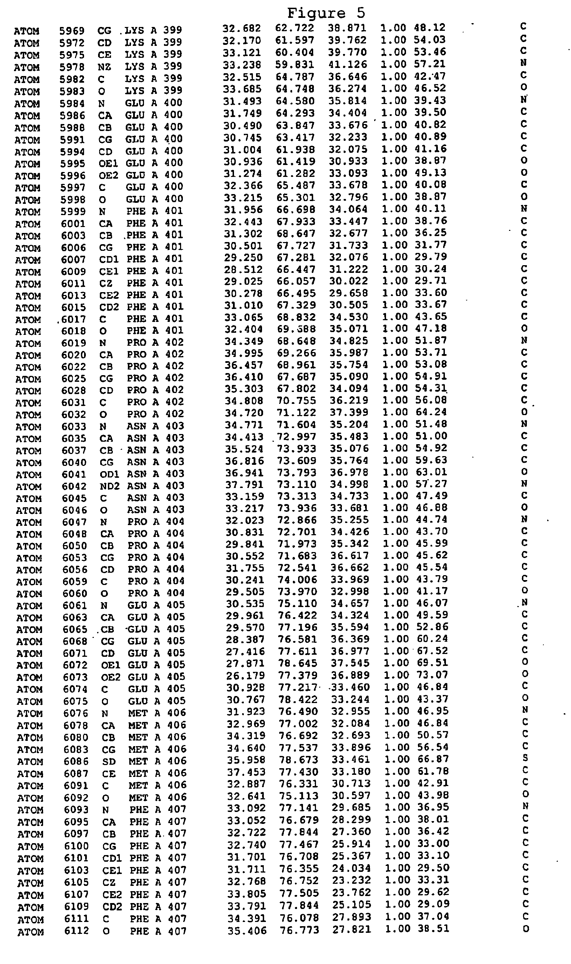

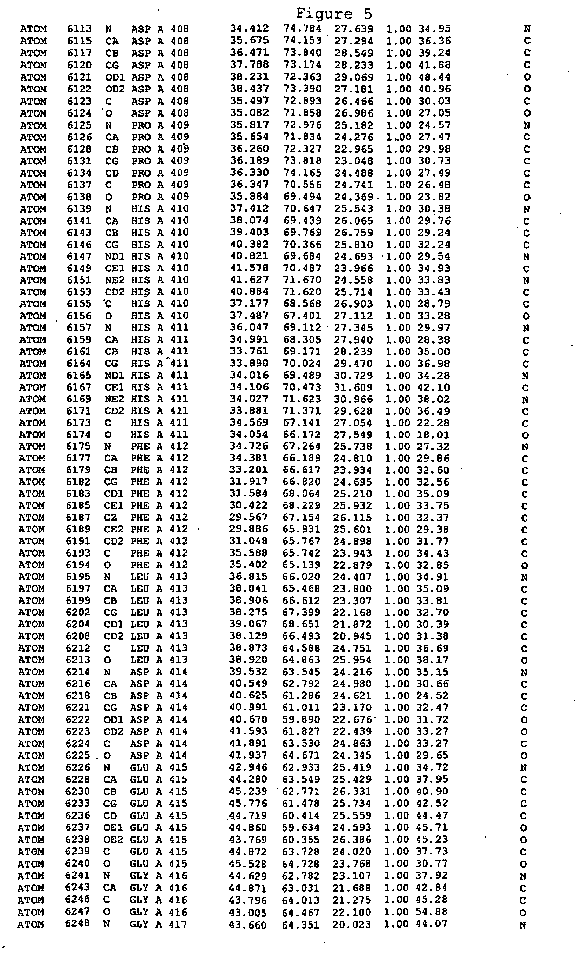

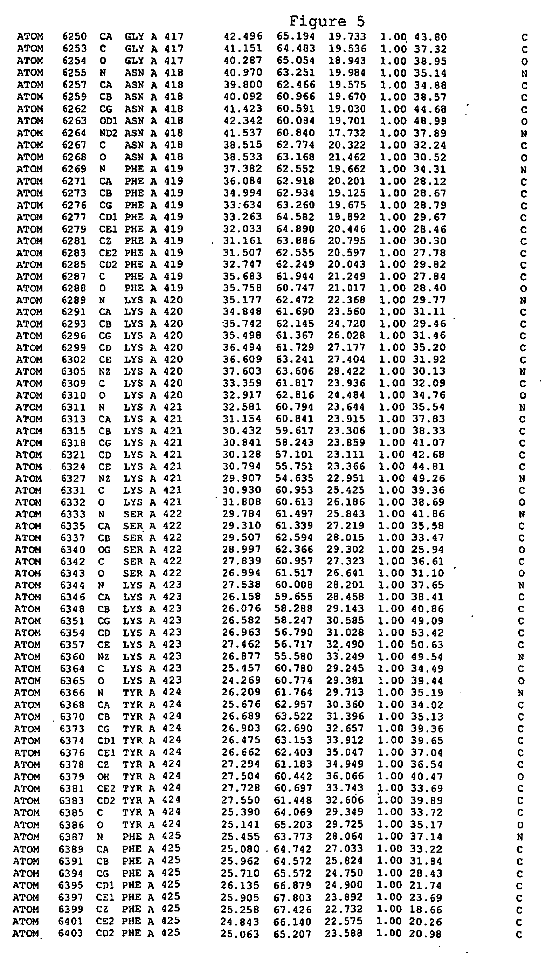

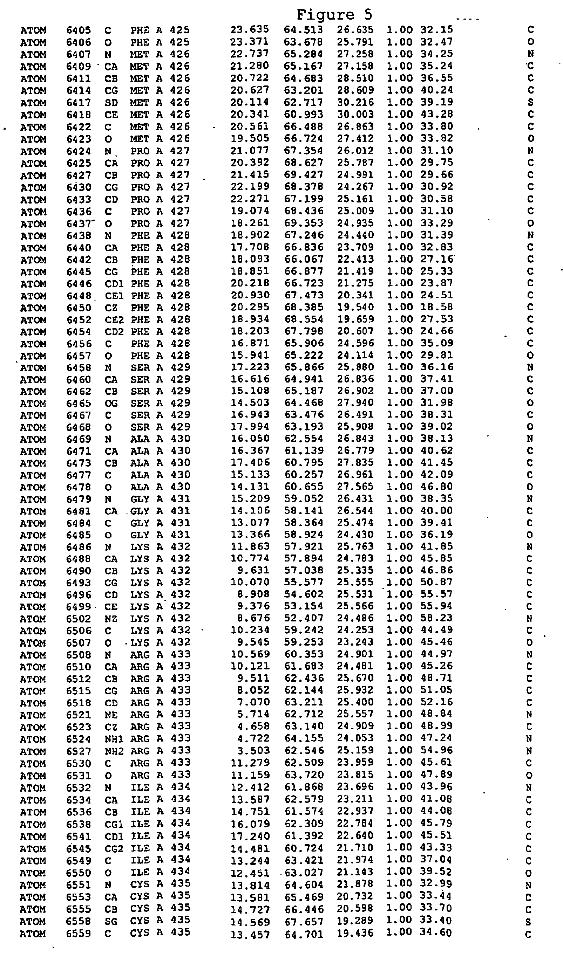

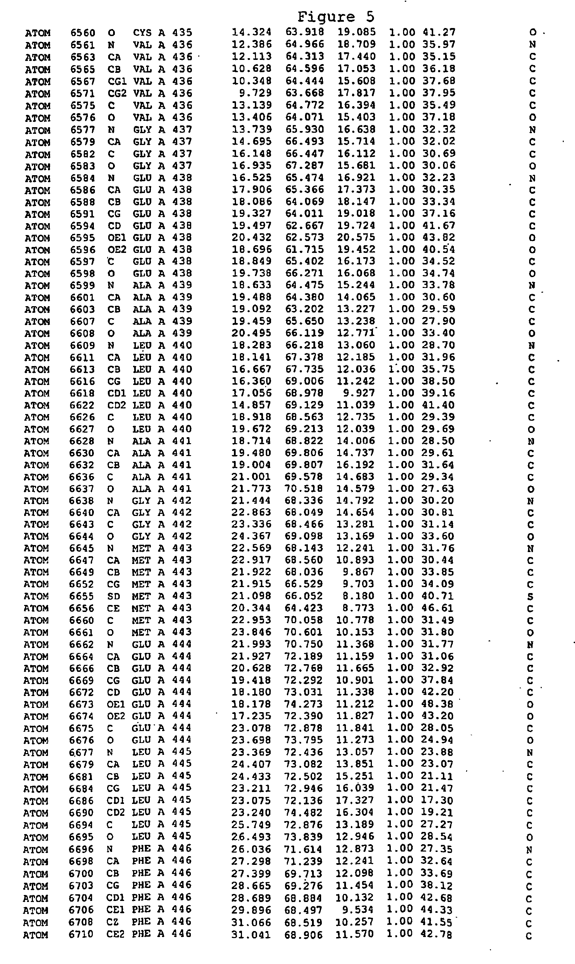

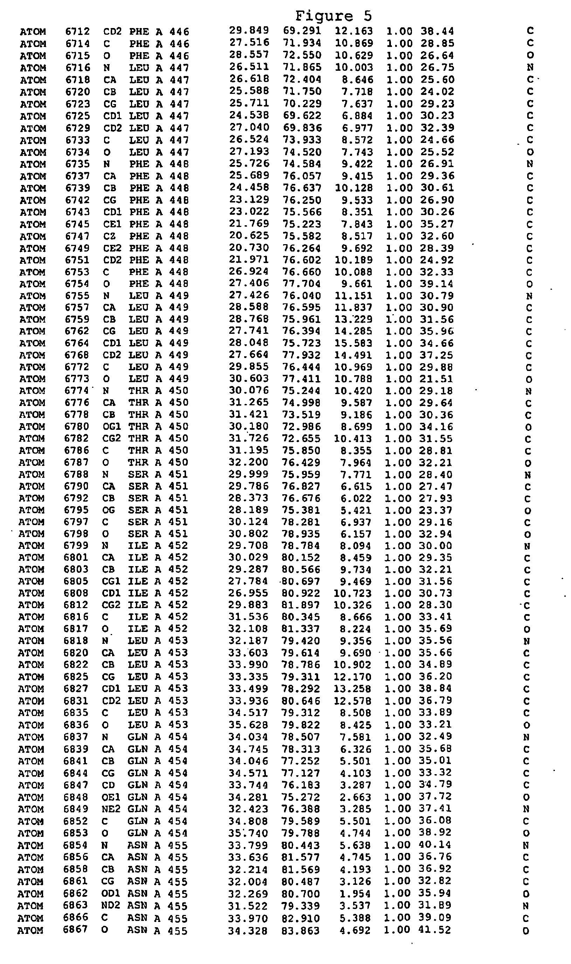

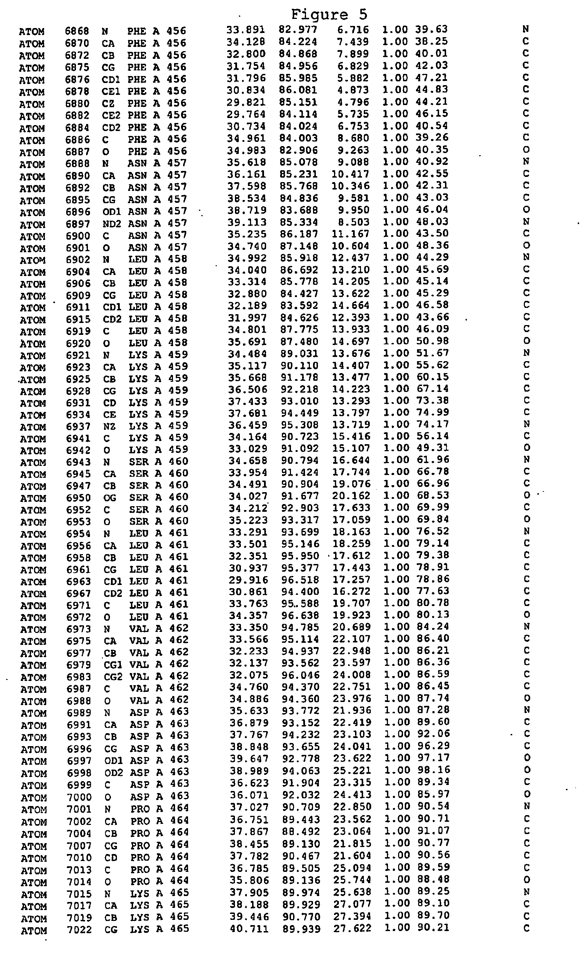

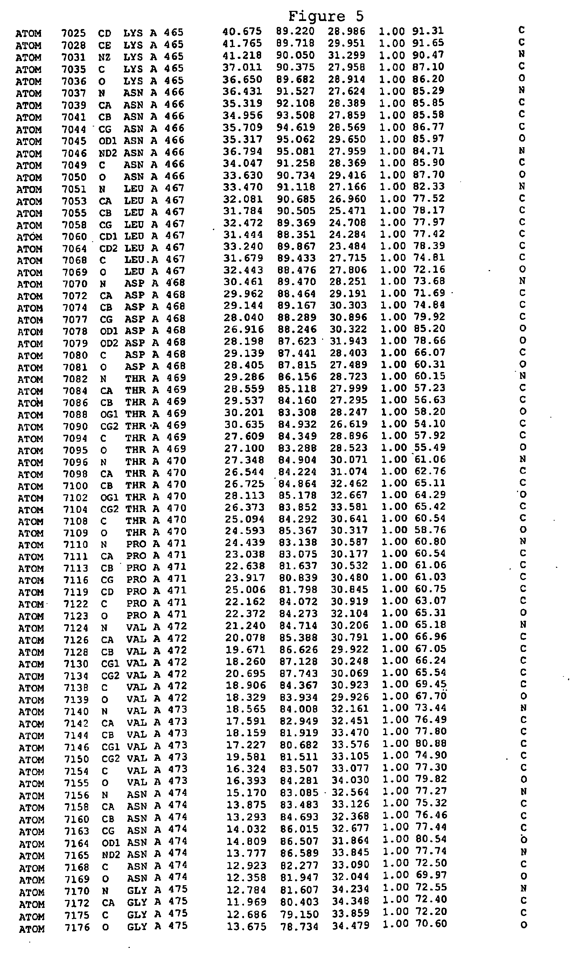

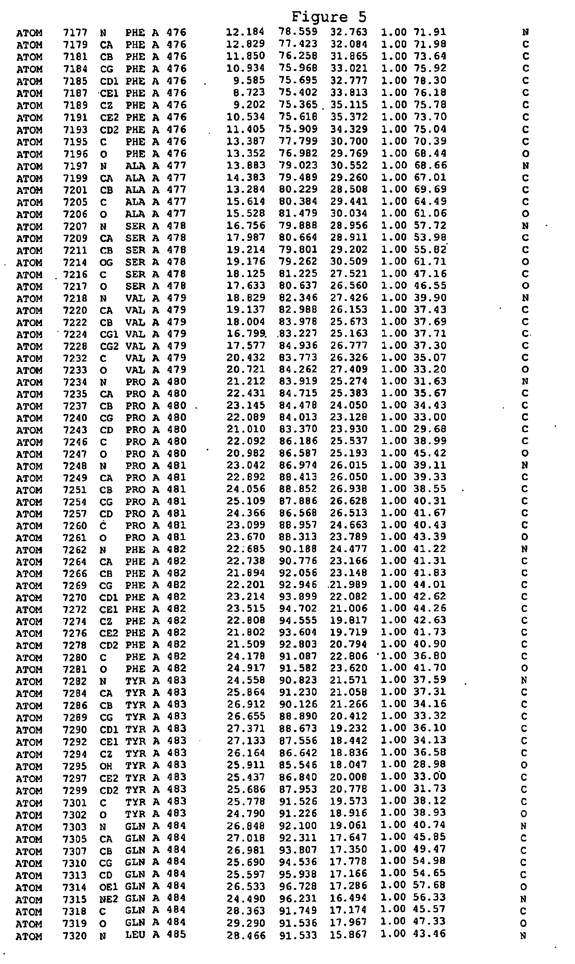

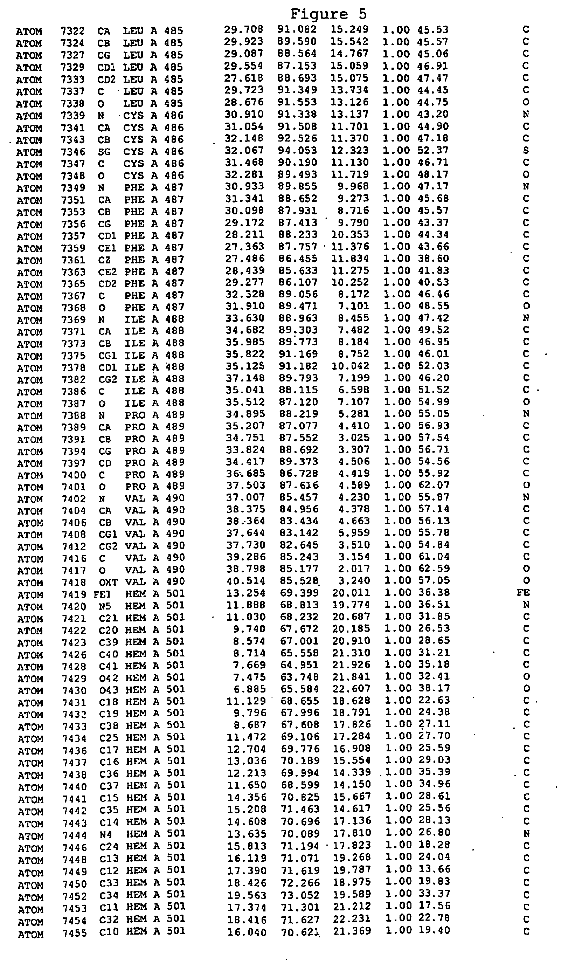

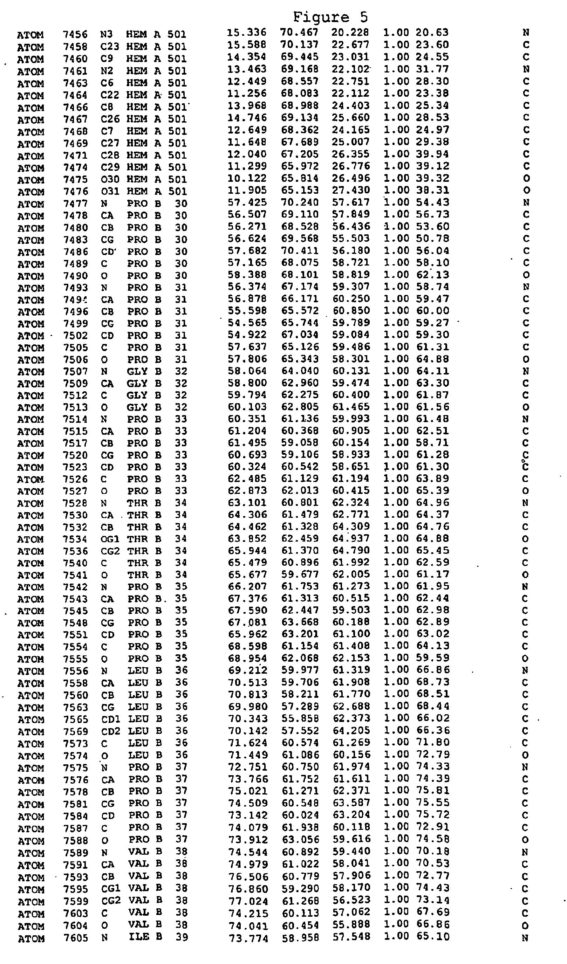

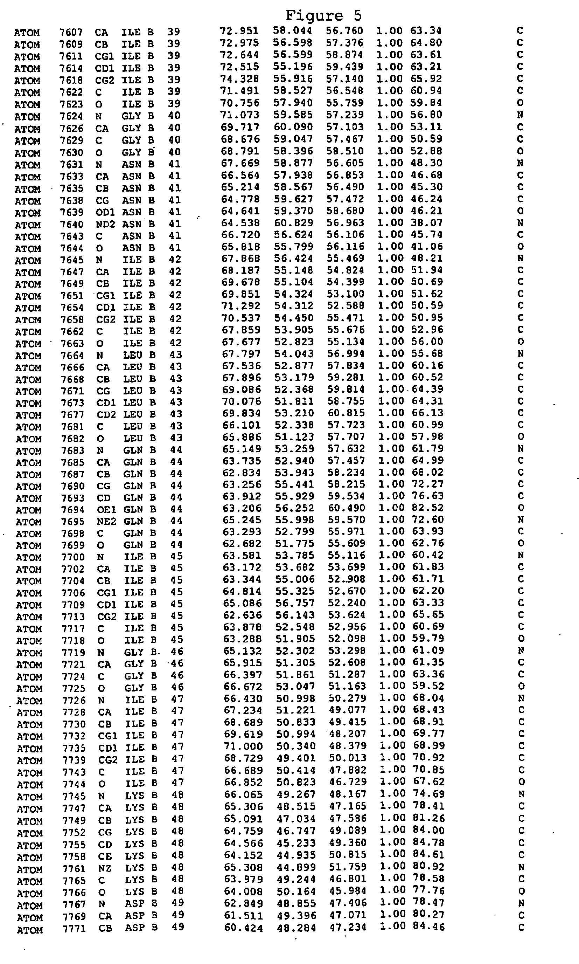

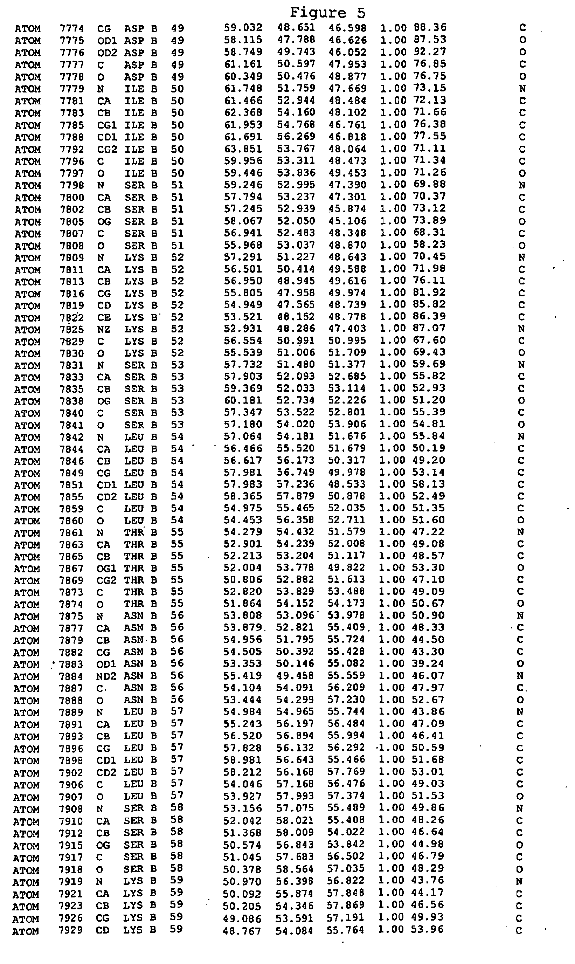

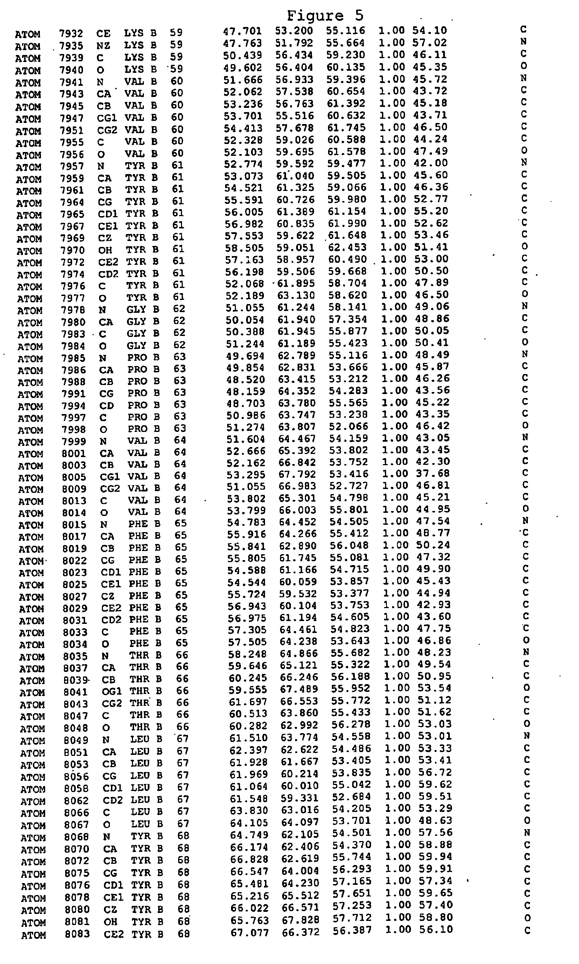

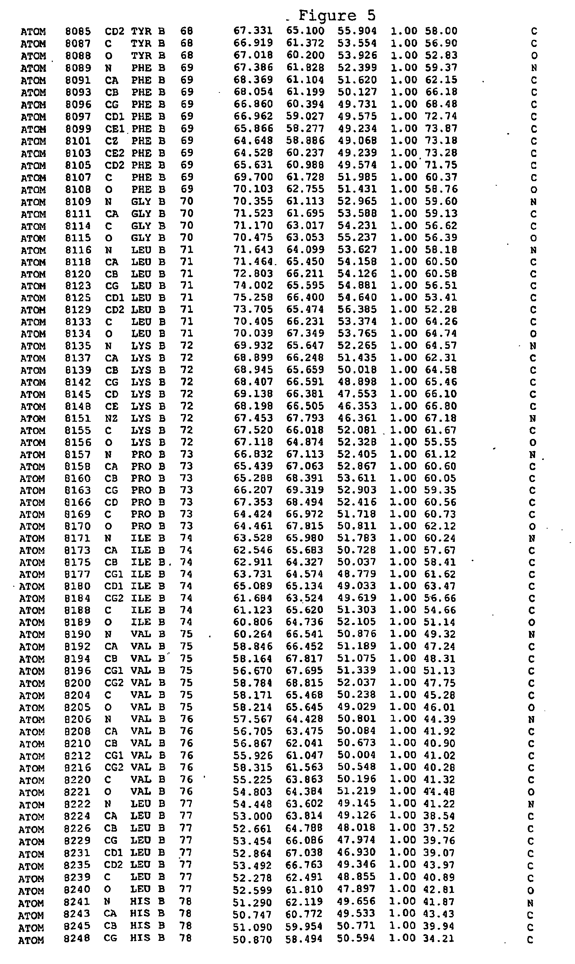

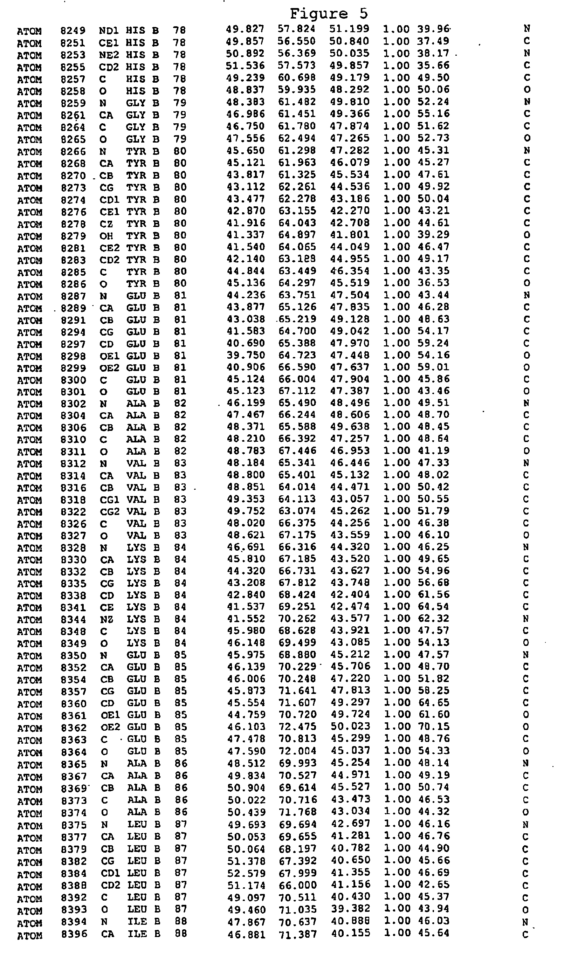

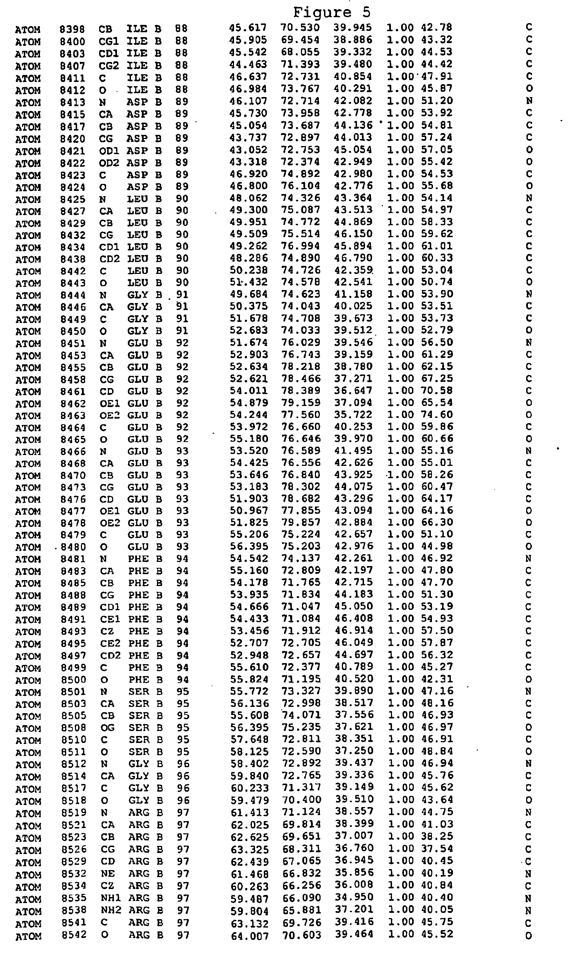

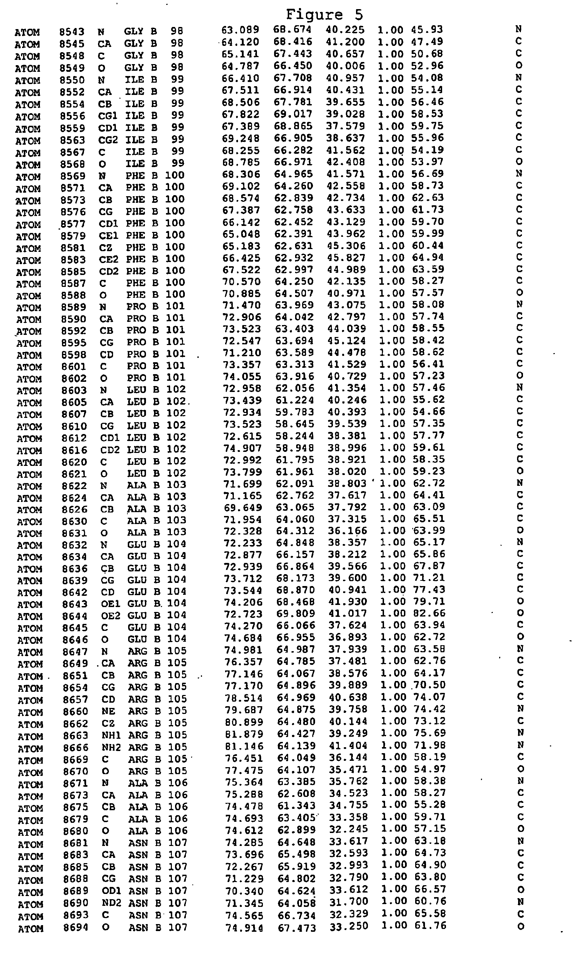

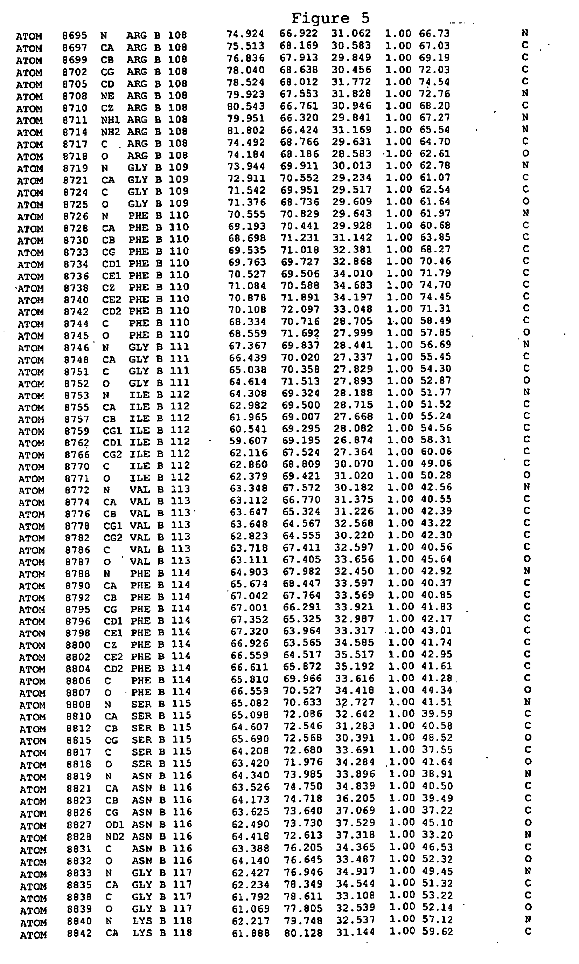

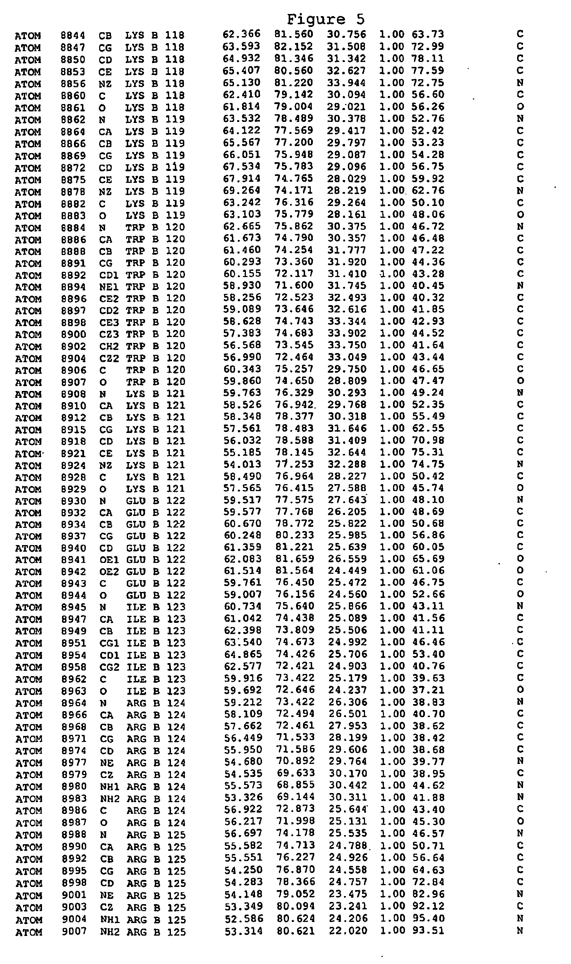

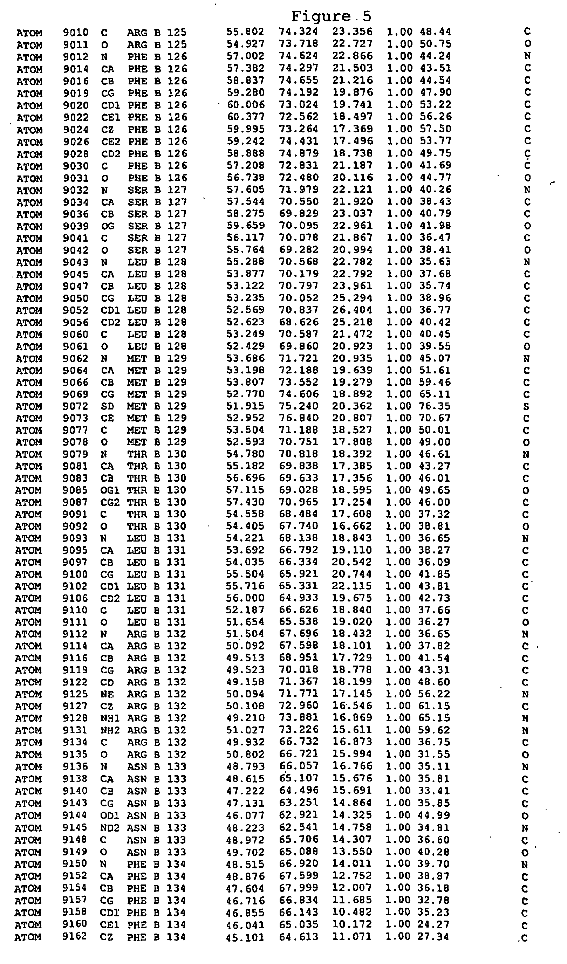

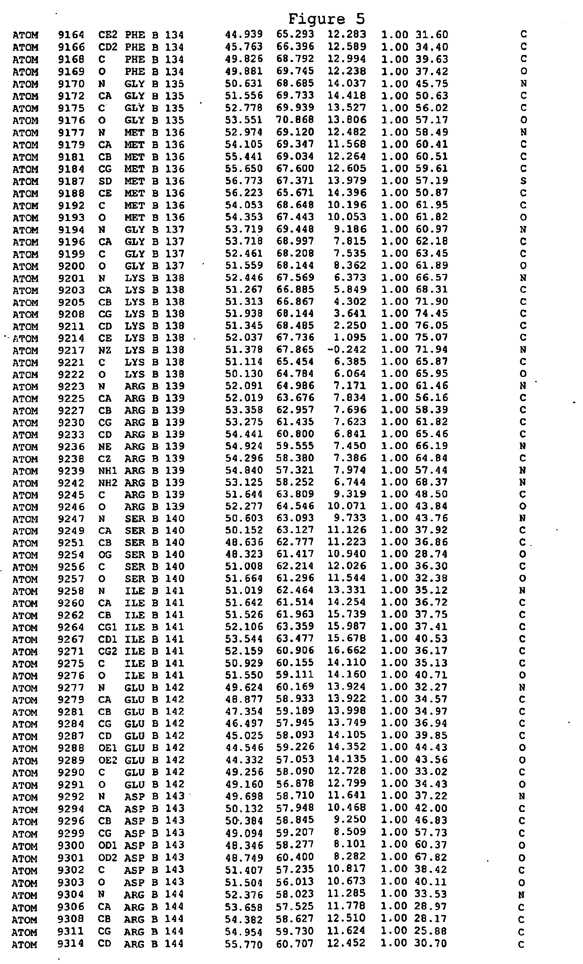

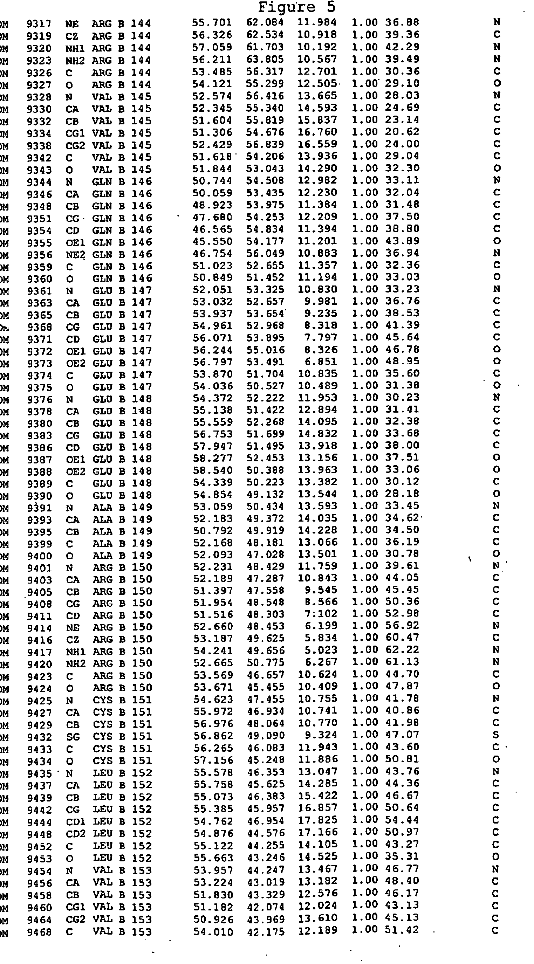

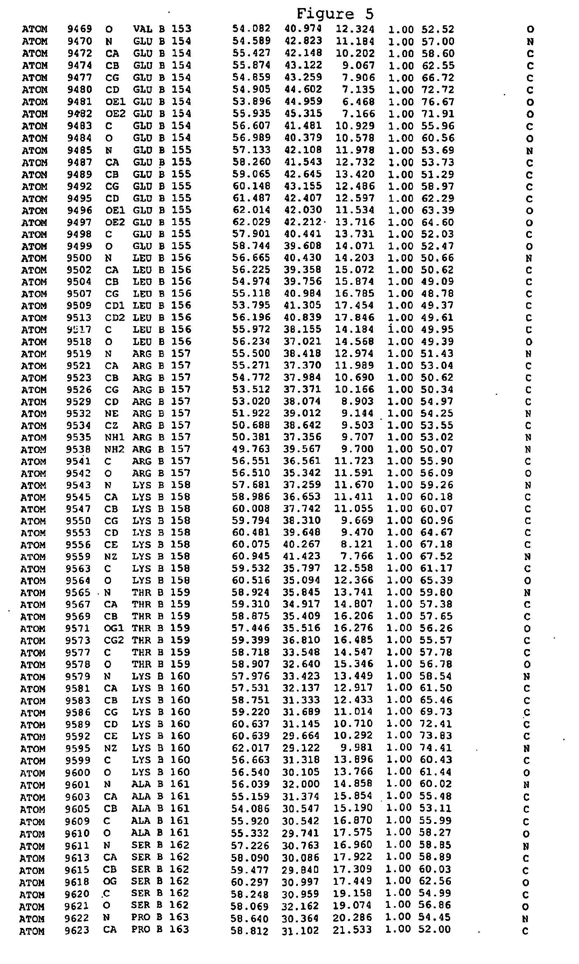

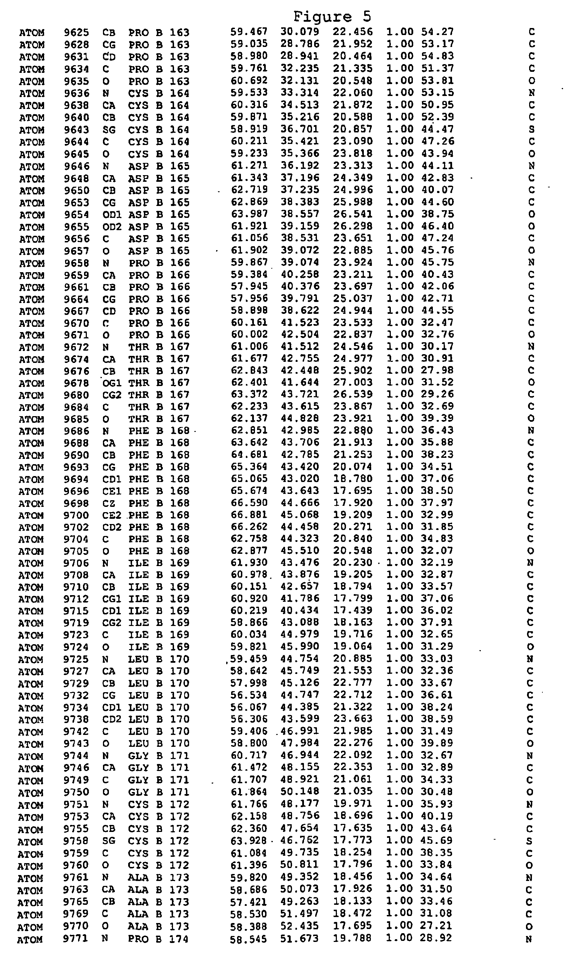

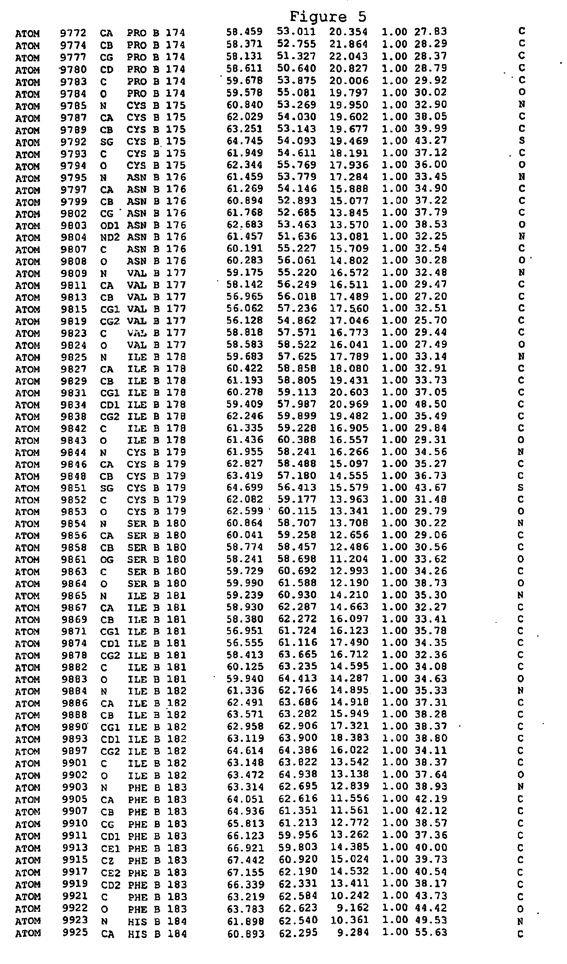

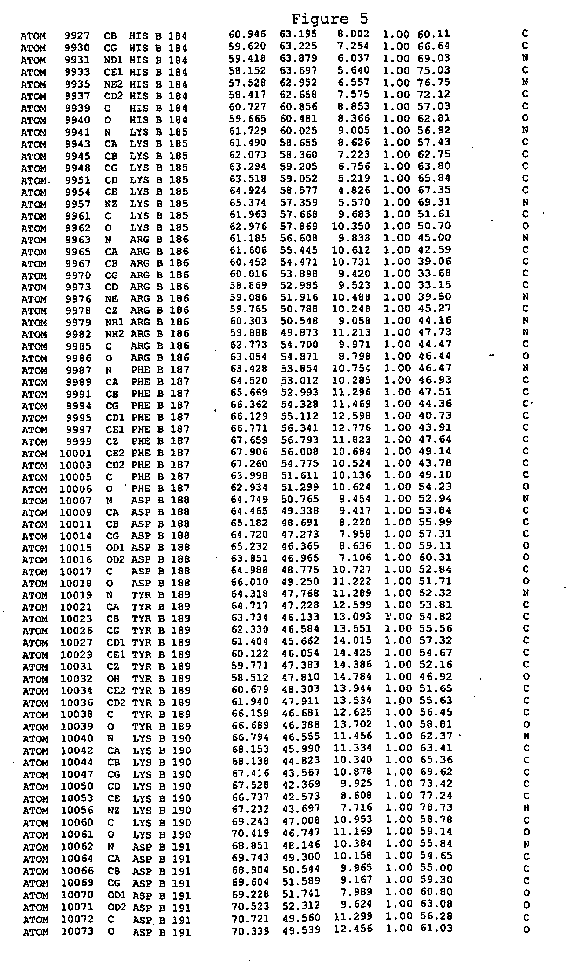

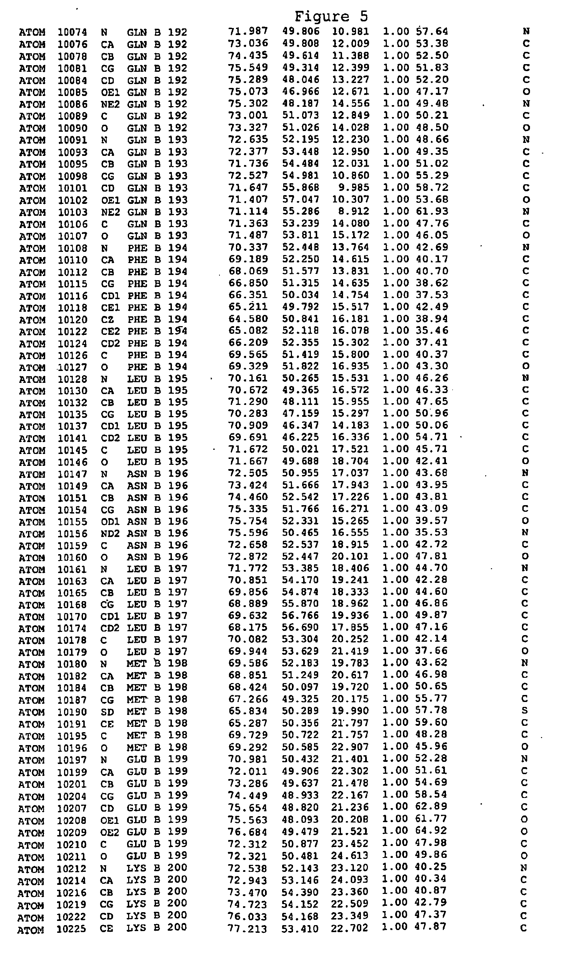

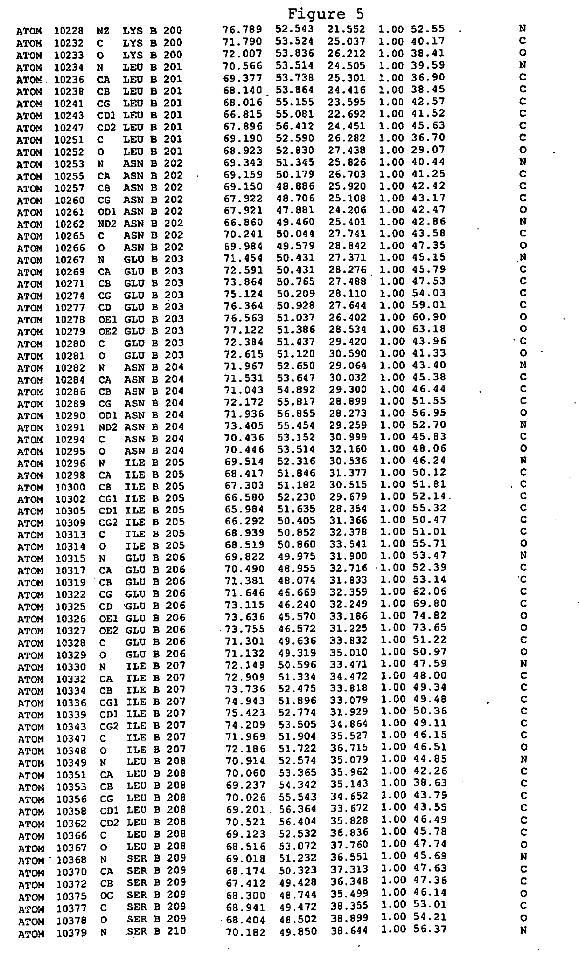

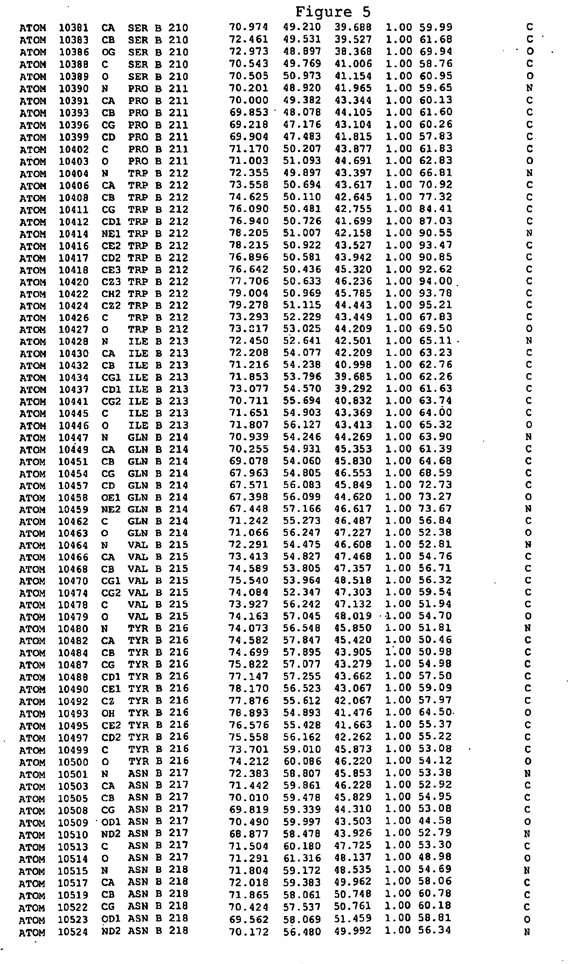

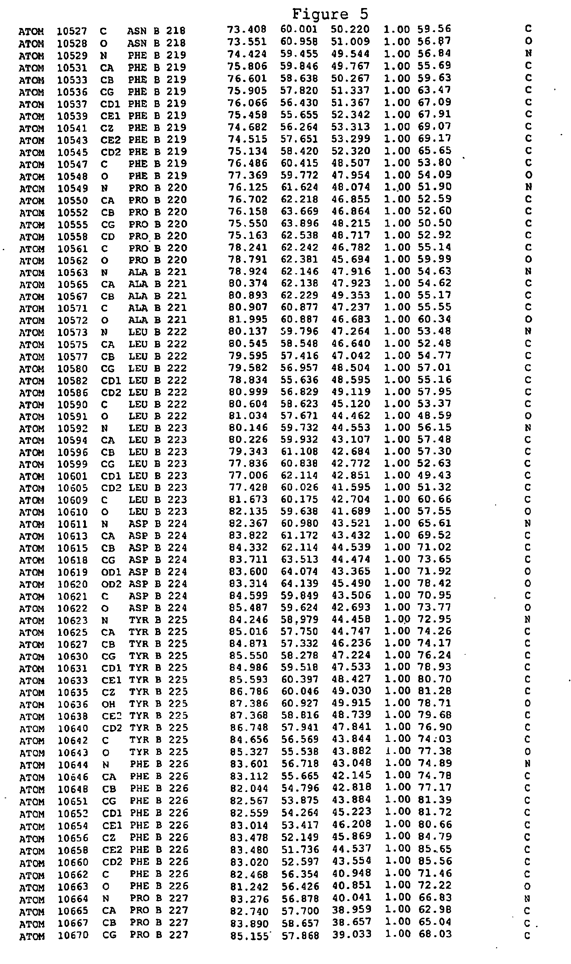

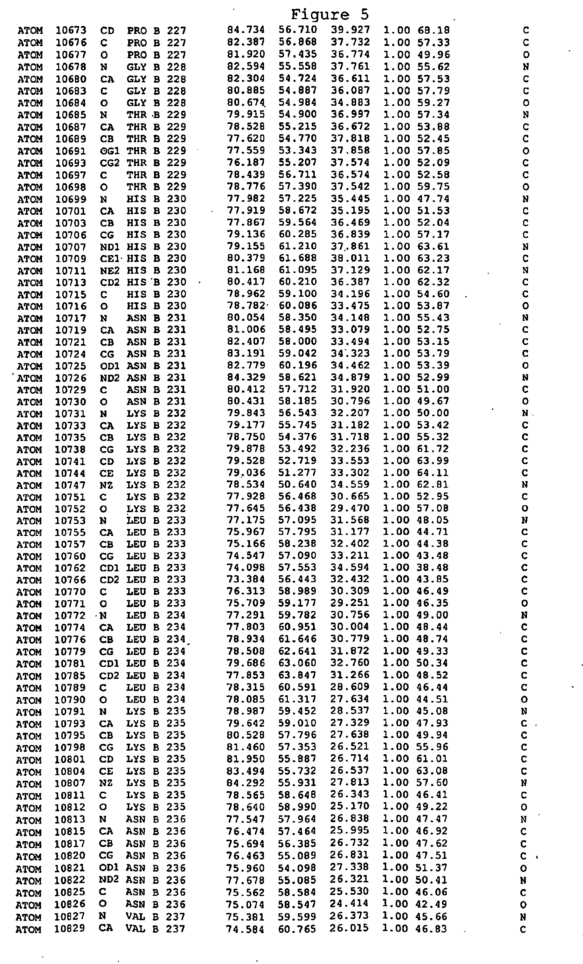

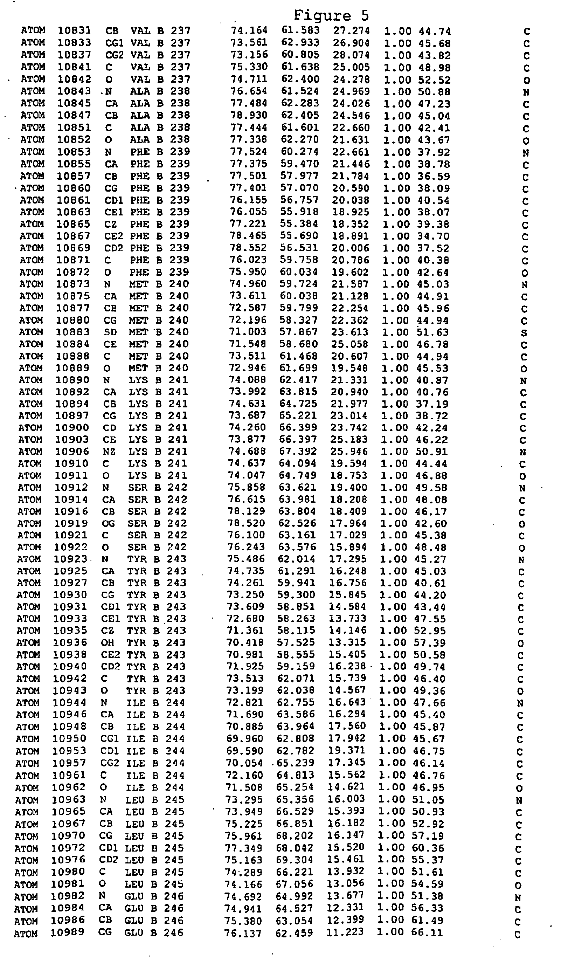

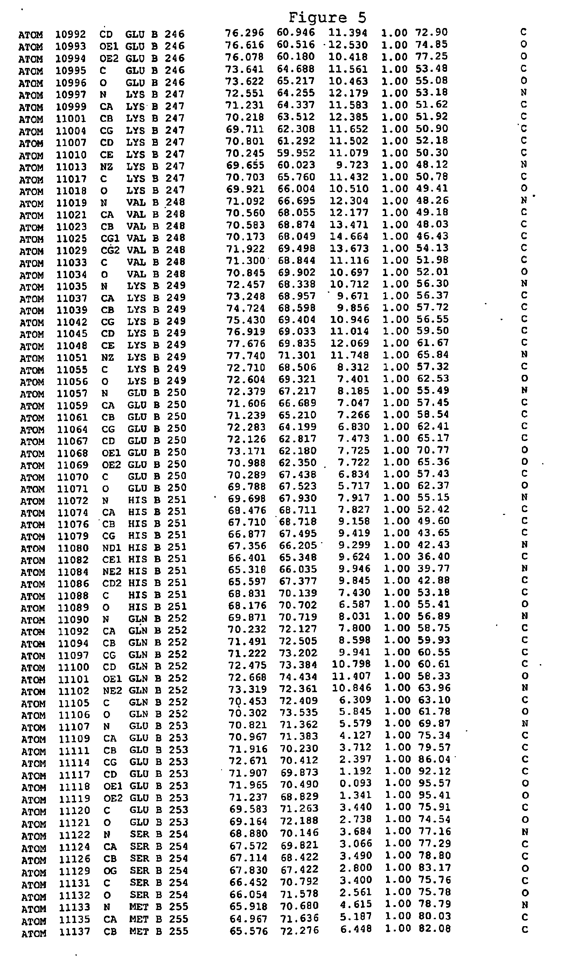

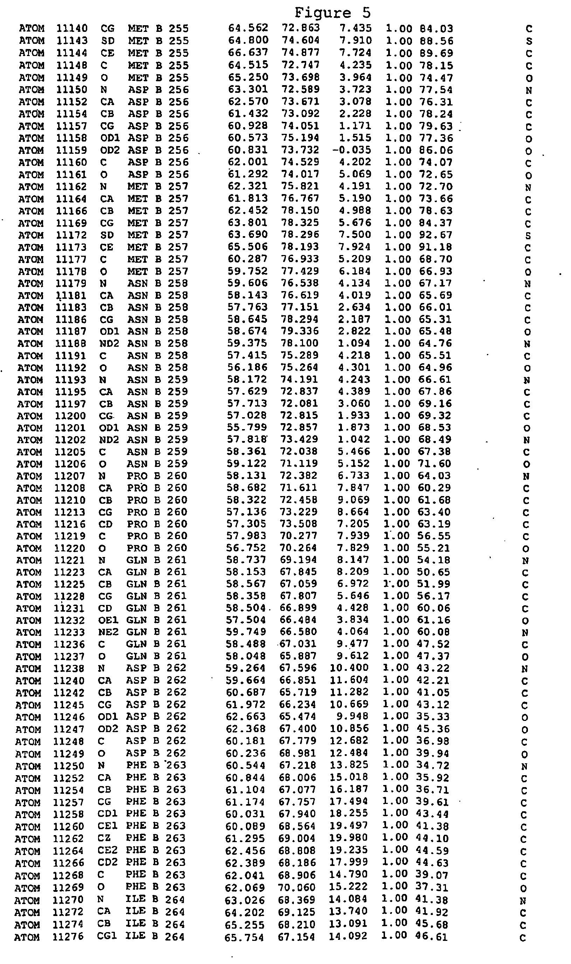

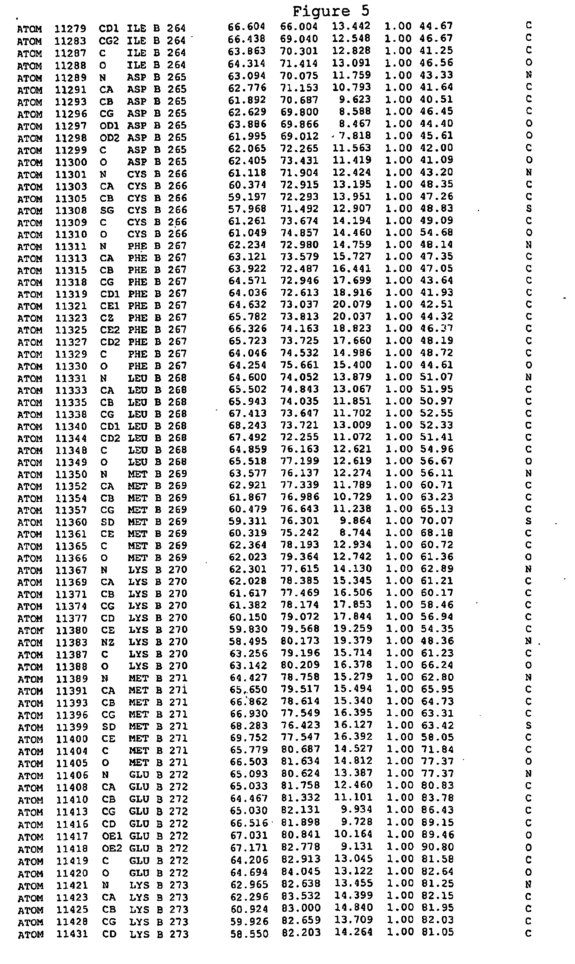

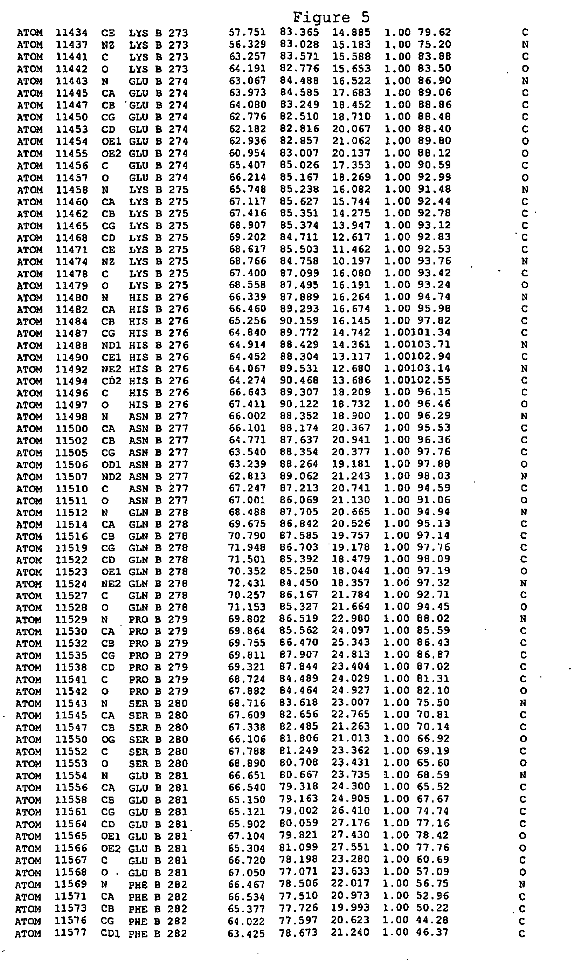

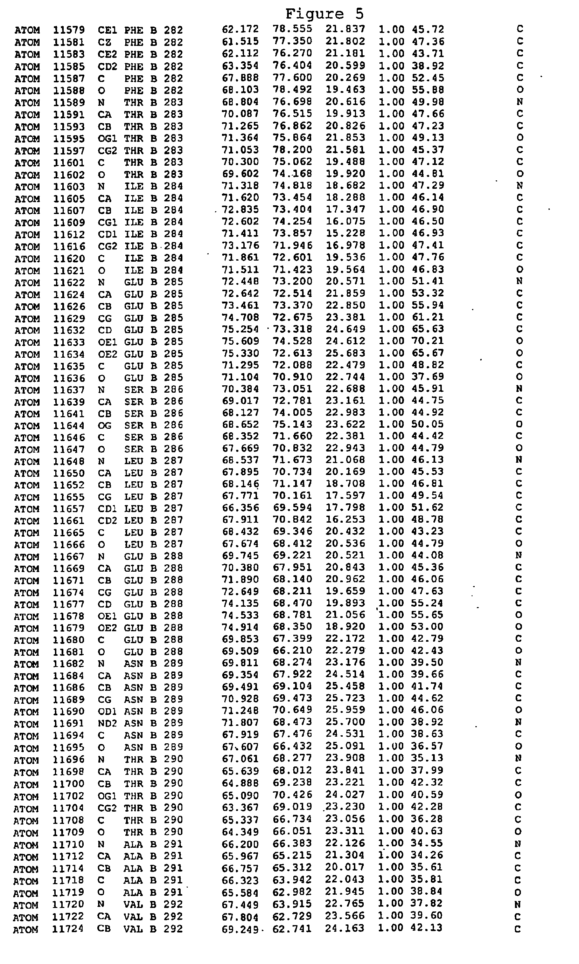

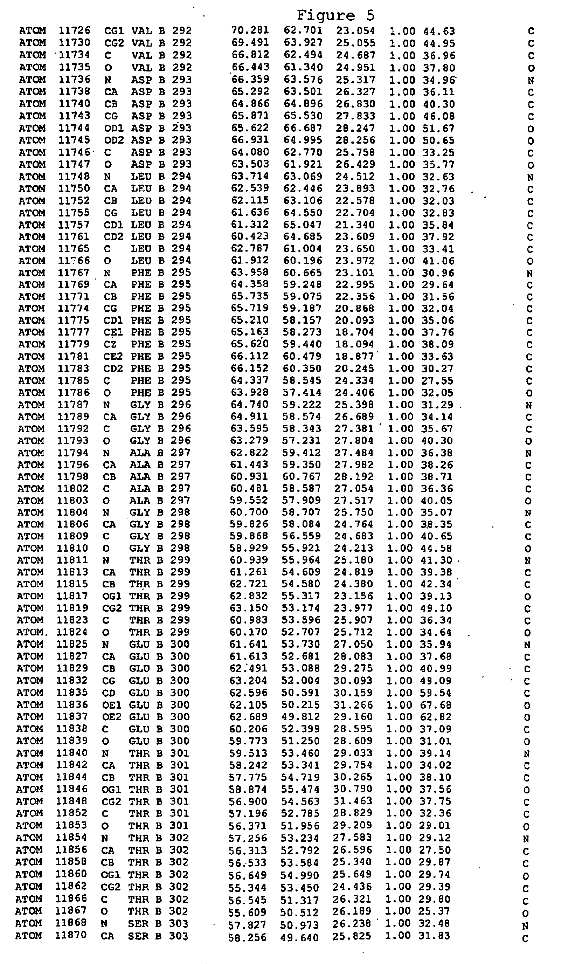

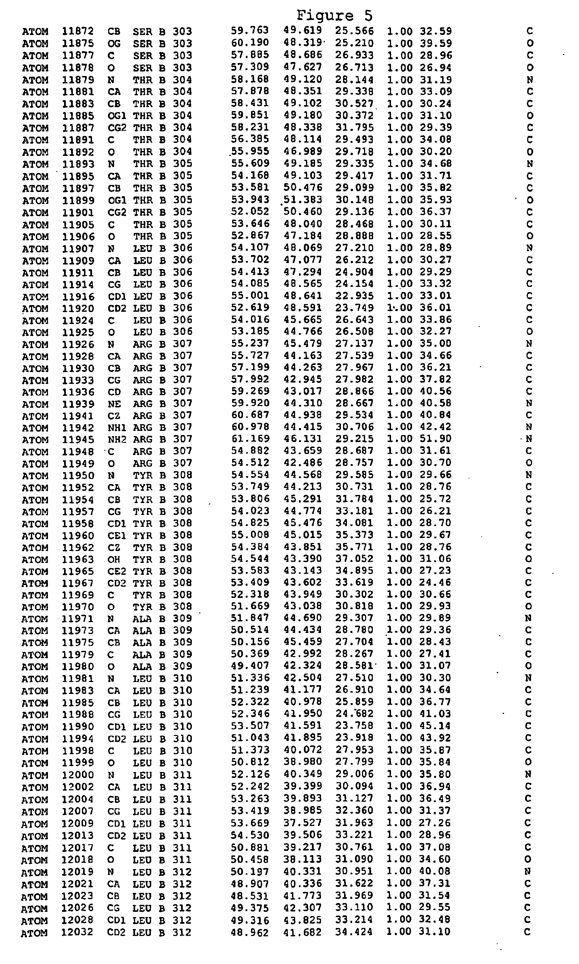

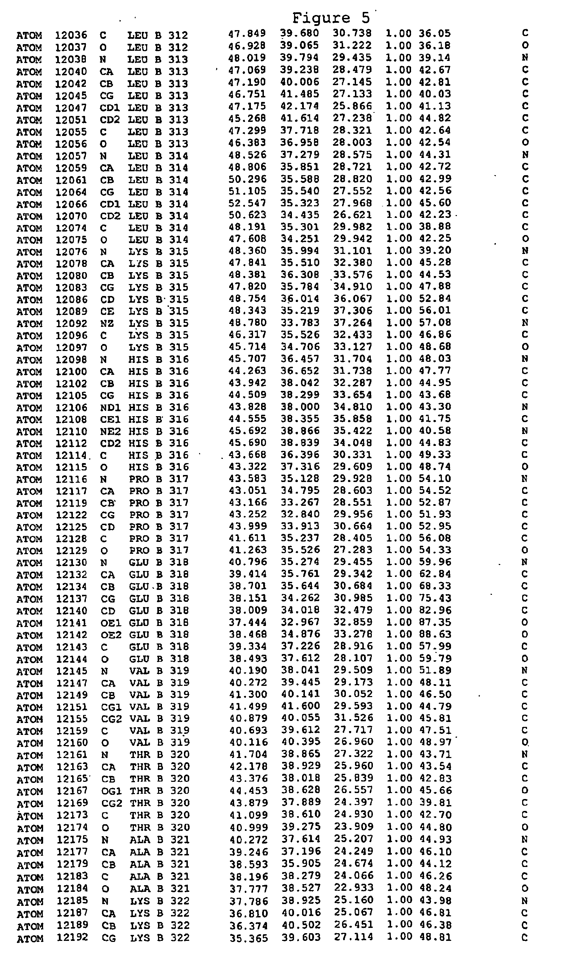

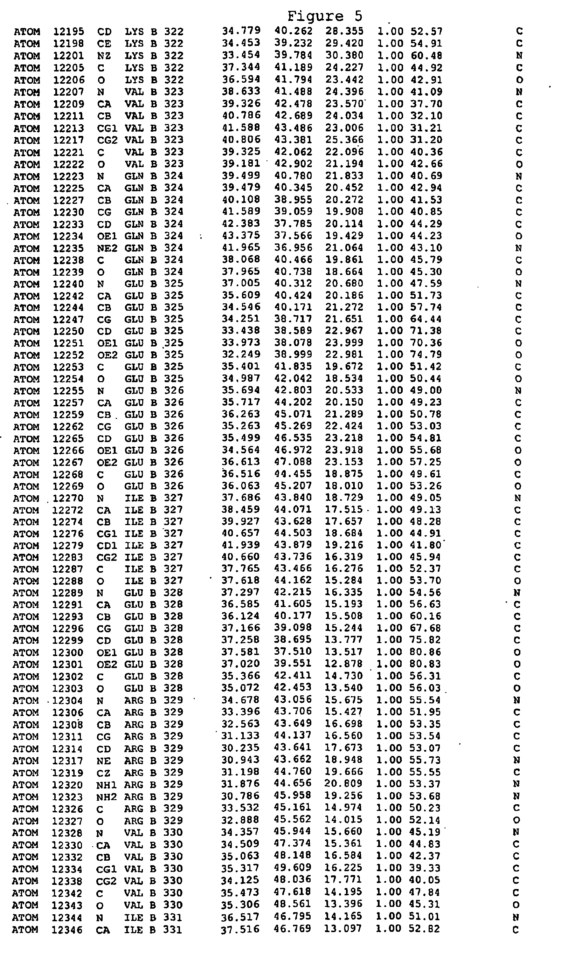

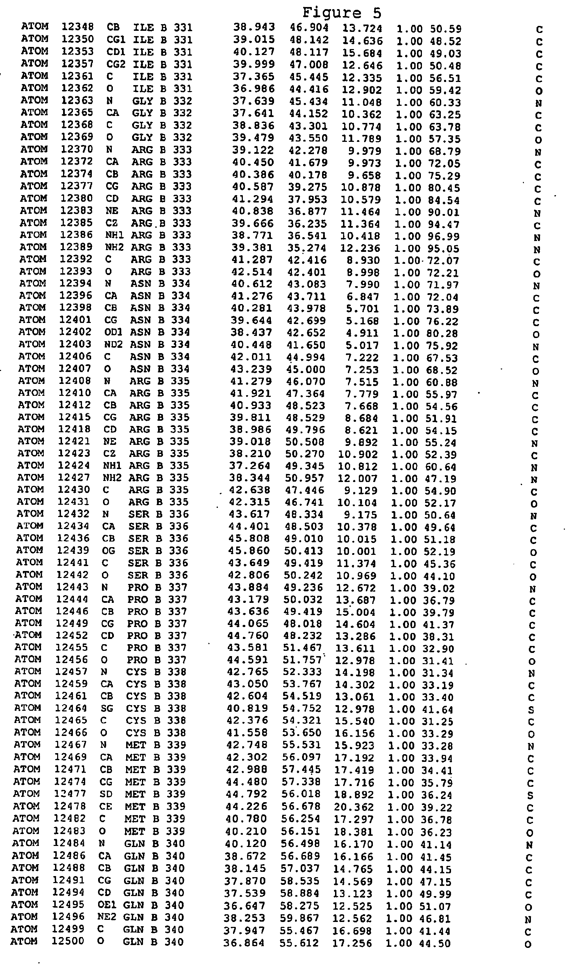

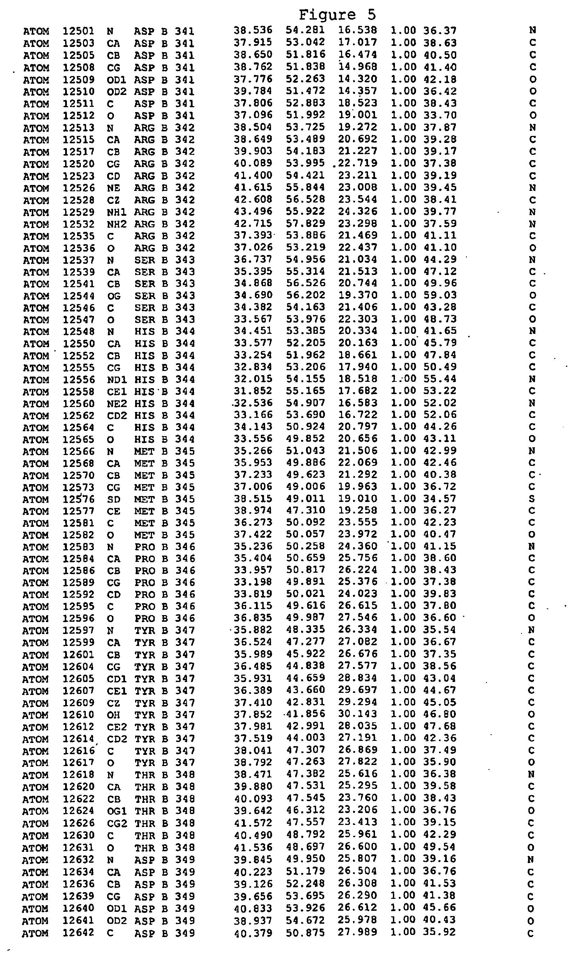

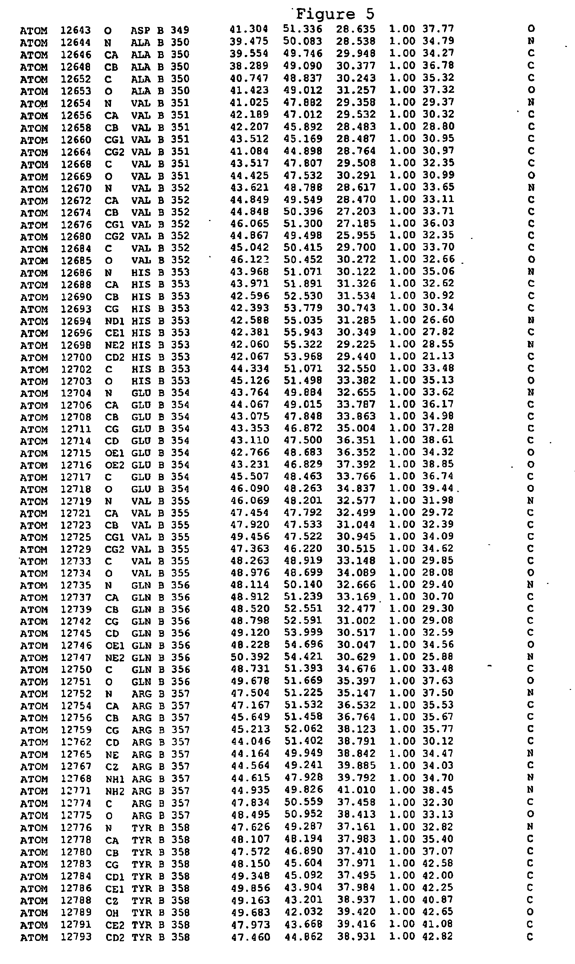

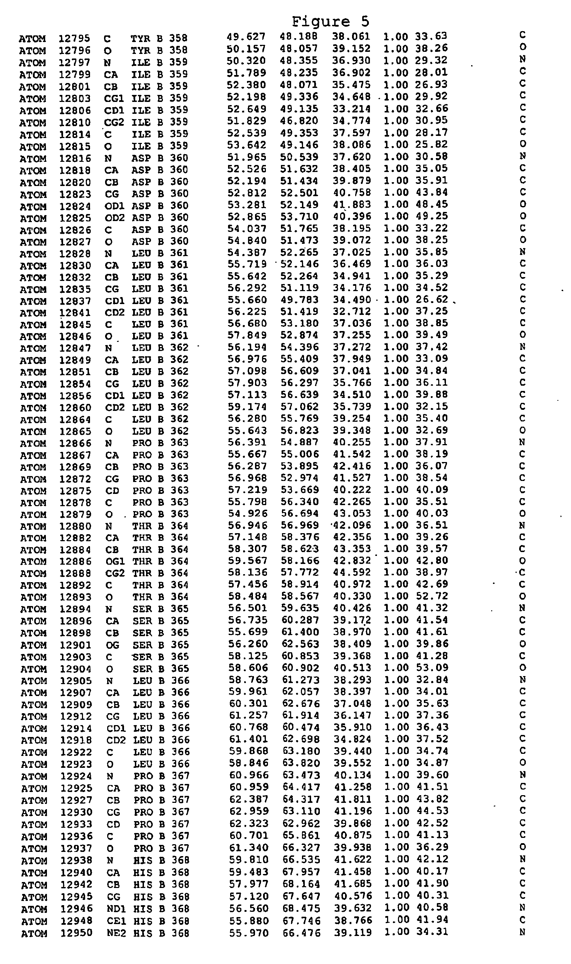

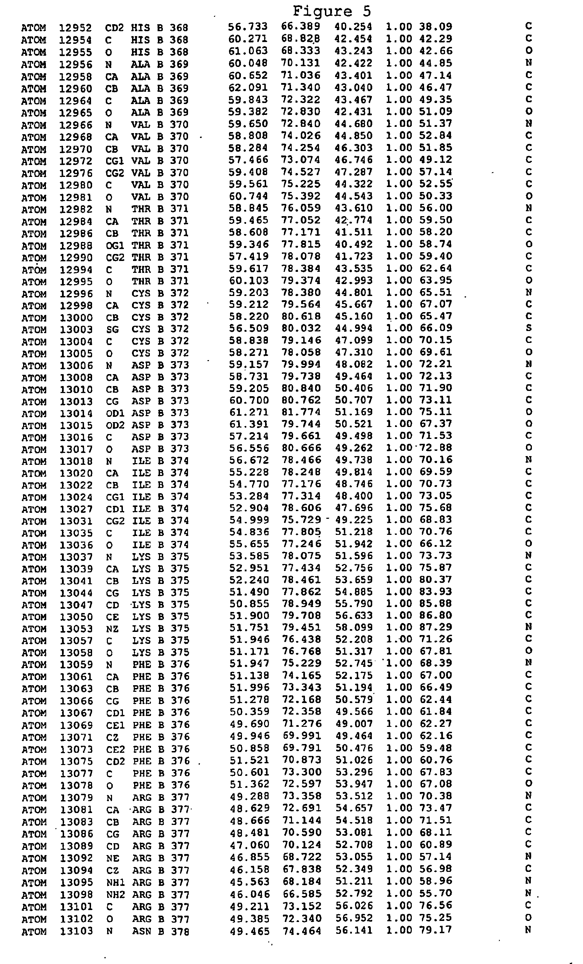

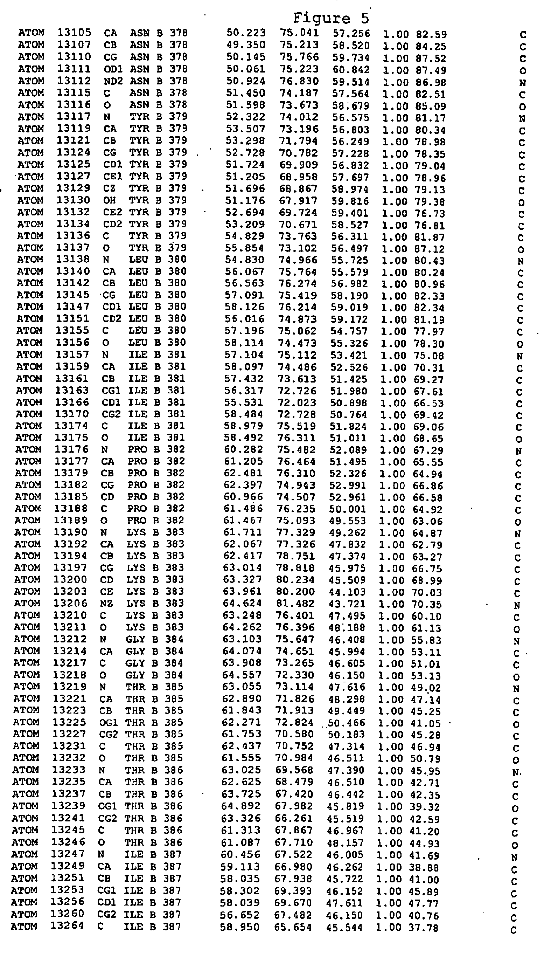

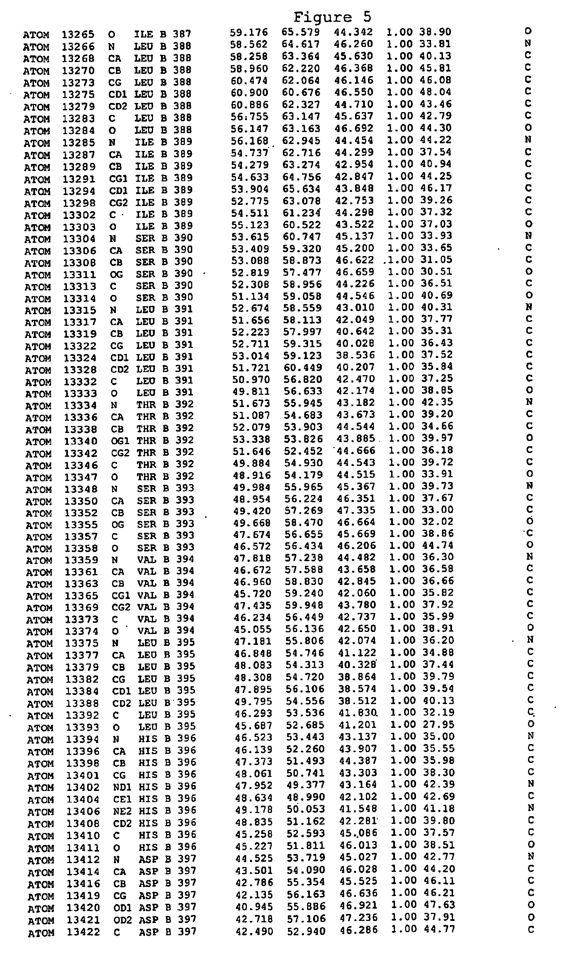

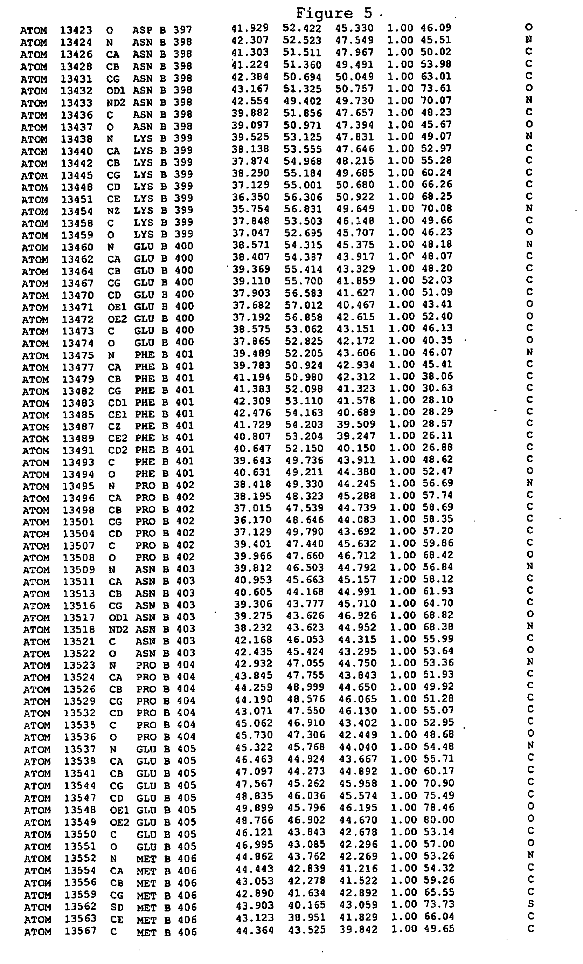

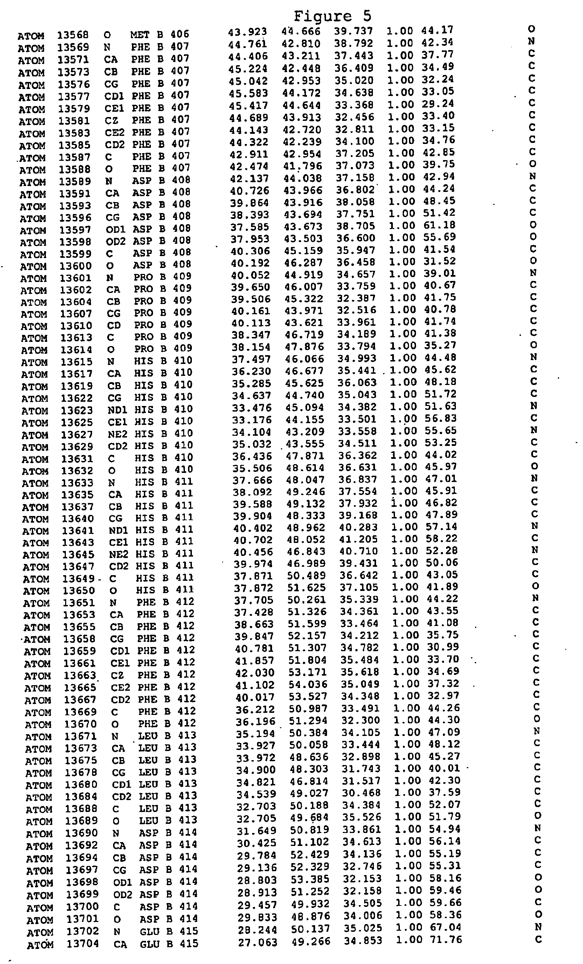

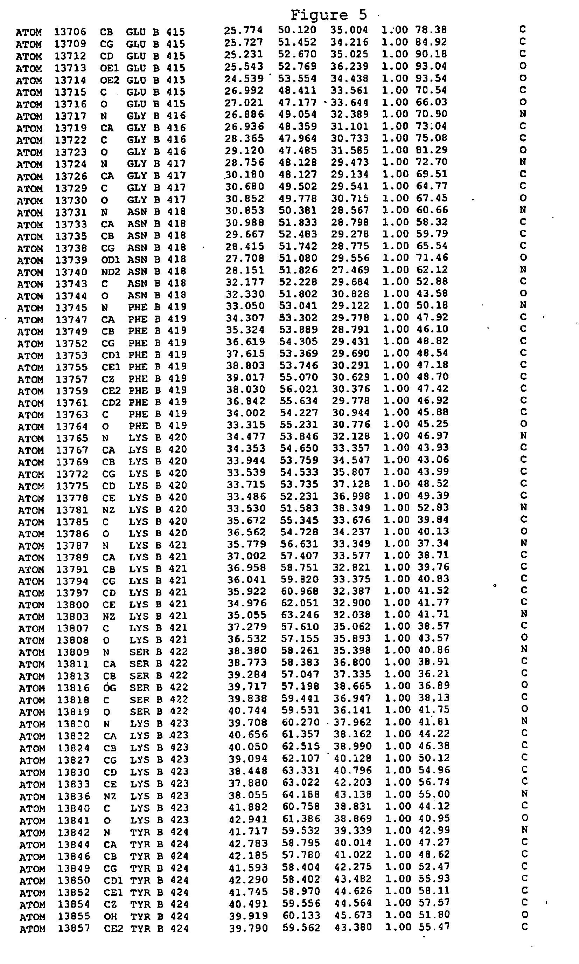

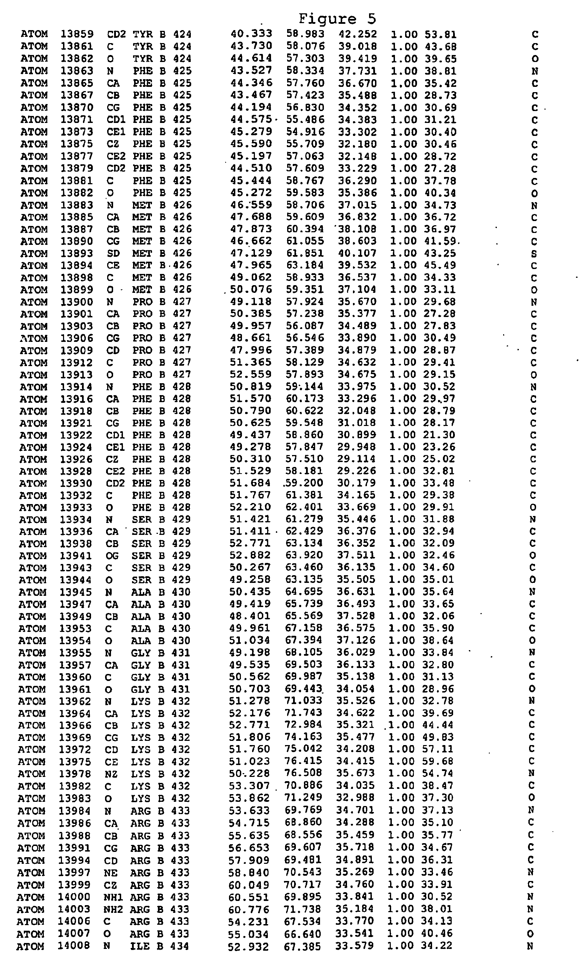

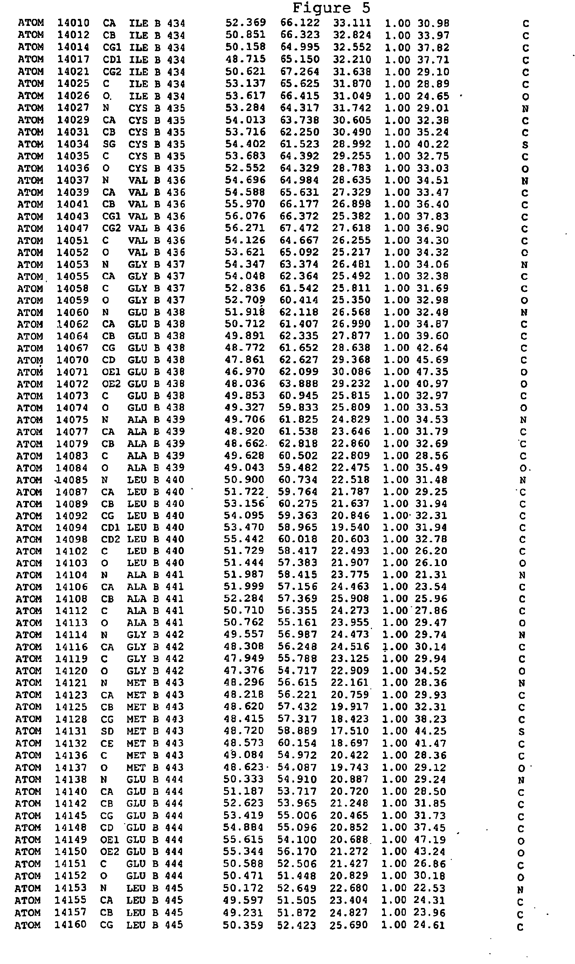

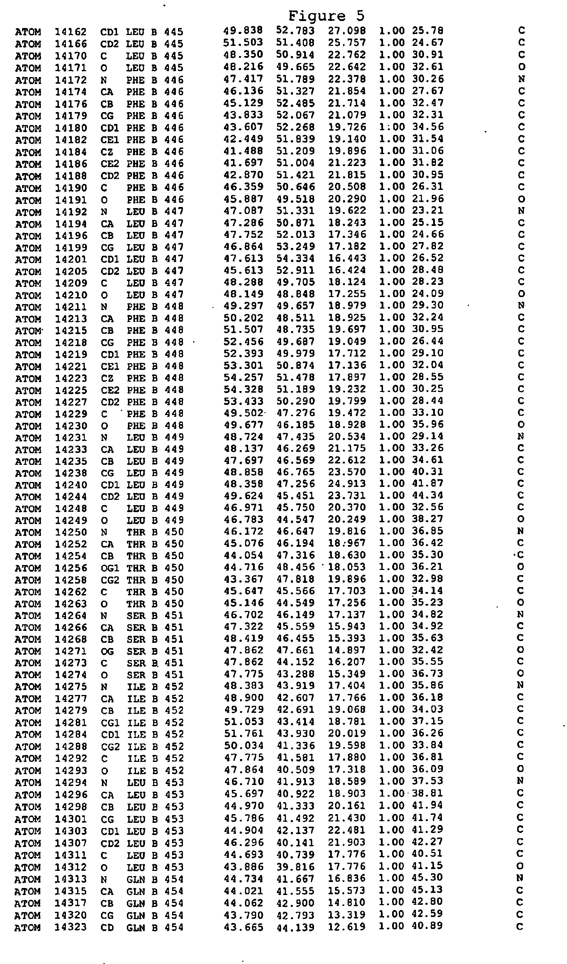

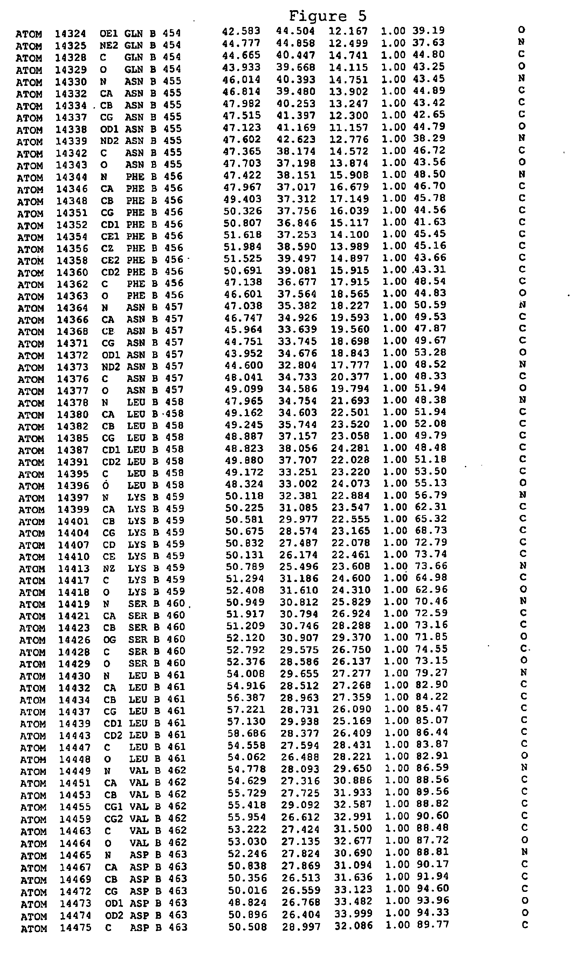

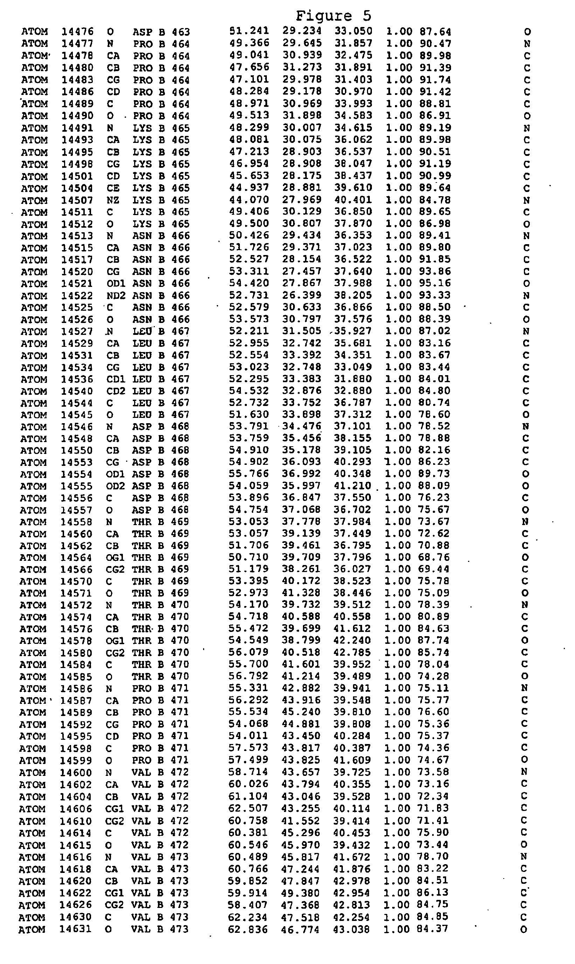

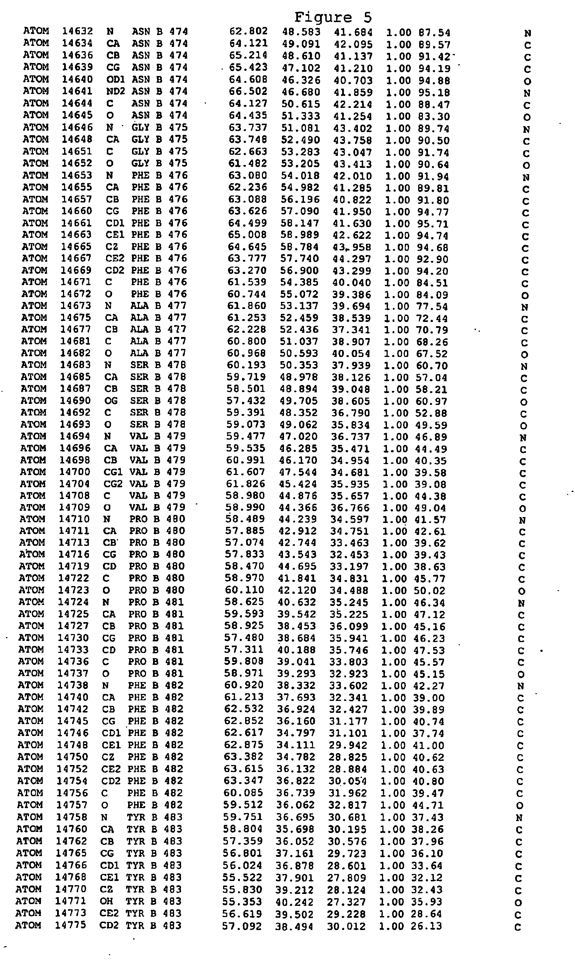

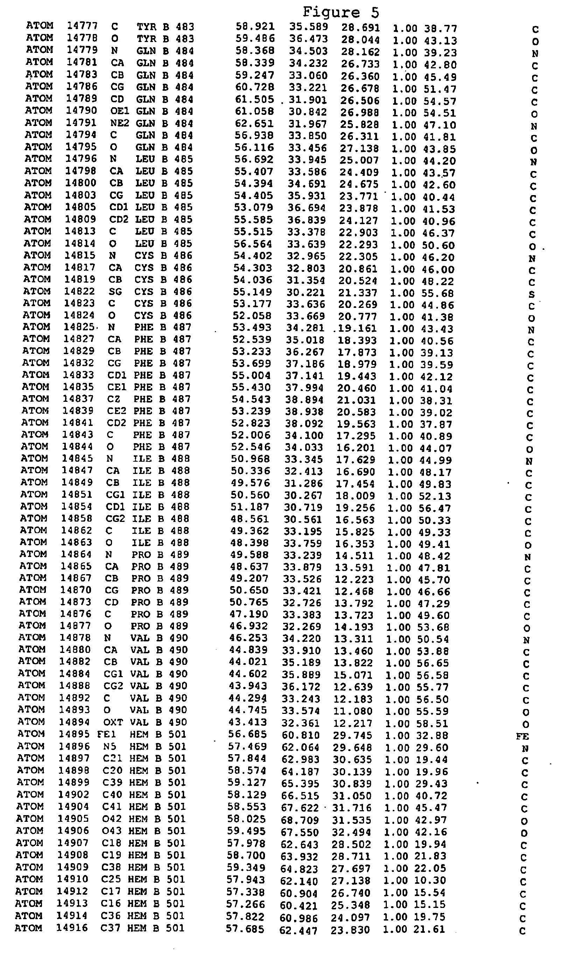

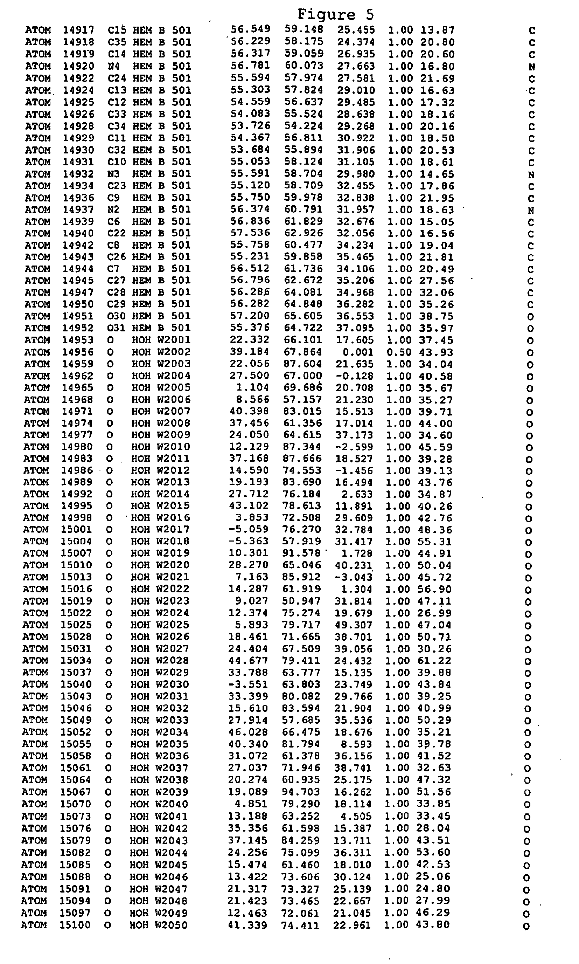

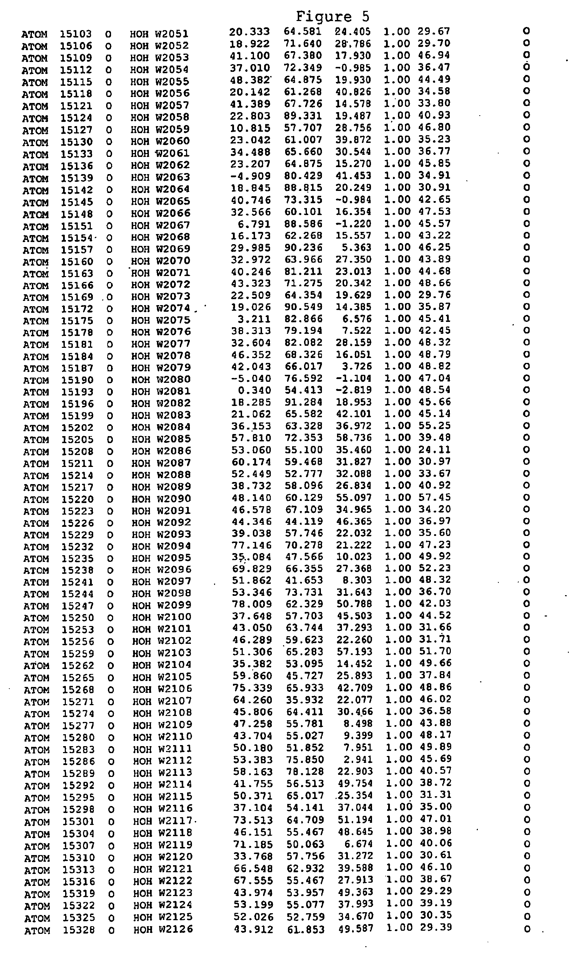

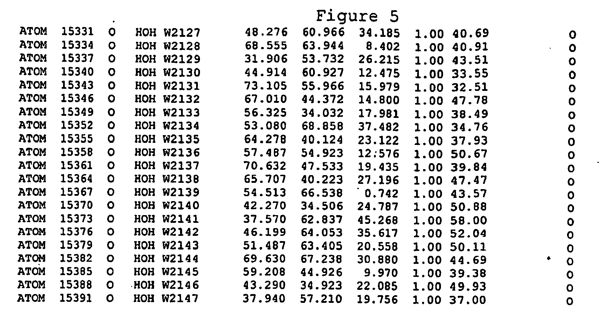

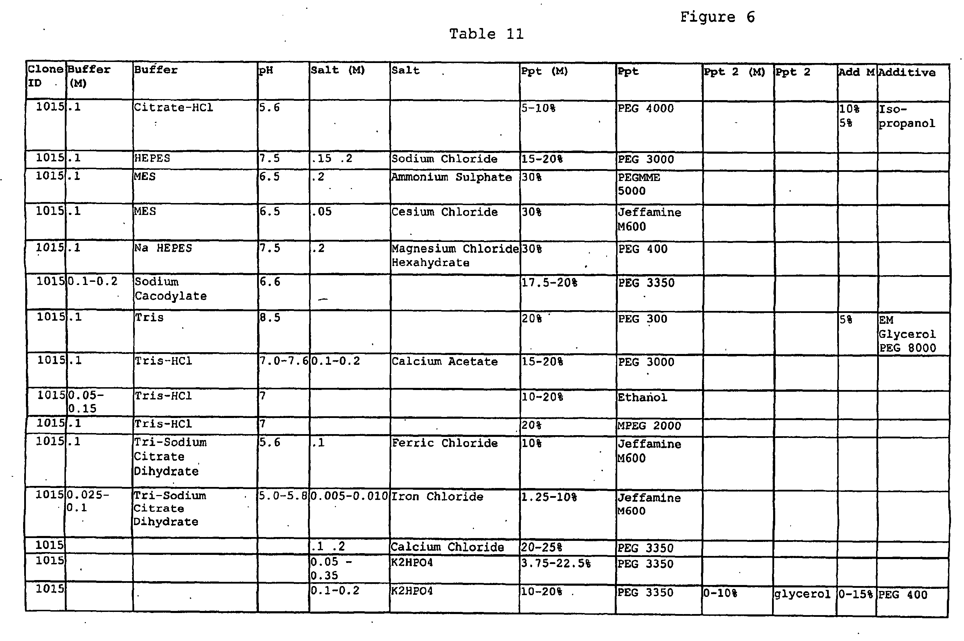

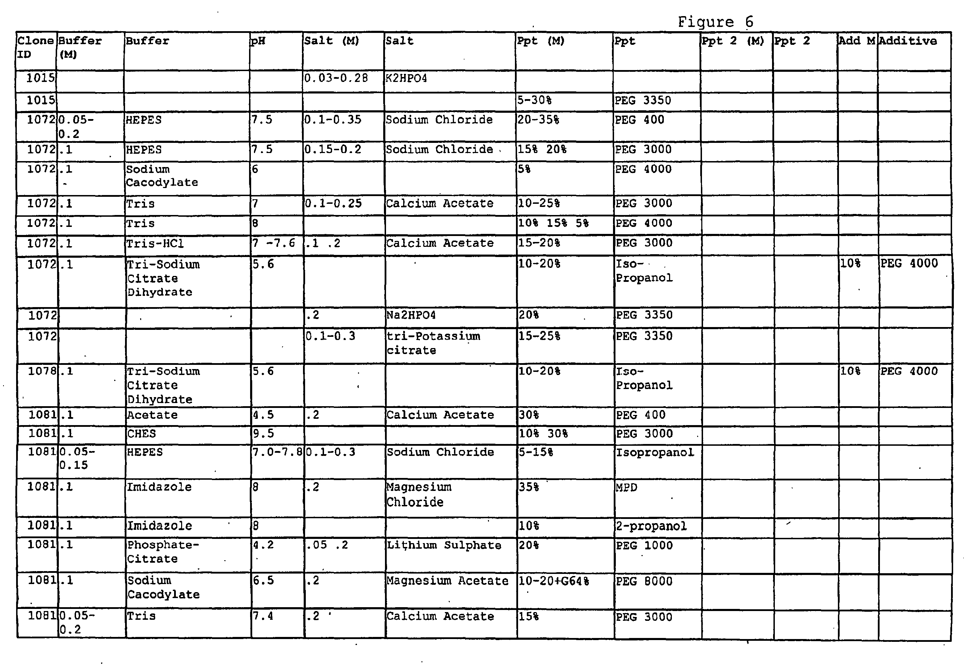

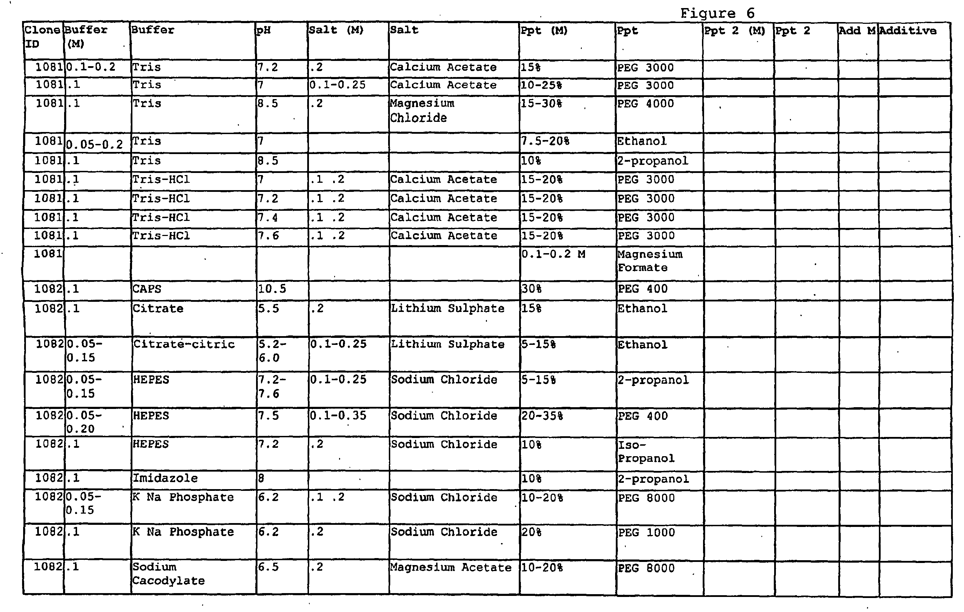

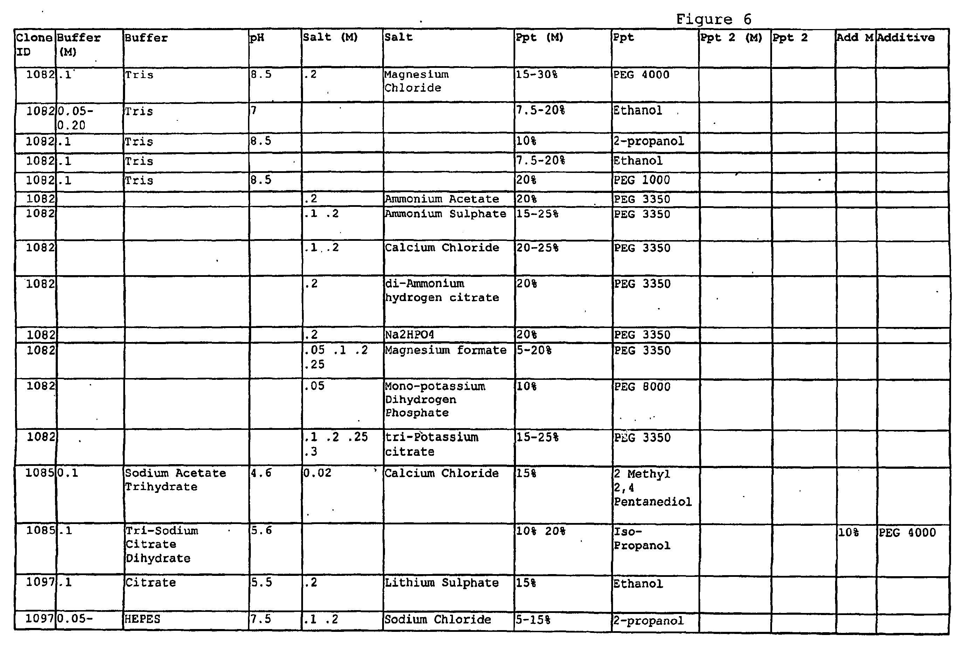

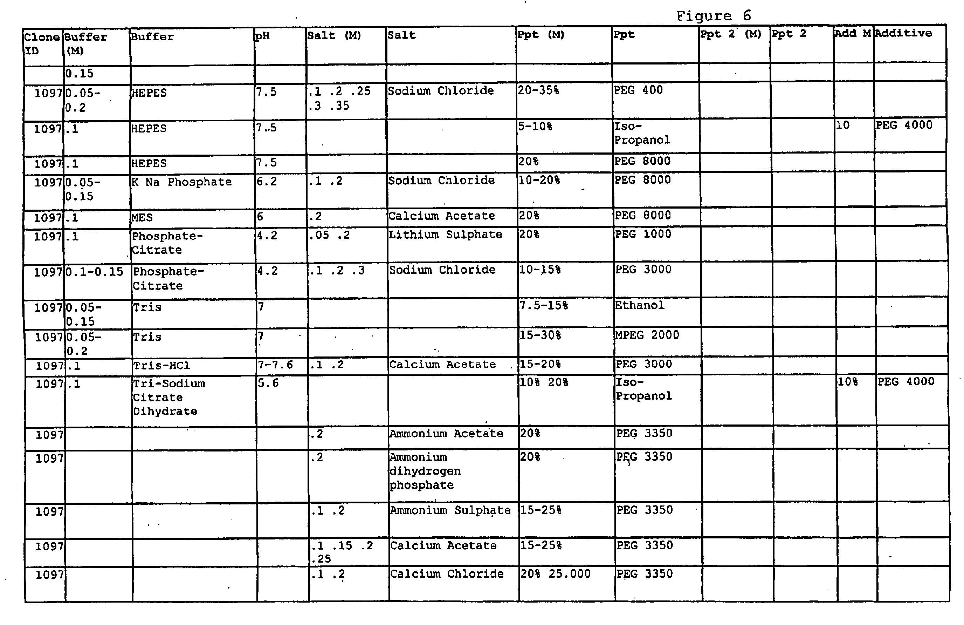

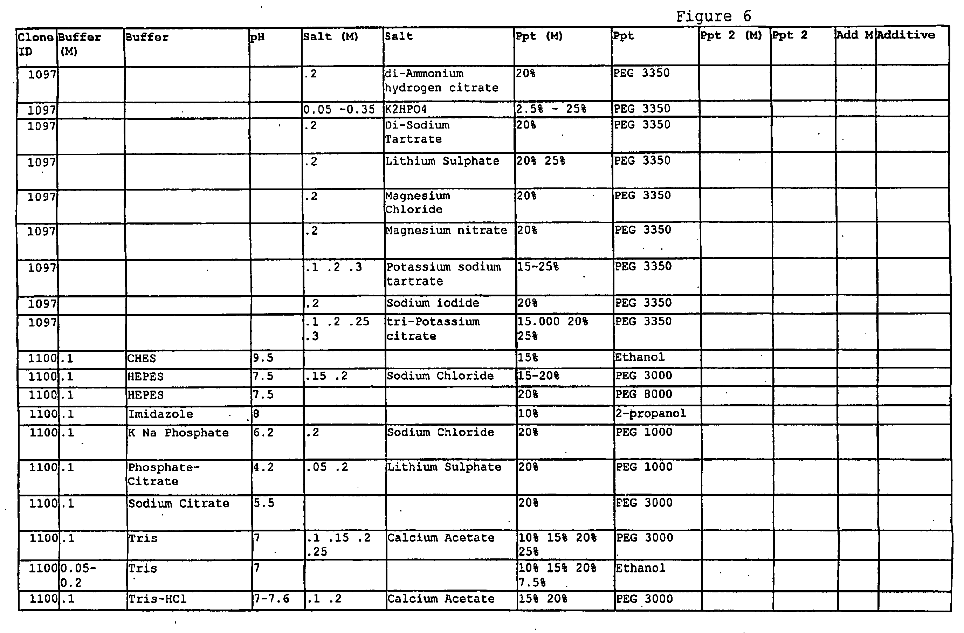

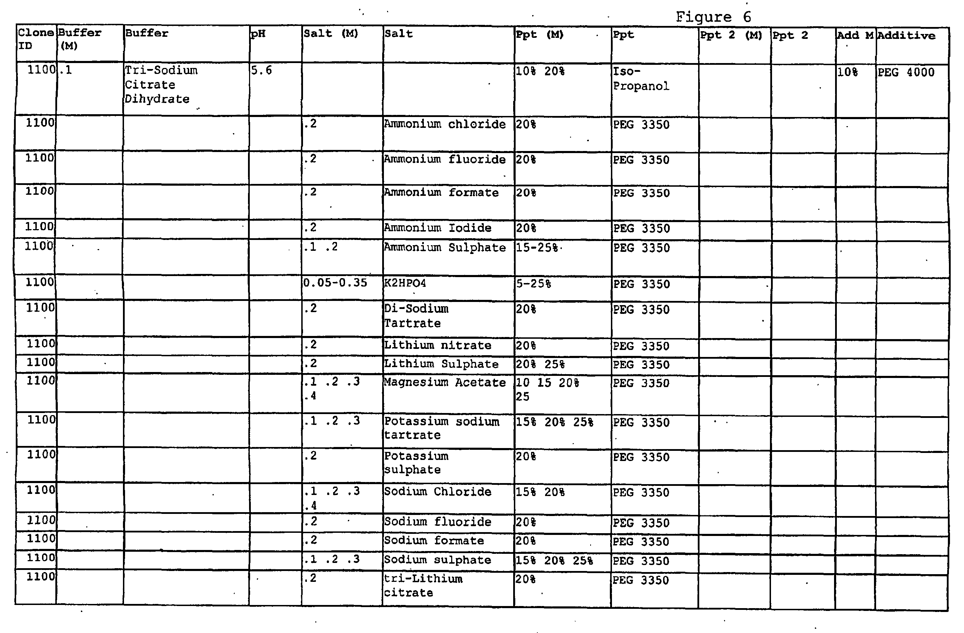

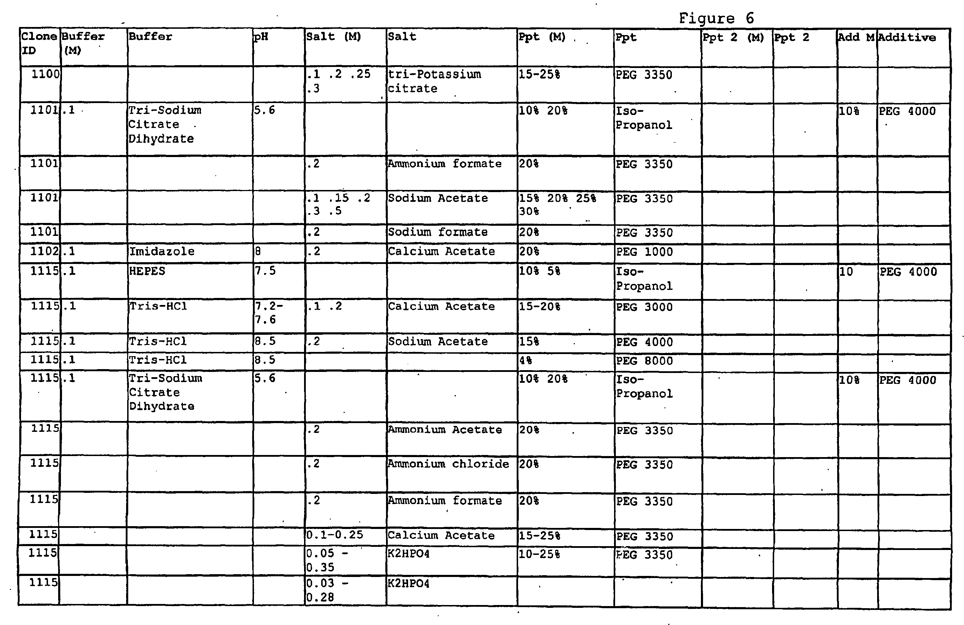

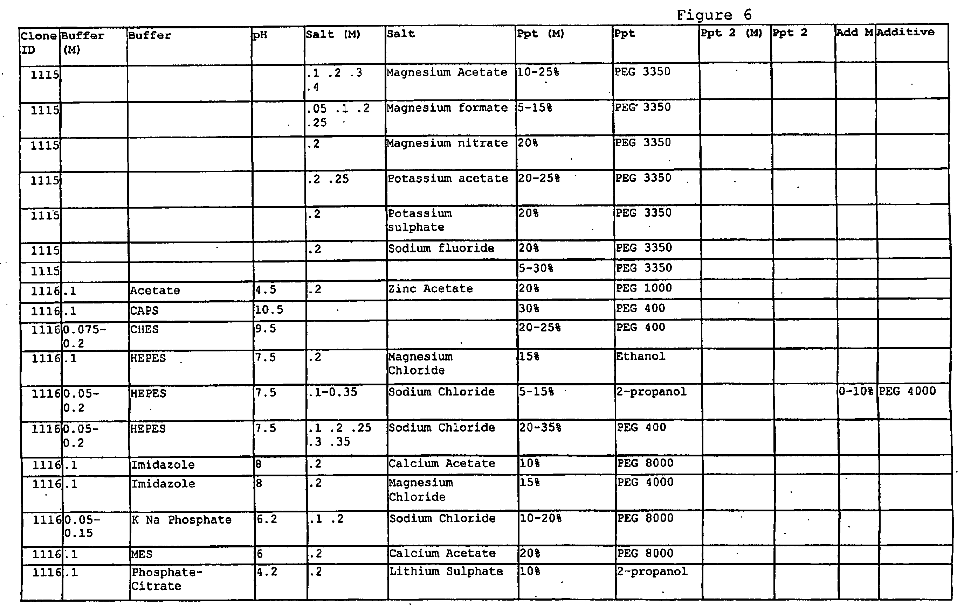

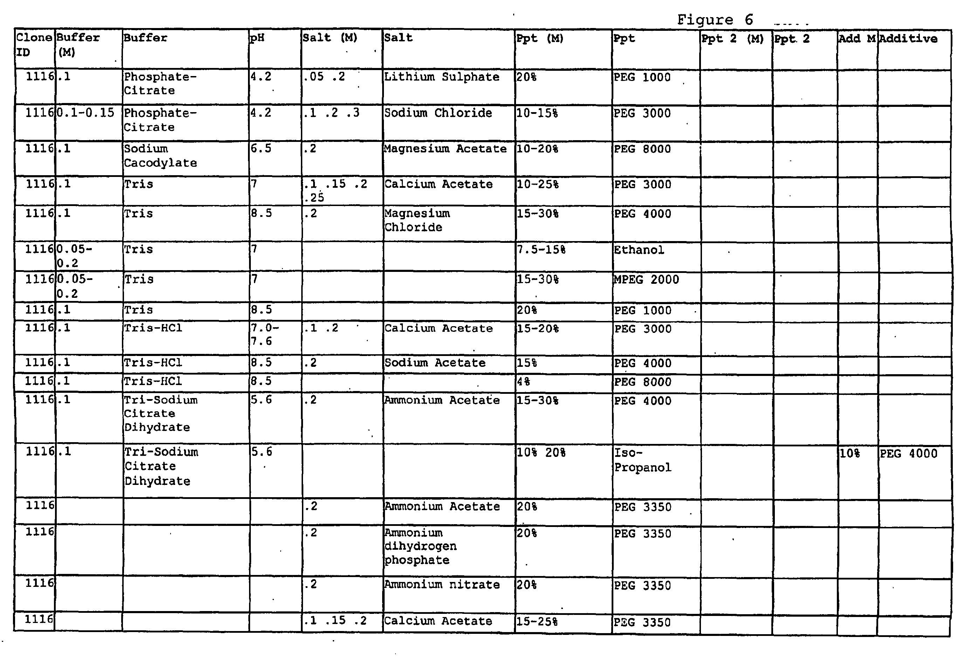

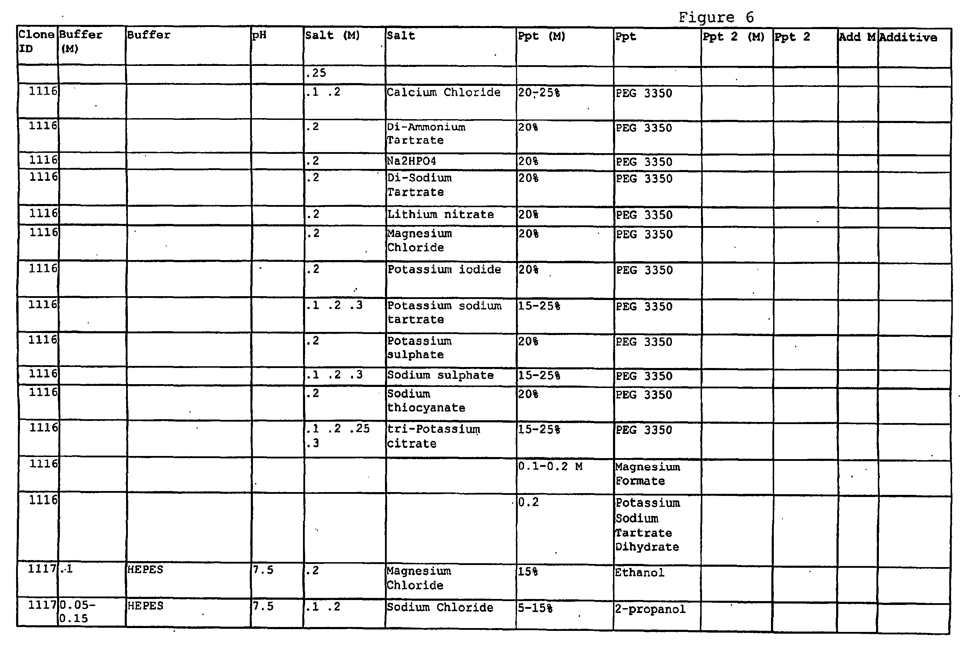

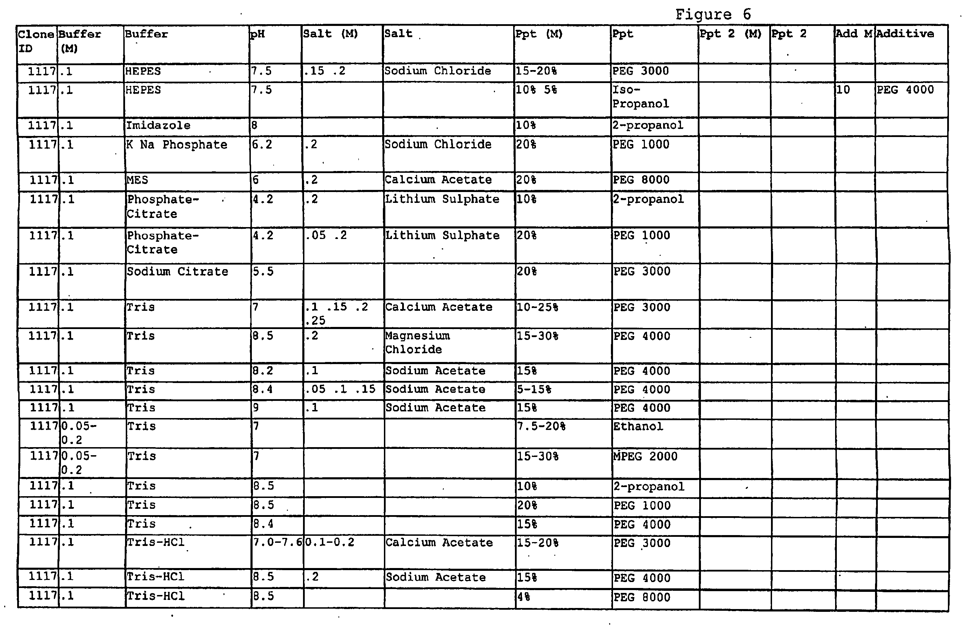

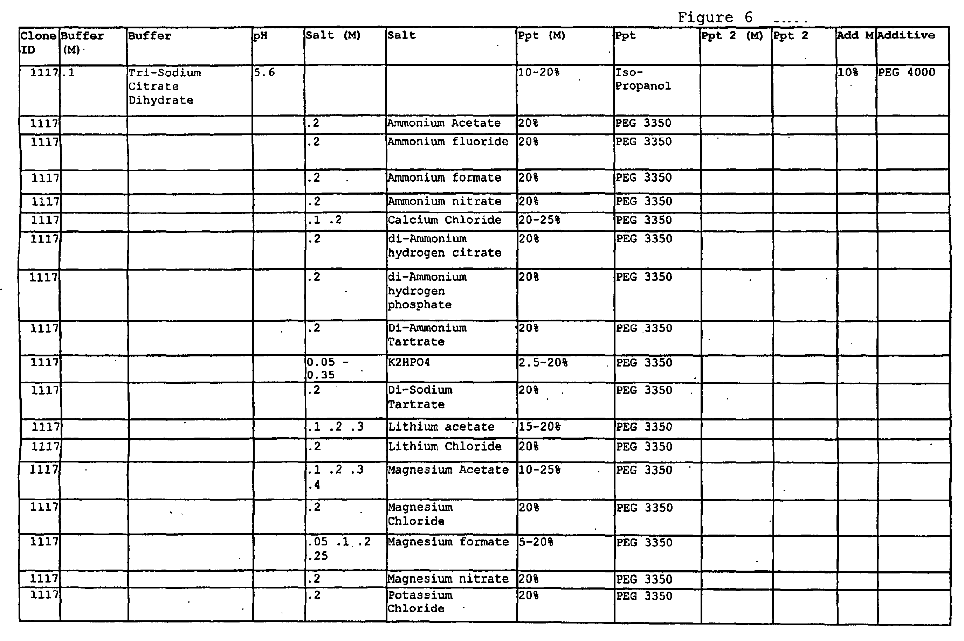

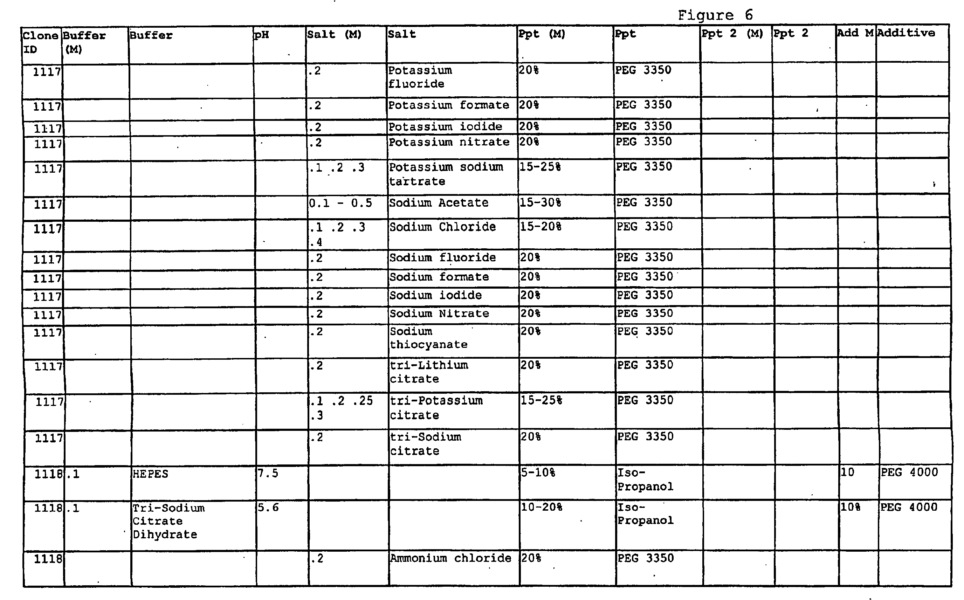

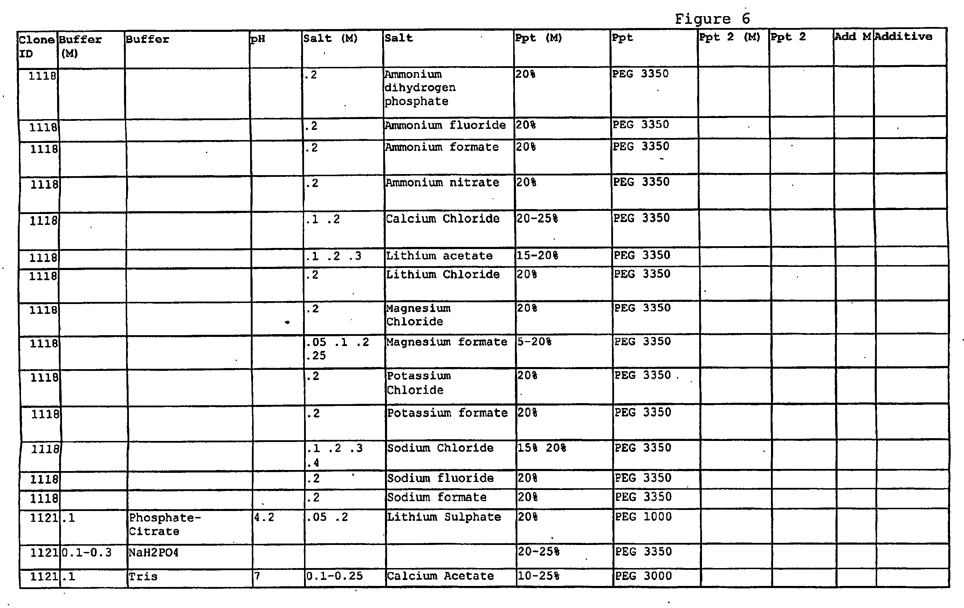

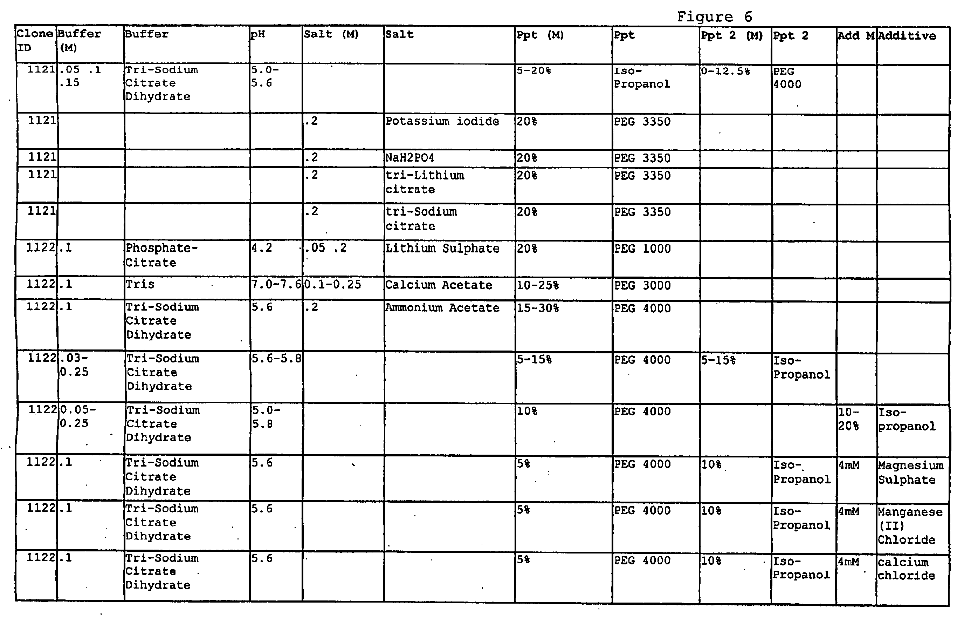

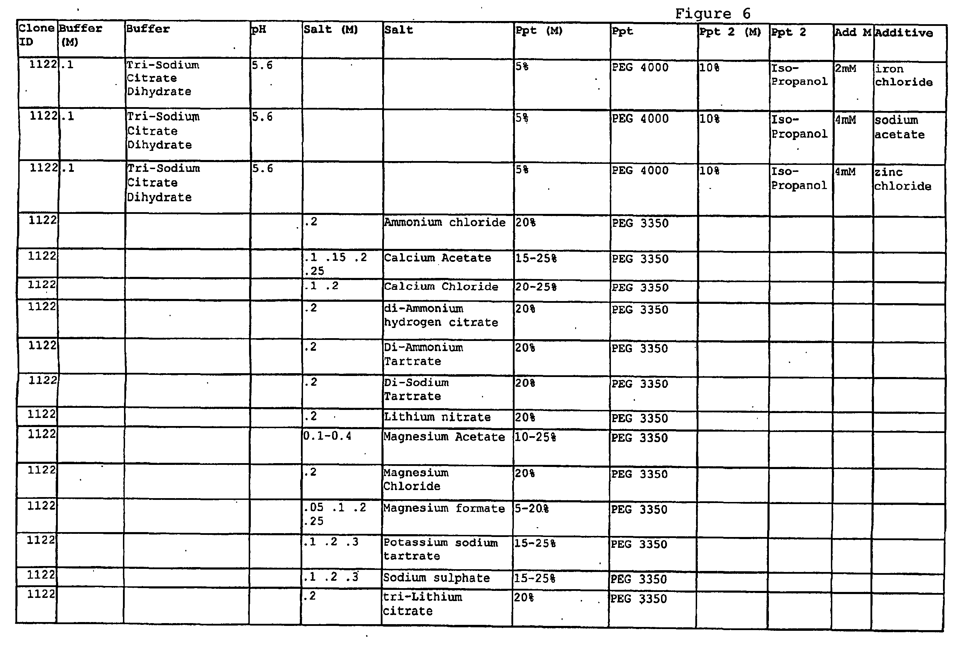

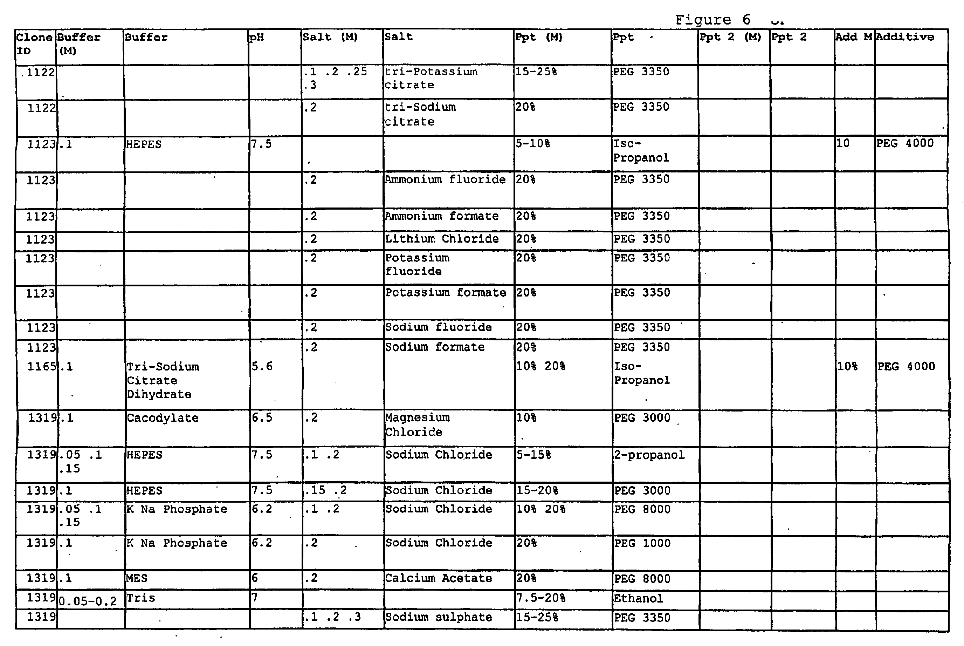

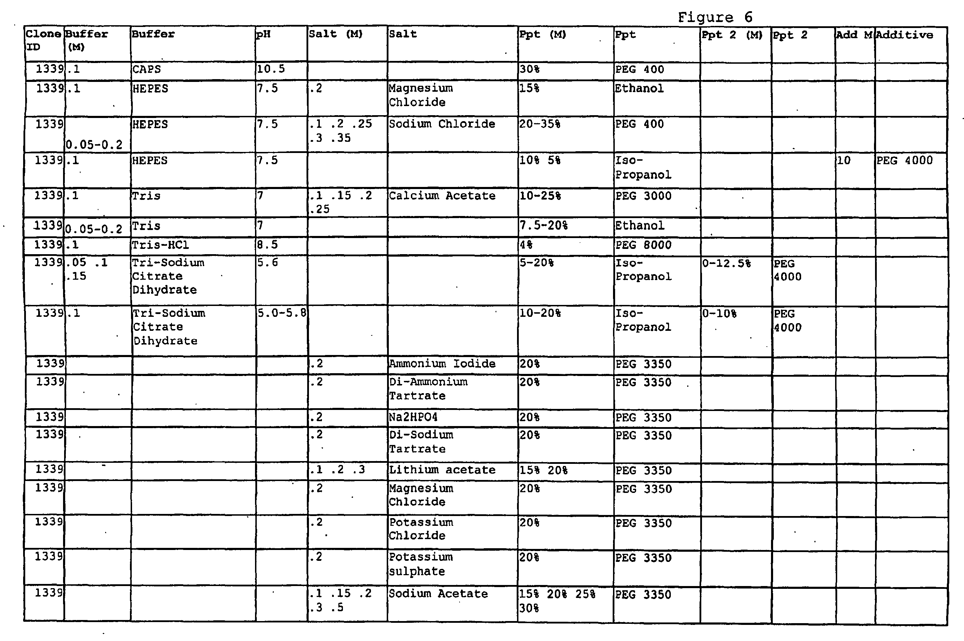

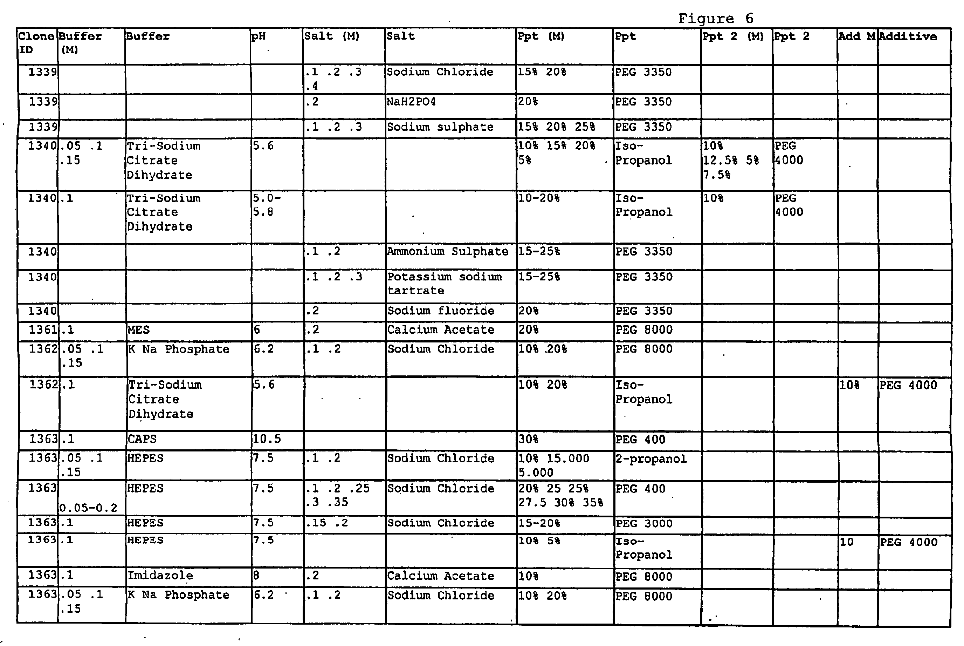

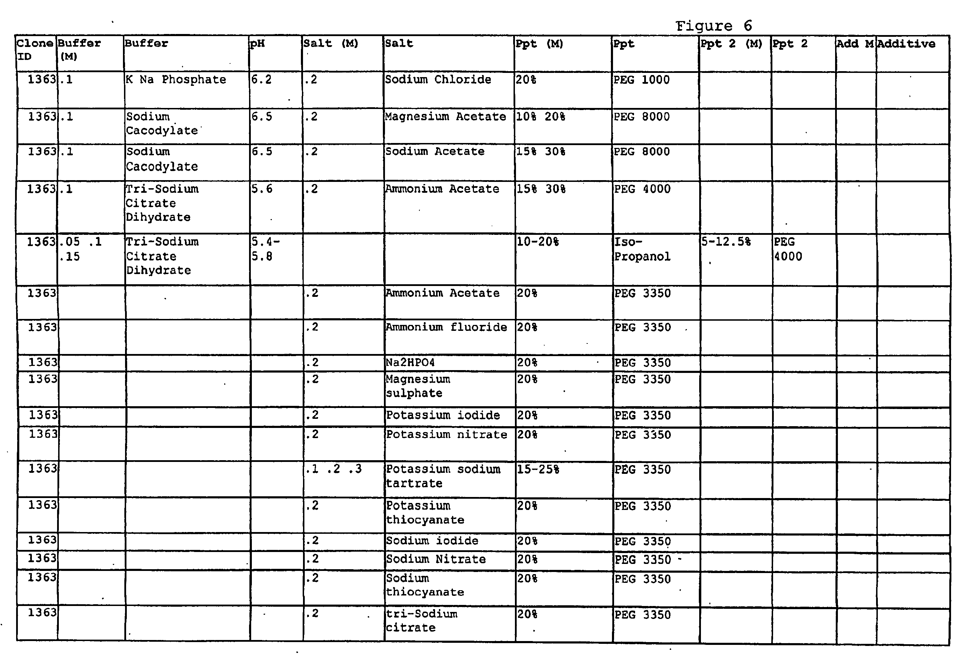

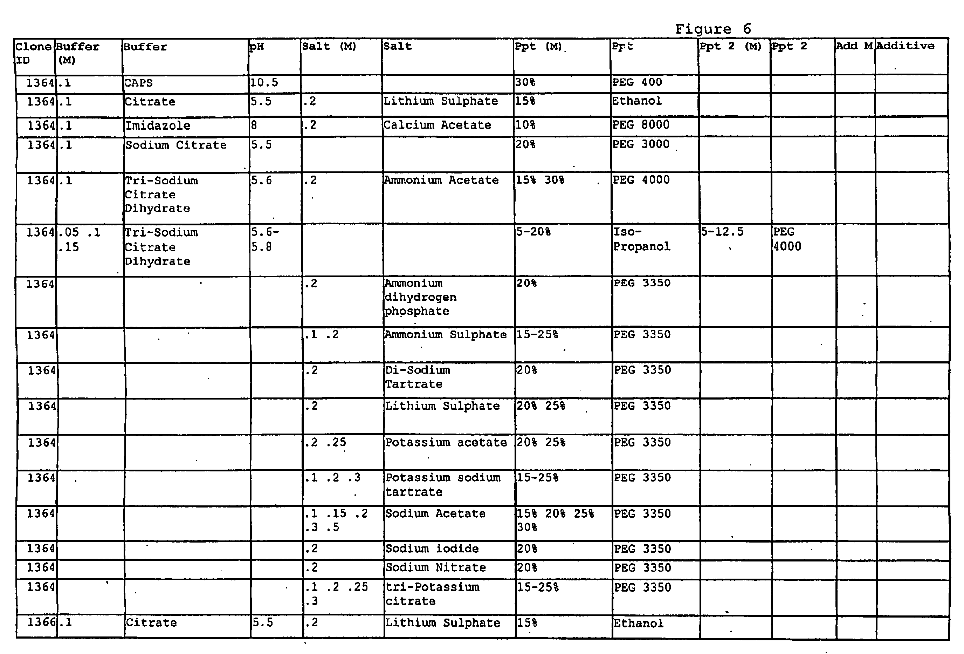

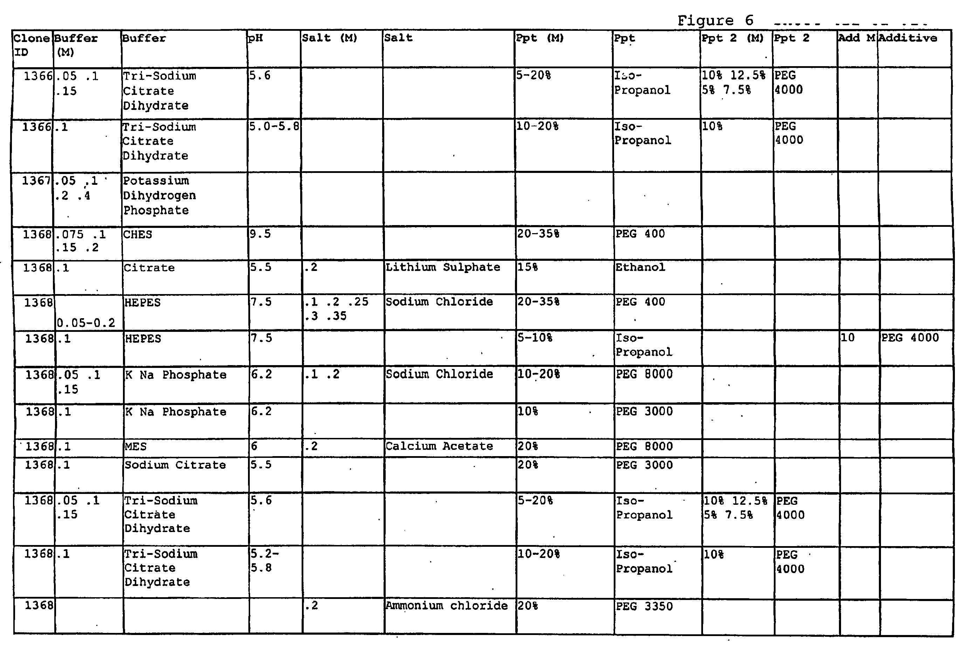

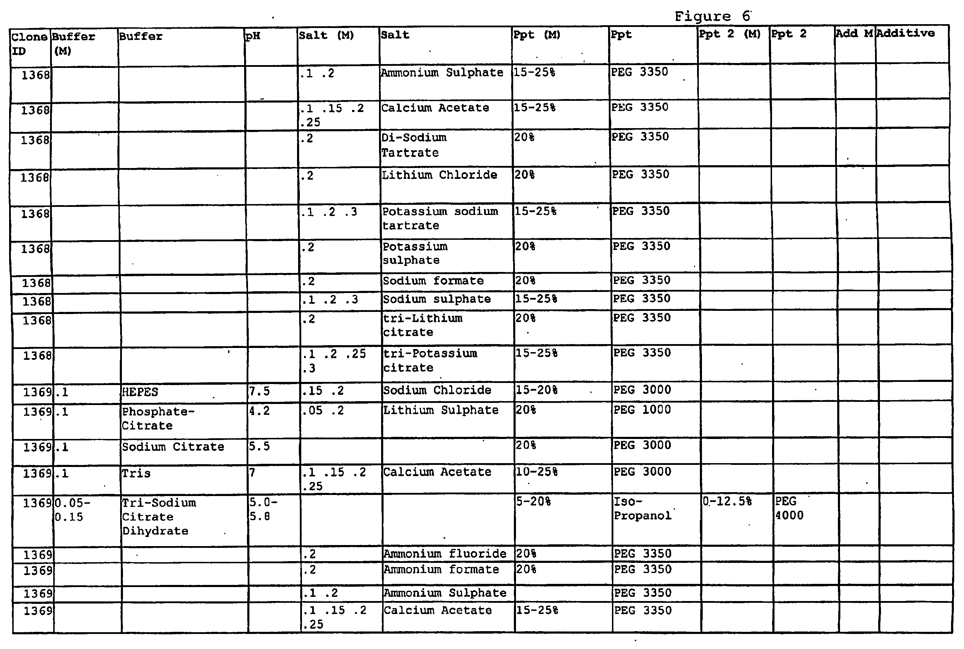

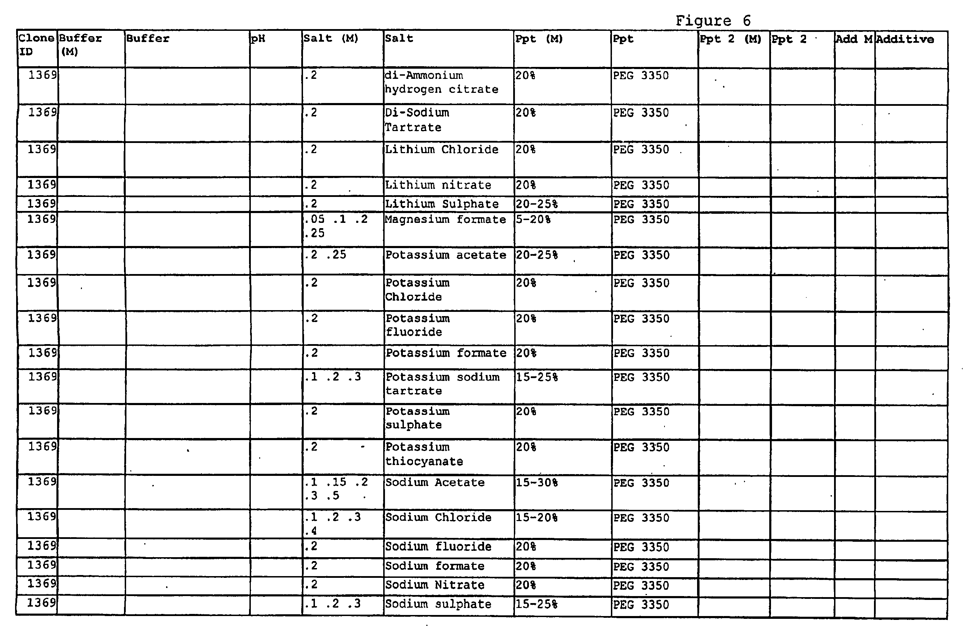

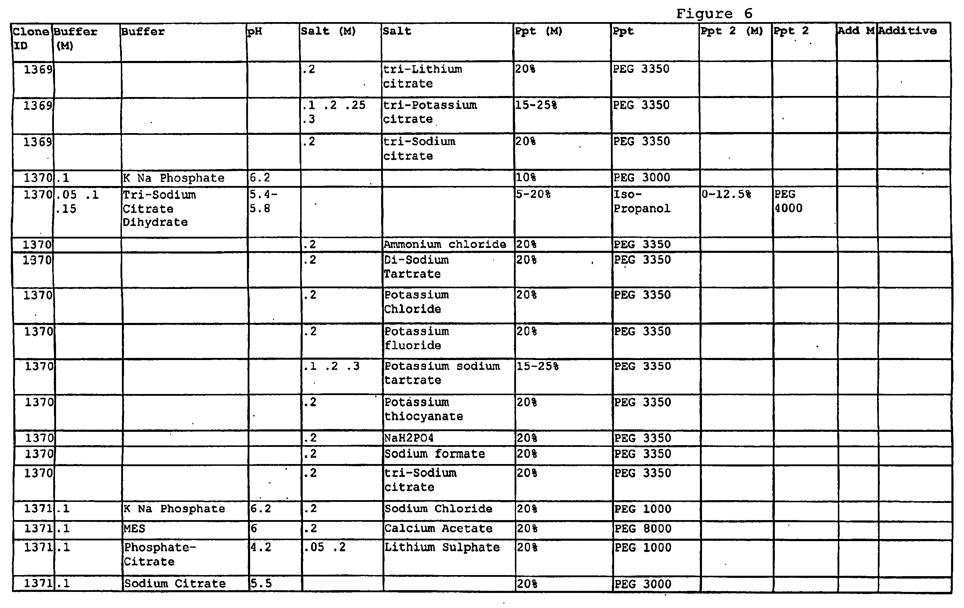

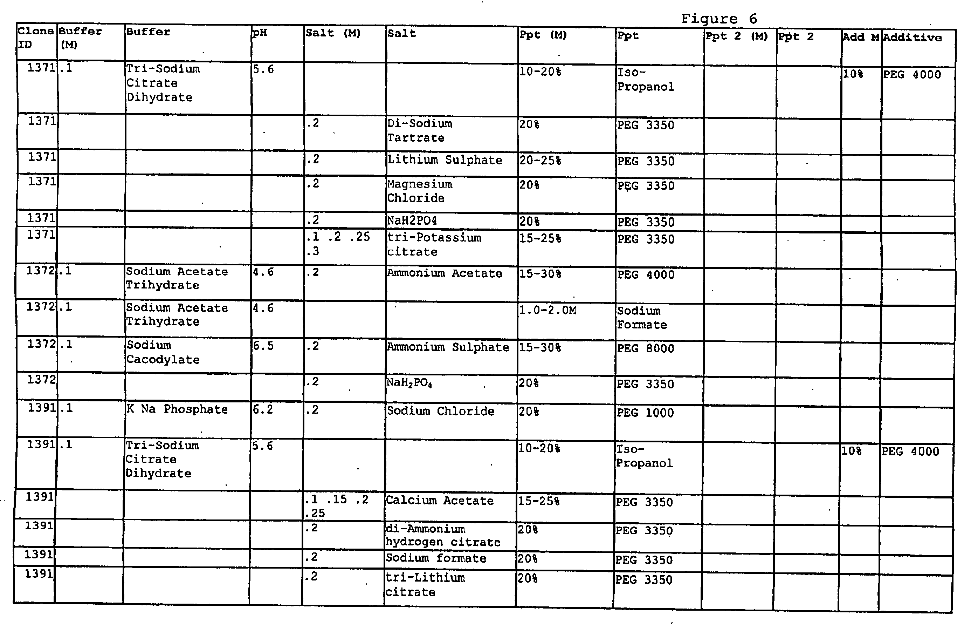

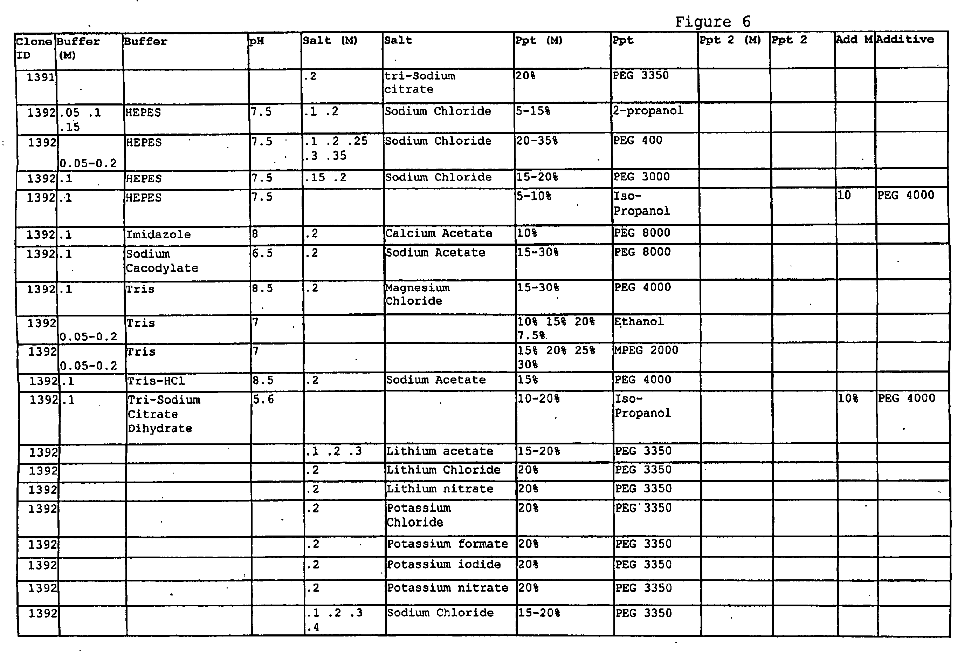

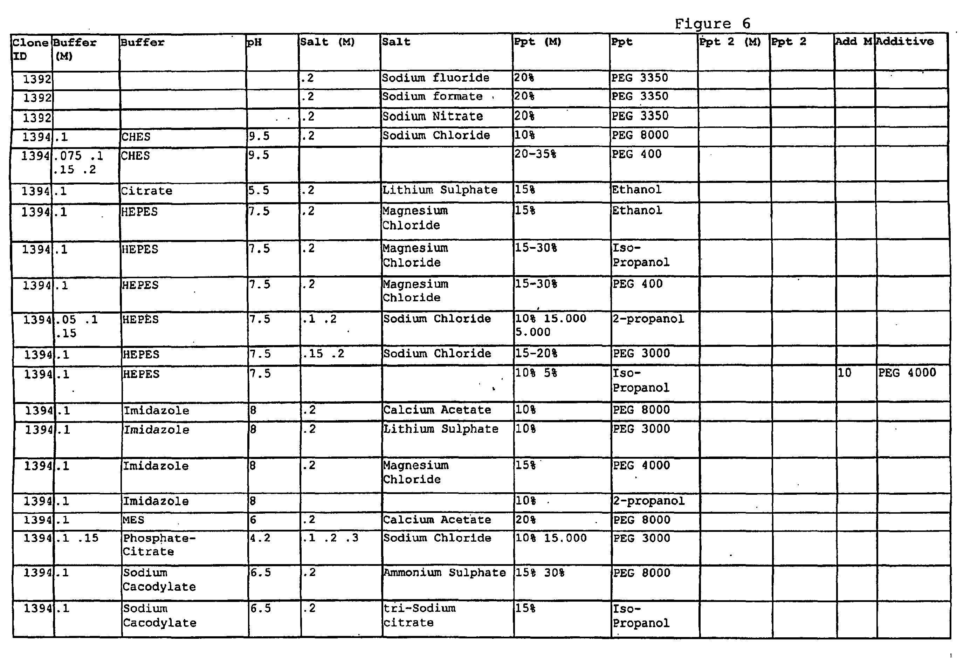

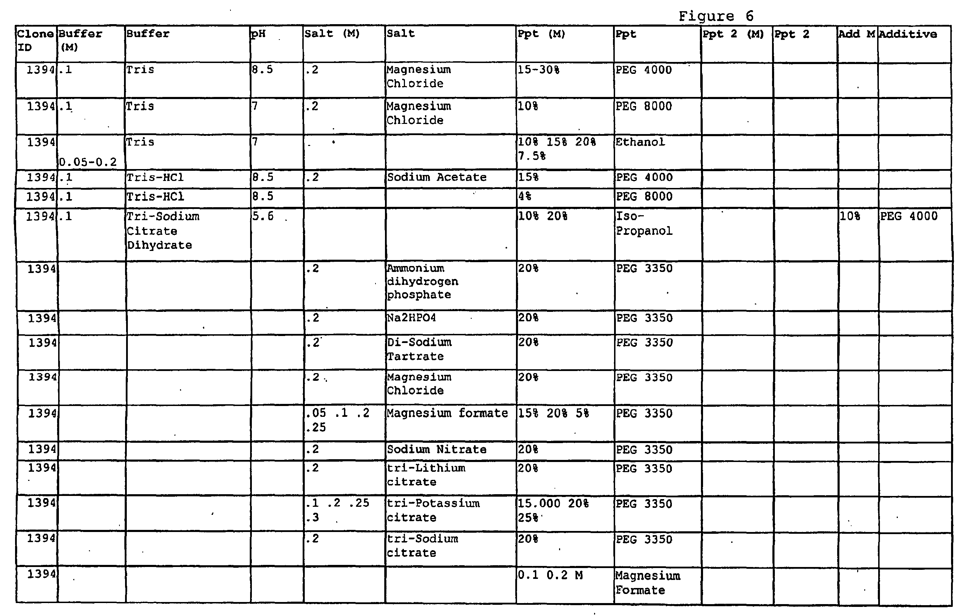

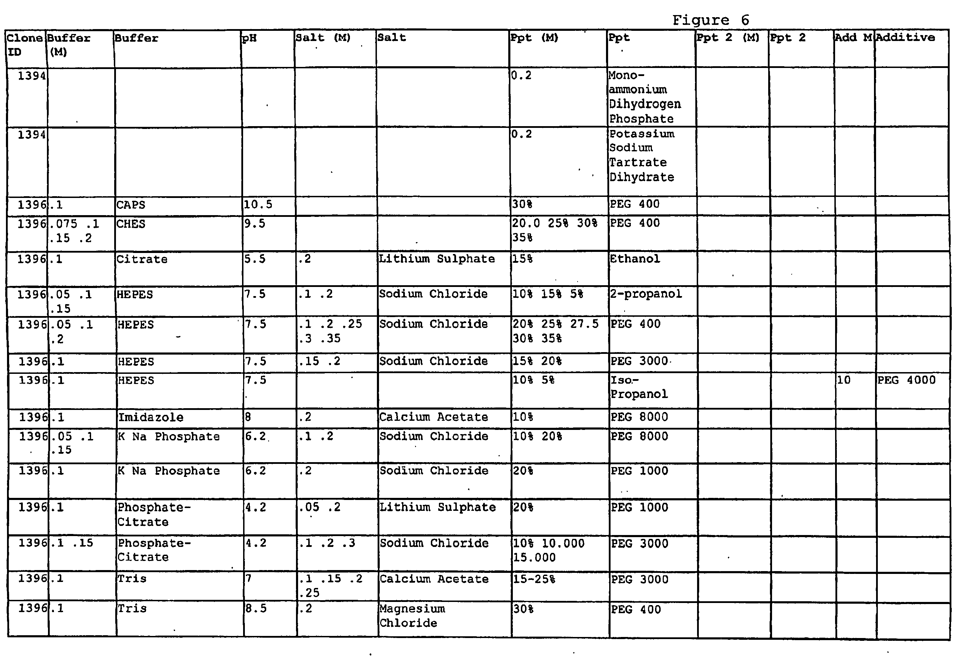

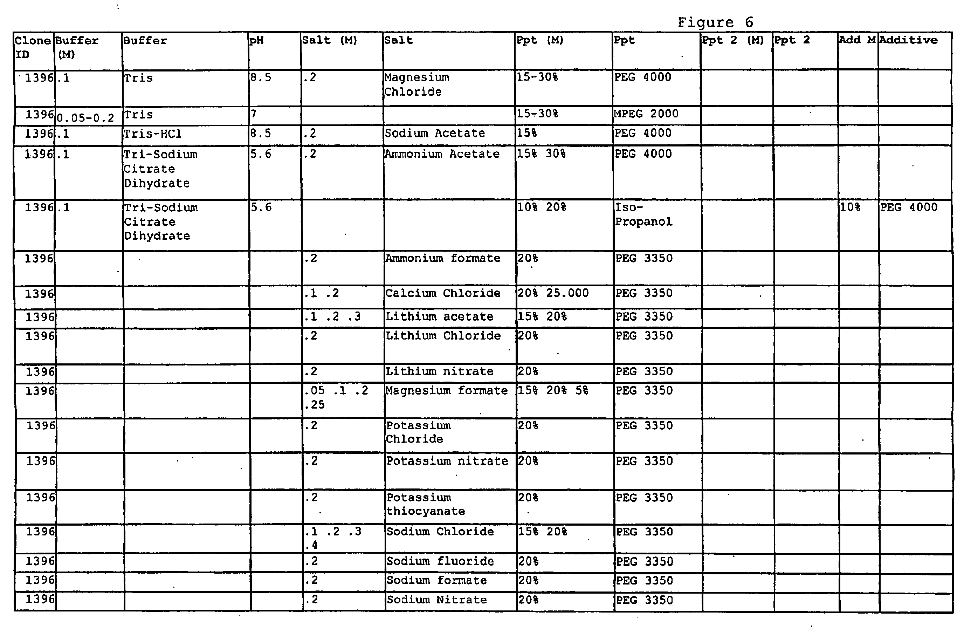

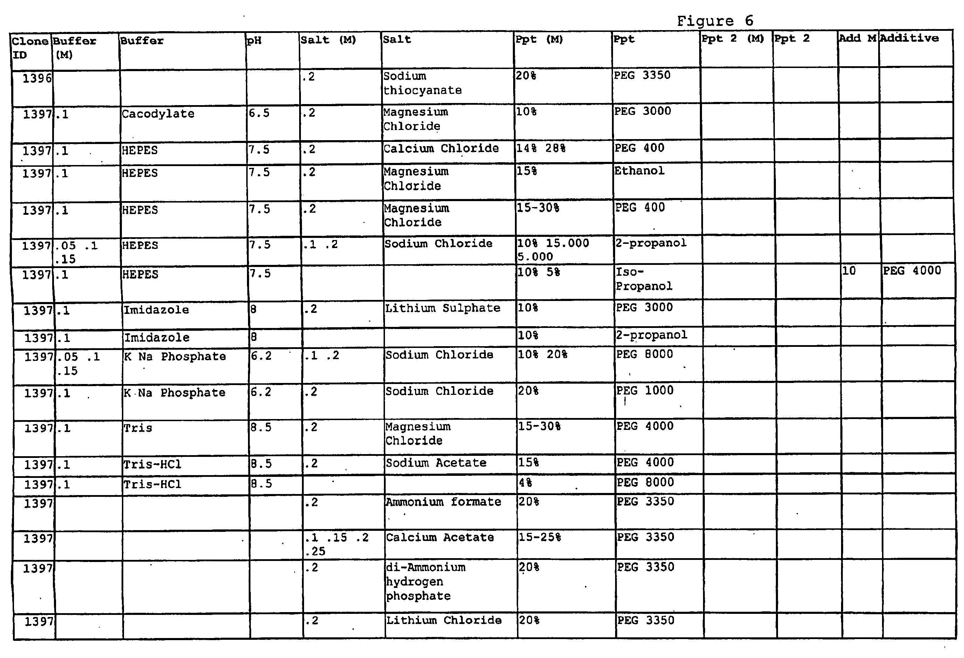

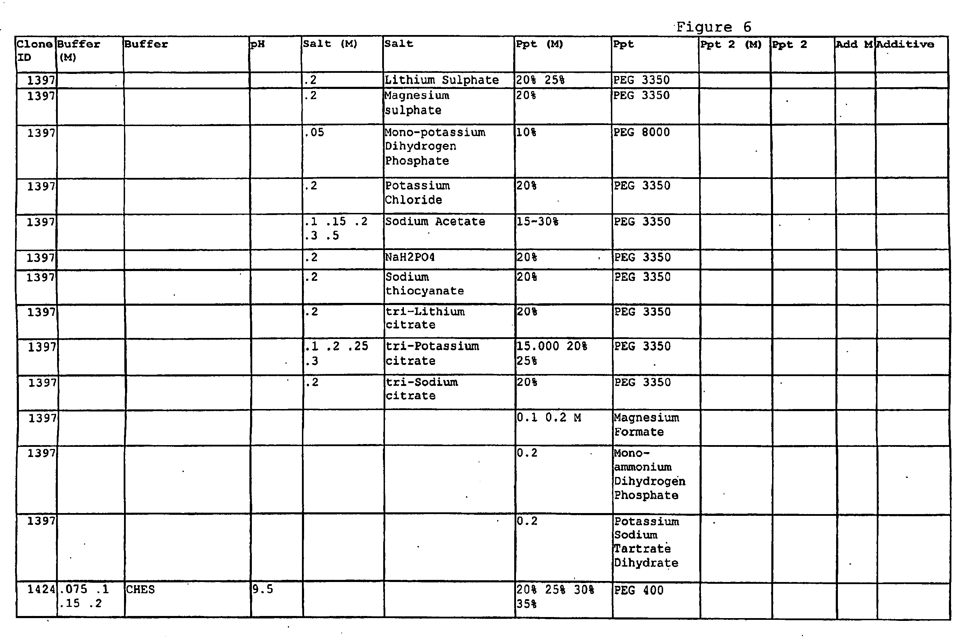

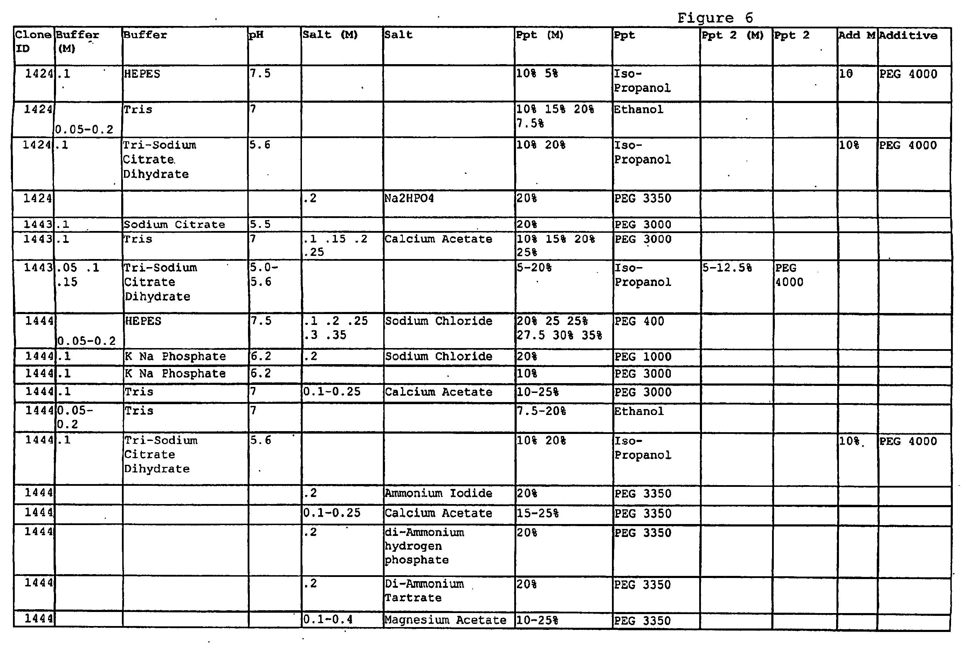

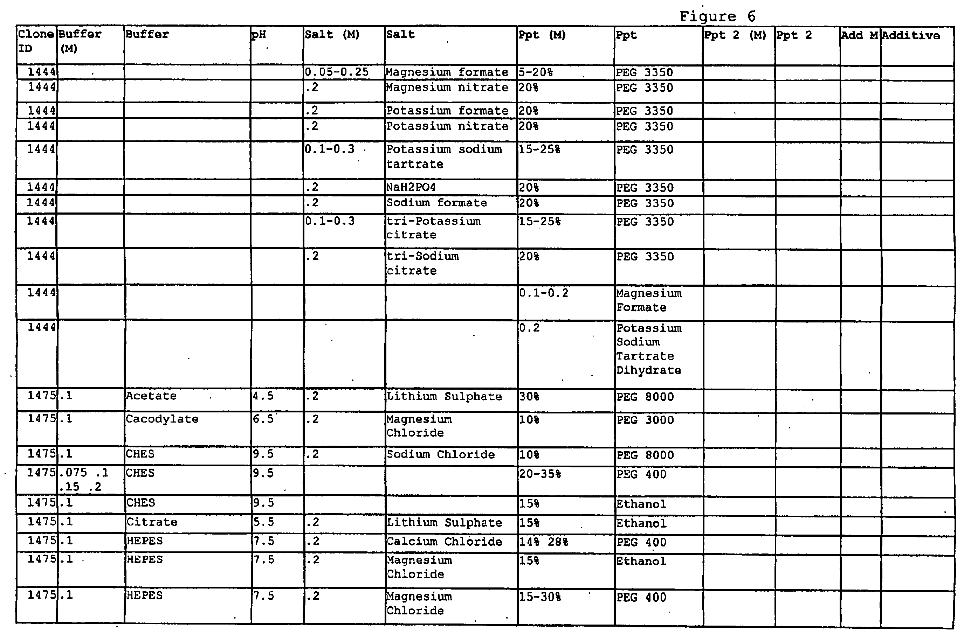

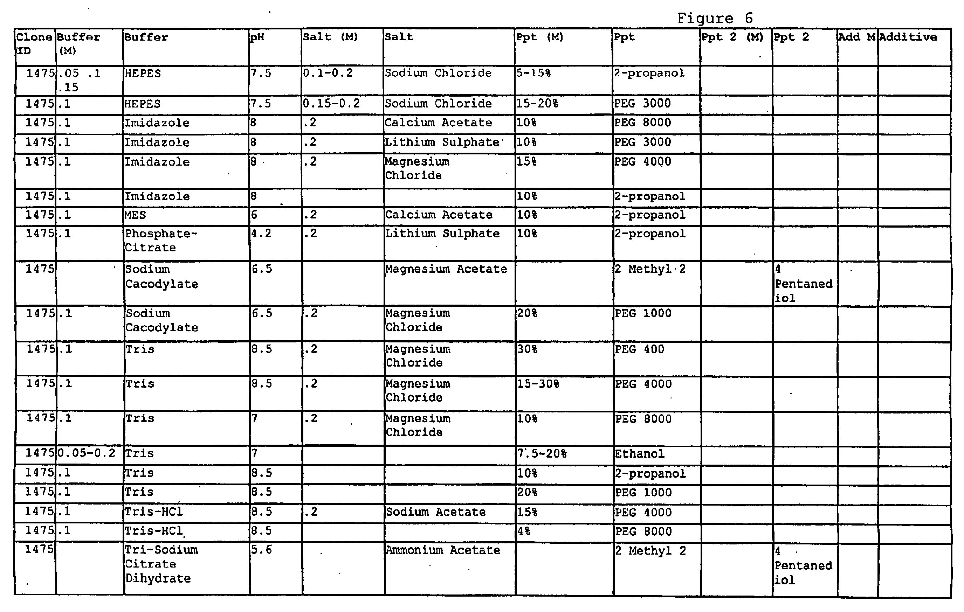

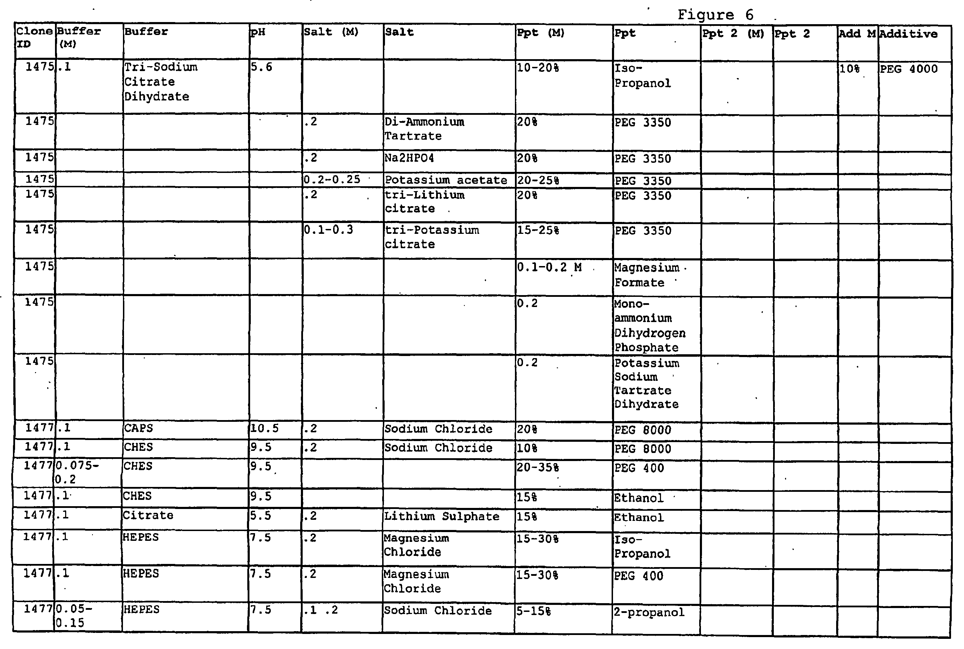

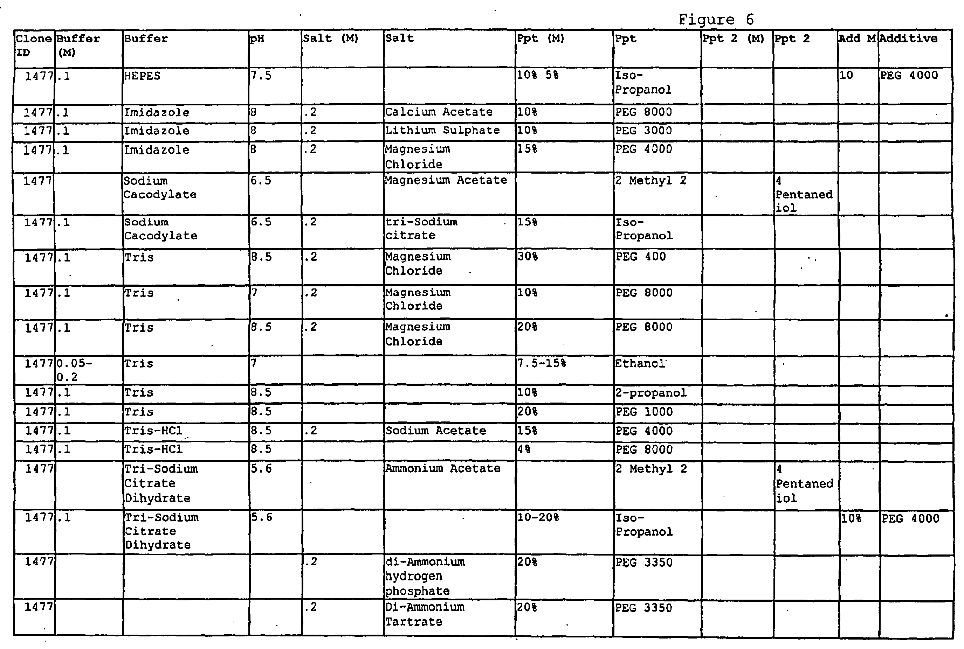

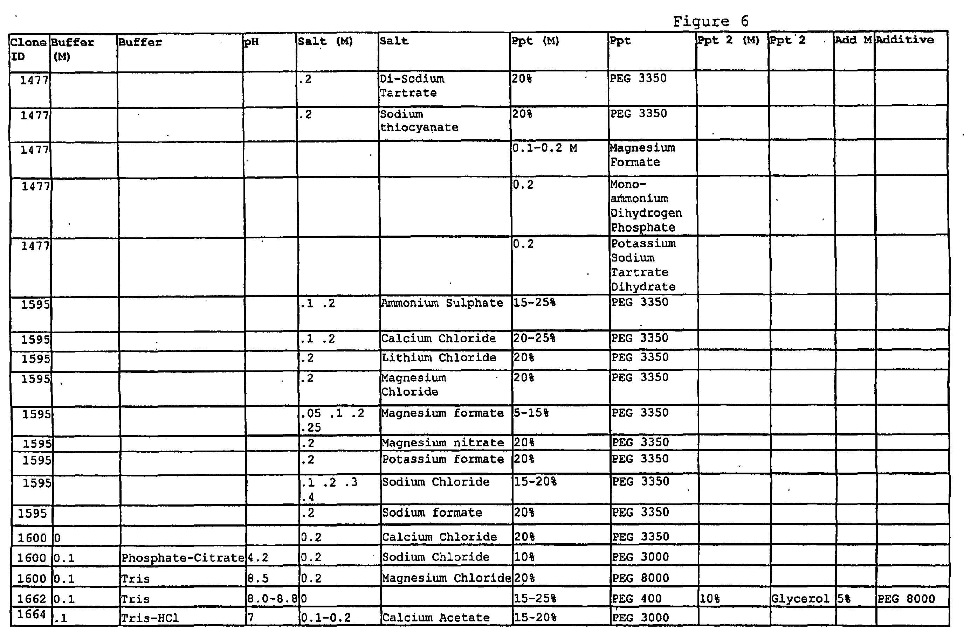

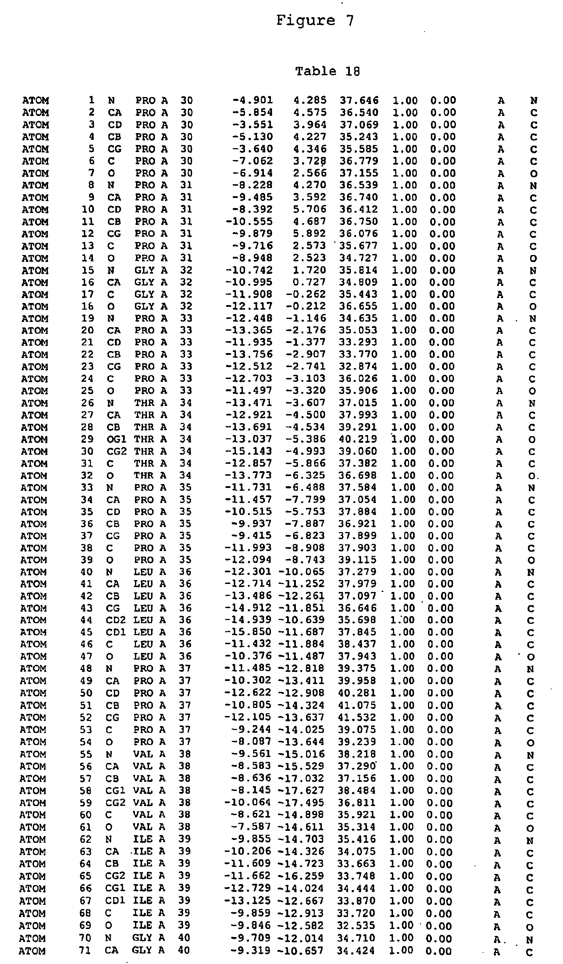

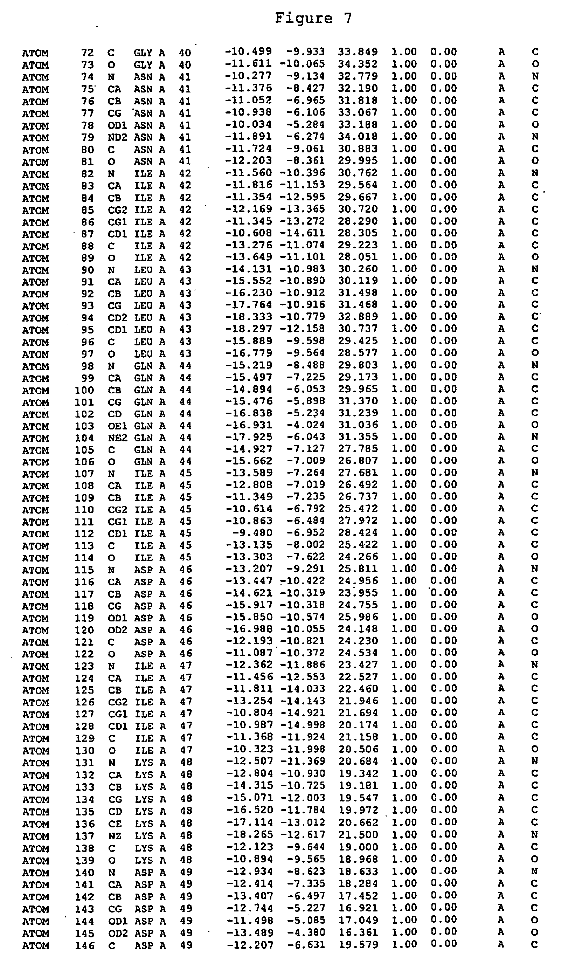

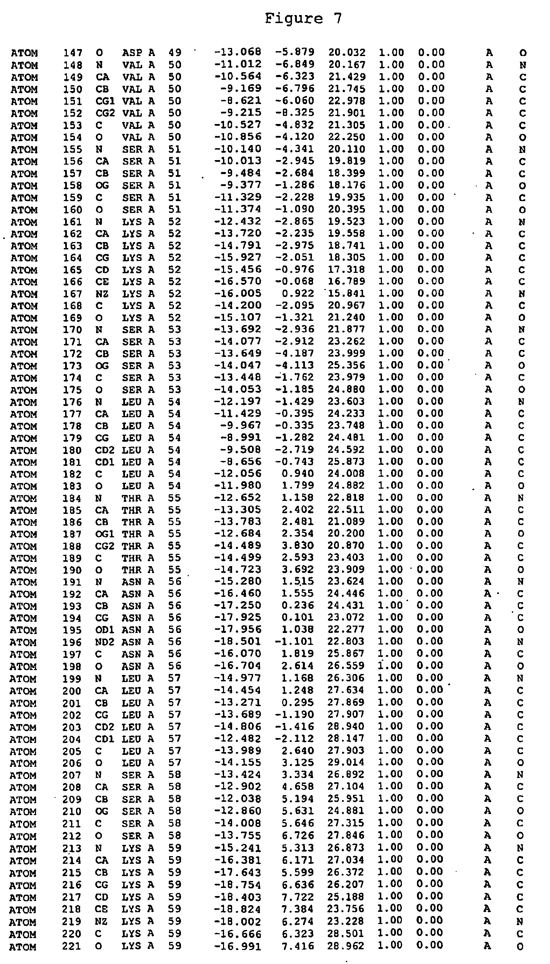

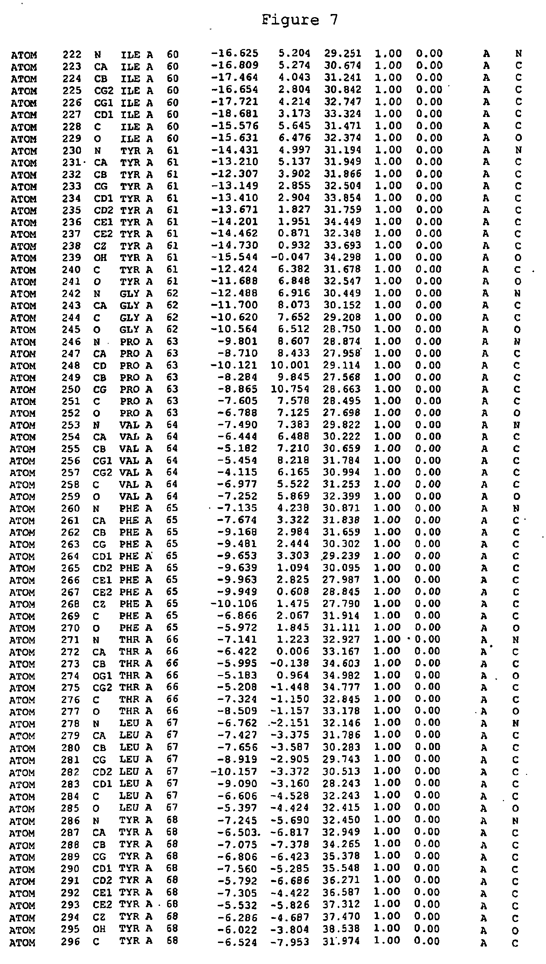

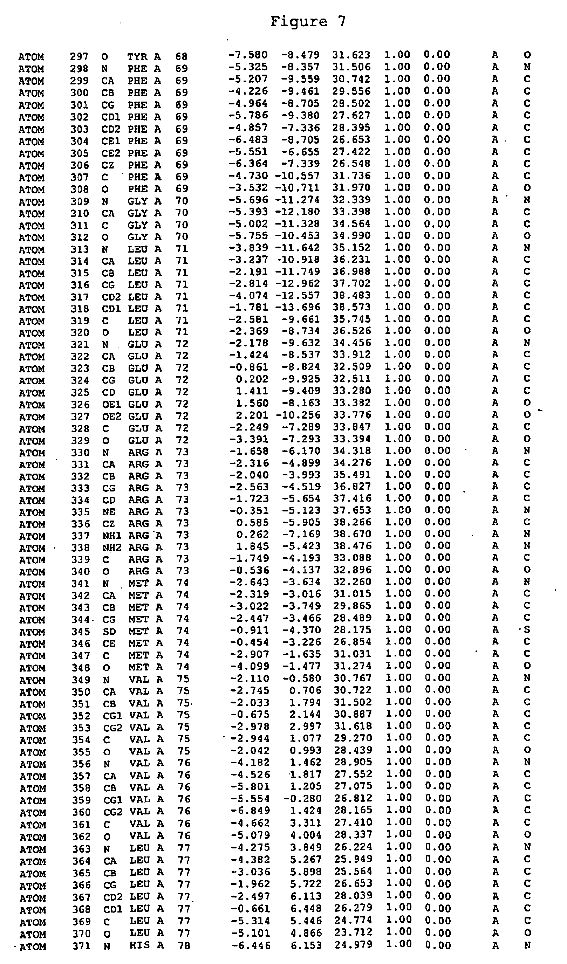

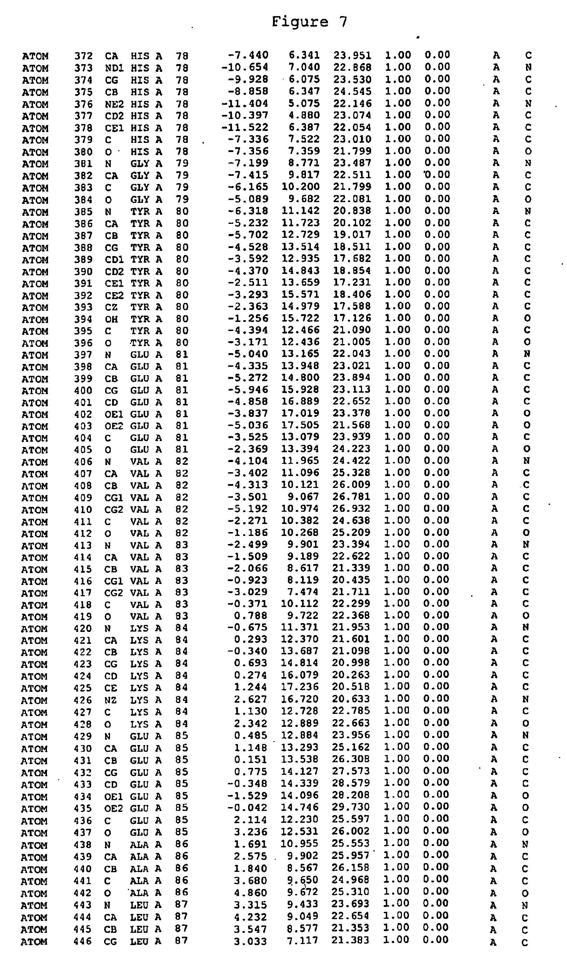

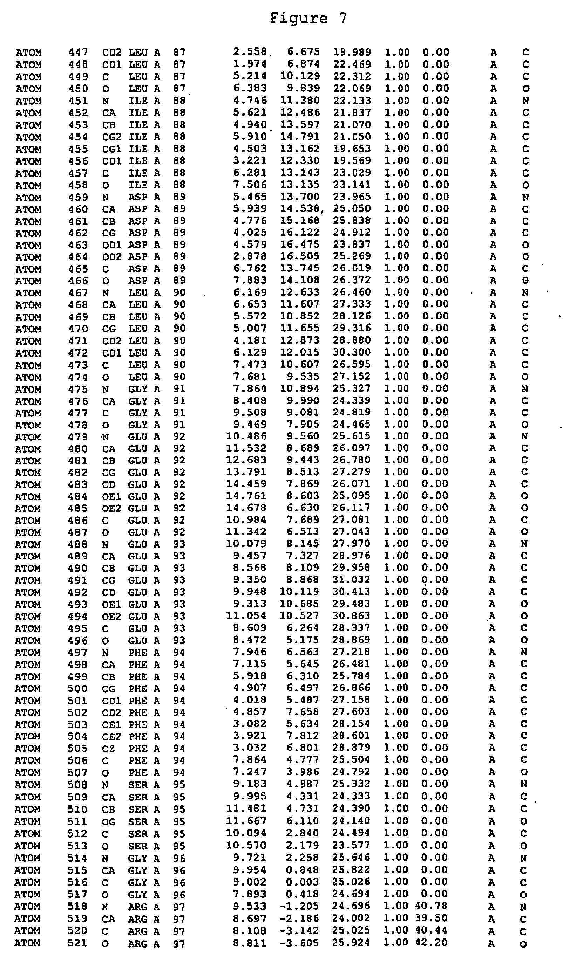

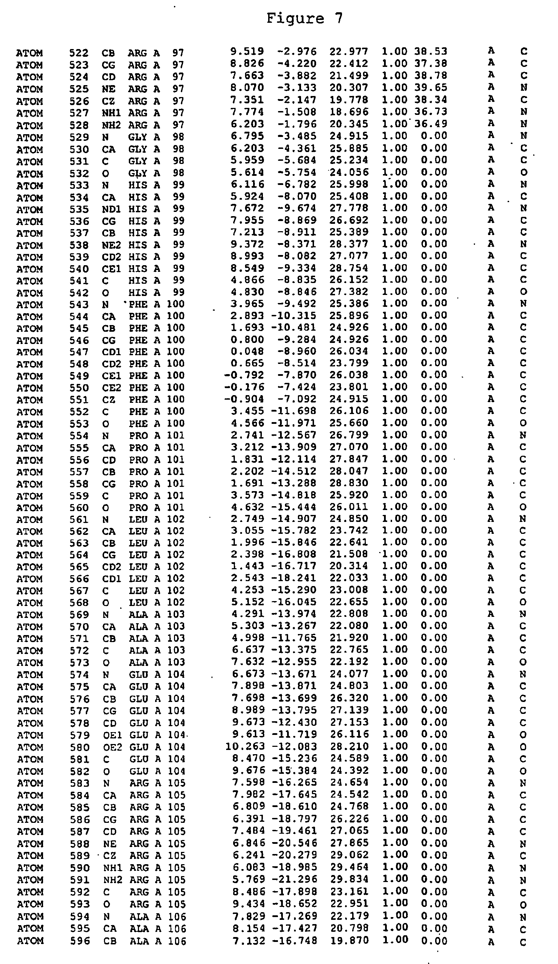

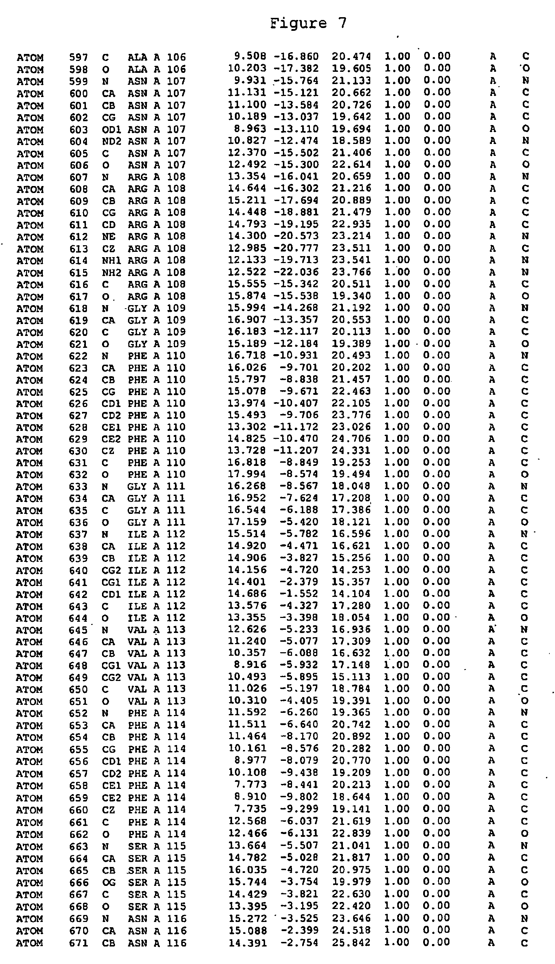

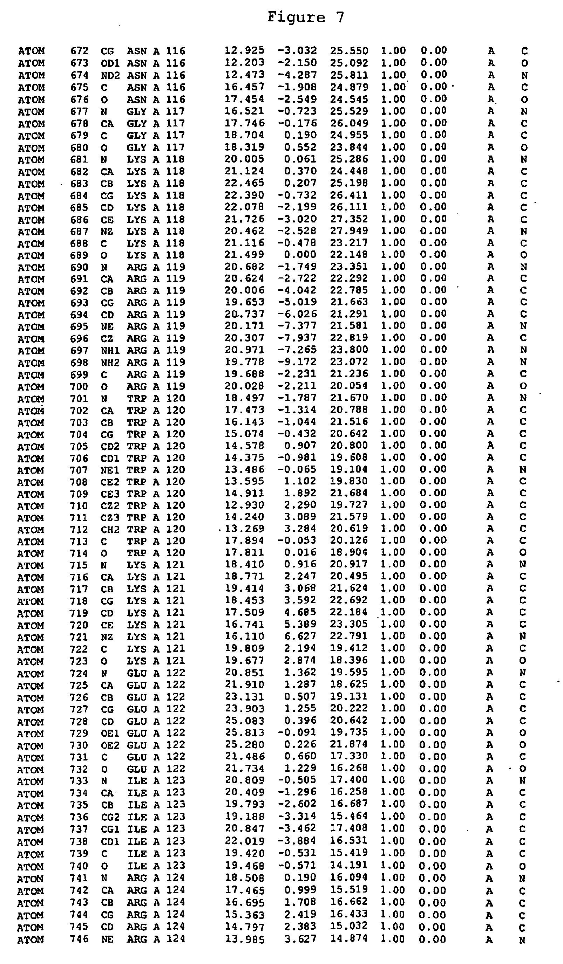

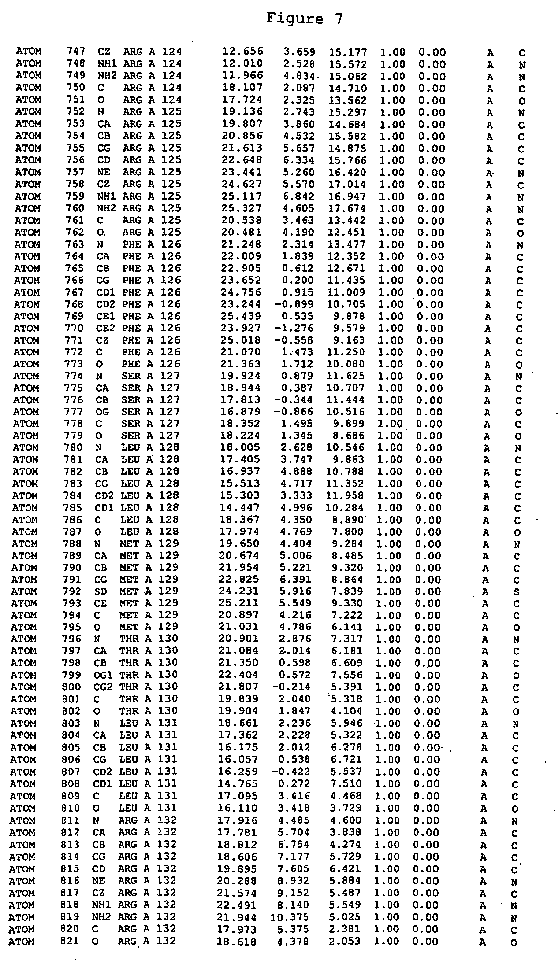

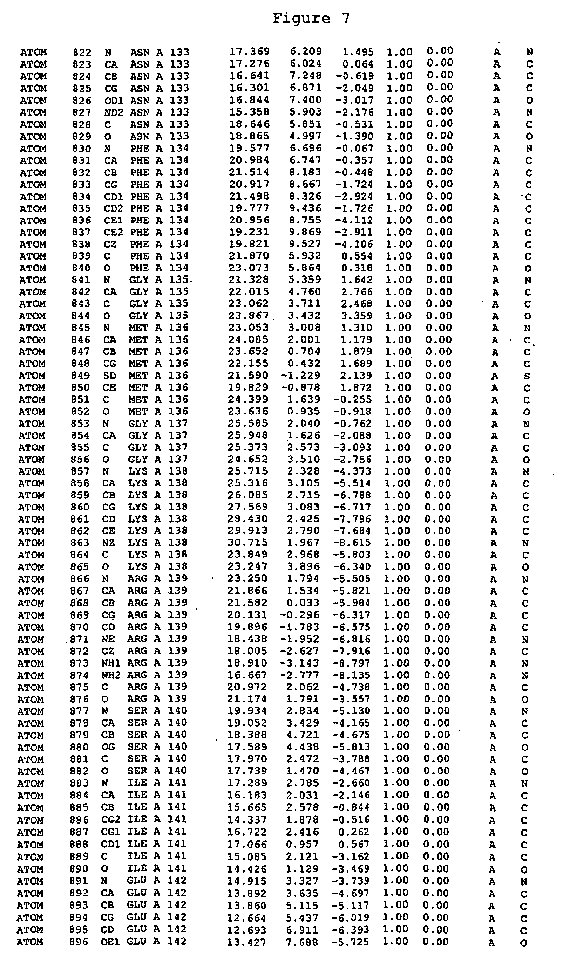

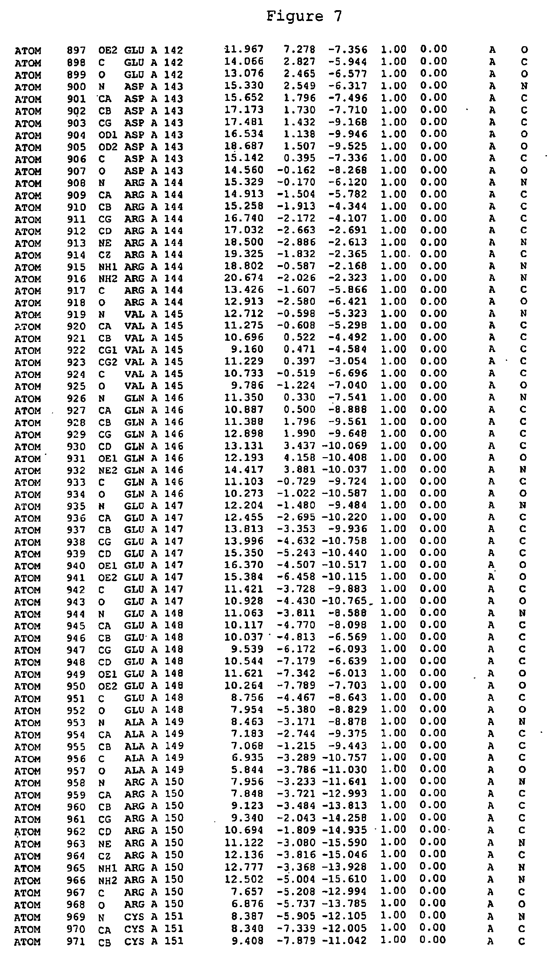

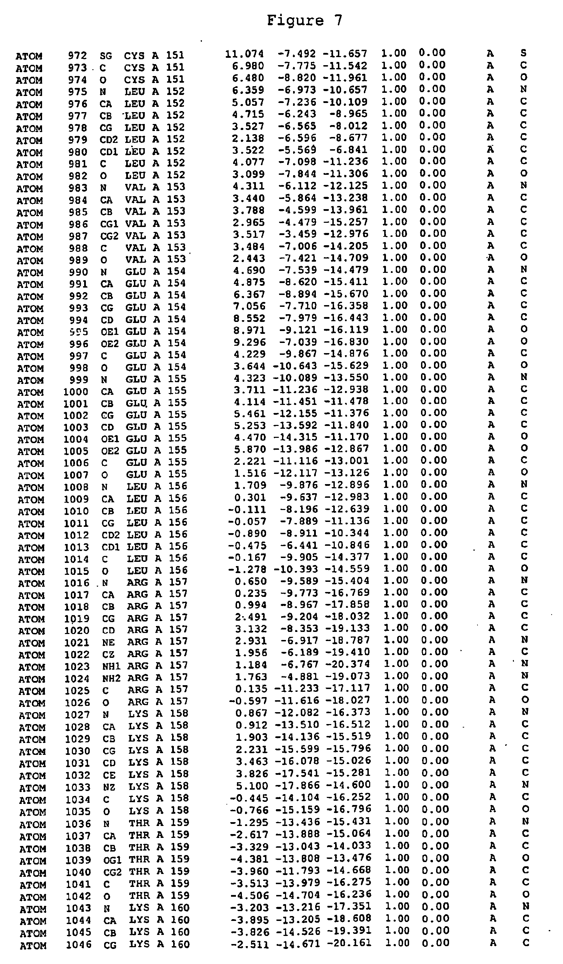

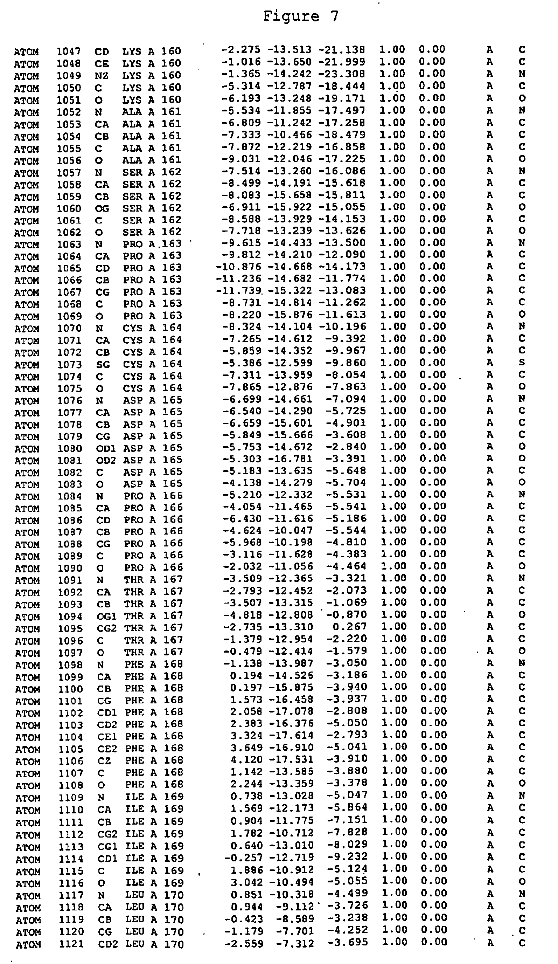

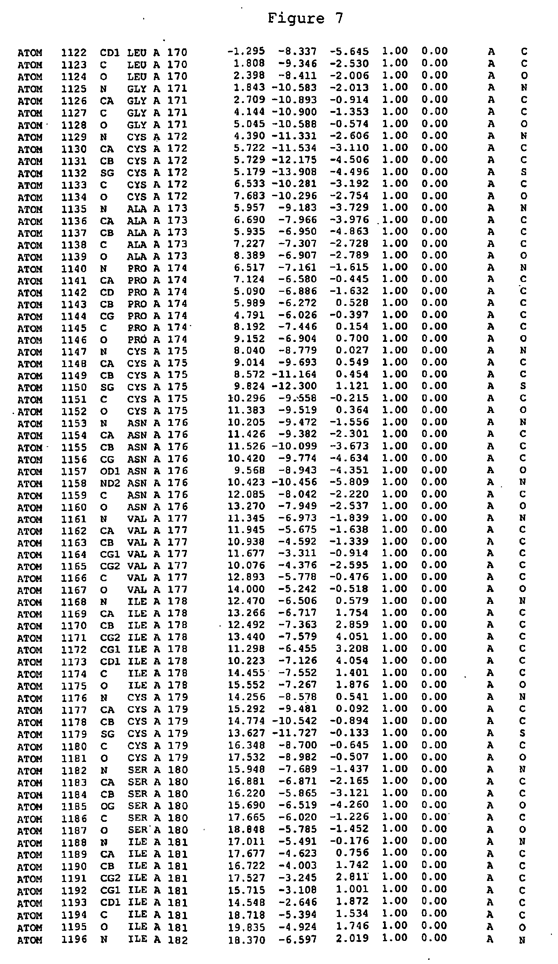

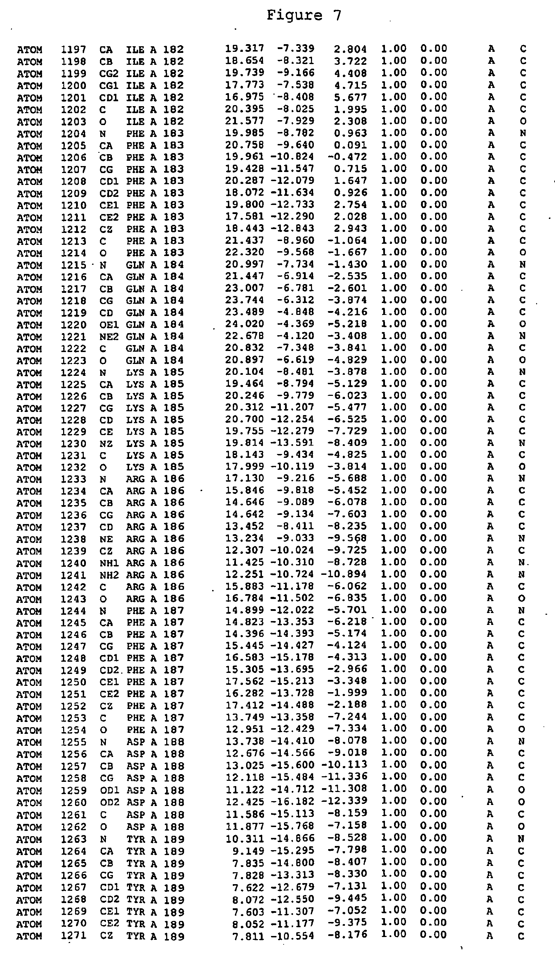

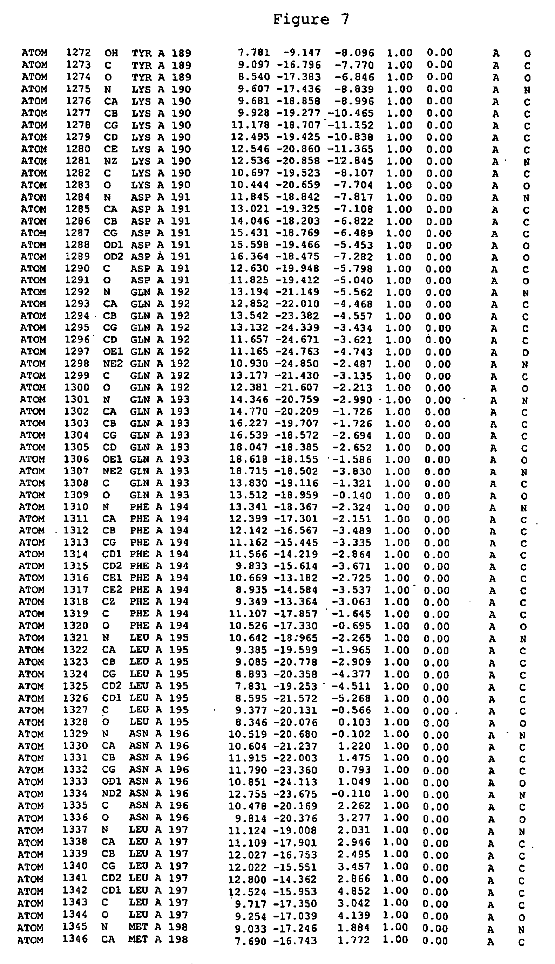

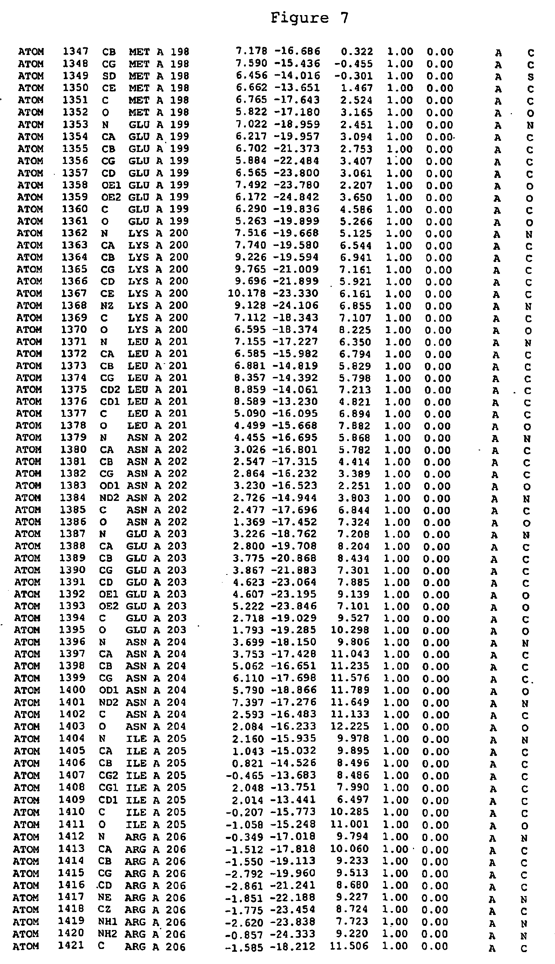

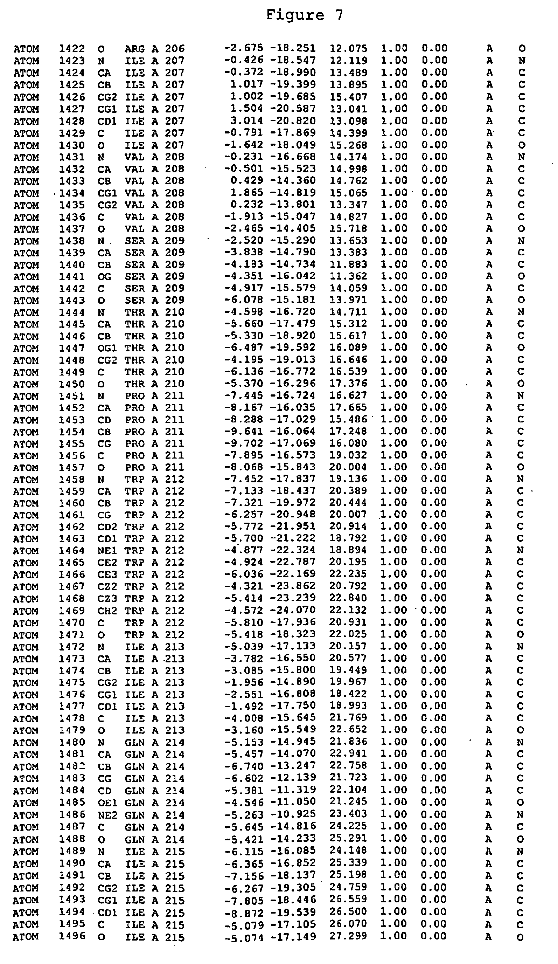

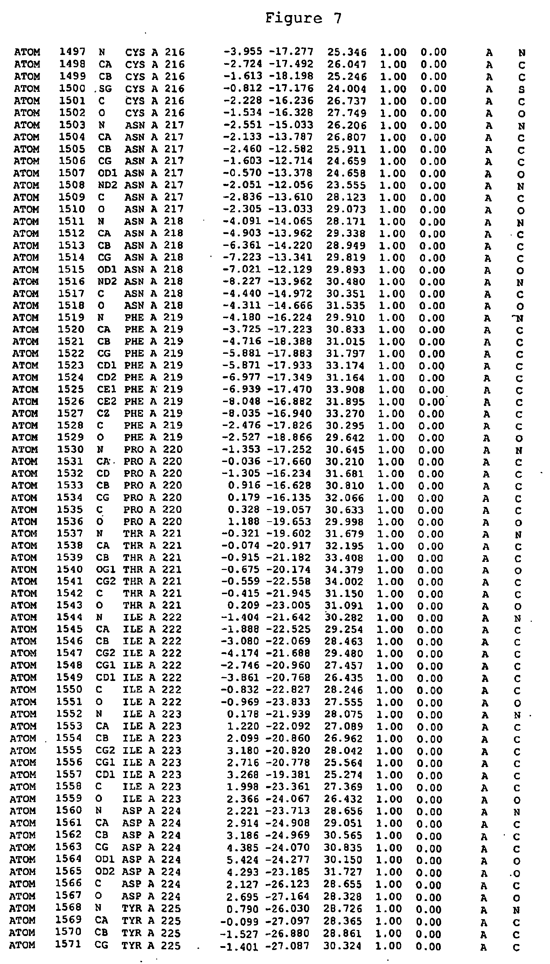

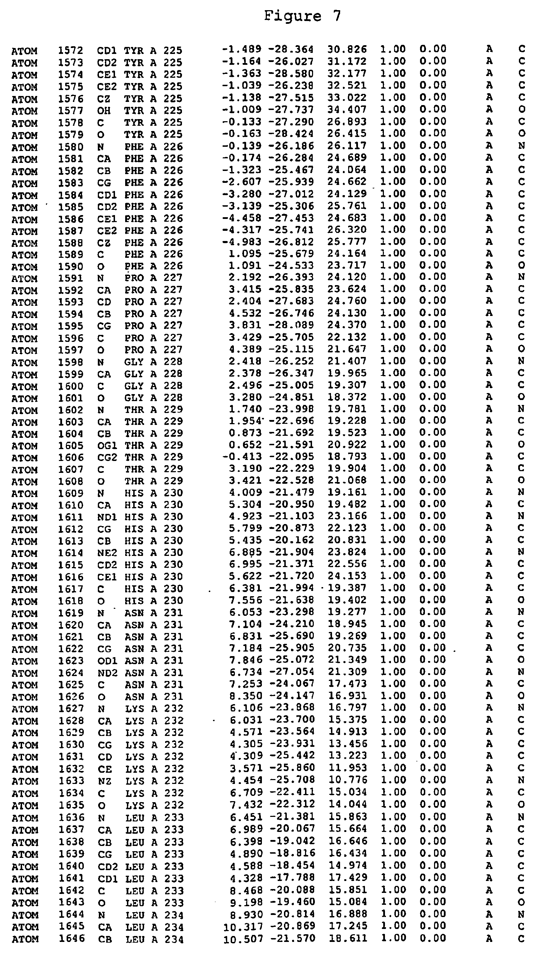

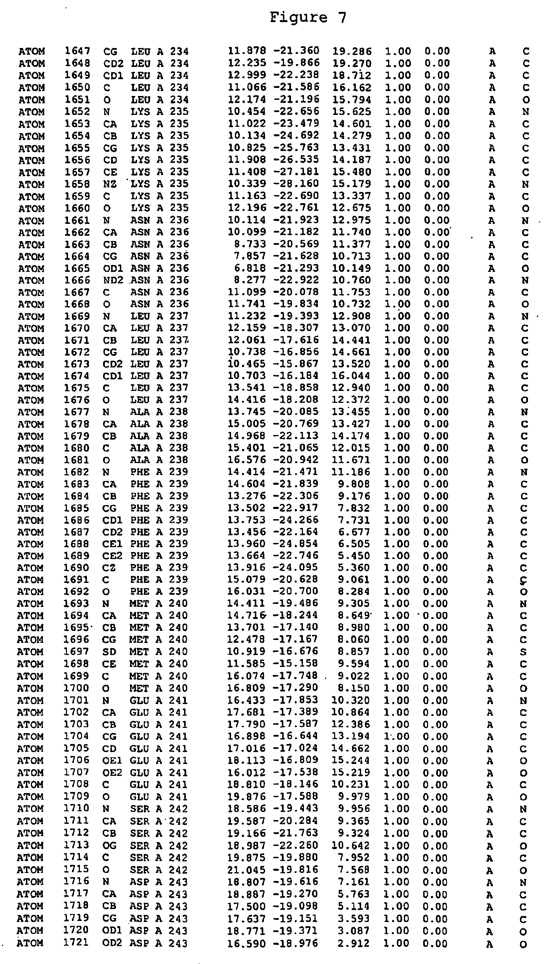

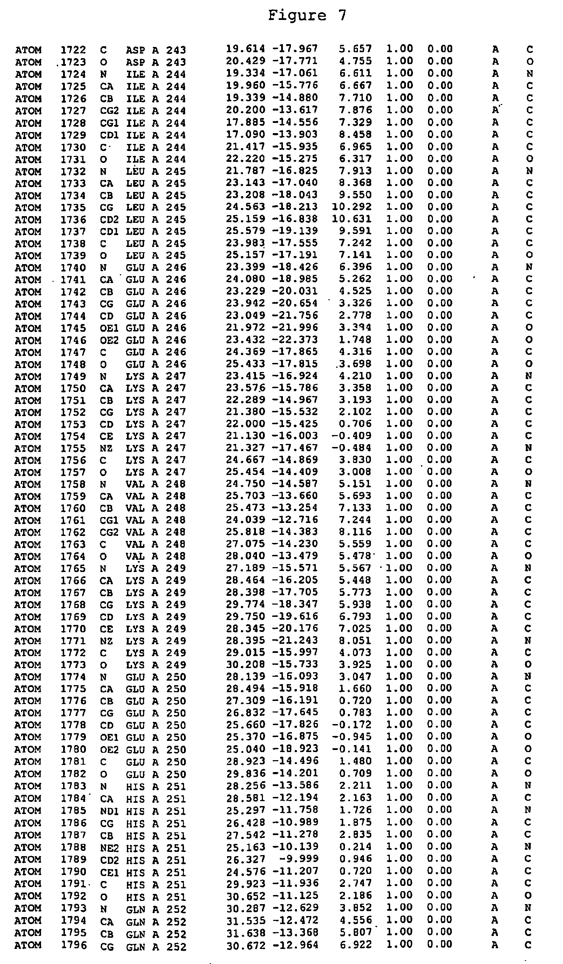

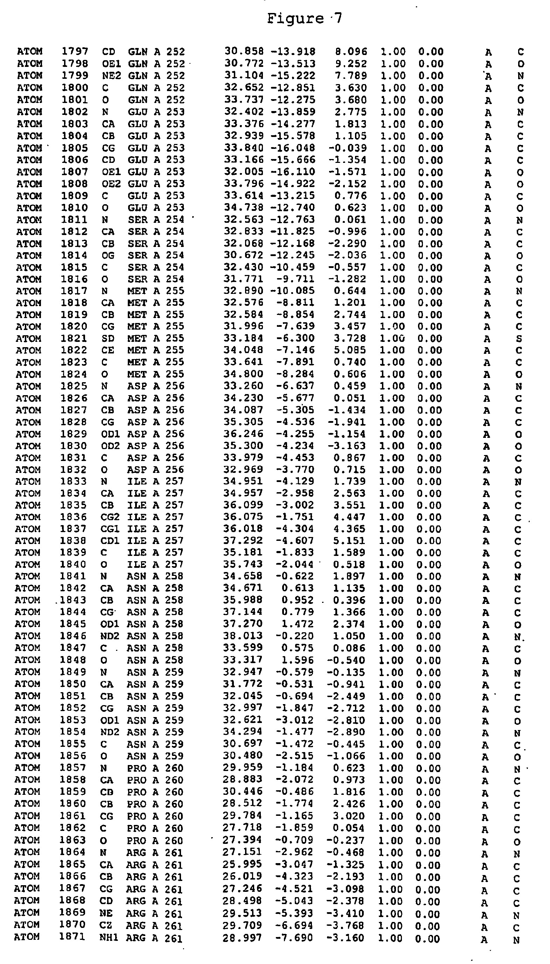

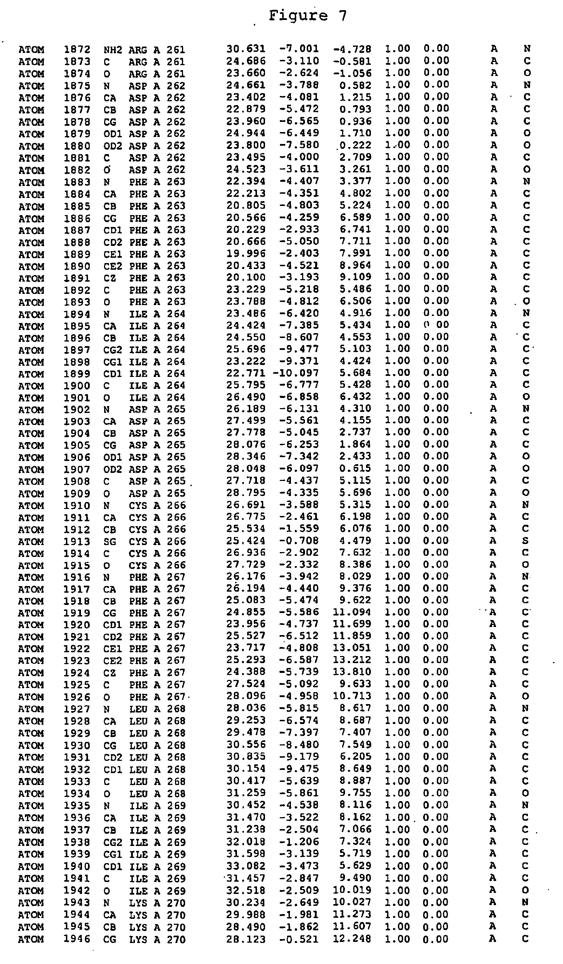

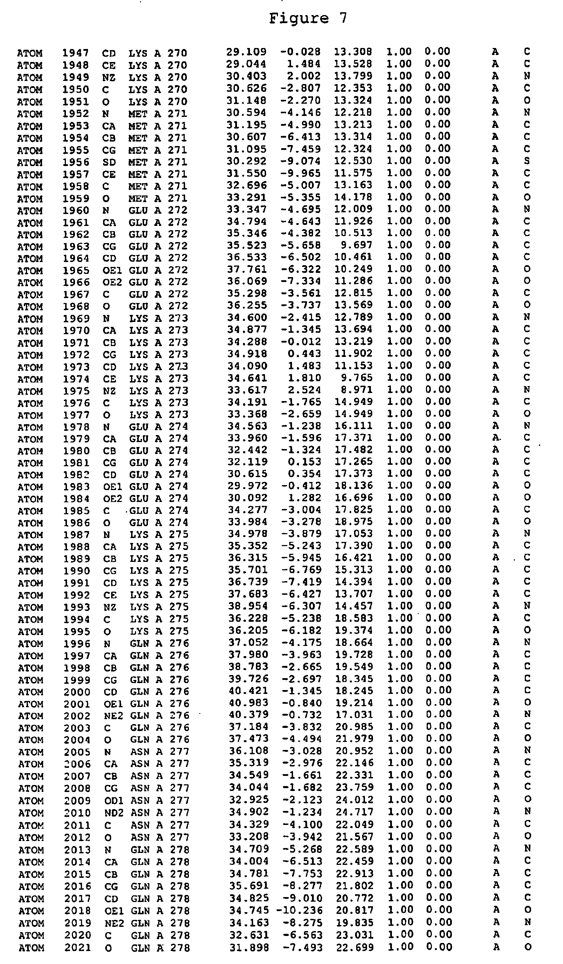

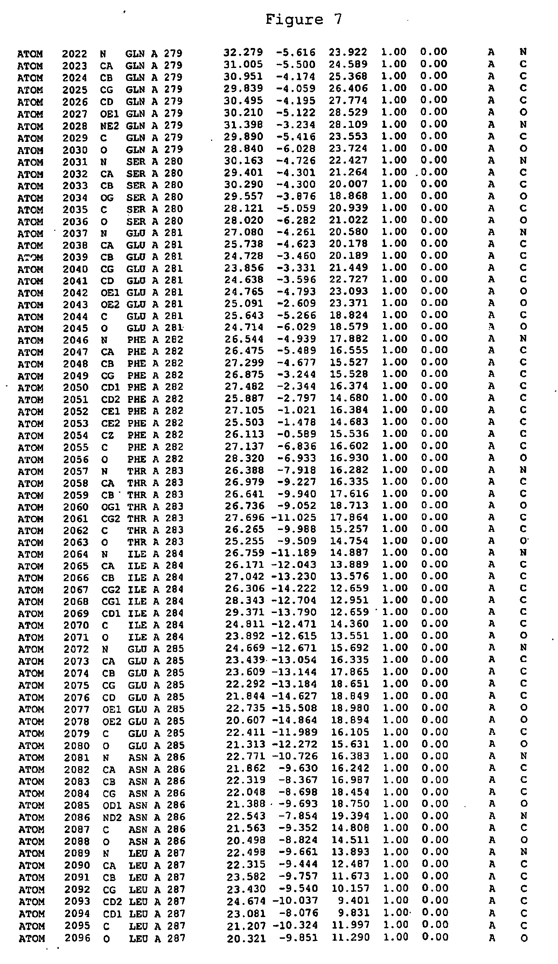

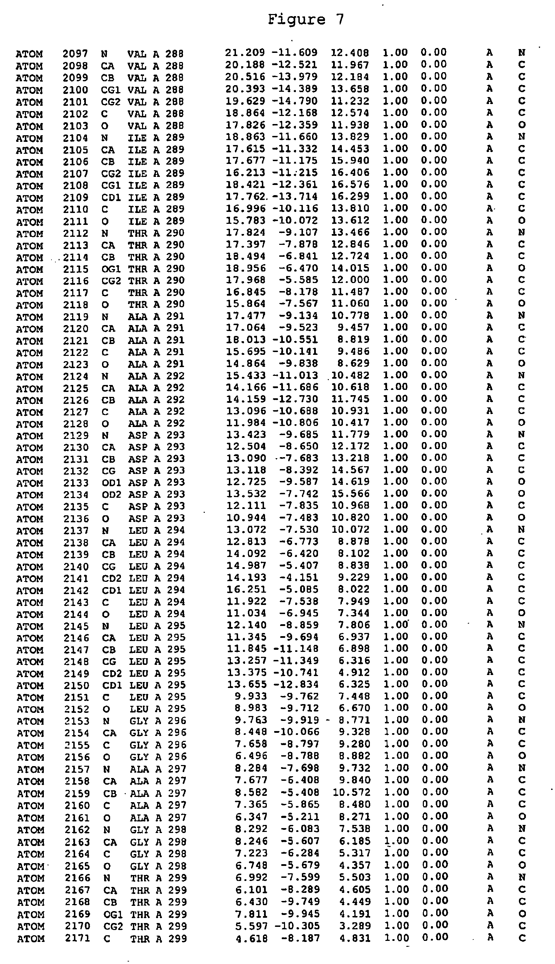

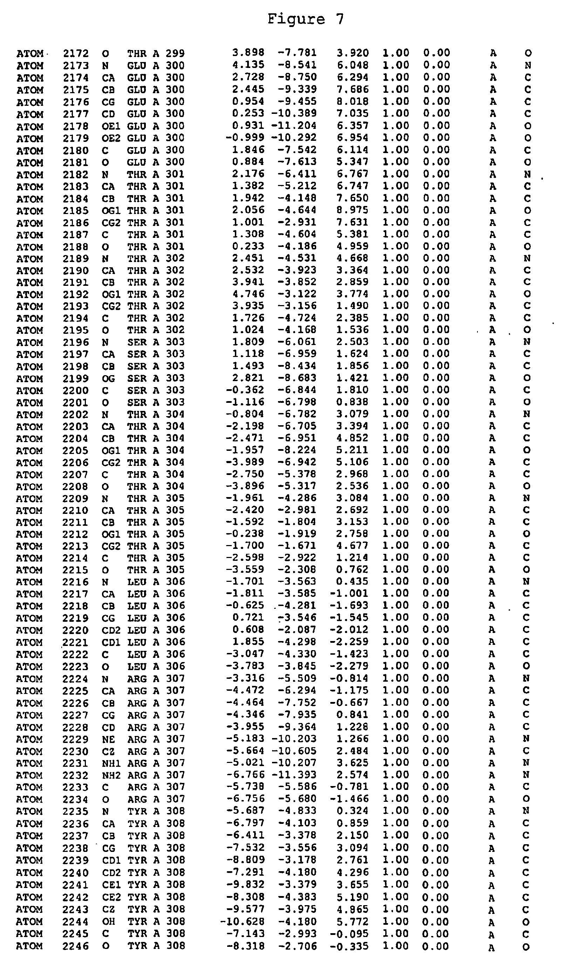

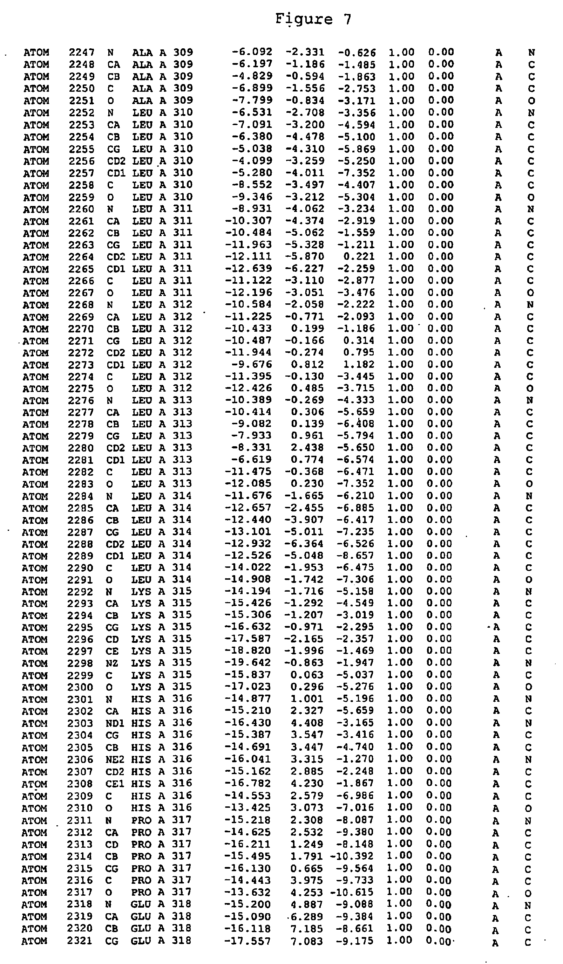

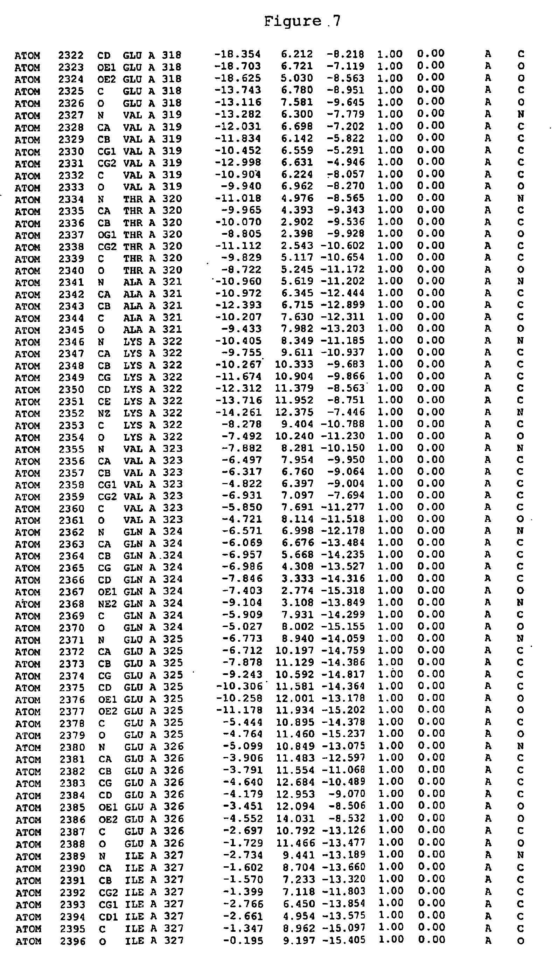

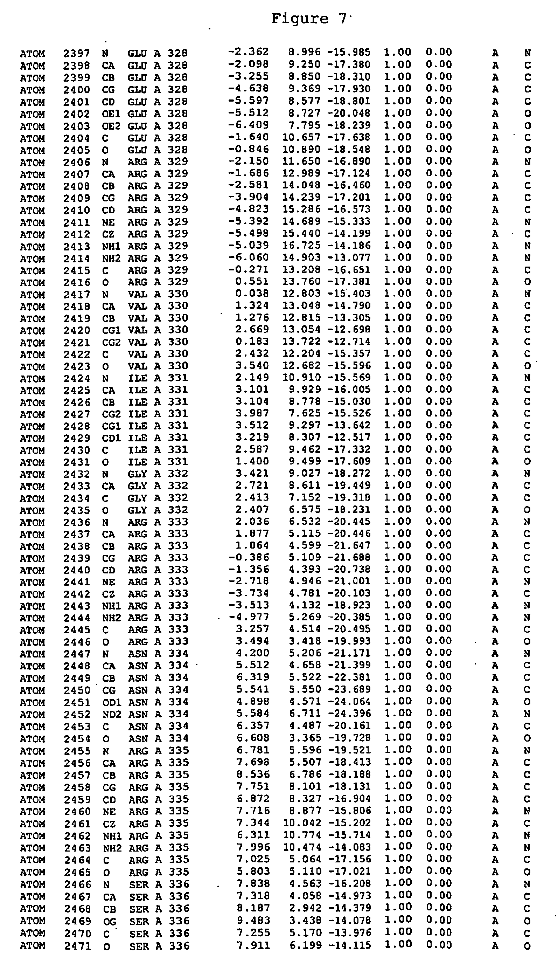

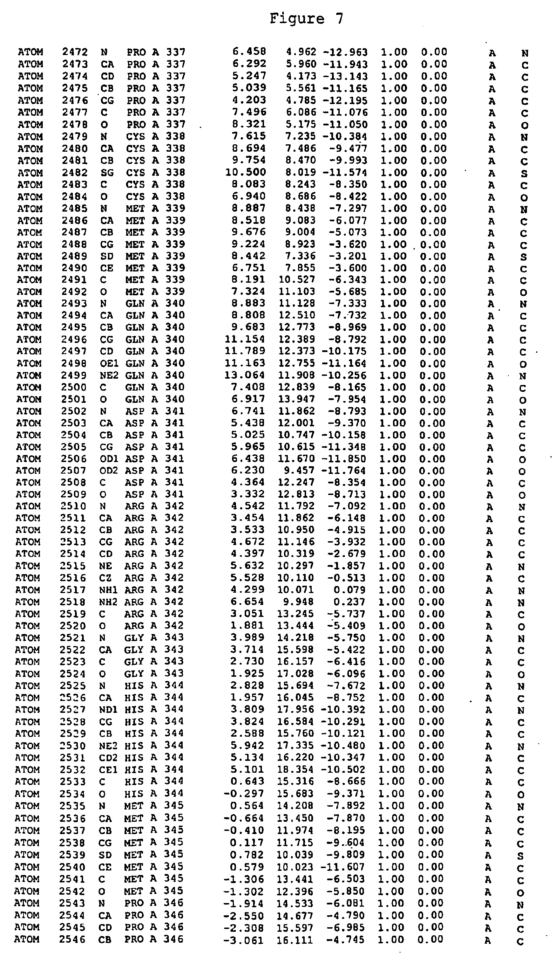

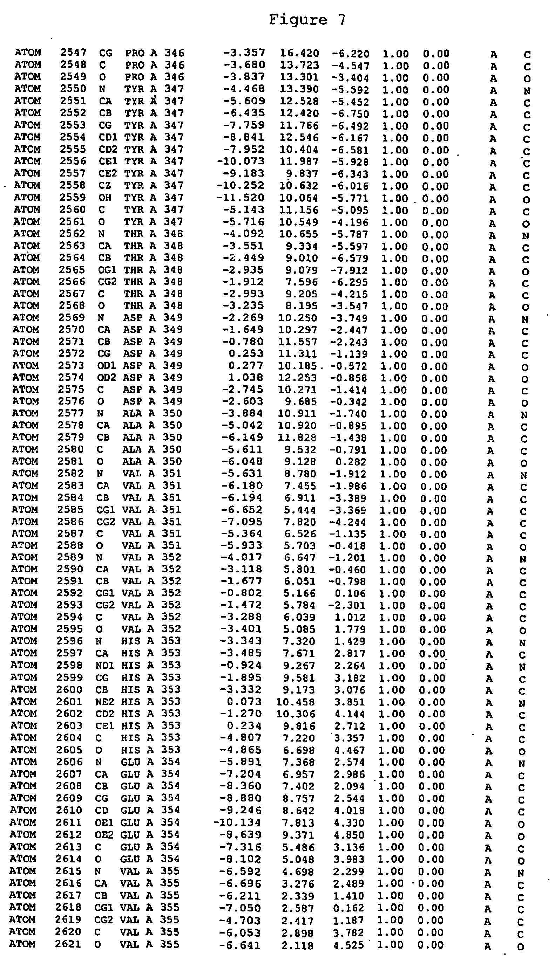

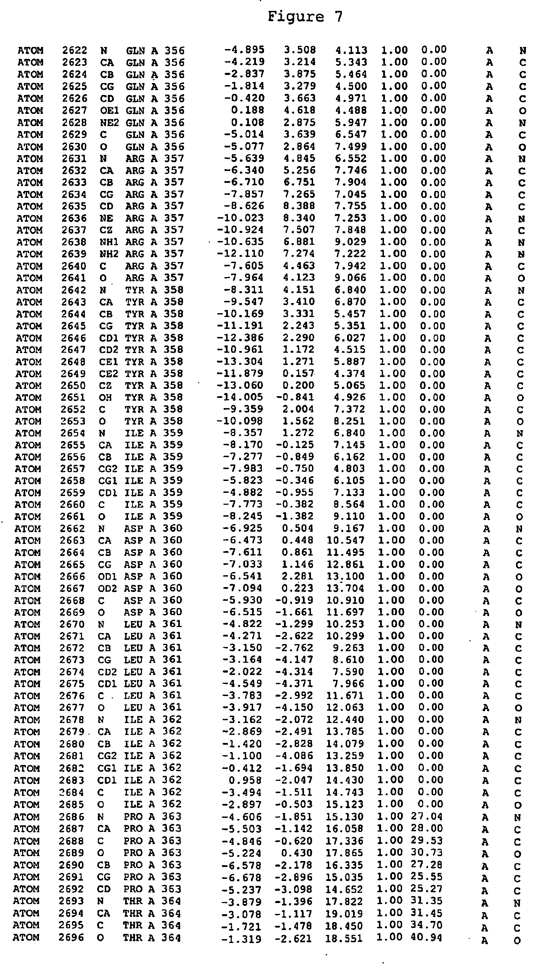

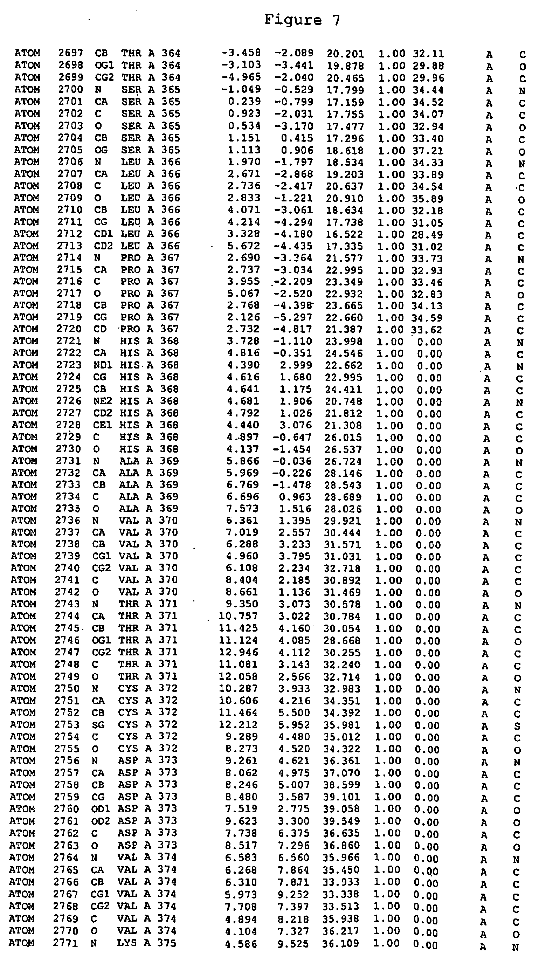

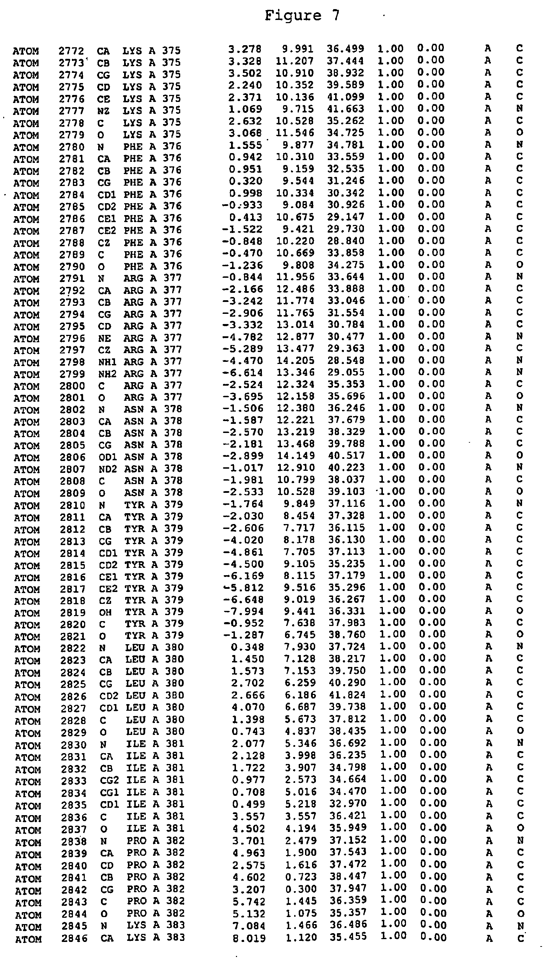

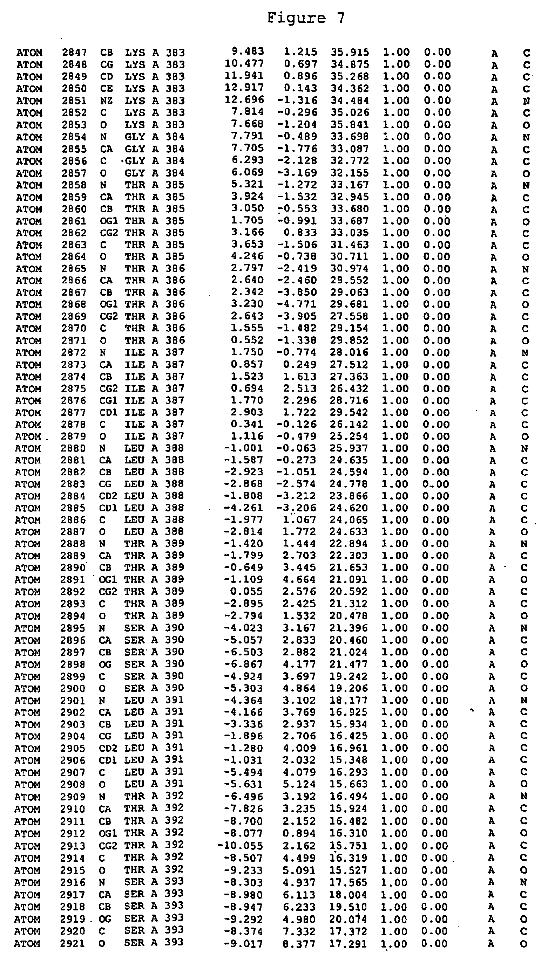

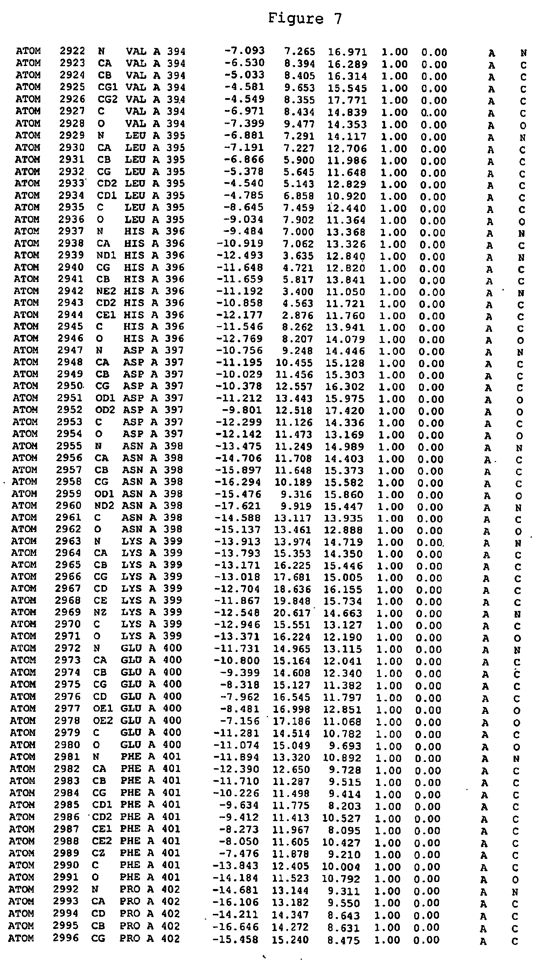

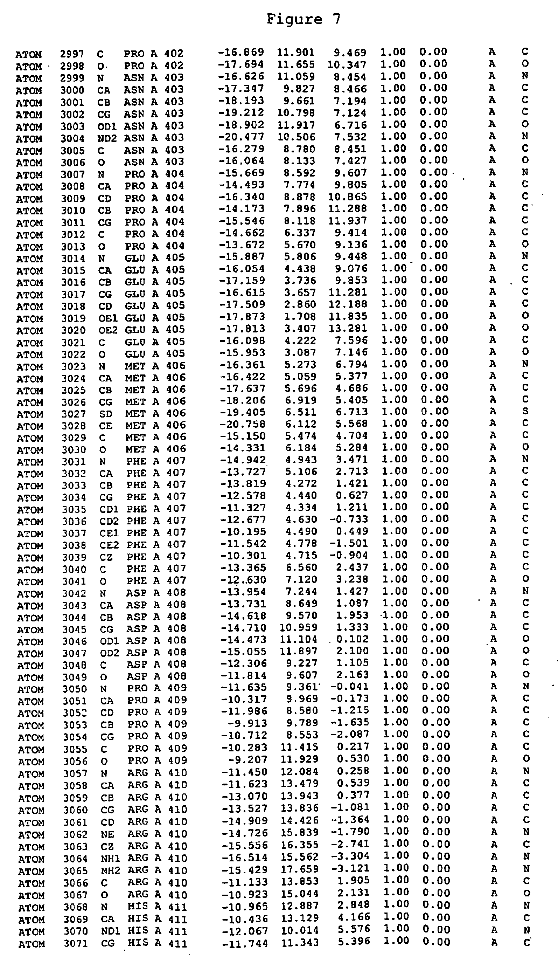

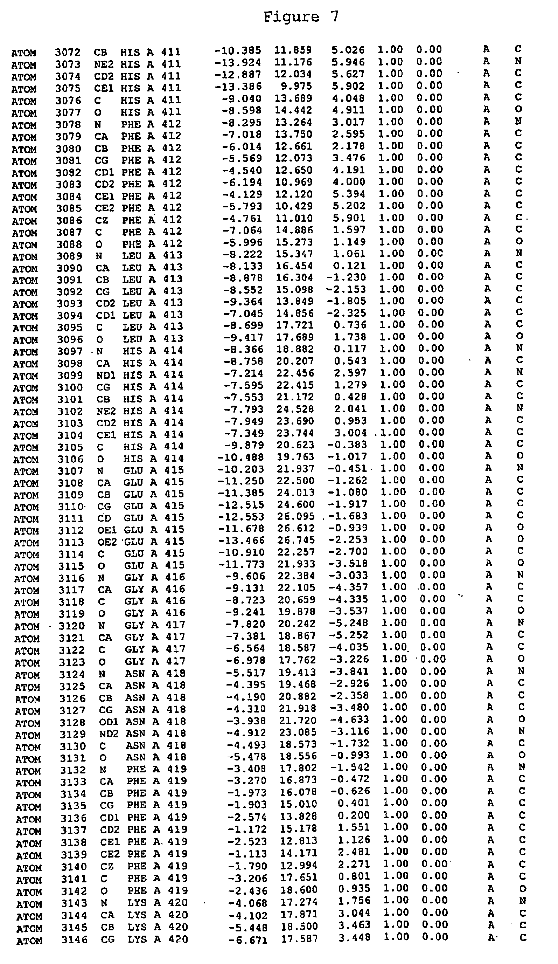

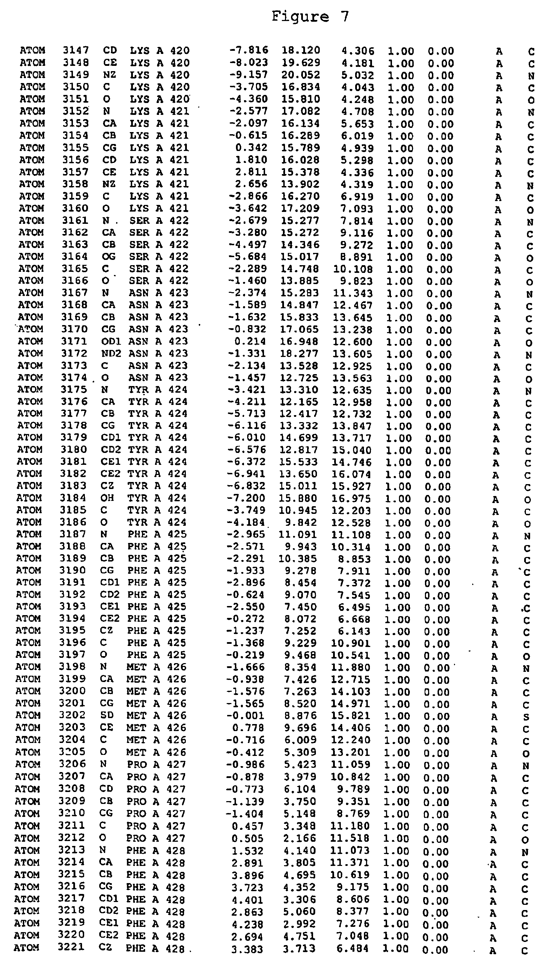

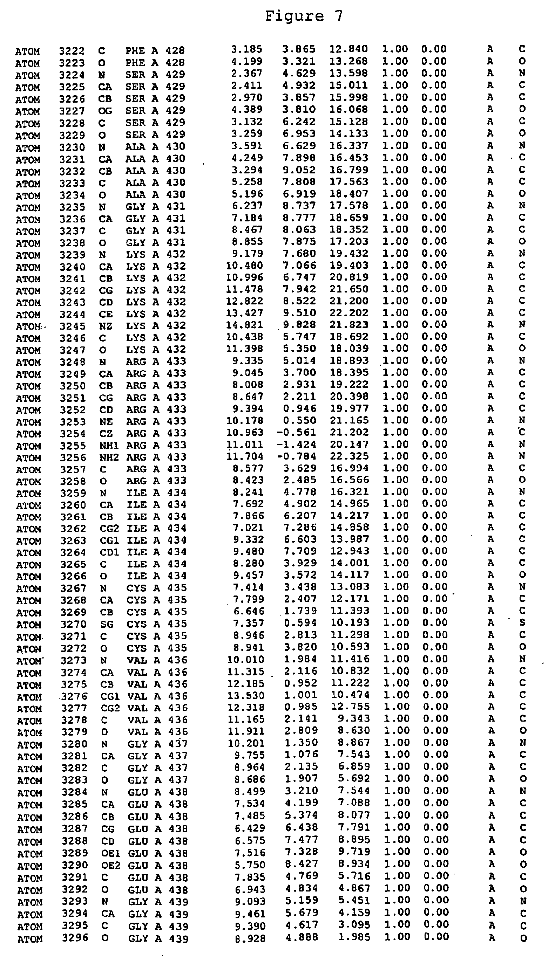

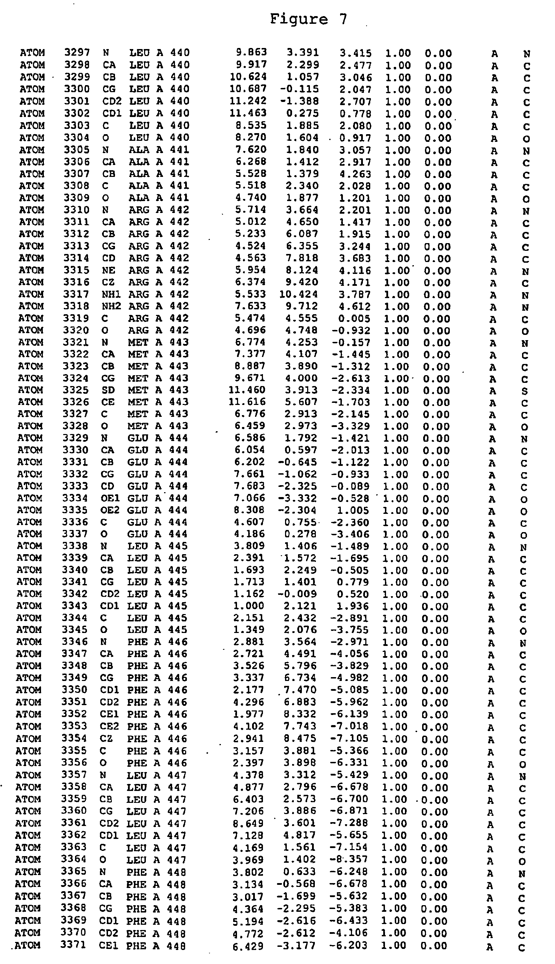

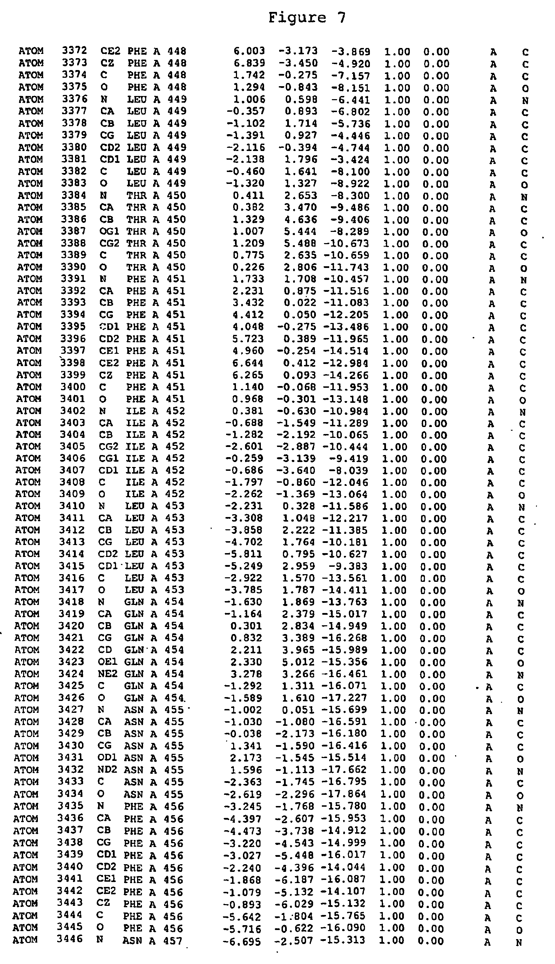

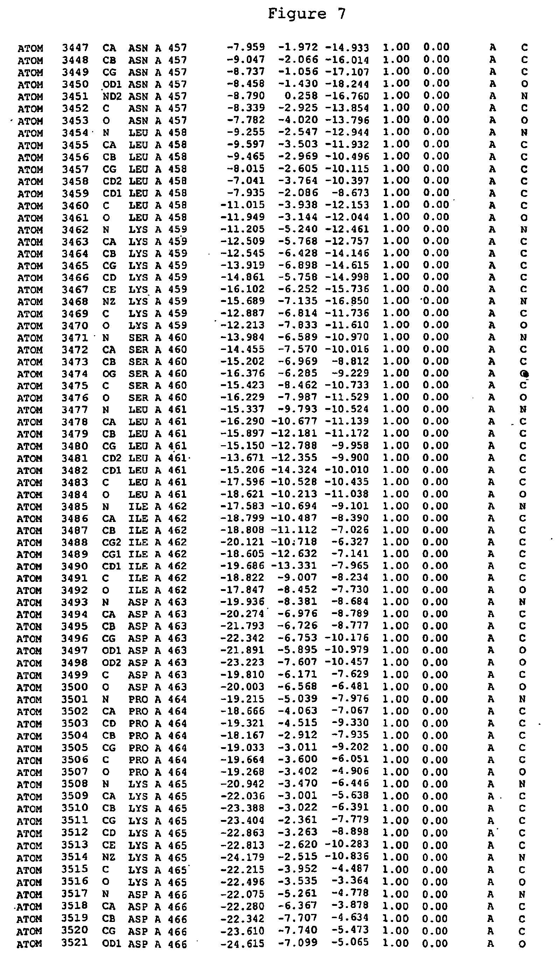

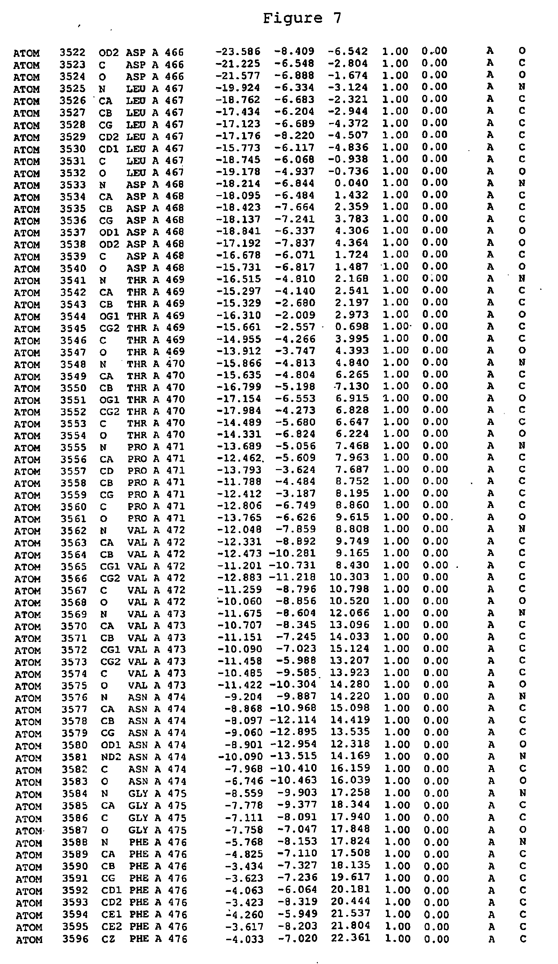

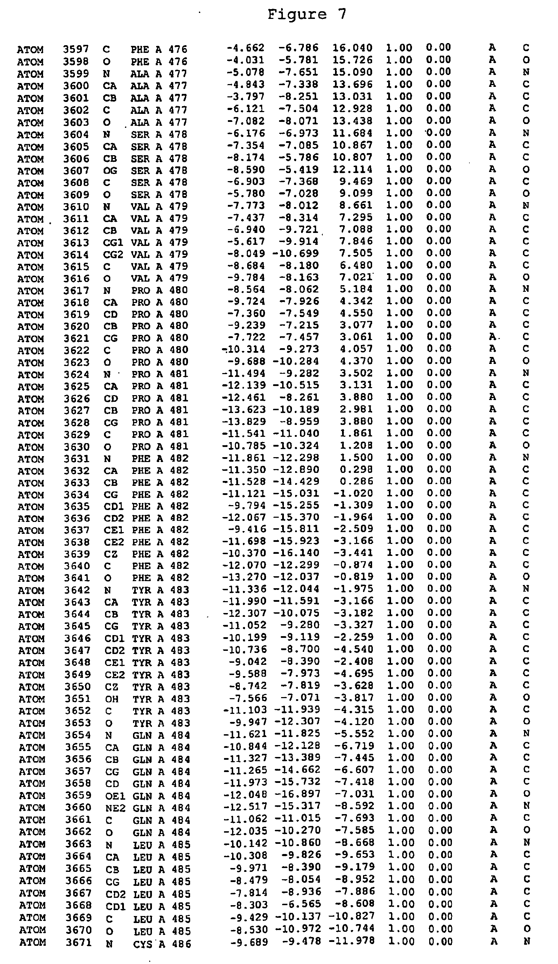

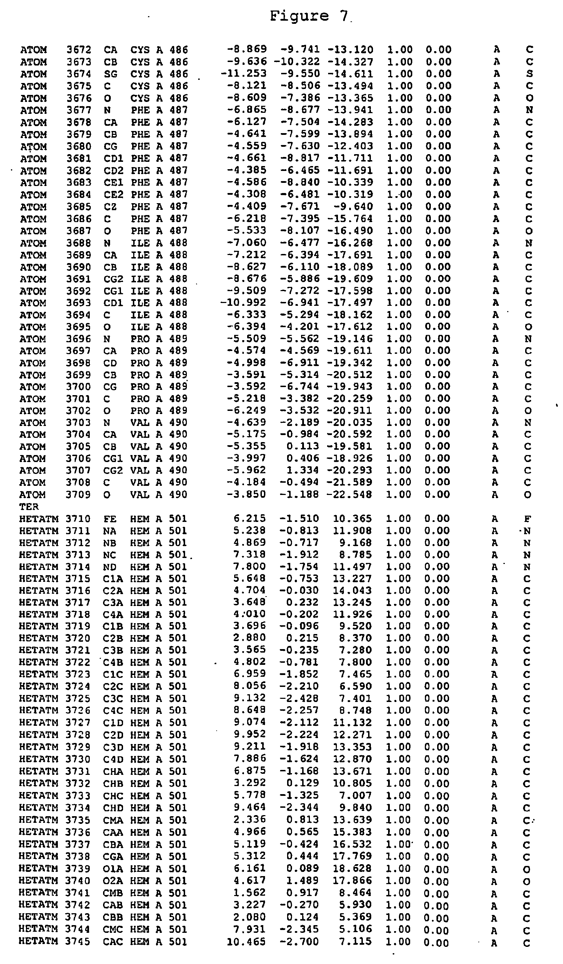

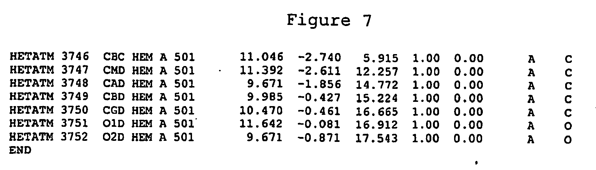

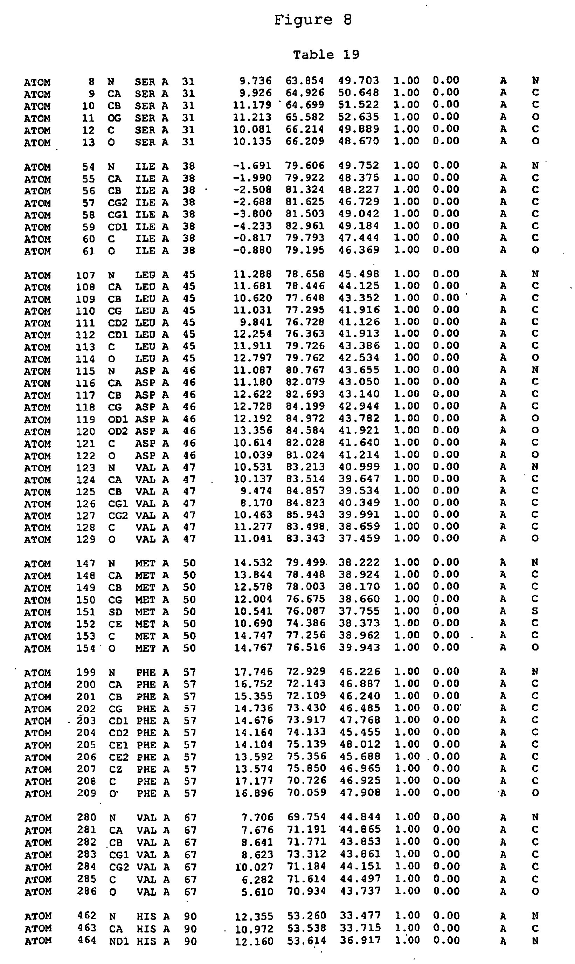

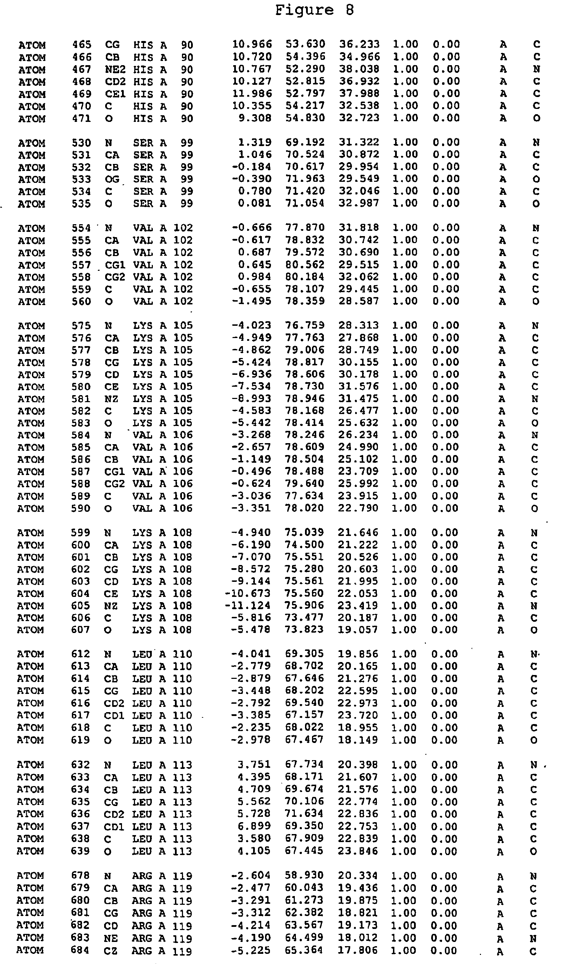

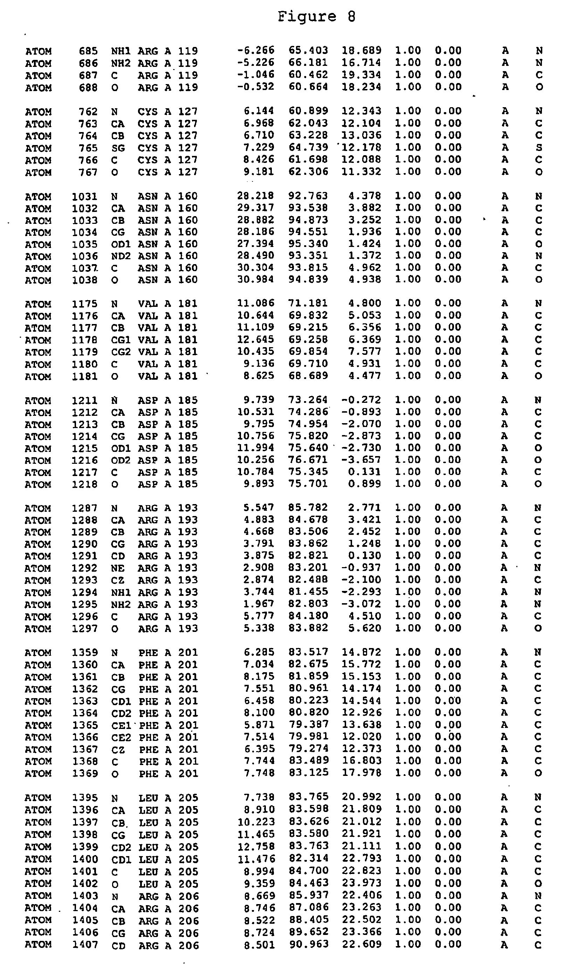

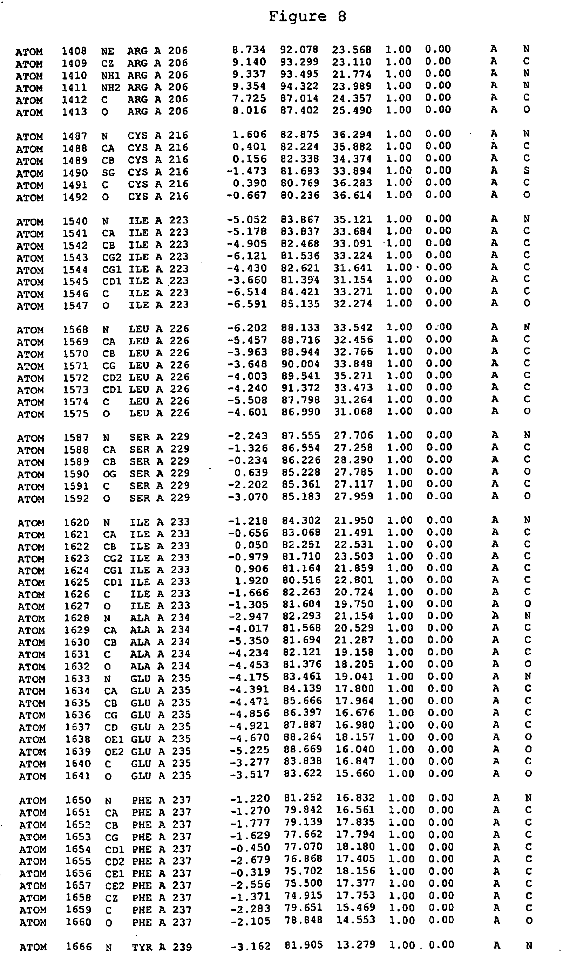

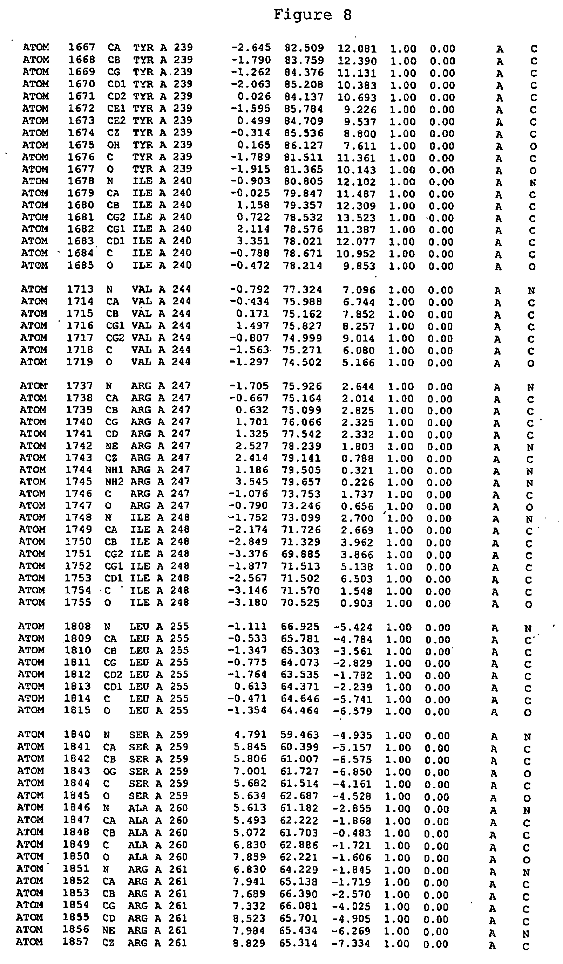

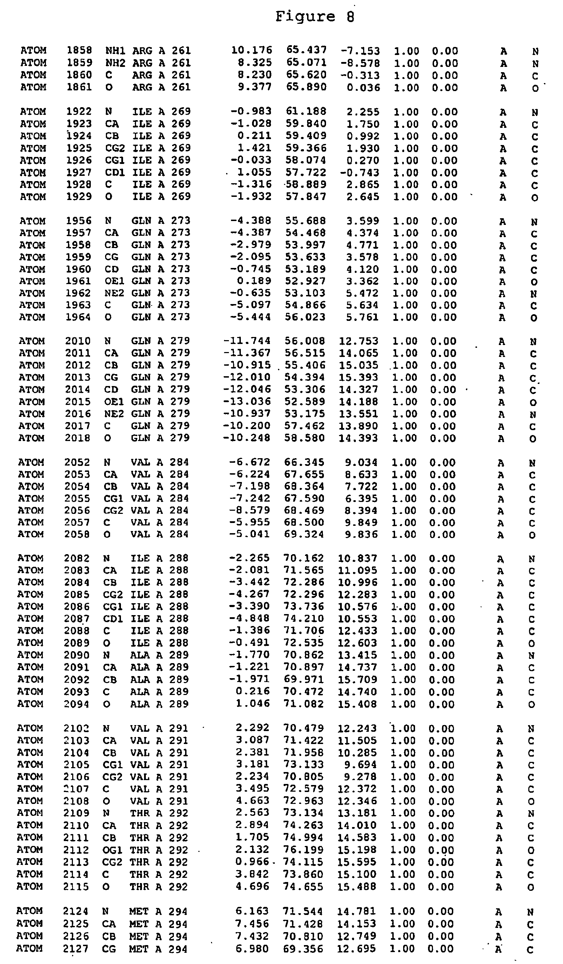

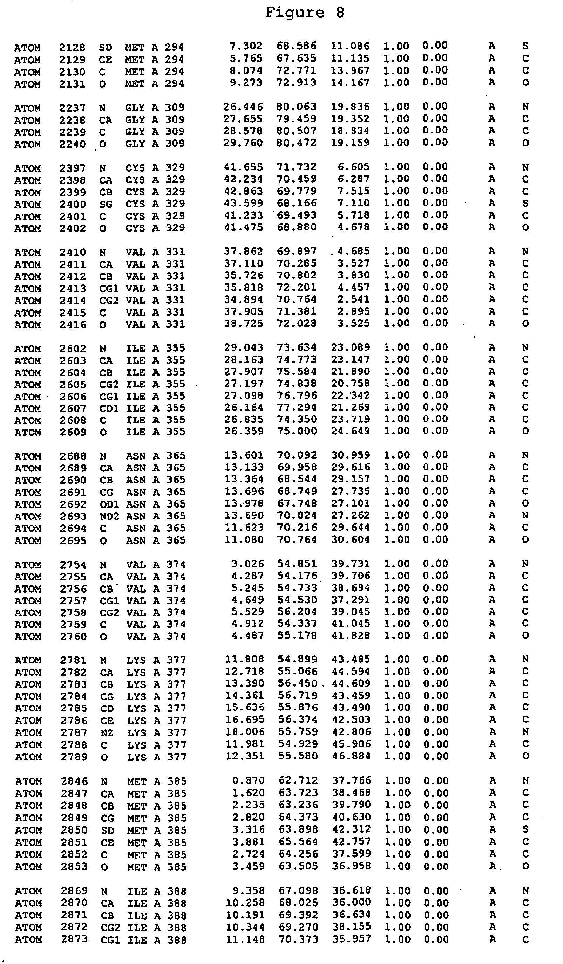

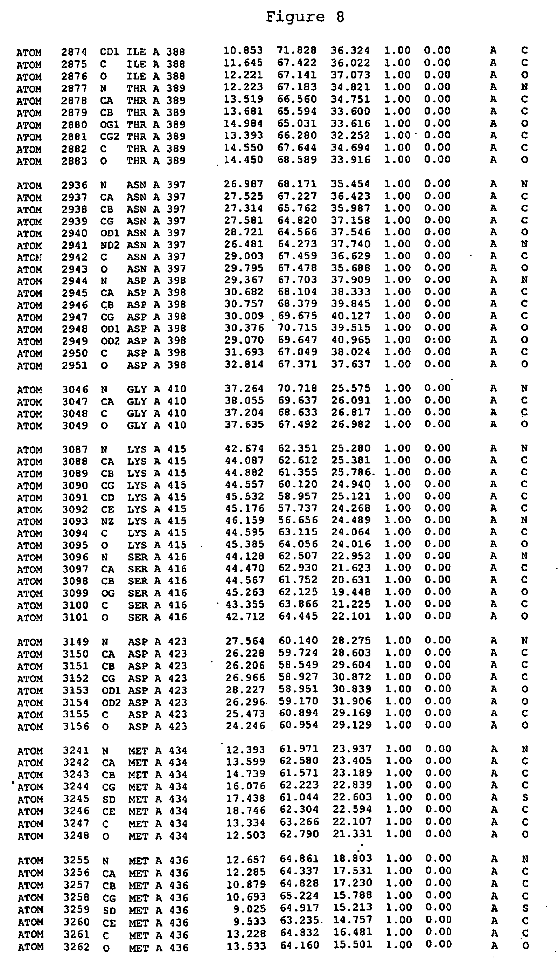

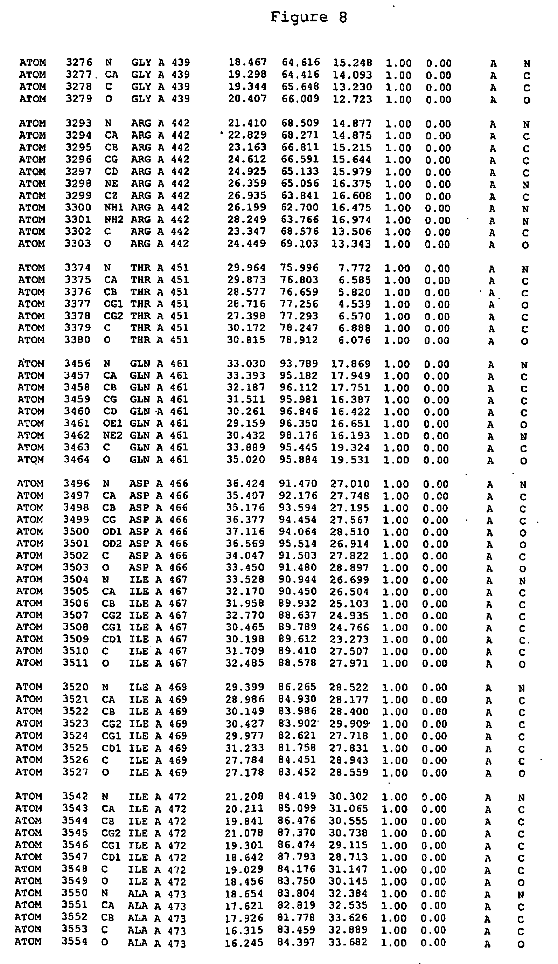

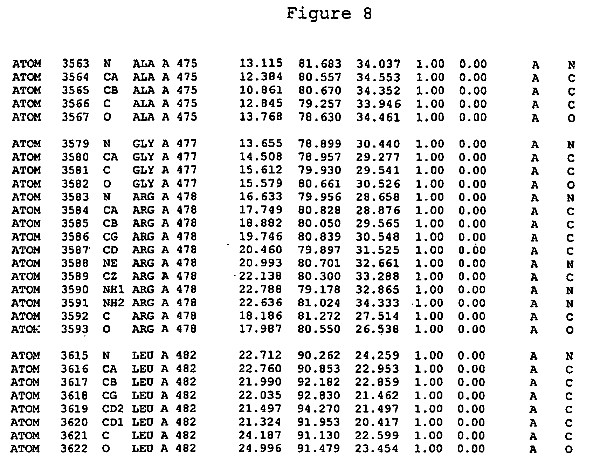

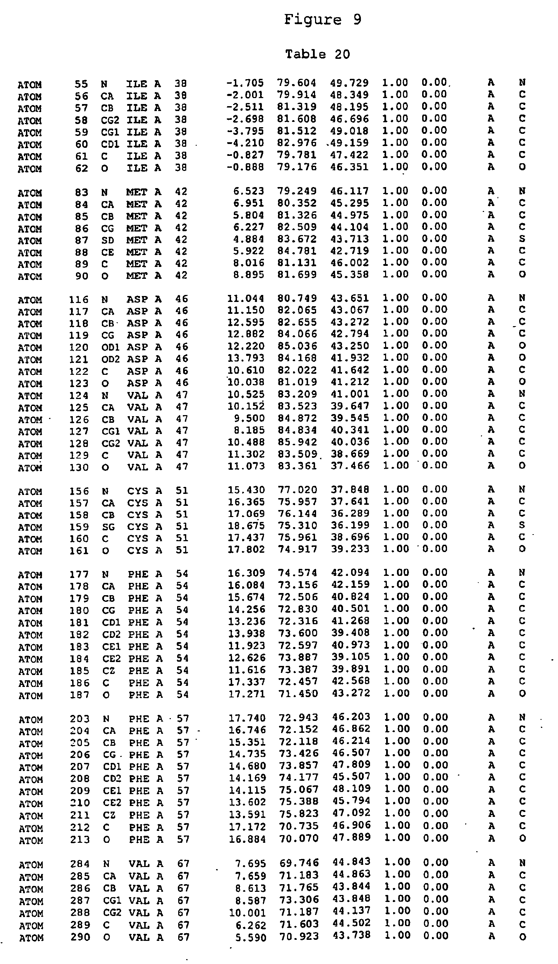

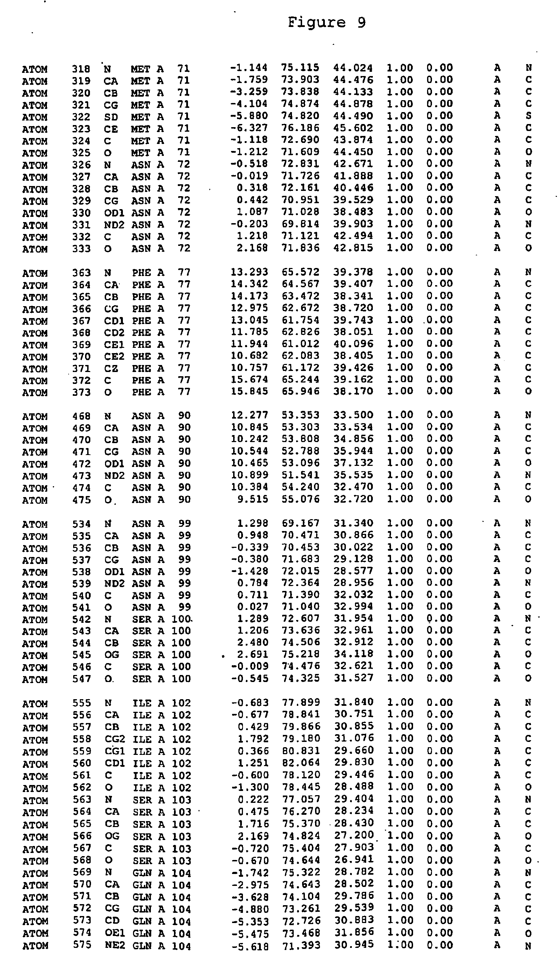

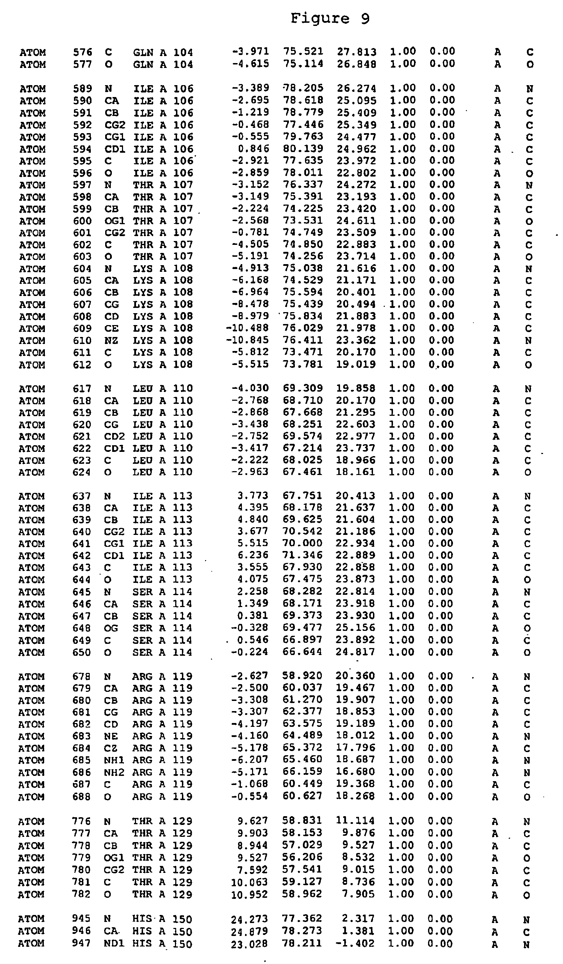

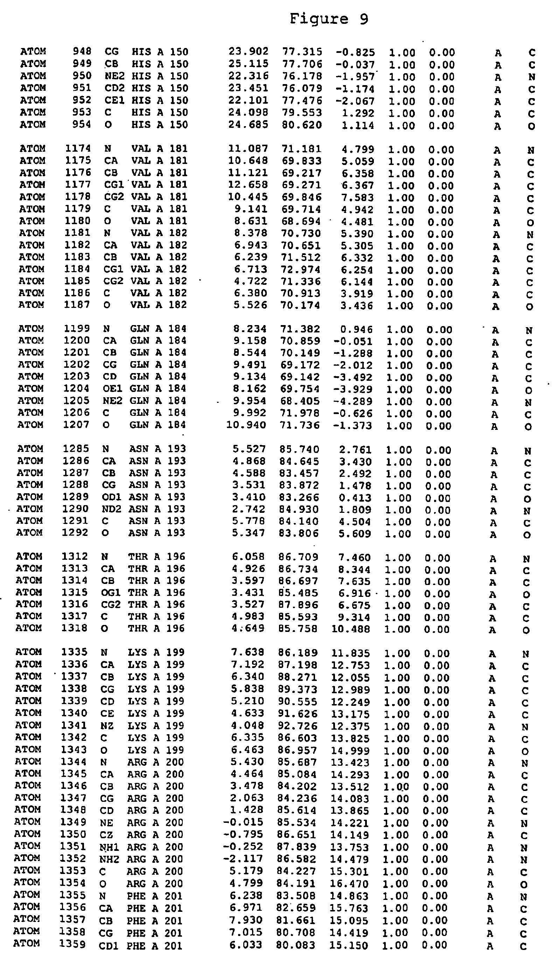

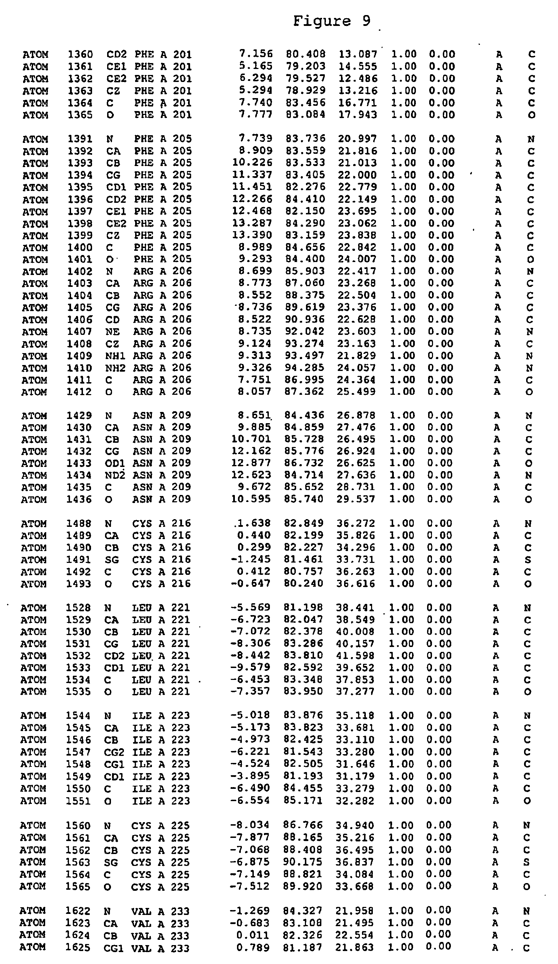

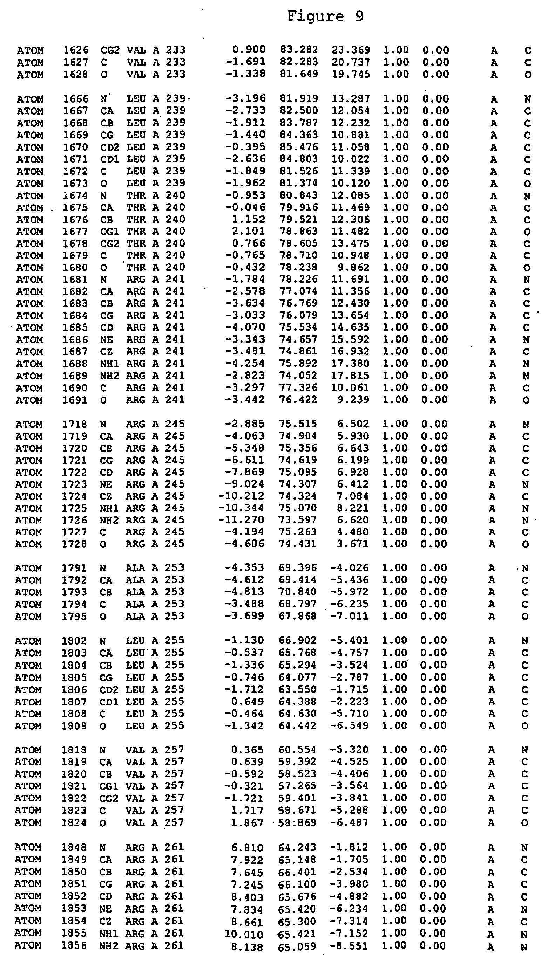

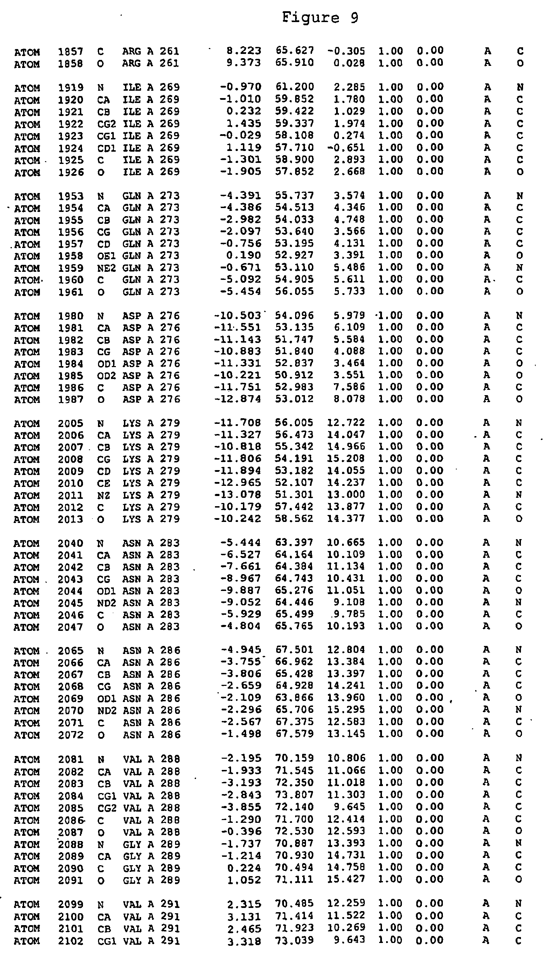

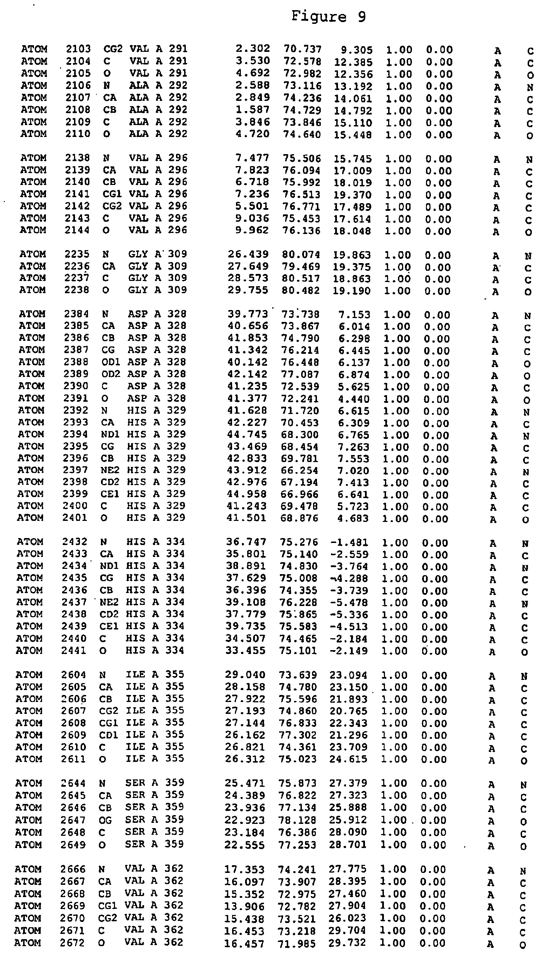

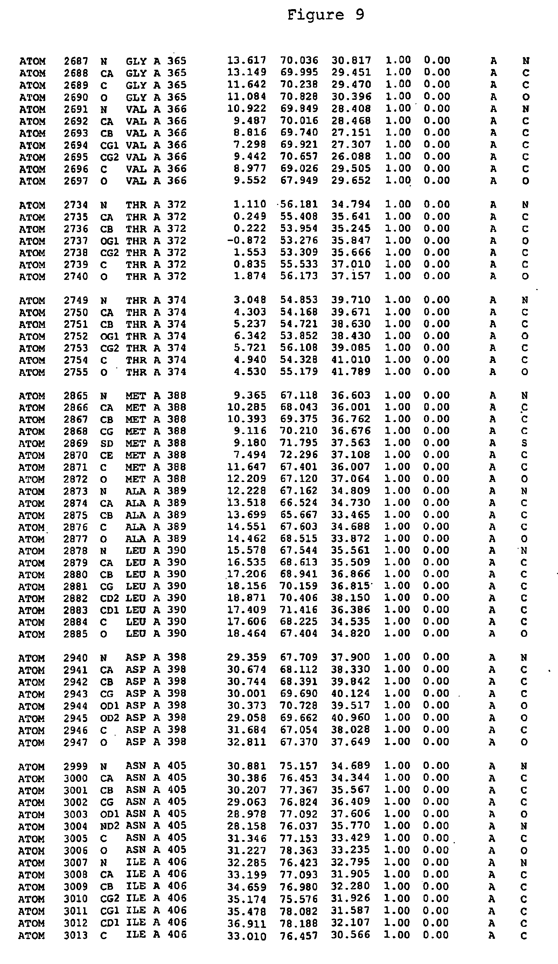

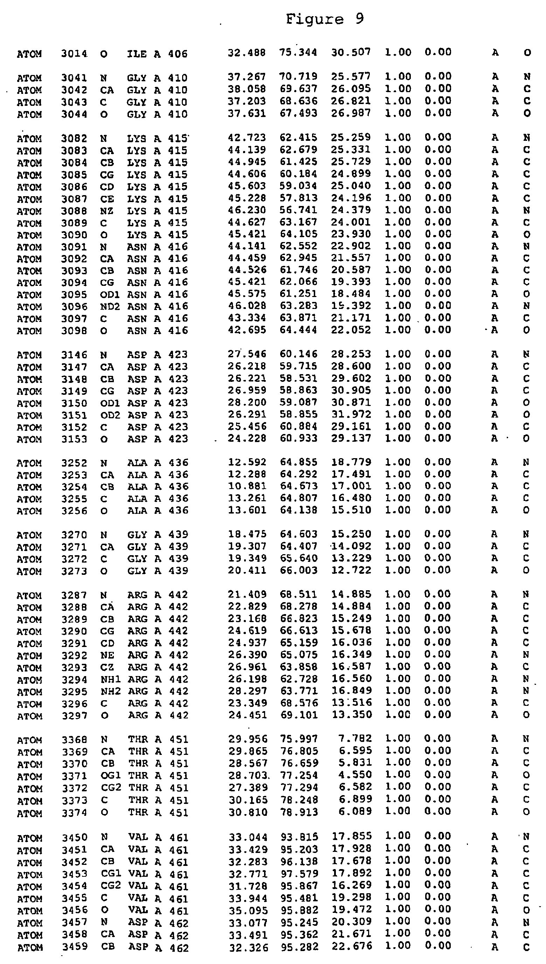

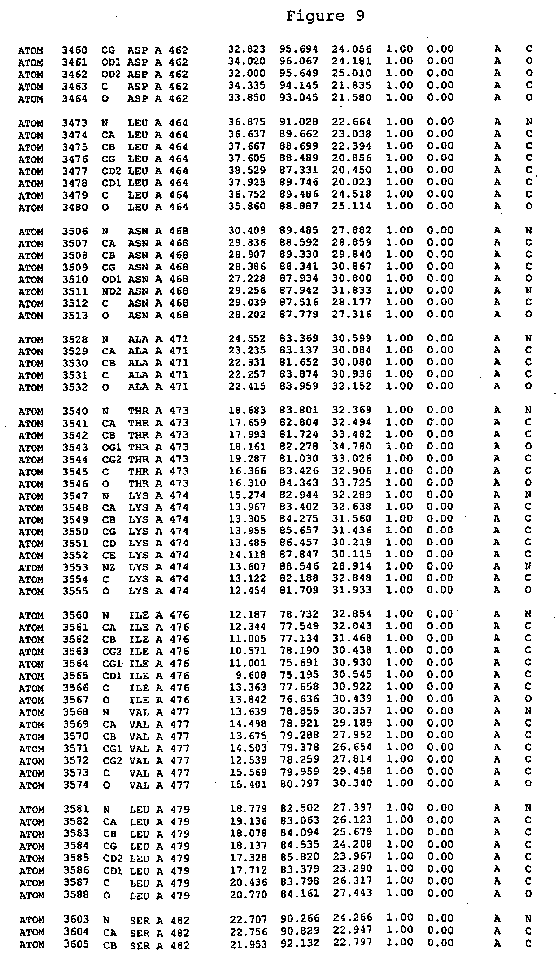

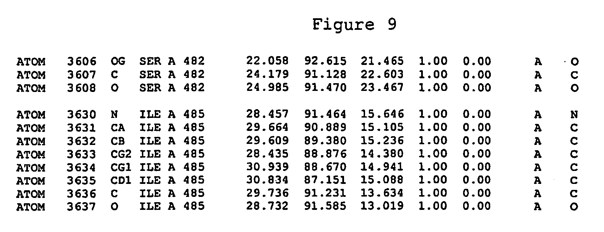

Table 1 (see Fig. 1) provides the coordinates of the 2C9 structure obtained in Example 9.
Table 2 (see Fig. 2) provides the coordinates of the 2C9-FGloop K206E structure obtained in Example 11.
Table 3 (see Fig. 3) provides the coordinates of 2C9-FGloop obtained in Example 12.
Table 4 (Example 13) lists residues that line the binding site of 2C9.
Table 5 (Example 13) lists residues previously inferred to be in the binding site.
Table 6 (Example 13) lists newly identified binding pocket residues.
Table 7 (see Fig. 4) provides modelled coordinates of residues 215, 216, 220, 221, 222, and 223 of a 2C9 wild type protein.
Table 8 (Example 16 and Figure 5) is a refined 2C9-FGloop K206E structure.
Table 9 (Example 17) describes further 2C9 proteins and the primers and methods used to obtain them.
Table 10 (Example 17) describes control 2C9 proteins and the primers and methods used to obtain them.
Table 11 (Examples 20 and 24, and Figure 6) shows crystallisation conditions for 2C9 proteins.
Table 12 (Example 18) sets out mass spectrometry data for 2C9 proteins.
Table 13 (Example 19) shows activity data for 2C9 proteins.
Table 14 (Example 21) shows 2C9-2C19 chimeras and the primers and/or methods used to obtain them.
Table 15 (Example 22) sets out mass spectrometry data for 2C9-2C19 chimeric proteins.
Table 16 (Example 23) shows activity of 2C9-FGtoop K206E (1155).
Table 17 (Example 24) shows activity of 2C9-2C 19 chimeras.
Table 18 (Example 25 and Figure 7) sets out a homology model of 2C19.
Table 19 (Example 26 and Figure 8) shows 2C 18 replacement coordinates.
Table 20 (Example 27 and Figure 9) shows 2C8 replacement coordinates.

### Description of sequences.

SEQ ID NO:1, is DNA sequence of 2C9trunc.
SEQ ID NO:2 is the amino acid sequence of 2C9trunc.
SEQ ID NO:3 is the DNA sequence of 2C9-P220 (also referred to as 1072).
SEQ ID NO:4 is the amino acid sequence of 2C9-P220.
SEQ ID NO:5 is the DNA sequence of 2C9-FGloop (also referred to as 1015).
SEQ ID NO:6 is the amino acid sequence of 2C9-FGloop.
SEQ ID NO:7 is the DNA sequence of 2C9-FGloop K206E (also referred to as 1155).
SEQ ID NO:8 is the amino acid sequence of 2C9-FGloop K206E.
SEQ ID NOs:(2x+7) and (2x+8) where x is an integer from 1 to 49 are the DNA and amino acid sequences, respectively, of the 2C9 proteins referred to as clones 1078, 1081, 1082, 1085, 1097, 1100, 1001, 1002, 1115, 1116, 1117, 1118, 1121, 1122, 1123, 1165, 1220, 1319, 1339, 1340, 1361, 1362, 1363, 1364, 1366, 1367, 1368,-1369, 1370, 1371, 1372, 1391, 1392, 1394, 1396, 1397, 1424, 1443, 1444, 1475, 1477, 1491, 1595, 1600, 1610, 1632, 1661, 1662 and 1664 respectively. Thus the DNA sequence encoding clone 1078 is SEQ ID NO:9 and its corresponding amino acid sequence is SEQ ID NO:10, and for clone 1664 the DNA is SEQ ID NO:105 and the corresponding amino acid sequence is SEQ ID NO:106.
SEQ ID NO:107 is the DNA sequence of clone 1039 (control clone).
SEQ ID NO:108 is the amino acid sequence of clone 1039.
SEQ ID NO:109 is the DNA sequence of clone 1365 (control clone).
SEQ ID NO:110 is the amino acid sequence of clone 1365.
SEQ ID NO:111 is the DNA sequence of clone 1423 (control clone).
SEQ ID NO:112 is the amino acid sequence of clone 1423.
Further sequences are identified in the accompanying text.

### Detailed Description of the Invention.

### A. 2C9 Proteins and their Production.

The sequence of 2C9 is available in the art, for example from a number of database sources cited in Nelson et al, 1996, *ibid.* This includes the SwissProt database, in which 2C9 is entry number P11712.

The 2C9 P450 protein is desirably truncated in its N-terminal region to delete the hydrophobic trans-membrane domain, and the region replaced by a short (e.g. 8 to 12 amino acid sequence containing one or more (e.g. 3, 4 or 5) positively charged amino acids. For expression of the human 2C9 P450, we have used an N-terminal sequence MAKKTSSKGR (SEQ ID NO:114) in place of the N-terminal 29 amino acid residues, which increases expression of the proteins in *E. coli.* and increases solubility.

The 2C9 P450 may optionally comprise a tag, such as a C-terminal polyhistidine tag to allow for recovery and purification of the protein.

We have found that the position of the proline residue in the F-G loop appears to play a significant role in the formation of a P450 crystal. In particular, the presence of a proline at position 220 or 222 in 2C9 appears to be important for crystallisation to occur.

In 2C9 wild type there is a proline residue at position 221. Moving it to position 220, by substituting position 220 by proline and removing the Pro221 (by substitution by any other residue, but preferably alanine or threonine) in 2C9 promotes crystallisation. Alternatively the proline may be moved to position 222, with position 221 likewise being substituted.

In 2C9 we have made the changes to positions 220 and 221 with and without other changes. Where other changes were made, these were I215V, C216Y, I222L and I223L, although it is not essential that any or all of these be made to provide for crystallisation.

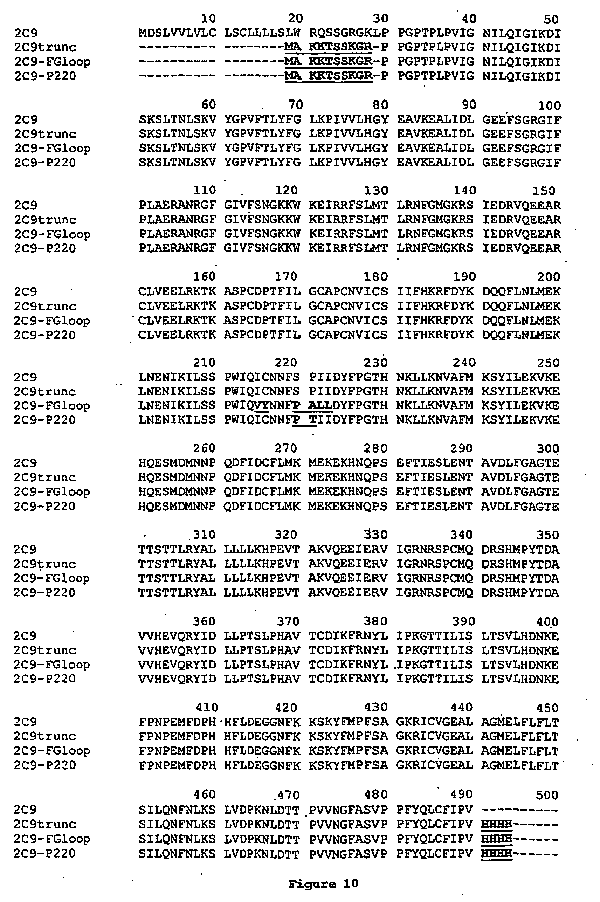

Our experiments have been based on the use of a particular N-terminal truncation of 2C9, as set out in SEQ ID NO:2 and shown in Figure 10. This protein also comprises a polyhistidine tag at the C-terminus. The N-terminal truncation and tag are both features which can be varied by those of skill in the art using routine skill. For example, alternative N-terminal sequence might be utilised, for example for production in host cells other than *E. coli.* Likewise, other tags may be used for purification of the protein as described below. These N- and C-terminal modification may be made to a 2C9 protein which retains the core sequence of residues 31-490 of the wild type sequence illustrated in Figure 10.

Described herein is a P450 2C9 protein which comprises the following changes:
position 220 or position 222 is proline; and
optionally up to 30, for example up to 25, for example up to 10, for example up to 5 other positions are altered,
the positions 220 and 222 being numbered according to wild type 2C9. This numbering is shown in Figure 10.

Preferably the change is to position 220.

It will be appreciated from the discussion above that by 2C9 protein, it is meant a protein comprising residues 31 to 490 of the wild type sequence, optionally with N- and/or C-terminal sequences provided to facilitate expression and recovery of the protein.

Where present, the N-terminal sequence is preferably not the wild-type sequence. Preferably, it is shorter that the wild type sequence (which is 30 amino acids). Preferably, the N-terminal region joined to residue 31 is the truncation illustrated in the accompanying examples, i.e. SEQ ID NO:114 plus a proline residue between it and residue 31 (also proline). This type of N-terminal sequence reduces the tendency of 2C9 to anchor to membranes and to aggregate compared to the wild type sequence.

Where present, the C-terminal sequence is preferably no larger than 30, and preferably no larger than 10 amino acids in size.

In a preferred aspect, one of the up to 30 changes is to the position 221, such that it is not proline. However this is not essential as it is shown herein (clone 1078) that crystals can be obtained with proline at position 221 as long as one of the changes made above is also included.

A particular advantage of the above proteins is that they are crystallisable. That is, we have found that we have been able to form crystals which diffract X-rays, and thus we have been able to analyse these crystals to provide structural coordinate data at a resolution of 3.1Å or better.

A further advantageous feature of some of the 2C9 proteins described herein is that we have been able to obtain crystals of a P450 protein in the absence of a ligand. Such crystals are useful for screening ligands with a view towards determining co-complex structures. Determining the molecular structure of 2C9 can also be used in computer-based in silico ligand screening.

We have also shown additional changes to the 2C9 wild type sequence in addition to the changes at any of 220-222 may be introduced. A number of specific changes are illustrated in the clones of 2C9 set out in the accompanying examples: These include:
- changes to the FG loop region. A number of clones have such changes, including the clone 2C9-FGloop (3 changes), clone 1363 (3 changes), clones 1361, 1362, 1364 1369 (2 changes), and clones 1366, 1371 (1 change).
- changes to the surface region of 2C9. Such changes are illustrated herein by clone 1123 (3 changes), clones 1102, 1340, 1397, 1443 (2 changes), and clones 1081, 1082, 1085, 1097, 1100, 1101, 1115, 1116, 1117, 1118, 1121, 1122, 1165, 1339, 1391, 1392, 1394, 1396 (1 change). These surface changes may be in addition to the FG loop changes.
- up to 20 changes in total on top of changes to positions 220 and 221; e.g. clone 1595 (20 changes), 1600 (13), and 1632 (11).

Thus clone 1595 has 22 changes form wild type in total- 6 in FG loop (including 220, 221), 3 in active.site, 12 on the surface. Of these 9 are conserved changes and 13 are non-conserved.

Our data illustrate that a variety of other positions in addition to the specific 220 or 222 changes may made while still providing a protein which can be crystallised.

In one aspect, the changes which may be introduced are changes which introduce residues found in the corresponding position in another cytochrome P450 molecule. The corresponding position may be found by alignment of the other P450 molecule with the sequence of 2C9 wild type to maximise homology between the two. The changes may be particularly from another cytochrome P450 molecule selected from the group consisting of 2C19, 2C18 and 2C8. Example 21 below sets out the production of proteins in which residues from 2C19 are substituted into the 2C9 sequence.

Examples 25 to 27 illustrate homology modelling of the proteins 2C19, 2C18 and 2C8 respectively. The Tables accompanying these examples may be used to identify the residues of these proteins which may be substituted into 2C9.

In another aspect, described herein is a protein which is selected from the group consisting of SEQ ID NO:(2x+2), wherein x is an integer from I to 52. These proteins all share the common feature of the introduction of a proline residue at position 220 or 222 which facilitates crystallisation of 2C9.

### Expression and Recovery of P450

The 2C9 P450 proteins are produced by recombinant DNA techniques. The nucleic acid sequences which encode wild type P450 proteins are available in the art, and the person of skill in the art may use routine methodology, e.g. site-directed mutagenesis, to introduce coding changes into the nucleic acid sequences so as to provide nucleic acids encoding the P450s of the invention.

Thus in another aspect, described herein is an isolated nucleic acid encoding a 2C9 P450 protein of the invention. Nucleic acid includes DNA (including both genomic and cDNA) and RNA. Nucleic acid of the invention may be single or double stranded polynucleotides.

Such nucleic acids can be incorporated into a recombinant replicable vector. The vector may be used to replicate the nucleic acid in a compatible host cell. Thus in a further embodiment, a method of making nucleic acids by introducing a nucleic acid into a replicable vector, introducing the vector into a compatible host cell, and growing the host cell under conditions which bring about replication of the vector is described. The vector may be recovered from the host cell.

Preferably, a nucleic acid is in a vector is operably linked to a control sequence which is capable of providing for the expression of the coding sequence by the host cell, i.e. the vector is an expression vector.

The term "operably linked" refers to a juxtaposition wherein the components described are in a relationship permitting them to function in their intended manner. A control sequence "operably linked" to a coding sequence is ligated in such a way that expression of the coding sequence is achieved under condition compatible with the control sequences.

Suitable vectors can be chosen or constructed, containing appropriate regulatory sequences, including promoter sequences, terminator fragments, polyadehylation sequences, enhancer sequences, marker genes and other sequences as appropriate. Vectors may be plasmids, viral e.g. phage phagemid or baculoviral, cosmids, YACs, BACs, or PACs as appropriate.

The vectors may be provided with an origin of replication, optionally a promoter for the expression of the said polynucleotide and optionally a regulator of the promoter. The vectors may contain one or more selectable marker genes, for example an ampicillin resistance gene in the case of a bacterial plasmid or a neomycin resistance gene for a mammalian vector. Vectors may be used *in vitro,* for example for the production of RNA or used to transfect or transform a host cell. Systems for cloning and expression of a polypeptide in a variety of different host cells are well known.

Promoters and other expression regulation signals may be selected to be compatible with the host cell for which the expression vector is designed. For example, bacterial promoters include the lacZ promoter, yeast promoters include S. cerevisiae GAL4 and ADH promoters, S. pombe nmtl and adh promoter, and mammalian promoters include the metallothionein promoter, the SV40 large T antigen promoter or adenovirus promoters.

For further details see, for example, Molecular Cloning: a Laboratory Manual: 3rd edition, Sambrook et al., 2001, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Current Protocols in Molecular Biology, Ausubel et al. eds., John Wiley & Sons, 1992.

A further embodiment described herein provides host cells transformed or transfected with the vectors for the replication and expression of 2C9-encoding nucleic acids. The cells will be chosen to be compatible with the said vector and may for example be bacterial, yeast, insect or mammalian.

The 2C9 P450 proteins may be expressed in any suitable host cell, which a person of skill in the art wishes to use as a matter of experimental convenience. Cytochrome P450 molecules have been widely expressed in *E. coli*, and there are numerous vector systems for this host cell which may be used.

Other host cells include yeast, e.g. *S. cerevisiae,* insect or mammalian, e.g. CHO, cells. Expression systems for these and many other host cell types are widely available in the art:

Host cells may be constructed so that the 2C9 P450 is expressed constitutively, or is induced.

Once the cells have been cultured to express 2C9 P450, they may be recovered by standard techniques available in the art. A convenient means is to recover the cells by low-speed centrifugation such that the cells are pelleted intact.

The process of the present invention is suitable for batch cell culture, and batches of cells from 100 ml to several, e.g. 10 litres can be conveniently handled by current laboratory equipment, though larger batches, e.g. 10 to 100 litres, are not excluded.

Also described herein is a method for expression and recovery of the herein disclosed 2C9 human cytochrome P450s from host cells. This method comprises:
(a) expressing in a host cell culture said cytochrome 2C9 P450 molecule;
(b) recovering said cells from said culture and suspending said cells in salt buffer having a conductivity of from 12 to 110 mS/cm;
(c) lysing said cells and removing cell debris to provide a high-salt lysate;
(d) adding detergent to said lysate (for example 0.015% to 1.2% v/v) to provide a high-salt-detergent lysate;
(e) recovering said P450 from said lysate.

In a preferred embodiment, the method comprises:
(a) expressing in *E. coli* said cytochrome 2C9 P450 molecule;
(b) recovering said cells and suspending them in a 200 mM to 1000 mM salt buffer;
(c) lysing said cells and removing cell debris to provide a high-salt lysate;
(d) adding detergent to said lysate (for example 0.015% to 1.2% v/v) to provide a high-salt-detergent lysate;
(e) recovering said P450 from said lysate.

The recovery step involves affinity purification of the 2C9 P450 from the high salt-detergent lysate, since the presence of the high salt rules out the alternative of an ionic exchange purification step.

However, once the P450 has been purified by affinity chromatography, the salt must be removed in order to allow further purification of the product so that crystallization can be performed. In the prior art, salt removal is typically performed by dialysis. However, we have found that this process, which removes salt gradually over a period of several hours, causes aggregation and denaturation of the P450s and thus is undesirable. We have found that rapid desalting alleviates this problem to a significant degree.

Thus in a further aspect, step (e) above may be performed by:
(e(i)) binding said 2C9 P450 to an affinity support;
(e(ii)) rinsing said support in a high-salt-detergent wash;
(e(iii)) removing said 2C9 P450 in a high-salt-detergent buffer to provide a P450-high-salt-detergent preparation; and
(f) exchanging the buffer to a low ionic strength buffer without detergent by size-exclusion chromatography to provide a P450-low-salt preparation.

The above steps e(i)-(iii) maintain the 2C9 P450 in a high-salt and detergent buffer throughout the initial stages of the purification process, which aids the recovery of the P450.

The preparation may be subject to additional purification and cleaning procedures, such as cation exchange chromatography, optionally followed by further size-exclusion chromatography or hydrophobic interaction chromatography to obtain a more purified preparation of protein.

### Salt buffer

This is buffer with a high ionic strength which is used to suspend the cells. It is a buffer comprising a salt which is readily soluble to provide a buffer having a conductivity of from 12 to 110 mS/cm. Such a buffer is desirably a salt having a concentration in the 200 -1000 mM range. Preferably the salt is a potassium or sodium salt of an anion. Desirably the anion may be chloride or phosphate. Potassium phosphate (KPi) is particularly preferred.

A preferred salt concentration is selected to provide a conductivity of 25 to 35 mS/cm, for example about 30 mS/cm. A particularly preferred salt concentration is around 500 mM, e.g. 500 + 50 mM.

The buffer will be maintained at a pH range of from 6.5 to 8.0, preferably from 7.0 to 7.6. The buffer may contain other reagents used conventionally in the art for protein purification, such as glycerol, β-mercaptoethanol, DNase, pH buffering agents, histidine, imidazole and protease inhibitors.

### Cell lysis

Cells may be lysed by physical means, such as sonication or in a French press or continuous flow cell disruptor, such that the cell walls are broken and the contents of the cells dispersed in the salt buffer. To achieve this in a French press, this may be operated at 10,000 to 20,000 psi.

Cell debris is removed (for example by low-speed centrifugation at about 10,000 -25,000 g (e.g. about 22,000 g or a short high speed centrifugation to 70000 g; i.e. such that any whole cells are pelleted but not the membrane fraction). The debris (e.g. pelleted cells) may be subject to a further round of lysis, and the debris-free lysate from this further round combined with the lysate obtained previously.

The lysate is then ready to use directly in the next stage of the process, without the need to isolate a membrane fraction by ultracentrifugation.

### Use of detergent

Once the lysate has been obtained, it is desirable that the detergent be added to the lysate as soon as possible, taking account of the constraints of the experimental set up. This will mean that the detergent is added to the lysate within 1 hour, preferably within 30 minutes or less of the preparation of the debris-free lysate.

The detergents that may be used are those conventionally used in the art of molecular and cell biology for the recovery and processing of biological materials. A large number of different types of detergents are available for this purpose. Many of these detergents are those of a molecular weightrange of from about 350 to 1000, such as from 400 to 800. They include anionic surfactants such as cholic acid or salts thereof (e.g. the sodium salt) and deoxycholic acid or salts thereof (e.g. the sodium salt) as well as zwitterionic surfactants such as CHAPS (3-[(3-cholamidopropyl)dimethylammonio]-1-propane sulphonate).

A particularly preferred class of detergents are non-ionic detergents. There are a wide variety of non-ionic detergents available in the art. Non-ionic detergents include octyl-β-D-glucopyranoside and ethers, such as C2-10 alkylphenol ethers, of polylethylene glycol. Such compounds may be of a molecular weight range of 500 - 800 Da, and include Nonident^{TM} P40, IGEPAL CA630, and Triton^{™} X-100, and the like, which are commercially available.

The detergent is added to provide a usual concentration of from 0.015% to 1.2% v/v of detergent in the lysate. The amount of detergent added is preferably in the range of 0.1 to 1.2%, more preferably about 0.2 to 0.4%, such as about 0.3%.

The detergent is added in a volume so that desirably, the concentration of salt or ionic strength does not decrease by more than 10%.

### Recovery of Purified 2C9 P450

We have found that the above high-salt-detergent lysate prepared in accordance with the invention provides for the recovery of 2C9 P450 protein in reduced aggregation form at a level much higher than experienced to date in the art. As mentioned above, the presence of the high salt buffer rules out an immediate ionic exchange chromatography step, but affinity purification may be performed as the next step.

Affinity purification may take the form of providing an affinity support matrix in which a ligand for the 2C9 P450 is attached. The support may be a resin, a bead (e.g. glass or polymer such as polystyrene), a magnetic bead, or the like. Where the 2C9 P450 contains a tag, the ligand will be cognate to the tag, e.g. Ni-NTA for a histidine tag, biotin for a streptavidin tag, etc. The ligand may also be an antibody, either to an epitope tag such as an HA tag, or to an epitope of the 2C9 P450.

The lysate is brought into contact with the affinity support under conditions for the 2C9 P450 to bind to the support. After binding, the support is rinsed. The rinse buffer should be a high-salt-detergent buffer, which may be the same or different to the lysate buffer. Preferably it is the same. If different, it will still have concentrations of salt and detergent as specified above.

After rinsing, the 2C9 P450 is removed from the support. This may be done in batch or by packing the support into a column and eluting the 2C9 P450 using a high-salt-detergent buffer, which is modified to remove the 2C9 P450 from its ligand. For example, for Ni-NTA, the buffer may contain histidine or imidazole at a sufficient excess concentration to displace the His tag of the 2C9 P450. Suitable competitors may be used for other types of tags.

### Desalting

The ionic strength of the resulting P450 solution is lowered by a rapid desalting process. We have found that a size exclusion column may be used for this purpose, with a flow rate such that the 2C9 P450 is separated from the high salt concentration within 10-30, preferably within 10 minutes. The 2C9 P450 is loaded to the column and eluted through the column with a low salt buffer.

While not wishing to be bound by any one particular theory, we have observed that whereas gradual desalting by, for example, dialysis, leads to aggregation and denaturation of cytochrome 2C9 P450, the rapid desalting process reduces aggregation to a significant degree.

The low salt buffer is preferably a similar salt to the high salt buffer described above, e.g. a sodium or potassium salt such as a chloride or phosphate, with potassium phosphate again being preferred. By "low salt", it is meant less than 50 mM, preferably less than 20 mM, and preferably about 10 mM. At this stage, it is not necessary to maintain detergent in the buffer.

### Further purification

It is desirable that after the desalting step, the preparation is subject to further purification promptly, i.e. without storage or freezing of the sample. This can be achieved by applying the desalted eluate directly to a further purification column. If not, the eluate from the desalting process is collected and applied within 1 hour to the column. A number of techniques are known as such in the art for the further purification or concentration of protein preparations, and examples of these are outlined in the accompanying examples. They include weak cation exchange columns, such as carboxymethyl-Sepharose^{™}, BioRex^{™}70, carboxymethyl-Biogel^{™}, and the like, and strong cation exchange columns such as MonoS, which may be used to further remove detergent. For example, the desalted cytochrome P450 may be directly applied to a CM Sepharose^{™} column (e.g. a 5 ml HiTrap column, Pharmacia), previously equilibrated with 10 mM KPi, pH 7.4, 20% glycerol, 0.2 - 2.0 mM DTT, I mM EDTA ("buffer 1"). The following step elution protocol may then be run on the AKTA FPLC system; wash with 10 - 20 column volumes of buffer 1 and then 10 - 20 column volumes of 10mM KPi, pH 7.4, 20% glycerol, 0.2 - 2.0 mM DTT, 1mM EDTA, 75 mM KCl or NaCl in order to remove any trace of detergent. The P450 is then eluted with the above latter buffer with KCl or NaCl concentration increased to 500 mM.

Optionally this is followed by a size exclusion column, e.g. Superose^{™}, Superdex^{™}, Sephacryl^{™}, and the like. The protein recovered from either the cation exchange or size exclusion step may be concentrated to provide a solution suitable for crystallisation or other use. A concentration of from 20 to 120, e.g. 20 to 80 mg/ml may be achieved by the use of the present invention.

### B. Crystallisation of 2C9 Proteins.

A number of methods are known as such in the art for obtaining protein crystals. Conveniently, the final protein is concentrated to 10-60, e.g. 20-40 mg/ml in 10-100 mM potassium phosphate with high salt (e.g. 500 mM NaCl or KCl) by using concentration devices that are commercially available. The protein may be concentrated in presence of 20% glycerol, 2.0 mM DTT and 1 mM EDTA.

The protein is crystallized by vapour diffusion at 5-25 °C against a range of buffer compositions. Crystals may be prepared using commercially available screening kits such as, Polyethylene glycol (PEG)/ion screens, PEG grid, Ammonium sulphate grid, PEG/ammonium sulphate grid or the like purchased from Hampton Research, Emerald Biostructure, Molecular Dimension and from others.

Typically the vapour diffusion buffer comprises 0 - 27.5%, preferably 2.5-27.5% PEG 1K-20 K, preferably 1-8K or PEG 2000MME-5000MME, preferably PEG 2000 MME, or 0-10% Jeffamine M-600 and/or 5-20%, e.g. 10-20% propanol or 15-20% ethanol or about 15%-30%, e.g. about 15% 2-methyl-2,4-pentanediol (MPD), optionally with 0.01 M -1.6 M salt or salts and/or 0-0.15, e.g. 0-0.1, M of a solution buffer and/or 0-35%, such as 0-15%, glycerol and/or 0-35% PEG300-400; but preferably:
10-25% PEG 1K-8K or PEG 2000MME or 0-10% Jeffamine M-600 and/or 5-15%, e.g. 10-15%, propanol or ethanol, optionally with 0.1 M -0.2 M salt or salts and/or 0-0.15, e.g. 0-0.1 M solution buffer and/or PEG400, but more preferably:
   15-20% PEG 3350 or PEG 4000 or PEG 2000MME or 0-10% Jeffamine M-600 or 5-15%, e.g. 10-15% propanol or ethanol, optionally with 0.1 M -0.2 M salt or salts and/or 0-0.15 M solution buffer.

Specifically preferred crystallisation conditions for the 2C9 proteins described herein are:
0.05-0.1 M Tris-HCl pH 8.0-8.8, 0.1-0.2 M Lithium sulphate, 10-1.5% PEG 4000;
0.1 M Tris pH 8.0-8.8, 15-30% PEG 400, 5% PEG 8000, 10% glycerol; and
0.1-0.4 M KH₂PO₄, 0-25 % PEG 3350, 0-10% glycerol.

The salt may be an alkali metal (particularly lithium, sodium and potassium), alkaline earth metal (e.g. magnesium or calcium), ammonium, ferric, ferrous or transition metal salt (e.g. zinc) of a halide (e.g. bromide, chloride or fluoride), acetate, formate, nitrate, sulphate, tartrate, citrate or phosphate. This includes sodium fluoride, potassium fluoride, ammonium fluoride, ammonium acetate, lithium acetate, magnesium acetate, sodium acetate, potassium acetate, calcium acetate, zinc acetate, ammonium chloride, lithium chloride, magnesium chloride, potassium chloride, sodium chloride, potassium bromide, magnesium formate, sodium formate, potassium formate, ammonium formate, ammonium nitrate, lithium nitrate, potassium nitrate, sodium nitrate, ammonium sulphate, potassium sulphate, lithium sulphate, sodium sulphate, di-sodium tartrate, potassium sodium tartrate, di-ammonium tartrate, potassium dihydrogen phosphate, tri-sodium citrate, tri-potassium citrate, zinc acetate, ferric chloride, calcium chloride, magnesium nitrate, magnesium sulphate, sodium dihydrogen phosphate, di-sodium hydrogen phosphate, di-potassium hydrogen phosphate, ammonium dihydrogen phosphate, di-ammonium hydrogen phosphate, tri-lithium citrate, nickel chloride, ammonium iodide, di-ammonium hydrogen citrate.

Solution buffers if present include, for example, Hepes, Tris, imidazole, cacodylate, tri-sodium citrate/citric acid, tri-sodium citrate/HCl, acetic acid/sodium acetate, phosphate-citrate, sodium potassium phosphate, 2-(N-morpholino)-ethane sulphonic acid/NaOH (MES), CHES, bis-trispropane, CAPS, potassium dihydrogen phosphate, sodium dihydrogen phosphate, dipotassium hydrogen phosphate or disodium hydrogen phosphate.

The pH range is desirably maintained at pH 4.2-10.5, preferably 4.2-8.5, more preferably 4.7-8.5 and most preferably 6.5-8.5.

Crystals may be prepared using a Hampton Research Screening kit, Poly-ethylene glycol (PEG)/ion screens, PEG grid, Ammonium sulphate grid, PEG/ammonium sulphate grid or the like.

Crystallisation may also be performed in the presence of an inhibitor or substrate of P450, e.g. fluoroxamine or 2-phenyl imidazole.

Additives can be added to a crystallisation condition identified to influence crystallisation. Additive Screens are to be used during the optimisation of preliminary crystallisation conditions where the presence of additives may assist in the crystallisation of the sample and the additives may improve the quality of the crystal e.g. Hampton additive Screens which use glycerol, polyols and other protein stabilizing agents in protein crystallisation (R. Sousa. Acta. Cryst. (1995) D51, 271-277) or divalent cations (Trakhanov, S. and Quiocho, F.A. Protein Science (1995) 4,9, 1914-1919).

### C. Crystals

In a further aspect, described herein is a crystal of human 2C9 P450 protein molecules, and a method of obtaining the crystal structure of a human 2C9 P450 molecule which comprises subjecting said crystal to X-rays; and analysing the diffraction pattern obtained to determine the 3-dimensional coordinates of the atoms of said 2C9 P450.

One such crystal is a crystal of P450 having the trigonal space group P321, and unit cell dimensions 165.46 Å, 165.46 Å, 111.70 Å, 90°, 90°, 120°. The crystal contains two copies of 2C9 in an asymmetric unit, denominated at A and B in Tables 1, 2, 3 and 8. Unit cell variability of 5% may be observed in all dimensions.

Such a crystal may be obtained using the methods described in the accompanying examples.

The crystal may be of a 2C9 protein which comprises the sequence of SEQ ID NO:2 other the following changes:
position 220 or position 222 is proline; and
optionally up to 21, for example up to 10, for example up to 5 other positions are altered, the positions being numbered according to wild type 2C9, and include the sequences described herein in the accompanying examples.

The methodology used to provide a P450 crystal illustrated herein may be used generally to provide a human P450 crystal resolvable at a resolution of at least 3.1 Å and preferably at least 3 Å.

Thus a crystal of a P450 protein described herein having a resolution of at least 3.1 Å and preferably at least 3 Å is disclosed.

In a further aspect, crystals of a P450 protein described herein may be made by a method which comprises growing a crystal by vapour diffusion using a reservoir buffer that contains a potassium salt and a PEG precipitate. The growing of the crystal is by vapour diffusion and is performed by placing an aliquot of the solution on a cover slip as a hanging drop above a well containing the reservoir buffer. Preferably the potassium salt is potassium phosphate, particularly 0.05 to 0.2 M potassium phosphate. The PEG precipitate concentration is preferably 10-30% PEG (more preferably 10-20% PEG). A higher weight PEG in the range of PEG 2000 to PEG 4000 may be used. Preferably PEG 3350 is used. The aliquot contains protein solution and reservoir buffer, typically in a ratio of 1 part protein solution to 1 part reservoir buffer. The protein solution was 0.7 mM. Most preferably the reservoir buffer is 0.2 M dibasic potassium phosphate and 20% PEG 3350. Alternative crystallisation conditions comprise (i) 0-0.2 M Tris-HCl (pH 8-9.5, preferably pH 8.4-8.8), 0-20% PEG 400, 0-20% PEG 8000, 0-20% glycerol or (ii) 0-0.2 M Tris-HCl (pH 8-9), 0-0.25 M Li₂SO₄, 0-20% PEG 4000; more particularly (iii) 0.1 M Tris-HCl (pH 8.8), 15% PEG 400, 5% PEG 8000, 10% glycerol, (iv) 0.1 M Tris-HCl (pH 8.5), 0.2 M Li₂SO₄, 15% PEG 4000 or (v) 0.1 M Tris-HCl (pH 8.4), 15% PEG 400, 5% PEG 8000, 10% glycerol. Condition (iv) is particularly preferred.

A total of 2648 crystallisation wells were set up to obtain a 3.0 Å dataset suitable for the solution of the first structure. A further 1584 wells were set up to achieve a crystal resolvable to 2.6 Å. This is an indication of the difficulty in obtaining crystals of suitable resolution for structure solution. The interpretation of the crystallisation screens and subsequent analysis of the results to determine which conditions to be set up, require significant experience.

Other crystals include crystals which have selected coordinates of the binding pocket, wherein the amino acid residues associated with those selected coordinates are located in a protein framework which holds these amino acids in a relative spatial configuration corresponding to the spatial configuration of those amino acids in Table 1, 2, 3 or 8. By "corresponding to", it is meant within a r.m.s.d. of less than 2.0 Å, preferably less than 1.5 Å, more preferably less than 1.0 Å, even more preferably less than 0.64 Å and most preferably less than 0.5 Å. The amino acids which provide the selected coordinates are preferably selected from amino acids which form part of the binding pocket of P450, and include those of Table 5 or 6, or combinations thereof as defined further herein below.

Crystals also include crystals of 2C9 mutants and chimeras as defined further below in Sections F and G.

The invention further relates to a method of assessing the ability of a compound to interact with P450 protein which comprises:
obtaining or synthesising said compound;
forming a crystallised complex of a P450 protein and said compound, said complex diffracting X-rays for the determination of atomic coordinates of said complex to a resolution of better than 3.1. Å and preferably at least 3 Å; and
analysing said complex by X-ray crystallography to determine the ability of said compound to interact with the P450 protein.

### D. Description of Structure.

The analysis of the crystals obtained in arriving at the present invention has allowed a detailed analysis of the structure of a human P450 molecule. Cytochrome P450 2C9 can be considered to be a two domain protein, with a smaller, predominantly beta strand domain and a larger, predominantly alpha helical domain, forming an overall triangular arrangement. All P450 structures solved to date have the same overall topology, leading to a nomenclature adopted by the literature to describe the individual alpha helices and beta strands within P450 structures (see Ravichandran et al, Science, 1993, 261, 731-736 for definitions). The protein as purified consists of residues 19-494 (numbering from full length 2C9), and all but the first and last few of these residues are distinguishable in the electron density. The beta strand domain consists of beta sheets 1 and 2 and alpha helices A and B. These structural elements are formed by the N-terminal region of the polypeptide chain (residues 30-90) and residues between the helices K and K'. These residues, along with the loops between helices B and C, and helices F and G (herein referred to as the B-C and F-G loops), are implicated in the interaction of mammalian P450s with the membrane when the protein is in its native membranous form. These loops also confer some of the reaction specificity to individual P450s and are among the most divergent regions of sequence.

The alpha helical domain consists of helices C through L. The heme moiety is located between the alpha helical and the beta strand domains, and sits above helix I (residues 284-315). The single protein ligand to the heme, cysteine 435, is found in a loop prior to the last alpha helix. Given the range of compounds that P450s metabolise, the substrate binding pockets of these enzymes can accommodate a variety of shapes and sizes. Access to and from the heme group may be regulated by the position of the loops that form the substrate binding site, leading to open and closed conformations of the enzyme. Mutational and activity data has allowed the mapping of regions of sequence to function.

A total of six substrate recognition sites (SRS) have been proposed by Gotoh (Gotoh, J. Biol. Chem., 267 (1992), 83-90). Some of the residues that line the binding pocket of the 2C9 structure include residues within these predicted SRS and include several residues that have been linked to changes in both specificity and reaction rates within mutant forms of the protein. The regiospecific hydroxylation of warfarin has been linked to polymorphism at residue 359; which lies above and to one side of the heme group, while residue 114 which has been shown to change warfarin and diclofenac hydroxylation rates, lies above and to the other side of the heme group.

The structure of the present invention confirms that many of the residues inferred as potential SRS residues in the prior art by other methods (e.g. sequence alignment and mutagenesis) are found in the various SRSs seen in our structure. We have also identified many other residues which are likely to provide side chains capable of interacting with many P450 substrates. For example, our structure indicates a number of residues, particularly with hydrophobic side chains, are in the SRS regions.

In the embodiments of the invention described herein where selected coordinates of the P450 structure may be used, the coordinates may include some or all of these residues.

An overlay of the 2C5 and 2C9 structure indicates that while the gross features of the protein are largely conserved between the two proteins, there are some interesting differences. The first resolvable residue in the electron density is residue 30 (all numbering is in relation to the full length protein), and the last residue is residue 490. Thus there are 10 residues without electron density at the N-terminus and the four histidine C-terminal tag is also not resolved.

Starting at the N-terminus, the two proteins adopt the same position at residue 48. Following the polypeptide chain back towards the N-terminus, the position of the two sequences is out of register by one, and towards the end, two residues, while the backbone trace of the two proteins is very close. The sequence identity in this region is particularly high, so such a difference seems somewhat surprising. It is probably attributable to the comparatively low resolution of the 2C5 structure which made accurately assigning the sequence at the N-terminus difficult. The higher resolution of the 2C9 structure has made assigning the sequence in this region less ambiguous. Thus this structure of 2C9 may be more representative of the true conformation of the N-termini of both 2C5 and 2C9.

The first region in which the two proteins differ substantially is the region between the B and C helices (residues 99 to 111). The temperature factors of the chain between residues 99 and 109 for 2C5 are high (the average B factor for all atoms in this range is 99.1 Å²), implying much mobility in this region, and hence little confidence can be placed in their position. In contract, the average B-factors for all atoms for residues 99 to 111 is 55.5 Å² in 2C9.

In the 2C9 structure residues 101 to 106 have adopted a helical formation (helix B') that has been observed in bacterial P450 structures. These residues form part of SRS 1, and thus contribute to the active site of the P450. The electron density has allowed unambiguous interpretation of all side chain positions in this region. A notable feature in this region is Arg97, which is proposed to be an important cation in the active site (2C9 substrate are predominantly acidic). The equivalent residue in 2C5 (Arg97) adopted a different conformation, and as a result did not form part of the active site. His99 has been implicated in omeprazole activity (Ibeanu et al., (1996), J Biol Chem, Vol. 271, 12496-12501); it is the only residue in SRS 1 not conserved between 2C9 and 2C19 (in 2C9 is it a Ile, in 2C19 a His), and mutation of this residue alone in 2C19 confers omeprazole activity to the resulting mutant protein. The 2C9 structure confirms that this residue forms part of the active site.

In the 2C9 structure the side chain position of Arg97 is clearly resolved, forming an interaction with the haem and Val113. Phe114 points into the active site and is well positioned to form pipi stacking interactions with substrates as has been suggested by a number of groups. Phe110 is in close proximity, but not as exposed at Phe114.

Arg105 and Arg108, which have also been suggested as potentially contributing to a cation site within the active site, both point away from the cavity.

The next region of divergence between the 2C5 and 2C9 structures is the region between the F and G helices. Residues 212 to 222 inclusive, which form part of the F-G loop, were absent in the published 2C5 structure. These residues are well resolved in the 2C9 structure, and form two turns of helix (all secondary structure assignment done using the program DSSP (Kabsch and Sander, Biopolymers 22 (1983) 2577-2637). Residues 220 and 221, while not contributing to the active site, clearly do have some impact on the accessibility of the active site, by mediating the position of the F-G loop. One of the disadvantages of mapping regions of sequence involved in substrate contact is the inability to distinguish between those regions which directly contact substrates (by lining the active site) and those that mediate the interaction the substrate has with the P450 by regulating structural elements within the enzyme. The 2C9 structure will allow the distinction between direct and indirect impact of individual residues on substrate specificity and activity. The redesign of compounds to facilitate or remove interactions with 2C9 is clearly going to be simplified by this distinction.

The residues at positions 286 and 289 have been implicated in substrate specificity (Klose et al., (1998), Arch. Biochem. Biophys., Vol. 357, 240-248). Only residue 289 actually lines the active site, but both are in close proximity to Phe110 of the B-C loop, and hence their role in substrate specificity may be an indirect one via the packing of structural elements, rather than a direct one through substrate contact.

Helices H and I adopt the same spatial conformation in the two proteins; the loop between the two helices is three residues longer and is clearly resolved in the electron density.

Phe476 forms a hydrophobic patch in the active site along with Phe100, Leu102, Leu208, Leu362, and Leu366.

There are 4 other alleles of 2C9 which have currently been identified, which have an amino acid substitution. 2C9*2 has R144C, 2C9*3 1359L, 2C9*4 I359T and 2C9*5 D360E. Ile359 does not lie in the active site, but is close to Thr305 and Thr361. It is not easy to envisage a direct effect of this residue on ability to catalyse compounds, but as has been noted for other residues, a mutation here may cause the shift of structural elements, which will impact on the active site. A similar effect may be true for Asp360. Arg 144 does not form part of the binding pocket of 2C9. It has however been widely believed that the variation in drug metabolism properties exhibited by those individuals possessing the 2C9 R144C allele variation is due to a modified interaction between the P450 and the reductase. The peripheral location of this residue in the structure of 2C9 would support this argument.

### Dimer Interface

The rotation angle between the two copies in the asymmetric unit is not 180°, and as a result the interface between the two copies (here referred to as A and B) is non-symmetrical. The interface involves a number of hydrogen bonds between residues in helix D of molecule A and the G-H loop bf molecule B, the G-H loop of molecule A and the C-terminus and helix D of molecule B, the C terminus of A and the G-H loop of molecule B.

### E. Crystal Coordinates.

In a further aspect, the invention also relates to the use of a crystal of P450 having the three dimensional atomic coordinates of Table 1, 2, 3 or 8. An advantageous feature of the structure defined by the atomic coordinates is that it has a high resolution (about 3 Å for Table 1, about 2.6 Å for Table 2, about 3.1 Å for Table 3 and about 2.6 Å for Table 8).

Another advantageous feature of the invention in that it provides atomic coordinate data relating to the loop between helices F and G (the FG loop). The FG loop is one of the most divergent topological regions between the mammalian and bacterial P450 enzymes. As such, it is one of the more difficult parts of the mammalian enzymes to model when using a bacterial structure as a modelling template. The structure of P450BM3 (Ravichandran et al, 1993, *ibid*) has been widely used within the field as a structural template for modelling the human forms. P450BM3 has just twelve residues in the FG loop, as opposed to the 21 residues in the 2C isoforms. The only mammalian P450 structure in the public domain is that of the rabbit 2C5 isoform, solved by X-ray crystallography to a resolution of 3.0 Å (Williams et al, Mol Cell (2000), 5, 121-131). While the 2C5 structure does provide an improved modelling template when compared to the bacterial structures, the position of the FG loop was not resolvable in the crystal structure. In contrast, the 2C9 structure described here includes the FG loop. Residues within the FG loop have not been widely implicated in the substrate selectivity of P450s, and lie outside the substrate recognition sites (SRS's) identified by Gotoh (Gotoh, O, J. Biol. Chem, 267; 83-90 (1992)). Residues within the FG loop have been shown to modify the compound binding specificity of 2C9 (Tsao et al, Biochemistry (2001), 40, 1937-1944). It was not clear whether this effect was due to direct interaction of residues within the FG loop and the compound, or a secondary effect caused by the interaction of these residues with residues within the pocket that fall within the substrate recognition sites (SRS) of the enzymes. It is now evident from our structure that the residues of the FG loop do not contribute to the binding pocket. The structure of 2C9 will therefore more readily facilitate the identification of direct and indirect interactions between compounds and 2C9.

Another advantageous feature is that the average B-factor of the 2C9 structure is 43.9 Å² in contrast to the 2C5 structure which had an overall B-factor of 58.6 Å², resulting in a better definition for most of the side chains within the structure. This is advantageous for all uses of the coordinates, especially *in silico* work, molecular replacement, and homology modelling.

A further advantage of the 2C9 structures described herein is that they are unliganded, apo structures. This makes them particularly suitable for soaking in ligands and hence determining co-complex structures and, are also ideal for homology modelling purposes as there is no conformational bias from a ligand.

The BC and FG loops are among the most varied features of cytochromes P450. Both loops contribute to the enzymes catalytic cycle; the BC loop directly by providing residues that form part of the active site, and mediate specificity and activity interactions, and the FG loop by movement allowing substrate entry and exit. In this high resolution 2C9 structure both of these loops are well resolved, in contrast to the 2C5 structure.

Tables 1, 2, 3 and 8 give atomic coordinate data for P450 2C9. In Tables 1, 2, 3 and 8 the third column denotes the atom, the fourth the residue type, the fifth the chain identification (either A or B), the sixth the residue number (the atom numbering is with respect to the full length wild type protein), the seventh, eighth and ninth columns are the X, Y, Z coordinates respectively of the atom in question, the tenth column the occupancy of the atom, the eleventh the temperature factor of the atom, the twelfth (where present) the chain identification, and the last the atom type.

The coordinates of Tables 1, 2, 3 and 8 provide a measure of atomic location in Angstroms, to 3 decimal places. The coordinates are a relative set of positions that define a shape in three dimensions, but the skilled person would understand that an entirely different set of coordinates having a different origin and/or axes could define a similar or identical shape. Furthermore, the skilled person would understand that varying the relative atomic positions of the atoms of the structure so that the root mean square deviation of the residue backbone atoms (i.e. the nitrogen-carbon-carbon backbone atoms of the protein amino acid residues) is less than 2.0 Å, preferably less than 1.5 Å, more preferably less than 1.0 Å, even more preferably less than 0.64 Å and most preferably less than 0.5 Å when superimposed on the coordinates provided in Table 1, 2, 3 or 8 for the residue backbone atoms, will generally result in a structure which is substantially the same as the structure of Table 1, 2, 3 or 8 in terms of both its structural characteristics and usefulness for structure-based analysis of P450-interactivity molecular structures.

Likewise the skilled person would understand that changing the number and/or positions of the water molecules and/or substrate molecules of Table 1, 2, 3 or 8 will not generally affect the usefulness of the structure for structure-based analysis of P450-interacting structure. Thus for the purposes described herein as being aspects of the present invention, it is within the scope of the invention if: the Table 1, 2, 3 or 8 coordinates are transposed to a different origin and/or axes; the relative atomic positions of the atoms of the structure are varied so that the root mean square deviation of residue backbone atoms is less than 2.0 Å, preferably less than 1.5 Å, more preferably less than 1.0 Å, even more preferably less than 0.64 Å and most preferably less than 0.5 Å when superimposed on the coordinates provided in Table 1, 2, 3 or 8 for the residue backbone atoms; and/or the number and/or positions of water molecules and/or substrate molecules is varied.

Reference herein to the coordinate data of Table 1, 2, 3 or 8 and the like thus includes the coordinate data in which one or more individual values of the Table are varied in this way. By "root mean square deviation" we mean the square root of the arithmetic mean of the squares of the deviations from the mean.

Thus, for example, varying the atomic positions of the atoms of the structure by up to about 0.5 Å, preferably up to about 0.3 Å in any direction will result in a structure which is substantially the same as the structure of Table 1, 2, 3 or 8 in terms of both its structural characteristics and utility e.g. for molecular structure-based analysis.

Those of skill in the art will appreciate that in many applications of the invention, it is not necessary to utilise all the coordinates of Table 1, 2, 3 or 8, but merely a portion of them. For example, as described below, in methods of modelling candidate compounds with P450, selected coordinates of 2C9 may be used.

By "selected coordinates" it is meant for example at least 5, preferably at least 10, more preferably at least 50 and even more preferably at least 100, for example at least 500 or at least 1000 atoms of the 2C9 structure. Likewise, the other applications of the invention described herein, including homology modelling and structure solution, and data storage and computer assisted manipulation of the coordinates, may also utilise all or a portion of the coordinates (i.e. selected coordinates) of Table 1, 2, 3 or 8. The selected coordinates may include or may consist of atoms found in the 2C9 P450 binding pocket, as described herein below.

### F. Chimaeras

The use of chimaeric proteins to achieve desired properties is now common in the scientific literature. For example, Sieber et al (Nature Biotechnology (2001) 19,456-460) produced hybrids between human cytochrome P450 isoform 1A2 and the bacterial P450 BM3, in order to make proteins with the specificity of 1A2, but which had desirable expression and solubility properties of BM3. Active site chimaeras are also described: for example, Swairjo et al (Biochemistry (1998) 37, 10928-10936) made loop chimaeras of HIV-1 and HIV-2 protease to try to understand determinants of inhibitor-binding specificity.

Of particular relevance are cases where the active site is modified so as to provide a surrogate system to obtain structural information. Thus Ikuta et al (J Biol Chem (2001) 276, 27548-27554) modified the active site of cdk2, for which they could obtain structural data, to resemble that of cdk4, for which no X-ray structure is currently available. In this way they were able to obtain protein/ligand structures from the chimaeric protein which were useful in cdk4 inhibitor design. In a similar way, based on comparison of primary sequences of highly related isoforms (such as 2C 19 or even 2D6), the active site of the 2C9 protein could be modified to resemble those isoforms. Protein structures or protein/ligand structures of the chimaeric proteins could be used in structure-based alteration of the metabolism of compounds which are substrates of that related P450 isoform.

Even if the percentage of the amino acid sequence identity between mammalian P450 ranks from 20 to 80%, the overall folding of mammalian P450s is expected to be very similar, with the same spatial distribution of the structural elements. Furthermore, this class of enzymes exhibits distinct substrate specificities that rely on only a limited number of residues located in non-contiguous parts of the polypeptide chain. The substrate-binding pocket of P450 is generally constituted by residues that fall in the SRS regions (substrate recognition sites) defined by Gotoh (Gotoh, O, J. Biol. Chem, 267; 83-90 (1992)) and in loops of the molecule.

Aspects of the present invention therefore relate to modification of P450 proteins such that the active sites mimic those of related isoforms. For example, from a knowledge of the structure and residues of the active site of the human 2C9 protein described herein, and that of the rabbit 2C5 protein published previously, a person skilled in the art could modify the 2C5 protein such that the active site mimicked that of human 2C9. This protein could then be used to obtain information on compound binding through the determination of protein/ligand complex structures using the chimaeric 2C5 protein.

For example, in one aspect the present invention provides a chimaeric protein having a binding cavity which provides a substrate specificity substantially identical to that of P450 2C9 protein, wherein the chimaeric protein binding cavity is lined by a plurality of atoms which correspond to selected P450 2C9 atoms lining the P450 2C9 binding cavity, the relative positions of the plurality of atoms corresponding to the relative positions, as defined by Table 1, 2, 3 or 8, of the selected P450 2C9 atoms.

It is possible to postulate that only few changes would be required to inter-convert the substrate specificities of P450 isoforms that exhibit more than 70% of amino acid identity. For example, 2C9 and 2C 19, although they differ at only 43 of 490 amino acids, exhibits clear substrate specificity differences. Using a panel of 2C9/2C19 chimaeric proteins, Jung *et al.* (Jung, F. Biochemistry, 37, 16270-16279 (1998)), have identified the sequences differences that confer to 2C19 a high affinity binding to sulfaphenazole, a very potent and specific inhibitor of 2C9. Site directed mutagenesis experiments have revealed that the conversion of 2C19 to a 2C9-like protein was possible by introducing a limited number of substitutions in the 2C 19 amino acid sequence. These mutations are located in the SRS3 and SRS4 regions of the proteins. Similar studies performed by Klose *et al.* (Arch. Biochem. Biophys. 357, 240-248 (1998)) and Tsao *et al.* (Biochemistry, 40, 1937-1944, (2001)) have demonstrated the feasibility of the transfer of substrate specificities between 2C9 and 2C 19 by mutating SRS regions.

The substrate specificity of an enzyme generally relies on only a limited number of residues located in non-contiguous parts of the polypeptide chain. The substrate specificities of these isoforms could be analysed by substituting these residues by site-directed mutagenesis. The minimal changes that would be required to convert another protein into a 2C9-like chimera could be at least two amino acids selected from Table 4. These mutations can be introduced by site-directed mutagenesis e.g. using a Stratagene QuikChange^{™} Site-Directed Mutagenesis Kit or cassette mutagenesis methods (Ausubel, F.M., Brent, R., Kingston, R.E. et al. editors. Current Protocols in Molecular Biology. John Wiley & Sons, Inc., New York, Sambrook, J., Fritsch, E.F., and Maniatis, T. (1989). Molecular Cloning: a Laboratory Manual. 2nd ed. Cold Spring Harbor Laboratory Press, Cold Spring Harbor, NY.). Thus the invention provides a chimaeric protein having one or more binding pockets defined by the residues of Table 4.

This strategy could clearly be applied for proteins that exhibit high sequence homology with or without overlapping substrate specificities and from different species. The rabbit 2C5 and the human 2C9 and 2C19 P450s have been reported to be involved in the metabolism of progesterone with different rates, the rabbit isoform being clearly the most efficient enzyme. The use of the crystal structures solved for 2C5 and 2C9 would allow the characterization of the binding mode of the progesterone molecule in the substrate pocket of these proteins. This in turn would allow the identification of residues to be modified in the human isoforms to convert them into efficient progesterone metabolising enzymes.

In one embodiment, a chimaeric 2C9 enzyme is produced which is isoformal with another enzyme of the 2C subfamily. For example, 2C9 could be turned into a 2C19-like isoform with a few amino acid changes. Based on the information available from the literature on the structure/activity studies performed on the 2C9 and 2C19 isoforms, and the analysis of the structure of the human 2C9, we postulate that the 2C9 protein could be converted to a 2C 19-like protein with the substrate specificities attributed to 2C19.
The residues to be mutated are one or more of:
Substitute SRS 1 of 2C9 with SRS 1 of 2C19 (the amino acid change introduced is I99H); and/or
Substitute SRS 3 of 2C9 with SRS 3 of 2C19 (the amino acid changes introduced are V237L and K241E); and/or
Substitute SRS 4 of 2C9 with SRS 4 of 2C19 (the amino acid changes introduced are S286N, E288V, N289I, V292A and F295L - the key changes could be S286N, N289I, V292A and F295L); and/or
Move SRS5 of 2C19 to 2C9 (the amino acid L362I is introduced).

The minimal changes that would be required to convert 2C9 to 2C 19 could be I99H, K241E, S286N, N289I, V292A, F295L and L362I and more likely to be I99H, S286N, N289I, V292A, and F295L. These mutations can be introduced by site-directed mutagenesis or cassette mutagenesis methods, as described herein.

A 2C19-like chimera can also be made by making the following changes: I99H, S286N, E288V, N2891, V292A, F295L. An alternative minimal change would be I99H, S286N, N289I.

The crystallization of such chimeras and the determination of the three-dimensional structures relies on the ability of our 2C9 proteins to yield crystals that diffract at high resolution. The aim is to modify the inside part or 2C9 to produce a new substrate binding site of 2C 19 without modifying the outside shell of the proteins that allow the protein to crystallize.

### G. Homology Modelling.

The invention also provides a means for homology modelling of other proteins (referred to below as target P450 proteins). By "homology modelling", it is meant the prediction of related P450 structures based either on x-ray crystallographic data or computer-assisted *de novo* prediction of structure, based upon manipulation of the coordinate data of Table 1, 2, 3 or 8.

The P450 structure set out in Table 1, 2, 3 or 8 is, as explained in further detail herein, a dimer structure. The various *in silico* modelling techniques described in this section and in the other sections of this application may utilize either the dimer structure of Table 1, 2, 3 or 8, or either of the subunits A and B. To avoid unnecessary repetition, reference is made herein to the coordinate data of Table 1, 2, 3 or 8, but this will be understood to mean either the data for both subunits or just one of the subunits.

"Homology modelling" extends to target P450 proteins which are analogues or homologues of the 2C9 P450 protein whose structure has been determined in the accompanying examples. It also extends to P450 protein mutants of 2C9 protein itself.

In general, the method involves comparing the amino acid sequences of the 2C9 P450 protein of Table 1, 2, 3 or 8 with a target P450 protein by aligning the amino acid sequences. Amino acids in the sequences are then compared and groups of amino acids that are homologous (conveniently referred to as "corresponding regions") are grouped together. This method detects conserved regions of the polypeptides and accounts for amino acid insertions or deletions.

Homology between amino acid sequences can be determined using commercially available algorithms. The programs *BLAST, gapped BLAST*, *BLASTN, PSI-BLAST* and *BLAST 2* sequences (provided by the National Center for Biotechnology Information) are widely used in the art for this purpose, and can align homologous regions of two amino acid sequences. These may be used with default parameters to determine the degree of homology between the amino acid sequence of the Table 1, 2, 3 or 8 protein and other target P450 proteins which are to be modelled.

Analogues are defined as proteins with similar three-dimensional structures and/or functions and little evidence of a common ancestor at a sequence level.

Homologues are defined as proteins with evidence of a common ancestor i.e. likely to be the result of evolutionary divergence and are divided into remote, medium and close sub-divisions based on the degree (usually expressed as a percentage) of sequence identity.

A homologue is defined here as a protein with at least 15% sequence identity or which has at least one functional domain, which is characteristic of 2C9. This includes polymorphic forms of 2C9.

There are two types of homologue: orthologues and paralogues. Orthologues are defined as homologous genes in different organisms, i.e. the genes share a common ancestor coincident with the speciation event that generated them. Paralogues are defined as homologous genes in the same organism derived from a gene/chromosome/genome duplication, i.e. the common ancestor of the genes occurred since the last speciation event.

A mutant is a 2C9 characterized by replacement or deletion of at least one amino acid from the wild type 2C9. Such a mutant may be prepared for example by site-specific mutagenesis, or incorporation of natural or unnatural amino acids.

The present invention contemplates "mutants" wherein a "mutant" refers to a polypeptide which is obtained by replacing at least one amino acid residue in a native or synthetic 2C9 with a different amino acid residue and/or by adding and/or deleting amino acid residues within the native polypeptide or at the N- and/or C-terminus of a polypeptide corresponding to 2C9 and which has substantially the same three-dimensional structure as 2C9 from which it is derived. By having substantially the same three-dimensional structure is meant having a set of atomic structure coordinates that have a root mean square deviation (r.m.s.d.) of less than or equal to about 2.0 Å when superimposed with the atomic structure coordinates of the 2C9 from which the mutant is derived when at least about 50% to 100% of the C_{α} atoms of the 2C9 are included in the superposition. A mutant may have, but need not have, enzymatic or catalytic activity.

To produce homologues or mutants, amino acids present in the said protein can be replaced by other amino acids having similar properties, for example hydrophobicity, hydrophobic moment, antigenicity, propensity to form or break α-helical or β-sheet structures, and so. Substitutional variants of a protein are those in which at least one amino acid in the protein sequence has been removed and a different residue inserted in its place. Amino acid substitutions are typically of single residues but may be clustered depending on functional constraints e.g. at a crystal contact. Preferably amino acid substitutions will comprise conservative amino acid substitutions. Insertional amino acid variants are those in which one or more amino acids are introduced. This can be amino-terminal and/or carboxy-terminal fusion as well as intrasequence. Examples of amino-terminal and/or carboxy-terminal fusions are affinity tags, MBP tag, and epitope tags.

Amino acid substitutions, deletions and additions which do not significantly interfere with the three-dimensional structure of the 2C9 will depend, in part, on the region of the 2C9 where the substitution, addition or deletion occurs. In highly variable regions of the molecule, non-conservative substitutions as well as conservative substitutions may be tolerated without significantly disrupting the three-dimensional structure of the molecule. In highly conserved regions, or regions containing significant secondary structure, conservative amino acid substitutions are preferred.

Conservative amino acid substitutions are well-known in the art, and include substitutions made on the basis of similarity in polarity, charge, solubility, hydrophobicity, hydrophilicity and/or the amphipathic nature of the amino acid residues involved. For example, negatively charged amino acids include aspartic acid and glutamic acid; positively charged amino acids include lysine and arginine; amino acids with uncharged polar head groups having similar hydrophilicity values include the following: leucine, isoleucine, valine; glycine, alanine; asparagine, glutamine; serine, threonine; phenylalanine, tyrosine. Other conservative amino acid substitutions are well known in the art.

In some instances, it may be particularly advantageous or convenient to substitute, delete and/or add amino acid residues to a 2C9 binding pocket or catalytic residue in order to provide convenient cloning sites in cDNA encoding the polypeptide, to aid in purification of the polypeptide, etc. Such substitutions, deletions and/or additions which do not substantially alter the three dimensional structure of 2C9 will be apparent to those having skills in the art.

It should be noted that the mutants contemplated herein need not exhibit enzymatic activity. Indeed, amino acid substitutions, additions or deletions that interfere with the catalytic activity of the 2C9 but which do not significantly alter the three-dimensional structure of the catalytic region are specifically contemplated by the invention. Such crystalline polypeptides, or the atomic structure coordinates obtained there from, can be used to identify compounds that bind to the protein.

Once the amino acid sequences of the polypeptides with known and unknown structures are aligned, the structures of the conserved amino acids in a computer representation of the polypeptide with known structure are transferred to the corresponding amino acids of the polypeptide whose structure is unknown. For example, a tyrosine in the amino acid sequence of known structure may be replaced by a phenylalanine, the corresponding homologous amino acid in the amino acid sequence of unknown structure.

The structures of amino acids located in non-conserved regions may be assigned manually by using standard peptide geometries or by molecular simulation techniques, such as molecular dynamics. The final step in the process is accomplished by refining the entire structure using molecular dynamics and/or energy minimization.

Homology modelling as such is a technique that is well known to those skilled in the art (see e.g. Greer, *Science,* Vol. 228, (1985), 1055, and Blundell *et al., Eur. J. Biochem,* Vol. 172, (1988), 513). The techniques described in these references, as well as other homology modelling techniques generally available in the art, may be used in performing the present invention.

Thus the invention provides a method of homology modelling comprising the steps of:
(a) aligning a representation of an amino acid sequence of a target P450 protein of unknown three-dimensional structure with the amino acid sequence of the P450 of Table 1, 2, 3 or 8 to match homologous regions of the amino acid sequences;
(b) modelling the structure of the matched homologous regions of said target P450 of unknown structure on the corresponding regions of the P450 structure as defined by Table 1, 2, 3 or 8; and
(c) determining a conformation (e.g. so that favourable interactions are formed within the target P450 of unknown structure and/or so that a low energy conformation is formed) for said target P450 of unknown structure which substantially preserves the structure of said matched homologous regions.

Preferably one or all of steps (a) to (c) are performed by computer modelling.

The presence of the FG loop in our structure is particularly advantageous for modelling of other P450s especially mammalian P450s, which have longer FG loops than bacterial P450s as there is currently nothing known in the art about the conformation of the FG loop in mammalian structures. This is advantageous for modelling compounds into this structure or modelled structures.

The data of Table 1, 2, 3 or 8 will be particularly advantageous for homology modelling of other human P450 proteins, in particular human P450s such as 2C8, 2C 18, 2C 19, 2D6, 3A4, 1A1, 1A2, 2E1. These proteins may be the target P450 protein in the method of the invention described above.

In a particularly preferred aspect, the homology model is selected from the group consisting of 2C19, 2C18 and 2C8. The accompanying examples show a complete homology model for 2C19 and the coordinates of 2C18 and 2C8 which may be introduced into the structures of 2C9 or 2C19 in order to provide a homology model of these proteins. The resulting homology models may be used in the methods described herein below in sections H, I and J.

### H. Structure Solution

The structure of the human 2C9 P450 can also be used to solve the crystal structure of other target P450 proteins including other crystal forms of 2C9, mutants, co-complexes of 2C9, where X-ray diffraction data of these target P450 proteins has been generated and requires interpretation in order to provide a structure.

In the case of 2C9, this protein may crystallize in more than one crystal form. The structure coordinates of 2C9, or portions thereof, as provided by this invention are particularly useful to solve the structure of those other crystal forms of 2C9. They may also be used to solve the structure of 2C9 mutants, 2C9 co-complexes, or of the crystalline form of any other protein with significant amino acid sequence homology to any functional domain of 2C9.

In the case of other target P450 proteins, particularly the human P450 proteins referred to in Section D above, the present invention allows the structures of such targets to be obtained more readily where raw X-ray diffraction data is generated.

Thus, where X-ray crystallographic or NMR spectroscopic data is provided for a target P450 of unknown three-dimensional structure, the structure of P450 as defined by Table 1, 2, 3, 8 or 18 may be used to interpret that data to provide a likely structure for the other P450 by techniques which are well known in the art, e.g. phasing in the case of X-ray crystallography and assisting peak assignments in NMR spectra.

One method that may be employed for these purposes is molecular replacement. In this method, the unknown crystal structure, whether it is another crystal form of 2C9, a 2C9 mutant, or a 2C9 co-complex, or the crystal of a target P450 protein with amino acid sequence homology to any functional domain of 2C9, may be determined using the 2C9 structure coordinates of this invention as provided herein. This method will provide an accurate structural form for the unknown crystal more quickly and efficiently than attempting to determine such information *ab initio.*

Examples of computer programs known in the art for performing molecular replacement are CNX (Brunger A.T.; Adams P.D.; Rice L.M., Current Opinion in Structural Biology, Volume 8, Issue 5, October 1998, Pages 606-611 (also commercially available from Accelerys San Diego, CA) or AMORE (Navaza, J. (1994). AMoRe: an automated package for molecular replacement. Acta Cryst. A50, 157-163).

Thus, in a further aspect of the invention provides a method for determining the structure of a protein, which method comprises;
providing the coordinates of Table 1, 2, 3 or 8, and positioning the coordinates in the crystal unit cell of said protein so as to provide a structure for said protein.

In a preferred aspect of this invention the coordinates are used to solve the structure of target P450s particularly homologues of 2C9 for example 2C19, 2C8, 2C18.

The invention may also be used to assign peaks of NMR spectra of such proteins, by manipulation of the data of Table 1, 2, 3 or 8.

### I. Computer Systems.

In another aspect, the systems, particularly a computer system are useful in performing methods of the invention. The systems contain either (a) atomic coordinate data according to Table 1, 2, 3, 8 or 18, said data defining the three-dimensional structure of P450 or at least selected coordinates thereof; (b) structure factor data (where a structure factor comprises the amplitude and phase of the diffracted wave) for P450; said structure factor data being derivable from the atomic coordinate data of Table 1, 2, 3, 8 or 18; (c) atomic coordinate data of a target P450 protein generated by homology modelling of the target based on the data of Table 1, 2, 3, 8 or 18; (d) atomic coordinate data of a target P450 protein generated by interpreting X-ray crystallographic data or NMR data by reference to the data of Table 1, 2, 3 or 18; or (e) structure factor data derivable from the atomic coordinate data of (c) or (d).

The atomic coordinate data may be the data of the entire Table or a selected portion thereof.

With regard to (c) above, it will be appreciated that Table 18 itself is atomic coordinate data of a 2C19 obtained by the homology modelling the 2C9 structure of the present invention and the data of Table 18, and its use, forms a further aspect of the invention.

Systems may also contain atomic coordinate data of target P450 proteins wherein such data has been generated according to the methods of the invention described herein based on the starting data provided by Table 1, 2, 3, 8 or 18.

Such data is useful for a number of purposes, including the generation of structures to analyse the mechanisms of action of P450 proteins and/or to perform rational drug design of compounds which interact with P450, such as compounds which are metabolised by P450s.

In a further aspect, we describe computer readable storage medium with either (a) atomic coordinate data according to Table 1, 2, 3, 8 or 18 recorded thereon, said data defining the three-dimensional structure of P450, or at least selected coordinates thereof; (b) structure factor data for P450 recorded thereon, the structure factor data being derivable from the atomic coordinate data of Table 1, 2, 3, 8 or 18; (c) atomic coordinate data of a target P450 protein generated by homology modelling of the target based on the data of Table 1, 2, 3,8 or 18; (d) atomic coordinate data of a target P450 protein generated by interpreting X-ray crystallographic data or NMR data by reference to the data of Table 1, 2, 3, 8 or 18; or (e) structure factor data derivable from the atomic coordinate data of (c) or (d).

The atomic coordinate data may be the data of the entire Table or a selected portion thereof.

As used herein, "computer-readable storage medium" refers to any medium or media which can be read and accessed directly by a computer. Such media include, but are not limited to: magnetic storage media such as floppy discs, hard disc storage medium and magnetic tape; optical storage media such as optical discs or CD-ROM; electrical storage media such as RAM and ROM; and hybrids of these categories such as magnetic/optical storage media.

By providing such a storage medium, the atomic coordinate data can be routinely accessed to model P450 or selected coordinates thereof. For example, RASMOL (Sayle et al., *TIBS,* Vol. 20, (1995), 374) is a publicly available computer software package which allows access and analysis of atomic coordinate data for structure determination and/or rational drug design.

On the other hand, structure factor data, which are derivable from atomic coordinate data (see e.g. Blundell et al., in *Protein Crystallography,* Academic Press, New York, London and San Francisco, (1976)), are particularly useful for calculating e.g. difference Fourier electron density maps.

As used herein, "a computer system" refers to the hardware means, software means and data storage means used to analyse the atomic coordinate data of the present invention. The minimum hardware means of the computer-based systems of the present invention typically comprises a central processing unit (CPU), a working memory and data storage means, and e.g. input means, output means etc. Desirably a monitor is provided to visualize structure data. The data storage means may be RAM or means for accessing computer readable media of the invention. Examples of such systems are microcomputer workstations available from Silicon Graphics Incorporated and Sun Microsystems running Unix based, Windows NT or IBM OS/2 operating systems.

In another aspect, the computer-readable storage medium, comprising a data storage material encoded with computer readable data, wherein the data are defined by all or a portion (i.e. selected coordinates as defined herein) of the structure coordinates of 2C9 of Table 1,2,3 or 8, or a homologue of 2C9 including the structure of 2C19 of Table 18, wherein said homologue comprises backbone atoms that have a root mean square deviation from the backbone atoms (nitrogen-carbon_{α}-carbon) of Table 1, 2, 3 or 8 of not more than 2.0 Å (preferably not more than 1.5-Å) may be provided.

A computer-readable data storage medium may comprise a data storage material encoded with a first set of computer-readable data comprising a Fourier transform of at least a portion (i.e. selected coordinates as defined herein) of the structural coordinates for 2C9 according to Table 1, 2, 3 or 8 or 2C 19 of Table 18; which, when combined with a second set of machine readable data comprising an X-ray diffraction pattern of a molecule or molecular complex of unknown structure, using a machine programmed with the instructions for using said first set of data and said second set of data, can determine at least a portion of the structure coordinates corresponding to the second set of machine readable data.

Data for generating structures and/or performing drug design with 2C9, 2C9 homologues or analogues, complexes of 2C9 with a compound, or complexes of 2C9 homologues or analogues with compounds in accordance with the invention may be provided by a method comprising:
(i) establishing communication with a remote device containing computer-readable data comprising at least one of: (a) atomic coordinate data according to Table 1, 2, 3 or 8, said data defining the three-dimensional structure of 2C9, at least one sub-domain of the three-dimensional structure of 2C9, or the coordinates of a plurality of atoms of 2C9; (b) structure factor data for 2C9, said structure factor data being derivable from the atomic coordinate data of Table 1, 2, 3 or 8; (c) atomic coordinate data of a target 2C9 homologue or analogue generated by homology modelling of the target based on the data of Table 1, 2, 3 or 8, such as the data of Table 18; (d) atomic coordinate data of a protein generated by interpreting X-ray crystallographic data or NMR data by reference to the data of Table 1, 2, 3 or 8; and (e) structure factor data derivable from the atomic coordinate data of (c) or (d); and
(ii) receiving said computer-readable data from said remote device.

Data for for generating structures and/or performing drug design with 2C19, 2C19 homologues or analogues, complexes of 2C19 with a compound, or complexes of 2C19 homologues or analogues with compounds, may be provided by a method comprising:
(i) establishing communication with a remote device containing computer-readable data comprising at least one of: (a) atomic coordinate data according to Table 18, said data defining the three-dimensional structure of 2C19, at least one sub-domain of the three-dimensional structure of 2C19, or the coordinates of a plurality of atoms of 2C 19; (b) structure factor data for 2C19, said structure factor data being derivable from the atomic coordinate data of Table 18; (c) atomic coordinate data of a target 2C 19 homologue or analogue generated by homology modelling of the target based on the data of Table 18; (d) atomic coordinate data of a protein generated by interpreting X-ray crystallographic data or NMR data by reference to the data of Table 18; and (e) structure factor data derivable from the atomic coordinate data of (c) or (d); and
(ii) receiving said computer-readable data from said remote device.

Thus the remote device may comprise e.g. a computer system or a computer-readable storage medium of one of the previous aspects of the invention. The device may be in a different country or jurisdiction from where the computer-readable data is received.

The communication may be via the internet, intranet, e-mail etc. Typically the communication will be electronic in nature, but some or all of the communication pathway may be optical, for example, over optical fibres.

### J. Uses of the Structures of the Invention.

The crystal structures obtained according to the present invention (including the structures of Table 1, 2, 3, 8 and 18 as well the structures of target P450 proteins obtained in accordance with the methods described herein) may be used in several ways for drug design. For example, many drugs or drug candidates fail to be of clinical use due to the detrimental interactions with P450 proteins, resulting in a rapid clearance of the drugs from the body. The present invention will allow those of skill in the art to attempt to rescue such compounds from development by following these structure-based chemical strategies.

In the case where a drug molecule is being metabolised by a P450, information on the binding orientation by either co-crystallization, soaking or computationally docking the binding orientation of the drug in the binding pocket can be determined. This will guide specific modifications to the chemical structure designed to mediate or control the interaction of the drug with the protein. Such modifications can be designed with an aim of reducing the metabolism of the drug by P450 and so of improving its therapeutic action.

The crystal structure could also be useful to understand drug-drug interactions. Many examples exist where adverse reactions to drugs are recorded if administered while the patient is already taking other medicines. The mechanism behind this detrimental and often dangerous drug-drug interaction scenario may be when one drug behaves as an inhibitor of a P450 resulting in toxic levels of the other drug building-up due to less or no metabolism occurring. The crystal structure of the present invention complexed to such an inhibitor (either *in* vitro or *in silico*) may also allow rational modifications either to modify the inhibitor such that it no longer inhibits or inhibits less, or to modify the second drug such that it could bind better to the P450 (so becoming metabolised) and so displace the inhibitor.

P450s display significant polymorphic variations dependent on ethnic origin of the patient. This can manifest itself in adverse reactions from some segments of patient populations to some drugs. By using the crystal structures of the present invention to map the relevant mutation with respect to the binding mode of the drug, chemical modifications could also be made to the drug to avoid interactions with the variable region of the protein. This would ensure more consistent therapeutic value from the drug for such segments of the population and avoid dangerous side-effects.

Some pharmaceutical compounds are converted by P450s into active metabolites. In the case of such compounds, a greater understanding of how such compounds are converted by a P450 will allow modification of the compound so that it can be converted at a different rate. For example, increasing the rate of conversion may allow a more rapid delivery of a desired therapeutic effect, whereas decreasing the rate of conversion may allow for higher doses to be administered or the development of sustained release pharmaceutical preparations, for example comprising a mixture of compounds which are metabolised at different rates to form the same active metabolite.

Thus, the determination of the three-dimensional structure of P450 provides a basis for the design of new compounds which interact with P450 in novel ways. For example, knowing the three-dimensional structure of P450, computer modelling programs may be used to design different molecules expected to interact with possible or confirmed active sites, such as binding sites or other structural or functional features of P450.

### (i) Obtaining and analysing crystal complexes.

In one approach, the structure of a compound bound to a P450 may be determined by experiment. This will provide a starting point in the analysis of the compound bound to P450, thus providing those of skill in the art with a detailed insight as to how that particular compound interacts with P450 and the mechanism by which it is metabolised.

Many of the techniques and approaches to structure-based drug design described above rely at some stage on X-ray analysis to identify the binding position of a ligand in a ligand-protein complex. A common way of doing this is to perform X-ray crystallography on the complex, produce a difference Fourier electron density map, and associate a particular pattern of electron density with the ligand. However, in order to produce the map (as explained e.g. by Blundell et al., mentioned above) it is necessary to know beforehand the protein 3D structure (or at least the protein structure factors). Therefore, determination of the P450 structure also allows production of difference Fourier electron density maps of P450-compound complexes and determination of the binding position of a drug, and hence may greatly assist the process of rational drug design.

Accordingly, the invention provides a method for determining the structure of a compound bound to P450, said method comprising:
providing a crystal of 2C9 P450 according to the invention;
soaking the crystal with said compounds; and
determining the structure of said 2C9 P450 compound complex by employing the data of Table 1, 2, 3, 8 or 18.

Alternatively, the P450 and compound may be co-crystallized. Thus the invention provides a method for determining the structure of a compound bound to P450, said method comprising; mixing the protein with the compound(s), crystallizing the protein-compound(s) complex; and determining the structure of said P450-compound(s) complex by reference to the data of Table 1,2,3,8 or 18.

The analysis of such structures may employ (i) X-ray crystallographic diffraction data from the complex and (ii) a three-dimensional structure of P450, or at least selected coordinates thereof, to generate a difference Fourier electron density map of the complex, the three-dimensional structure being defined by atomic coordinate data according to Table 1, 2, 3 or 8. The difference Fourier electron density map may then be analysed.

Therefore, such complexes can be crystallized and analysed using X-ray diffraction methods, e.g. according to the approach described by Greer et al., *J. of Medicinal Chemistry,* Vol. 37, (1994), 1035-1054, and difference Fourier electron density maps can be calculated based on X-ray diffraction patterns of soaked or co-crystallized P450 and the solved structure of uncomplexed P450. These maps can then be analysed e.g. to determine whether and where a particular compound binds to P450 and/or changes the conformation of P450.

Electron density maps can be calculated using programs such as those from the CCP4 computing package (Collaborative Computational Project 4. The CCP4 Suite: Programs for Protein Crystallography, *Acta Crystallographica,* D50, (1994), 760-763.). For map visualization and model building programs such as "O" (Jones et al., *Acta Crystallographica,* A47, (1991), 110-119) can be used.

In addition, in accordance with this invention, 2C9 mutants may be crystallized in co-complex with known 2C9 substrates or inhibitors or novel compounds. The crystal structures of a series of such complexes may then be solved by molecular replacement and compared with that of the 2C9 of Table 1, 2, 3 or 8. Potential sites for modification within the various binding sites of the enzyme may thus be identified. This information provides an additional tool for determining the most efficient binding interactions, for example, increased hydrophobic interactions, between 2C9 and a chemical entity or compound.

For example there are alleles of 2C9, which differ from the native 2C9 by only 1 or 2 amino acid substitutions, and yet individuals who express these allelic variants may exhibit very different drug metabolism profiles. By generating these allelic proteins and determining the co-complex with compounds a greater understanding of allelic interactions with compounds may be developed.

All of the complexes referred to above may be studied using well-known X-ray diffraction techniques and may be refined against 1.5 to 3.5 Å resolution X-ray data to an R value of about 0.30 or less using computer software, such as CNX (mentioned above) X-PLOR (Yale University, ©1992, distributed by Accelerys - also see, e.g., Blundell et al; Methods in Enzymology, vol. 114 & 115, H. W. Wyckoff et al., eds., Academic Press (1985) (23)).

This information may thus be used to optimise known classes of 2C9 substrates or inhibitors, and more importantly, to design and synthesize novel classes of 2C9 inhibitors and design drugs with modified P450 metabolism.

### (ii) In silico analysis and design.

Although the invention will facilitate the determination of actual crystal structures comprising a P450 and a compound which interacts with the P450, current computational techniques provide a powerful alternative to the need to generate such crystals and generate and analyse diffraction date. Accordingly, a particularly preferred aspect of the invention relates to *in silico* methods directed to the analysis and development of compounds which interact with P450 structures of the present invention.

Thus as a result of the determination of the P450 three-dimensional structure, more purely computational techniques for rational drug design may also be used to design structures whose interaction with P450 is better understood (for an overview of these techniques see e.g. Walters et al (*Drug Discovery Today,* Vol.3, No.4, (1998), 160-178). For example, automated ligand-receptor docking programs (discussed e.g. by Jones et al. in *Current Opinion in Biotechnology,* Vol.6, (1995), 652-656) which require accurate information on the atomic coordinates of target receptors may be used.

The aspects of the invention described herein which utilize the P450 structure *in silico* may be equally applied to both the 2C9 structure of Table 1, 2, 3 or 8 and the models of target P450 proteins obtained by other aspects of the invention. Thus having determined a conformation of a P450 by the method described above, such a conformation may be used in a computer-based method of rational drug design as described herein. In addition the availability of the structure of the P450 2C9 will allow the generation of highly predictive pharmacophore models for virtual library screening or compound design.

Accordingly, the invention provides a computer-based method for the analysis of the interaction of a molecular structure with a P450 structure of the invention, which comprises:
providing the structure of a P450 of the invention;
providing a molecular structure to be fitted to said P450 structure; and
fitting the molecular structure to the P450 structure.

The P450 structure of the invention may be the structure of any one of Table 1, 2, 3, 8 or 18 or selected coordinates thereof.

In an alternative aspect, the method of the invention may utilize the coordinates of atoms of interest of the P450 which are in the vicinity of a putative molecular structure binding region in order to model the pocket in which the structure binds. These coordinates may be used to define a space which is then analysed *"in silico".* Thus the invention provides a computer-based method for the analysis of molecular structures which comprises:
providing the coordinates of at least two atoms of a P450 structure of the invention ("selected coordinates");
providing a molecular structure to be fitted to said coordinates; and
fitting the structure to the selected coordinates of the P450.

In practice, it will be desirable to model a sufficient number of atoms of the P450 as defined by the coordinates of Table 1, 2, 3, 8 or 18 which represent a binding pocket. Binding pockets and other features of the interaction of P450 with co-factor are described in the accompanying example. Thus, in this embodiment of the invention, there will preferably be provided the coordinates of at least 5, preferably at least 10, more preferably at least 50 and even more preferably at least 100 selected atoms such as at least 500 or at least 1000 atoms of the P450 structure.

Although every different compound metabolised by P450 may interact with different parts of the binding pocket of the protein, the structure of this P450 allows the identification of a number of particular sites which are likely to be involved in many of the interactions of P450 with a drug candidate. The residues are set out in the accompanying example. Thus in this aspect of the invention, the selected coordinates may comprise coordinates of some or all of these residues.

In order to provide a three-dimensional structure of compounds to be fitted to a P450 structure of the invention, the compound structure may be modelled in three dimensions using commercially available software for this purpose or, if its crystal structure is available, the coordinates of the structure may be used to provide a representation of the compound for fitting to a P450 structure of the invention.

By "fitting", it is meant determining by automatic, or semi-automatic means, interactions between at least one atom of a molecular structure and at least one atom of a P450 structure of the invention, and calculating the extent to which such an interaction is stable. Interactions include attraction and repulsion, brought about by charge, steric considerations and the like. Various computer-based methods for fitting are described further herein.

More specifically, the interaction of a compound with P450 can be examined through the use of computer modelling using a docking program such as GRAM, DOCK, or AUTODOCK (see Walters et al., *Drug Discovery Today,* Vol.3, No.4, (1998), 160-178, and Dunbrack et al., *Folding and Design,* 2, (1997), 27-42). This procedure can include computer fitting of compounds to P450 to ascertain how well the shape and the chemical structure of the compound will bind to the P450.

Also computer-assisted, manual examination of the active site structure of P450 may be performed. The use of programs such as GRID (Goodford, *J. Med. Chem.,* 28, (1985), 849-857) - a program that determines probable interaction sites between molecules with various functional groups and an enzyme surface - may also be used to analyse the active site to predict, for example, the types of modifications which will alter the rate of metabolism of a compound.

Computer programs can be employed to estimate the attraction, repulsion, and steric hindrance of the two binding partners (i.e. the P450 and a compound).

If more than one P450 active site is characterized and a plurality of respective smaller compounds are designed or selected, a compound may be formed by linking the respective small compounds into a larger compound which maintains the relative positions and orientations of the respective compounds at the active sites. The larger compound may be formed as a real molecule or by computer modelling.

Detailed structural information can then be obtained about the binding of the compound to P450, and in the light of this information adjustments can be made to the structure or functionality of the compound, e.g. to alter its interaction with P450. The above steps may be repeated and re-repeated as necessary.

As indicated above, molecular structures which may be fitted to the P450 structure of the invention include compounds under development as potential pharmaceutical agents. The agents may be fitted in order to determine how the action of P450 modifies the agent and to provide a basis for modelling candidate agents which are metabolised at a different rate by a P450.

Molecular structures which may be used in the present invention will usually be compounds under development for pharmaceutical use. Generally such compounds will be organic molecules which are typically from about 100 to 2000 Da, more preferably from about 100 to 1000 Da in molecular weight. Such compounds include peptides and derivatives thereof, steroids, anti-inflammatory drugs, anti-cancer agents, anti-bacterial or antiviral agents, neurological agents and the like. In principle, any compound under development in the field of pharmacy can be used in the present invention in order to facilitate its development or to allow further rational drug design to improve its properties.

A single reductase provides several different isoforms of P450 with the electrons required in the catalytical cycle. As such, knowledge of the cytochrome P450 reductase (CPR) binding site on P450 and its characteristics present a means of altering the rate of catalysis, by mediating the P450 CPR interactions. The structure of 2C9 will allow the *in silico* identification of residues important in the P450 - CPR interface.

### (iii) Analysis and modification of compounds and metabolites

Where the primary metabolite of a potential or actual pharmaceutical compound is known, and this metabolite is generated by the action of P450, the structure of the agent and its metabolite may both be modelled and compared to each other in order to better determine residues of P450 which interact with the agent. In any event, the present invention provides a process for predicting potential pharmaceutical compounds with a desired activity which are metabolised by P450 at a rate different from a starting compound having the same desired activity, which method comprises:
fitting a starting compound to a P450 structure of the invention or selected coordinates thereof;
determining or predicting how said compound is metabolised by said P450 structure; and
modifying the compound structure so as to alter the interaction between it and the P450.

It would be understood by those of skill in the art that modification of the structure will usually occur *in silico,* allowing predictions to be made as to how the modified structure interacts with the P450.

Modification will be those conventional in the art known to the skilled medicinal chemist, and will include, for example, substitutions or removal of groups containing residues which interact with the amino acid side chain groups of a P450 structure of the invention. For example, the replacements may include the addition or removal of groups in order to decrease or increase the charge of a group in a test compound, the replacement of a charge group with a group of the opposite charge, or the replacement of a hydrophobic group with a hydrophilic group or vice versa. It will be understood that these are only examples of the type of substitutions considered by medicinal chemists in the development of new pharmaceutical compounds and other modifications may be made, depending upon the nature of the starting compound and its activity.

Although it is usually desired to alter a compound to prevent its metabolism by P450, or at least to reduce the rate at which P450 metabolises the compound, the present invention also includes developing compounds which are metabolised more rapidly than a starting compound, for example where such a compound blocks metabolism of another drug.

Where a potential modified compound has been developed by fitting a starting compound to the P450 structure of the invention and predicting from this a modified compound with an altered rate of metabolism, the invention further includes the step of synthesizing the modified compound and testing it in a in vivo or in vitro biological system in order to determine its activity and/or the rate at which it is metabolised.

The above-described processes of the invention may be iterated in that the modified compound may itself be the basis for further compound design.

### (iv) Fragment linking and growing.

The provision of the crystal structures of the invention will also allow the development of compounds which interact with the binding pocket regions of P450s (for example to act as inhibitors of a P450) based on a fragment linking or fragment growing approach.

For example, the binding of one or more molecular fragments can be determined in the protein binding pocket by X-ray crystallography. Molecular fragments are typically compounds with a molecular weight between 100 and 200 Da. This can then provide a starting point for medicinal chemistry to optimise the interactions using a structure-based approach. The fragments can be combined onto a template or used as the starting point for 'growing out' an inhibitor into other pockets of the protein. The fragments can be positioned in the binding pocket of the P450 and then 'grown' to fill the space available, exploring the electrostatic, van der Waals or hydrogen-bonding interactions that are involved in molecular recognition. The potency of the original weakly binding fragment thus can be rapidly improved using iterative structure-based chemical synthesis.

At one or more stages in the fragment growing approach, the compound may be synthesized and tested in a biological system for its activity. This can be used to guide the further growing out of the fragment.

Where two fragment-binding regions are identified, a linked fragment approach may be based upon attempting to link the two fragments directly, or growing one or both fragments in the manner described above in order to obtain a larger, linked structure which may have the desired properties.

### (v) Compounds.

Where a potential modified compound has been developed by fitting a starting compound to the P450 structure of the invention and predicting from this a modified compound with an altered rate of metabolism (including a slower, faster or zero rate), the invention further includes the step of synthesizing the modified compound and testing it in a in vivo or in vitro biological system in order to determine its activity and/or the rate at which it is metabolised.

Following identification of such a compound, it may be manufactured and/or used in the preparation, i.e. manufacture or formulation, of a composition such as a medicament, pharmaceutical composition or drug. These may be administered to individuals.

Thus, a compound identified by use of the present invention may be in the form of a pharmaceutical composition, medicament, drug or other composition comprising such a compound e.g. for treatment (which may include preventative treatment) of disease; a method comprising administration of such a composition to a patient, e.g. for treatment of disease; and such a compound used as an inhibitor in the manufacture of a composition for administration, e.g. for treatment of disease; and used in a method of making a pharmaceutical composition comprising admixing such an inhibitor with a pharmaceutically acceptable excipient, vehicle or carrier, and optionally other ingredients.

### Summary of Examples.

The invention is illustrated by the examples, which illustrate the invention as follows:
Example 1 shows the production of DNA encoding 2C9trunc, 2C9-FGloop, 2C9-FGloop K206E and 2C9P220.
Example 2 shows the expression of 2C9P220 and 2C9-FGloop in bacteria and the recovery of protein.
Example 3 shows quality assays of the proteins of example 2.
Example 4 shows crystallisation conditions used to obtain crystals of 2C9-FGloop.
Example 5 shows crystallisation conditions used to obtain crystals of 2C9P220.
Example 6 shows a further production of 2C9-FGloop and the mass spectrometry and activity data of the recovered protein.
Example 7 shows the production of crystals of 2C9-FGloop.
Example 8 shows the expression and recovery of 2C9-FGloop K206E and the mass spectrometry and activity data of the recovered protein, plus crystallisation of the protein.
Example 9 shows the crystallisation and structure analysis of 2C9-FGloop K206E at a 3Å resolution, as set out in Table 1.
Example 10 shows a further crystallisation of 2C9-FGloop K206E.
Example 11 shows the production of a higher resolution (2.6Å) structure of 2C9-FGloop K206E
Example 12 shows the production of a high resolution (3.1Å) structure of 2C9-FGloop.
Example 13 identifies residues of the P450 binding pocket and describes their use in the practice of the present invention.
Example 14 describes the use of modelling techniques using structures of the invention.
Example 15 outlines a docking experiment.
Example 16 shows the refinement of 2C9-FGloop K206E structure.
Example 17 shows the production of further 2C9 proteins.
Example 18 shows the production of 2C9 proteins.
Example 19 shows the activity of 2C9 Proteins of the invention.
Example 20 shows crystallisation of 2C9 proteins.
Example 21 describes 2C9-2C19 Chimeras.
Example 22 shows the production of 2C9-2C 19 chimeras.
Example 23 shows validation of 2C9-FGloop K206E.
Example 24 shows the activity of 2C9-2C 19 Chimeras.
Example 25 shows crystallisation of 2C9-2C19 chimeric proteins.
Example 26 shows homology Modelling of 2C19.
Example 27 shows homology modelling of 2C 18.
Example 28 shows homology modelling of 2C8.

### Example 1: Production of DNA encoding 2C9 proteins.

### Summary

Cytochrome P450 2C9 was targeted for crystallisation. Conversion of this intrinsic membranous protein to a more water-soluble form, by removal of the N-terminus trans-membrane domain was performed prior to crystallisation.

Several N-terminus truncations, largely described in the literature, have been used to produce N-truncated cytochrome P450s (including 2E1, 2D6, 2B1 and others). However, most of these N-terminal truncations failed to produce fully soluble proteins and in most cases, the truncated P450s still remained associated with membranes.

The membrane anchor domain MDSLVVLVLCLSCLLLLSLWRQSSGRGKL (SEQ ID NO:113) present in 2C9 (residues 2 to 29) was substituted by a short hydrophilic peptide MAKKTSSKGR (SEQ ID NO:114). The introduction of a highly charged polypeptide at the N-terminus of this protein was found to greatly decrease the membrane association of these proteins. It has also been found that the nature of the second codon in a lacZ expression system influences the level of expression (Looman et al, EMBO J., 6;2489-24992, 1987) and here alanine at position 2 provided good expression in *E. coli.*

Cytochrome P450 exhibits a high tendency to form large aggregates. The N-terminal deletion of cytochrome P450 has prevented aggregation and reduced polydispersity. This, in turn, facilitates the crystallisation of these proteins.

A four histidine tag was inserted at the C-terminus of 2C9 to help purification in high salt buffers.

Our preliminary results, using conditions from commercially available screening kits, indicated that the apo and native N-terminus truncated 2C9, 2C9trunc, did not produce any useful crystals. Thus the protein requires further modifications to promote crystallisation, and more importantly to promote production of useful crystals. Accordingly, the FG loop of the protein was considered for modification.

The design of the modification in the F-G loop was based on the published results on the crystallisation of the rabbit cytochrome P450 that indicated that the F and G helices were involved in the formation of a crystal contact. We predicted that the relative position of the F-G loop in the protein 2C9trunc could interfere with the ability of the F and G helices to constitute crystal contacts. It was proposed that the F-G loop, longer and more mobile than the counterpart found in the bacterial P450 BM3, may be stabilized or conformationally changed by six amino acid substitutions: Ile215Val, Cys216Tyr, Ser220Pro, Pro221Ala, Ile222Leu and Ile223Leu. In the resultant construct, 2C9-FGloop, the position of proline 220 is moved by one residue. The proline residue, often reported as initiating changes in secondary structure, may induce a conformational change in the F-G loop and facilitate the formation of crystal contacts. In the generation of the protein 2C9-P220, the proline is moved from position 221, as seen in 2C9 wild type to position 220 as seen in 2C 19 wild type. Thus the serine 220 was mutated to proline and proline 221 was mutated to threonine. The introduction of these two changes alone was sufficient to promote crystallisation. A single mutation of S220P, retaining the proline at 221 was also sufficient to get crystallisation.

In the generation of the protein 1424, the proline is moved from position 221, as seen in 2C9 wild type to position 222. This shows that the proline can be moved one amino acid either side of 221 to promote successful crystallisation.

We believe having a proline at 220 or 222, preferably proline 220 is a critical determinant for crystallisation of 2C9. In particular it is a critical determinant for obtaining apo crystals of 2C9. It is also important for obtaining diffraction quality crystals of 2C9. Residue 221 can be alanine, or threonine. It can also be proline or serine.

The mutagenesis of human 2C9 cytochrome P450 was performed by a variety of standard recombinant DNA techniques including cassette mutagenesis, site-directed mutagenesis or specific cloning protocols. For cassette mutagenesis, complementary oligonucleotides bearing the mutations were annealed and cloned, using natural restriction sites or sites that have been introduced by PCR mutagenesis into the P450 cDNA. The constructs were verified by restriction mapping followed by full sequencing. Other techniques are described herein or are well known as such to those of skill in the art.

### N-terminal truncation of P450

The expression vector pCWOri+, provided by Prof. F. W. Dahlquist, University of Oregon, Eugene, Oregon, USA, was used to express the truncated human cytochrome P450s in the *E. coli* strain XL1 Blue (Stratagene). A full-length cDNAs encoding cytochrome P450 2C9 was used as a template for PCR amplification, engineering the 5' terminus deletion, insertion of silent restriction sites and insertion of a four Histidine tag at the C-terminus.

A *NotI* restriction site (underlined) was introduced in 2C9 at position 87 by PCR amplification using the following 5'oligonucleotide:
5'-ATAAGAATGCGGCCGCCTGGCCCCACTCCTCTCCCAGTGATTGGAAATATC-3' (SEQ ID NO:115).
The 3' oligonucleotides:
5'-TGCGGTCGACTCAGTGGTGGTGGTGGACAGGAATGAAGCAGAGCTGGTAG-3' (SEQ ID NO: 116) with a *SalI* cloning site (underlined) and the four Histidine tag (italics) was used. A total of 30 cycles at 94 °C for 1 min, 52 °C for 1 min, and 72 °C for 2 min were followed by an extension of 10 min at 72 °C. The 1420-bp PCR fragment was double digested with *NotI*/*SalI* and purified by gel agarose elution and extraction.

The complementary oligonucleotides
5'-TATGGCTAAGAAAACGAGCTCTAAAGGGC-3'(SEQ ID NO:117) and
5'-GGCCGCCCTTTAGAGCTCGTTTTCTTAGCCA-3' (SEQ ID NO:118) with the *NdeI* and *NotI* overhang restriction sites (underlined) were designed to substitute the residues 2-29 of the native N terminus of human cytochrome P450 2C9 by the short AKKTSSKGR polypeptide. The oligonucleotides were annealed by mixing 10 µg of each Oligonucleotide in 100 µl of water, heating at 100°C for 5 min and slow cooling at room temperature.

The 1420-bp PCR fragment was mixed to the double stranded oligonucleotide and ligated in the vector pCWori+, previously digested with *NdeI* and *SalI.* An aliquot of the ligation product was used to transform *E. coli* XL1 Blue strain to yield the plasmid pCW-2C9trunc that encodes for the amino-terminal truncated 2C9.

The truncated 2C9 was used to make the proteins for further crystallisation experiments.

### Construction of 2C9-FGloop

The plasmid pCW-2C9trunc was used as template for the insertion of six amino acids substitutions, Ile215Val, Cys216Tyr, Ser220Pro, Pro221Ala, Ile222Leu, Ile223Leu in the FG loop. pCW-2C9trunc was digested by *NdeI* and *BamHI* restriction enzyme and the 579-bp corresponding to the 5' terminus of the P450 gene was purified by gel agarose extraction and elution. A double strand oligonucleotide designed to introduce the six amino acids substitution in the FG loop, was generated by annealing the following complementary oligonucleotides 5'-GATCCAG*GTCTAC*AATAATTTC*CCTGCTCTCCTT*GATTATTTC-3' (SEQ ID NO:119) and 5'-CCGGGAAATAATC*AAGGAGAGCAGG*GAAATTATTG*TAGAC*CTG-3' (SEQ ID NO:120) with the overhang *BamHI* and *XmaI* restriction sites (underlined) and the six mutated codons (italics). The 579-bp fragment and the double strand oligonucleotide were ligated in the vector pCW-2C9trunc, previously digested by *NdeI* and *Xmal.* An aliquot of the ligation was used to transform Xll Blue *E. coli* and yield the plasmid pCW-2C9-FGloop.

### Construction of 2C9-P220

2C9-P220 is a 2C9trunc mutant carrying the mutations S220P and P221 T. This mutant was made using the Stratagene Quikchange™ mutagenesis kit (catalogue number #200518), according to manufacturers instructions. The Quikchange™ mutagenesis method generates a mutated plasmid with staggered nicks and uses DpnI digestion to remove all parental DNA. Reactions were made incorporating 5.0 µL x10 reaction buffer, 5-50 ng pCW-2C9trunc plasmid DNA, 1.0 µL dNTP and 125 ng oligonucleotide primers as follows, with mutated bases shown in lowercase and the two amino acid change underlined:
5' CCAGATCTGCAATAATTTTcCgaCcACATTGATTACTTCCC 3' (SEQ ID NO:121)
5' GGGAAGTAATCAATGATgGtcGgAAAATTATTGCAGATCTGG 3' (SEQ ID NO: 122) Reactions were made to 50 µL with sterile water, 2.5U Pfu Turbo was then added and the reaction overlayed with 30 µL mineral oil. Thermocycling was then carried out as follows: 95°C, 30 sec (1 cycle), 95°C, 30 sec, 55°C, 1 min, 68°C 13.5 min (18 cycles) and finally a holding period at 4°C. A control reaction was also included with water in place of oligonucleotide primers.

Following thermocycling 10 U DpnI was added, under the level of the mineral oil, to each reaction. The reactions were then gently mixed followed by centrifugation in a bench top microcentrifuge, 1 min, 13,000 rpm and incubated at 37°C for 3 hr. Digested product (1 µL) was then used to transform 50 µL competent *E. coli* XL1-Blue cells. The whole transformation as then plated onto Luria agar plates containing 100 µg/ml carbenicillin, inverted, and incubated overnight at 37 °C. Colonies were isolated and the plasmid DNA pCW-2C9-P220 isolated and sequenced to check for the insertion of the correct mutation.

### Construction of 2C9-FGloop-K206E

The plasmid pCW-2C9-FGloop was used as a template for the substitution Lys206Glu (where the numbering is of the full length wild type 2C9, SwissProt: P11712, not that of SEQ ID NO:2 or 4). Primers were designed to lie across the region to be mutated;
5'-GGAAAAGTTGAATGAAAACATCGAGATTTTGAGCAGCCCCTGG-3' (SEQ ID NO:123)
5'-CCAGGGGCTGCTCAAAATCTCGATGTTTTCATTCAACTTTTCC-3' (SEQ ID NO:124)
where the mutated codon is shown in bold. These primers were then used in the protocol for Quikchange™ mutagenesis (Stratagene) which is briefly summarised.
Primers were resuspended to 125 ng/µl and used in a PCR reaction which elongated around the plasmid from the mutagenic primer. The template DNA was then digested using DpnI, a methylation specific restriction endonuclease which preferentially degrades the template due to its methylation. After DpnI treatment 1 µl of the resultant sample was transformed into *E. coli* XL1 Blue strain. Colonies were picked and sequenced. Plasmids containing the mutation were chosen and digested with the restriction endonucleases NdeI and SalI. The NdeI SalI DNA fragment corresponding to the coding sequence of the 2C9-FGloop K206E mutant was then subcloned into a pCW vector digested with NdeI and SalI. This served to remove any errors incorporated during the PCR phase of the Quickchange mutagenesis.

### Example 2: Expression of 2C9P220 and 2C9-FGloop.

### Bacteria expression

A single ampicillin resistant colony of XL1 blue cells was grown overnight at 37 °C in Terrific Broth (TB) with shaking to near saturation and used to inoculate fresh TB media. Bacteria were grown to an OD600nm =0.4 in 1 litre of TB broth containing 100 µg/ml of ampicillin at 37 °C at 185 rpm in 2 litre flask. The haem precursor delta aminolevulinic acid (80 mg/l) was added 30 min prior to induction with 1 mM isopropyl-β-D-thiogalactopyranoside (IPTG) and the temperature lowered to 30 °C. The bacterial culture was continued under agitation at 30 °C for 48 to 72 hours.

### (a) Protein purification

The cells were pelleted at 10000 g for 10 min and resuspended in a buffer containing 500 mM KPi, pH 7.4, 20 % glycerol, 10 mM mercaptoethanol, 0.1 % (v/v) of protease inhibitor cocktail (Calbiochem), 10 mM imidazole, 0.01 mg/ml DNase 1 and 5 mM MgSO₄.

The cells were lysed by passing twice through a Constant Systems Cell Homogeniser at 12000 psi. The cell debris was then removed by centrifugation at 70000 g at 4 °C for 30 min.

Detergent IGEPAL CA630 (Sigma) was added dropwise from a 10% stock solution to the lysate at a final concentration of 0.3% (v/v) and the lysate was incubated with previously washed NiNTA resin (Qiagen) overnight at 4 °C, using agitation. The protein bound-NiNTA resin was pelleted by centrifugation at 2000 g for 2 min at 4 °C. The resin was washed with 20 resin volumes of 500 mM KPi, pH 7.4, 20% glycerol, 10 mM mercaptoethanol, 10 mM imidazole, 1:1000 dilution of protease inhibitor cocktail, 0.3%(v/v) IGEPAL CA630 and the resin pelleted by centrifugation at 2000 xg for 2 min at 4 °C. The resin was then washed with 10 resin volumes of 500 mM KPi, pH 7.4, 20% glycerol, 10 mM mercaptoethanol, 20 mM imidazole, 0.1% (v/v) protease inhibitors, 0.3% IGEPAL CA630 and the resin recovered by centrifugation as described above. The washing step was repeated as described above with buffer containing 50 mM imidazole. The resin was packed into a column at 4 °C and the cytochrome P450 eluted with 500 mM KPi, pH 7.4, 20 % glycerol, 10 mM mercaptoethanol, 300 mM imidazole, 0.1% (v/v) of protease inhibitor cocktail, 0.3%(v/v) IGEPAL CA630.

### (b) An alternative method for protein purification is as follows:

The cells were pelleted at 10000 g for 10 min and resuspended in a buffer containing 500 mM KPi, pH 7.4, 20 % glycerol, 10 mM mercaptoethanol, 0.1 % (v/v) of protease inhibitor cocktail (Calbiochem), 0.01 mg/ml DNase 1 and 5 mM MgSO₄.

The cells were lysed by passing twice through a Constant Systems Cell Homogeniser at 12000 psi. The cell debris was then removed by centrifugation at 70000 g at 4°C for 30 min.

Detergent IGEPAL CA630 (Sigma) was added dropwise from a 10% stock solution to the lysate at a final concentration of 0.3% (v/v) and the lysate was incubated with previously washed NiNTA resin (Qiagen) overnight at 4°C, using agitation. The NiNTA resin was pelleted by centrifugation at 2000 g for 2 min at 4°C and washed, as described above, with 20 resin volumes of 500 mM KPi, pH 7.4, 20% glycerol, 10 mM mercaptoethanol, 50 mM glycine 0.1 % (v/v) protease inhibitors, 0.3% IGEPAL CA630, followed by washing with 10 resin volumes of 500 mM KPi, pH 7.4, 20% glycerol, 10 mM mercaptoethanol, 7.5 mM Histidine, 0.1% (v/v) protease inhibitors, 0.3% IGEPAL CA630. The resin was recovered by centrifugation between washing steps and then the resin was packed into a column at 4 °C. The protein was eluted with 500 mM KPi, pH 7.4, 20 % glycerol, 10 mM mercaptoethanol, 100 mM histidine, 0.1% (v/v) of protease inhibitor cocktail, 0.3%(v/v) IGEPAL CA630.

The cytochrome P450 obtained from the NiNTA column by either elution protocol was quickly desalted (<10 min) into 10 mM KPi, pH 7.4, 20% glycerol, 0.2 mM DTT, I mM EDTA using a HiPrep 26/10 desalting column (Pharmacia), at a flow rate of 5 ml/min and collecting 16 ml fractions. The desalted cytochrome P450 was directly applied to a CM Sepharose column (Pharmacia), previously equilibrated with 10 mM KPi, pH 7.4, 20% glycerol, 0.2 mM DTT, 1 mM EDTA. The following step elution was applied: wash with 10 column volumes of 10 mM KPi, pH 7.4, 20% glycerol, 0.2 mM DTT, I mM EDTA, wash with the above buffer with 75 mM KCl in order to remove any trace of detergent, then eluted with the above buffer with KCl concentration increased to 500 mM. The protein was concentrated up to 40 mg/ml using a microconcentrator for crystallization assays.

At this stage, the protein can be optionally further purified by running a gel filtration column. The concentrated P450 sample was applied on the top of a Superose 6 HR10/30 gel filtration column (Pharmacia) and eluted at 0.2 ml/min with buffer containing 100 mM KPi, pH 7.4, 300 mM KCl, 20% glycerol, 0.2 mM DTT. The protein was collected and concentrated up to 40 mg/ml, as described above, for crystallization and quality assays.

### Example 3: Quality Assays.

The quality of the final preparation of proteins from Example 2 was evaluated by:

### (a) SDS polyacrylamide gel electrophoresis.

This was performed using commercial gels (Nugen) followed by CBB (coomassie brilliant blue) staining according to the manufacturer's instructions. The purity as estimated by scanning a digital image of a gel was estimated to be at least 95%.

### (b) Gel filtration chromatography.

This was done using a Superose 6 HR10-30 column (Pharmacia) was performed to assess the aggregation state. The fractionation range for this column is 5x10³ to 5x10⁶ Da and is thus well adapted to the resolution of large complexes. The column was eluted at 0.2 ml/min with buffer containing 100 mM KPi, pH 7.4, 300 mM KCl, 20% glycerol, 0.2 mM DTT, 1 mM EDTA. 0.2 ml protein samples at a concentration of approximately 40 mg/ml were used. Absorbance at 280 nm was monitored and the peak was collected and analysed using dynamic light scattering.

### (c) Light scattering.

Samples (0.15 ml) collected after the CM Sepharose column and/or the gel filtration column were analysed by DLS in fluorimeter quartz cells at 90° using laser radiation at 830.3 nm. Data was collected using a log correlator with variable expansion spanning a wide dynamic range. All measurements were performed at 20 °C with samples collected immediately from the gel filtration column. A run was on average 10 runs of 10 seconds each. To obtain an estimation of the molecular weight, we used a frictional ratio of 1.26 and a partial specific volume of 0.726.

Samples prepared using our new method of purification possessed a good solubility and an absence of significant aggregation as shown by:
- the ratio of far channel extrapolation and measured average scattering was always between 0.999 and 1.003.
- the average count rate did not vary significantly, with approximately 1% standard deviation.
- analysis of the autocorrelation function using bi exponential fitting showed that 2C9 had an estimated Mr of approximately 180 KDa, i.e. it is an oligomer composed of no more than four subunits.
- good stability of the samples (over 24h) at 20°C.

Samples prepared by published protocols showed signs of a severe aggregation:
- large fluctuations of the scattered light intensity, with a standard deviation of more than 10%.
- analysis of the autocorrelation function showed very slow exponential decay an indication of the presence of large aggregates (Mr > 10⁶ Da), composed of a large number of P450 subunits. These samples also show a high degree of polydispersity.
- samples also showed further aggregation as a function of time.

These signs of severe aggregation in samples prepared by published methods were still present after sample filtration through 20 nm diameter pores or centrifugation at 200,000g for 30 min.

### (d) Mass Spectroscopy

Mass spectroscopy was performed on a single quadrupole mass spectrometer (platformII, Micromass UK Ltd.). Samples (25 µl of purified protein at 25-60 mg/ml) were dialyzed against 0.1 M ammonium acetate at 4 °C for 4 hours, using microcell dialyser (Pierce). The samples were diluted by a factor of 100 in 1:1 v/v methanol:0.1% aqueous formic acid and were then infused into the ionisation source of the mass spectrometer with a flow rate of 20 µl/min.

The mass spectrometer was fitted with a standard electrospray ionisation source. Positive electrospray ionisation was affected with a probe tip voltage of 3.5 kV, and a counter electrode voltage of 0.5 kV. Nitrogen was employed as both the nebulising and the drying gas, with a nebulising gas flow rate of 20 L/hr and a drying flow rate of 200L/hr. The sampling cone voltage was maintained at 40V. Data were acquired over the appropriate m/z range and were subsequently processed by manual identification of the components wherever possible, followed by transposition onto a true molecular mass scale for more facile identification using Maximum Entropy processing techniques. The mass accuracy obtained for the analysed protein was 0.01% of the mass.

### (e) Functionality assays

Activity assays on P450 2C9 were performed in a 96-well plate assay format with a Fluoroscan Ascent FL Instruments (Labsystem), using the methoxy-4-(trifluoromethyl)-coumarin as a fluorescent substrate.

Fifteen pmoles of P450 were reconstituted with 0.1 unit of purified human oxidoreductase, in presence of 140 µM of substrate methoxy-4-(trifluoromethyl)-coumarin, a NADPH regenerating system that includes 0.15 mM NADP⁺, 0.38 mM Glucose-6-phosphate and 2.9 unit/ml glucose-6-phosphate dehydrogenase in 170 µl final volume of 25 mM KPi, pH 7.4, 0.38 mM MgCl₂. Incubations were performed at 37°C for several minutes and 7-hydroxy -4-(trifluoromethyl)-coumarin was used as metabolite standard to determinate the metabolic rate. The excitation and emission wavelengths used were respectively 409 and 530nm.

### Example 4: Crystallisation conditions for 2C9-FGloop.

Crystallization of P450 2C9-FGloop was achieved at 10-60 mg/ml protein in 10 mM Potassium phosphate, pH 7.4; 0.5 M KCl; 0.2 mM DTT; 1.0 mM EDTA; 20% glycerol against the conditions listed below. Crystals grew over a two week period in the morphologies indicated.

### Appearance: Needles and rods

Cell dimensions: a=161 Å, b= 161 Å, c=110 Å, α=90°, β=90°, γ=120°.
Space Group: P321

0.2 M Sodium Fluoride, 20% PEG 3350
0.2 M Potassium Fluoride, 20% PEG 3350
0.2 M Ammonium Fluoride, 20% PEG 3350
0.2 M Lithium Chloride, 20% PEG 3350
0.2 M Magnesium Chloride, 20% PEG 3350
0.2 M Sodium Chloride, 20% PEG 3350
2.0 M Sodium Chloride, 10% PEG 6K
0.2 M Calcium Chloride, 20% PEG 3350
0.2 M Potassium Chloride, 20% PEG 3350
0.2 M Ammonium Chloride, 20% PEG 3350
0.2 M Lithium Nitrate, 20% PEG 3350
0.2 M Magnesium Nitrate, 20% PEG 3350
0.2 M Sodium Nitrate, 20% PEG 3350
0.2 M Potassium Nitrate, 20% PEG 3350
0.2 M Ammonium Nitrate, 20% PEG 3350
0.2 M Magnesium Formate, 20% PEG 3350
0.2 M Sodium Formate, 20% PEG 3350
0.2 M Potassium Formate, 20% PEG 3350
0.2 M Ammonium Formate, 20% PEG 3350
0.2 M Lithium Acetate, 20% PEG 3350
0.2 M Magnesium Acetate, 20% PEG 3350
0.2 M Sodium Acetate, 20% PEG 3350
0.2 M Sodium Acetate pH 4.6, 10-20% PEG 4000
0.2 M Calcium Acetate, 20% PEG 3350
0.2 M Potassium Acetate, 20% PEG 3350
0.2 M Ammonium Acetate, 20% PEG 3350
0.2 M Ammonium Acetate pH 4.6, 10-20% PEG 4000
0.2 M Sodium Sulfate, 20% PEG 3350
0.2 M Magnesium Sulfate, 20% PEG 3350
0.2 M Potassium Sulfate, 20% PEG 3350
0.2 M Ammonium Sulfate, 20% PEG 3350
0.2 M di-Sodium Tartrate, 20% PEG 3350
0.2 M Potassium Sodium Tartrate, 20% PEG 3350
0.2 M di-Ammonium Tartrate, 20% PEG 3350
0.2 M Sodium dihydrogen Phosphate, 20% PEG 3350
0.2 M di-Sodium hydrogen phosphate dihydrate, 20% PEG 3350
0.2 M Potassium dihydrogen Phosphate, 20% PEG 3350
0.2 M di-Potassium hydrogen Phosphate, 20% PEG 3350
0.2 M Ammonium dihydrogen Phosphate, 20% PEG 3350
0.2 M di-Ammonium hydrogen Phosphate, 20% PEG 3350
0.2 M tri-Lithium Citrate, 20% PEG 3350
0.2 M tri-Sodium Citrate, 20% PEG 3350
0.2 M tri-Potassium Citrate, 20% PEG 3350
15% PEG 1500
30% PEG 1500
0.1 M MES pH 6.0, 5-20% PEG 6000
0.1 M MES pH 6.5, 12% PEG 20,000
0.1 M Citric acid pH 5.0, 10% PEG 6000
0.1 M Sodium Cacodylate, pH 6.6, 10-25% PEG 1500
0.1 M Sodium Cacodylate, pH 6.4-6.8, 0.05-0.2 M Magnesium acetate, 10-20% PEG 8000
0.05-0.1 M Potassium dihydrogen phosphate, 10-20% PEG 8000
0.2 M Potassium dihydrogen phosphate, 20% PEG 3000
0-0.2 M Sodium Chloride, 0.1 M Potassium dihydrogen phosphate/di-Sodium hydrogen phosphate pH 5.8-6.6, 5-20% PEG 8000
0-0.2 M Sodium Chloride, 0.1 M Potassium dihydrogen phosphate/di-Sodium hydrogen phosphate pH 5.8-6.6, 20% PEG 1000
0-0.2 M Sodium Chloride, 0.1 M Potassium dihydrogen phosphate/di-Sodium hydrogen phosphate pH 5.8-6.6, 20% PEG 3350.
0-0.2 M Sodium Chloride, 0.1 M Potassium dihydrogen phosphate/di-Sodium hydrogen phosphate pH 5.8-6.6, 15-20% PEG 5000MME
0.5 M Ammonium Sulfate, 0.1 M HEPES pH 7.5, 30% 2-Methyl-2,4-pentanediol
0.01 M Nickel (II) Chloride, 0.1 M Tris pH 8.5, 20% PEG MME 2000
0.05-0.2 M Calcium Acetate, 0.1 M Tris HCl pH 7.0-7.6, 10-22.5% PEG 3000
0.1 M phosphate-citrate, pH 4.2, 0.05 M Lithium sulphate, 20% PEG 1000
0.025-0.25 M di-potassium hydrogen phosphate, pH 7.0-7.8
0.2 M di-potassium hydrogen phosphate, pH 8.4, 17.5% PEG 3350
0.2 M Ammonium iodide, 20% PEG 3350
0.2 M di-Ammonium hydrogen citrate, 20% PEG 3350
0.2 M Lithium sulphate, 20% PEG 3350
0.05-0.2 M K2HPO4, 10% PEG 4000
0.05-0.2 M K2HPO4, 6.25%-20% PEG 3350
0.2 M K2HPO4, 3.75-25% PEG 3350
0.2-0.35 M K2HPO4, 20% PEG 3350
0.1-0.15 M K2HPO4, 10% MPEG 2000
0.2M K2HP04, 3.75-10 % MPEG 2000
0.5 M K2HPO4, 10% MPEG 2000
0.1-0.15 M K2HPO4, 10% PEG 1000
0.2 M K2HPO4, 3.75-10% PEG 1000
0.5 M K2HPO4, 10% PEG 1000
0.1M Citrate-HCl pH 5.6, 20% PEG 3000
0.1 M Tris-HCl pH 7.0, 20% MPEG 2000
0.1 M HEPES pH 7.5, 0.2 M sodium chloride, 20% PEG 3000
0.1 M Imidazole-HCl pH 8.0, 0.2 M Calcium acetate, 10% PEG 8000
0.1 M Imidazole-HCl pH 8.0, 10% Iso-Propanol
0.1 M Imidazole-HCl pH 6.5, 0.5 M Sodium acetate
0.1 M Sodium cacodylate pH 6.6, 20% PEG 3350
0.1 M Citrate-HCl pH 5.6, 10% PEG 4000, 10% Isopropanol
0.1 M Tris-HCl pH 7.0-7.6, 0.1-0.2 M Calcium acetate, 15-20% PEG 3000
0.1 M phosphate-citrate pH 4.2, 0.2 M Lithium sulphate, 10% 2-propanol
0.1 M citrate pH 5.5, 0.2 M Lithium sulphate, 15% ethanol
0.1 M HEPES pH 7.5, 0.2 M Magnesium chloride, 15% ethanol
20% PEG 300, 10% Glycerol, 0.1 M Tris pH8.5, 5% PEG 8000

### Example 5: Crystallisation conditions for 2C9P220.

Crystallization of P450 2C9P220 was achieved at 10-60 mg/ml protein in 10 mM Potassium phosphate, pH 7.4; 0.5 M KCl; 0.2 mM DTT; 1.0 mM EDTA; 20% glycerol against the conditions listed below. Crystals grew over a two week period in the morphologies indicated.

### Appearance: Spherical clusters

0.1 M Tris-HCl pH 8.5, 0.2 M sodium acetate, 15% PEG 4000
0.1 M Tris-HCl pH 8.5, 4% PEG 8000
0.1 M Tri-Sodium Citrate Dihydrate pH 5.6, 10% Iso-PropanolPEG 4000
0.1 M HEPES pH 7.5, 0.2 M sodium chloride, 20% PEG 3000
0.1 M Na/K phosphate pH 6.2, 10% PEG 3000
0.1 M Tris pH 7.0, 0.2 M calcium acetate, 20% PEG 3000
0.1 M Tris pH 8.5, 20% PEG 1000
0.1 M HEPES pH 7.5, 0.2 M sodium chloride, 30% PEG 400
0.2M di-Sodium tartrate, 20% PEG 3350
0.2 M di-Sodium hydrogen phosphate dihydrate, 20% PEG 3350
0.2 M di-Potassium hydrogen phosphate, 20% PEG 3350
0.2 M tri-Lithium citrate, 20% PEG 3350
0.2 M tri-Sodium citrate, 20% PEG 3350
0.2 M tri-Potassium citrate, 20% PEG 3350
0.1M Tris-HCl pH 7.0, 0.2 M Calcium acetate, 20% PEG 3000
0.2 M K2 H P04, 15% PEG 3350
0.2M K2 H P04, 15% PEG 3350
0.1 M Tris-HCl pH 7.2, 0.2 M Calcium acetate, 20% PEG 3000
0.1 M Tris-HCl pH 7.2, 0.2 M Calcium acetate, 15% PEG 3000
0.2M K2 H PO4,17.5% PEG 3350
0.2 M K2 H PO4, 20%PEG 3350
0.3M K2 H PO4,20%PEG 3350
0.2 M K2 H PO4, 22.5% PEG 3350
0.2 M K2 H PO4, 25% PEG 3350
0.1M Tris-HCl pH 7.6, 0.2 M Calcium acetate, 20% PEG 3000
0.1 M Tris-HCl pH 7.6, 0.2 M Calcium acetate, 15% PEG 3000
0.1 M Tri-Sodium Citrate Dihydrate pH 5.0, 5% PEG 4000
0.1 M HEPES 7.0, 5% PEG 4000
0.1 M Tris pH 8.0, 5%-15% PEG 4000
0.1 M Bis-Tris Propane pH 9.0, 5%-10% PEG 4000

### Example 6: Further production of 2C9-FGloop.

2C9-FGloop was prepared in, and recovered from, a bacterial expression system as described in Example 2(a) above, and subject to further analysis by mass spectroscopy and an activity assay.

### Mass Spectroscopy

Mass spectroscopy was performed using a Bruker "BioTOF" electrospray time of flight instrument. Samples were either diluted by a factor of 1000 straight from storage buffer into methanol/water/formic acid (50:48:2 v/v/v), or subjected to reverse phase HPLC separation using a C4 column. Calibration was achieved using Bombesin and angiotensin I using the 2+ and 1 + charge state. Data were acquired between 200 and 2000*m*/*z* range and were subsequently processed using Bruker's X-mass program. Mass accuracy was typically below 1 in 10 000.

| | |
|---|---|
| Mass spec of 2C9-FGloop: | 53967 Da (observed) |
| | 53963.72 Da (predicted) |

### Functionality assays

Activity assays on P450 2C9 were performed in a 96-well plate assay format with a Fluoroscan Ascent FL Instruments (Labsystem), using the methoxy-4-(trifluoromethyl)-coumarin as a fluorescent substrate.

Fifteen pmoles of P450 were reconstituted with 0.1 unit of purified human oxidoreductase, in presence of 140 µM of substrate methoxy-4-(trifluoromethyl)-coumarin, a NADPH regenerating system that includes 0.15 mM NADP⁺, 0.38 mM Glucose-6-phosphate and 2.9 unit/ml glucose-6-phosphate dehydrogenase in 170 µl final volume of 25 mM KPi, pH 7.4, 0.38 mM MgCl₂. Incubations were performed at 37°C for several minutes and 7-hydroxy-4-(trifluoromethyl)-coumarin was used as metabolite standard to determinate the metabolic rate. The excitation and emission wavelengths used were respectively 409 and 530nm. The activity of 2C9-FGloop was 0.110 pmol/min/pmol P450 with 2C9 substrate.

### Example 7: Crystals of 2C9-FGloop.

Crystals of the 2C9-FGloop were grown using the hanging drop vapour diffusion method. Protein from example 6 at 40mg/ml in 10mM Kpi pH 7.4, 0.5 M KCl, 2mM DTT, 1 mM EDTA, 20% glycerol, was mixed in a 1:1 ratio, using 0.5 µl drops, with a reservoir solution. The crystals of 2C9-FGloop grew over a reservoir solution containing 0.1 M Tris-HCl, pH 8.8; 15% PEG 400; 5% PEG 8000; 10% glycerol. Crystals formed within 1-7 days at 25°C, and had morphologies of hexagonal needles and rods. In a first experiment, a first crystal ("1"), was found to have approximate cell dimensions of 161 Å, 161 Å, 110 Å, 90°, 90°, 120°. In a second experiment, a second crystal ("2"), was found to have approximate cell dimensions of 164 Å, 164 Å, 111 Å, 90°, 90°, 120°. This illustrates a typical range of variation within the 5% variability mentioned above.

The crystals were flash frozen in liquid nitrogen, using 80% reservoir solution, 10% PEG 400 and 10% glycerol as a cryoprotectant.

Data was collected from a 2C9-FGloop crystal to 3.3 Å resolution at beamline ID14.1 (wavelength 0.933 Å) at the European Synchrotron Radiation Source using a Quantum4 CCD detector from a single crystal at 100K. The crystals belong to spacegroup P321. Crystal 1 was found to have cell dimensions 161.35 Å, 161.35 Å, 110.75 Å, 90°, 90°, 120°; in the case of crystal 2 the dimensions were 163.95 Å, 163.95 Å, 111.06 Å, 90°, 90°, 120° and the data were collected to 3.0 Å for the crystal.

Coordinates of Table 1 or 2 can be used to solve the structure of 2C9-FGloop by molecular replacement.

### Example 8: Crystallisation and structure analysis of 2C9-FGloop K206E.

*E. coli* transformed with the 2C9-FGloop K206E vector described above were grown and described in Example 2.

### Protein Purification

The cells were pelleted at 10000 g for 10 min and resuspended in a buffer containing 500 mM KPi, pH 7.4, 20 % glycerol, 10 mM mercaptoethanol, 0.1 % (v/v) of protease inhibitor cocktail (Calbiochem), 10 mM imidazole, 40U/ml DNase 1 and 5 mM MgSO₄.

The cells were lysed by passing twice through a Constant Systems Cell Homogeniser at 12000 psi. The cell debris was then removed by centrifugation at 70000 g at 4°C for 30 min.

Detergent IGEPAL CA630 (Sigma) was added dropwise from a 10% stock solution to the lysate at a final concentration of 0.3% (v/v) and the lysate was incubated with previously washed NiNTA resin (Qiagen) overnight at 4°C, using agitation. The protein bound-NiNTA resin was pelleted by centrifugation at 2000 g for 2 min at 4°C. The resin was washed with 20 resin volumes of 500 mM KPi, pH 7.4, 20% glycerol, 10 mM mercaptoethanol, 10 mM imidazole, 1:1000 dilution of protease inhibitor cocktail, 0.3%(v/v) IGEPAL CA630 and the resin pelleted by centrifugation at 2000 xg for 2 min at 4°C. The resin was then washed with 10 resin volumes of 500 mM KPi, pH 7.4, 20% glycerol, 10 mM mercaptoethanol, 20 mM imidazole, 0.1% (v/v) protease inhibitors, 0.3% IGEPAL CA630 and the resin recovered by centrifugation as described above.

The resin was packed into a column at 4°C and the cytochrome P450 eluted with 500 mM KPi, pH 7.4, 20 % glycerol, 10 mM mercaptoethanol, 300 mM imidazole, 0.1% (v/v) of protease inhibitor cocktail, 0.3%(v/v) IGEPAL CA630.

The cytochrome P450 obtained from the NiNTA column by either elution protocol was quickly desalted into 10 mM KPi, pH 7.4, 20% glycerol, 2.0 mM DTT, 1 mM EDTA using a HiPrep 26/10 desalting column (Pharmacia), at a flow rate of 5 ml/min and collecting 17 ml fractions.

The desalted cytochrome P450 was directly applied to a CM Sepharose column (Pharmacia), previously equilibrated with 10 mM KPi, pH 7.4, 20% glycerol, 2.0 mM DTT, 1 mM EDTA. The following step elution was applied: wash with 10 column volumes of 10 mM KPi, pH 7.4, 20% glycerol, 2.0 mM DTT, 1 mM EDTA, wash with the above buffer with 75 mM KCl in order to remove any trace of detergent, then eluted with the above buffer with KCl concentration increased to 500 mM.

The protein was concentrated up to 40 mg/ml using a microconcentrator for crystallization assays. To characterize the protein, the quality of the final preparation was evaluated by:
*(a) SDS polyacrylamide gel electrophoresis*
This was performed using commercial gels (Nugen) followed by CBB staining according to the manufacturer's instructions. The purity as estimated by scanning a digital image of a gel was estimated to be at least 95%.
*(b) Mass Spectroscopy*
Mass spectroscopy was performed using a Bruker "BioTOF" electrospray time of flight instrument. Samples were either diluted by a factor of 1000 straight from storage buffer into methanol/water/formic acid (50:48:2 v/v/v), or subjected to reverse phase HPLC separation using a C4 column. Calibration was achieved using Bombesin and angiotensin I using the 2+ and 1+ charge state. Data were acquired between 200 and 2000*m*/*z* range and were subsequently processed using Bruker's X-mass program. Mass accuracy was typically below 1 in 10 000.

| | |
|---|---|
| Mass spec of 2C9-FGloop-K206E: | 53966 Da (observed) |
| | 53964.67 Da (predicted) |

*(c) Functionality assays*
Activity assays on P450 2C9 were performed in a 96-well plate assay format with a Fluoroscan Ascent FL Instruments (Labsystem), using the methoxy-4-(trifluoromethyl)-coumarin as a fluorescent substrate.

Fifteen pmoles of P450 were reconstituted with 0.1 unit of purified human oxidoreductase, in presence of 140 µM of substrate methoxy-4-(trifluoromethyl)-coumarin, a NADPH regenerating system that includes 0.15 mM NADP⁺, 0.38 mM Glucose-6-phosphate and 2.9 unit/ml glucose-6-phosphate dehydrogenase in 170 µl final volume of 25 mM KPi, pH 7.4, 0.38 mM MgCl₂. Incubations were performed at 37°C for several minutes and 7-hydroxy -4-(trifluoromethyl)-coumarin was used as metabolite standard to determinate the metabolic rate. The excitation and emission wavelengths used were respectively 409 and 530 nm. The activity of the 2C9-FGloop-K206E was 0.083 pmol/min/pmol P450 with 2C9 substrate.

### Crystallization of 2C9-FGLoop-K206E

Crystals of the 2C9-FGloop-K206E were grown using the hanging drop vapour diffusion method. Protein at 40mg/ml in 10mM Kpi pH 7.4, 0.5 M KCl, 2mM DTT, 1mM EDTA, 20% glycerol, was mixed in a 1:1ratio, using 0.5 µl drops, with a reservoir solution. The crystals of 2C9-FGloop-K206E grew over a reservoir solution containing 0.2 M dibasic potassium phosphate and 20% PEG 3350 (Alternative conditions were also used, which were 0.1 M Tris-HCl, pH 8.5; 0.2 M LiS04; 15% PEG 4000). Crystals formed within 1-7 days at 25°C, and had morphologies of hexagonal needles and rods. The approximate cell dimensions of the crystals were 165 Å, 165 Å, 112 Å, 90°, 90°, 120°. The crystals were flash frozen in liquid nitrogen, using 80% reservoir solution, 10% PEG 400 and 10% glycerol as a cryoprotectant.

### Example 9: Structure of 2C9-FGloop K206E.

Data was collected from a 2C9-FGloop-K206E crystal (prepared as described in Example 8) to 3.0 Å resolution at beamline ID14.1 (wavelength 0.933 Å) at the European Synchrotron Radiation Source using a Quantum4 CCD detector from a single crystal at 100K. A total of 90 one degree oscillation images were collected and processed using MOSFLM 6.11 (Leslie, A. G. W. (1992). *Jnt CCP4*/*ESF-EACMB Newslett. Protein Crystallogr.* 26), scaled using SCALA 4.1, and reduced using the CCP4 suite of programs (Collaborative Computational Project, Number 4, (1994). *The CCP4 suite: programs for protein crystallography. Acta Cryst.* D50, 760-763).

**Table of data statistics**

| | | |
|---|---|---|
| Resolution | 15-3.0 Å | 3.16-3.0 Å |
| Completeness (%) | 99.4 | 98.7 |
| Multiplicity | 5.2 | 4.8 |
| I/Sigma(I) | 3.5 | 1.3 |
| Rmerge (%) | 12.7 | 54.2 |

The crystals belong to spacegroup P321 and have cell dimensions 165.46 Å, 165.46 Å, 111.70 Å, 90°, 90°, 120°. There are two copies in the asymmetric unit, and the crystals have a solvent content of 68%. The structure was solved by molecular replacement using the 2C5 structure (pdbid 1DT6) (Williams, P A; Cosme, J; Sridhar, V; Johnson, E F; McRee, D E, *Molecular Cell, Volume 5, Issue 1, January 2000,Pages 121-131)* and the program AMORE (Navaza, J. (1994). *AMoRe: an automated package for molecular replacement. Acta Cryst.* A**50**, 157-163), giving a correlation coefficient of 67.8% and an R-factor of 38.9%. The coordinates of the structure are set out in Table 1. The two copies in the asymmetric unit are related by a rotation of 145° about the Z-axis. The initial maps (both averaged and unaveraged) were relatively clean, and containing unmistakable electron density for the heme group which was omitted from the search model. This solution was using as a starting point for refinement using the program CNX (*ibid).*

### Example 10: Further crystallisation of 2C9-FGloop K206E.

### Bacteria Expression

A single ampicillin resistant colony of XL1 blue cells transformed with the 2C9-FGloop K206E-expressing plasmid described above was grown overnight at 37°C in Terrific Broth (TB) with shaking to near saturation and used to inoculate fresh TB media. Bacteria were grown to an OD600nm = 0.4 in 1 litre of TB broth containing 100 µg/ml of ampicillin at 37°C at 185 rpm in 2 litre flask. The heme precursor delta aminolevulinic acid (80 mg/l) was added 30 min prior to induction with 1 mM isopropyl-β-D-thiogalactopyranoside (IPTG) and the temperature lowered to 25°C. The bacterial culture was continued under agitation at 25°C for 72 hours.

### Protein Purification

The cells were pelleted at 10000 g for 10 min and resuspended in a buffer containing 500 mM KPi, pH 7.4, 20% glycerol, 10 mM mercaptoethanol, 0.1 % (v/v) of protease inhibitor cocktail (Calbiochem), 10 mM imidazole, 40 U/ml DNase 1 and 5 mM MgSO₄.

The cells were lysed by passing twice through a Constant Systems Cell Homogeniser at 10000 psi. The cell debris was then removed by centrifugation at 22000 x g at 4°C for 30 min.

Detergent IGEPAL CA630 (Sigma) was added dropwise from a 10% stock solution to the lysate at a final concentration of 0.3% (v/v) and the lysate was incubated with previously washed NiNTA resin (Qiagen) overnight at 4°C, using agitation. The protein bound-NiNTA resin was pelleted by centrifugation at 2000 g for 2 min at 4°C. The resin was washed with 30 resin volumes of 500 mM KPi, pH 7.4, 20% glycerol, 10 mM mercaptoethanol, 10 mM imidazole, 1:1000 dilution of protease inhibitor cocktail, 0.3%(v/v) IGEPAL CA630 and the resin pelleted by centrifugation at 2000 xg for 2 min at 4°C. The resin was then washed with 15 resin volumes of 500 mM KPi, pH 7.4,20% glycerol, 10 mM mercaptoethanol, 20 mM imidazole, 0.1% (v/v) protease inhibitors, 0.3% IGEPAL CA630 and the resin recovered by centrifugation as described above.

The resin was packed into a column at 4°C and the cytochrome P450 eluted with 500 mM KPi, pH 7.4, 20 % glycerol, 10 mM mercaptoethanol, 300 mM imidazole, 0.1% (v/v) of protease inhibitor cocktail, 0.3%(v/v) IGEPAL CA630.

The cytochrome P450 obtained from the NiNTA column was quickly desalted into 10 mM KPi, pH 7.4, 20% glycerol, 2.0 mM DTT, 1 mM EDTA using a HiPrep 26/10 desalting column (Pharmacia), at a flow rate of 5 ml/min.

The desalted cytochrome P450 was directly applied to a CM Sepharose column (Pharmacia), previously equilibrated with 10 mM KPi, pH 7.0, 20% glycerol, 2.0 mM DTT, 1 mM EDTA. The following step elution was applied: wash with 20 column volumes of 10 mM KPi, pH 7.0, 20% glycerol, 2.0 mM DTT, 1 mM EDTA, wash with the above buffer with 75 mM KCl in order to remove any trace of detergent, then eluted with the above buffer with KCl concentration increased to 500 mM.

The protein was concentrated up to 40 mg/ml using a microconcentrator for crystallization assays.

### Crystallization of 2C9-FGloop K206E

Crystals of the 2C9-FGloop-K206E were grown using the hanging drop vapour diffusion method. Protein at 40 mg/ml in 10mM Kpi pH 7.0, 0.5 M KCl, 2mM DTT, 1mM EDTA, 20% glycerol, was mixed in a 1:1 ratio, using 0.5 µl drops, with a reservoir solution. The crystals of 2C9-FGloop-K206E were grown over a reservoir solution containing: 0.1 M Tris-HCl pH 8.4, 15% PEG 400, 5% PEG 8000, 10% glycerol.

Rod shaped crystals formed within 1 day at 25°C. The crystals were flash frozen in liquid nitrogen, using the reservoir solution as a cryoprotectant. The approximate cell dimensions of the crystals were 164.9 Å, 164.9 Å, 111.1 Å, α = 90°, β = 90°, γ = 120°.

### Example 11: Production of a 2.6Å resolution structure of 2C9-FGloop K206E

Data was collected to 2.6 Å resolution from a crystal of 2C9-FGloop-K206E crystal (prepared as described in Example 4) at beam line 14.1 at the European Synchrotron Radiation Facility, using a Quantum4 CCD detector from a single crystal at 100 K. The crystal was grown against a reservoir solution of 0.1M Tris pH 8.4, 15% PEG 400, 5% PEG 8000, 10% Glycerol, and was frozen directly from the reservoir solution. A total of 50 images were collected and processed using MOSFLM (Leslie, A. G. W. (1992). *Jnt CCP4*/*ESF-EACMB Newslett. Protein Crystallogr.* 26), scaled using SCALA and reduced using the CCP4 suite of programs (Collaborative Computational Project, Number 4, (1994). *The CCP4 suite: programs for protein crystallography. Acta Cryst.* D50, 760-763).

**Table of data statistics**

| | | |
|---|---|---|
| Resolution | 50-2.6 Å | 2.74-2.60 Å |
| Completeness | 96.5% | 84.3% |
| Multiplicity | 2.6 | 2.0 |
| I/ Sigma I | 6.8 | 1.2 |
| R merge | 8.7 | 57.0 |

This data was used in refinement, using the model generated by the refinement against the initial 3.0 Å data, to generate the coordinates of Table 2. A consistent set of 5% of the reflections was flagged for Free R calculation, and extended to the higher resolution. The refinement was continued using the programs CNX (Brunger et al., *Current Opinion in Structural Biology,* Vol. 8, Issue 5, October 1998, 606-611, and commercially available from Accelerys, San Diego, CA) and REFMAC (Collaborative Computational Project, Number 4, (1994). *The CCP4 suite: programs for protein crystallography. Acta Cryst.* **D50,** 760-763), to an R factor of 21.9% and an R free factor 25.0%.

### Example 12: Structure of 2C9-FGloop.

Data were collected from a 2C9-FGloop crystal 2 (prepared as described in Example 7) to 3.1 Å resolution at beamline ID 14.1 (wavelength 0.933 A) at the European Synchrotron Radiation Source using a Quantum4 CCD detector from a single crystal, frozen directly from the crystallisation solution (0.1 M Tris-HCl pH 8.8, 15% PEG 400, 5% PEG 8000, 10% glycerol) at 100K. The crystal belong to space group P321. Crystal 2 was found to have cell dimensions 163.95 Å, 163.95 Å, 111.06 Å, 90°, 90°, 120°.

A total of 100 degrees of data were collected, processing using MOSFLM, scaled using SCALA and reduced further using the CCP4 suite of programs. The structure of 2C9-FGloop was solved by molecular replacement using the program AMORE and the 2.6 Å 2C9-FGloop-K206E structure (Table 2) as a search model. The structure was refined using strict non-crystallographic symmetry using the program CNX to generate the coordinates of Table 3. The final structure has an R factor of 26.8% and a Free R factor of 29.8% for all data between 30 and 3.1 Å.

**Table of data statistics:**

| Resolution | R merge | Completeness | Mult | I/Sig I |
|---|---|---|---|---|
| 9.80 | 0.041 | 96.8 | 3.0 | 15.0 |
| 6.93 | 0.056 | 99.8 | 3.1 | 8.0 |
| 5.66 | 0.101 | 99.8 | 3.1 | 6.5 |
| 4.90 | 0.113 | 99.8 | 3.1 | 5.3 |
| 4.38 | 0.117 | 98.4 | 2.8 | 5.4 |
| 4.00 | 0.118 | 92.1 | 2.4 | 5.2 |
| 3.71 | 0.141 | 80.4 | 2.0 | 4.1 |
| 3.47 | 0.183 | 71.7 | 1.8 | 1.4 |
| 3.27 | 0.242 | 65.1 | 1.7 | 2.4 |
| 3.10 | 0.374 | 58.7 | 1.7 | 1.7 |
| Overall | 0.099 | 81.9 | 2.4 | 5.0 |

### Example 13: Identification and use of P450 binding pocket residues.

The crystal structure for 2C9 has for the first time allowed the precise identification of all the residues that line the binding site of the enzyme (Table 4). Some residues proposed to be in the catalytic site by a variety of sources can now be shown not to be binding pocket residues but residues that hold the catalytic residues in place.

**Table 4: All residues lining the 2C9 binding pocket**

| | | | | | | | | | | | |
|---|---|---|---|---|---|---|---|---|---|---|---|
| ARG | 97 | GLY | 98 | ILE | 99 | PHE | 100 | LEU | 102 | ALA | 103 |
| ALA | 106 | ASN | 107 | GLY | 109 | PHE | 110 | GLY | 111 | ILE | 112 |
| VAL | 113 | PHE | 114 | THR | 167 | PHE | 168 | ILE | 178 | CYS | 179 |
| ILE | 181 | ILE | 182 | MET | 198 | LEU | 201 | ASN | 202 | ASN | 204 |
| ILE | 205 | LEU | 208 | SER | 209 | SER | 210 | PRO | 211 | ILE | 213 |
| GLN | 214 | ASN | 217 | LEU | 233 | VAL | 237 | MET | 240 | LYS | 241 |
| ASN | 289 | VAL | 292 | ASP | 293 | LEU | 294 | PHE | 295 | GLY | 296 |
| ALA | 297 | GLY | 298 | THR | 299 | GLU | 300 | THR | 301 | THR | 302 |
| SER | 303 | THR | 304 | THR | 305 | ARG | 307 | ASP | 360 | LEU | 361 |
| LEU | 362 | PRO | 363 | THR | 364 | SER | 365 | LEU | 366 | PRO | 367 |
| ASN | 474 | GLY | 475 | PHE | 476 | ALA | 477 | SER | 478 | VAL | 479 |

Residues previously inferred to be in the binding site of 2C9 from modelling (e.g. homology modelling, SRS proposals, 3D/4D-QSAR, sequence alignments, or mutagenesis studies) which with the aid of the crystal structure are now known to line the 2C9 binding pocket are in Table 5.

**Table 5: Residues previously inferred to be in the binding site of 2C9**

| | | | | | | | | | | | |
|---|---|---|---|---|---|---|---|---|---|---|---|
| ARG | 97 | GLY | 98 | ILE | 99 | PHE | 100 | LEU | 102 | ALA | 103 |
| ALA | 106 | ASN | 107 | GLY | 109 | PHE | 110 | GLY | 111 | ILE | 112 |
| VAL | 113 | PHE | 114 | LEU | 201 | ASN | 202 | ASN | 204 | ILE | 205 |
| LEU | 208 | SER | 209 | SER | 210 | GLN | 214 | LEU | 233 | VAL | 237 |
| MET | 240 | LYS | 241 | ASN | 289 | VAL | 292 | ASP | 293 | LEU | 294 |
| PHE | 295 | GLY | 296 | ALA | 297 | GLY | 298 | THR | 299 | GLU | 300 |
| THR | 301 | THR | 302 | SER | 303 | THR | 304 | THR | 305 | ARG | 307 |
| ASP | 360 | LEU | 361 | LEU | 362 | PRO | 363 | THR | 364 | SER | 365 |
| LEU | 366 | PRO | 367 | ASN | 474 | GLY | 475 | PHE | 476 | ALA | 477 |
| SER | 478 | | | | | | | | | | |

Some residues found in the binding pocket have never before been identified as binding site residues. These are listed in Table 6. The identification of these will greatly facilitate the modelling of compound binding.

**Table 6: Residues newly identified as lining the 2C9 binding pocket**

| | | | | | | | | | | | |
|---|---|---|---|---|---|---|---|---|---|---|---|
| THR | 167 | PHE | 168 | ILE | 178 | CYS | 179 | ILE | 181 | ILE | 182 |
| MET | 198 | PRO | 211 | ILE | 213 | ASN | 217 | VAL | 479 | | |

Accordingly, in a preferred aspect of the invention, the selected coordinates used in a method of the invention will comprise at least one coordinate, preferably at least one side-chain coordinate of an amino acid residue selected from either Table 5 or 6.

Preferably, the selected coordinates include the coordinates of all the atoms of Table 1, 2, 3 or 8 relating to at least one amino acid from Table 5 or 6.

Also preferred, whether all or just some atoms of a particular amino acid are selected, is that at least 2, more preferably at least 5, and most preferably at least 10 of the selected coordinates are of side chain residues from the corresponding number of different amino acid residues. These may be selected exclusively from either of Table 5 or 6, or a combination thereof. Preferably at least one side chain residue coordinate of Table 6 is included.

### Example 14: Modelling other P450 structures.

Some of the residues in Tables 5 and 6 are residues which do not occur at the sequence positions indicated in the Tables in a naturally occurring human 2C9 or are residues which differ in other human P450 structures. For these residues in particular, molecular modelling techniques (including but not limited to molecular replacement or computer assisted semi-manual methods) may be used to obtain a model in which a different residue is provided at such a location. For example, position 206 (recited in Table 5 above) in the protein 2C9-FGloop is lysine, which comprises a positive charge. Using the X-ray diffraction data of the 2C9-FGloopK206E we have modelled the 2C9-FGloop protein to provide coordinate data for this protein.

The coordinate data corresponds to Table 1 apart from the data for residue 206, which is as follows:

| | | | | | | | | | | | | |
|---|---|---|---|---|---|---|---|---|---|---|---|---|
| ATOM | 1328 | N | LYS | A | 206 | -9.209 | 86.411 | 32.115 | 1.00 | 52.64 | A | N |
| ATOM | 1329 | CA | LYS | A | 206 | -8.030 | 86.236 | 32.948 | 1.00 | 53.62 | A | C |
| ATOM | 1330 | CB | LYS | A | 206 | -6.751 | 86.197 | 32.104 | 1.00 | 56.05 | A | C |
| ATOM | 1331 | CG | LYS | A | 206 | -6.295 | 84.776 | 31.762 | 1.00 | 60.03 | A | C |
| ATOM | 1332 | CD | LYS | A | 206 | -7.406 | 83.981 | 31.026 | 1.00 | 61.59 | A | C |
| ATOM | 1333 | CE | LYS | A | 206 | -7.093 | 82.478 | 30.921 | 1.00 | 62.61 | A | C |
| ATOM | 1334 | NZ | LYS | A | 206 | -6.906 | 81.756 | 32.235 | 1.00 | 63.34 | A | N |
| ATOM | 1335 | C | LYS | A | 206 | -7.966 | 87.351 | 33.963 | 1.00 | 52.66 | A | C |
| ATOM | 1336 | O | LYS | A | 206 | -7.663 | 87.117 | 35.125 | 1.00 | 52.36 | A | O |

It will be observed that the CB carbon atom, i.e. the first carbon atom in the side-chain is in an almost identical position to the Glu CB carbon atom of Table 1, whereas the remaining atoms of the side chain (CD, CE & NZ) are in locations based upon a low-energy configuration of the lysine side chain, taking into account the connection of the side chain to the CA carbon atom. It is thus relatively simple for a person skilled in the art of molecular modelling to arrive at a model for a P450 in which one or more residues of the 2C9-FGloopK206E is replaced in an analogous manner. The coordinate data for 2C9-FGloop are set out in Table 3. It will be appreciated by those of skill in the art that in the space group P321 it is possible to index diffraction data in one of two ways. The data can be converted from one indexing to the other using the operator k, h, -1. In the case of 2C9-FGloop K206E at 3.0 Å (Table 1) the data were indexed differently compared to the data of 2C9-FGloop K206E at 2.6 Å (Table 2) or 2C9-FGloop (Table 3), and hence while the crystal forms of the proteins are substantially identical, the crystal structures are not in the same absolute space. Hence the co-ordinate data for Glu206 in Table 3 is not numerically equivalent to that shown above as this modelled in Table 1. Those of skill in the art will be able to convert the above data for residue 206 accordingly.

Thus where in modelling the interaction of a compound or a metabolite thereof with a P450 structure of the invention, it is found that the residue at position 206 may be involved in the interaction with that particular metabolite, the above data for residue 206 may be used in Table 1 in place of the data for 206Glu. This is in view of the change of charge which results from the difference. For compounds or metabolites which are found to interact with other regions of P450, there may be no need to amend Table 1 in this manner.

However, similar modelling may be performed for other parts of P450 where it is determined to be important that the potential interactions of a compound with the binding pocket in those parts of the protein is of particular interest. Thus the residues Pro220, Ala221, Leu222 and Leu223 in particular were remodelled in a similar manner to that discussed above in order to predict the coordinates of wild type residue side chains in a P450 structure.

The modelled coordinates of the 2C9 wild type protein are the same as those contained in Table 1, 2, 3 or 8 except that the residues listed in Table 7 substitute for the corresponding residues of Table 1, 2, 3 or 8.

Thus the present invention covers a structure of 2C9 for use *in silico* in which the coordinates are those of Table 1, 2, 3 or 8, except that the atoms and corresponding coordinates of one or more of residues 215, 216, 220, 221, 222, and 223 are substituted by the atoms and corresponding coordinates of the wild typed residues of Table 7. Thus to the extent that previous aspects of the invention relate to Table 1, 2, 3 or 8, they also relate to Table 1, 2, 3 or 8 with the atoms and corresponding coordinates of one or more of residues 215, 216, 220, 221, 222, and 223 substituted by those of the wild typed residues listed in Table 7.

### Example 15: Docking Experiment.

The crystal structure of 2C9 was used to computationally dock a drug molecule into the binding pocket. The drug diclofenac, a known substrate for human 2C9, was generated and placed into the 2C9 binding pocket using interactive computer graphics. The observed interactions can now be used to chemically modify diclofenac via a structure-based design strategy to mediate its interaction with human 2C9 and improve its therapeutic potential.

### Example 16: Refinement of 2C9-FGloop K206E structure.

Data generated in Example 11 was further refined to generated Table 8 (Figure 5). A total of 147 water molecules have been added (manually and automatically) and included in the refinement. This resulted in an Rfactor of 20.7% and a R free factor of 25.9%.

### Example 17: Production of further 2C9 proteins.

The nucleic acid encoding 2C9trunc, 2C9P220 (also called 1072), 2C9-FGloop (1015) and 2C9-FGloop K206E (1155) were used to produce further 2C9-encoding nucleic acids using either cassette mutagenesis (CM) or site-directed mutagenesis (QC). Site-Directed Mutatgenesis (PCR mutagenesis) was performed using the Stratagene Quikchange^{™} mutagenesis kit (catalogue number #200518), according to manufacturers instructions. The Quikchange^{™} mutagenesis method generates a mutated plasmid with staggered nicks and uses DpnI digestion to remove all parental DNA. Reactions were made incorporating 5.0 µL of 10X reaction buffer, 5-50 ng template plasmid DNA, 1.0 µL dNTP mix and 125 ng oligonucleotide primers. The primers and template used for each construct are as listed in the table below.

Reactions were made to 50 µL with sterile water, 2.5U Pfu Turbo was then added and the reaction overlayed with 30 µL mineral oil. Thermocycling was then carried out as follows: 95°C, 30 see (1 cycle), 95°C, 30 sec, 55°C, 1 min, 68°C 13.5 min (18 cycles) and finally a holding period at 4°C. A control reaction was also included with water in place of oligonucleotide primers. Following thermocycling 10 U DpnI was added, under the level of the mineral oil, to each reaction. The reactions were then gently mixed followed by centrifugation in a bench top microcentrifuge, 1 min, 13,000 rpm and incubated at 37°C for 3 hr.

Digested product (1 µL) was then used to transform 50 µL competent *E. coli* XL1-Blue cells (Stratagene). The whole transformation as then plated onto Luria agar plates containing 100 µg/ml carbenicillin, inverted, and incubated overnight at 37°C. Plasmid DNA was prepared from individual colonies and sequenced to check for the insertion of the correct mutation(s).

Cassette mutagenesis was performed on the 2C9 FG region (residues 215 to 226) utilising the BamHI and XmaI sites, two unique and natural restriction sites that are present in this region. Complementary oligonucleotides with the 5' BamHI and 3' Xmal overhang restriction sites were designed to introduce mutations in the FG region (Tables 9, 10 and 14). Double stranded oligonucleotides were prepared by heating 10 µg of a mixture of complementary Oligonucleotides at 100°C for 5 min in 100 µl of water and slow cooling at 25°C. Double stranded Oligonucleotides were ligated into purified plasmid pCW-2C9 wt opened by *BamHI and XmaI* restriction enzymes and an aliquot of the ligation was used to transform Xll Blue *E*. *coli.*

2C9 proteins of the invention produced by the above methods are set out in Table 9, which also indicates the primers used. Crystals of all these proteins were obtained under a variety of conditions, shown in Table 11 (see Example 20).

As controls, 2C9 proteins without proline at 220 were made using the same techniques. The proteins made are shown in Table 10. Under a range of conditions tested, no protein crystals were recovered.

**Table 9. Further 2C9 Proteins of the invention.**

| Clone | Mutations | Primers (SEQ ID NOs) | Template | Cloning strategy |
|---|---|---|---|---|
| 1078 | 2C9 S220P | Fw 5 'ccagatctgcaataattttccgcctatcattgattacttccc3' (125) | 2C9 trunc | QC |
| | | Rev 5'ggtctagacgttaaaaggcggatagtaactaatgaaggg3' (126) | | |
| 1081 | 2C9-FGloop + N466D | Fw 5'ctctggttgacccaaaggaccttgacaccactccag3' (127) | 2C9-FGloop | QC |
| | | Rev 5'ctggagtggtgtcaaggtcctttgggtcaaccagag3' (128) | | |
| 1082 | 2C9-FGloop + F482S | Fw 5'gcctctgtgccgccctcctaccagctgtgcttcatt3' (129) | 2C9-FGloop | QC |
| | | Rev 5'aatgaagcacagctggtaggagggcggcacagaggc3' (130) | | |
| 1085 | 2C9-FGloop+ Q192E | Fw 5'gcgctttgattataaagatgagcaatttcttaacttaatggaaaag3' (131) | 2C9-FGloop | QC |
| | | Rev 5'cttttccattaagttaagaaattgctcatcmataatcaaagcgc3' (132) | | |
| 1097 | 2C9-FGloop + Q193E | Fw 5'gattataaagatcaggaatttcttaacttaatggaaaag3' (133) | 2C9-FGloop | QC |
| | | Rev 5'cttttccattaagttaagaaattcctgatctttataatcaaagcgc3' (134) | | |
| 1100 | 2C9-FGloop + E253K | Fw 5'gtaaaagaacaccaaaaatcaatggacatgaacaaccctc3' (135) | 2C9-FGloop | QC |
| | | Rev 5'gagggttgttcatgtccattgatttttggtgttctmac3' (136) | | |
| 1101 | 2C9-FGloop + K273Q | Fw 5'cctgatgaaaatggagcaggaaaagcacaaccaacc3' (137) | 2C9-FGloop | QC |
| | | Rev 5'ggttggttgtgcttttcctgctccattttcatcagg3' (138) | | |
| 1102 | 2C9-FGloop+ K275DH276D | Fw 5'gatgaaaatggagaaggaagacgacaaccaaccatctgaatttac3' (139) | 2C9-FGloop | QC |
| | | Rev 5'taaattcagatggttggttgtcgtcttccttctccattttcatc3' (140) | | |
| 1115 | 2C9-FGloop+ E415A | Fw 5'catcactttctggatgcaggtggcaattttaagaaaag3' (141) | 2C9-FGloop | QC |
| | | Rev 5'cttttcttaaaattgccacctgcatccagaaagtgatg3' (142) | | |
| 1116 | 2C9-FGloop + K465A | Fw 5'cctgaaatctctggttgacccagcgaaccttgacaccac3' (143) | 2C9-FGloop | QC |
| | | Rev 5'gtggtgtcaaggttcgctgggtcaaccagagatttcagg3' (144) | | |
| 1117 | 2C9-FGloop+ K48A | Fw 5'cctacagataggtattgcggacatcagcaaatccttaacc3' (145) | 2C9-FGloop | QC |
| | | Rev 5'ggttaaggatttgctgatgtccgcaatacctatctgtagg3' (146) | | |
| 1118 | 2C9-FGloop + K160A | Fw 5'catcactttctggatgcaggtggcaattttaagaaaag3' (147) | 2C9-FGloop | QC |
| | | Rev 5'cttttcttaaaattgccacctgcatccagaaagtgatg3' (148) | | |
| 1121 | 2C9-FGloop + K273A | Fw 5'gatgaaaatggaggcggaaaagcacaaccaaccatc3' (149) | 2C9-FGloop | QC |
| | | Rev 5'gatggttggttgtgcttttccgcctccattttcatc3' (150) | | |
| 1122 | 2C9-FGloop + E81A | Fw 5'gtggtgctgcatggatatgcagcagtgaaggaagccc3' (151) | 2C9-FGloop | QC |
| | | Rev 5'gggcttccttcactgctgcatatccatgcagcaccac3' (152) | | |
| 1123 | 2C9-FGloop+ K118AK119AK 121A | Fw 5'gttttcagcaatggagcggcatgggcggagatccggcg3' (153) | 2C9-FGloop | QC |
| | | Rev 5'cgccggatctccgcccatgccgctccattgctgaaaac3' (154) | | |
| 1165 | 2C9-FGloop + del HI loop | Fw 5'ggagaaggaaaagcactctgaatttactattgaaagcttgg3' (155) | 2C9-FGloop | QC |
| | | Rev 5'ccaagctttcaatagtaaattcagagtgcttttccttctcc3' (156) | | |
| 1220 | 2C9 S220P P221S | Fw 5' gatctgcaataattttccttctatcattgattacttc3' (157) | 2C9 trunc | QC |
| | | Rev 5' gaagtaatcaatgatagaaggaaaattattgcagatc3' (158) | | |
| 1319 | 2C9-FGloop + L71S | Fw 5'gtgttcactctgtattttggctcgaaacccatagtggtgc3' (159) | 2C9-FGloop | QC |
| | | Rev 5'gcaccactatgggtttcgagccaaaatacagagtgaacac3' (160) | | |
| 1339 | 2C9-FGloop + Y243F | Fw 5'cgttgcttttatgaaaagttttattttggaaaaagtaaaagaacacc3' (161) | 2C9-FGloop | QC |
| | | Rev 5'ggtgttcttttactttttccaaaataaaacttttcataaaagcaacg3' (162) | | |
| 1340 | 2C9-FGloop + E81A Y243F | Fw 5'gtggtgctgcatggatatgcagcagtgaaggaagccc3' (163) | 2C9-FGloop | QC |
| | | Rev 5'gggcttccttcactgctgcatatccatgcagcaccac3' (164) | | |
| | | Fw 5'cgttgcttttatgaaaagttttattttggaaaaagtaaaagaacacc3' (165) | | |
| | | Rev 5'ggtgttcttttactttttccaaaataaaacttttcataaaagcaacg3' (166) | | |
| 1361 | 2C9 C216Y S220P P221A I222L I223L | Fw 5'gatccagatttacaataatttccctgctctccttgattatttc3' (167) | 2C9 trunc | CM |
| | | Rev 5'ccgggaaataatcaaggagagcagggaaattattgtaaatctg3' (168) | | |
| 1362 | 2C9 I215V S220P P221A 1222L I223L | Fw 5'gatccaggtctgcaataatttccctgctctccttgattatttc3' (169) | 2C9 trunc | CM |
| | | Rev 5'ccgggaaataatcaaggagagcagggaaattattgcagacctg3' (170) | | |
| 1363 | 2C9121SV C216Y S220P P221A I223L | Fw 5'gatccaggtctacaataatttccctgctatccttgattatttc3' (171) | 2C9 trunc | CM |
| | | Rev 5'ccgggaaataatcaaggatagcagggaaattattgtagacctg3' (172) | | |
| 1364 | 2C9 1215V C216Y S220P P221A 1222L | Fw 5'gatccaggtctacaataatttccctgctctcattgattatttc3' (173) | 2C9 trunc | CM |
| | | Rev 5'ccgggaaataatcaatgagagcagggaaattattgtagacctg3' (174) | | |
| 1366 | 2C9 S220P P221A 1222L 1223L | Fw 5'gatccagatttgcaataatttccctgctctccttgattatttc3' (175) | 2C9 trunc | CM |
| | | Rev 5'ccgggaaataatcaaggagagcagggaaattattgcaaatctg3' (176) | | |
| 1367 | 2C9 S220P P221A 1222L | Fw 5'gatccagatttgcaataatticcctgctctcattgattamc3' (177) | 2C9 trunc | CM |
| | | Rev 5'ccgggaaataatcaatgagagcagggaaattattgcaaatctg3' (178) | | |
| 1368 | 2C9 S220P P221A | Fw 5'gatccagatttgcaataaMecctgctatcattgattattic3' (179) | 2C9 trunc | CM |
| | | Rev 5'ccgggaaataatcaatgatagcagggaaattattgcaaatctg3' (180) | | |
| 1369 | 2C9 I215V C216Y S220P P221A | Fw 5'gatccaggtctacaataaMccctgctatcattgattatttc3' (181) | 2C9 trunc | CM |
| | | Rev 5'ccgggaaataatcaatgatagcagggaaattattgtagacctg3' (182) | | |
| 1370 | 2C9 1215V S220P P221A | Fw 5'gatccaggtctgcaataatttccctgctatcattgattatttc3' (183) | 2C9 trunc | CM |
| | | Rev 5'ccgggaaataatcaatgatagcagggaaattattgcagacctg3' (184) | | |
| 1371 | 2C9 C216Y S220P P221A I222L | Fw 5'gatccagamataataatttccctgctctcattgattatttc3' (185) | 2C9 trunc | CM |
| | | Rev 5'ccgggaaataatcaatgagagcagggaaattattataaatctg3' (186) | | |
| 1372 | 2C9 C216S S220P P221A | Fw 5'gatccagatttctaataatttccctgctatcattgattatttc3' (187) | 2C9 trunc | CM |
| | | Rev 5'ccgggaaataatcaatgatagcagggaaattattagaaatctg3' (188) | | |
| 1391 | 2C9-FGloop + N2S8H | Fw 5'caatggacatgcacaaccctc3' (189) | 2C9-FGloop | QC |
| | | Rev 5'gagggttgtgcatgtccattg3' (190) | | |
| 1392 | 2C9-FGloop+ Q252H | Fw 5'gtaaaagaacaccatgaatcaatggacatg3' (191) | 2C9-FGloop | QC |
| | | Rev 5'catgtccattgattcatggtgttctmac3' (192) | | |
| 1394 | 2C9-FGloop + Q484H | Fw 5'gccgcccttctaccacctctgcttc3' (193) | 2C9-FGloop | QC |
| | | Rev 5'gaagcagaggtggtagaagggcggc3' (194) | | |
| 1396 | 2C9-FGloop + Q340S | Fw 5'ggagcccctgcatgagcgacaggagcc3' (195) | 2C9-FGloop | QC |
| | | Rev 5'ggctcctgtcgctcatgcaggggctcc3' (196) | | |
| 1397 | 2C9-FGloop + E415V E4381 | Fw 5'ccctcatcactttctggatgttggtggcaattttaag3' (197) | 2C9-FGloop | QC |
| | | Rev 5'cttaaaattgccaccaacatccagaaagtgatgaggg3' (198) | | |
| | | Fw 5'ggatttgtgtgggaatcgccctggccggcatgg3' (199) | | |
| | | Rev 5'ccatgccggccagggcgattcccacacaaatcc3' (200) | | |
| 1424 | 2C9 P221A 1222P | Fw 5'gatctgcaataatttttctgctcccattgattacttcccgggaac3' (201) | 2C9 trunc | QC |
| | | Rev 5'gttcccgggaagtaatcaatgggagcagaaaaattattgcagatc3' (202) | | |
| 1443 | 2C9-FGloop + R329N Q484H | Fw 5'ccaggaagagattgaaaatgtgattggc3' (203) | 2C9-FGloop | QC |
| | | Rev 5'gccaatcacattttcaatctcttcctgg3' (204) | | |
| | | Fw 5'gccgcccttctaccacctctgcttc3' (205) | | |
| | | Rev 5'gaagcagaggtggtagaagggcggc3' (206) | | |
| 1444 | 2C9-FGloop + K206H E415V | Fw 5'gaatgaaaacatccacattttgagcagcccc3' (207) | 1155 | QC |
| | | Rev 5'ggggctgctcaaaatgtggatgttttcattc3' (208) | | |
| | | Fw 5'ccctcatcactttctggatgttggtggcaattttaag3' (209) | | |
| | | Rev 5'cttaaaattgccaccaacatccagaaagtgatgaggg3' (210) | | |
| 1475 | 2C9-FGloop + K206E N231H | Fw 5'cccgggaactcaccacaaattacttaaaaacg3' (211) | 1155 | QC |
| | | Rev 5'cgtttttaagtaatttgtggtgagttcccggg3' (212) | | |
| 1477 | 2C9-FGloop+ K206E F100S | Fw 5'ctggaagaggcattagcccactggctgaaag3' (213) | 1155 | QC |
| | | Rev 5'ctttcagccagtgggctaatgcctcttccag3' (214) | | |
| 1491 | 2C9-FGloop + K206E L208A | Fw 5'gaaaacatcgagattgcgagcagcccctggatcc3' (215) | 1155 | QC |
| | | Rev 5'ggatccaggggctgctcgcaatctcgatgttttc3' (216) | | |

**Table 10. Control 2C9 Proteins.**

| Clone | Mutations | Primers (SEQ ID NOs) | Template | Cloning strategy |
|---|---|---|---|---|
| 1039 | 2C9 P221A | Fw 5'cctggatccagatctgcaataatttttctgctatcattgattacttcccgggaactatc3' (217) | 2C9 trunc | QC |
| | | Rev 5'gatagttcccgggaagtaatcaatgatagcagaaaaattangcagatetggatccagg3' (218) | | |
| 1365 | 2C9 1215V C216Y | Fw 5'gatccaggtctacaataatttctctgctctccttgattatttc3' (219) | 2C9 trunc | CM |
| | P221A 1222L 1223L | Rev 5'ccgggaaataatcaaggagagcagagaaattattgtagacctg3' (220) | | |
| 1423 | 2C9 F219P P221A | Fw 5'gatccagatctgcaataatccttctgctatcattgartacttcc3' (221) | 2C9 trunc | QC |
| | | Rev 5'ggaagtaatcaatgatagcagaaggattattgcagatctggatc3' (222) | | |

### Example 18: Production of 2C9 proteins.

### Bacteria Expression.

The 2C9 proteins of Example 17 were produced in a bacterial expression system. A single ampicillin resistant colony of XL1 blue cells was grown overnight at 37°C in Terrific Broth (TB) with shaking to near saturation and used to inoculate fresh TB media. Bacteria were grown to an OD600nm = 0.4 in 1 litre of TB broth containing 100 µg/ml of ampicillin at 37°C at 185 rpm in 2 litre flask. The heme precursor delta aminolevulinic acid (80 mg/l) was added 30 min prior to induction with 1 mM isopropyl-β-D-thiogalactopyranoside (IPTG) and the temperature lowered to 25°C. The bacterial culture was continued under agitation at 25°C for 72 hours.

### Protein Purification

The cells were pelleted at 10000 g for 10 min and resuspended in a buffer containing 500 mM KPi, pH 7.4, 20% glycerol, 10 mM mercaptoethanol, 0.1 % (v/v) of protease inhibitor cocktail (Calbiochem), 10 mM imidazole, 40 U/ml DNase 1 and 5 mM MgSO₄.

The cells were lysed by passing twice through a Constant Systems Cell Homogeniser at 10000 psi. The cell debris was then removed by centrifugation at 22000 x g at 4°C for 30 min.

Detergent IGEPAL CA630 (Sigma) was added dropwise from a 10% stock solution to the lysate at a final concentration of 0.3% (v/v) and the lysate was incubated with previously washed NiNTA resin (Qiagen) overnight at 4°C, using agitation. The protein bound-NiNTA resin was pelleted by centrifugation at 2000 g for 5 min at 4°C. The resin was washed with 30 resin volumes of 500 mM KPi, pH 7.4, 20% glycerol, 10 mM mercaptoethanol, 10 mM imidazole, 1:1000 dilution of protease inhibitor cocktail, 0.3%(v/v) IGEPAL CA630 and the resin pelleted by centrifugation at 2000 xg for 5 min at 4°C. The resin was then washed with 15 resin volumes of 500 mM KPi, pH 7.4, 20% glycerol, 10 mM mercaptoethanol, 20 mM imidazole, 0.1 % (v/v) protease inhibitors, 0.3% IGEPAL CA630 and the resin recovered by centrifugation as described above.

The resin was packed into a column at 4°C and the cytochrome P450 eluted with 500 mM KPi, pH 7.4, 20 % glycerol, 10 mM mercaptoethanol, 300 mM imidazole, 1:1000(v/v) of protease inhibitor cocktail, 0.3%(v/v) IGEPAL CA630.

The cytochrome P450 obtained from the NiNTA column was quickly desalted into 10 mM KPi, pH 7.4, 20% glycerol, 2.0 mM DTT, 1 mM EDTA using a HiPrep 26/10 desalting column (Pharmacia), at a flow rate of 5 ml/min.

The desalted cytochrome P450 was directly applied to a CM Sepharose column (Pharmacia), previously equilibrated with 10 mM KPi, pH 7.4, 20% glycerol, 2.0 mM DTT, 1 mM EDTA. The following step elution was applied: wash with 20 column volumes of 10 mM KPi, pH 7.4, 20% glycerol, 2.0 mM DTT, 1 mM EDTA, wash with the above buffer with 75 mM KCl in order to remove any trace of detergent, then eluted with the above buffer with KCl concentration increased to 500 mM.

The protein was concentrated up to 40 mg/ml using a microconcentrator for crystallization assays. The quality of the final preparation was evaluated by:
*(a) SDS polyacrylamide gel electrophoresis*
   This was performed using commercial gels (Nugen) followed by CBB staining according to the manufacturer's instructions. The purity as estimated by scanning a digital image of a gel was estimated to be at least 95%.
*(b) Mass Spectroscopy*
   Mass spectrometry was performed using a Bruker BioTOF II electrospray time of flight instrument. Samples were either diluted by a factor of 1000 straight from storage buffer into methanol/water/formic acid (50:48:2 v/v/v), or subjected to a reverse phase separation using a C4 Millipore 'zip-tip' or a C4 HPLC column, before being diluted into methanol/water/formic acid. Calibration was achieved by measurement of the 2+ and 1+ charge states of a peptide mixture containing Bombesin and angiotensin I or by using the multiple charge states of Horse Myoglobin. Data were acquired in the *m*/*z* range 200 to 2000 and were subsequently processed using Bruker's X-mass program. Mass accuracy was expected to be better than 1 in 10 000 (100ppm).

Predicted and observed mass spectrometry data for the proteins is listed in Table 12.

**Table 12. Mass Spectrometry Data for 2C9 proteins.**

| Clone ID | Calculated Mass (without N-met) (Da) | Observed Mass (Da) | Mass Diff (without N-met) (ppm) |
|---|---|---|---|
| 1015 | 53964 | 53966.65 | 2.65 |
| 1039 | 53907.96 | 53911.25 | 3.29 |
| 1072 | 53948.02 | 53950.23 | 2.21 |
| 1078 | 53944.03 | 53944 | -0.03 |
| 1081 | 53964.98 | 53967 | 2.02 |
| 1082 | 53903.9 | 53909 | 5.1 |
| 1085 | 53964.98 | 53964.95 | -0.03 |
| 1097 | 53964.98 | 53971 | 6.02 |
| 1100 | 53963.06 | 53971 | 7.94 |
| 1101 | 53963.96 | 53965 | 1.04 |
| 1102 | 53928.86 | 53930.37 | 1.51 |
| 1115 | 53905.96 | 53905 | -0.96 |
| 1116 | 53906.9 | 53910 | 3.1 |
| 1117 | 53906.9 | 53909 | 2.1 |
| 1118 | 53906.9 | 53907 | 0.1 |
| 1121 | 53906.9 | 53910 | 3.1 |
| 1122 | 53905.96 | 53908 | 2.04 |
| 1123 | 53792.72 | 53795.16 | 2.44 |
| 1155 | 53964.94 | 53964.84 | -0.1 |
| 1165 | 53624.65 | 53627 | 2.35 |
| 1220 | 53933.99 | 53936.03 | 2.04 |
| 1319 | 53937.92 | 53942.38 | 4.46 |
| 1339 | 53948 | 53953 | 5 |
| 1340 | 53889.96 | 53892 | 2.04 |
| 1361 | 53978.03 | 53978.83 | 0.8 |
| 1362 | 53903.97 | 53901.62 | -2.35 |
| 1363 | 53964 | 53964.32 | 0.32 |
| 1364 | 53964 | 53966.32 | 2.32 |
| 1365 | 53953.96 | 53955.22 | 1.26 |
| 1366 | 53918 | 53920.06 | 2.06 |
| 1367 | 53918 | 53919.31 | 1.31 |
| 1368 | 53918 | 53900.75 | 33.02 |
| 1369 | 53964 | 53964.08 | 0.08 |
| 1370 | 53903.97 | 53907.52 | 3.55 |
| 1371 | 53978.03 | 53978.74 | 0.71 |
| 1372 | 53901.93 | 53901.83 | -0.1 |
| 1391 | 53987.04 | 53987.61 | 0.57 |
| 1392 | 53973.01 | 53975.41 | 2.4 |
| 1394 | 53973.01 | 53973.65 | 0.64 |
| 1396 | 53922.95 | 53924.21 | 1.26 |
| 1397 | 53918.06 | 53914.83 | -3.23 |
| 1423 | 53857.9 | 53858.94 | 1.04 |
| 1424 | 53891.92 | 53891.92 | 0 |
| 1443 | 53930.93 | 53932.6 | 30.97 |
| 1444 | 53942.98 | 53943.2 | 0.22 |
| 1475 | 53987.98 | 53988.71 | 0.73 |
| 1477 | 53904.84 | 53907.07 | 2.23 |
| 1491 | 53922.86 | 53923.9 | 1.04 |

### Example 19: Activity of 2C9 Proteins of the invention.

Activity assays on P450 2C9 were performed in a 96-well plate assay format with a Fluoroscan Ascent FL Instruments (Labsystem), using the 7-methoxy-4-(trifluoromethyl)-coumarin as a fluorescent substrate.

Fifteen pmoles of purified P450 were reconstituted with 0.1 unit of purified human oxidoreductase, in presence of 137 µM of substrate 7-methoxy-4-(trifluoromethyl)-coumarin and a NADPH regenerating system that includes 0.14 mM NADP⁺, 0.37 mM Glucose-6-phosphate, 0.38 mM MgCl₂ and 2.8 unit/ml glucose-6-phosphate dehydrogenase, in 180 µl final volume of 25 mM KPi, pH 7.4. Incubations were performed at 37°C for 40 minutes and 37.5 pmoles of metabolite standard 7-hydroxy -4-(trifluoromethyl)-coumarin were used to determinate the metabolic rate. The excitation and emission wavelengths used were respectively 409 and 530 nm. The results for the clones tested are set out in Table 13.

**Table 13. Activity Data.**

| Clone | Activity |
|---|---|
| 1015 (2C9-FGloop) | 0.26 |
| 1072 (2C9-P220) | 0.43 |
| 1361 | 0.68 |
| 1362 | 0.33 |
| 1363 | 0.27 |
| 1364 | 0.15 |
| 1366 | 0.27 |
| 1367 | 0.56 |
| 1368 | 0.52 |
| 1369 | 0.43 |
| 1370 | 0.69 |
| 1371 | 0.17 |
| 1372 | 0.20 |

As a control, the activity of the protein 2C9trunc (wild type) and 1365 (which both have no proline at 220) was determined and found to be 0.47 and 0.43, respectively.

### Example 20: Crystallisation of 2C9 proteins.

Crystals of the 2C9 mutants were grown using the hanging drop vapour diffusion method. Protein at 10-60 mg/ml (usually 40 mg/ml) in 10mM Kpi pH 7.4, 0.5 M KCl, 2mM DTT, 1mM EDTA, 20% glycerol, was mixed in a 1:1 ratio, using 0.5 µl drops, with a reservoir solution. A number of different 2C9 proteins of the invention formed crystals under the following reservoir solution conditions:
0.05-0.1 M Tris-HCl pH 8.0-8.8, 0.1-0.2 M Lithium sulphate, 10-15% PEG 4000;
0.1 M Tris pH 8.0-8.8, 15-30% PEG 400, 5% PEG 8000, 10% glycerol; and
0.1-0.4 M KH₂PO₄, 0-25 % PEG 3350, 0-10% glycerol.

Further reservoir solutions containing the conditions listed in Table 11 (Figure 6) were also used to obtain further crystals of various different 2C9 proteins of the invention. In Table 11, crystallisation of the clones identified by clone number was obtained by using a reservoir solution containing the constituent parts listed in the columns, wherein these are as follows:
Buffer (M)-Molarity of buffer (in M).
Buffer-Buffer type.
pH-pH of buffer used.
Salt (M)-Molarity of salt (in M).
Salt-Salt type.
Ppt (M)-Molarity of precipitant (in M).
Ppt-Precipitant type.
Ppt 2 (M)-Molarity of precipitant 2 (in M).
Ppt 2-Precipitant 2 used.
Add M-Molarity of additive (in M).
Additive-Additive used.

### Example 21: 2C9-2C19 Chimeras.

Seven further 2C9 proteins were generated which were based upon substitution into 2C9 of residues found in 2C19. Three chimeras (1661, 1662 and 1664) were generated by site directed mutagenesis as described in Example 17 above and using the primers listed below in Table 14. The four other chimeras, 1595, 1600, 1610 and 1632 were generated by cloning methods as follows:

### Chimera 1595

The mutant 1155 I99H was first generated by the Quikchange™ mutagenesis method, using the oligonucleotides listed in Table 14. Residues 227 to 339 were then substituted in the construct 1155 199H by those present in cytochrome P450 2C19 (clone 1026) by cloning the XmaI/SphI 339-bp DNA fragment of 2C19 into the plasmid pCW-1155 I99H that was opened by the same restriction enzymes, to yield the chimera 1595.

### Chimera 1600

Chimera 1600 was yielded from chimera 1595 by substituting residues 1 to 282 by those found in the 1155 I99H construct. A silent restriction site *EcoRI* (underlined) was introduced into the 1155 I99H construct at position 784 by PCR amplification using the following 5'oligonucleotides: 5'ctttcaatagtgaattcagatggttggttgtgc3' (SEQ ID NO:226) and 5'tatggctaagaaaacgagctctaaagggc3' (SEQ ID NO:225) with the EcoRI restriction site underlined. A total of 28 cycles at 94 °C for 30 sec, 55 °C for 1 min, and 72 °C for 1 min were followed by an extension of 10 min at 72°C. The 795-bp PCR fragment was double digested with *NotI*/*EcoRI* and purified by agarose gel extraction and elution. The NotI/EcoRI DNA fragment was then cloned into the plasmid 1595 opened by the NotI/EcoRI restriction enzymes to yield the 1155 199H/1595 chimera. Finally, the L362I change was introduced in the 1155 199H/1595 chimera by the Quikchange^{™} mutagenesis method, using the oligonucleotides listed in Table 14, to yield the chimera 1600.

### Chimera 1610

Chimera 1610 was yielded from the construct 1155 by substituting residues 215 to 328 by those found in the chimera 1600. The BamHI/AffIII DNA fragment was isolated from the chimera 1600 and cloned into the plasmid pCW-115S opened with the BamHI/AffIII restriction enzymes.

### Chimera 1632

The construct 1632 was yielded from the chimera 1600 by substituting residues 329 to 476 in 1600 by those found in the construct 1155. The AffIII/SalI DNA fragment was isolated from the construct 1155 and cloned into the plasmid pCW-1600 opened with the AffIII/SalI restriction enzymes. Table 14 sets out the chimeras.

**Table 14. 2C9-2C19 chimeras**

| Clone | Mutations | Primers (SEQ ID NOs) |
|---|---|---|
| 1595 | 2C9-FGloop + K206E | Fw 5'ctggaagaggccatttcccactggctgaaag3' (223) |
| | I99H V237L K241E | Rev 5'ctttcagccagtgggaaatggcctcttccag3' (224) |
| | Y243D M2571 Q261R | |
| | M269I H276Q P279Q | |
| | S286N E288V N289I | |
| | V292A F295L I331V | |
| 1600 | 2C9-FGloop + K206E | Fw 5'tatggctaagaaaacgagctctaaagggc3' (225) |
| | 199H S286N E288V | Rev 5'ctttcaatagtgaattcagatggttggttgtgc3'[EcoRI] (226) |
| | N289I V292A F295L | Fw 5'gagatacattgaccttattcccaccagcctgc3' [L362I] (227) |
| | L362I I331V | Rev 5'gcaggctggtgggaataaggtcaatgtatctc3' (228) |
| 1610 | 2C9-FGloop + K206E | (see text) |
| | S286N E288V N289I | |
| | V292A F295L | |
| 1632 | 2C9-FG loop + K206E | (see text) |
| | 199H S286N E288V | |
| | N2891 V292A F295L | |
| 1661 | 2C9-FGloop + K206E | Fw 5'ctgaatttactattgaaaacttggaaatcactgcagttgacttgtttgg3' (229) |
| | 199H S286N N289I | Rev 5'ccaaacaagtcaactgcagtgatttccaagttttcaatagtaaattcag3' (230) |
| 1662 | 2C9-FGloop + K206E | Fw 5'gaatttactattgaaaacttggaaaacactgcagttg3' (231) |
| | S286N | Rev 5'caactgcagtgttttccaagttttcaatagtaaattc3' (232) |
| 1664 | 2C9-FGloop + K206E | Fw 5'gaatttactattgaaaacttggaaaacactgcagttg3' (233) |
| | S286N N289I | Rev 5'caactgcagtgttttccaagttttcaatagtaaattc3' (234) |
| | | Fw 5'ctattgaaagcttggaaatcactgcagttgacttg3' (235) |
| | | Rev 5'caagtcaactgcagtgatttccaagctttcaatag3' (236) |

### Example 22: Production of 2C9-2C19 chimeras.

These protein were produced as described above for the 2C9 proteins of Example 18 above. Predicted and observed mass spectrometry data for the proteins is listed in Table 15.

**Table 15. Mass Spectrometry for 2C9 proteins.**

| Clone ID | Calculated Mass (without N-met) (Da) | Observed Mass (Da) | Mass Diff (without N-met) (ppm) |
|---|---|---|---|
| 1595 | 53889.79 | 53891.6 | 1.81 |
| 1600 | 53908.92 | 53907.07 | -1.85 |
| 1610 | 53898.97 | 53900.75 | 1.78 |
| 1632 | 53922.95 | 53922.27 | -0.68 |
| 1661 | 54015 | 54018.32 | 3.32 |
| 1662 | 53991.97 | 53997.11 | 5.14 |
| 1664 | 53908.92 | 53910.93 | 37.29 |

### Example 23: Validation of 2C9-FGloop K206E.

The substrate specificity of 2C9-FGloop K206E was characterized by performing metabolic assays with diclofenac as substrate, in combination with inhibition assays with six substrates/inhibitors of 2C9 reported in the literature

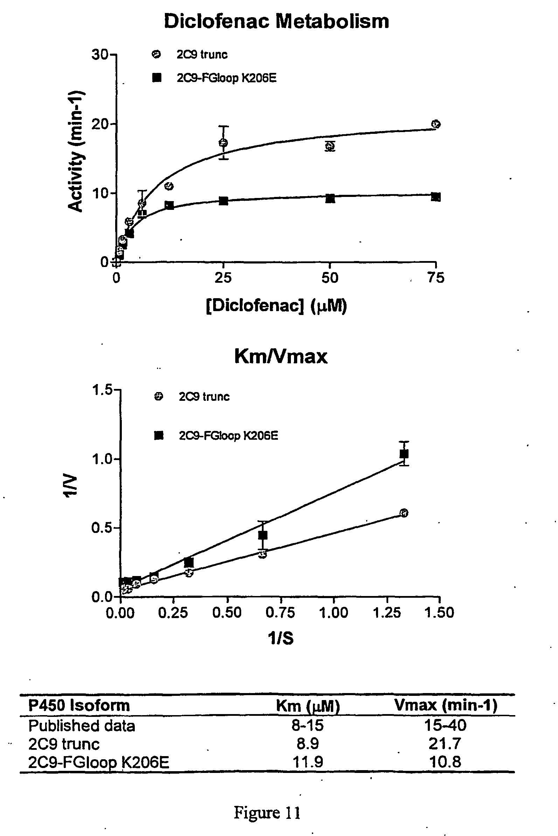

The 4-diclofenac hydroxylase assays. (Figure 11), determined following the method described by Mancy *et al.,* (Biochemistry (1999) 38, 14264-14270) indicate that the Km value of 2C9-FGloop K206E mutant for diclofenac is similar to that obtained for the native N-truncated 2C9, and falls within the range of values reported in the literature for the native full-length 2C9. However, cytochrome P450 2C9-FGloop K206E exhibits a two-fold lower Vmax value that may reflect altered interactions with its redox partner. Results from the inhibition studies (Table 16) also indicate that the inhibition profile of 2C9-FGloop K206E is unchanged when compared to the native N-truncated enzyme, with Ki and IC50 values that match closely those reported in the literature.

These results, altogether, clearly demonstrate that the mutations introduced in the FG loop region to promote the crystallization of 2C9 do not change the substrate specificity, nor do they modify the integrity of the substrate-binding pocket. Therefore, 2C9-FGloop K206E represents a suitable model of the native 2C9 to study the binging mode of chemical compounds into the active site.

**Table 16. Activity of 2C9-FGloop K206E (1155)**

| Compound | 2C9 | 2C9trunc | 2C9-FGloop |
|---|---|---|---|
| | Published data (µM) | IC50/Ki (µM) | K206E |
| | | | IC50/Ki (µM) |
| Bisphenol A | Km=4 | 4.8/2.9 | 7.8/3.3 |
| Fluoxetine | Ki=13 | 1.5/1.5 | 2.1/4.1 |
| Phenytoin | Ki=6 | 250/116 | 40/62 |
| Sulfaphenazole | Ki=0.5 to 1.6 | 0.4/0.9 | 1.6/0.93 |
| 4 Phenyl Imidazole | NA | 3/1.6 | 2.8/1.2 |
| Fluvoxamine | Ki=2-5 | 0.6/0.7 | 0.4/0.85 |

### Example 24: Activity of 2C9-2C19 Chimeras.

The substrate specificity of the proteins made in Example 22 was characterized by performing inhibition assays with six substrates/inhibitors of 2C 19 and 2C9 reported in the literature.

The activity and inhibition assays were performed on the 2C9-2C19 chimeric proteins and the results are shown in Table 17.

**Table 17. Activity of 2C9-2C19 chimeras.**

| | AA | 7-MFC | Sulfaphenazole | Diclofenac | Piroxicam | 4-Phenyl | Fluvoxamine |
|---|---|---|---|---|---|---|---|
| | differences | (min-1) | Ki (µM) | Ki (µM) | Ki (µM) | Imidazole Ki (µM) | Ki (µM) |
| | | | | | | | |
| 1155 | - | 0.23 | 1 | 4 | 30 | 3 | 0.4 |
| 2C19 (published) | 43 | 2.98 | >500 | 231 | 133 | 0.5 | 1.5 |
| 1595 | 15 | 3.2 | | | | | |
| 1600 | 8 | 3.3 | >500 | >200 | >100 | 1 | <1 |
| 1632 | 6 | 3.31 | >500 | >200 | > 100 | 1.1 | 1.6 |
| 1661 | 3 | 2.14 | >500 | > 200 | >100 | 0.9 | 0.5 |

As can be seen 1632 and 1661 display 2C 19 like activity. Hence, a 2C9-2C 19 chimera can also be made by making the following changes I99H S286N E288V N2891 V292A F295L. An alternative minimal mutant is 199H S286N N289I.

### Example 25: Crystallisation of 2C9-2C19 chimeric proteins.

Crystals were prepared as described in Example 20 above. The crystals were grown over a reservoir solution containing the following conditions:
0.05-0.1 M Tris-HCl pH 8.0-8.8, 0.1-0.2 M Lithium sulphate, 10-15% PEG 4000;
0.1 M Tris pH 8.0-8.8, 15-30% PEG 400, 5% PEG 8000, 10% glycerol; and
0.1-0.4 M KH₂PO₄, 0-25 % PEG 3350, 0-10% glycerol; and also the conditions listed in Table 11 (Figure 6).

### Example 26: Homology Modelling of 2C19.

Using homology modelling, a model of the 2C 19 protein was produced. The model was constructed from an alignment of the 2C9 template structure and the target sequence using CLUSTALW. The alignments were adjusted with information from the PSIPRED secondary structure program and optimised manually. The program MODELLER was used to build and optimise the three-dimensional models, with the final model being the one which had the lowest energy and closely satisfied the restraints generated by the program. The 2C 19 model produced is set out in Table 18 (Figure 7).

### Example 27: Homology modelling of 2C18.

This was performed by determining the residues that differ in 2C 18 from 2C 19 and using the techniques described above in Example 25 to determine the coordinates of those residues. These coordinates, set out in Table 19 (Figure 8), may be substituted into the 2C9 or 2C 19 coordinate tables.

### Example 28: Homology modelling of 2C8.

This was performed by determining the residues that differ in 2C8 from 2C 19 and using the techniques described above in Example 25 to determine the coordinates of those residues. These coordinates, set out in Table 20 (Figure 9), may be substituted into the 2C9 or 2C 19 coordinate tables.

### Sequence Listing:

### 2C9trunc

SEQ ID NO:1 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTCCATAAACGTTTTGATTATAAAGAT
CAGCAATTTCTTAACTTAATGGAAAAGTTGAATGAAAACATCAAGATTTTGAGCAGCCCCTGGATCCAGATCTGCAATAATTTTTC
TCCTATCATTGATTACTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA
SEQ ID NO:2

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQICNNFSPIIDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH

### 1072 (2C9-P220)

SEQ ID NO:3 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGATCTGCAATAATTTTCC
GACCATCATTGATTACTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACTGA
SEQ ID NO:4

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQICNNFPTIIDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH

### 2C9-FGloop (1015)

SEQ ID NO:5 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
 
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA
SEQ ID NO:6

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH

### 1155 (2C9-FGloop K206E)

SEQ ID NO:7 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCGAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA
SEQ ID N0:8 
MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIEILSSPWIQVYNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1078 SEQ ID NO:9 & 10 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAATTCTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAGCGCTTTGATTATAAAGAT
CAGCAATTTCTTAACTTAATGGAAAAGTTGAATGAAAACATCAAGATTTTGAGCAGCCCCTGGATCCAGATCTGCAATAATTTTCC
GCCTATCATTGATTACTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
 
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTGTGCTTCATTCCTGTCCATCATCATCATTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQICNNFPPIIDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1081 SEQ ID NO:11 & 12 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGGACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKDLDTTPVVNGFASVPPFYQLCFIPVHHHH
1082 SEQ ID NO:13 & 14 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTCCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPSYQLCFIPVHHHH
1085 SEQ ID NO:15 & 16 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
GAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
EQFLNLMEKLNENIKILSSPWIQVYNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1097 SEQ ID NO:17 & 18 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGGAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QEFLNLMEKLNENIKILSSPWIQVYNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1100 SEQ ID NO:19 & 20 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAAAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
 
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSYILEKVKEHQKSMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1101 SEQ ID NO:21 & 22 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGCAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSYILEKVKEHQESMDMNNPQDFIDCFLMKMEQEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1102 SEQ ID NO:23 & 24 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAGACGACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSYILEKVKEHQESMDMNNPQDFIDCFLMKMEKEDDN
QPSEFTIESLENTAVDLFGAGTETTSTTLRYALLLLLKHPEVTAKVQEEIERVIGRNRSPCMQDRSHMPYTDAVVHEVQRYIDLLP
 
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1115 SEQ ID NO:25 & 26 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGCAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDAGGNFKKSKYFMPFSAGKRICVGEALAGMELFLFL
TSILQNFNLKSLVDPKNLDTTPVVNGFASVPPFYQLCFIPVHHHH
1116 SEQ ID NO:27 & 28 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAGCGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPANLDTTPVVNGFASVPPFYQLCFIPVHHHH
1117 SEQ ID NO:29 & 30 
ATGGCTAAGAAAACGAGCTCTAAAGGGCGGCCGCCTGGCCCCACTCCTCTCCCAGTGATTGGAAATATCCTACAGATAGGTATTGC
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
 
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIADISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1118 SEQ ID NO:31 & 32 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCGCGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTAASPCDPTFILGCAPCNVICSIIFHKRFDYKD
QQFLNLMEKLNENIKILSSPWIQVYNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1121 SEQ ID NO:33 & 34 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGGCGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA
 
MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSYILEKVKEHQESMDMNNPQDFIDCFLMKMEAEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1122 SEQ ID NO:35 & 36 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGCAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYAAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1123 SEQ ID NO:37 & 38 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAGCGGCATGGGCGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGAAWAEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1165 SEQ ID NO:39 & 40 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
 
TGCATGGATATGAAGCAGTGAAGGAAGCCCTGATTGATCTTGGAGAGGAGTTTCTGGAAGAGGCATTTTCCCACTGGCTGAAAGA
GCTAACAGAGGATTTGGAATTGTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACTCT
GAATTTACTATTGAAAGCTTGGAAAACACTGCAGTTGACTTGTTTGGAGCTGGGACAGAGACGACAAGCACAACCCTGAGATATGC
TCTCCTTCTCCTGCTGAAGCACCCAGAGGTCACAGCTAAAGTCCAGGAAGAGATTGAACGTGTGATTGGCAGAAACCGGAGCCCCT
GCATGCAAGACAGGAGCCACATGCCCTACACAGATGCTGTGGTGCACGAGGTCCAGAGATACATTGACCTTCTCCCCACCAGCCTG
CCCCATGCAGTGACCTGTGACATTAAATTCAGAAACTATCTCATTCCCAAGGGCACAACCATATTAATTTCCCTGACTTCTGTGCT
ACATGACAACAAAGAATTTCCCAACCCAGAGATGTTTGACCCTCATCACTTTCTGGATGAAGGTGGCAATTTTAAGAAAAGTAAAT
ACTTCATGCCTTTCTCAGCAGGAAAACGGATTTGTGTGGGAGAAGCCCTGGCCGGCATGGAGCTGTTTTTATTCCTGACCTCCATT
TTACAGAACTTTAACCTGAAATCTCTGGTTGACCCAAAGAACCTTGACACCACTCCAGTTGTCAATGGATTTGCCTCTGTGCCGCC
CTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSYILEKVKEHQESMDMNNPQDFIDCFLMKMEKEKHS
EFTIESLENTAVDLFGAGTETTSTTLRYALLLLLKHPEVTAKVQEEIERVIGRNRSPCMQDRSHMPYTDAVVHEVQRYIDLLPTSL
PHAVTCDIKFRNYLIPKGTTILISLTSVLHDNKEFPNPEMFDPHHFLDEGGNFKKSKYFMPFSAGKRICVGEALAGMELFLFLTSI
LQNFNLKSLVDPKNLDTTPVVNGFASVPPFYQLCFIPVHHHH
1220 SEQ ID NO:41 & 42 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGATCTGCAATAATTTTCC
TTCTATCATTGATTACTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQICNNFPSIIDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1319 SEQ ID NO:43 & 44 
ATGGCTAAGAAAACGAGCTCTAAAGGGCGGCCGCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCTCGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
 
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGSKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1339 SEQ ID NO:45 & 46 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTTTATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTGTGCTTCATTCCTGTCCATCATCATCATTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSFILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1340 SEQ ID NO: 47 & 48 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGCAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTTTATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTGTGCTTCATTCCTGTCCATCATCATCATTGA

MAKKTSSKGRPPGPTPLPVIGNILQIGIKDISKSLTNLSKVYGPVFTLYFGLKPIVVLHGYAAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSFILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1361 SEQ ID NO:49 & 50 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGATTTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTGTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQIYNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1362 SEQ ID NO:51 & 52 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTGCAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTGTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVCNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1363 SEQ ID NO:53 & 54 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTATCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
 
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPAILDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1364 SEQ ID NO:55 & 56 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCATTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALIDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1366 SEQ ID NO: 57 & 58 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGATTTGCAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTTAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQICNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
 
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1367 SEQ ID NO:59 & 60 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGATTTGCAATAATTTCCC
TGCTCTCATTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQICNNFPALIDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1368 SEQ ID NO:61 & 62 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGATTTGCAATAATTTCCC
TGCTATCATTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQICNNFPAIIDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1369 SEQ ID NO:63 & 64 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
 
CAGCAATTTCTTAACTTAATGGAAAAGTTGAATGAAAACATCAAGATTTTGAGCAGCCCCTGGATCCAGGTCTACAATAATTTCCC
TGCTATCATTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPAIIDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1370 SEQ ID NO:65 & 66 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTGCAATAATTTCCC
TGCTATCATTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVCNNFPAIIDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1371 SEQ ID NO:67 & 68 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGATTTATAATAATTTCCC
TGCTCTCATTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTGTGCTTCATTCCTGTCCACCACCACTGA
 
MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQIYNNFPALIDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1372 SEQ ID NO:69 & 70 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGATTTCTAATAATTTCCC
TGCTATCATTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQISNNFPAIIDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1391 SEQ ID NO:71 & 72 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGCACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSYILEKVKEHQESMDMHNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1392 SEQ ID NO:73 & 74 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
 
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCATGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSYILEKVKEHHESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1394 SEQ ID NO:75 & 76 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCACCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYHLCFIPVHHHH
1396 SEQ ID NO:77 & 78 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGAGCGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
 
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMSDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1397 SEQ ID NO:79 & 80 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGTTGGTGGCAATTTTAAGA
AAAGTAAATACTTCATGCCTTTCTCAGCAGGAAAACGGATTTGTGTGGGAATCGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDVGGNFKKSKYFMPFSAGKRICVGIALAGMELFLFL
TSILQNFNLKSLVDPKNLDTTPVVNGFASVPPFYQLCFIPVHHHH
1424 SEQ ID NO:81 & 82 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTTTATGGCCCTGTGTTCACTCTGTATTTTGGCCTGAAACCCATAGTGGTGC
TGCATGGATATGAAGCAGTGAAGGAAGCCCTGATTGATCTTGGAGAGGAATTCTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAGCGCTTTGATTATAAAGAT
CAGCAATTTCTTAACTTAATGGAAAAGTTGAATGAAAACATCAAGATTTTGAGCAGCCCCTGGATCCAGATCTGCAATAATTTTTC
TGCTCCTATTGATTACTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTGTGCTTCATTCCTGTCCATCATCATCATTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQICNNFSAPIDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1443 SEQ ID NO:83 & 84 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAAAATGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCACCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIENVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYHLCFIPVHHHH
1444 SEQ ID NO:85 & 86 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCCACATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGTTGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIHILSSPWIQVYNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDVGGNFKKSKYFMPFSAGKRICVGEALAGMELFLFL
TSILQNFNLKSLVDPKNLDTTPVVNGFASVPPFYQLCFIPVHHHH
1475 SEQ ID NO:87 & 88 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCGAGATTTTGAGCAGCCCCTGGATCCAGGTCTACAATAATTTCCC
TGCTCTCCTTGATTATTTCCCGGGAACTCACCACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
 
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIEILSSPWIQVYNNFPALLDYFPGTHHKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1477 SEQ ID NO:89 & 90 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTAGCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCGAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGISPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIEILSSPWIQVYNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1491 SEQ ID NO:91 & 92 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCGAGATTGCGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIEIASSPWIQVYNNFPALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
 
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1595 SEQ ID NO:93 & 94 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCCATTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTCCATAAACGTTTTGATTATAAAGAT
CAGCAATTTCTTAACTTAATGGAAAAGTTGAATGAAAACATCGAGATTTTGAGCAGCCCCTGGATCCAGGTCTACAATAATTTCCC
TGCTCTCCTTGATTATTTCCCGGGAACCCATAACAAATTACTTAAAAACCTTGCTTTTATGGAAAGTGATATTTTGGAGAAAGTAA
AAGAACACCAAGAATCGATGGACATCAACAACCCTCGGGACTTTATTGATTGCTTCCTGATCAAAATGGAGAAGGAAAAGCAAAAC
CAACAGTCTGAATTCACTATTGAAAACTTGGTAATCACTGCAGCTGACTTACTTGGAGCTGGGACAGAGACAACAAGCACAACCCT
GAGATATGCTCTCCTTCTCCTGCTGAAGCACCCAGAGGTCACAGCTAAAGTCCAGGAAGAGATTGAACGTGTCGTTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGHFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIEILSSPWIQVYNNFPALLDYFPGTHNKLLKNLAFMESDILEKVKEHQESMDINNPRDFIDCFLIKMEKEKQN
QQSEFTIENLVITAADLLGAGTETTSTTLRYALLLLLKHPEVTAKVQEEIERVVGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1600 SEQ ID NO:95 & 96 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCCATTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTCCATAAACGTTTTGATTATAAAGAT
CAGCAATTTCTTAACTTAATGGAAAAGTTGAATGAAAACATCGAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTCACTATTGAAAACTTGGTAATCACTGCAGCTGACTTACTTGGAGCTGGGACAGAGACAACAAGCACAACCCT
GAGATATGCTCTCCTTCTCCTGCTGAAGCACCCAGAGGTCACAGCTAAAGTCCAGGAAGAGATTGAACGTGTCGTTGGCAGAAACC
GGAGCCCCTGCATGCAAGACAGGAGCCACATGCCCTACACAGATGCTGTGGTGCACGAGGTCCAGAGATACATTGACCTTATCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGHFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIEILSSPWIQVYNNFPALLDYFPGTHNKLLKNVAFMKSYILEKVKEHQESMDMNNPQDFIDCFLMKMEKEKHN
QPSEFTIENLVITAADLLGAGTETTSTTLRYALLLLLKHPEVTAKVQEEIERVVGRNRSPCMQDRSHMPYTDAVVHEVQRYIDLIP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1610 SEQ ID NO:97 & 98 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
 
CAGCAATTTCTTAACTTAATGGAAAAGTTGAATGAAAACATCGAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTCACTATTGAAAACTTGGTAATCACTGCAGCTGACTTACTTGGAGCTGGGACAGAGACAACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIEILSSPWIQVYNNFPALLDYFPGTHNKLLKNVAFMKSYILEKVKEHQESMDMNNPQDFIDCFLMKMEKEKHN
QPSEFTIENLVITAADLLGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1632 SEQ ID NO:99 & 100 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCCATTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCGAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTCACTATTGAAAACTTGGTAATCACTGCAGCTGACTTACTTGGAGCTGGGACAGAGACAACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGHFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIEILSSPWIQVYNNFPALLDYFPGTHNKLLKNVAFMKSYILEKVKEHQESMDMNNPQDFIDCFLMKMEKEKHN
QPSEFTIENLVITAADLLGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1661 SEQ ID NO:101 & 102 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCCATTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTCCATAAACGTTTTGATTATAAAGAT
CAGCAATTTCTTAACTTAATGGAAAAGTTGAATGAAAACATCGAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAACTTGGAAATCACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA
 
MAKKTSSKGRPPGPTPLPVIGNILQIGIKDISKSLTNLSKVYGPVFTLYFGLKPIVVLHGYEAVKEALIDLGEEFSGRGHFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIEILSSPWIQVYNNFPALLDYFPGTHNKLLKNVAFMKSYILEKVKEHQESMDMNNPQDFIDCFLMKMEKEKHN
QPSEFTIENLEITAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1662 SEQ ID NO:103 & 104 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCGAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAACTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIEILSSPWIQVYNNFPALLDYFPGTHNKLLKNVAFMKSYILEKVKEHQESMDMNNPQDFIDCFLMKMEKEKHN
QPSEFTIENLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1664 SEQ ID NO:105 & 106 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCGAGATTTTGAGCAGCCCCTGGATCCAGGTCTACAATAATTTCCC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAACTTGGAAATCACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIEILSSPWIQVYNNFPALLDYFPGTHNKLLKNVAFMKSYILEKVKEHQESMDMNNPQDFIDCFLMKMEKEKHN
QPSEFTIENLEITAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1039 SEQ ID NO:107 & 108 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTcTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
 
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTCCATAAACGTTTGATTATAAAGAT
CAGCAATTTCTTAACTTAATGGAAAAGTTGAATGAAAACATCAAGATTTTGAGCAGCCCCTGGATCCAGATCTGCAATAATTTTTC
TGCTATCATTGATTACTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCcTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQICNNFSAIIDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1365 SEQ ID NO: 109 & 110 
ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGGTCTACAATAATTTCTC
TGCTCTCCTTGATTATTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG
ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQVYNNFSALLDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH
1423 SEQ ID NO:111 & 112

ATGGCTAAGAAAACGAGCTCTAAAGGGCGGCCGCCTGGCCCCACTCCTCTCCCAGTGATTGGAAATATCCTACAGATAGGTATTAA
GGACATCAGCAAATCCTTAACCAATCTCTCAAAGGTCTATGGCCCTGTGTTCACTCTGTATTTTGGCCTGAAACCCATAGTGGTGC
TGCATGGATATGAAGCAGTGAAGGAAGCCCTGATTGATCTTGGAGAGGAGTTTTCTGGAAGAGGCATTTTCCCACTGGCTGAAAGA
GCTAACAGAGGATTTGGAATTGTTTTCAGCAATGGAAAGAAATGGAAGGAGATCCGGCGTTTCTCCCTCATGACGCTGCGGAATTT
TGGGATGGGGAAGAGGAGCATTGAGGACCGTGTTCAAGAGGAAGCCCGCTGCCTTGTGGAGGAGTTGAGAAAAACCAAGGCCTCAC
CCTGTGATCCCACTTTCATCCTGGGCTGTGCTCCCTGCAATGTGATCTGCTCCATTATTTTCCATAAACGTTTTGATTATAAAGAT
CAGCAATTTCTTAACTTAATGGAAAAGTTGAATGAAAACATCAAGATTTTGAGCAGCCCCTGGATCCAGATCTGCAATAATCCTTC
TGCTATCATTGATTACTTCCCGGGAACTCACAACAAATTACTTAAAAACGTTGCTTTTATGAAAAGTTATATTTTGGAAAAAGTAA
AAGAACACCAAGAATCAATGGACATGAACAACCCTCAGGACTTTATTGATTGCTTCCTGATGAAAATGGAGAAGGAAAAGCACAAC
CAACCATCTGAATTTACTATTGAAAGCTTGGAAAACACTGCAGTTGACTTGTTTGGAGCTGGGACAGAGACGACAAGCACAACCCT
GAGATATGCTCTCCTTCTCCTGCTGAAGCACCCAGAGGTCACAGCTAAAGTCCAGGAAGAGATTGAACGTGTGATTGGCAGAAACC
GGAGCCCCTGCATGCAAGACAGGAGCCACATGCCCTACACAGATGCTGTGGTGCACGAGGTCCAGAGATACATTGACCTTCTCCCC
ACCAGCCTGCCCCATGCAGTGACCTGTGACATTAAATTCAGAAACTATCTCATTCCCAAGGGCACAACCATATTAATTTCCCTGAC
TTCTGTGCTACATGACAACAAAGAATTTCCCAACCCAGAGATGTTTGACCCTCATCACTTTCTGGATGAAGGTGGCAATTTTAAGA
AAAGTAAATACTTCATGCCTTTCTCAGCAGGAAAACGGATTTGTGTGGGAGAAGCCCTGGCCGGCATGGAGCTGTTTTTATTCCTG

ACCTCCATTTTACAGAACTTTAACCTGAAATCTCTGGTTGACCCAAAGAACCTTGACACCACTCCAGTTGTCAATGGATTTGCCTC
TGTGCCGCCCTTCTACCAGCTCTGCTTCATTCCTGTCCACCACCACCACTGA

MAKKTSSKGRPPGPTPLPVIGNILQIGIKDISKSLTNLSKVYGPVFTLYFGLKPIVVLHGYEAVKEALIDLGEEFSGRGIFPLAER
ANRGFGIVFSNGKKWKEIRRFSLMTLRNFGMGKRSIEDRVQEEARCLVEELRKTKASPCDPTFILGCAPCNVICSIIFHKRFDYKD
QQFLNLMEKLNENIKILSSPWIQICNNPSAIIDYFPGTHNKLLKNVAFMKSYILEKVKEHQESMDMNNPQDFIDCFLMKMEKEKHN
QPSEFTIESLENTAVDLFGAGTETTSTTLRYALLLLLKHPEVTAKVQEEIERVIGRNRSPCMQDRSHMPYTDAVVHEVQRYIDLLP
TSLPHAVTCDIKFRNYLIPKGTTILISLTSVLHDNKEFPNPEMFDPHHFLDEGGNFKKSKYFMPFSAGKRICVGEALAGMELFLFL
TSILQNFNLKSLVDPKNLDTTPVVNGFASVPPFYQLCFIPVHHHH

## Claims

1. A computer-based method for the analysis of the interaction of a molecular structure with a P450 structure, which comprises:
(a) providing a 2C9 P450 structure of any one of Tables 1, 2, 3 and 8 or coordinates having an rmsd of less than 2.0 Å from the backbone atoms of said Tables; or at least 100 selected coordinates thereof;
(b) providing a molecular structure to be fitted to said P450 structure or selected coordinates thereof; and
(c) fitting the molecular structure to said P450 structure.

2. The method of claim 1 wherein said selected coordinates include atoms from one or more of the residues of Table 4.

3. The method of claim 1 wherein at least one of said atoms is from a residue of Table 6.

4. The method of claim 1 wherein at least 10 of the selected coordinates are of side chain residues from the corresponding number of different amino acids residues selected from Table 5 or 6, or a combination thereof.

5. A method of predicting three dimensional structures of P450 homologues or analogues of unknown structure, the method comprises the steps of:
aligning a representation of an amino acid sequence of a target P450 protein of unknown three-dimensional structure with the amino acid sequence of the P450 of Table 1, 2, 3 or 8 to match homologous regions of the amino acid sequences;
modelling the structure of the matched homologous regions of said target P450 of unknown structure on the corresponding regions of a 2C9 P450 structure having an rmsd of less than 2.0Å from the backbone atoms of any one of Tables 1, 2, 3 and 8;
determining a conformation for said target P450 of unknown structure which substantially preserves the structure of said matched homologous regions.

6. The method of claim 5 wherein said target P450 protein is selected from the group consisting of 2C8, 2C18 and 2C19.

7. The method of claim 6 wherein the conformation of the target P450 is the structure of Table 18.

8. A method for determining the structure of a protein, which method comprises;
providing the co-ordinates of a 2C9 P450 structure having an rmsd of less than 2.0Å from the backbone atoms of any one of Tables 1, 2, 3 and 8, or at least 100 selected coordinates thereof, and
either (a) positioning said co-ordinates in the crystal unit cell of said protein so as to provide a structure for said protein, or (b) assigning NMR spectra peaks of said protein by manipulating said co-ordinates.

9. A method for determining the structure of a compound bound to P450 protein, said method comprising:
providing a crystal of P450 protein;
soaking the crystal with the compound to form a complex; and
determining the structure of the complex by employing the data of a 2C9 P450 structure having an rmsd of less than 2.0Å from the backbone atoms of any one of Tables 1, 2, 3 and 8, or at least 100 selected coordinates thereof.

10. A method for determining the structure of a compound bound to P450 protein, said method comprising:
mixing P450 protein with the compound;
crystallizing a P450 protein-compound complex; and
determining the structure of the complex by employing the data of a 2C9 P450 structure having an rmsd of less than 2.0Å from the backbone atoms of any one of Tables 1,2,3 and 8, or at least 100 selected coordinates thereof.

11. The method of any one of claims 1 to 4 or 8 to 10 wherein at least 500 selected coordinates are used.

12. The method of any one of claims 1 to 11 wherein said rmsd of less than 2 Å is less than 1 Å.

13. The method of any one of claims 1 to 11 wherein said rmsd of less than 2 Å is less than 0.64 Å.

## Patentansprüche

1. Computergestütztes Verfahren zur Analyse der Wechselwirkung zwischen einer Molekülstruktur und einer P450-Struktur, umfassend:
(a) Bereitstellen einer 2C9-P450-Struktur aus Tabelle 1, 2, 3 oder 8 oder von Koordinaten mit einem RMSD-Wert von weniger als 2,0 Å von den Rückgratatomen aus diesen Tabellen; oder von zumindest 100 davon ausgewählten Koordinaten;
(b) Bereitstellen einer Molekülstruktur, die an die P450-Struktur oder ausgewählte Koordinaten davon angepasst werden soll; und
(c) Anpassen der Molekülstruktur an die P450-Struktur.

2. Verfahren nach Anspruch 1, worin die ausgewählten Koordinaten Atome von einem oder mehreren Resten aus Tabelle 4 enthalten.

3. Verfahren nach Anspruch 1, worin zumindest eines der Atome von einem Rest aus Tabelle 6 stammt.

4. Verfahren nach Anspruch 1, worin zumindest 10 der ausgewählten Koordinaten von Seitenkettenresten der entsprechenden Anzahl an unterschiedlichen Aminosäureresten aus Tabelle 5 oder 6 oder eine Kombination daraus sind.

5. Verfahren zur Vorhersage dreidimensionaler Strukturen von P450-Homologen oder -Analogen mit unbekannter Struktur, wobei das Verfahren folgende Schritte umfasst:
Ausrichten einer Darstellung einer Aminosäuresequenz eines P450-Zielproteins mit unbekannter dreidimensionaler Struktur mit der Aminosäuresequenz des P450 aus Tabelle 1, 2, 3 oder 8, um homologe Regionen der Aminosäuresequenzen zur Deckung zu bringen;
Modellieren der Struktur der zur Deckung gebrachten homologen Regionen des Ziel-P450 mit unbekannter Struktur an den entsprechenden Regionen einer 2C9-P450-Struktur mit einem RMSD-Wert von weniger als 2,0 Å von den Rückgratatomen aus Tabelle 1, 2, 3 oder 8; und
Bestimmen einer Konformation für das Ziel-P450 mit unbekannter Struktur, welche die Struktur der zur Deckung gebrachten homologen Regionen im Wesentlichen beibehält.

6. Verfahren nach Anspruch 5, worin das P450-Zielprotein aus der aus 2C8, 2C18 und 2C19 bestehenden Gruppe ausgewählt ist.

7. Verfahren nach Anspruch 6, worin die Konformation des Ziel-P450 die Struktur aus Tabelle 18 ist.

8. Verfahren zur Bestimmung der Struktur eines Proteins, wobei das Verfahren Folgendes umfasst:
Bereitstellen der Koordinaten einer 2C9-P450-Struktur mit einem RMSD-Wert von weniger als 2,0 Å von den Rückgratatomen aus Tabelle 1, 2, 3 oder 8 oder von zumindest 100 davon ausgewählten Koordinaten und
entweder (a) Positionieren der Koordinaten in der Kristallelementarzelle des Proteins, um eine Struktur für das Protein bereitzustellen, oder (b) Zuordnen von NMR-Spektren-Peaks des Proteins durch Manipulieren der Koordinaten.

9. Verfahren zur Bestimmung der Struktur einer an P450-Protein gebundenen Verbindung, wobei das Verfahren Folgendes umfasst:
Bereitstellen eines Kristalls von P450-Protein;
Einweichen des Kristalls in der Verbindung, um einen Komplex zu bilden; und
Bestimmen der Struktur des Komplexes mithilfe der Daten einer 2C9-P450-Struktur mit einem RMSD-Wert von weniger als 2,0 Å von den Rückgratatomen aus Tabelle 1, 2, 3 oder 8 oder von zumindest 100 davon ausgewählten Koordinaten.

10. Verfahren zur Bestimmung der Struktur einer an P450-Protein gebundenen Verbindung, wobei das Verfahren Folgendes umfasst:
Mischen des P450-Proteins mit der Verbindung;
Kristallisieren eines P450-Protein-Verbindung-Komplexes; und
Bestimmen der Struktur des Komplexes mithilfe der Daten einer 2C9-P450-Struktur mit einem RMSD-Wert von weniger als 2,0 Å von den Rückgratatomen aus Tabelle 1, 2, 3 oder 8 oder von zumindest 100 davon ausgewählten Koordinaten.

11. Verfahren nach einem der Ansprüche 1 bis 4 oder 8 bis 10, worin zumindest 500 ausgewählte Koordinaten verwendet werden.

12. Verfahren nach einem der Ansprüche 1 bis 11, worin der RMSD-Wert von weniger als 2,0 Å weniger als 1 Å beträgt.

13. Verfahren nach einem der Ansprüche 1 bis 11, worin der RMSD-Wert von weniger als 2,0 Å weniger als 0,64 Å beträgt.

## Revendications

1. Méthode assistée par ordinateur pour l'analyse de l'interaction d'une structure moléculaire avec une structure de P450, qui consiste à :
(a) prévoir une structure de P450 2C9 de l'un quelconque des Tableaux 1, 2, 3 et 8 ou des coordon nées ayant une valeur de rmsd de moins de 2,0 Å à partir des atomes d'épine dorsale desdits tableaux ; ou au moins 100 coordonnées sélectionnées ;
(b) prévoir une structure moléculaire à adapter à ladite structure de P450 ou des coordonnées sélectionnées de celle-ci ;
(c) adapter la structure moléculaire à ladite structure de P450.

2. Méthode de la revendication 1, où lesdites coordonnées sélectionnées comprennent des atomes de un ou plusieurs des résidus du Tableau 4.

3. Méthode de la revendication 1, où au moins l'un desdits atomes est un résidu du Tableau 6.

4. Méthode de la revendication 1, où au moins 10 des coordonnées sélectionnées sont de résidus de chaîne latérale du nombre correspondant de résidus d'acides aminés différents sélectionnés dans les Tableaux 5 ou 6 ou une combinaison.

5. Méthode de prédiction de structures tridimensionnelles d'homologues de P450 ou analogues d'une structure inconnue, la méthode comprenant les étapes de :
aligner une représentation d'une séquence d'acides aminés d'une protéine P450 cible d'une structure tridimensionnelle inconnue avec la séquence d'acides aminés de P450 du Tableau 1, 2, 3 ou 8 pour adapter les régions homologues des séquences d'acides aminés ;
modéliser la structure des régions homologues adaptées de ladite P450 cible de structure inconnue sur les régions correspondantes d'une structure de P450 2C9 ayant une valeur de rmsd de moins de 2,0 Å à partir des atomes d'épine dorsale de l'un des Tableaux 1, 2, 3 et 8 ;
déterminer une conformation pour ladite P450 cible de structure inconnue qui préserve sensiblement la structure desdites régions homologues adaptées.

6. Méthode de la revendication 5, où ladite protéine P450 cible est sélectionnée dans le groupe consistant en 2C8, 2C18 et 2C19.

7. Méthode de la revendication 6, où la conformation de P450 cible est la structure du Tableau 18.

8. Méthode pour déterminer la structure d'une protéine, laquelle méthode consiste à :
prévoir les coordonnées d'une structure de P450 2C9 ayant une valeur de rmsd de m oins de 2,0 Å à partir des atomes d'épine dorsale de l'un quelconque des Tableaux 1, 2, 3 et 8 ou au moins 100 coordonnées sélectionnées, et
soit (a) positionner lesdites coordonnées dans la cellule cristalline unitaire de ladite protéine afin de produire une structure pour ladite protéine, ou (b) assigner les pics de spectre de RMN de ladite protéine par manipulation desdites coordonnées.

9. Méthode pour déterminer la structure d'un composé lié à la protéine P450, ladite méthode consistant à :
prévoir un cristal de la protéine P450 ;
inhiber le cristal du composé pour former un complexe ; et
déterminer la structure du complexe en employant les données d'une structure de P450 2C9 ayant une valeur de rmsd de moins de 2,0 Å à partir des atomes d'épine dorsale de l'un quelconque des Tableaux 1, 2, 3 et 8 ou au moins 100 coordonnées sélectionnées.

10. Méthode pour déterminer la structure d'un composé lié à la protéine P450, ladite méthode consistant à :
mélanger la protéine P450 au composé ;
cristalliser un complexe composé-protéine P450 ; et
déterminer la structure du complexe en employant les données d'une structure de P450 2C9 ayant une valeur de rmsd de moins de 2,0 Å à partir des atomes d'épine dorsale de l'un quelconque des Tableaux 1, 2, 3 et 8 ou au moins 100 coordonnées sélectionnées.

11. Méthode de l'une quelconque des revendications 1 4 ou 8 à 10, où au moins 500 coordonnées sélectionnées sont utilisées.

12. Méthode de l'une quelconque des revendications 1 à 11, où ladite valeur de rmsd de moins de 2,0 Å est inférieure à 1 Å.

13. Méthode de l'une quelconque des revendications 1 à 11, où ladite valeur de rmsd de moins de 2 Å est inférieure à 0,64 Å.
